(19) Europäisches Patentamt
European Patent Office
Office européen des brevets

(11) EP 4 613 749 A1

(12) EUROPEAN PATENT APPLICATION
published in accordance with Art. 153(4) EPC

(43) Date of publication:
**10.09.2025 Bulletin 2025/37**

(21) Application number: **23885109.1**

(22) Date of filing: **03.11.2023**

(51) International Patent Classification (IPC):
***C07D 487/04*** (2006.01) ***C07D 237/34*** (2006.01)
***C07D 401/12*** (2006.01) ***C07D 405/12*** (2006.01)
***C07D 471/04*** (2006.01) ***C07D 237/26*** (2006.01)
***C07D 495/04*** (2006.01) ***C07D 409/12*** (2006.01)
***A61K 31/502*** (2006.01) ***A61K 31/504*** (2006.01)
***A61K 31/53*** (2006.01) ***A61P 37/02*** (2006.01)

(52) Cooperative Patent Classification (CPC):
**A61K 31/50; A61K 31/501; A61K 31/502; A61K 31/504; A61K 31/53; A61P 29/00; A61P 37/02; A61P 37/06; C07D 237/20; C07D 237/26; C07D 237/34; C07D 401/12; C07D 405/12; C07D 409/12; C07D 471/04;** (Cont.)

(86) International application number:
**PCT/CN2023/129677**

(87) International publication number:
**WO 2024/094185 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 CN 202211378563**
**23.03.2023 CN 202310292375**
**02.06.2023 CN 202310646466**
**05.07.2023 CN 202310817782**

(71) Applicant: **TransThera Sciences (Nanjing), Inc.**
**Nanjing, Jiangsu 210032 (CN)**

(72) Inventors:
- **LI, Lin**
  **Nanjing, Jiangsu 210032 (CN)**
- **WU, Frank**
  **Nanjing, Jiangsu 210032 (CN)**

(74) Representative: **Ström & Gulliksson AB**
**Box 5275**
**102 46 Stockholm (SE)**

# (54) NLRP3 INFLAMMASOME INHIBITOR AND USE THEREOF

(57) The present invention belongs to the technical field of medicine, relates to an NLRP3 inflammasome inhibitor and a use thereof, and specifically relates to a compound represented by a general formula (A') or a pharmaceutically acceptable salt, stereoisomer and deuterated product thereof, the definition of each group being as defined in the description. Studies have shown that the compound represented by general formula (A') or the pharmaceutically acceptable salt, stereoisomer and deuterated product thereof have high biological activity against an NLRP3 inflammasome, and have important clinical development value for the treatment of NLRP3-related diseases.

$$Y\text{-}W\text{-}R_3 \qquad (A')$$

EP 4 613 749 A1

(52) Cooperative Patent Classification (CPC): (Cont.)
**C07D 487/04; C07D 495/04**

**Description**

## TECHNICAL FIELD

The present disclosure relates to the technical field of medicines, and particularly to an NLRP3 inflammasome inhibitor and use thereof.

## BACKGROUND

Nucleotide-binding oligomerization domain (NOD)-like receptor protein 3 (NLRP3) belongs to the family of NOD-like receptors (NLRs) and is also known as "pyrin domain-containing protein 3". NLRP3 contains three modules which are a pyrin domain (PYD), a nucleotide-binding site domain (NBD), and a leucine-rich repeat (LRR). Upon receiving stimulation from a sterile inflammatory risk signal, NLRP3 interacts with adaptor apoptosis-associated speck-like protein containing a CARD (ASC) and pro-caspase 1 to form an NLRP3 inflammasome. Activation of the NLRP3 inflammasome results in the release of interleukin-1β (IL-1β) and interleukin-18 (IL-18).

Activation of NLRP3 inflammasome usually requires two steps. The first step involves a priming signal, where Toll-like receptors recognize pathogen-associated molecular patterns (PAMPs) or damage-associated molecular patterns (DAMPs), which in turn transmits a signal into the cells to mediate the activation of the NF-xB signaling pathway, thereby up-regulating the transcription levels of NLRP3 inflammasome-associated components including inactive NLRP3 and pro-IL-1β. The second step involves an activation signal; after a P2X7 receptor and the like receive signal stimulation of ATP, nigericin and the like, NLRP3 monomers are oligomerized to form an NLRP3 oligomer, and then ASC and pro-caspase 1 are recruited, thus forming an NLRP3 inflammasome complex by assembling. This triggers the conversion of pro-caspase 1 to caspase 1 and also the production and secretion of mature IL-1β and IL-18.

Activation of NLRP3 inflammasome is associated with various diseases, for example, autoinflammatory fever syndromes such as cryo-pyrin-associated periodic syndromes (CAPS), sickle cell disease, systemic lupus erythematosus (SLE), chronic liver disease, nonalcoholic steatohepatitis (NASH), gout, pseudogout (chondrocalcinosis), type I and type II diabetes and related complications (e.g., nephropathy or retinopathy), neuroinflammation-related disorders (e.g., multiple sclerosis, brain infection, acute injury, neurodegenerative disease, or Alzheimer's disease), atherosclerosis and cardiovascular risk (e.g., hypertension), hidradenitis suppurativa, wound healing and scarring, and cancer (e.g., colorectal cancer, lung cancer, myeloproliferative tumors, leukemia, myelodysplastic syndrome (MDS), or myelofibrosis). Most treatment methods include symptomatic treatment, slowing of the progression of the diseases/disorders, and surgeries as the last treatment means.

WO2020234715A1 discloses a series of NLRP3 inhibitors and is the first to reveal the use of pyrazine-3-yl phenol compounds in the treatment of NLRP3-mediated diseases.

Currently, as the varieties of NLRP3 inflammasome inhibitors under development are relatively few, developing an NLRP3 inflammasome inhibitor with relatively high activity and better druggability has become a clinical necessity.

## SUMMARY

### Problems to be solved by the present disclosure

The present disclosure studies the following compound or a pharmaceutically acceptable salt, a stereoisomer or a deuteride thereof and discovers that the compound or the pharmaceutically acceptable salt, the stereoisomer or the deuteride thereof has relatively high biological activity on an NLRP3 inflammasome and has an important clinical development value for the treatment of NLRP3 -associated diseases.

### Solutions for solving the problems

A compound represented by general formula (A') or a pharmaceutically acceptable salt, a stereoisomer or a deuteride thereof:

$$Y\text{-}W\text{-}R_3 \qquad (A')$$

wherein W is selected from

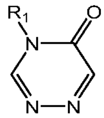 , 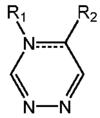 , 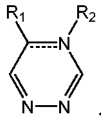 ;

and

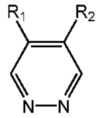 ;

----- is selected from a single bond and a double bond;

$R_1$ is independently selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, $-N(C_{1-6}\ alkyl)_2$, and $-S-C_{1-6}$ alkyl, or is absent;

$R_2$ is independently selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, $-N(C_{1-6}\ alkyl)_2$, and $-S-C_{1-6}$ alkyl, or is absent;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;
or

$R_1$ and $R_2$, together with the C or N atom to which they are attached, form a 5-12 membered ring A, wherein the 5-12 membered ring A is optionally substituted with 1-4 substituents selected from hydroxy, amino, carboxyl, cyano, nitro, halogen, carbonyl, oxo, $C_{1-6}$ alkyl, $-NH-C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and $-N(C_{1-6}\ alkyl)_2$; the 5-12 membered ring is selected from 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl;

$R_3$ is selected from $-(C_{1-6}alkylene)_{0-2}-NR_4R_5$, $-(C_{1-6}alkylene)_{0-2}-NR_4-COR_5$, $-(C_{1-6}alkylene)_{0-2}-CO-NR_4-R_5$, and $-(C_{1-6}alkylene)_{0-2}-NR_4-C_{1-6}alkylene-R_5$;

$R_4$ is selected from hydrogen and $C_{1-6}$ alkyl;

$R_5$ is selected from 3-7 membered heterocyclyl, 3-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, $C_{1-6}$ alkylcarbonyl, ureido $C_{1-6}$ alkyl, hydroxy $C_{1-6}$ alkyl, hydrazino, and $C_{1-6}$ alkylsulfonyl $C_{1-6}$ alkyl;

the substituent on $R_5$, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl;

Y is selected from aryl, 5-14 membered heteroaryl, 3-14 membered heterocyclyl, and 3-12 membered cycloalkyl;

Y is substituted with 1-2 substituents selected from $C_{2-6}$ alkenyl and $C_{2-6}$ alkynyl, and may be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$

alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl;

when the substituent on Y is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, the substituent is optionally substituted with 1-3 substituents selected from halogen, cyano, amino, hydroxy, carbonyl, $C_{1-6}$ alkyl, and 3-6 membered cycloalkyl;

when the substituent on Y is selected from $C_{2-6}$ alkenyl and $C_{2-6}$ alkynyl, the substituent is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

when W is selected from

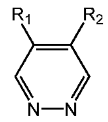

, $R_1$ and $R_2$, together with the C atom to which they are attached, do not form a ring.

In any one of the above technical solutions,

W is selected from

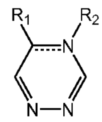

;

$R_1$ is selected from hydrogen, hydroxy, amino, carboxyl, cyano, nitro, halogen, carbonyl, halogenated $C_{1-6}$ alkyl, halogenated $C_{1-6}$ alkoxy, $C_{2-6}$ alkenyl, $C_{2-6}$ alkynyl, 3-7 membered heterocyclyl, 4-7 membered cycloalkyl, aryl, and 5-7 membered heteroaryl, and preferably, $R_1$ is selected from hydrogen, cyano, and halogenated $C_{1-6}$ alkyl.

In any one of the above technical solutions,

W is selected from

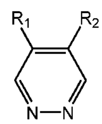

;

Y is selected from aryl, 5-14 membered heteroaryl, 3-14 membered heterocyclyl, and 3-12 membered cycloalkyl;

Y is substituted with $C_{2-6}$ alkynyl, and may be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl;

the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;

the substituent on Y, which is selected from $C_{2-6}$ alkynyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

$R_5$ is optionally substituted with a substituent selected from hydroxy $C_{1-6}$ alkyl, $C_{1-6}$ alkoxy $C_{1-6}$ alkyl, hydrazino, ureido $C_{1-6}$ alkyl, and preferably, $R_5$ is substituted with hydroxy $C_{1-6}$ alkyl and $C_{1-6}$ alkoxy $C_{1-6}$ alkyl.

In any one of the above technical solutions,

W is selected from

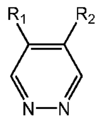;

Y is selected from aryl, 5-14 membered heteroaryl, 3-14 membered heterocyclyl, and 3-12 membered cycloalkyl;

Y is substituted with $C_{2-6}$ alkenyl, and may be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl;

the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;

the substituent on Y, which is selected from $C_{2-6}$ alkenyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

$R_5$ is selected from 3-7 membered heterocyclyl, 5-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, $C_{1-6}$ alkylcarbonyl, ureido $C_{1-6}$ alkyl, hydroxy $C_{1-6}$ alkyl, hydrazino, and $C_{1-6}$ alkylsulfonyl $C_{1-6}$ alkyl.

In any one of the above technical solutions,

W is selected from

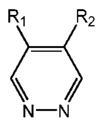;

at least one of $R_1$ and $R_2$ is selected from $C_{2-6}$ alkenyl, $C_{2-6}$ alkynyl, and cyano, and preferably, at least one of $R_1$ and $R_2$ is selected from $C_{2-3}$ alkenyl, $C_{2-3}$ alkynyl, and cyano;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl.

In any one of the above technical solutions,

W is selected from

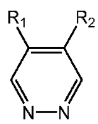;

$R_1$ is selected from hydrogen;

$R_2$ is selected from hydrogen.

In any one of the above technical solutions,

W is selected from

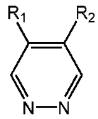

;

Y is selected from aryl, 5-14 membered heteroaryl, 3-14 membered heterocyclyl, and 3-12 membered cycloalkyl;

Y is substituted with propynyl, and may be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl;

the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;

the substituent on Y, which is selected from propynyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

$R_5$ is selected from 3-7 membered cycloalkyl, and preferably, $R_5$ is selected from cyclobutane and cyclohexane.

In any one of the above technical solutions,
$R_5$ is selected from 3-7 membered heterocyclyl, 3-7 membered cycloalkyl, aryl, and 5-7 membered heteroaryl; $R_5$ is substituted with 1-2 substituents selected from deuterium, deuterated $C_{1-6}$ alkyl, ethyl, cyclopropyl, halogen, and halogenated $C_{1-6}$ alkyl, and preferably, $R_5$ is substituted with 1-2 substituents selected from deuterium, deuterated methyl, ethyl, cyclopropyl, and difluoroethane.

In any one of the above technical solutions,

W is selected from

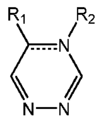

;

$R_5$ is selected from 3-7 membered heterocyclyl, 3-7 membered cycloalkyl, aryl, and 5-7 membered heteroaryl; $R_5$ is substituted with 1-2 substituents selected from hydroxy $C_{1-6}$ alkyl, and preferably, $R_5$ is substituted with hydroxyethyl.

In any one of the above technical solutions,

Y is selected from aryl;
Y is substituted with 1-2 substituents selected from $C_{2-6}$ alkenyl and $C_{2-6}$ alkynyl, and may be further substituted with 1-2 substituents selected from hydroxy, halogen, and halogenated $C_{1-6}$ alkyl, preferably further substituted with hydroxy and halogenated $C_{1-6}$ alkyl; preferably, Y is substituted with a substituent selected from $C_{2-6}$ alkynyl and hydroxy, wherein the $C_{2-6}$ alkynyl may be further substituted with halogenated $C_{1-6}$ alkyl; preferably, Y is substituted with a substituent selected from $C_{2-6}$ alkynyl, hydroxy, and halogen, more preferably, Y is substituted with a substituent selected from trifluoromethylethynyl and hydroxy, and more preferably, Y is substituted with a substituent selected from acetylene and propynyl, and may be further substituted with hydroxy and fluorine substituents.

In any one of the above technical solutions,

W is selected from

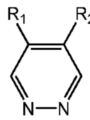;

when $R_2$ is hydrogen, $R_1$ is not selected from methyl and cyclopropyl. In any one of the above technical solutions, $R_5$ is optionally substituted with a substituent selected from hydroxy $C_{1-6}$ alkyl and $C_{1-6}$ alkoxy $C_{1-6}$ alkyl, but the following compounds are excluded:

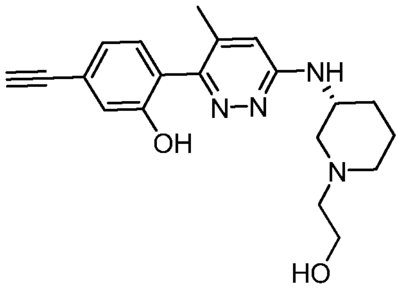 and 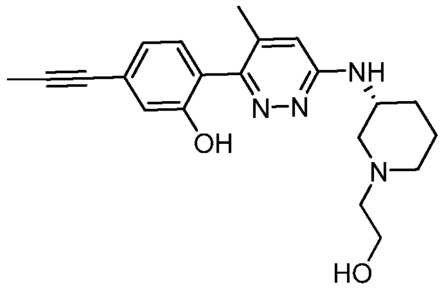.

In any one of the above technical solutions,

W is selected from

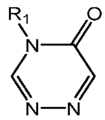;

$R_1$ is selected from hydrogen, halogen, $C_{1-6}$ alkyl, halogenated $C_{1-6}$ alkyl, and 3-7 membered cycloalkyl;

$R_3$ is selected from $-(C_{1-6}\ alkylene)_{0-2}-NR_4R_5$;

$R_4$ is selected from hydrogen and $C_{1-6}$ alkyl;

$R_5$ is selected from 3-7 membered heterocyclyl and 3-7 membered cycloalkyl; $R_5$ is optionally substituted with 1-4 substituents selected from deuterated $C_{1-6}$ alkyl, $C_{1-6}$ alkyl, halogenated $C_{1-6}$ alkyl, 3-7 membered cycloalkyl, hydroxy, and hydroxy $C_{1-6}$ alkyl;

Y is selected from aryl;

Y is substituted with a substituent selected from $C_{2-6}$ alkynyl, and may be further optionally substituted with 1-2 substituents selected from hydroxy, $C_{1-6}$ alkyl, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

In any one of the above technical solutions,

$R_1$ and $R_2$ are selected from hydrogen, halogen, $C_{1-6}$ alkyl, trifluoromethyl, difluoromethyl, cyclopropyl, and cyclobutyl;

$R_3$ is selected from $-NR_4R_5$;

$R_4$ is selected from hydrogen and methyl;

$R_5$ is selected from piperidine, cyclohexyl, cyclopentyl, and cyclobutyl;

Y is selected from a phenyl;

Y is substituted with a substituent selected from ethynyl and propynyl, and may be further optionally substituted with 1-2 substituents selected from hydroxy, $C_{1-6}$ alkyl, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

$R_1$ and $R_2$ are selected from methyl, trifluoromethyl, difluoromethyl, and cyclopropyl;

$R_3$ is selected from $-NR_4R_5$;

$R_4$ is selected from hydrogen;

$R_5$ is selected from piperidinyl;

Y is selected from a phenyl;

Y is substituted with a substituent selected from ethynyl and propynyl, and may be further optionally substituted with 1-2 substituents selected from hydroxy, methyl, trifluoromethyl, difluoromethyl, and cyclopropyl;

the ethynyl and propynyl substituents are optionally substituted with a substituent selected from fluorine, methyl, cyclopropyl, trifluoromethyl, and difluoromethyl;

the substituent on $R_5$ is selected from methyl, ethyl, cyclopropyl, fluorine, chlorine, bromine, difluoroethane, and hydroxyethyl.

In any one of the above technical solutions,

W is selected from

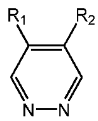 ;

$R_1$ and $R_2$ are each independently selected from hydrogen, cyano, $C_{1-6}$ alkyl, halogenated $C_{1-6}$ alkyl, 3-7 membered cycloalkyl, and $-N(C_{1-6}\ alkyl)_2$, or are absent, and preferably, $R_1$ and $R_2$ are each independently selected from hydrogen, cyano, $C_{1-6}$ alkyl, trifluoromethyl, difluoromethyl, cyclopropyl, cyclobutyl, and $-N(CH_3)_2$;

$R_3$ is selected from $-(C_{1-6}\ alkylene)_{0-2}-NR_4R_5$;

$R_4$ is selected from hydrogen and $C_{1-6}$ alkyl;

Y is selected from aryl;

Y is substituted with $C_{2-6}$ alkynyl, and may be further optionally substituted with 1-2 substituents selected from hydroxy, $C_{1-6}$ alkyl, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

$R_5$ is selected from 3-7 membered heterocyclyl and 3-7 membered cycloalkyl; $R_5$ is optionally substituted with a substituent selected from hydroxy $C_{1-6}$ alkyl, $C_{1-6}$ alkoxy $C_{1-6}$ alkyl, hydrazino, and ureido $C_{1-6}$ alkyl, and preferably, $R_5$ is substituted with hydroxy $C_{1-6}$ alkyl and $C_{1-6}$ alkoxy $C_{1-6}$ alkyl.

In any one of the above technical solutions, formula (A') does not represent the following compounds:

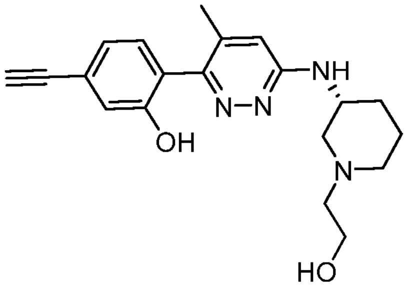

and

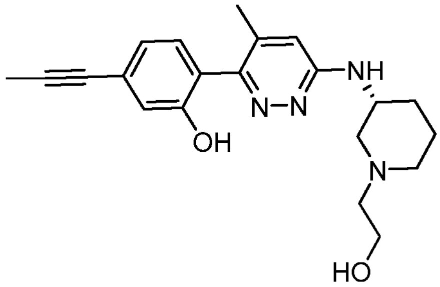

.

In any one of the above technical solutions, when $R_5$ is substituted with hydroxyethyl, $R_1$ is not methyl.

In any one of the above technical solutions, when $R_5$ is substituted with hydroxyethyl, $R_1$ or $R_2$ is selected from cyclopropyl. In any one of the above technical solutions,

W is selected from

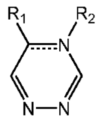

;

$R_1$ and $R_2$ are each independently selected from hydrogen, cyano, $C_{1-6}$ alkyl, halogenated $C_{1-6}$ alkyl, 3-7 membered cycloalkyl, and $-N(C_{1-6}\ alkyl)_2$, or are absent;

$R_3$ is selected from $-(C_{1-6}\ alkylene)_{0-2}-NR_4R_5$;

$R_4$ is selected from hydrogen and $C_{1-6}$ alkyl;

$R_5$ is selected from 3-7 membered heterocyclyl and 3-7 membered cycloalkyl; $R_5$ is substituted with 1-2 substituents selected from hydroxy $C_{1-6}$ alkyl.

Y is selected from aryl;

Y is substituted with a substituent selected from $C_{2-6}$ alkynyl, and may be further optionally substituted with 1-2 substituents selected from hydroxy, $C_{1-6}$ alkyl, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

In any one of the above technical solutions,

W is selected from

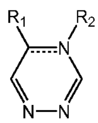

;

$R_1$ and $R_2$ are each independently selected from hydrogen, cyano, $C_{1-6}$ alkyl, trifluoromethyl, difluoromethyl, cyclopropyl, cyclobutyl, and $-N(CH_3)_2$;

$R_3$ is selected from $NR_4R_5$;

$R_4$ is selected from hydrogen;

$R_5$ is selected from piperidinyl, cyclobutyl, and cyclohexyl; $R_5$ is optionally substituted with 1-4 substituents selected from hydroxy, $C_{1-6}$ alkyl, cyano $C_{1-6}$ alkyl, and hydroxy $C_{1-6}$ alkyl;

Y is selected from a phenyl;

Y is substituted with a substituent selected from ethynyl and propynyl, and may be further optionally substituted with 1-2 substituents selected from hydroxy, $C_{1-6}$ alkyl, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

Y is selected from phenyl;

Y is substituted with 1-2 substituents selected from $C_{2-6}$ alkenyl and $C_{2-6}$ alkynyl, and may be further substituted with 1-2 substituents selected from hydroxy and halogenated $C_{1-6}$ alkyl, preferably further substituted with hydroxy and halogenated $C_{1-6}$ alkyl; preferably, Y is substituted with $C_{2-6}$ alkynyl and hydroxy substituents, wherein the $C_{2-6}$ alkynyl may be further substituted with halogenated $C_{1-6}$ alkyl, and more preferably, Y is substituted with trifluoromethylethynyl and hydroxy substituents.

In any one of the above technical solutions,
at least one of $R_1$ and $R_2$ is selected from halogenated $C_{1-6}$ alkyl, preferably, at least one of $R_1$ and $R_2$ is selected from fluorine-substituted $C_{1-6}$ alkyl, and more preferably, at least one of $R_1$ and $R_2$ is selected from trifluoromethyl.

In any one of the above technical solutions,

$R_1$ and $R_2$ are each independently selected from hydrogen, cyano, $C_{1-6}$ alkyl, halogenated $C_{1-6}$ alkyl, 3-7 membered cycloalkyl, and -N($C_{1-6}$ alkyl)$_2$, or are absent;

$R_3$ is selected from -($C_{1-6}$ alkylene)$_{0-2}$-$NR_4R_5$;

$R_4$ is selected from hydrogen and $C_{1-6}$ alkyl;

$R_5$ is selected from 3-7 membered heterocyclyl and 3-7 membered cycloalkyl; $R_5$ is substituted with 1-2 substituents selected from deuterium, deuterated $C_{1-6}$ alkyl, ethyl, and cyclopropyl, and preferably, $R_5$ is substituted with 1-2 substituents selected from deuterium, deuterated methyl, ethyl, and cyclopropyl;

Y is selected from aryl;

Y is substituted with a substituent selected from $C_{2-6}$ alkynyl, and may be further optionally substituted with 1-2 substituents selected from hydroxy, $C_{1-6}$ alkyl, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

In any one of the above technical solutions,

$R_1$ and $R_2$ are each independently selected from hydrogen, cyano, $C_{1-6}$ alkyl, trifluoromethyl, difluoromethyl, cyclopropyl, cyclobutyl, and -N($CH_3$)$_2$;

$R_3$ is selected from -$NR_4R_5$;

$R_4$ is selected from hydrogen;

$R_5$ is selected from piperidinyl, cyclobutyl, and cyclohexyl; $R_5$ is substituted with 1-2 substituents selected from deuterium, deuterated $C_{1-6}$ alkyl, ethyl, and cyclopropyl, and preferably, $R_5$ is substituted with 1-2 substituents selected from deuterium, deuterated methyl, ethyl, and cyclopropyl;

Y is selected from phenyl;

Y is substituted with a substituent selected from ethynyl and propynyl, and may be further optionally substituted with 1-2 substituents selected from hydroxy, $C_{1-6}$ alkyl, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

W is selected from

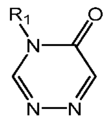 ;

$R_1$ is selected from hydrogen, halogen, $C_{1-6}$ alkyl, trifluoromethyl, difluoromethyl, cyclopropyl, and cyclobutyl;

$R_3$ is selected from $-NR_4R_5$;

$R_4$ is selected from hydrogen and methyl;

$R_5$ is selected from piperidine, cyclohexyl, cyclopentyl, and cyclobutyl; $R_5$ is optionally substituted with 1-3 substituents selected from deuterated $C_{1-6}$ alkyl, $C_{1-6}$ alkyl, trifluoromethyl, cyclopropyl, hydroxy, and hydroxy $C_{1-6}$ alkyl;

Y is selected from a phenyl;

Y is substituted with a substituent selected from ethynyl and propynyl, and may be further optionally substituted with 1-2 substituents selected from hydroxy, $C_{1-6}$ alkyl, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

W is selected from

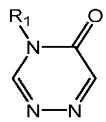 ;

$R_1$ is selected from methyl, trifluoromethyl, difluoromethyl, and cyclopropyl;

$R_3$ is selected from $-NR_4R_5$;

$R_4$ is selected from hydrogen;

$R_5$ is selected from piperidinyl; $R_5$ is optionally substituted with a substituent selected from deuterated methyl, deuterated ethyl, methyl, ethyl, cyclopropyl, and hydroxyethyl;

Y is selected from a phenyl;

Y is substituted with a substituent selected from ethynyl and propynyl, and may be further optionally substituted with 1-2 substituents selected from hydroxy, methyl, trifluoromethyl, difluoromethyl, and cyclopropyl;

the ethynyl and propynyl substituents are optionally substituted with a substituent selected from fluorine, methyl, cyclopropyl, trifluoromethyl, and difluoromethyl.

In any one of the above technical solutions,

W is selected from

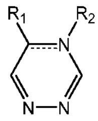 ;

$\overline{\text{- - - - -}}$ is selected from a double bond;

$R_1$ is selected from $C_{1-6}$ alkyl and 3-7 membered cycloalkyl;

$R_2$ is absent;

$R_3$ is selected from $-(C_{1-6}\ alkylene)_{0-2}-NR_4R_5$;

$R_4$ is selected from hydrogen and $C_{1-6}$ alkyl;

$R_5$ is selected from 3-7 membered heterocyclyl and 3-7 membered cycloalkyl; $R_5$ is optionally substituted with 1-4 substituents selected from deuterated $C_{1-6}$ alkyl, ethyl, halogenated $C_{1-6}$ alkyl, 3-7 membered cycloalkyl, hydroxy, and hydroxy $C_{1-6}$ alkyl;

Y is selected from aryl;

Y is substituted with a substituent selected from $C_{2-6}$ alkynyl, and may be further optionally substituted with 1-2 substituents selected from hydroxy, $C_{1-6}$ alkyl, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

In any one of the above technical solutions,

W is selected from

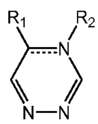 ;

$\overline{\text{- - - - -}}$ is selected from a double bond;

$R_1$ is selected from $C_{1-6}$ alkyl and cyclopropyl;

$R_2$ is absent;

$R_3$ is selected from $-NR_4R_5$;

$R_4$ is selected from hydrogen and methyl;

$R_5$ is selected from piperidine, cyclohexyl, cyclopentyl, and cyclobutyl; $R_5$ is optionally substituted with 1-3 substituents selected from deuterated $C_{1-6}$ alkyl, ethyl, trifluoromethyl, cyclopropyl, hydroxy, and hydroxy $C_{1-6}$ alkyl;

Y is selected from a phenyl;

Y is substituted with a substituent selected from ethynyl and propynyl, and may be further optionally substituted with 1-2 substituents selected from hydroxy, $C_{1-6}$ alkyl, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

W is selected from

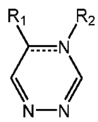 ;

----- is selected from a double bond;

$R_1$ is selected from methyl and cyclopropyl;

$R_2$ is absent;

$R_3$ is selected from $-NR_4R_5$;

$R_4$ is selected from hydrogen;

$R_5$ is selected from piperidinyl; $R_5$ is optionally substituted with a substituent selected from deuterated methyl, deuterated ethyl, ethyl, cyclopropyl, and hydroxyethyl;

Y is selected from a phenyl;

Y is substituted with a substituent selected from ethynyl and propynyl, and may be further optionally substituted with 1-2 substituents selected from hydroxy, methyl, trifluoromethyl, difluoromethyl, and cyclopropyl;

the ethynyl and propynyl substituents are optionally substituted with a substituent selected from fluorine, methyl, cyclopropyl, trifluoromethyl, and difluoromethyl.

In any one of the above technical solutions, $R_1$ and $R_2$ are each independently selected from hydrogen, cyano, $C_{1-6}$ alkyl, halogenated $C_{1-6}$ alkyl, 3-7 membered cycloalkyl, and $-N(C_{1-6}\ alkyl)_2$, or are absent.

In any one of the above technical solutions, $R_1$ and $R_2$ are each independently selected from hydrogen, cyano, $C_{1-6}$ alkyl, trifluoromethyl, difluoromethyl, cyclopropyl, cyclobutyl, and $-N(CH_3)_2$.

In any one of the above technical solutions, W is selected from

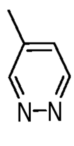 , 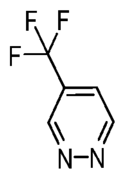 ,

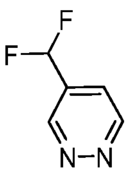 , 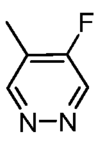 , 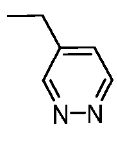 , 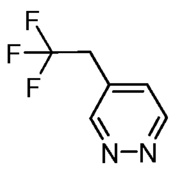 , 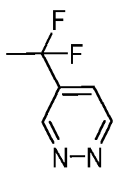 , 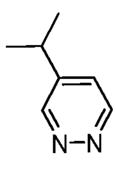 , 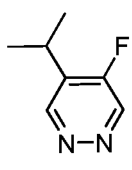 , 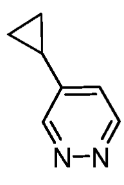 ,

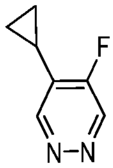, 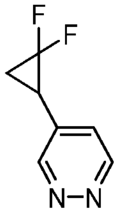, 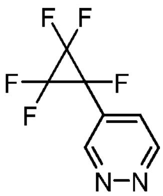, 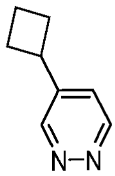, 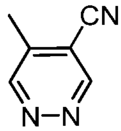, 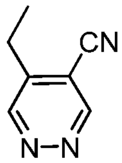, 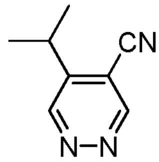,

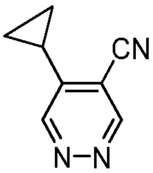, 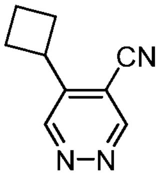, 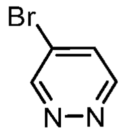, 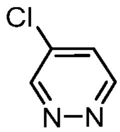, 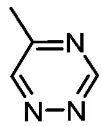, 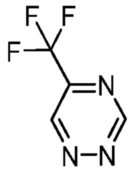, 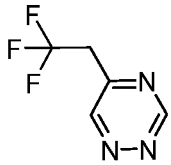,

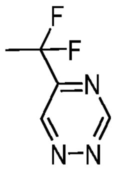, 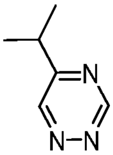, 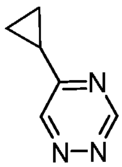, 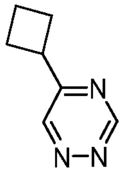, 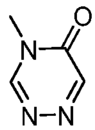, 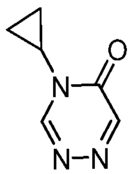, 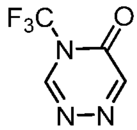, 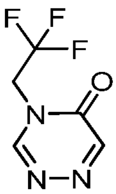,

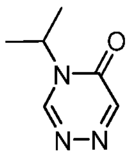, and 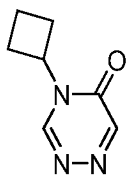.

In any one of the above technical solutions, $R_3$ is selected from -($C_{1-6}$ alkylene)$_{0-2}$-$NR_4R_5$, and preferably, $R_3$ is selected from $NR_4R_5$.

In any one of the above technical solutions, $R_4$ is selected from hydrogen and $C_{1-6}$ alkyl, preferably, $R_4$ is selected from hydrogen and methyl, and preferably, $R_4$ is selected from hydrogen.

In any one of the above technical solutions,

$R_5$ is selected from 3-7 membered heterocyclyl and 3-7 membered cycloalkyl; $R_5$ is optionally substituted with 1-4 substituents selected from halogen, cyano, amino, hydroxy, $C_{1-6}$ alkyl, halogenated $C_{1-6}$ alkyl, 3-7 membered heterocyclyl, and hydroxy $C_{1-6}$ alkyl;

the substituent on $R_5$ is optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl.

In any one of the above technical solutions, R5 is selected from piperidinyl, cyclobutyl, and cyclohexyl; $R_5$ is optionally substituted with 1-4 substituents selected from hydroxy, $C_{1-6}$ alkyl, cyano $C_{1-6}$ alkyl, and hydroxy $C_{1-6}$ alkyl.

In any one of the above technical solutions, $R_5$ is selected from 3-7 membered heterocyclyl, 3-7 membered cycloalkyl, aryl, and 5-7 membered heteroaryl; $R_5$ is substituted with 1-2 substituents selected from deuterium, deuterated $C_{1-6}$ alkyl, ethyl, cyclopropyl, halogen, and halogenated $C_{1-6}$ alkyl.

In any one of the above technical solutions, $R_5$ is selected from piperidinyl, cyclobutyl, and cyclohexyl; $R_5$ is substituted with 1-2 substituents selected from deuterium, deuterated methyl, ethyl, cyclopropyl, fluorine, chlorine, bromine, and difluoroethane.

In any one of the above technical solutions, $R_3$ is selected from

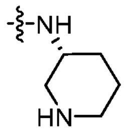,

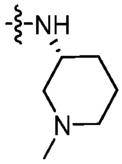, 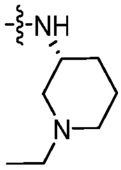, 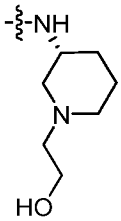, 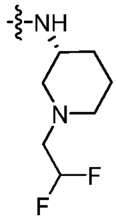, 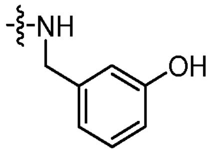, 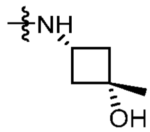, 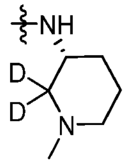,

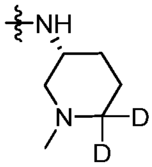, 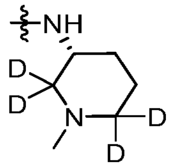, and 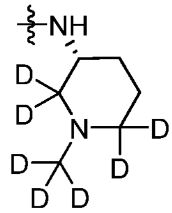.

In any one of the above technical solutions,

Y is selected from aryl;

Y is substituted with a substituent selected from $C_{2-6}$ alkynyl, and may be further optionally substituted with 1-2 substituents selected from hydroxy, $C_{1-6}$ alkyl, halogen, halogenated $C_{1-6}$ alkyl, and 3-7 membered cycloalkyl;

the $C_{2-6}$ alkynyl substituent is optionally substituted with a substituent selected from halogen, $C_{1-6}$ alkyl, 3-6 membered cycloalkyl, and halogenated $C_{1-6}$ alkyl.

In any one of the above technical solutions,

Y is selected from a phenyl;

Y is substituted with a substituent selected from ethynyl and propynyl, and may be further optionally substituted with 1-2 substituents selected from hydroxy, $C_{1-6}$ alkyl, fluorine, chlorine, bromine, trifluoromethyl, difluoromethyl, and cyclopropyl;

the ethynyl and propynyl substituents are optionally substituted with a substituent selected from halogen, $C_{1-6}$ alkyl, cyclopropyl, trifluoromethyl, and difluoromethyl.

In any one of the above technical solutions,

Y is selected from a phenyl;

Y is substituted with a substituent selected from ethynyl and propynyl, and may be further optionally substituted with 1-2 substituents selected from hydroxy, methyl, fluorine, trifluoromethyl, difluoromethyl, and cyclopropyl;

the ethynyl and propynyl substituents are optionally substituted with a substituent selected from bromine, fluorine, methyl, cyclopropyl, trifluoromethyl, and difluoromethyl.

In any one of the above technical solutions, Y is selected from

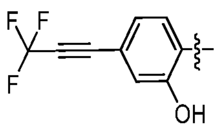,

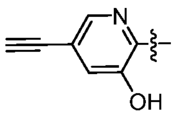, 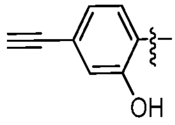, 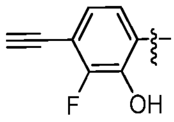, 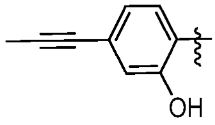, 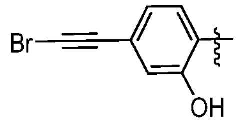

, and

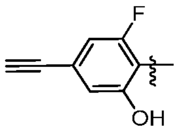.

A compound represented by general formula (A) or a pharmaceutically acceptable salt or a stereoisomer thereof:

$Y\text{-}W\text{-}R_3$ (A)

wherein W is selected from

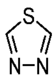, 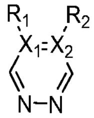, and 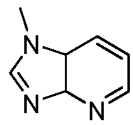;

$X_1$ and $X_2$ are each independently selected from C and N;

$R_1$ is selected from hydrogen, hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, $-N(C_{1-6}\ alkyl)_2$, and $-S-C_{1-6}$ alkyl, or is absent;

$R_2$ is selected from hydrogen, hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, $-N(C_{1-6}\ alkyl)_2$, and $-S-C_{1-6}$ alkyl, or is absent;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;
or

$R_1$ and $R_2$, together with the C or N atom to which they are attached, form a 5-12 membered ring A, wherein the 5-12 membered ring A is optionally substituted with 1-4 substituents selected from hydroxy, amino, carboxyl, cyano, nitro, halogen, carbonyl, $C_{1-6}$ alkyl, $-NH-C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and $-N(C_{1-6}\ alkyl)_2$; the 5-12 membered ring is selected from 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl;

$R_3$ is selected from $-(C_{1-6}alkylene)_{0-2}-NR_4R_5$, $-(C_{1-6}alkylene)_{0-2}-NR_4-COR_5$, $-(C_{1-6}alkylene)_{0-2}-CO-NR_4-R_5$, and $-(C_{1-6}alkylene)_{0-2}-NR_4-C_{1-6}alkylene-R_5$;

$R_4$ is selected from hydrogen and $C_{1-6}$ alkyl;

$R_5$ is selected from 3-7 membered heterocyclyl, 3-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, and $C_{1-6}$ alkylcarbonyl;

when $R_5$ is substituted,

the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl;

Y is selected from aryl, 5-14 membered heteroaryl, 3-14 membered heterocyclyl, and 3-12 membered cycloalkyl;

(1) when $X_1$ and $X_2$ are each selected from C,
Y is substituted with a substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl;

(2) when W is selected from

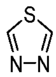 and 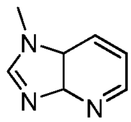

or when any one or both of $X_1$ and $X_2$ are selected from N,

Y is optionally substituted with 1-3 substituents selected from $C_{2-6}$ alkenyl, $C_{2-6}$ alkynyl, halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, $-N(C_{1-6}\ alkyl)_2$, and $-S-C_{1-6}$ alkyl;

when Y is substituted,

the substituents on Y, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl;

the substituents on Y, which are $C_{2-6}$ alkenyl and $C_{2-6}$ alkynyl, are optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl.

In any one of the above technical solutions, the structure formed by attachment of Y and $R_3$ to W has a general formula selected from

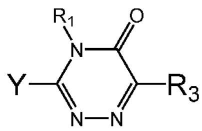, 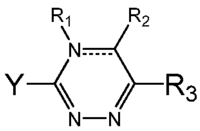,

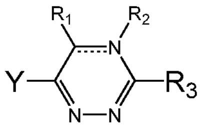, and 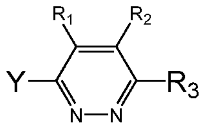..

The present disclosure further provides a compound represented by general formula (I) or a pharmaceutically acceptable salt or a stereoisomer thereof:

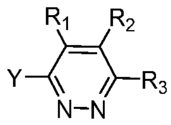

(I)

wherein

$R_1$ is selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, and 5-7 membered heteroaryl;

$R_2$ is selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, and 5-7 membered heteroaryl;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;
or

$R_1$ and $R_2$, together with the carbon atoms to which they are attached, form a 5-12 membered ring A, wherein the 5-12 membered ring A is optionally substituted with 1-4 substituents selected from hydroxy, amino, carboxyl, cyano, nitro, halogen, carbonyl, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$; the 5-12 membered ring is selected from 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl;

$R_3$ is selected from -($C_{1-6}$alkylene)$_{0-2}$-$NR_4R_5$, -($C_{1-6}$alkylene)$_{0-2}$-$NR_4$-$COR_5$, -($C_{1-6}$alkylene)$_{0-2}$-CO-$NR_4$-$R_5$, and -($C_{1-6}$alkylene)$_{0-2}$-$NR_4$-$C_{1-6}$alkylene-$R_5$;

$R_4$ is selected from hydrogen and $C_{1-6}$ alkyl;

$R_5$ is selected from 3-7 membered heterocyclyl, 3-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, and $C_{1-6}$ alkylcarbonyl;

Y is selected from aryl, 5-14 membered heteroaryl, 3-14 membered heterocyclyl, and 3-12 membered cycloalkyl, and Y is substituted with a substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl;

when $R_5$ is substituted,

the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl;

when Y is substituted,

the substituents on Y, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl;

the substituents on Y, which are $C_{2-6}$ alkenyl and $C_{2-6}$ alkynyl, are optionally substituted with 1-3 substituents selected

from halogen, cyano, amino, hydroxy, carbonyl, $C_{1-6}$ alkyl, 3-6 membered cycloalkyl, and halogenated $C_{1-6}$ alkyl.

Further, the present disclosure provides a compound represented by general formula (I) or a pharmaceutically acceptable salt or a stereoisomer thereof:

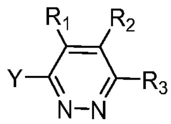

(I)

wherein

$R_1$ is selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, and 5-7 membered heteroaryl;

$R_2$ is selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, and 5-7 membered heteroaryl;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;
or

$R_1$ and $R_2$, together with the carbon atoms to which they are attached, form a 5-12 membered ring A, wherein the 5-12 membered ring A is optionally substituted with 1-4 substituents selected from hydroxy, amino, carboxyl, cyano, nitro, halogen, carbonyl, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$; the 5-12 membered ring is selected from 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl;

$R_3$ is selected from -($C_{1-6}$alkylene)$_{0-2}$-$NR_4R_5$, -($C_{1-6}$alkylene)$_{0-2}$-$NR_4$-$COR_5$, and -($C_{1-6}$alkylene)$_{0-2}$-CO-$NR_4$-$R_5$;

$R_4$ is selected from hydrogen and $C_{1-6}$ alkyl;

$R_5$ is selected from 3-7 membered heterocyclyl, 3-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, and $C_{1-6}$ alkylcarbonyl;

Y is selected from aryl, 5-14 membered heteroaryl, 3-14 membered heterocyclyl, and 3-12 membered cycloalkyl, and Y is substituted with a substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl;

when $R_5$ is substituted,

the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl;

when Y is substituted,

the substituents on Y, which are $C_{1-6}$ alkyl, halogenated $C_{1-6}$ alkyl, $C_{1-6}$ alkoxy, 3-7 membered heterocyclyl, 3-7

membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl;

the substituents on Y, which are $C_{2-6}$ alkenyl and $C_{2-6}$ alkynyl, are unsubstituted.

The present disclosure further provides the compound or the pharmaceutically acceptable salt or the stereoisomer thereof, which has a structure shown as general formula (B):

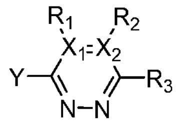
(B)

wherein $R_1$ is selected from hydrogen, hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, -N($C_{1-6}$ alkyl)$_2$, and -S-$C_{1-6}$ alkyl, or is absent;

$R_2$ is selected from hydrogen, hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, -N($C_{1-6}$ alkyl)$_2$, and -S-$C_{1-6}$ alkyl, or is absent;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;

any one or both of $X_1$ and $X_2$ are selected from N;

Y is substituted with hydroxy, and is optionally substituted with 1-2 substituents selected from $C_{2-6}$ alkenyl, $C_{2-6}$ alkynyl, halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl.

In any one of the above technical solutions, Y is selected from phenyl, 5-7 membered heteroaryl, 3-8 membered heterocyclyl, and 3-7 membered cycloalkyl; Y is substituted with $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl;

when Y is substituted, the substituents on Y, which are $C_{1-6}$ alkyl, halogenated $C_{1-6}$ alkyl, $C_{1-6}$ alkoxy, 3-7 membered heterocyclyl, 3-7 membered cycloalkyl, and sulfonyl, are optionally substituted with 1-3 substituents selected from halogen, cyano, amino, hydroxy, carbonyl, and $C_{1-6}$ alkyl;

$R_3$ is selected from -NH-$R_5$, and $R_5$ is 3-7 membered heterocyclyl substituted with 1-2 substituents selected from $C_{1-6}$ alkyl.

In any one of the above technical solutions, the compound or the pharmaceutically acceptable salt or the stereoisomer thereof provided in the present disclosure has a structure shown as general formula (B):

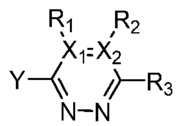
(B)

wherein $R_1$ and $R_2$, together with the C or N atom to which they are attached, form 5-8 membered cycloalkyl, 5-8 membered cycloalkenyl, 5-8 membered heterocyclyl, phenyl, or 5-8 membered heteroaryl, wherein the 5-8 membered cycloalkyl, 5-8 membered cycloalkenyl, 5-8 membered heterocyclyl, phenyl, and 5-8 membered heteroaryl are optionally substituted with 1-4 substituents selected from hydroxy, amino, carboxyl, cyano, nitro, halogen, carbonyl,

$C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$;

any one or both of $X_1$ and $X_2$ are selected from N;

Y is substituted with hydroxy, and is optionally substituted with 1-2 substituents selected from $C_{2-6}$ alkenyl, $C_{2-6}$ alkynyl, halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl.

In any one of the above technical solutions, ring A is selected from 5-12 membered cycloalkyl, 5-12 membered cycloalkenyl, 5-12 membered heterocyclyl, aryl, and 5-12 membered heteroaryl.

In any one of the above technical solutions, ring A is selected from 5-8 membered cycloalkyl, 5-8 membered cycloalkenyl, 5-8 membered heterocyclyl, phenyl, and 5-8 membered heteroaryl.

In any one of the above technical solutions, ring A is selected from phenyl and 5-7 membered heteroaryl; ring A is optionally substituted with 1-4 substituents selected from hydroxy, amino, carboxyl, cyano, nitro, halogen, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, $C_{1-6}$ alkylsulfonyl, and -N($C_{1-6}$ alkyl)$_2$.

In any one of the above technical solutions, ring A is selected from phenyl, 5-6 membered heteroaryl containing 1-2 heteroatoms selected from O, S, and N, and a 5-6 membered saturated ring.

In any one of the above technical solutions, ring A is optionally substituted with 1-4 substituents selected from hydroxy, amino, carboxyl, cyano, nitro, halogen, carbonyl, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$.

In any one of the above technical solutions, ring A is unsubstituted.

In any one of the above technical solutions, ring A is substituted with 1-4 substituents selected from hydroxy, amino, carboxyl, cyano, nitro, halogen, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$.

In any one of the above technical solutions, ring A is substituted with 1-2 substituents selected from cyano, $C_{1-6}$ alkoxy, $C_{1-6}$ alkyl, halogenated $C_{1-6}$ alkyl, $C_{1-6}$ alkylsulfonyl, and -N($C_{1-6}$ alkyl)$_2$.

In any one of the above technical solutions, ring A is substituted with 1 substituent selected from cyano, $C_{1-6}$ alkoxy, $C_{1-6}$ alkyl, halogenated $C_{1-6}$ alkyl, $C_{1-6}$ alkylsulfonyl, and -N($C_{1-6}$ alkyl)$_2$.

In any one of the above technical solutions, ring A is selected from phenyl, 5-6 membered heteroaryl containing 1-2 heteroatoms selected from O, S, and N, and a 5-6 membered saturated ring, and ring A is unsubstituted or substituted with 1 substituent selected from cyano, $C_{1-6}$ alkoxy, $C_{1-6}$ alkyl, halogenated $C_{1-6}$ alkyl, $C_{1-6}$ alkylsulfonyl, and -N($C_{1-6}$ alkyl)$_2$.

In any one of the above technical solutions, ring A is selected from

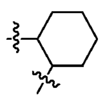,

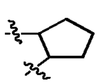, 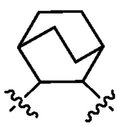, 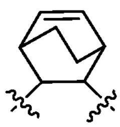, 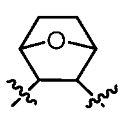, and 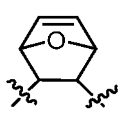

; ring A is optionally substituted with 1-4 substituents selected from hydroxy, amino, carboxyl, cyano, nitro, halogen, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, $C_{1-6}$ alkylsulfonyl, and -N($C_{1-6}$ alkyl)$_2$.

In any one of the above technical solutions, ring A is selected from

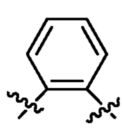,

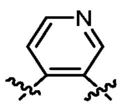, 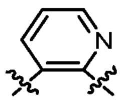, 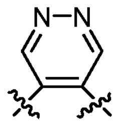, 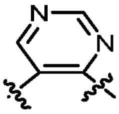, 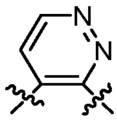, 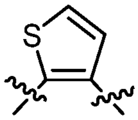, 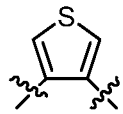, 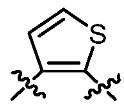,

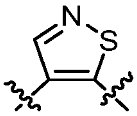, 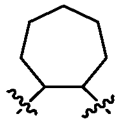, 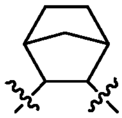, 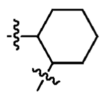, 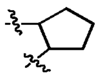, 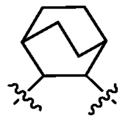, 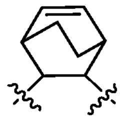, 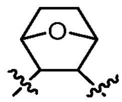

, and

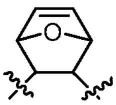

; ring A is optionally substituted with 1-2 substituents selected from cyano, nitro, halogen, $C_{1-6}$ alkyl, halogenated $C_{1-6}$ alkyl, $C_{1-6}$ alkoxy, 3-7 membered cycloalkyl, 5-7 membered heteroaryl, $C_{1-6}$ alkylsulfonyl, and $-N(C_{1-6}\ alkyl)_2$.

In any one of the above technical solutions, ring A is

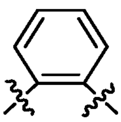, 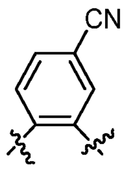,

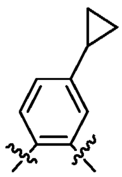, 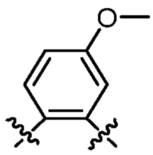, 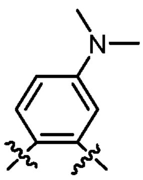, 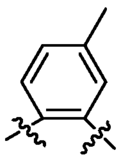, 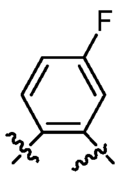, 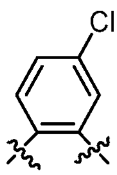, 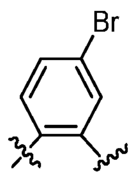, 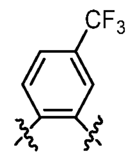,

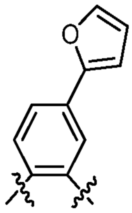, 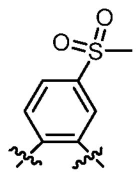, 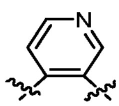, 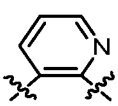, 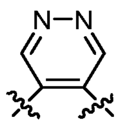, 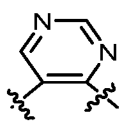, 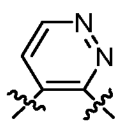, 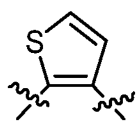,

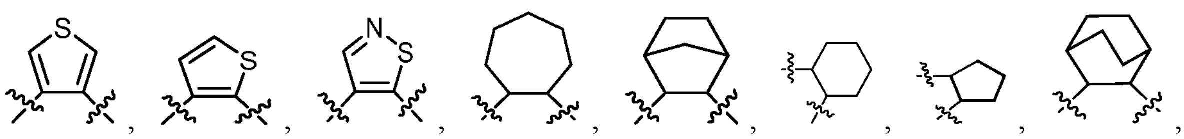

, , , , , , , ,

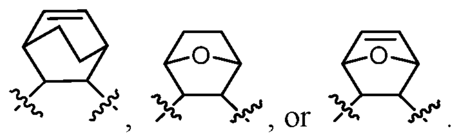

, , or .

**In** any one of the above technical solutions, $R_3$ is selected from $-(C_{1-6}\ alkylene)_{0-2}-NR_4R_5$, $-(C_{1-6}\ alkylene)_{0-2}-NR_4-COR_5$, and $-(C_{1-6}\ alkylene)_{0-2}-CO-NR_4-R_5$; $R_4$ is selected from hydrogen and $C_{1-6}$ alkyl; $R_5$ is selected from 3-7 membered heterocyclyl, 3-7 membered cycloalkyl, aryl, and 5-7 membered heteroaryl.

In any one of the above technical solutions, $R_4$ is selected from hydrogen.

In any one of the above technical solutions, $R_4$ is selected from $C_{1-6}$ alkyl.

In any one of the above technical solutions, $R_4$ is selected from methyl and ethyl.

In any one of the above technical solutions, $R_3$ is selected from $-NR_4R_5$ and $-NR_4-C_{1-6}$ alkylene-$R_5$, wherein $R_4$ is selected from hydrogen, and $R_5$ is selected from 3-7 membered heterocyclyl, 3-7 membered cycloalkyl, aryl, and 5-7 membered heteroaryl.

In any one of the above technical solutions, $R_3$ is selected from $-NR_4R_5$ and $-NR_4-C_{1-6}$ alkylene-$R_5$, wherein $R_4$ is selected from hydrogen, and $R_5$ is selected from 3-7 membered cycloalkyl and 3-7 membered heterocyclyl; preferably, $R_3$ is selected from $-NHR_5$, wherein $R_5$ is selected from 4-6 membered cycloalkyl and 4-6 membered heterocyclyl.

In any one of the above technical solutions, $R_5$ is selected from 4-6 membered cycloalkyl, and 4-6 membered heterocyclyl containing 1 heteroatom selected from O, N, and S.

In any one of the above technical solutions, $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, and $C_{1-6}$ alkylcarbonyl; when $R_5$ is substituted, the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl.

In any one of the above technical solutions, $R_5$ is unsubstituted.

In any one of the above technical solutions, $R_5$ is substituted with 1-2 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, and $C_{1-6}$ alkylcarbonyl.

In any one of the above technical solutions, when $R_5$ is substituted, the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are unsubstituted.

In any one of the above technical solutions, when $R_5$ is substituted, the substituents on $R_5$, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are substituted with 1-3 substituents selected from halogen, cyano, amino, hydroxy, carbonyl, $C_{1-6}$ alkyl, and 3-6 membered cycloalkyl.

In any one of the above technical solutions, $R_5$ is selected from

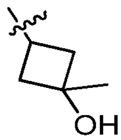 ,

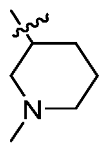 , 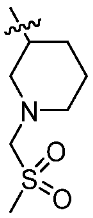 , 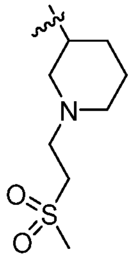 , 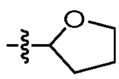 , 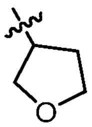 , 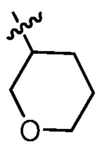 , 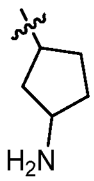 , 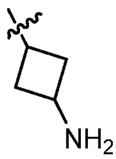 , 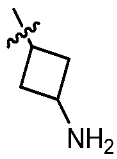 ,

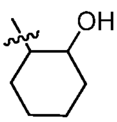 , 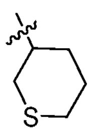 , 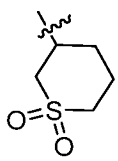 , 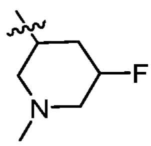 , 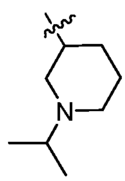 , 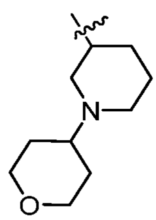 , 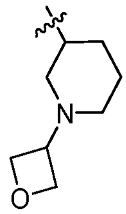 ,

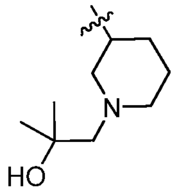, 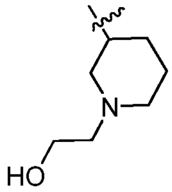, 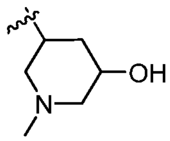, 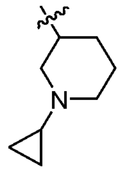, 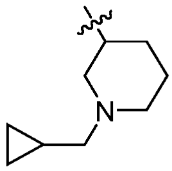, 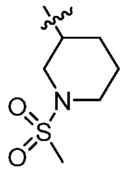

, and

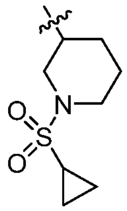.

Preferably, $R_5$ is selected from

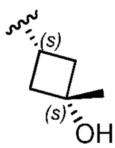, 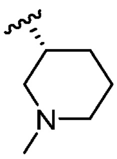, 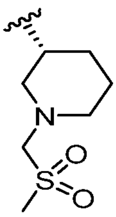, 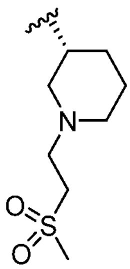,

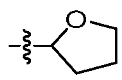, 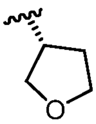, 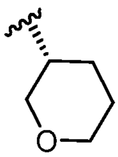, 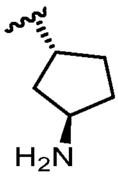, 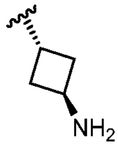, 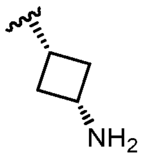, 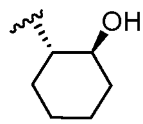, 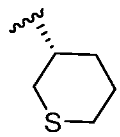,

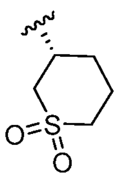, 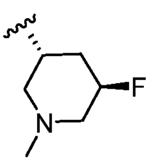, 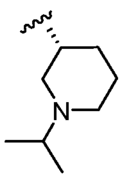, 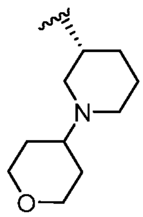, 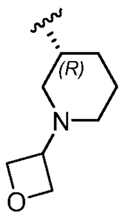, 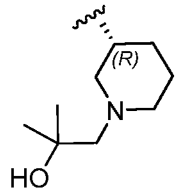, 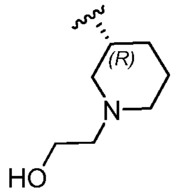,

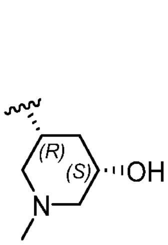, 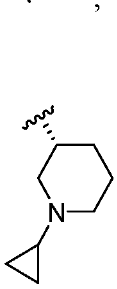, 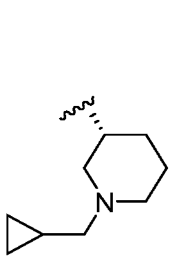, 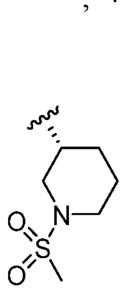, and 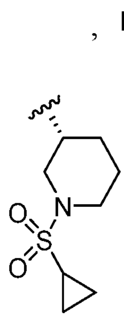.

In any one of the above technical solutions, Y is selected from aryl, 5-14 membered heteroaryl, 3-14 membered heterocyclyl, and 3-12 membered cycloalkyl, and Y is substituted with a substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered

heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl.

In any one of the above technical solutions, Y is selected from phenyl, 5-7 membered heteroaryl, 3-8 membered heterocyclyl, and 3-7 membered cycloalkyl; Y is substituted with $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl.

In any one of the above technical solutions, Y is selected from phenyl, 5-8 membered heteroaryl, 3-8 membered heterocyclyl, and 3-7 membered cycloalkyl.

In any one of the above technical solutions, Y is selected from phenyl and 5-6 membered heteroaryl.

In any one of the above technical solutions, Y is selected from phenyl, and 5-6 membered heteroaryl containing 1-2 N heteroatoms.

In any one of the above technical solutions, Y is substituted with 1 substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl.

In any one of the above technical solutions, Y is substituted with 1-2 substituents selected from $C_{2-6}$ alkenyl and $C_{2-6}$ alkynyl.

In any one of the above technical solutions, Y is substituted with 1 substituent selected from $C_{2-6}$ alkenyl and $C_{2-6}$ alkynyl.

In any one of the above technical solutions, Y is optionally substituted with 1-3 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl.

In any one of the above technical solutions, Y is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl.

In any one of the above technical solutions, Y is substituted with 1-2 substituents selected from $C_{2-6}$ alkenyl and $C_{2-6}$ alkynyl, and is optionally substituted with 1-3 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl.

In any one of the above technical solutions, Y is substituted with 1 substituent selected from $C_{2-6}$ alkenyl and $C_{2-6}$ alkynyl, and Y is optionally substituted with 1 substituent selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl.

In any one of the above technical solutions, Y is substituted with $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, and sulfonyl.

In any one of the above technical solutions, when Y is substituted, the substituents on Y, which are $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, are optionally substituted with 1-3 substituents selected from halogen, cyano, amino, hydroxy, carbonyl, $C_{1-6}$ alkyl, and 3-6 membered cycloalkyl.

In any one of the above technical solutions, the substituents on Y, which are $C_{2-6}$ alkenyl and $C_{2-6}$ alkynyl, are substituted with 1-3 substituents selected from halogen, cyano, amino, hydroxy, carbonyl, $C_{1-6}$ alkyl, 3-6 membered cycloalkyl, and halogenated $C_{1-6}$ alkyl.

In any one of the above technical solutions, the substituents on Y, which are $C_{2-6}$ alkenyl and $C_{2-6}$ alkynyl, are unsubstituted.

In any one of the above technical solutions, Y is selected from phenyl and 5-6 membered heteroaryl, and Y is substituted with a substituent selected from $C_{2-6}$ alkenyl and $C_{2-6}$ alkynyl, and is optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, and 3-7 membered cycloalkyl, wherein the $C_{2-6}$ alkenyl and $C_{2-6}$ alkynyl are substituted with 1-3 substituents selected from halogen, cyano, amino, hydroxy, carbonyl, $C_{1-6}$ alkyl, 3-6 membered cycloalkyl, and halogenated $C_{1-6}$ alkyl.

In any one of the above technical solutions, Y is selected from

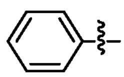

,

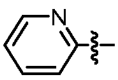, 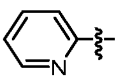, and 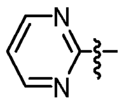.

In any one of the above technical solutions, when Y is substituted, the substituent on Y is selected from:

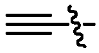, 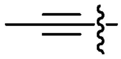, 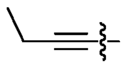, 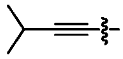, 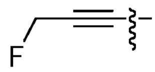,

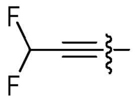, 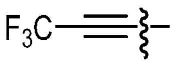, 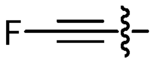, 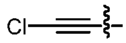, 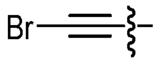, 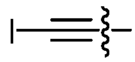, 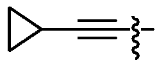,

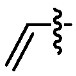, 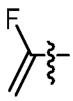, 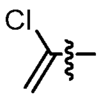, 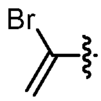, 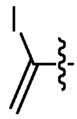, 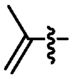, 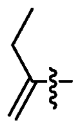, 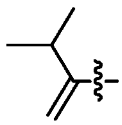, 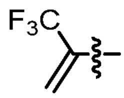, 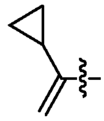,

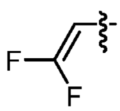, 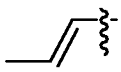, 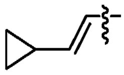, 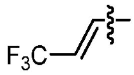, 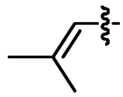, 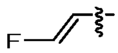, and 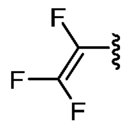.

In any one of the above technical solutions, deuterides formed by replacing hydrogen in the compound structure with deuterium are also included.

In any one of the above technical solutions, deuterides formed by replacing hydrogen on optional substituents of Y, W, or $R_3$ with deuterium are also included.

In one embodiment of the present disclosure, the compounds of formula (A') or the pharmaceutically acceptable salts, the stereoisomers or the deuterides thereof described above are shown in Table 1:

Table 1

| No. | Structure | No. | Structure |
|---|---|---|---|
| 17 | 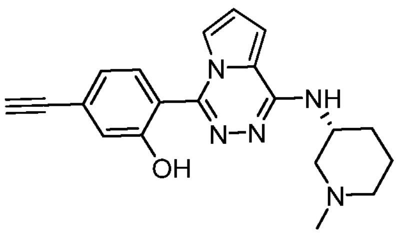 | 18 | 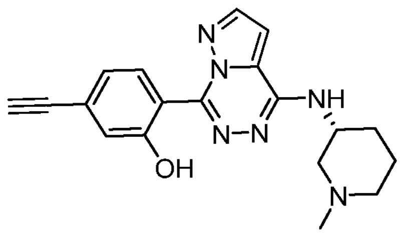 |
| 19 | 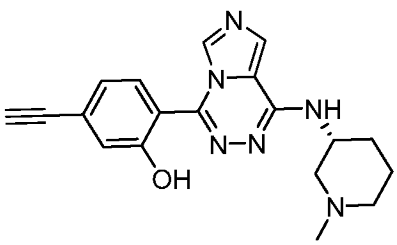 | 20 | 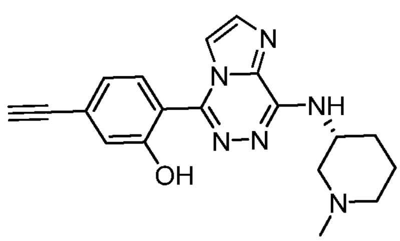 |

(continued)

| No. | Structure | No. | Structure |
|---|---|---|---|
| 21 | 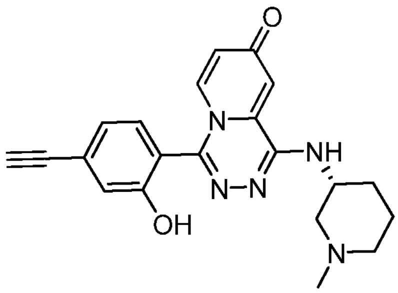 | 22 | 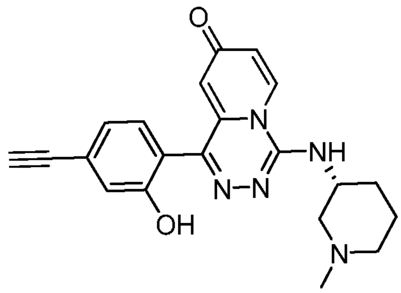 |
| 23 | 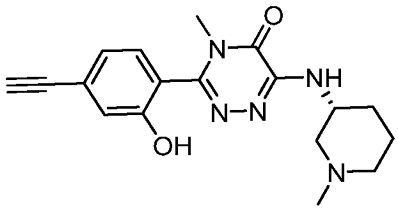 | 24 | 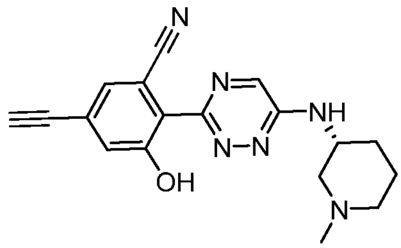 |
| 27 | 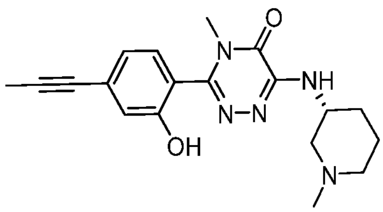 | 33 | 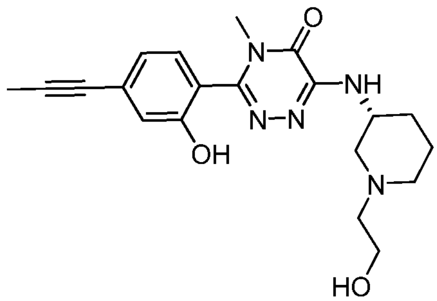 |
| 34 | 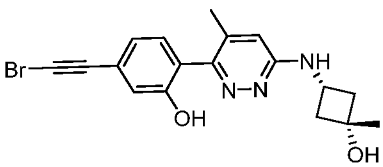 | 35 | 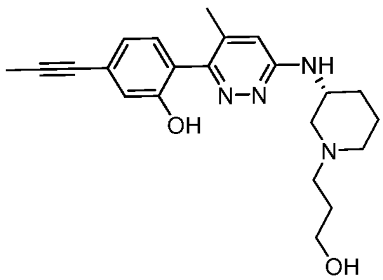 |
| 36 | 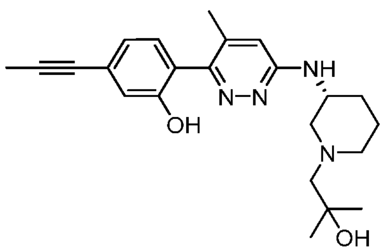 | 37 | 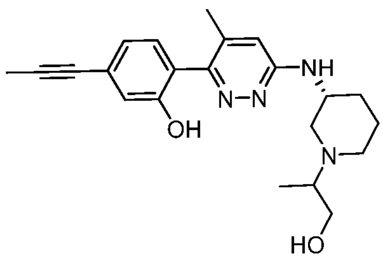 |

(continued)

| No. | Structure | No. | Structure |
|---|---|---|---|
| 38 | 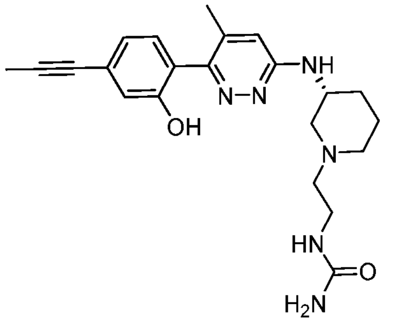 | 39 | 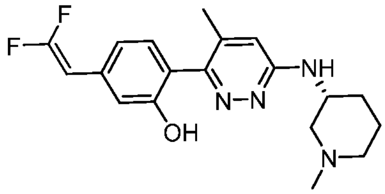 |
| 40 | 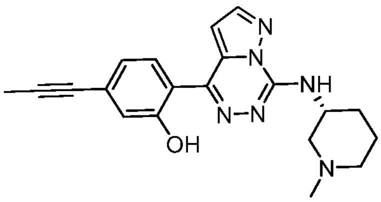 | 41 | 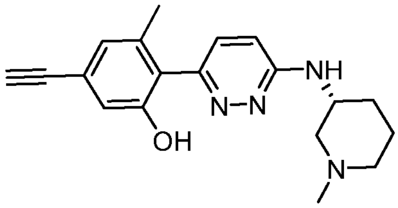 |
| 42 | 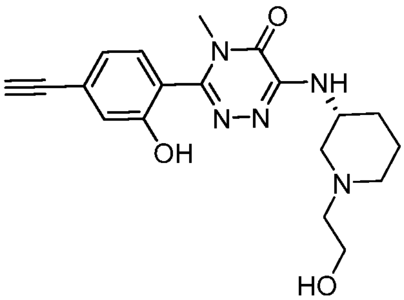 | 43 | 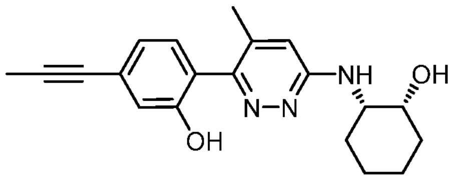 |
| 44 | 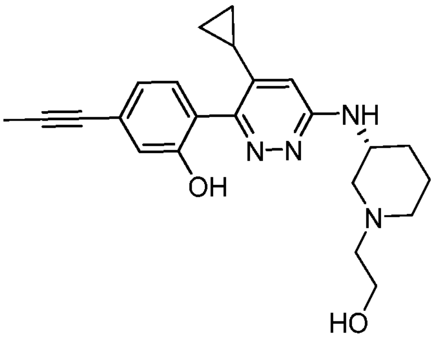 | 45 | 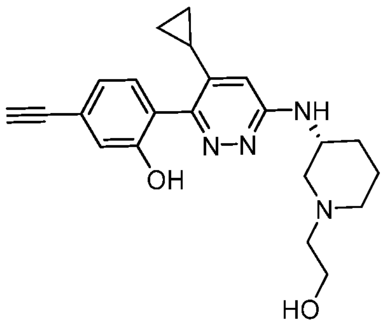 |
| 46 | 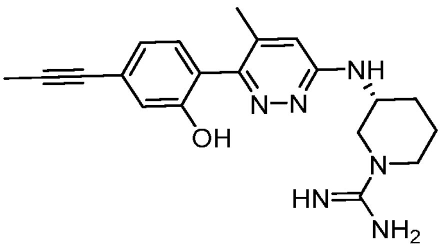 | 47 | 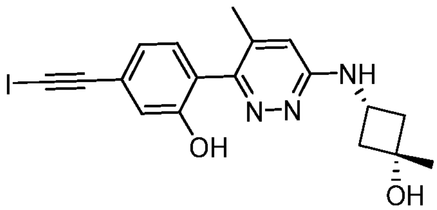 |

(continued)

| No. | Structure | No. | Structure |
|---|---|---|---|
| 48 | 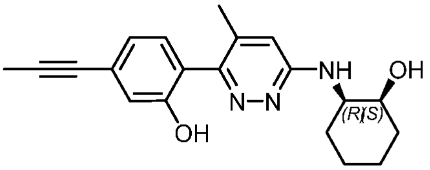 | 49 | 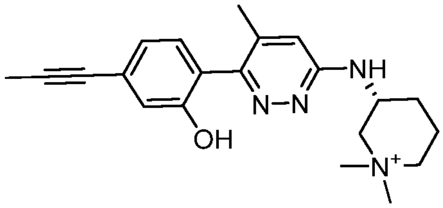 |
| 50 | 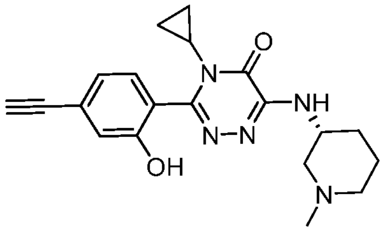 | 51 | 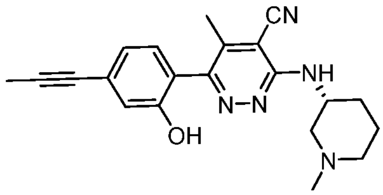 |
| 52 | 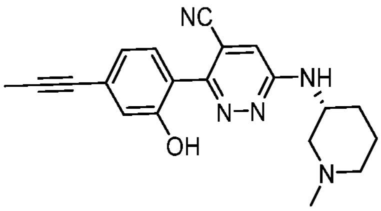 | 53 | 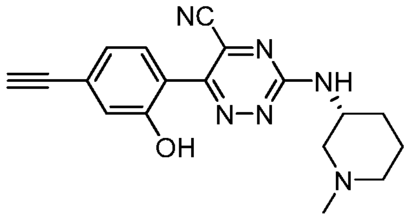 |
| 54 | 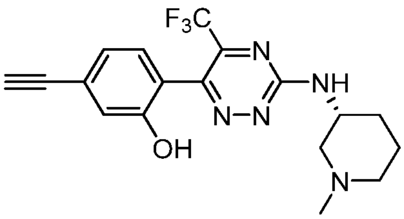 | 55 | 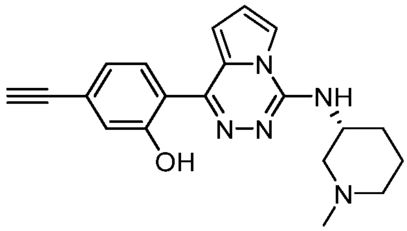 |
| 56 | 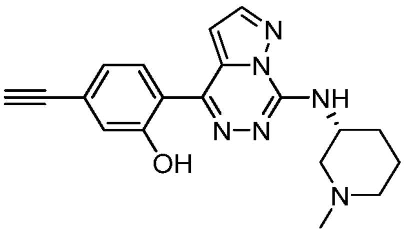 | 57 | 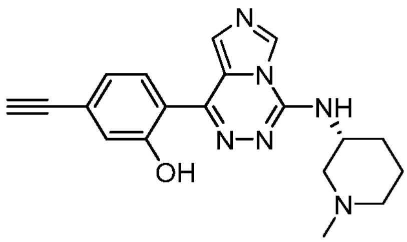 |
| 58 | 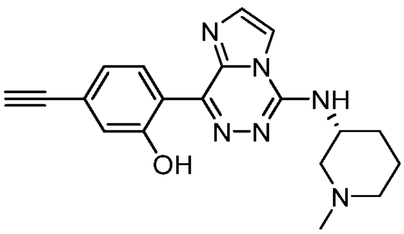 | 59 | 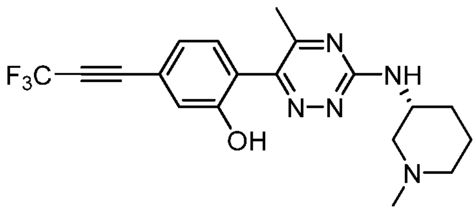 |

(continued)

| No. | Structure | No. | Structure |
|---|---|---|---|
| 60 | 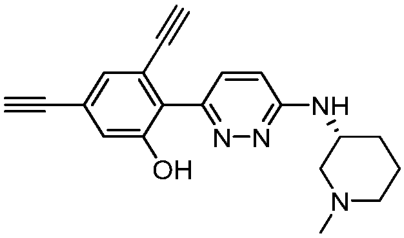 | 64 | 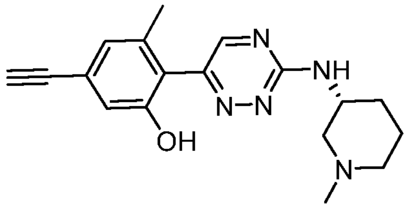 |
| 65 | 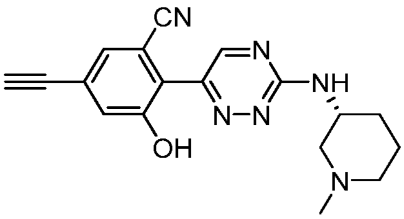 | 66 | 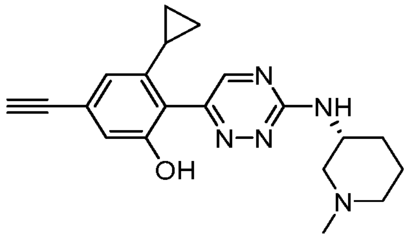 |
| 67 | 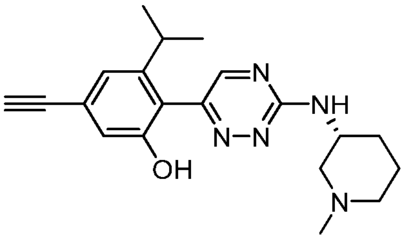 | 68 | 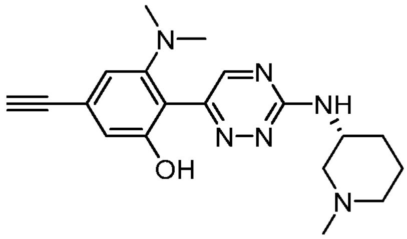 |
| 69 | 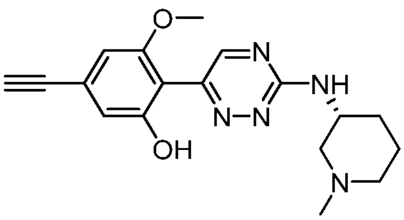 | 70 | 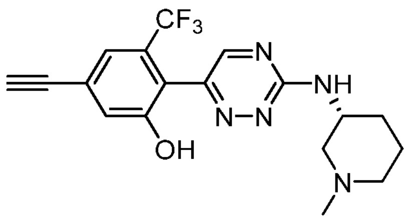 |
| 71 | 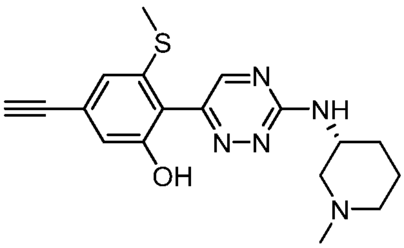 | 72 | 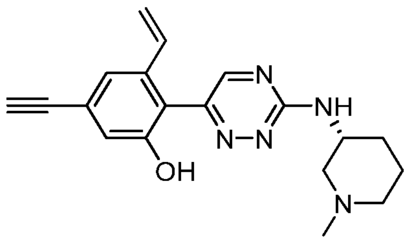 |
| 73 | 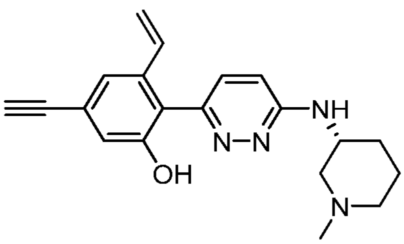 | 74 | 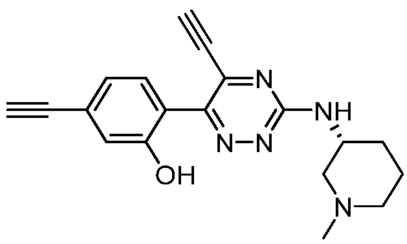 |

(continued)

| No. | Structure | No. | Structure |
|---|---|---|---|
| 75 | 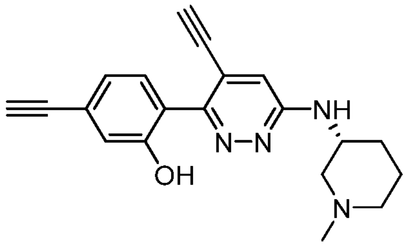 | 76 | 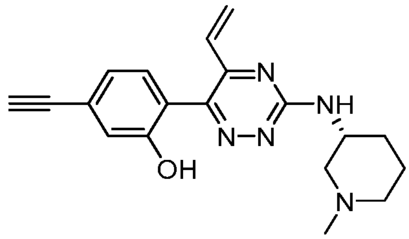 |
| 77 | 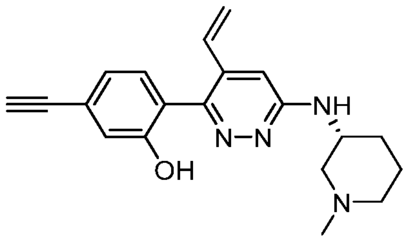 | 78 | 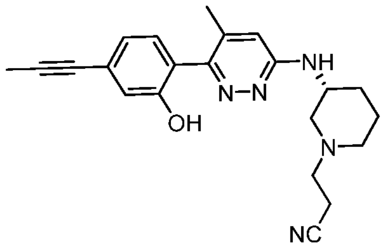 |
| 79 | 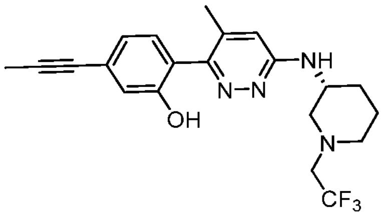 | 80 | 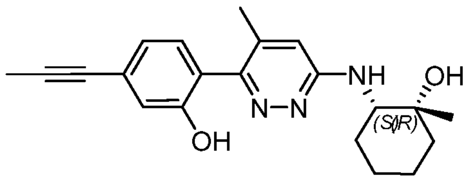 |
| 81 | 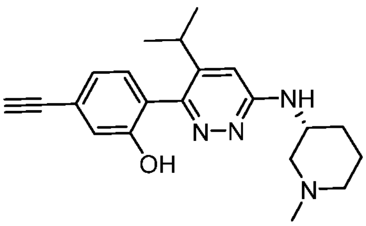 | 82 | 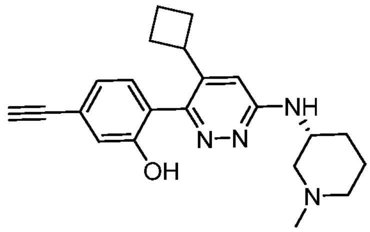 |
| 83 | 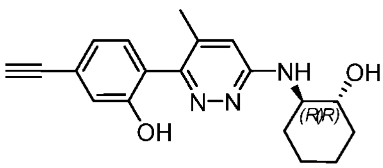 | 84 | 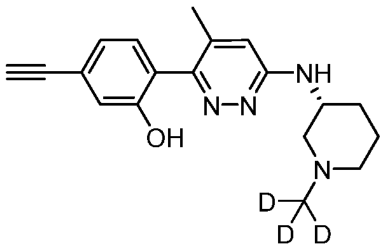 |
| 85 | 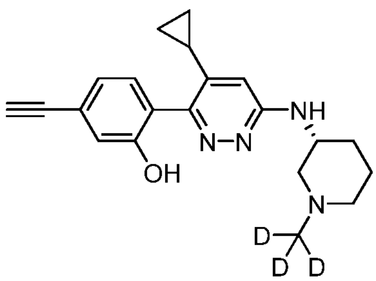 | 86 | 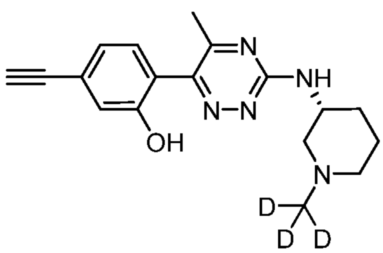 |

(continued)

| No. | Structure | No. | Structure |
|---|---|---|---|
| 87 | 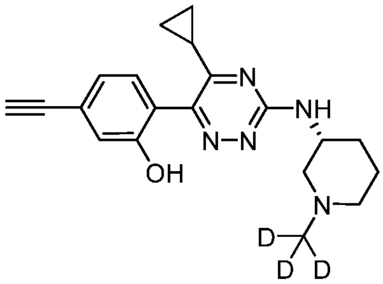 | 88 | 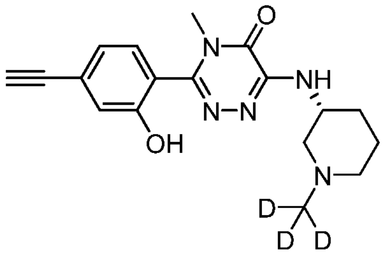 |
| 89 | 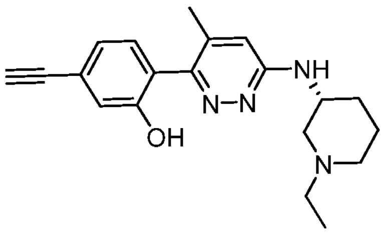 | 90 | 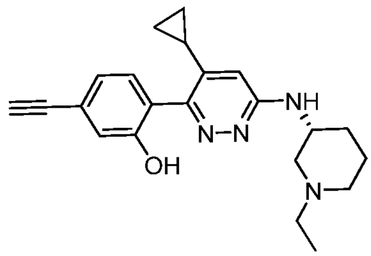 |
| 91 | 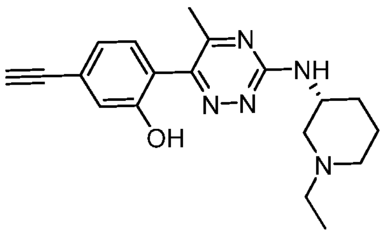 | 92 | 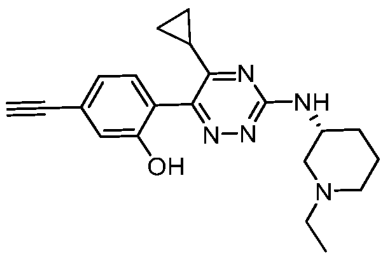 |
| 93 | 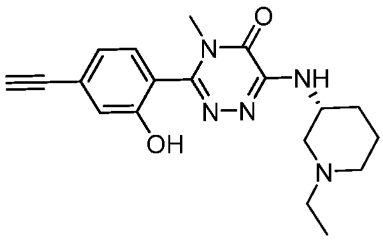 | 94 | 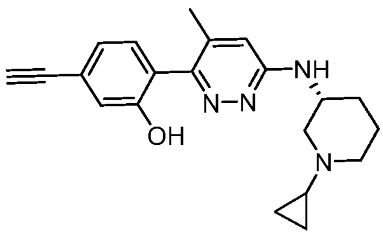 |
| 95 | 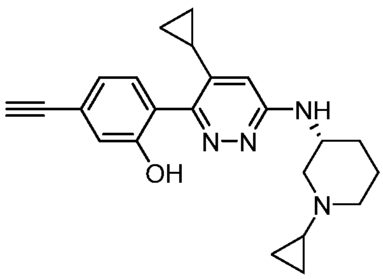 | 96 | 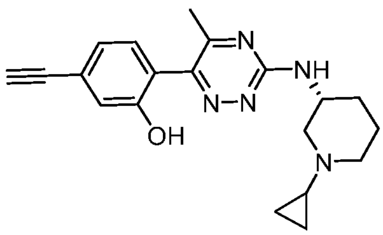 |
| 97 | 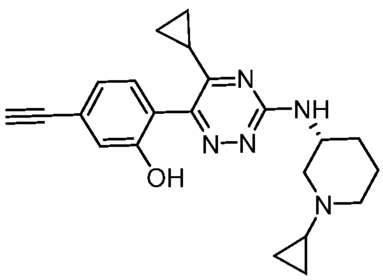 | 98 | 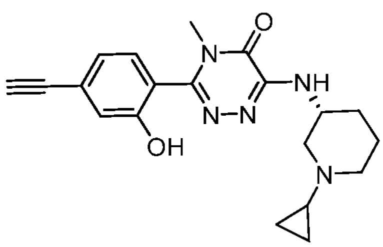 |

(continued)

| No. | Structure | No. | Structure |
|---|---|---|---|
| 99 | 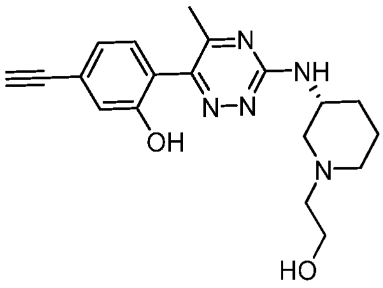 | 100 | 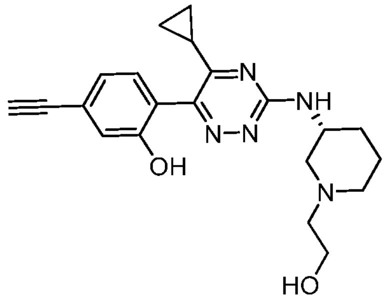 |
| 101 | 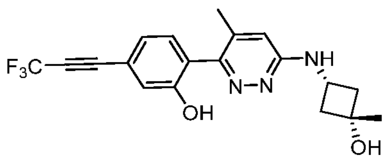 | 102 | 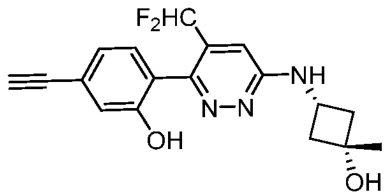 |
| 103 | 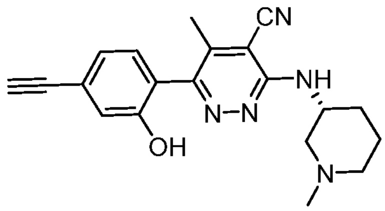 | 104 | 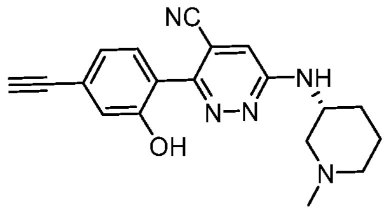 |
| 105 | 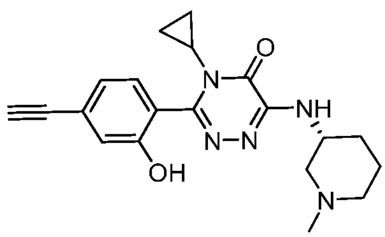 | 106 | 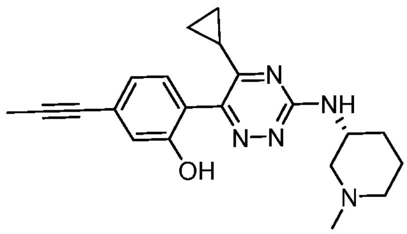 |
| 107 | 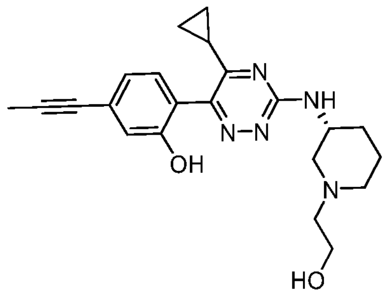 | 108 | 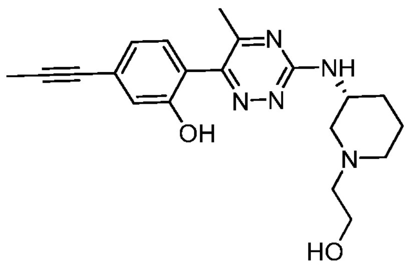 |
| 109 | 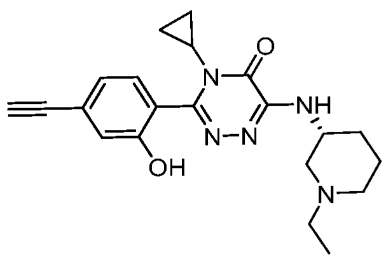 | 110 | 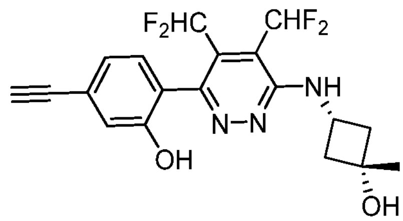 |

(continued)

| No. | Structure | No. | Structure |
|---|---|---|---|
| 111 | 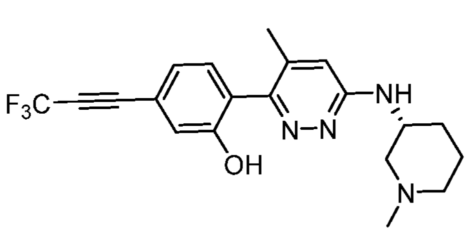 | 112 | 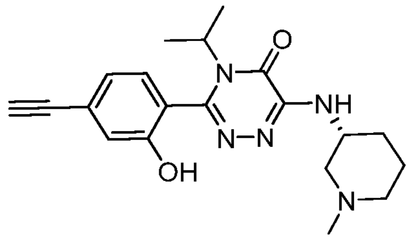 |
| 113 | 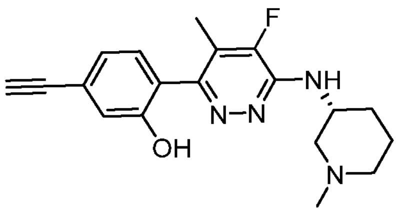 | 114 | 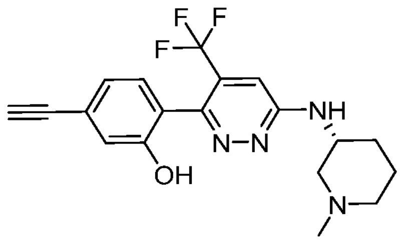 |
| 115 | 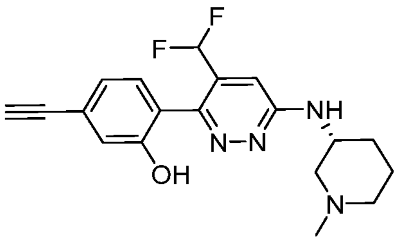 | 116 | 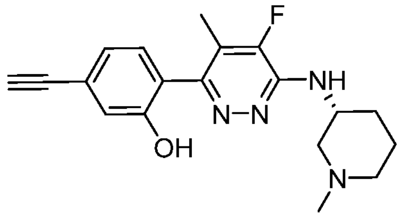 |
| 117 | 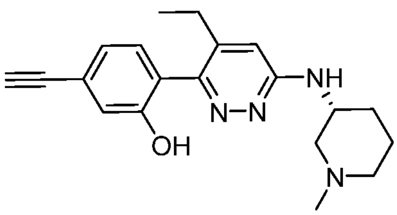 | 118 | 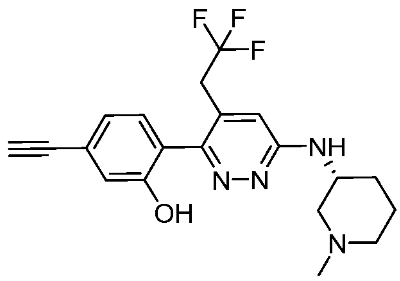 |
| 119 | 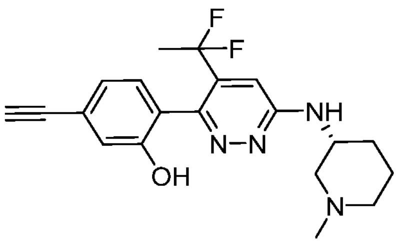 | 120 | 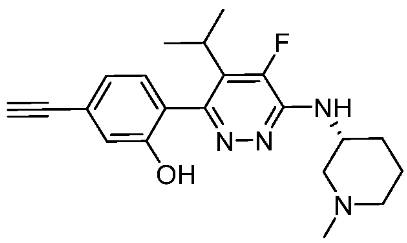 |
| 121 | 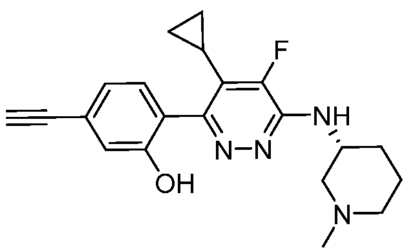 | 122 | 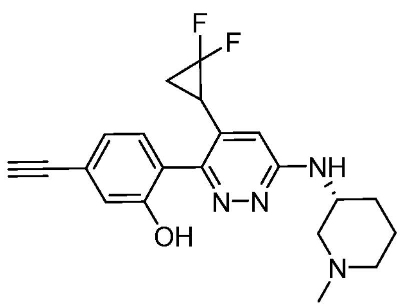 |

(continued)

| No. | Structure | No. | Structure |
|---|---|---|---|
| 123 | 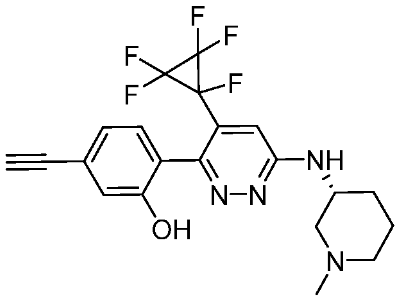 | 124 | 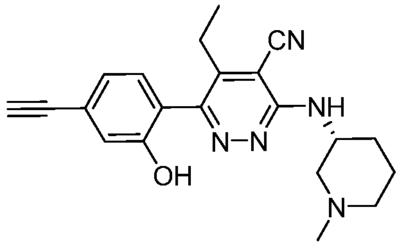 |
| 125 | 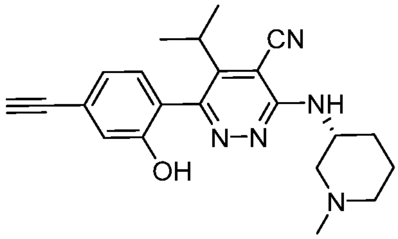 | 126 | 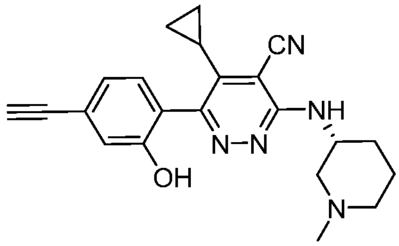 |
| 127 | 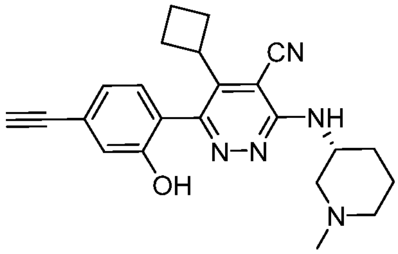 | 128 | 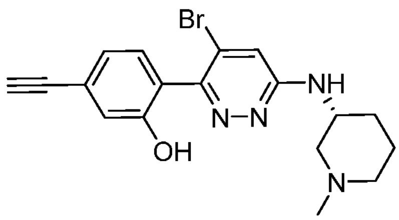 |
| 129 | 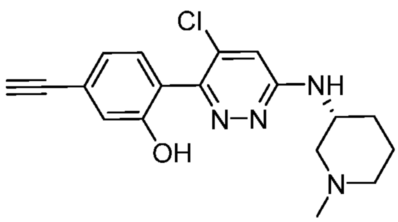 | 130 | 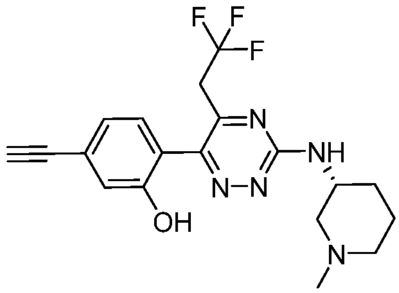 |
| 131 | 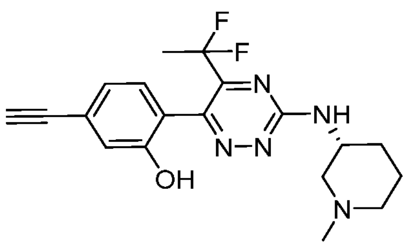 | 132 | 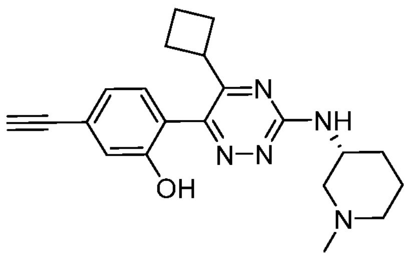 |

(continued)

| No. | Structure | No. | Structure |
|---|---|---|---|
| 133 | 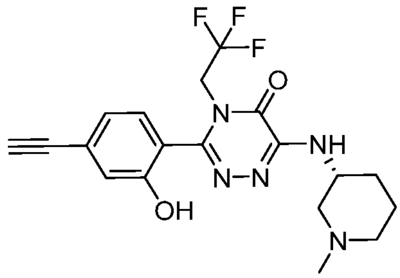 | 134 | 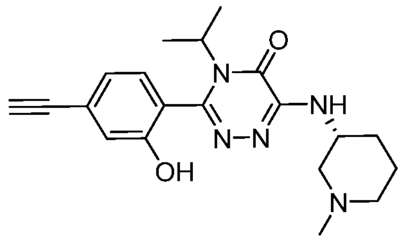 |
| 135 | 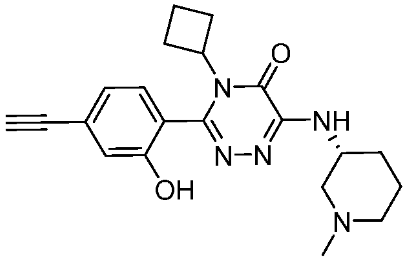 | 136 | 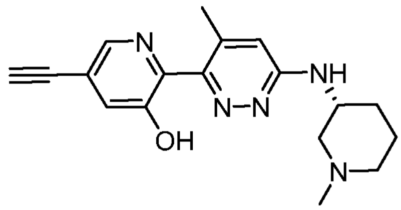 |
| 137 | 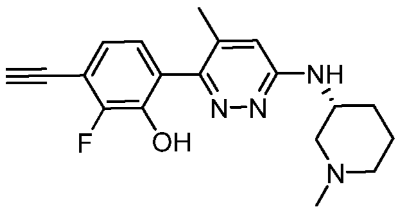 | 138 | 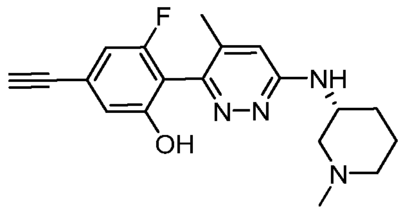 |
| 139 | 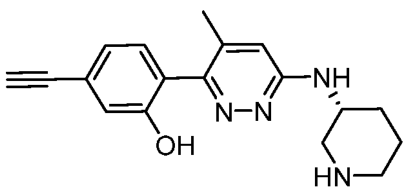 | 140 | 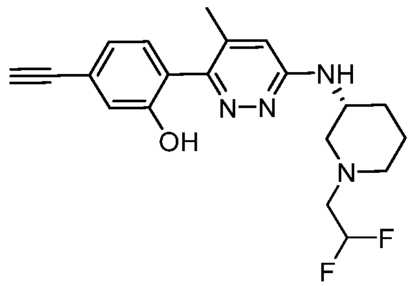 |
| 141 | 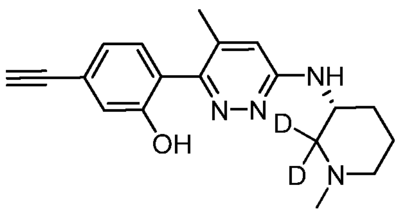 | 142 | 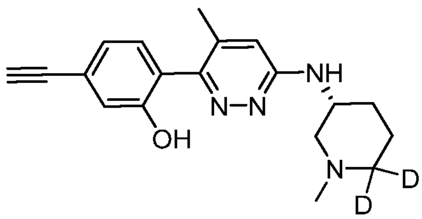 |
| 143 | 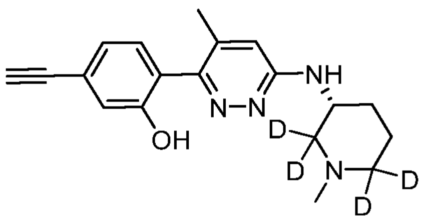 | 144 | 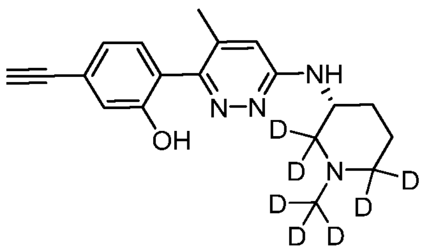 |

(continued)

| No. | Structure | No. | Structure |
|---|---|---|---|
| 145 | 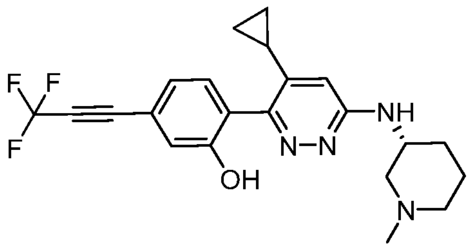 | 146 | 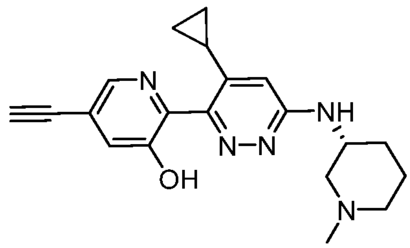 |
| 147 | 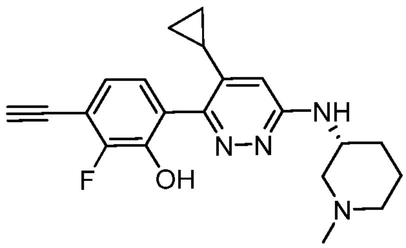 | 148 | 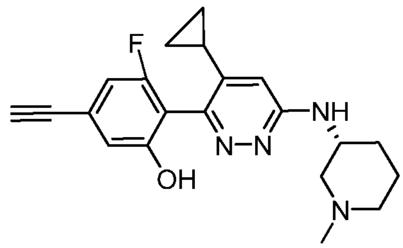 |
| 149 | 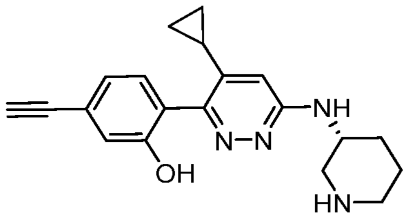 | 150 | 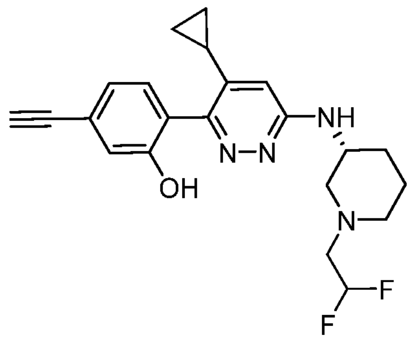 |
| 151 | 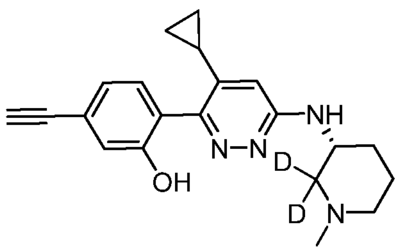 | 152 | 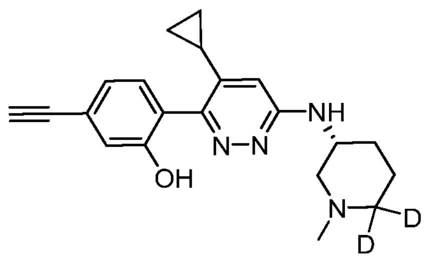 |
| 153 | 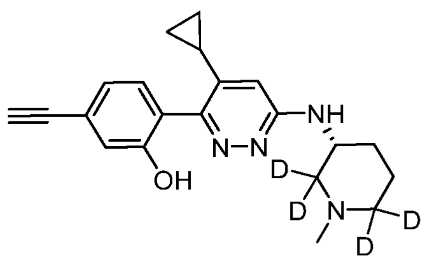 | 154 | 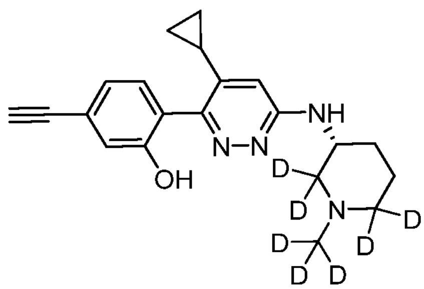 |
| 155 | 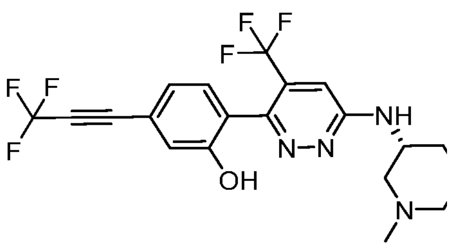 | 156 | 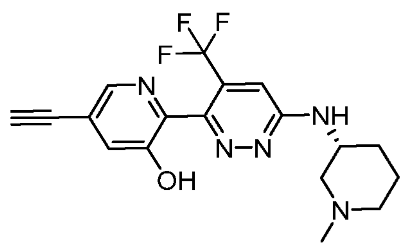 |

(continued)

| No. | Structure | No. | Structure |
|---|---|---|---|
| 157 | 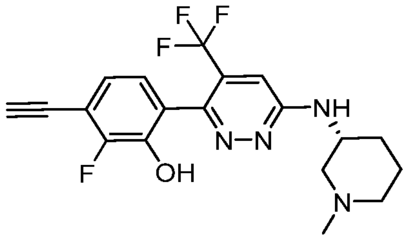 | 158 | 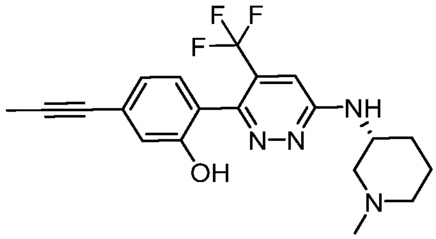 |
| 159 | 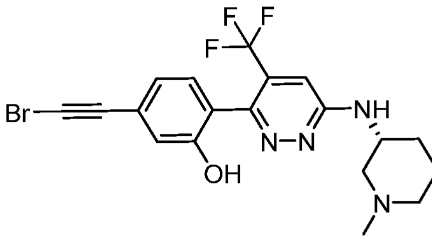 | 160 | 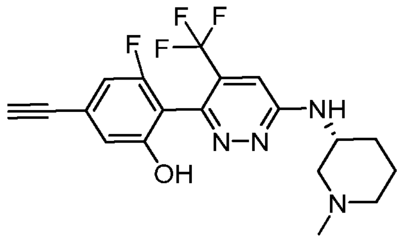 |
| 161 | 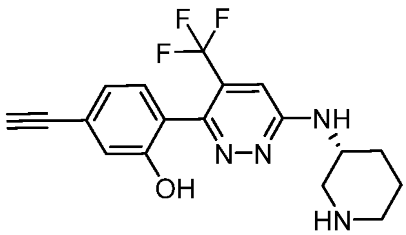 | 162 | 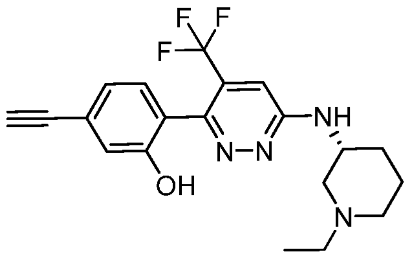 |
| 163 | 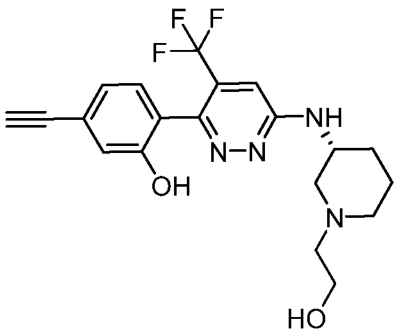 | 164 | 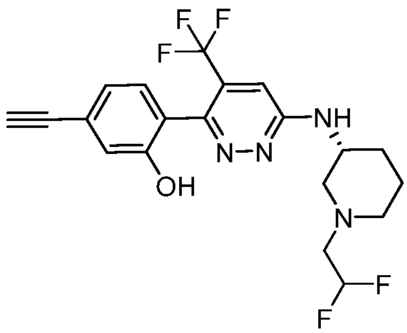 |
| 165 | 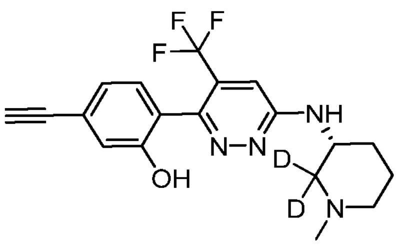 | 166 | 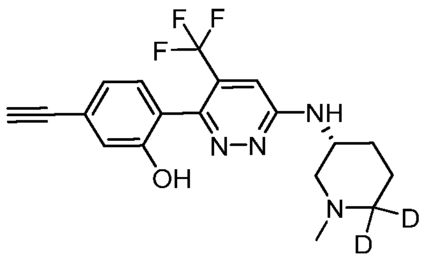 |
| 167 | 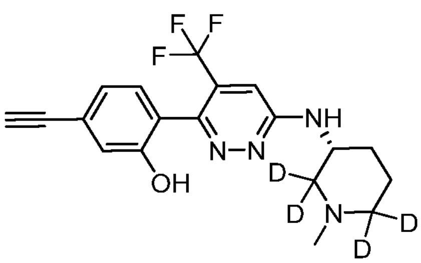 | 168 | 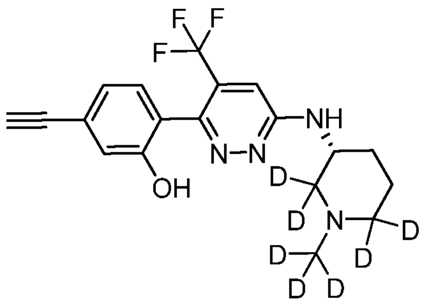 |

(continued)

| No. | Structure | No. | Structure |
|---|---|---|---|
| 169 | 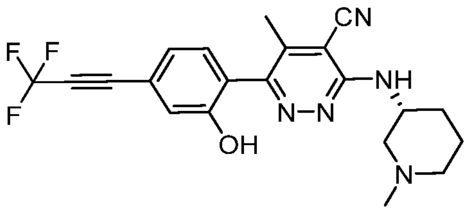 | 170 | 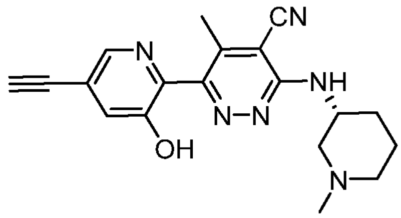 |
| 171 | 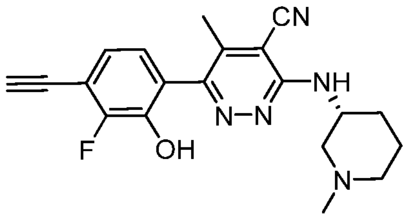 | 172 | 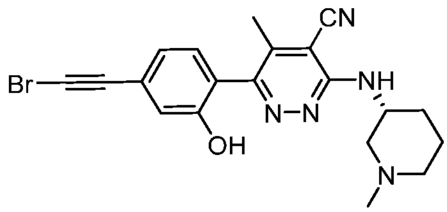 |
| 173 | 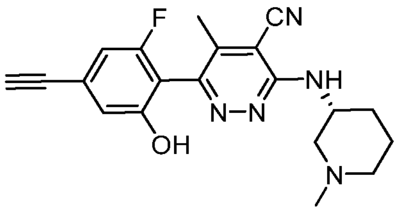 | 174 | 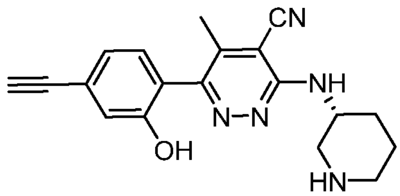 |
| 175 | 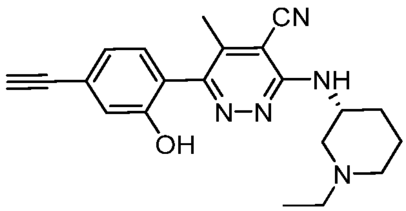 | 176 | 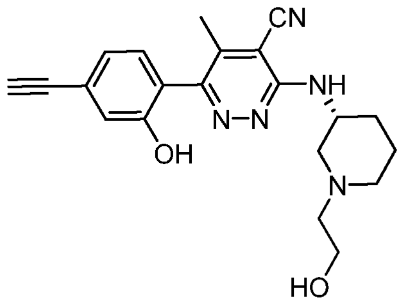 |
| 177 | 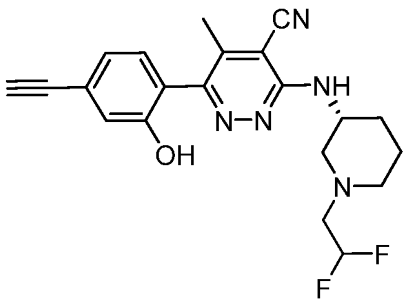 | 178 | 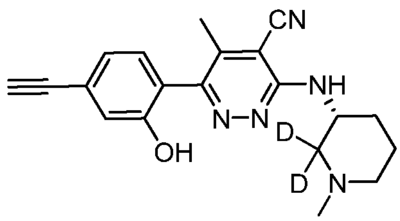 |
| 179 | 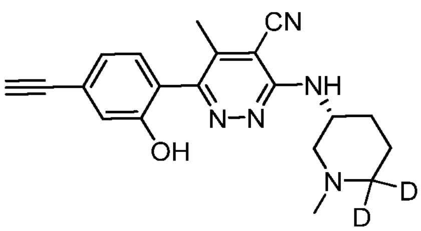 | 180 | 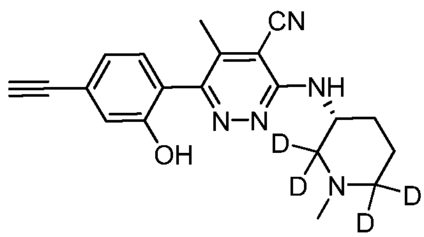 |

(continued)

| No. | Structure | No. | Structure |
|---|---|---|---|
| 181 | 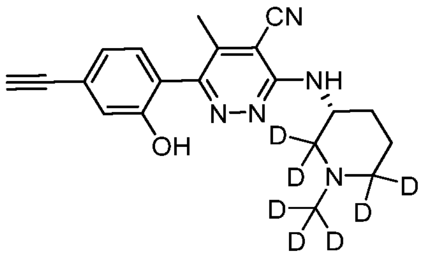 | 182 | 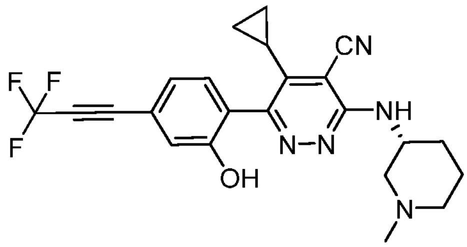 |
| 183 | 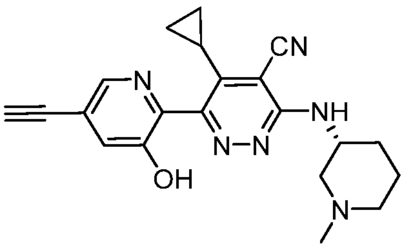 | 184 | 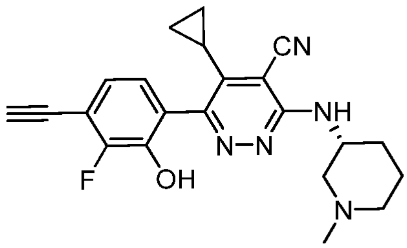 |
| 185 | 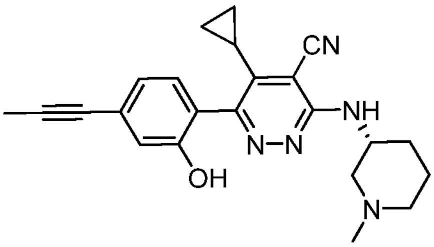 | 186 | 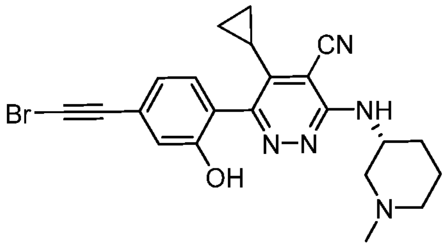 |
| 187 | 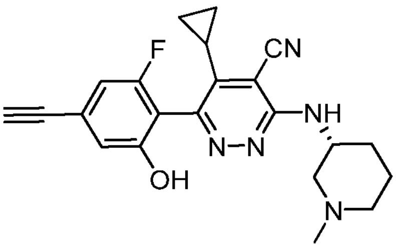 | 188 | 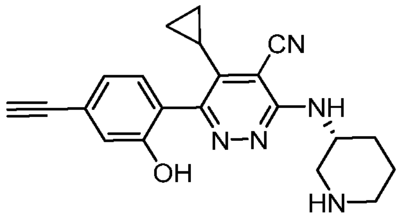 |
| 189 | 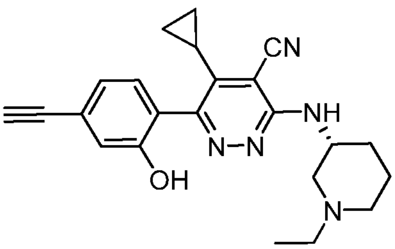 | 190 | 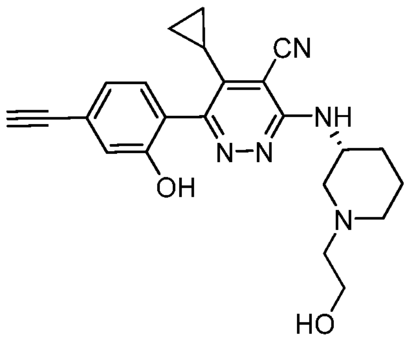 |

(continued)

| No. | Structure | No. | Structure |
|---|---|---|---|
| 191 | 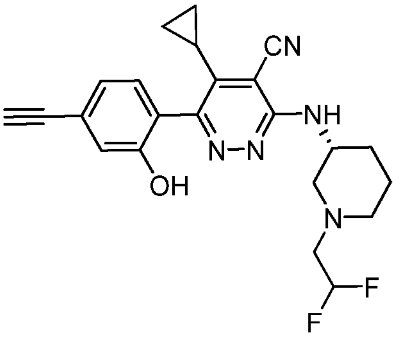 | 192 | 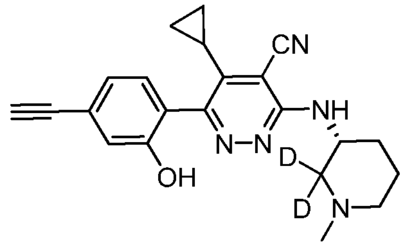 |
| 193 | 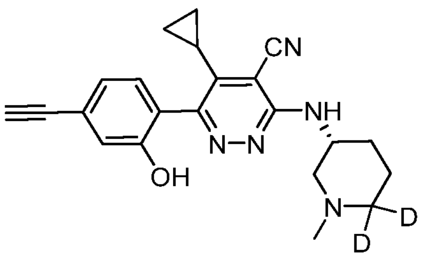 | 194 | 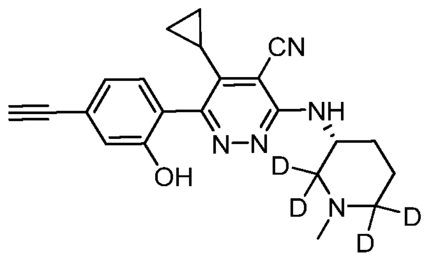 |
| 195 | 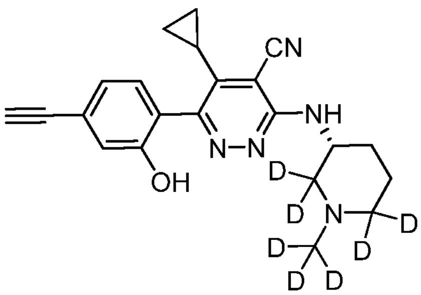 | 196 | 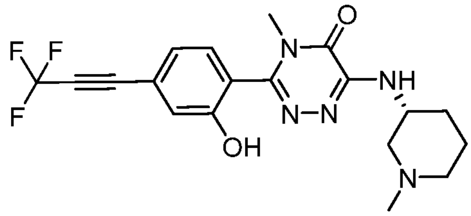 |
| 197 | 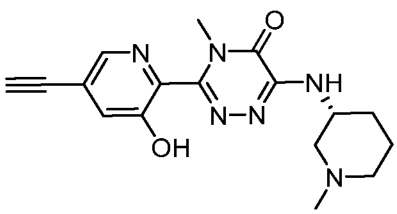 | 198 | 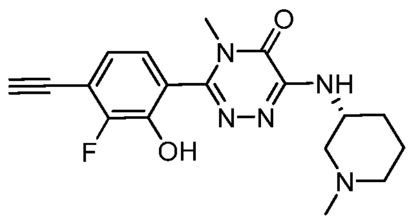 |
| 199 | 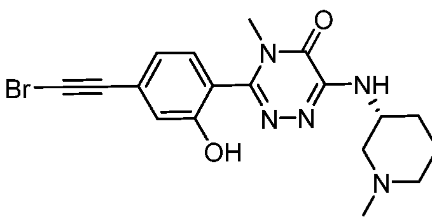 | 200 | 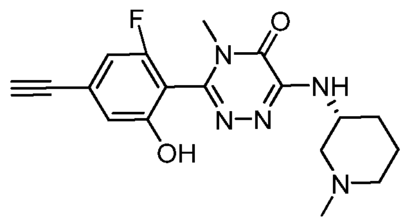 |
| 201 | 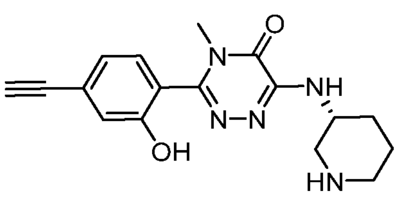 | 202 | 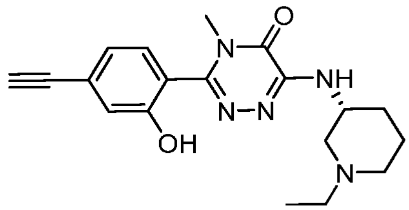 |

(continued)

| No. | Structure | No. | Structure |
|---|---|---|---|
| 203 | 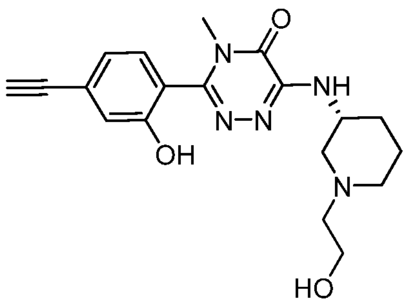 | 204 | 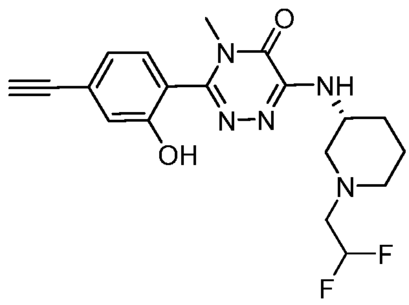 |
| 205 | 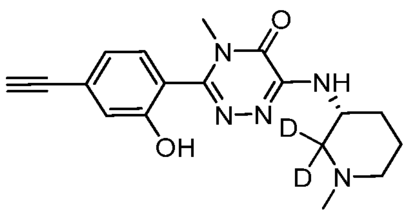 | 206 | 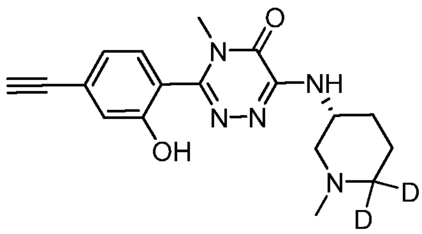 |
| 207 | 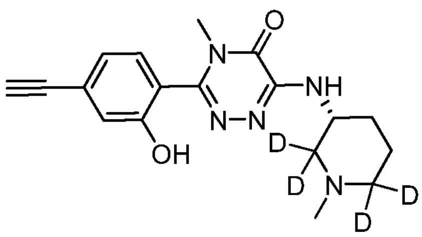 | 208 | 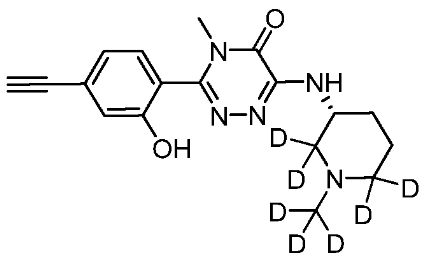 |
| 209 | 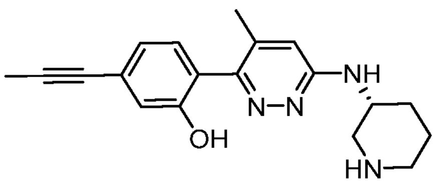 | 210 | 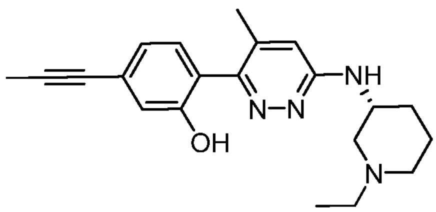 |
| 211 | 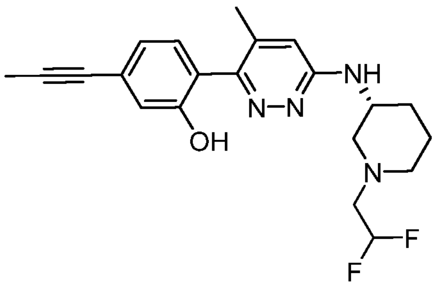 | 212 | 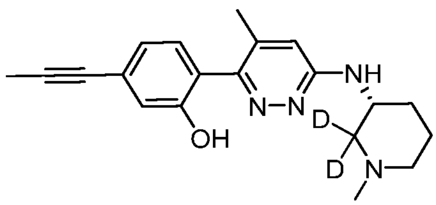 |
| 213 | 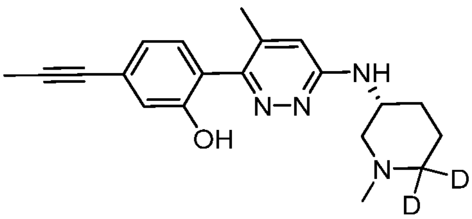 | 214 | 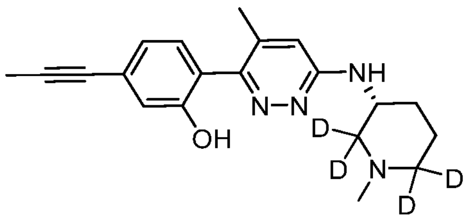 |

(continued)

| No. | Structure | No. | Structure |
|---|---|---|---|
| 215 | 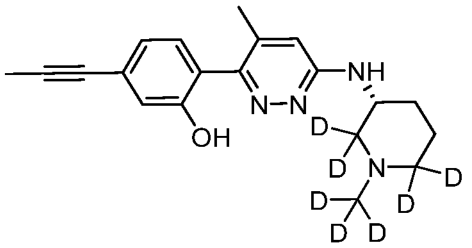 | 216 | 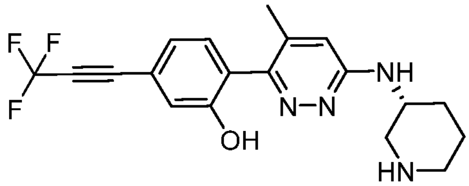 |
| 217 | 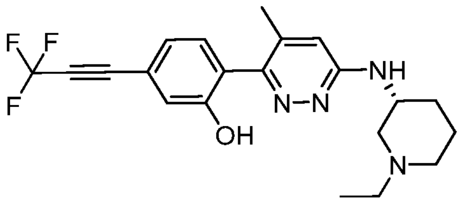 | 218 | 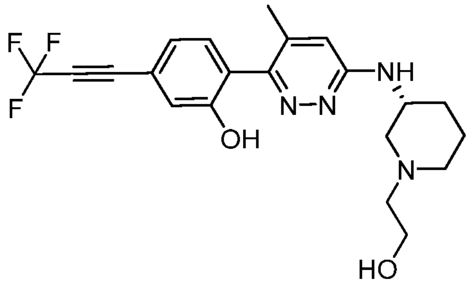 |
| 219 | 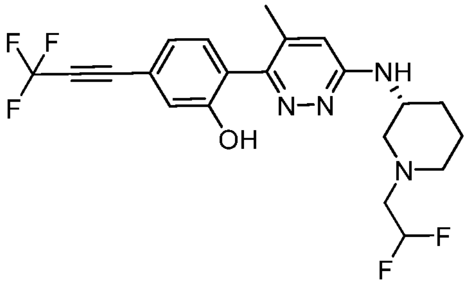 | 220 | 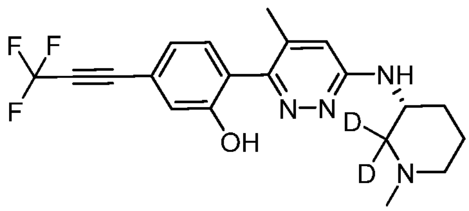 |
| 221 | 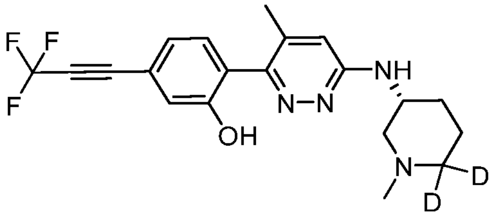 | 222 | 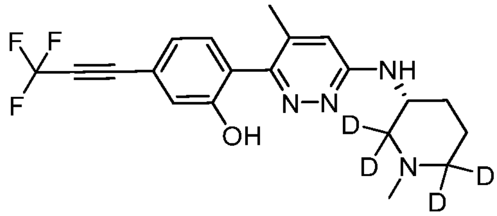 |
| 223 | 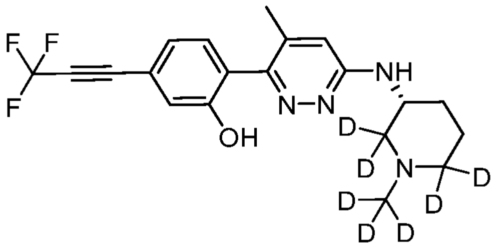 | 224 | 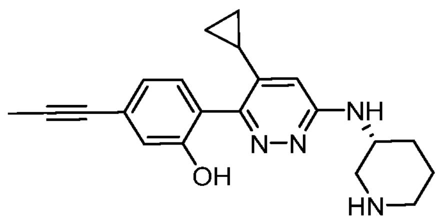 |
| 225 | 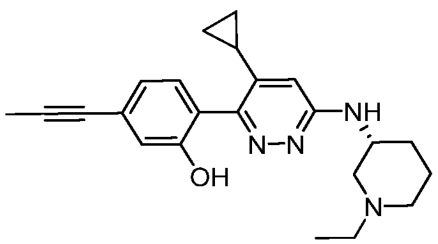 | 226 | 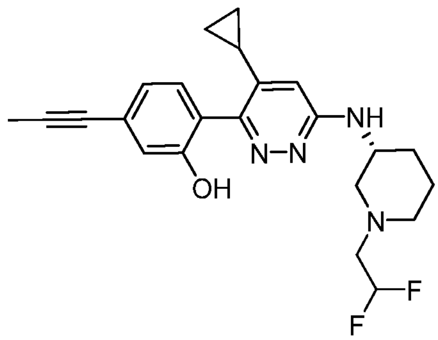 |

(continued)

| No. | Structure | No. | Structure |
|---|---|---|---|
| 227 | 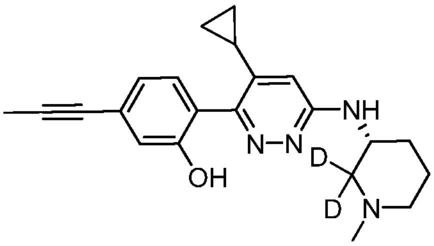 | 228 | 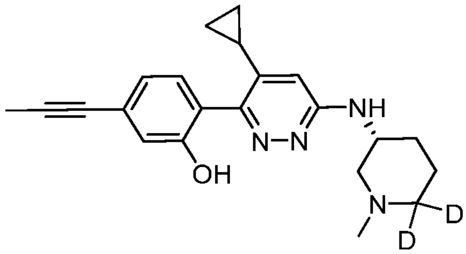 |
| 229 | 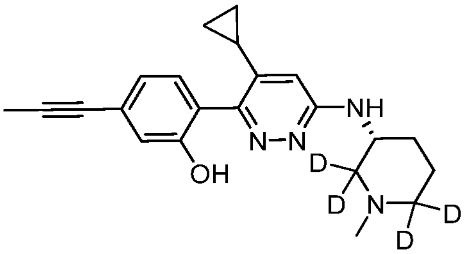 | 230 | 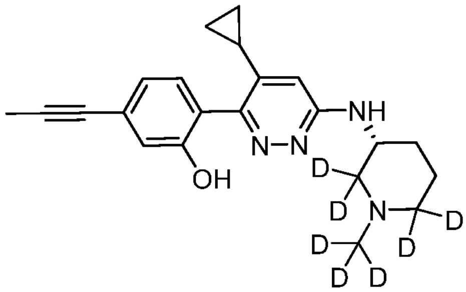 |
| 231 | 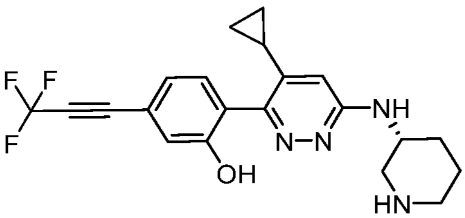 | 232 | 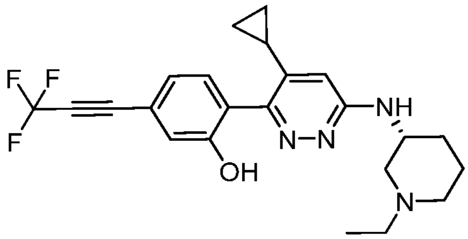 |
| 233 | 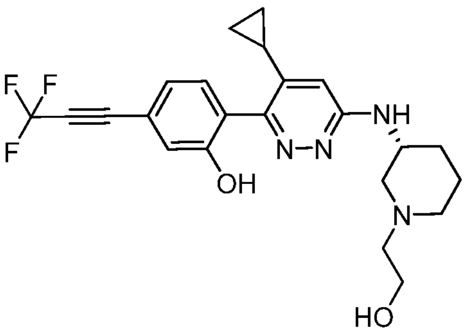 | 234 | 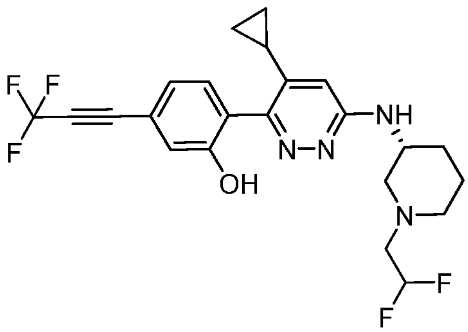 |
| 235 | 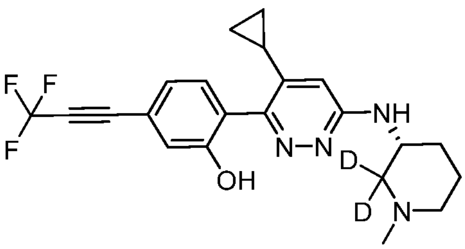 | 236 | 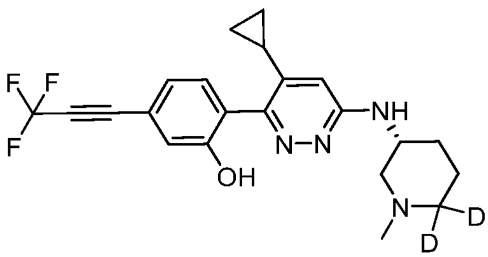 |

(continued)

| No. | Structure | No. | Structure |
|---|---|---|---|
| 237 | 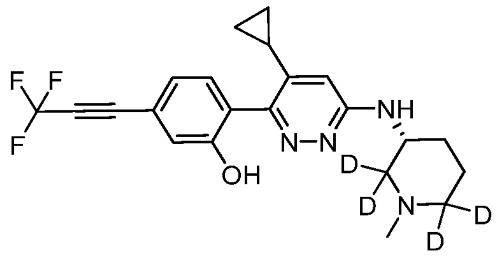 | 238 | 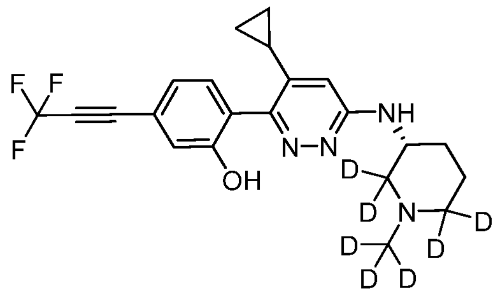 |
| 239 | 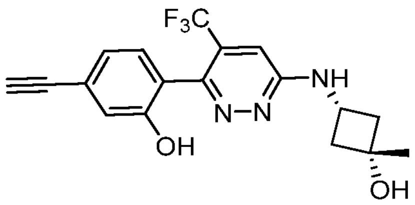 | 240 | 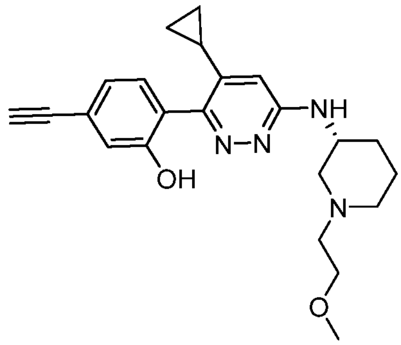 |

In one embodiment of the present disclosure, provided is a pharmaceutical composition, which comprises the compound or the pharmaceutically acceptable salt, the stereoisomer or the deuteride thereof according to any one of the above embodiments and a pharmaceutically acceptable carrier.

In one embodiment of the present disclosure, the pharmaceutical composition may comprise one or more pharmaceutically acceptable carriers and may be administered to a patient or subject in need of such treatment by routes such as oral administration, parenteral administration, rectal administration, or transpulmonary administration. For oral administration, the pharmaceutical composition may be prepared into a conventional solid formulation, such as a tablet, a capsule, a pill, and a granule, or may also be prepared into an oral liquid formulation, such as an oral solution, an oral suspension, and a syrup. In the preparation of an oral formulation, an appropriate filler, binder, disintegrant, lubricant, and the like may be added. For parenteral administration, the pharmaceutical composition may be prepared into an injection, including a solution injection, a sterile powder for injection, and a concentrated solution for injection. The injection may be produced by a conventional method existing in the pharmaceutical field, and during the preparation process, no additive may be added, or an appropriate additive may be added according to the properties of the medicament. For rectal administration, the pharmaceutical composition may be prepared into a suppository or the like. For transpulmonary administration, the pharmaceutical composition may be prepared into an inhalant, a spray, or the like.

The present disclosure further provides use of the compound represented by general formula (A') or the pharmaceutically acceptable salt, the stereoisomer or the deuteride thereof described above or the pharmaceutical composition described above in the manufacture of a medicament for preventing and/or treating NLRP3 inflammasome-associated diseases.

The present disclosure further provides use of the compound or the pharmaceutically acceptable salt, the stereoisomer or the deuteride thereof described above or the pharmaceutical composition described above in the manufacture of a medicament for preventing and/or treating inflammasome-associated diseases, immune diseases, inflammatory diseases, autoimmune diseases, or autoinflammatory diseases.

The "halogen" described herein refers to fluorine, chlorine, bromine, and iodine.

The "hydroxyl" described herein refers to the -OH group.

The "cyano" described herein refers to the -CN group.

The "amino" described herein refers to the $-NH_2$ group.

The "carboxyl" described herein refers to the -COOH group.

The "nitro" described herein refers to the $-NO_2$ group.

The "oxo" described herein refers to the =O group.

The "ureido" described herein refers to the $-HNCONH_2$ group.

The "hydrazino" described herein refers to the $-NHNH_2$ group.

The "deuterated $C_{1-6}$ alkyl" described herein refers to an alkyl group substituted with one or more deuterium atoms.

The "$C_{1-6}$ alkyl" described herein refers to linear or branched alkyl derived by removing one hydrogen atom from a hydrocarbon moiety containing 1 to 6 carbon atoms, such as methyl, ethyl, *n*-propyl, isopropyl, *n*-butyl, isobutyl, *sec*-butyl, *tert*-butyl, *n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1-methyl-2-methylpropyl.

The "ene" of "$C_{1-6}$ alkylene" described herein refers to a divalent group derived by removing two hydrogen atoms from a $C_{1-6}$ alkyl group.

The "halogenated $C_{1-6}$ alkyl" described herein refers to a $C_1$-$C_6$ alkyl group substituted with one or more halogen groups as defined above. Examples of halogenated $C_{1-6}$ alkyl include, but are not limited to, trifluoromethyl, difluoromethyl, fluoromethyl, trichloromethyl, 2,2,2-trifluoroethyl, 1,3-dibromopropan-2-yl, 3-bromo-2-fluoropropyl, and 1,4,4-trifluorobutan-2-yl.

The "$C_{1-6}$ alkoxy" described herein refers to a group in which the "$C_{1-6}$ alkyl" defined above is linked to a parent molecule via an oxygen atom, i.e., a "$C_{1-6}$ alkyl-O-" group, such as methoxy, ethoxy, *n*-propoxy, isopropoxy, *n*-butoxy, *tert*-butoxy, *n*-pentyloxy, neopentyloxy, and *n*-hexyloxy.

The "halogenated $C_{1-6}$ alkoxy" described herein refers to a $C_1$-$C_6$ alkoxy group substituted with one or more halogen groups as defined above, and the examples thereof include, but are not limited to, fluoromethoxy, chloromethoxy, trifluoromethoxy, trifluoroethoxy, fluoroethoxy, and fluoropropoxy.

The "$C_{2-6}$ alkenyl" described herein refers to linear or branched alkenyl derived by removing one hydrogen atom from an alkene moiety containing 2-6 carbon atoms and at least one carbon-carbon double bond, such as vinyl, 1-propenyl, 2-propenyl, 1-butenyl, 2-butenyl, 1,3-butadien-1-yl, 1-penten-3-yl, 2-penten-1-yl, 3-penten-1-yl, 3-penten-2-yl, 1,3-pentadien-1-yl, 1,4-pentadien-3-yl, 1-hexen-3-yl, and 1,4-hexadien-1-yl. Preferably, "$C_{2-6}$ alkenyl" contains one carbon-carbon double bond.

The "$C_{2-6}$ alkynyl" described herein refers to linear or branched alkynyl derived by removing one hydrogen atom from an alkyne moiety containing 2-6 carbon atoms and at least one carbon-carbon triple bond, such as ethynyl, propynyl, butynyl, pentynyl, and hexynyl. Preferably, "$C_{2-6}$ alkynyl" contains one carbon-carbon triple bond.

The "$C_{1-6}$ alkylamino", "$C_{1-6}$ alkylcarbonylamino", "$C_{1-6}$ alkylsulfonyl", and "aminocarbonyl" described herein refer to $C_{1-6}$ alkyl-NH- group, $C_{1-6}$ alkyl-C(O)-NH- group, $C_{1-6}$ alkyl-$S(O)_2$- group, and $NH_2$-C(O)- group, respectively.

It can be understood that, in the present disclosure, when $R_1$ and $R_2$ in

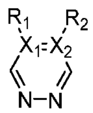

form a ring with the C or N atom to which they are attached, a double bond or a single bond is formed between $X_1$ and $X_2$ according to the chemical bonding rules.

The "5-12 membered ring" described herein includes carbocyclic rings or heterocyclic rings which may be chemically formed, such as 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl.

The "3-12 membered cycloalkyl" described herein refers to a monovalent group or (as required) divalent group (e.g, 5-12 membered cycloalkyl) derived from 3-12 membered cycloalkane, which may be a monocyclic, bicyclic, or polycyclic cycloalkyl system. Unless otherwise specified, all possibly formed monocyclic or fused cycloalkyl groups (such as 6-12 membered fused cycloalkyl) are included, including those fused in the form of ortho-, spiro-, and bridged. A monocyclic system is typically a cyclic hydrocarbon group containing 3-12 carbon atoms (such as 3-8 or 3-6 carbon atoms). Examples of cycloalkyl include, but are not limited to: cyclopropyl, cyclobutyl, cyclopentyl, cyclohexyl, cycloheptyl, cyclooctyl, cyclopentyl-1,3-diyl, cyclohexyl-1,4-diyl, and cycloheptyl-1,4-diyl. Fused cycloalkyl includes ortho-fused cycloalkyl, bridged cycloalkyl, and spiro-cycloalkyl. Ortho-fused cycloalkyl may be 6-11 membered ortho-fused cycloalkyl (such as 7-10 membered ortho-fused cycloalkyl), and the representative examples include, but are not limited to, bicyclo [3.1.1]heptane, bicyclo[2.2.1]heptane, bicyclo[2.2.2]octane, bicyclo[3.2.2]nonane, bicyclo[3.3.1]nonane, and bicyclo [4.2.1]nonyl. Spiro-cycloalkyl may be 7-12 membered spiro-cycloalkyl (such as 7-11 membered spiro-cycloalkyl), and the examples thereof include, but are not limited to:

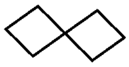, 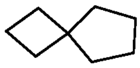, 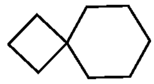,

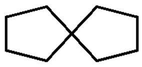, 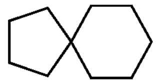, and 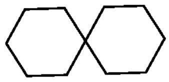

groups. Bridged cycloalkyl may be 6-10 membered bridged cycloalkyl (such as 7-10 membered bridged cycloalkyl), and the examples thereof include, but are not limited to:

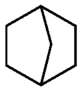, 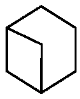, 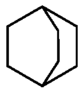, 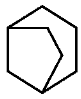, 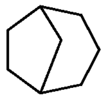,

and

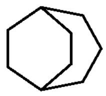

groups.

The "3-7 membered cycloalkyl" described herein refers to a monovalent group or (as required) divalent group derived from 3-7 membered cycloalkane. The "3-7 membered cycloalkyl" may be 3, 4, 5, 6, or 7 membered cycloalkyl, and examples of the 3-7 membered cycloalkyl include cyclopropyl, cyclobutyl, cyclopentyl, and cyclohexyl.

The "cycloalkenyl" described herein refers to a group obtained by forming at least one double bond in the above cycloalkyl. It may be, for example, "3-12 membered cycloalkenyl", i.e., may have 3, 4, 5, 6, 7, 8, 9, 10, 11, or 12 ring-forming carbon atoms. Unless otherwise specified, a certain membered cycloalkenyl encompasses all possibly formed monocyclic or fused cycloalkenyl groups (including those fused in the form of ortho-, spiro-, and bridged). Cycloalkenyl may be 3-12 membered cycloalkenyl, 3-8 membered cycloalkenyl, 4-6 membered cycloalkenyl, 7-11 membered spiro-cycloalkenyl, 7-11 membered ortho-fused cycloalkenyl, 6-11 membered bridged cycloalkenyl, etc. Examples of cycloalkenyl include, but are not limited to, cyclobutenyl, cyclopentenyl, cyclopentadienyl, cyclohexenyl, 1,4-cyclohexadien-1-yl, cycloheptenyl, 1,4-cycloheptadien-1-yl, cyclooctenyl, and 1,5-cyclooctadien-1-yl.

The "5-7 membered cycloalkenyl" described herein refers to a group obtained by forming at least one double bond in a 5-7 membered cycloalkyl group, such as cyclobutenyl, cyclopentenyl, cyclohexenyl, and cycloheptenyl.

The "3-14 membered heterocyclyl" described herein refers to a monovalent group or (as required) divalent group derived from 3-14 membered heterocycloalkane, i.e., a non-aromatic cyclic group obtained by substituting at least one ring carbon atom of 3-14 membered heterocycloalkane with a heteroatom selected from O, S, S(O), $S(O)_2$, C(O), and N, which preferably contains 1-3 heteroatoms. The "3-14 membered heterocyclyl" (e.g., 5-14 membered heterocyclyl or 5-12 membered heterocyclyl) includes monocyclic heterocyclyl, bicyclic heterocyclyl systems, or polycyclic heterocyclyl systems in which one or more rings may be saturated or partially saturated, but excluding aromatic rings. Unless otherwise specified, all possibly formed monocyclic, fused (including fused in the form of ortho-, spiro-, and bridged), saturated, and partially saturated cases are included.

Monocyclic heterocyclyl may be 3-8 membered heterocyclyl (such as 5-7 membered heterocyclyl, 3-7 membered heterocyclyl, 4-7 membered heterocyclyl, or 5-6 membered heterocyclyl), 3-8 membered nitrogen-containing heterocyclyl (such as 4-7 membered nitrogen-containing heterocyclyl, or 5-6 membered nitrogen-containing heterocyclyl), or 3-8 membered saturated heterocyclyl (such as 5-6 membered saturated heterocyclyl), and the examples thereof include, but are not limited to, aziridinyl, oxiranyl, thiiranyl, azetidinyl, oxetanyl, thietanyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro-1*H*-pyrazolyl, 4,5-dihydro-3*H*-pyrazolyl, 4,5-dihydrothiazolyl, 2,5-dihydrothiazolyl, 2*H*-pyranyl, 4*H*-pyranyl, 2*H*-thiopyranyl, 4*H*-thiopyranyl, 2,3,4,5-tetrahydropyridyl, 1,2-isoxazinyl, 1,4-isoxazinyl, 6*H*-1,3-oxazinyl, or the like.

Fused heterocyclyl (e.g., 6-12 membered fused heterocyclyl) includes ortho-fused heterocyclyl, spiro-heterocyclyl, and bridged heterocyclyl, which may be saturated, partially saturated or unsaturated, but non-aromatic. Fused heterocyclyl may be a 5-6 membered monocyclic heterocyclyl ring which is fused to a phenyl, 5-6 membered monocyclic

cycloalkyl, 5-6 membered monocyclic cycloalkenyl, 5-6 membered monocyclic heterocyclyl, or 5-6 membered monocyclic heteroaryl.

The ortho-fused heterocyclyl may be 6-12 membered ortho-fused heterocyclyl (such as 6-11 membered ortho-fused heterocyclyl or 7-10 membered ortho-fused heterocyclyl), 6-11 membered saturated ortho-fused heterocyclyl, or 6-11 membered nitrogen-containing ortho-fused heterocyclyl, and the representative examples thereof include, but are not limited to: 3-azabicyclo[3.1.0]hexyl, 3,6-diazabicyclo[3.2.0]heptyl, 3,8-diazabicyclo[4.2.0]octyl, 3,7-diazabicyclo [4.2.0]octyl, octahydropyrrolo[3,4-c]pyrrolyl, octahydropyrrolo[3,4-*b*]pyrrolyl, octahydropyrrolo[3,4-*b*][1,4]oxazinyl, octahydro-1*H*-pyrrolo[3,4-c]pyridyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1*H*-indolyl, and octahydrobenzofuranyl.

The spiro-heterocyclyl may be 6-12 membered spiro-heterocyclyl (such as 7-12 membered spiro-heterocyclyl), 7-12 membered saturated spiro-heterocyclyl, or 7-12 membered nitrogen-containing spiro-heterocyclyl, and the examples thereof include, but are not limited to:

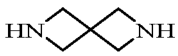, 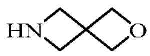, 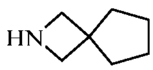, 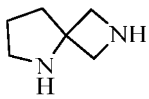, 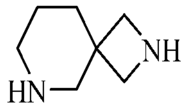,

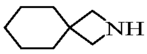, 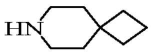, 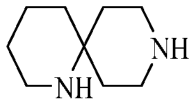, 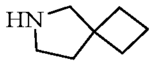, 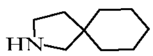 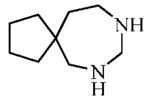,

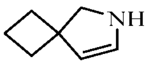, 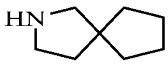, and 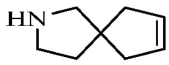.

The bridged heterocyclyl may be 6-12 membered bridged heterocyclyl (such as 6-10 membered bridged heterocyclyl (e.g., 6-10 membered nitrogen-containing bridged heterocyclyl, particularly 7-membered nitrogen-containing bridged heterocyclyl), or 7-10 membered bridged heterocyclyl), and the examples thereof include, but are not limited to:

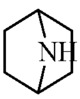, 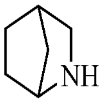, 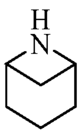, 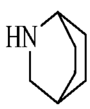, 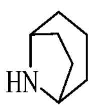, 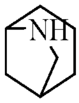, 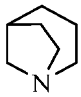, 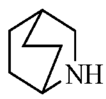, 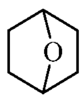, 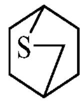,

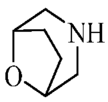, and 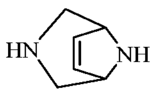.

The "aryl" described herein refers to a monovalent or (as required) divalent cyclic aromatic group containing 6-14 carbon atoms derived from aromatic carbocyclic hydrocarbon, including phenyl, naphthyl, phenanthryl, etc.

The "5-14 membered heteroaryl" described herein refers to an aromatic 5-14 membered cyclic group in which at least one ring carbon atom is substituted with a heteroatom selected from O, S, and N, and the "5-14 membered heteroaryl" may be 5, 6, 7, 8, 9, 10, 11, 12, 13, or 14 membered heteroaryl and preferably contains 1-3 heteroatoms, with the case that carbon atoms or sulfur atoms are oxidized or nitridized (e.g., carbon atoms are substituted with C(O) and sulfur atoms are substituted with S(O) or S(O)2) being included. Heteroaryl includes monocyclic heteroaryl and fused heteroaryl. Unless otherwise specified, a certain membered heteroaryl includes all possibly formed monocyclic, fused, fully aromatic, and partially aromatic cases. Monocyclic heteroaryl may be 5-7 membered heteroaryl (such as 5-6 membered heteroaryl), and the examples thereof include, but are not limited to, furanyl, imidazolyl, isoxazolyl, thiazolyl, isothiazolyl, oxadiazolyl, oxazolyl, pyridyl, pyridazinyl, pyrimidinyl, pyrazinyl, pyrazolyl, pyrrolyl, tetrazolyl, thiadiazolyl, thienyl, triazolyl, and triazinyl.

In certain technical solutions, fused heteroaryl refers to a group formed by fusing a monocyclic heteroaromatic ring to phenyl, cycloalkenyl, heteroaryl, cycloalkyl, or heterocyclyl. In certain technical solutions, fused heteroaryl (such as 8-14 membered fused heteroaryl) may be 8-14 membered ortho-fused heteroaryl (such as 9-10 membered ortho-fused heteroaryl), and examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridyl, 4,5,6,7-tetrahydro[c][1,2,5]oxadiazolyl, and 6,7-dihydro[*c*][1,2,5]oxadiazol-4(5*H*)keto.

The "pharmaceutically acceptable salt" described herein refers to a pharmaceutically acceptable addition salt of acid and base and a solvate. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid or $HOOC-(CH_2)n-COOH$ (wherein n is 0-4)), etc. Such pharmaceutically acceptable salts further include salts of the following bases: sodium, potassium, calcium, ammonium, etc. Those skilled in the art know a variety of pharmaceutically acceptable non-toxic addition salts.

All numerical ranges described herein include both endpoints of the ranges, all integers within the range, and subranges formed by these integers. For example, "3-7 membered" includes 3, 4, 5, 6, and 7 membered; "1-4" includes 1, 2, 3, and 4; "1-3" includes 1, 2, and 3.

The term "optionally" means that the subsequently described event may or may not occur, and that the description includes both the occurrence and non-occurrence of the event.

The "stereoisomer" of the compound described herein refers to an isomer resulting from the different spatial arrangement of the atoms in a molecule. An enantiomer will be produced when an asymmetric carbon atom is present in a compound, or a *cis-trans* isomer will be produced when a carbon-carbon double bond or a ring structure is present in a compound.

The term "tautomer" refers to a particular functional group isomer in which different functional group isomers are in dynamic equilibrium and are rapidly transformed into each other. For example, in the presence of a ketone or oxime, a tautomer will be produced, and representative examples are: keto-enol tautomers, phenol-keto tautomers, nitroso-oxime tautomers, imine-enamine tautomers, etc.

All enantiomers, diastereomers, racemates, *cis*-trans isomers, tautomers, geometric isomers, and epimers of all of the compounds, and mixtures thereof are included in the scope of the present disclosure.

In the chemical configuration of the compound of the present disclosure, the bond "" represents an unspecified configuration, that is, if a chiral isomer is present in the chemical structure, the bond "" may be "" or "", or includes both "" and "". In the chemical structure of the compound of the present disclosure, "" indicates the attachment point to the parent molecule.

The "deuterated" described herein refers to the replacement of one or more hydrogen atoms with deuterium atoms in a compound or group.

**General preparation methods for compounds of the present disclosure**

Unless otherwise indicated, all starting materials or intermediates lacking preparation processes in the preparation methods may be purchased or synthesized using methods known in published literature. The suitable post-treatment methods described in the preparation methods may include one or a combination of two or more of conventional post-treatment methods such as: quenching with water, concentration, pH adjustment, extraction with a suitable solvent (such as ethyl acetate and dichloromethane), filtration, and drying. The suitable purification methods described in the preparation methods may include one or a combination of two or more of purification methods such as: silica gel column chromatography, preparative thin-layer chromatography, preparative reversed phase chromatography, recrystallization, and slurrying.

Compounds represented by general formula (A') containing an alkynyl substitution on Y can be prepared via the route shown in the following **reaction formula 1** or **reaction formula 2.**

## Reaction formula 1

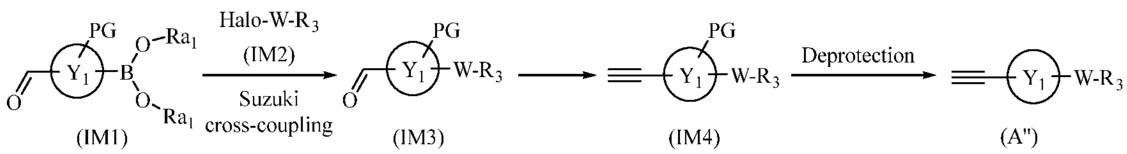

## Reaction formula 2

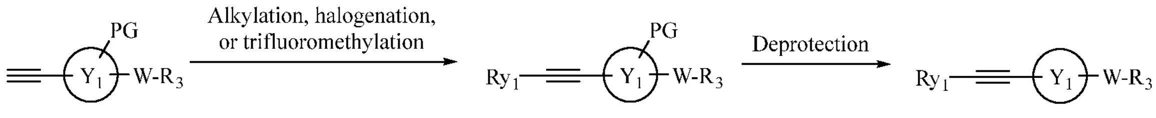


(IM4) (IM5) (A''')

In the reaction formulas,

W is selected from

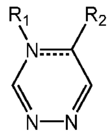 , 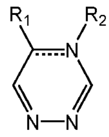 , and 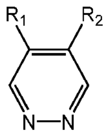 ;

- - - - is selected from a single bond and a double bond;

$R_3$, $R_1$, and $R_2$ are as defined above;

ring Yi is selected from aryl, 5-14 membered heteroaryl, 3-14 membered heterocyclyl, and 3-12 membered cycloalkyl, and the substituents on Yi are selected from the substituents for further substitution on Y as defined above;

PG is selected from suitable protecting groups on O or N (such as methyl, ethoxymethyl, methoxymethyl, benzyl, 4-methoxybenzyl, *tert*-butyl, *tert*-butoxycarbonyl, benzyloxycarbonyl, trimethylsilyl, and *tert*-butyldimethylsilyl);

Rai is selected from H and $C_{1-6}$ alkyl, and Rai may form a 5-7 membered heterocyclic ring with the B and O atoms through a carbon atom connection.

Halo is halogen (e.g., iodine, bromine, or chlorine);

$Ry_1$ is selected from hydrogen, halogen, $C_{1-6}$ alkyl, 3-6 membered cycloalkyl, halogenated $C_{1-6}$ alkyl, cyano, amino, carbonyl, and $C_{1-6}$ alkyl-trisubstituted silyl.

The following provides a method for preparing a compound containing an alkynyl substitution on Y represented by general formula (A") from a compound of formula (IM1) as shown in **reaction formula 1:**
A compound of formula (IM1) and a compound of formula (IM2) may be subjected to a Suzuki coupling reaction to prepare a compound of formula (IM3). The compound of formula (IM1) and the compound of formula (IM2) are added to a suitable solvent (e.g., 1,4-dioxane and water), a suitable catalyst (e.g., [1,1'-bis(diphenylphosphino)ferrocene]palladium(II) dichloride or tetrakis(triphenylphosphine)
palladium) is added, followed by the addition of a suitable base (e.g., sodium bicarbonate, sodium carbonate, or potassium carbonate), and the resulting mixture is heated and stirred at a suitable temperature (e.g., 90 °C-110 °C) for a suitable period of time (e.g., 1-20 h) under an inert gas atmosphere (e.g., nitrogen). After the reaction is completed, the compound of formula (IM3) is separated out through suitable post-treatment and purification methods.

The compound of formula (IM3) is dissolved in a suitable solvent (e.g., methanol), a suitable base (e.g., potassium carbonate) and dimethyl (1-diazo-2-oxopropyl)phosphonate are added, and the resulting mixture is stirred at room temperature for a suitable period of time (e.g., 1-5 h). After the reaction is completed, a compound of formula (IM4) is separated out through suitable post-treatment and purification methods.

After the compound of formula (IM4) undergoes a reaction under suitable deprotection conditions, the compound of formula (A") is separated out through suitable post-treatment and purification methods. For example, when the protecting group PG on formula (IM4) is ethoxymethyl, the compound of formula (IM4) is added to a suitable solvent (e.g., dichloromethane), an appropriate amount of an acid (e.g., a solution of hydrogen chloride in 1,4-dioxane, or trifluoroacetic acid) is added, and the resulting mixture is allowed to react at a suitable temperature (e.g., room temperature) for a suitable period of time (e.g., 5 min-2.5 h) to complete the deprotection reaction; when the protecting group PG on formula (IM4) is methyl, the compound of formula (IM4) is added to a suitable solvent (e.g., dichloromethane), an appropriate amount of boron tribromide is added at a suitable temperature (e.g., 0 °C), and the resulting mixture is

allowed to react at a suitable temperature (e.g., room temperature) for a suitable period of time (e.g., 17 h) to complete the deprotection reaction.

When the substituent $Ry_1$ on the alkynyl group is not hydrogen, compounds represented by general formula (A"') can be prepared via the route shown in the following **reaction formula 2.** The following provides a method for preparing a compound containing a substituent on the alkynyl group represented by general formula (A"') from the compound of formula (IM4) as shown in **reaction formula 2:**

The compound of formula (IM4) may be used to prepare compounds of formula (IM5) with different substitution patterns under different reaction conditions. For example, the compound of formula (IM4) can be subjected to an alkylation reaction with an alkyl halide (e.g., iodomethane) under the action of a base (e.g., sodium bis(trimethylsilyl)amide) to prepare a compound of formula (IM5) containing an alkyl- or haloalkyl-substituted alkynyl group; the compound of formula (IM4) can be reacted with a halogenating reagent (e.g., N-bromosuccinimide) under the action of a suitable catalyst (e.g., silver nitrate) to prepare a compound of formula (IM5) containing a halogen-substituted alkynyl group; the compound of formula (IM4) can be reacted with a trifluoromethylation reagent (e.g., Togni's reagent or Umemoto reagent) under the action of a suitable catalyst (e.g., cuprous iodide), a ligand (e.g., 1,10-phenanthroline) and a base (e.g., potassium bicarbonate) to prepare a compound of formula (IM5) containing a trifluoromethyl-substituted alkynyl group.

The compound of formula (IM1) can be prepared via the route shown in **reaction formula 3.**

## Reaction formula 3

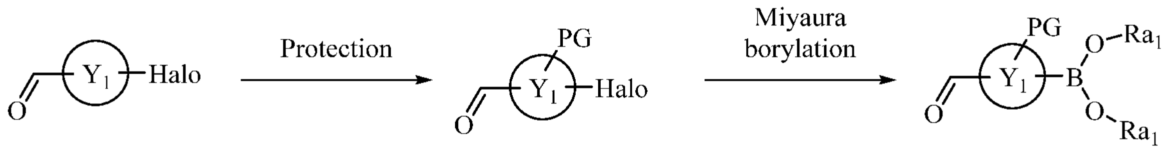


(IM1a) (IM1b) (IM1)

In the reaction formula,

$Y_1$, Halo, PG, and $Ra_1$ are as defined above.

The following provides a method for preparing the compound of formula (IM1) from a compound of formula (IM1a) as shown in **reaction formula 3:**

The compound of formula (IM1a) is dissolved in a suitable solvent (e.g., dichloromethane), a suitable protecting group reagent (e.g., chloromethoxyethane) and a suitable base (e.g., sodium hydride) are added, and the resulting mixture is stirred at a suitable temperature (e.g., 0 °C to room temperature) for a suitable period of time (e.g., 2-16 h). After the reaction is completed, a compound of formula (IM1b) is separated out through suitable post-treatment and purification methods.

The compound of formula (IM1b) can be subjected to a Miyaura coupling reaction with a suitable borate (e.g., bis(pinacolato)diboron) to prepare the compound of formula (IM1). The compound of formula (IM1b) and bis(pinacolato) diboron are added to a suitable solvent (e.g., 1,4-dioxane), a suitable catalyst (e.g., [1,1'-bis(diphenylphosphino) ferrocene]palladium(II) dichloride) is added, followed by the addition of a suitable base (e.g., potassium acetate), and the resulting mixture is heated and stirred at a suitable temperature (e.g., 100 °C) for a suitable period of time (e.g., 20 h) under an inert gas atmosphere (e.g., nitrogen). After the reaction is completed, the compound of formula (IM1) is separated out through suitable post-treatment and purification methods.

The compound of formula (IM1) can be prepared via the route shown in **reaction formula 4** or **reaction formula 5.**

## Reaction formula 4

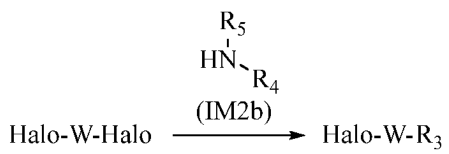


(IM2a) (IM2)

**Reaction formula 5**

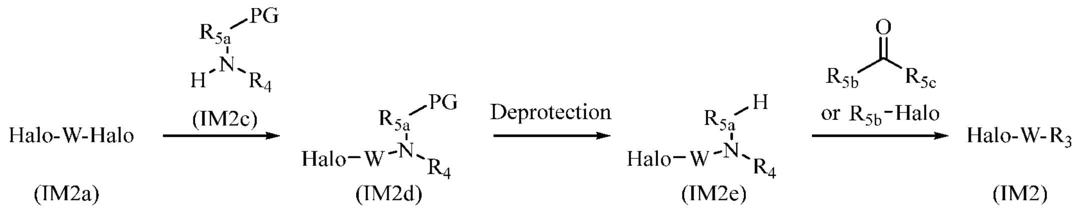

In the reaction formulas,

W is selected from

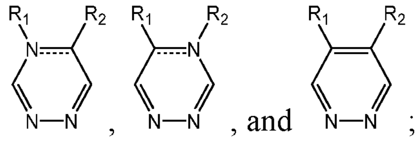

----- is selected from a single bond and a double bond;

Halo, $R_3$, $R_4$, $R_5$, and PG are as defined above;

$R_{5a}$ is selected from $R_5$ as defined above, and $R_{5a}$ contains O or N that can be protected by a protecting group;

$R_{5b}$ and $R_{5c}$ are selected from hydrogen, $C_{1-6}$ alkyl, $C_{3-7}$ cycloalkyl, halogenated $C_{1-6}$ alkyl, halogenated $C_{3-6}$ cycloalkyl, 3-7 membered heterocyclyl, aryl, and 5-7 membered heteroaryl, where the substituents are optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl;

$R_{5b}$ and $R_{5c}$ may be joined to form a 3-7 membered carbocyclic ring or a 3-7 membered heterocyclic ring.

The following provides a method for preparing the compound of formula (IM2) from a compound of formula (IM2a):
For example, as shown in **reaction formula 4,** the compound of formula (IM2a) and a compound of formula (IM2b) are added to a suitable solvent (e.g., 1,4-dioxane, *N,N*-dimethylacetamide, or *n*-butanol), a suitable base (e.g., *N,N*-diisopropylethylamine) is added, and the resulting mixture is heated and stirred at a suitable temperature (e.g., 100 °C-120 °C) for a suitable period of time (e.g., 1-72 h). After the reaction is completed, the compound of formula (IM2) is separated out through suitable post-treatment and purification methods.
In some embodiments, the compound of formula (IM2) can be prepared via the route shown in **reaction formula 5.**
For example, the compound of formula (IM2a) and a compound of formula (IM2c) are added to a suitable solvent (e.g., N,N-dimethylacetamide, toluene, or n-butanol), a suitable base (e.g., N,N-diisopropylethylamine) is added, and the resulting mixture is heated and stirred at a suitable temperature (e.g., 120 °C) for a suitable period of time (e.g., 1-72 h). After the reaction is completed, a compound of formula (IM2d) is separated out through suitable post-treatment and purification methods.
The compound of formula (IM2d) can be subjected to suitable deprotection conditions to give a compound of formula (IM2e). For example, the compound of formula (IM2d) is dissolved in a suitable solvent (e.g., dichloromethane), an appropriate amount of an acid (e.g., a solution of hydrogen chloride in 1,4-dioxane, or trifluoroacetic acid) is added, and the resulting mixture is allowed to react at a suitable temperature (e.g., room temperature) for a suitable period of time (e.g., 5 min-2.5 h) to complete the deprotection reaction.
The compound of formula (IM2e) can be further subjected to a reductive amination reaction or an alkylation reaction to prepare the compound of the formula (IM2). For example, the compound (IM2e) is dissolved in a suitable solvent (e.g., methanol), the corresponding aldehyde or ketone or its corresponding acetal or ketal is added, followed by the addition of a suitable reducing agent (e.g., sodium cyanoborohydride), and the resulting mixture is allowed to react at a suitable temperature (e.g., room temperature), thus achieving the conversion to the corresponding target product. As another example, the compound of formula (IM2e) is dissolved in a suitable solvent (e.g., dichloromethane), the

corresponding alkyl halide is added, and the resulting mixture is allowed to react in the presence of a suitable base (e.g., triethylamine or potassium carbonate), thus achieving the conversion to the corresponding target product. After the reaction is completed, the compound of formula (IM2) is separated out through suitable post-treatment and purification methods.

When W in the compound of formula (A') is selected from

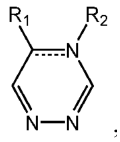

, the compound of formula (IM2d) in the above **reaction formula 5** can also be prepared via the route shown in **reaction formula 6.**

In the reaction formula,
$R_1$, $R_4$, $R_{5a}$, PG, and Halo are as defined above.

## Reaction formula 6

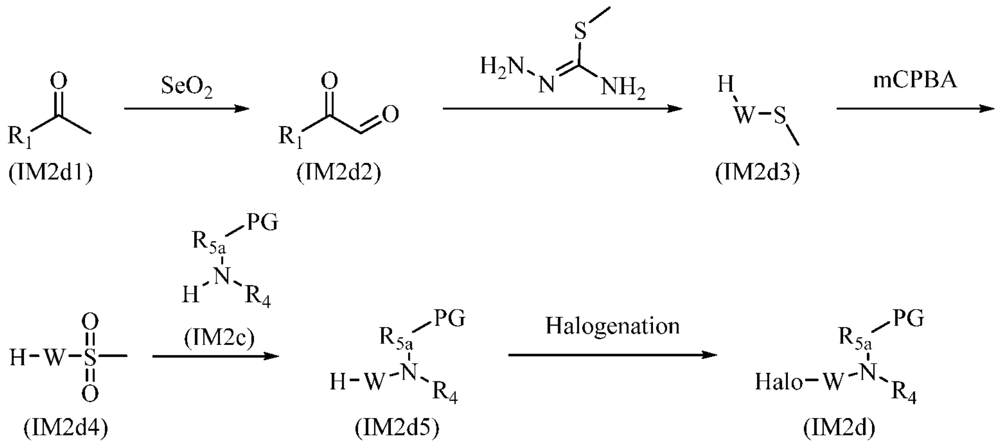


The preparation method is as follows:
A compound of formula (IM2d1) is oxidized to a compound of formula (IM2d2) under the action of the oxidant selenium dioxide, followed by cyclization with S-methylisothiourea hydroiodide to produce a compound of formula (IM2d3). The compound of formula (IM2d3) is subjected to an oxidation reaction with meta-chloroperoxybenzoic acid to produce a methylsulfonyl compound of formula (IM2d4). The compound of formula (IM2d4) is reacted with the compound of formula (IM2c) in the presence of a suitable base (e.g., *N,N*-diisopropylethylamine) to produce a compound of formula (IM2d5). The compound of formula (IM2d5) can then be reacted with a halogenating reagent (e.g., *N*-bromosuccinimide or dibromohydantoin) to prepare the compound of formula (IM2d).

When W in the compound of formula (A') is selected from

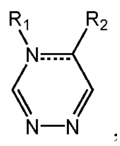

, the compound of formula (IM2e) in the above **reaction formula 5** can also be prepared via the route shown in **reaction formula 7.**

## Reaction formula 7

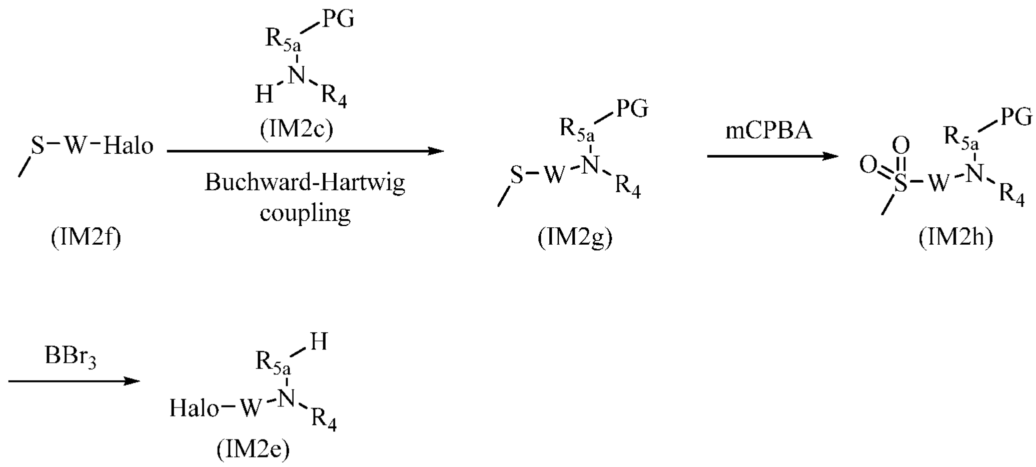


In the reaction formula,

$R_4$, $R_{5a}$, and PG are as defined above; Halo is bromine.

The preparation method is as follows:

A compound of formula (IM2f) is subjected to a Buchwald coupling reaction with the compound of formula (IM2c) in the presence of a suitable catalyst (tris(dibenzylideneacetone)dipalladium(0)), a ligand (e.g., 1,1'-binaphthyl-2,2'-bis(diphenylphosphine)) and a base (e.g., cesium carbonate) to produce a compound of formula (IM2g). The compound of formula (IM2g) is oxidized in the presence of *meta*-chloroperoxybenzoic acid to produce a compound of formula (IM2h). The compound of formula (IM2h) is then reacted with boron tribromide to prepare the compound of formula (IM2e).

In some embodiments, the compound of formula (IM4) described in **reaction formula 1** and **reaction formula 2** can be prepared via the route shown in **reaction formula 8.**

## Reaction formula 8

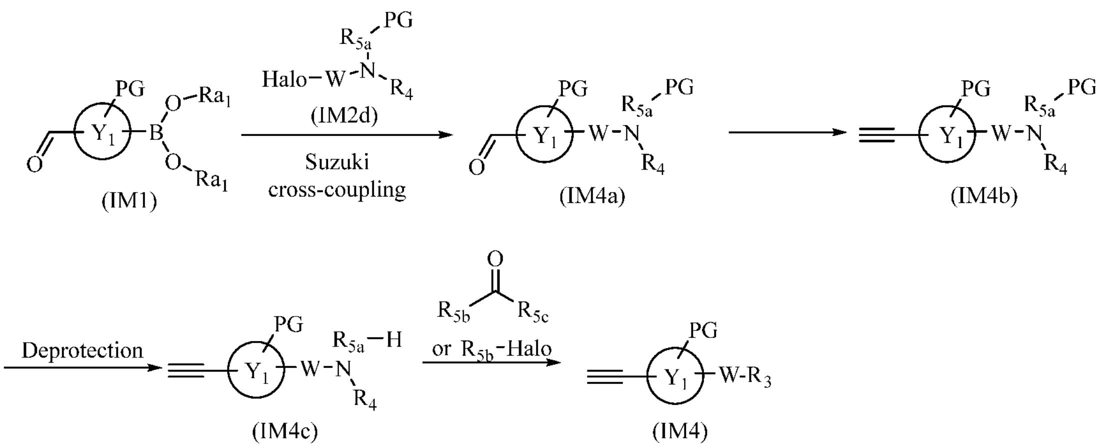


In the reaction formula,

W is selected from

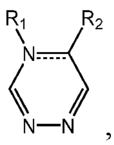 , 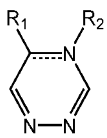 , and 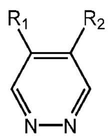 ;

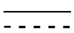 is selected from a single bond and a double bond;

$Y_1$, Halo, $R_1$, $R_2$, Rai, $R_3$, $R_4$, $R_5$, PG, $R_{5a}$, $R_{5b}$, and $R_{5c}$ are as defined above.

The following provides a method for preparing the compound of formula (IM4) from the compound of formula (IM1):
The compound of formula (IM1) and the compound of formula (IM2d) may be subjected to a Suzuki coupling reaction to prepare a compound of formula (IM4a). The compound of formula (IM1) and the compound of formula (IM2) are added to a suitable solvent (e.g., 1,4-dioxane and water), a suitable catalyst (e.g., [1,1'-bis(diphenylphosphino)ferrocene]palladium(II) dichloride or tetrakis(triphenylphosphine)
palladium) is added, followed by the addition of a suitable base (e.g., sodium bicarbonate, sodium carbonate, or potassium carbonate), and the resulting mixture is heated and stirred at a suitable temperature (e.g., 90 °C-110 °C) for a suitable period of time (e.g., 1-20 h) under an inert gas atmosphere (e.g., nitrogen). After the reaction is completed, the compound of formula (IM4a) is separated out through suitable post-treatment and purification methods.

The compound of formula (IM4a) is dissolved in a suitable solvent (e.g., methanol), a suitable base (e.g., potassium carbonate) and dimethyl (1-diazo-2-oxopropyl)phosphonate are added, and the resulting mixture is stirred at room temperature for a suitable period of time (e.g., 1-5 h). After the reaction is completed, a compound of formula (IM4b) is separated out through suitable post-treatment and purification methods.

After the compound of formula (IM4b) undergoes a reaction under suitable deprotection conditions (e.g., a solution of hydrogen chloride in 1,4-dioxane, or trifluoroacetic acid), the compound of formula (IM4c) is separated out through suitable post-treatment and purification methods.

The compound of formula (IM4c) can be subjected to a reductive amination reaction or an alkylation reaction to prepare the compound of the formula (IM4). For example, the compound (IM4c) is dissolved in a suitable solvent (e.g., methanol), the corresponding aldehyde or ketone or its corresponding acetal or ketal is added, followed by the addition of a suitable reducing agent (e.g., sodium cyanoborohydride), and the resulting mixture is allowed to react at a suitable temperature (e.g., room temperature), thus achieving the conversion to the corresponding target product. As another example, the compound of formula (IM4c) is dissolved in a suitable solvent (e.g., dichloromethane), the corresponding alkyl halide is added, and the resulting mixture is allowed to react in the presence of a suitable base (e.g., triethylamine or potassium carbonate), thus achieving the conversion to the corresponding target product. After the reaction is completed, the compound of formula (IM4) can be separated out through suitable post-treatment and purification methods.

When the substituent $Ry_1$ on the alkynyl group is not hydrogen, the compound of formula (IM5) shown in **reaction formula 2** can be prepared via the route shown in the following **reaction formula 9.**

## Reaction formula 9

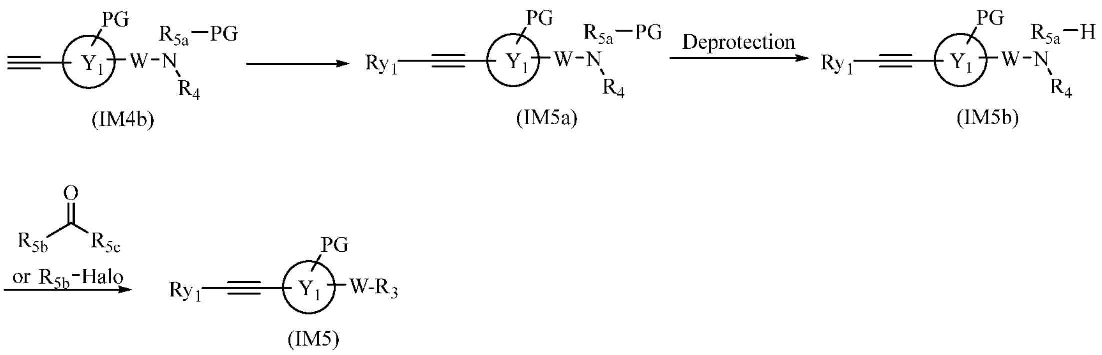

(IM4b) (IM5a) (IM5b)
(IM5)

In the reaction formula,

W is selected from

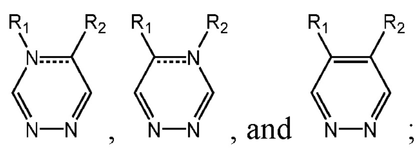
, , and ;

- - - - - is selected from a single bond and a double bond;

$Y_1$, $R_1$, $R_2$, $R_3$, $R_4$, PG, $R_{5a}$, $R_{5b}$, $R_{5c}$, and Halo are as defined above;

$Ry_1$ is as defined above, and $Ry_1$ is not hydrogen.

The following provides a method for preparing the compound of formula (IM5) from the compound of formula (IM4b) as shown in **reaction formula 9:**
The compound of formula (IM4b) can be used to prepare compounds of formula (IM5a) with different substitution patterns on the alkynyl group under different reaction conditions described in the first step of **reaction formula 2.** The compound of formula (IM5a) undergoes a reaction under suitable deprotection conditions (e.g., a solution of hydrogen chloride in 1,4-dioxane, or trifluoroacetic acid) to give a compound of formula (IM5b). The compound of formula (IM5b) can be subj ected to a reductive amination reaction or an alkylation reaction to prepare the compound of formula (IM5).
In some embodiments, the compound of formula (IM5) can be prepared via the route shown in **reaction formula 10.**

## Reaction formula 10

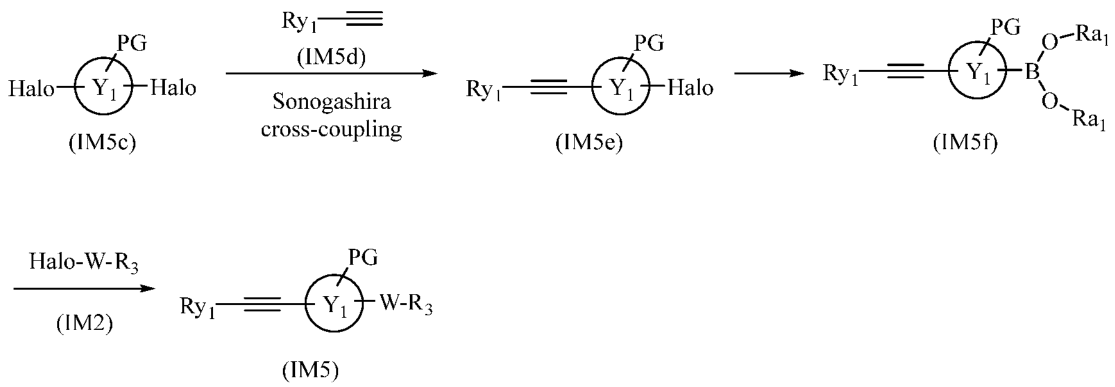

(IM5c) (IM5e) (IM5f) (IM5)

In the reaction formula, $Y_1$, Halo, W, $Ry_1$, Rai, R, and PG are as defined above;
The following provides a method for preparing the compound of formula (IM5) from a compound of formula (IM5c) as shown in **reaction formula 10:**
Under a nitrogen atmosphere, the compound of formula (IM5c) and a compound of formula (IM5d) can be subjected to a Sonogashira cross-coupling reaction in the presence of a suitable palladium catalyst (e.g., bis(triphenylphosphine) palladium(II) chloride), copper catalyst (e.g., cuprous iodide) and base (e.g., *N,N*-diisopropylethylamine) to prepare a compound of formula (IM5e). The compound of formula (IM5e) can be subjected to a Miyaura coupling reaction with a suitable borate (e.g., bis(pinacolato)diboron) to prepare a compound of formula (IM5f); alternatively, the compound of formula (IM5e) can be subjected to a lithiation reaction with an organolithium reagent, (e.g., *n*-butyllithium), followed by a reaction with a suitable borate (e.g., isopropoxyboronic acid pinacol ester) to prepare a compound of formula (IM5f). The compound of formula (IM5f) and the compound of formula (IM2) can be subjected to the Suzuki reaction described in **reaction formula 1** to prepare the compound of formula (IM5).
In some embodiments, the compound of formula (IM5a) in **reaction formula 9** may be prepared by the reaction shown in the following **reaction formula 11.**

## Reaction formula 11

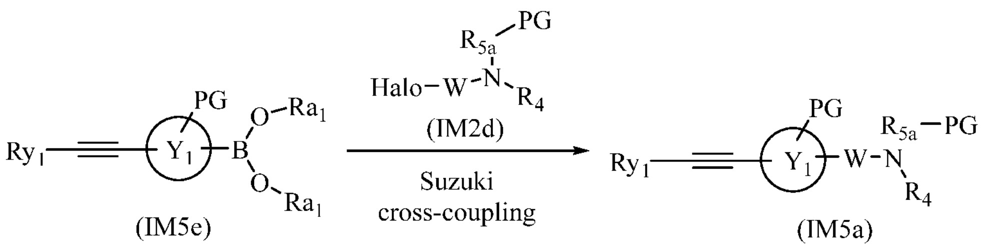

(IM5e) (IM5a)

In the reaction formula, $Ry_1$, $Y_1$, $Ra_1$, $R_4$, W, PG, $R_{5a}$, and Halo are as defined above;
the compound of formula (IM5e) and the compound of formula (IM2d) can be subj ected to the aforementioned Suzuki reaction to prepare the compound of formula (IM5a).
When W in the compound represented by general formula (A') is selected from

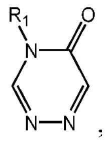

, the compound of formula (A') can be prepared by **reaction formula 12.**

## Reaction formula 12

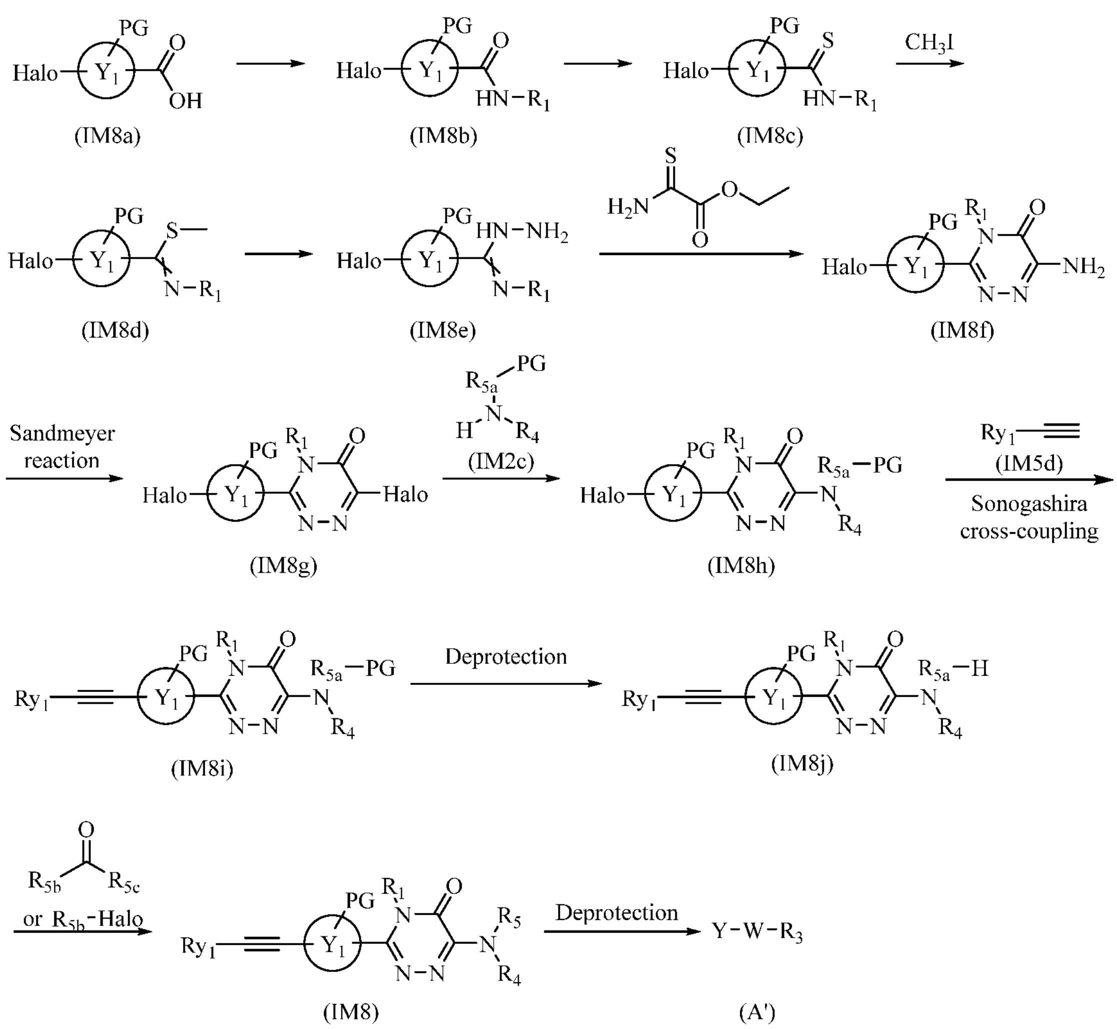


In the reaction formula, $Y_1$, $R_1$, $R_2$, $R_3$, $R_4$, $R_5$, PG, $R_{5a}$, $R_{5b}$, $R_{5c}$, Halo, and $Ry_1$ are as defined above.

The following provides a method for preparing the compound of formula (A') from a compound of formula (IM8a) via the route shown in **reaction formula 12:**

The compound of formula (IM8a) is reacted under the action of thionyl chloride or oxalyl chloride to produce the corresponding acyl chloride, which is then reacted with the corresponding amine to produce a compound of formula (IM8b). The compound of formula (IM8b) is reacted under the action of a sulfurizing reagent (e.g., Lawesson's reagent) to produce a compound of formula (IM8c). The compound of formula (IM8c) is reacted with iodomethane to produce a compound of formula (IM8d). The compound of formula (IM8d) is reacted with hydrazine hydrate to produce a compound of formula (IM8e). The compound of formula (IM8e) is subjected to a cyclization reaction with ethyl thiooxamate under the action of triethylamine to produce a compound of formula (IM8f). The compound of formula (IM8f) undergoes a Sandmeyer reaction with *tert*-butyl nitrite under catalysis by a copper salt (e.g., cuprous bromide or cuprous chloride) to produce a compound of formula (IM8g). The compound of formula (IM8g) is heated and reacted with the compound of formula (IM2c) under the action of a suitable base (e.g., *N,N*-diisopropylethylamine) to produce a compound of formula (IM8h). The compound of formula (IM8h) and the compound of formula (IM5d) can be subjected to a Sonogashira cross-coupling

reaction in the presence of a suitable palladium catalyst (e.g., bis(triphenylphosphine)palladium(II) chloride), copper catalyst (e.g., cuprous iodide) and base (e.g., *N,N*-diisopropylethylamine) to prepare a compound of formula (IM8i). The compound of formula (IM8i) undergoes a reaction under suitable deprotection conditions (e.g., a solution of hydrogen chloride in 1,4-dioxane, or trifluoroacetic acid) to give a compound of formula (IM8j). The compound of formula (IM8j) can be subjected to a reductive amination reaction or an alkylation reaction to prepare a compound of formula (IM8). The compound of formula (IM8) is subjected to deprotection under suitable deprotection conditions (e.g., boron tribromide/dichloromethane) to give the compound of formula (A').

In some embodiments, the compound of formula (IM8) can also be prepared via the route shown in **reaction formula 13.**

## Reaction formula 13

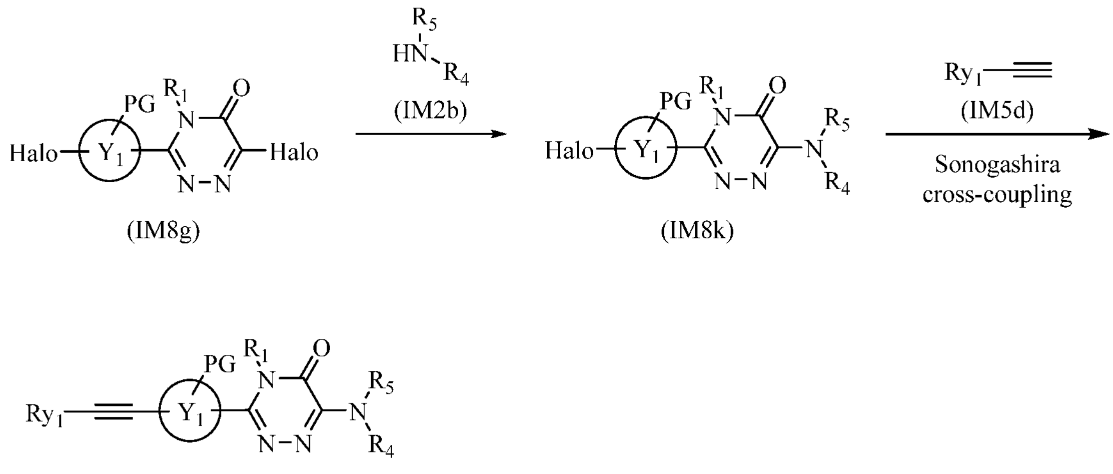


(IM8g) (IM8k) (IM8)

In the reaction formula, $Y_1$, $R_1$, $R_4$, $R_5$, PG, Halo, and $Ry_1$ are as defined above;

The compound of formula (IM8g) is reacted with the compound of formula (IM2b) under the action of a suitable base (e.g., *N,N*-diisopropylethylamine) to produce a compound of formula (IM8k). The compound of formula (IM8k) and the compound of formula (IM5d) can be subjected to a Sonogashira cross-coupling reaction in the presence of a suitable palladium catalyst (e.g., bis(triphenylphosphine)palladium(II) chloride), copper catalyst (e.g., cuprous iodide) and base (e.g., *N,N*-diisopropylethylamine) to prepare the compound of formula (IM8).

When the substituent on Y in the compound represented by general formula (A') is selected from an alkene group, the compound of formula (A') can be prepared via the route shown in **reaction formula 14** or **reaction formula 15.**

## Reaction formula 14

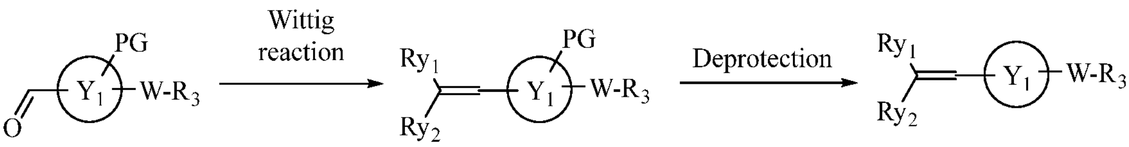


(IM3) (IM6) (A-A')

## Reaction formula 15

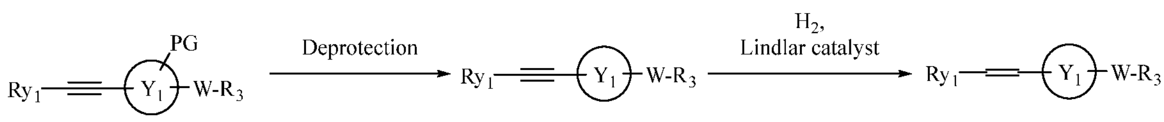


(IM5) (IM7) (A-A")

In the reaction formulas,

$Y_1$, Y, W, $Ry_1$, $R_3$, and PG are as defined above;

$Ry_2$ is selected from the groups as defined for $Ry_1$;

the compound of formula (IM3) can be reacted with the corresponding phosphonium ylide reagent to produce a compound of formula (IM6), which is then subjected to the aforementioned suitable deprotection conditions to produce a compound of formula (A-A');

alternatively, the compound of formula (IM5) is subjected to suitable deprotection conditions to produce a compound of formula (IM7), and the compound of formula (IM7) is reduced with hydrogen in the presence of a Lindlar palladium catalyst to give a compound of formula (A-A").

The compound of formula (IM6) shown in **reaction formula 15** can also be prepared via the route shown in **reaction formula 16.**

## Reaction formula 16

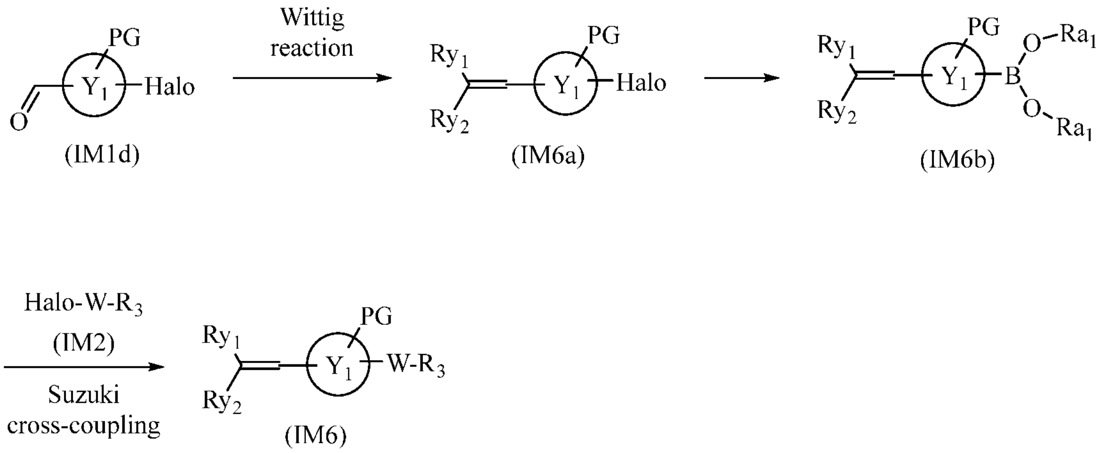


(IM1d) (IM6a) (IM6b) (IM6)

In the reaction formula,
$Y_1$, W, $Ry_1$, $Ry_2$, $R_3$, $Ra_1$, PG, and Halo are as defined above.

The following provides a method for preparing the compound of formula (IM6) from the compound of formula (IM1d):
The compound of formula (IM1d) is subjected to a Wittig reaction with the corresponding phosphonium ylide reagent to produce a compound of formula (IM6a), which is then subjected to the aforementioned Miyaura coupling reaction with a suitable borate (e.g., bis(pinacolato)diboron) to produce a compound of formula (IM6b); the compound of formula (IM6b) is subjected to the aforementioned Suzuki coupling reaction with the compound of formula (IM2) to produce the compound of formula (IM6).

In some embodiments, the compound of formula (IM6) can also be prepared via the route shown in **reaction formula 17.**

## Reaction formula 17

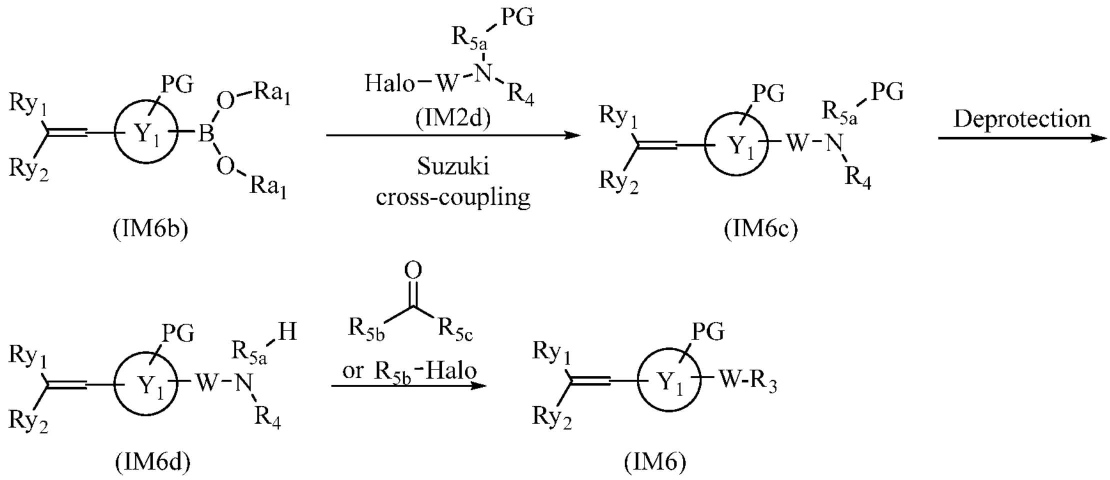

(IM6b) (IM6c) (IM6d) (IM6)

In the reaction formula,
$Y_1$, W, $Ry_1$, $Ry_2$, $R_3$, $Ra_1$, $R_{5a}$, $R_{5b}$, $R_{5c}$, PG, and Halo are as defined above.

The following provides a method for preparing the compound of formula (IM6) from the compound of formula (IM6b):
The compound of formula (IM6b) is subjected to the aforementioned Suzuki coupling reaction with the compound of formula (IM2d) to produce a compound of formula (IM6c); the compound of formula (IM6c) is subjected to the aforementioned suitable deprotection conditions to produce a compound of formula (IM6d); the compound of formula (IM6d) then undergoes a reductive amination or alkylation reaction with an aldehyde, a ketone or an alkyl halide to give the compound of formula (IM6).

<u>Effects of the Present Disclosure</u>

The compound or the pharmaceutically acceptable salt, the stereoisomer or the deuteride thereof provided in the present disclosure has good inhibitory activity against the NLRP3 inflammasome, and meanwhile, the compound of the present disclosure exhibits significant advantages in various aspects, such as liver microsome, PK, brain penetration rate and safety, across multiple species, so that the compound of the present disclosure can be safely and effectively used for preventing and/or treating NLRP3 inflammasome-associated diseases.

**DETAILED DESCRIPTION**

In order to make the objective, technical solutions, and advantages of the present disclosure more apparent, the present disclosure is further illustrated in detail below. It is apparent that the examples described herein are only some, but not all, examples of the present disclosure. All other examples obtained by those of ordinary skill in the art based on the examples in the present disclosure without making inventive efforts shall fall within the protection scope of the present disclosure.

The abbreviations and English expressions used in the present disclosure have the following meanings:

"THF" refers to tetrahydrofuran; "DMF" refers to *N,N*-dimethylformamide; "MeOH" refers to methanol; "EA" refers to ethyl acetate; "DCM" refers to dichloromethane; "DMA" refers to *N,N*-dimethylacetamide; "MTBE" refers to methyl *tert*-butyl ether; "EtOH" refers to ethanol; "DMAC" refers to dimethylacetamide; "PE" refers to petroleum ether; "*n*-BuLi" refers to *n*-butyllithium;
"FBS" refers to fetal bovine serum; "PBS" refers to phosphate-buffered saline; "PMA" refers to phorbol 12-myristate 13-acetate; "LPS" refers to lipopolysaccharide; and "Nigericin" refers to nigericin sodium salt.

**Examples**

The embodiments of the present disclosure will be described in detail with reference to the following examples, but those skilled in the art will understand that the following examples are provided solely to illustrate the present disclosure and should not be construed as limiting the scope of the present disclosure. Experimental procedures without specified

conditions in the examples were conducted under conventional conditions or conditions recommended by the manufacturers. Reagents or instruments without specified manufacturers used herein are conventional products that are commercially available.

**Example 1: Synthesis of (R)-3-(4-ethynyl-2-hydroxyphenyl)-4-methyl-6-((1-methylpiperidin-3-yl)amino)-1,2,4-triazin-5(4*H*)-one (Compound 23)**

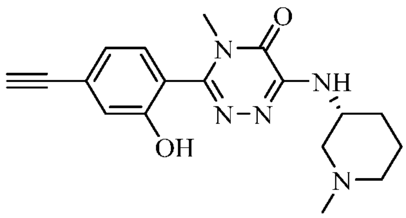

Step 1: Synthesis of 4-bromo-2-methoxy-N-methylbenzamide

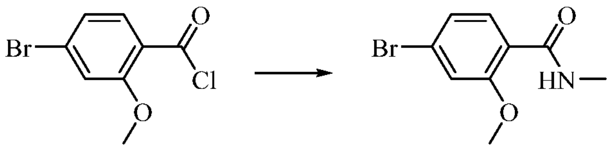

A solution of 4-bromo-2-methoxybenzoyl chloride (32.4 g, 129.85 mmol, 1.0 eq) in dichloromethane (200 mL) was added dropwise to an aqueous methylamine solution (100 mL), and the mixture was allowed to react at room temperature for 10 min. TLC analysis showed the completion of the reaction. The reaction solution was poured into water (100 mL) and extracted with dichloromethane (100 mL × 2). The organic phase was dried and concentrated to give the product (30 g, yield: 94.6%).

Step 2: Synthesis of 4-bromo-2-methoxy-*N*-methylbenzothioamide

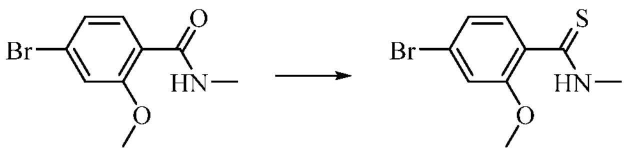

4-Bromo-2-methoxy-*N*-methylbenzamide (30 g, 122.90 mmol, 1.0 eq) and Lawesson's reagent (34.8 g, 86.03 mmol, 0.7 eq) were dissolved in THF (300 mL), and the solution was allowed to react at 70 °C for 1 h. TLC analysis showed the completion of the reaction. The reaction solution was poured into water (300 mL) and extracted with ethyl acetate (300 mL × 3). The organic phase was dried and concentrated to give the product (31.97 g, yield: 100%).

Step 3: Synthesis of methyl 4-bromo-2-methoxy-*N*-methylbenzimidothioate

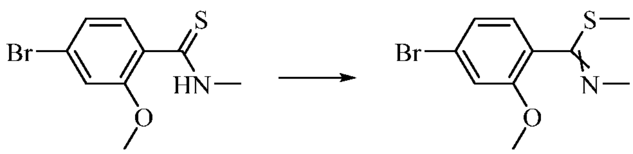

4-Bromo-2-methoxy-*N*-methylbenzothioamide (31.94 g, 122.90 mmol, 1.0 eq) and iodomethane (26.17 g, 184.35 mmol, 1.5 eq) were dissolved in THF (300 mL), and the solution was allowed to react at room temperature for 19 h. TLC analysis showed the completion of the reaction. The reaction solution was poured into water (500 mL), and the pH was adjusted to about 8 with sodium bicarbonate, followed by extraction with ethyl acetate (300 mL × 2). The organic phase was dried and concentrated to give the product (33.7 g, yield: 100%).

Step 4: Synthesis of *N*-amino-4-bromo-2-methoxy-*N'*-methylbenzamidine

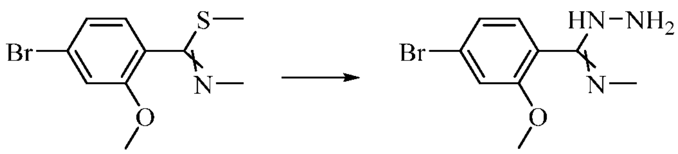

Methyl 4-bromo-2-methoxy-*N*-methylbenzimidothioate (33.7 g, 122.90 mmol, 1.0 eq) and hydrazine hydrate (7.24 g, 122.90 mmol, 1.0 eq) were dissolved in EtOH (300 mL), and the solution was allowed to react at 80 °C for 0.5 h. LC-MS monitoring showed the completion of the reaction. The reaction solution was concentrated under reduced pressure to give a crude product (31.7 g, yield: 100%).

Step 5: Synthesis of 6-amino-3-(4-bromo-2-methoxyphenyl)-4-methyl-1,2,4-triazin-5(4*H*)-one

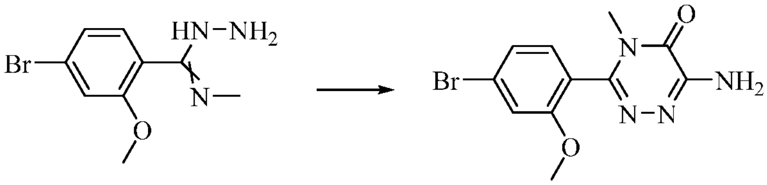

*N*-Amino-4-bromo-2-methoxy-*N'*-methylbenzamidine (31.7 g, 122.90 mmol, 1.0 eq), ethyl thiooxamate (16.37 g, 122.90 mmol, 1.0 eq), and triethylamine (12.44 g, 122.90 mmol, 1.0 eq) were dissolved in EtOH (300 mL), and the solution was allowed to react at 70 °C for 16 h. LC-MS monitoring showed the completion of the reaction. The reaction solution was cooled to room temperature and concentrated under reduced pressure, and the crude product was purified by silica gel column chromatography (methanol:dichloromethane = 1:100), then slurried with ethyl acetate (100 mL), and filtered under vacuum to give the product (15.5 g, yield: 40.6%).

Step 6: Synthesis of 3-(4-bromo-2-methoxyphenyl)-6-chloro-4-methyl-1,2,4-triazin-5(4*H*)-one

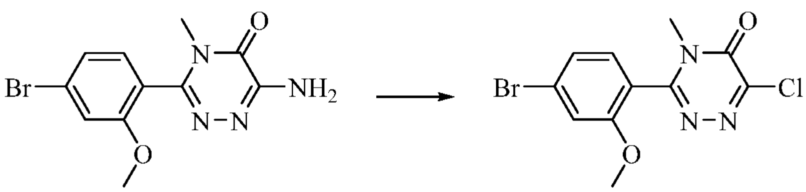

6-Amino-3-(4-bromo-2-methoxyphenyl)-4-methyl-1,2,4-triazin-5(4*H*)-one (5.0 g, 16.07 mmol, 1.0 eq) and CuCl (3.18 g, 32.14 mmol, 2.0 eq) were dispersed in acetonitrile (50 mL), and the dispersion was allowed to react at 70 °C for 5 min under a nitrogen atmosphere. *tert*-Butyl nitrite (3.31 g, 32.14 mmol, 2.0 eq) was added dropwise and slowly, and the resulting mixture was allowed to react at 70 °C for 2 h. TLC monitoring showed the completion of the reaction. The reaction solution was concentrated under reduced pressure, and the crude product was purified by silica gel column chromatography (ethyl acetate:dichloromethane = 1:5) to give the product (1.5 g, yield: 28.2%).

Step 7: Synthesis of *tert*-butyl (*R*)-3-((3-(4-bromo-2-methoxyphenyl)-4-methyl-5-oxo-4,5-dihydro-1,2,4-triazin-6-yl)amin o)piperidine-1-carboxylate

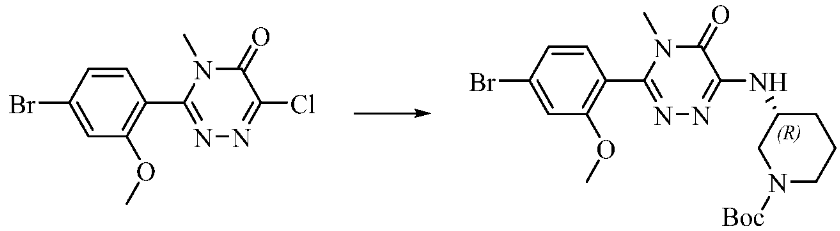

3-(4-Bromo-2-methoxyphenyl)-6-chloro-4-methyl-1,2,4-triazin-5(4*H*)-one (1.5 g, 4.54 mmol, 1.0 eq), *tert*-butyl (*R*)-3-aminopiperidine-1-carboxylate (1.09 g, 5.45 mmol, 1.2 eq), and *N,N*-diisopropylethylamine (880 mg, 6.81 mmol, 1.5 eq) were dissolved in 1,4-dioxane (30 mL), and the solution was allowed to react at 100 °C for 18 h. LC-MS analysis showed the completion of the reaction. The reaction solution was poured into water (30 mL) and extracted with ethyl acetate (30 mL × 2). The organic phase was dried and concentrated, and the crude product was purified by silica gel column chromatography (methanol:dichloromethane = 1: 100) to give the product (1.2 g, yield: 53.6%).

Step 8: Synthesis of *tert*-butyl (*R*)-3-((3-(2-methoxy-4-((trimethylsilyl)ethynyl) phenyl)-4-methyl-5-oxo-4,5-dihydro-1,2,4-triazin-6-yl)amino)piperidine-1-carboxylate

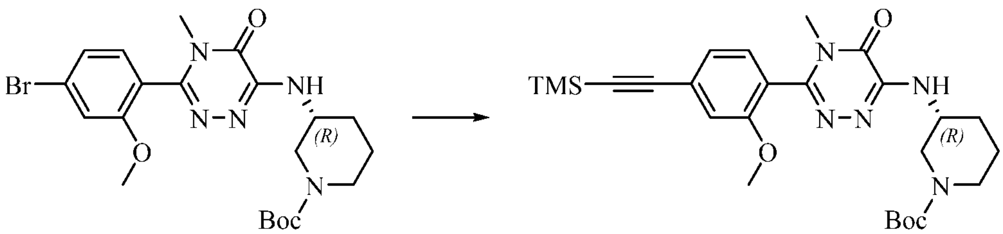

*tert*-Butyl (*R*)-3-((3-(4-bromo-2-methoxyphenyl)-4-methyl-5-oxo-4,5-dihydro-1,2,4-triazin-6-yl)amino)piperidine-1-carboxylate (0.6 g, 1.21 mmol, 1.0 eq), $PdCl_2(PPh_3)_2$ (84 mg, 0.12 mmol, 0.1 eq), and CuI (68 mg, 0.36 mmol, 0.3 eq) were added to diisopropylamine (20 mL), and the mixture was allowed to react at 60 °C for 10 min under a nitrogen atmosphere. Trimethylsilylacetylene (1.19 g, 12.10 mmol, 10 eq) was added, and the resulting mixture was allowed to react at 60 °C for 5 h. TLC analysis showed the completion of the reaction. The reaction solution was concentrated under reduced pressure, and the crude product was purified by silica gel column chromatography (methanol:dichloromethane = 1:100) to give the product (500 mg, yield: 80.8%).

Step 9: Synthesis of (*R*)-3-(4-ethynyl-2-hydroxyphenyl)-4-methyl-6-(piperidin-3-ylamino)-1,2,4-triazin-5(4*H*)-o ne

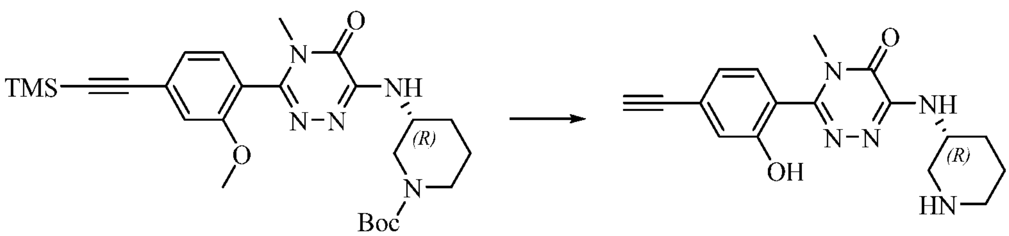

*tert*-Butyl (*R*)-3-((3-(2-methoxy-4-((trimethylsilyl)ethynyl)phenyl)-4-methyl-5-oxo-4,5-dihydro-1,2,4-triazin-6-yl)amino)piperidine-1-carboxylate (0.5 g, 0.98 mmol, 1.0 eq) was dissolved in dichloromethane (10 mL), and the solution was cooled to -70 °C. Boron tribromide (736 mg, 2.94 mmol, 3.0 eq) was added, and the mixture was naturally warmed to room

temperature and allowed to react for 17 h. LC-MS analysis showed the completion of the reaction. The reaction solution was quenched by adding an appropriate amount of methanol, poured into water (20 mL), and extracted with methanol/dichloromethane (1:10, 30 mL × 6). The organic phase was dried and concentrated to give a crude product (200 mg, yield: 62.9%).

Step 10: Synthesis of (*R*)-3-(4-ethynyl-2-hydroxyphenyl)-4-methyl-6-((1-methylpiperidin-3-yl)amino)-1,2,4-triazin-5(4H)-one

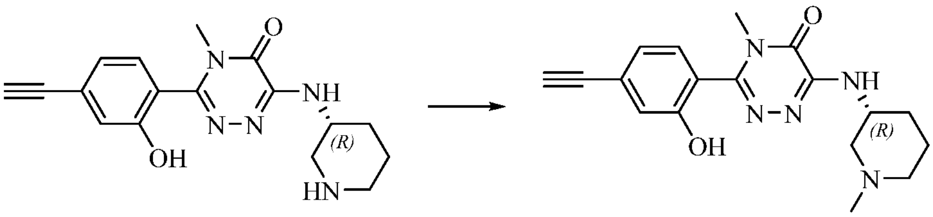

(*R*)-3-(4-Ethynyl-2-hydroxyphenyl)-4-methyl-6-(piperidin-3-ylamino)-1,2,4-tr iazin-5(4*H*)-one (200 mg, 0.61 mmol, 1.0 eq) and an aqueous formaldehyde solution (37%) (50 mg, 0.61 mmol, 1.0 eq) were dissolved in methanol (30 mL), and the solution was stirred at room temperature for 10 min. Sodium cyanoborohydride (38 mg, 0.61 mmol, 1.0 eq) was added, and the mixture was allowed to react at room temperature for 10 min. TLC monitoring showed the completion of the reaction. The reaction solution was concentrated under reduced pressure, and the crude product was dispersed in water (10 mL). The dispersion was extracted with dichloromethane (20 mL × 4). The organic phase was dried and concentrated, and the residue was purified by preparative thin-layer chromatography (dichloromethane:methanol = 7:1) to give the product (55 mg, yield: 26.6%).

$^1$HNMR(400MHz, DMSO-$d_6$) δ(ppm): 10.46(s, 1H), 7.32-7.30 (d, 1H), 7.05-7.03 (d, 2H), 6.89 (s, 1H), 4.29 (s, 1H), 4.05 (s, 1H), 3.18 (s, 1H), 2.81 (s, 1H), 2.27 (s, 5H), 1.71-1.56 (d, 5H).

Molecular formula: $C_{18}H_{21}N_5O_2$ Exact molecular weight: 339.17 LC-MS (Pos, *m*/*z*) = 340.15 $[M+H]^+$.

**Example 2: Synthesis of (*R*)-3-(2-hydroxy-4-(prop-1-yn-1-yl)phenyl)-4-methyl-6-((1-methylpiperidin-3-yl)amino)-1,2,4-triazin-5(4*H*)-one (Compound 27)**

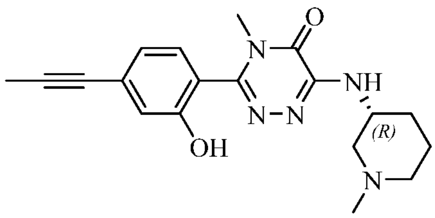

Step 1: Synthesis of *tert*-butyl (*R*)-3-((3-(2-methoxy-4-(prop-1-yn-1-yl)phenyl)-4-methyl-5-oxo-4,5-dihydro-1,2,4-triazin-6-yl)amino)piperidine-1-carboxylate

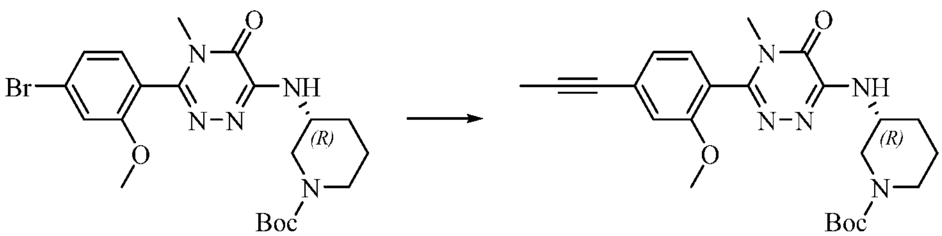

*tert*-Butyl (*R*)-3-((3-(4-bromo-2-methoxyphenyl)-4-methyl-5-oxo-4,5-dihydro-1,2,4-triazin-6-yl)amino)piperidine-1-carboxylate (0.6 g, 1.21 mmol, 1.0 eq), $PdCl_2(PPh_3)_2$ (84 mg, 0.12 mmol, 0.1 eq), and CuI (68 mg, 0.36 mmol, 0.3 eq) were dissolved to diisopropylamine (10 mL), and the mixture was allowed to react at 60 °C for 10 min under a nitrogen atmosphere. A solution of propyne in tetrahydrofuran (1 mol/L, 12.1 mL, 10 eq) was added, and the mixture was allowed to react at 60 °C for 16 h. LC-MS analysis showed the presence of the target product. The reaction solution was poured into water (30 mL) and extracted with ethyl acetate (30 mL × 2). The organic phase was dried and concentrated, and the crude product was purified by silica gel column chromatography (ethyl acetate:PE = 1:1) to give the product (300 mg, yield: 54.6%).

Step 2: Synthesis of (*R*)-3-(2-hydroxy-4-(prop-1-yn-1-yl)phenyl)-4-methyl-6-(piperidin-3-ylamino)-1,2,4-triazin -5(4*H*)-one

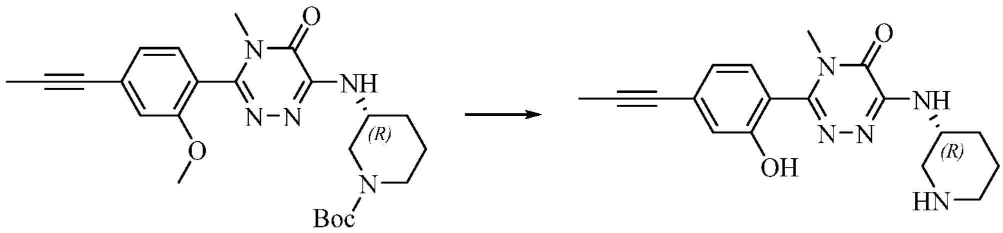

*tert*-Butyl (*R*)-3-((3-(2-methoxy-4-(prop-1-yn-1-yl)phenyl)-4-methyl-5-oxo-4,5-dihydro-1,2,4-triazin-6-yl)amino) piperidine-1-carboxylate (0.3 g, 0.66 mmol, 1.0 eq) was dissolved in dichloromethane (10 mL), and the solution was cooled to -70 °C. Boron tribromide (496 mg, 1.98 mmol, 3.0 eq) was added, and the mixture was allowed to react for 3 h. LC-MS analysis showed the completion of the reaction. The reaction solution was quenched by adding an appropriate amount of methanol, poured into water (20 mL), and extracted with dichloromethane (10 mL × 4). The organic phase was dried and concentrated to give a crude product (100 mg, yield: 44.6%).

Step 3: Synthesis of (*R*)-3-(2-hydroxy-4-(prop-1-yn-1-yl)phenyl)-4-methyl-6-((1-methylpiperidin-3-yl)amino)-1 ,2,4-triazin-5(4*H*)-one

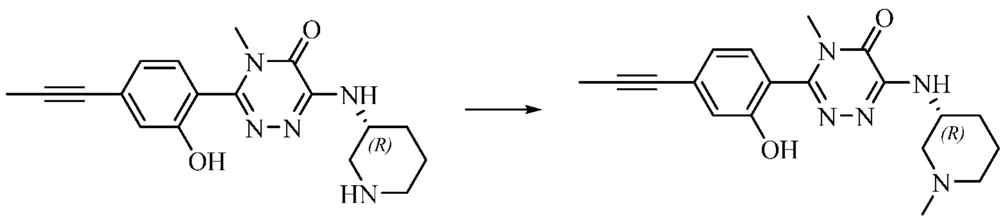

(*R*)-3-(2-Hydroxy-4-(prop-1-yn-1-yl)phenyl)-4-methyl-6-(piperidin-3-ylamino )-1,2,4-triazin-5(4*H*)-one (100 mg, 0.29 mmol, 1.0 eq) and an aqueous formaldehyde solution (mass fraction: 37%) (24 mg, 0.29 mmol, 1.0 eq) were dissolved in methanol (3 mL), and the solution was stirred at room temperature for 5 min. Sodium cyanoborohydride (18 mg, 0.29 mmol, 1.0 eq) was added, and the mixture was allowed to react at room temperature for 5 min. TLC monitoring showed the completion of the reaction. The reaction solution was poured into water (10 mL) and extracted with dichloromethane (20 mL × 3). The organic phase was dried and concentrated, and the crude product was purified by preparative thin-layer chromatography (dichloromethane:methanol = 8:1) to give the product (10 mg, yield: 9.8%).

$^1$HNMR (400MHz, $CDCl_3$) δ(ppm): 7.23-7.21 (d, 1H), 7.12 (s, 1H), 6.99-6.97 (d, 1H), 6.48 (s, 1H), 4.25 (s, 1H), 3.57 (s, 3H), 2.58 (s, 2H), 2.33 (s, 4H), 2.09 (s, 3H), 1.75-1.69 (d, 5H).

Molecular formula: $C_{19}H_{23}N_5O_2$ Exact molecular weight: 353.19 LC-MS (Pos, *m*/*z*) = 354.07 $[M+H]^+$.

**Example 3: Synthesis of (*R*)-3-(2-hydroxy-4-(prop-1-yn-1-yl)phenyl)-6-((1-(2-hydroxyethyl)piperidin-3-yl)amin o)-4-methyl-1,2,4-triazin-5(4*H*)-one (Compound 33)**

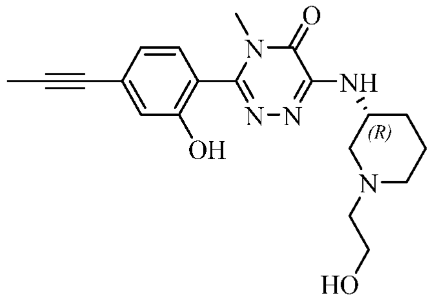

Step 1: Synthesis of (*R*)-3-(2-hydroxy-4-(prop-1-yn-1-yl)phenyl)-6-((1-(2-hydroxyethyl)piperidin-3-yl)amino)-4 -methyl-1,2,4-triazin-5(4*H*)-one

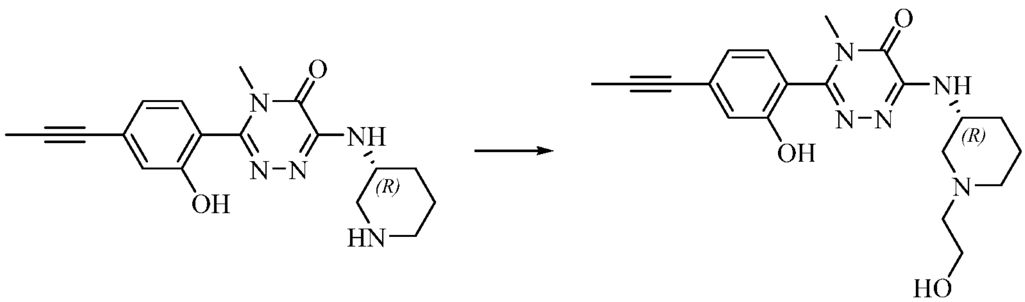

(*R*)-3-(2-Hydroxy-4-(prop-1-yn-1-yl)phenyl)-4-methyl-6-(piperidin-3-ylamino )-1,2,4-triazin-5(4*H*)-one (150 mg, 0.44 mmol, 1.0 eq), bromoethanol (165 mg, 1.32 mmol, 3.0 eq), and TEA (233 mg, 2.20 mmol, 5.0 eq) were dissolved in THF (5 mL), and the solution was allowed to react at 60 °C for 3 h. LC-MS monitoring showed the completion of the reaction. The reaction solution was poured into water (20 mL) and extracted with DCM (20 mL × 3). The organic phase was dried and concentrated, and the residue was purified by preparative thin-layer chromatography (DCM:MeOH = 8:1) to give the product (80 mg, yield: 47.6%).

$^1$HNMR (400 MHz, $CD_3OD$) δ(ppm): 7.29-7.27 (d, 1H), 7.00-7.98 (d, 1H), 6.95 (s, 1H), 4.25-4.21 (m, 1H), 3.79-3.76 (t, 2H), 3.34 (s, 3H), 3.28 (s, 1H), 2.97 (s, 1H), 2.84-2.83 (d, 2H), 2.66-2.61 (t, 2H), 2.06 (s, 3H), 1.97-1.95 (d, 1H), 1.93-1.90 (t, 1H), 1.83-1.78 (m, 1H), 1.77-1.68 (m, 1H).

Molecular formula: $C_{20}H_{25}N_5O_3$ Exact molecular weight: 383.20 LC-MS (Pos, m/z) = 384.22 $[M+H]^+$.

**Example 4: Synthesis of (*R*)-3-(4-ethynyl-2-hydroxyphenyl)-6-((1-(2-hydroxyethyl)piperidin-3-yl)amino)-4-met hyl-1,2,4-triazin-5(4*H*)-one (Compound 42)**

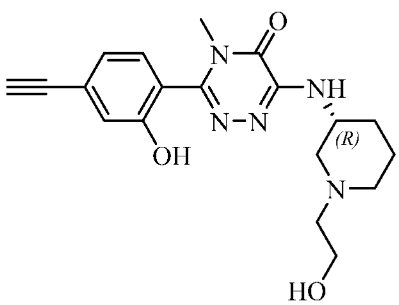

Step 1: Synthesis of (*R*)-3-(4-ethynyl-2-hydroxyphenyl)-6-((1-(2-hydroxyethyl)piperidin-3-yl)amino)-4-methyl-1,2,4-triazin-5(4*H*)-one

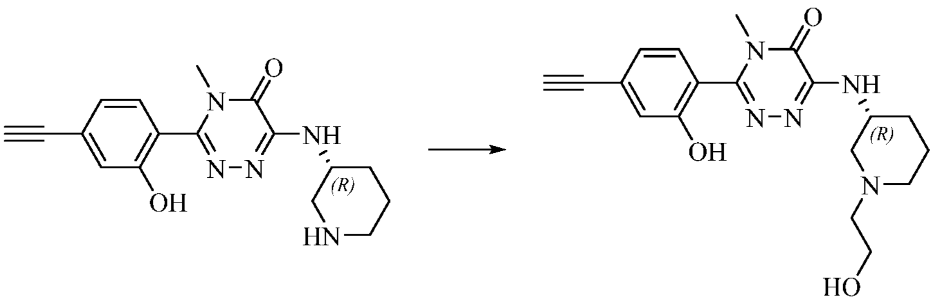

(*R*)-3-(4-Ethynyl-2-hydroxyphenyl)-4-methyl-6-(piperidin-3-ylamino)-1,2,4-tr iazin-5(4H)-one (300 mg, 0.92 mmol, 1.0 eq), bromoethanol (172 mg, 1.38 mmol, 3.0 eq), and TEA (279 mg, 2.76 mmol, 5.0 eq) were dissolved in 1,4-dioxane (10 mL), and the solution was allowed to react at 100 °C for 1 h. TLC monitoring showed the completion of the reaction. The reaction solution was concentrated under reduced pressure, and the crude product was purified by silica gel column chromatography (DCM:MeOH = 10:1) and then slurried with EA (5 mL) to give the product (170 mg, yield: 50%).

$^1$HNMR(400MHz, $CD_3OD$) δ(ppm): 7.35-7.33(d, 1H), 7.12-7.10(d, 1H), 7.06(s, 1H), 4.29-4.26 (m, 1H), 3.82-3.79 (t, 2H), 3.64 (s, 1H), 3.41 (s, 1H), 3.35 (s, 3H), 3.09 (s, 1H), 2.94 (s, 2H), 2.78-2.68 (m, 2H), 2.00 (s, 1H), 1.98-1.95 (m, 1H), 1.87-1.81 (m, 1H), 1.78-1.68 (m, 1H).

Molecular formula: $C_{19}H_{23}N_5O_3$ Exact molecular weight: 369.18 LC-MS (Pos, m/z) = 370.15 $[M+H]^+$.

**Example 5: Synthesis of 5-(bromoethynyl)-2-(6-(((*cis*)-3-hydroxy-3-methylcyclobutyl)amino)-4-methylpyridazi n-3-yl)phenol (Compound 34)**

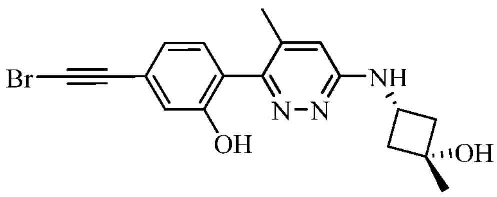

Step 1: Synthesis of intermediate (*cis*)-3-((6-chloro-5-methylpyridazin-3-yl)amino)-1-methylcyclobutan-1-ol

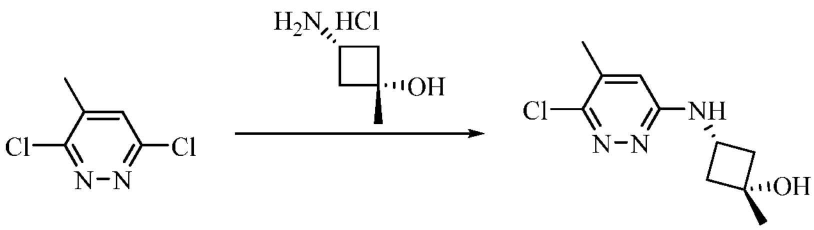

3,6-Dichloro-4-methylpyridazine (1 g, 6.13 mmol, 1.0 eq) was dissolved in n-butanol (5 mL), and (*cis*)-3-amino-1-methylcyclobutan-1-ol hydrochloride (844 mg, 6.13 mmol, 1.0 eq), and DIPEA (1.5 g, 12.27 mmol, 2.0 eq) were sequentially added. The mixture was stirred under microwave at 150 °C for 2 h. TLC analysis showed the completion of the reaction. The reaction solution was concentrated. Water (5 mL) was added, and extraction was performed with dichloromethane (5 mL × 3). The organic phase was dried and filtered under vacuum. The filtrate was concentrated, and the crude product was purified by silica gel column chromatography (dichloromethane:methanol = 80:1 to 20:1) to give the product (225 mg, yield: 16.1%).

Step 2: Synthesis of intermediate 3-(ethoxymethoxy)-4-(6-(((*cis*)-3-hydroxy-3-methylcyclobutyl)amino)-4-methylpyridazin-3 -yl)benzaldehyde

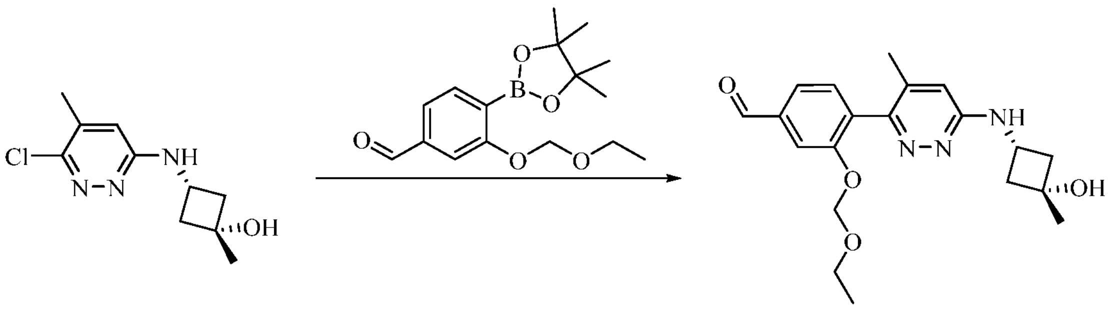

The (*cis*)-3-((6-chloro-5-methylpyridazin-3-yl)amino)-1-methylcyclobutan-1-ol obtained in the previous step (500 mg, 2.20 mmol, 1.0 eq) was dissolved in 1,4-dioxane (5 mL) and water (2 mL), and 3-(ethoxymethoxy)-4-(4,4,5,5-tetramethyl-1,3,2-dioxaborolan-2-yl)benzaldehyde (874 mg, 2.85 mmol, 1.3 eq), sodium bicarbonate (370 mg, 4.40 mmol, 2.0 eq), and Pd(dppf)$Cl_2$ (161 mg, 0.22 mmol, 0.1 eq) were sequentially added. The mixture was stirred at 90 °C for 2 h under a nitrogen atmosphere. LC-MS analysis showed the completion of the reaction. The reaction solution was filtered through celite under vacuum and washed with ethyl acetate. The organic phase was dried and filtered under vacuum. The filtrate was concentrated, and the crude product was purified by silica gel column chromatography (dichloromethane:methanol = 40:1 to 10:1) to give the product (700 mg, yield: 85.7%).

Step 3: Synthesis of intermediate (*cis*)-3-((6-(2-(ethoxymethoxy)-4-ethynylphenyl)-5-methylpyridazin-3-yl)amino)-1-methyl cyclobutan-1-ol

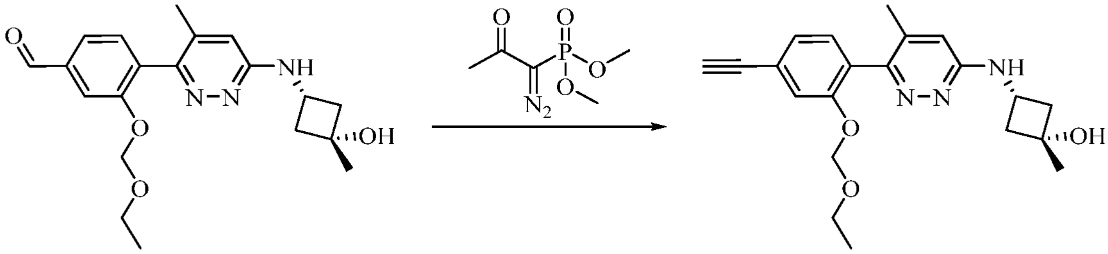

3-(Ethoxymethoxy)-4-(6-(((*cis*)-3-hydroxy-3-methylcyclobutyl)amino)-4-met hylpyridazin-3-yl)benzaldehyde (350 mg, 0.94 mmol, 1.0 eq) was dissolved in methanol (4 mL), and dimethyl (1-diazo-2-oxopropyl)phosphonate (217 mg, 1.13 mmol, 1.2 eq) and potassium carbonate (260 mg, 1.88 mmol, 2.0 eq) were sequentially added. The mixture was stirred at room temperature for 2 h. LC-MS analysis showed the completion of the reaction. The reaction solution was concentrated. Water (5 mL) was added, and extraction was performed with dichloromethane (5 mL × 3). The organic phase was dried and concentrated to give the product (330 mg, yield: 95.5%).

Step 4: Synthesis of intermediate (*cis*)-3-((6-(4-(bromoethynyl)-2-(ethoxymethoxy)phenyl)-5-methylpyridazin-3-yl)amino)-1 -methylcyclobutan-1-ol

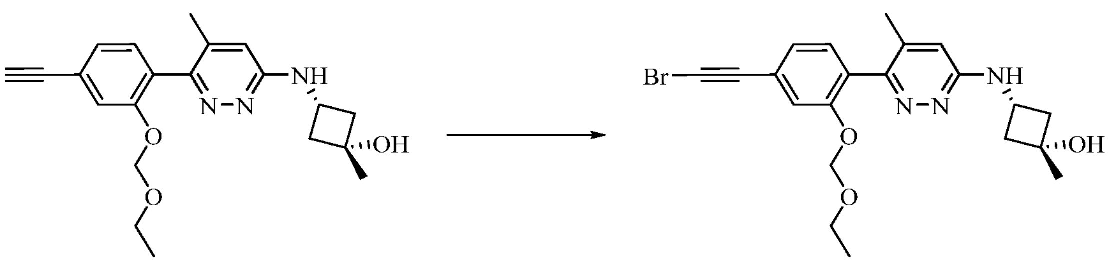

(*cis*)-3-((6-(2-(Ethoxymethoxy)-4-ethynylphenyl)-5-methylpyridazin-3-yl)ami no)-1-methylcyclobutan-1-ol (200 mg, 0.54 mmol, 1.0 eq) was dissolved in acetone (3 mL), and silver nitrate (46 mg, 0.27 mmol, 0.5 eq) and NBS (214 mg, 0.65 mmol, 1.2 eq) were sequentially added. The mixture was stirred at room temperature for 3 h. LC-MS analysis showed

the completion of the reaction. The reaction solution was diluted with dichloromethane and filtered through celite under vacuum. The filtrate was concentrated, and the crude product was purified by preparative thin-layer chromatography (dichloromethane:7% solution of ammonia in methanol = 20:1) to give the product (160 mg, yield: 66.4%).

Step 5: Synthesis of compound 5-(bromoethynyl)-2-(6-(((*cis*)-3-hydroxy-3-methylcyclobutyl)amino)-4-methylpyridazin-3-yl)phenol

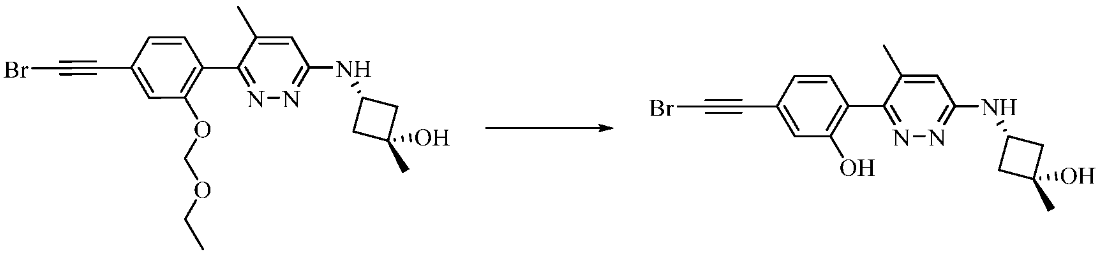

The (*cis*)-3-((6-(4-(bromoethynyl)-2-(ethoxymethoxy)phenyl)-5-methylpyridazin-3-yl)amino)-1-methylcyclobutan-1-ol obtained in the previous step (160 mg, 0.36 mmol, 1.0 eq) was dissolved in dichloromethane (2 mL), and a 4 mol/L solution of hydrogen chloride in 1,4-dioxane (0.45 mL) was added. The mixture was stirred at room temperature for 30 min, and LC-MS analysis showed the completion of the reaction. The reaction solution was poured into water, and the pH was adjusted to 8 with sodium bicarbonate solid, followed by liquid separation and extraction with dichloromethane (5 mL × 2). The organic phase was dried, filtered under vacuum, and concentrated, and the crude product was purified by preparative thin-layer chromatography (dichloromethane:7% solution of ammonia in methanol = 15:1) to give the product (80 mg, yield: 57.2%).

$^1$H-NMR (400 MHz, DMSO-$d_6$) δ(ppm): 10.16 (s, 1H), 7.18-7.17 (m, 1H), 7.04-6.98 (m, 3H), 6.62 (s, 1H), 4.99 (s, 1H), 3.93-3.86 (m, 1H), 2.44-2.39 (m, 2H), 2.03 (s, 3H), 1.98-1.93 (m, 2H), 1.29 (s, 3H).

Molecular formula: $C_{18}H_{18}BrN_3O_2$ Exact molecular weight: 387.06 LC-MS (*m*/*z*): 388.04 $[M+H]^+$.

**Example 6: Synthesis of (*R*)-2-(6-((1-(3-hydroxypropyl)piperidin-3-yl)amino)-4-methylpyridazin-3-yl)-5-(prop-1-yn-1-yl)phenol (Compound 35)**

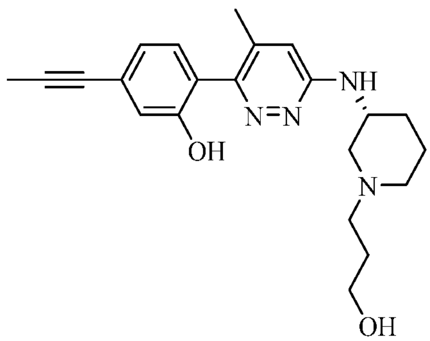

Step 1: Synthesis of *tert-butyl* (*R*)-3-((6-chloro-5-methylpyridazin-3-yl)amino)piperidine-1-carboxylate

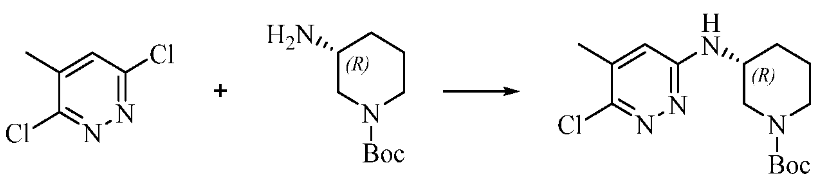

3,6-Dichloro-4-methylpyridazine (5.0 g, 30.67 mmol, 1.0 eq), *tert*-butyl (*R*)-3-aminopiperidine-1-carboxylate (6.1 g, 30.67 mmol, 1.0 eq), and *N,N*-diisopropylethylamine (7.9 g, 61.34 mmol, 2.0 q) were added to *N,N*-dimethylacetamide (20.0 mL). The mixture was stirred at 120 °C for 6 h, and TLC monitoring showed the completion of the reaction. The system was cooled to room temperature. Water (100.0 mL) was added, and extraction was performed with EA (100.0 mL). The organic phase was dried over anhydrous sodium sulfate and filtered. The filtrate was concentrated under reduced pressure, and the crude product was purified by silica gel column chromatography (specification of silica gel: 100-200 mesh, petroleum ether:ethyl acetate = 10:1 to 5:1) to give the product (1.5 g, yield: 15.0%).

Step 2: Synthesis of (*R*)-6-chloro-5-methyl-*N*-(piperidin-3-yl)pyridazin-3-amine

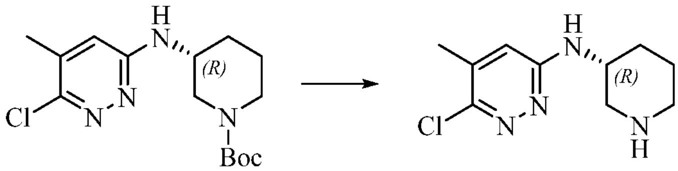

*tert*-Butyl (*R*)-3-((6-chloro-5-methylpyridazin-3-yl)amino)piperidine-1-carboxylate (1.5 g, 4.58 mmol, 1.0 eq) was added to dichloromethane (2.0 mL), and a solution of hydrogen chloride in 1,4-dioxane (4.0 mol/L, 4.0 mL) was added dropwise. The mixture was allowed to react at room temperature for 2 h, and TLC monitoring showed the completion of the reaction. The pH of the system was adjusted to 7-8 with a saturated aqueous sodium bicarbonate solution, and extraction was performed with dichloromethane (100.0 mL). The organic phase was dried over anhydrous sodium sulfate and filtered, and the filtrate was concentrated under reduced pressure to give the product (980.0 mg, yield: 94.5%).

Step 3: Synthesis of (*R*)-6-(2-(ethoxymethoxy)-4-(prop-1-yn-1-yl)phenyl)-5-methyl-*N*-(piperidin-3-yl)pyridazin -3-amine

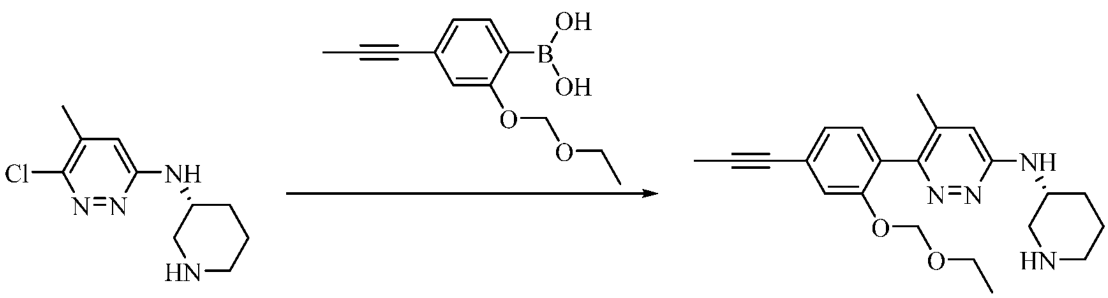

(*R*)-6-Chloro-5-methyl-*N*-(piperidin-3-yl)pyridazin-3-amine (1.40 g, 6.18 mmol, 1.0 eq), (2-(ethoxymethoxy)-4-(prop-1-yn-1-yl)phenyl)boronic acid (1.73 g, 7.42 mmol, 1.2 eq), Pd(dppf)$Cl_2$ (452 mg, 0.618 mmol, 0.1 eq), and $NaHCO_3$ (1.04 g, 12.4 mmol, 2.0 eq) were sequentially added to 1,4-dioxane (30 mL), and $H_2O$ (15 mL) was added. The mixture was heated to 110 °C and allowed to react for 2 h under a nitrogen atmosphere. The reaction mixture was cooled to room temperature, quenched by adding water (50 mL), and extracted with EA (40 mL × 3). The organic phase was dried over anhydrous sodium sulfate and filtered. The filtrate was concentrated under reduced pressure, and the crude product was purified by silica gel column chromatography (DCM:MeOH = 50:1 to 10:1) to give the product (1.82 g, yield: 77.5%).

Step 4: Synthesis of (*R*)-3-(3-((6-(2-(ethoxymethoxy)-4-(prop-1-yn-1-yl)phenyl)-5-methylpyridazin-3-yl)amino) piperidin-1-yl)propan-1-ol

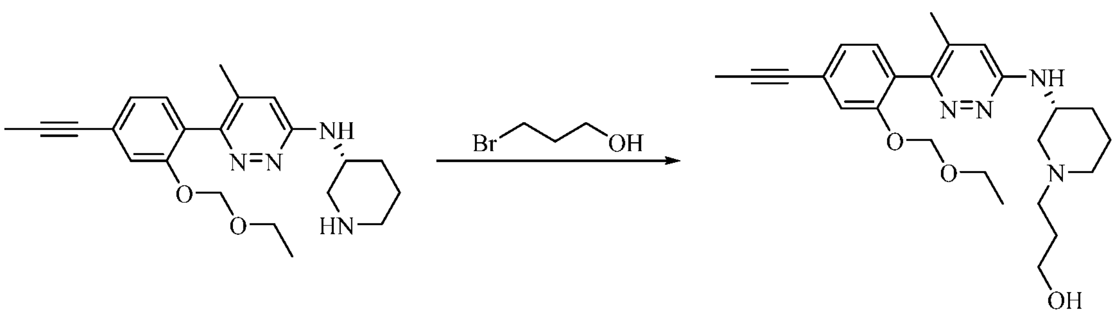


(*R*)-6-(2-(Ethoxymethoxy)-4-(prop-1-yn-1-yl)phenyl)-5-methyl-*N*-(piperidin-3-yl)pyridazin-3-amine (300 mg, 0.788 mmol, 1.0 eq) was dissolved in DCM (10 mL), and triethylamine (353 mg, 3.49 mmol, 5.0 eq) and 3-bromopropanol (548 mg, 3.49 mmol, 5.0 eq) were added. The mixture was stirred at room temperature for 20 h. The reaction mixture was concentrated, and the crude product was purified by silica gel column chromatography (DCM:MeOH = 50:1 to 10:1) to give the product (165 mg, yield: 47.7%).

Step 5: Synthesis of (*R*)-2-(6-((1-(3-hydroxypropyl)piperidin-3-yl)amino)-4-methylpyridazin-3-yl)-5-(prop-1-yn -1-yl) phenol

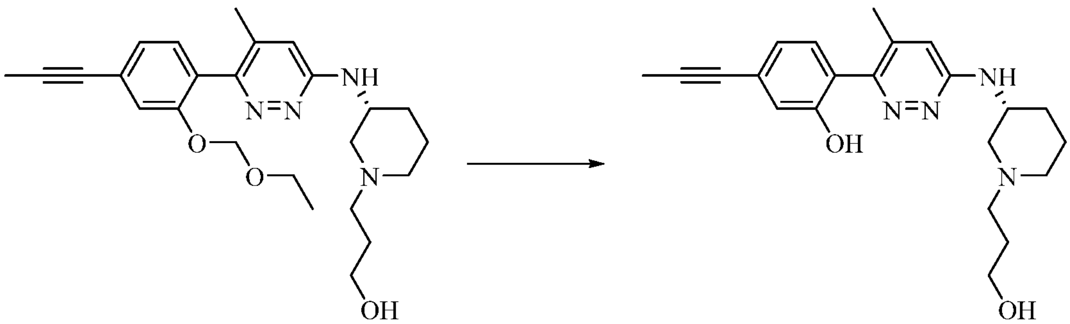

(*R*)-3-(3-((6-(2-(Ethoxymethoxy)-4-(prop-1-yn-1-yl)phenyl)-5-methylpyridazi *n*-3-yl)amino)piperidin-1-yl)propan-1-ol (160 mg, 0.365 mmol, 1.0 eq) was dissolved in DCM (4 mL), and then a solution of hydrogen chloride in 1,4-dioxane (4 mol/L, 0.27 mL, 1.85 mmol, 3.0 eq) was added dropwise. The mixture was stirred at room temperature for 1 h. The pH was adjusted to 8 with a saturated aqueous $NaHCO_3$ solution, and then extraction was performed with DCM (10 mL × 3). The organic phase was dried over anhydrous sodium sulfate and filtered. The filtrate was concentrated under reduced pressure, and the crude product was purified by preparative thin-layer chromatography (DCM:MeOH = 5:1) to give the product (76.0 mg, yield: 54.8%).

$^1$HNMR (400 MHz, DMSO-$d_6$) δ(ppm): 10.07 (s, 1H), 7.12 *(d, J=* 7.8 Hz, 1H), 6.96-6.89 (m, 3H), 6.71 (s, 1H), 4.30 (s, 1H), 3.48-3.45 (m, 3H), 3.35 (s, 4H), 2.95 (s, 2H), 2.05 (s, 3H), 2.03 (s, 3H), 1.94 (s, 2H), 1.79 (s, 3H), 1.51 (s, 1H).

Molecular formula: $C_{22}H_{28}N_4O_2$ Exact molecular weight: 380.22 LC-MS (Pos, m/z) = 381.26 $[M+H]^+$.

**Example 7: Synthesis of (*R*)-2-(6-((1-(2-hydroxy-2-methylpropyl) piperidin-3-yl)amino)-4-methylpyridazin-3-yl)-5-(prop-1-yn-1-yl)phenol (Compound 36)**

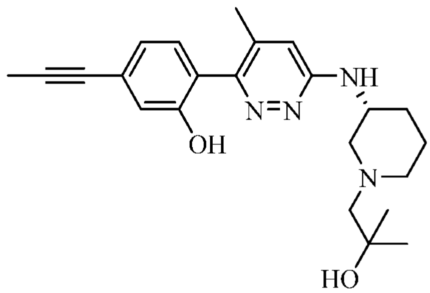

Step 1: Synthesis of (*R*)-1-(3-((6-(2-(ethoxymethoxy)-4-(prop-1-yn-1-yl)phenyl)-5-methylpyridazin-3-yl)amino) piperidin-1-yl)-2-methylpropan-2-ol

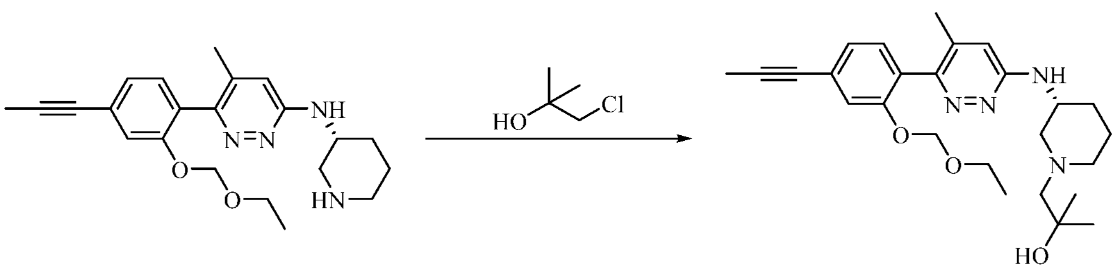

(*R*)-6-(2-(Ethoxymethoxy)-4-(prop-1-yn-1-yl)phenyl)-5-methyl-*N*-(piperidin-3-yl)pyridazin-3-amine (300 mg, 0.788 mmol, 1.0 eq) was dissolved in DMF (10 mL), and $K_2CO_3$ (218 mg, 1.58 mmol, 2.0 eq) and 1-chloro-2-methyl-2-propanol (171 mg, 1.58 mmol, 2.0 eq) were added. The mixture was heated to 90 °C and allowed to react for 20 h. The reaction mixture was quenched by adding water (50 mL) and extracted with EA (20 mL × 2). The organic phase was dried over anhydrous sodium sulfate and filtered. The filtrate was concentrated under reduced pressure, and the crude product was purified by silica gel column chromatography (DCM:MeOH = 100:1 to 20: 1) to give the product (262 mg, yield: 73.4%).

Step 2: Synthesis of (*R*)-2-(6-((1-(2-hydroxy-2-methylpropyl)piperidin-3-yl)amino)-4-methylpyridazin-3-yl)-5-( prop-1-yn-1-yl)phenol

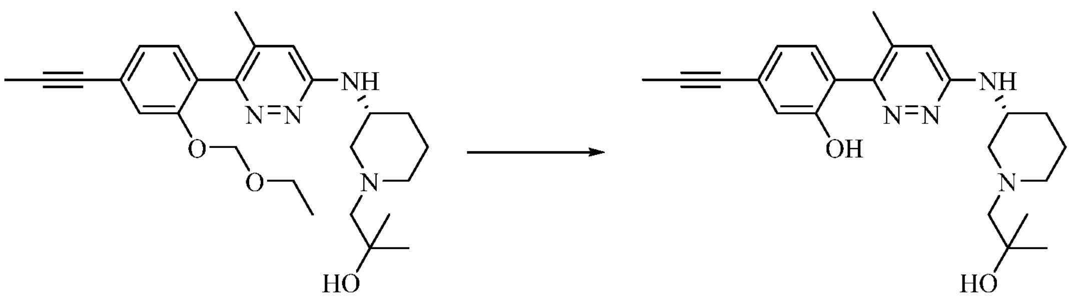

(*R*)-1-(3-((6-(2-(Ethoxymethoxy)-4-(prop-1-yn-1-yl)phenyl)-5-methylpyridazi n-3-yl)amino)piperidin-1-yl)-2-methylpropan-2-ol (260 mg, 0.574 mmol, 1.0 eq) was dissolved in DCM (5 mL), and then a solution of hydrogen chloride in 1,4-dioxane (4 mol/L, 0.43 mL, 1.72 mmol, 3.0 eq) was added dropwise. The mixture was stirred at room temperature for 1 h. The pH was adjusted to 8 with a saturated aqueous $NaHCO_3$ solution, and then extraction was performed with DCM (10 mL × 3). The organic phase was dried over anhydrous sodium sulfate and filtered. The filtrate was concentrated under reduced pressure, and the crude product was purified by silica gel column chromatography (DCM:MeOH = 100:1 to 10:1) to give the product (170 mg, yield: 75.0%).

$^1$HNMR (400 MHz, DMSO-$d_6$) δ(ppm): 10.02 (s, 1H), 7.13 (d, *J* = 8.1 Hz, 1H), 6.90-6.88 (m, 2H), 6.67 (s, 1H), 6.56 (d, *J* = 8.2 Hz, 1H), 4.08 (s, 1H), 4.06-4.03 (m, 1H), 3.00 (d, *J* = 9.0 Hz, 1H), 2.73 (d, *J* = 10.6Hz, 1H), 2.27-2.14 (m, 4H), 2.05

(s, 3H), 2.03 (s, 3H), 1.83-1.80 (m, 1H), 1.70-1.67 (m, 1H), 1.56-1.52 (m, 1H), 1.30-1.24 (m, 1H), 1.09 (s, 6H).

Molecular formula: $C_{23}H_{30}N_4O_2$ Exact molecular weight: 394.24 LC-MS (Pos, m/z) = 395.23 [M+H]$^+$.

**Example 8: Synthesis of 2-(6-(((3*R*)-1-(1-hydroxypropan-2-yl)piperidin-3-yl)amino)-4-methylpyridazin-3-yl)-5-(prop-1-yn-1-yl)phenol (Compound 37)**

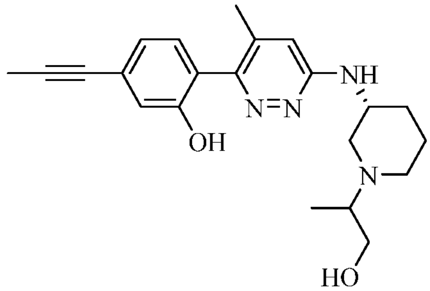

Step 1: Synthesis of 2-((*R*)-3-((6-(2-(ethoxymethoxy)-4-(prop-1-yn-1-yl)phenyl)-5-methylpyridazin-3-yl)amino) piperidin-1-yl)propan-1-ol

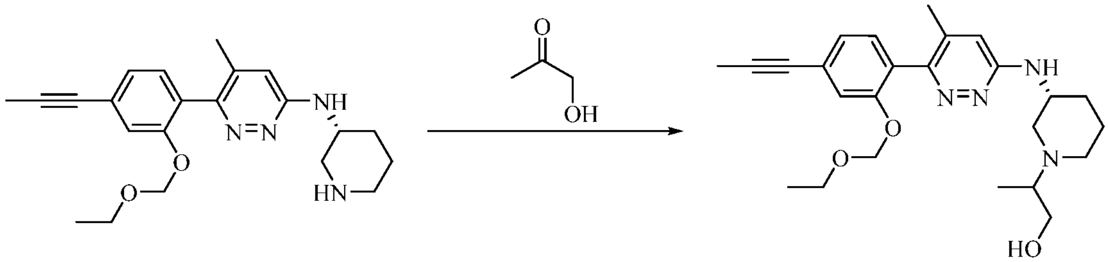

(*R*)-6-(2-(Ethoxymethoxy)-4-(prop-1-yn-1-yl)phenyl)-5-methyl-*N*-(piperidin-3-yl)pyridazin-3-amine (300 mg, 0.788 mmol, 1.0 eq) was dissolved in MeOH (10 mL), and 1-hydroxypropan-2-one (87.4 mg, 1.18 mmol, 1.5 eq) was added. The mixture was allowed to react at room temperature for 0.5 h, and then $NaBH_3CN$ (74.2 mg, 1.18 mmol, 1.5 eq) was added. The resulting mixture was allowed to react at room temperature for 2 h. The reaction mixture was then concentrated. A saturated aqueous $NaHCO_3$ solution was added, and extraction was performed with DCM (20 mL × 3). The organic phase was dried over anhydrous sodium sulfate and filtered. The filtrate was concentrated under reduced pressure, and the crude product was purified by silica gel column chromatography (DCM:MeOH = 50:1 to 10:1) to give the product (192 mg, yield: 55.5%).

Step 2: Synthesis of 2-(6-(((3*R*)-1-(1-hydroxypropan-2-yl)piperidin-3-yl)amino)-4-methylpyridazin-3-yl)-5-(pro p-1-yn-1-yl)phenol

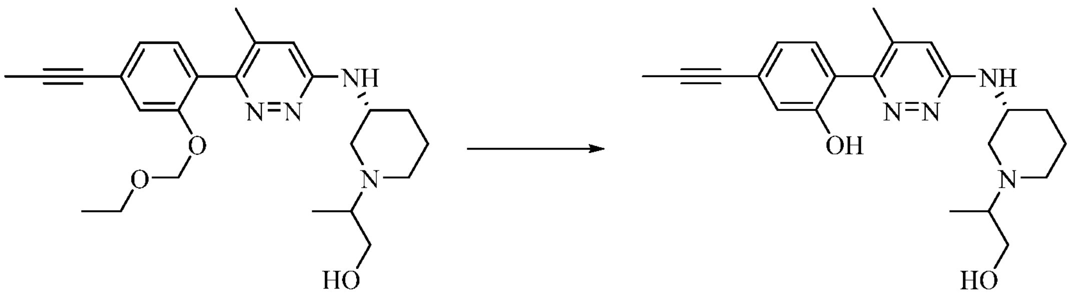

2-((*R*)-3-((6-(2-(Ethoxymethoxy)-4-(prop-1-yn-1-yl)phenyl)-5-methylpyridazi n-3-yl)amino)piperidin-1-yl)propan-1-ol (192 mg, 0.438 mmol, 1.0 eq) was dissolved in DCM (4 mL), and then a solution of hydrogen chloride in 1,4-dioxane (4 mol/L, 0.33 mL, 1.31 mmol, 3.0 eq) was added dropwise. The mixture was stirred at room temperature for 1 h. The pH was adjusted to 8 with a saturated aqueous $NaHCO_3$ solution, and then extraction was performed with DCM (10 mL × 3). The organic phase was dried over anhydrous sodium sulfate and filtered. The filtrate was concentrated under reduced pressure, and the crude product was purified by preparative thin-layer chromatography (DCM:MeOH = 80:1) to give the product (125 mg, yield: 75.0%).

$^1$HNMR(400MHz, DMSO-$d_6$) δ(ppm): 10.03(s, 1H), 7.13(d, *J*=8.2Hz, 1H), 6.90-6.88(m, 2H), 6.68(s, 1H), 6.61-6.56(m, 1H), 4.25(s, 1H), 4.03-4.01 (m, 1H), 3.48-3.41 (m, 1H), 3.26-3.23 (m, 1H), 2.94-2.87 (m, 1H), 2.64-2.63 (m, 2H), 2.23-2.19 (m, 2H), 2.05 (s, 3H), 2.03 (s, 3H), 1.80-1.79(m, 1H), 1.71-1.69(m, 1H), 1.52-1.47 (m, 1H), 1.40-1.34 (m, 1H), 0.91-0.89 (m, 3H).

Molecular formula: $C_{22}H_{28}N_4O_2$ Exact molecular weight: 380.22 LC-MS (Pos, m/z) = 381.22 $[M+H]^+$.

**Example 9: Synthesis of (*R*)-1-(2-(3-((6-(2-hydroxy-4-(prop-1-yn-1-yl)phenyl)-5-methylpyridazin-3-yl)amino)pi peridin-1-yl)ethyl)urea (Compound 38)**

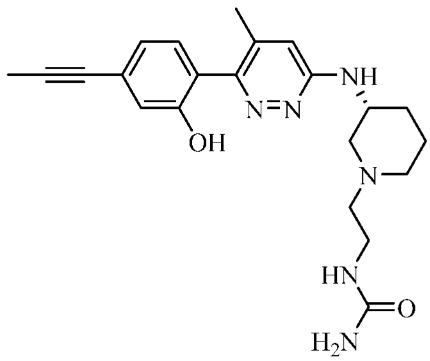

Step 1: Synthesis of (*R*)-1-(2-(3-((6-(2-(ethoxymethoxy)-4-(prop-1-yn-1-yl)phenyl)-5-methylpyridazin-3-yl)ami no)piperidin-1-yl)ethyl)urea

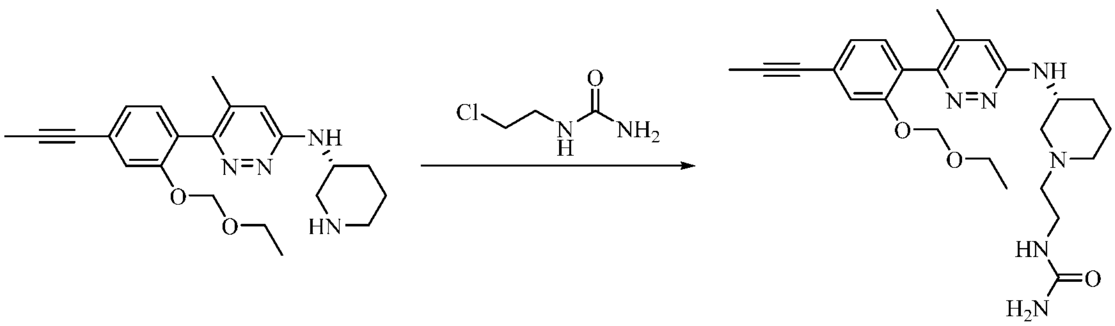

(*R*)-6-(2-(Ethoxymethoxy)-4-(prop-1-yn-1-yl)phenyl)-5-methyl-*N*-(piperidin-3-yl)pyridazin-3-amine (300 mg, 0.788 mmol, 1.0 eq), 1-(2-chloroethyl)urea (194 mg, 1.58 mmol, 2.0 eq), and $K_2CO_3$ (218 mg, 1.58 mmol, 2.0 eq) were added to DMF (10 mL), and the mixture was heated to 90 °C and allowed to react for 20 h. The reaction mixture was quenched by adding water (30 mL) and extracted with DCM (20 mL × 5). The organic phase was dried over anhydrous sodium sulfate and filtered. The filtrate was concentrated under reduced pressure, and the crude product was purified by silica gel column chromatography (DCM:MeOH = 50:1 to 10:1) to give the product (146 mg, yield: 39.7%).

Step 2: Synthesis of (*R*)-1-(2-(3-((6-(2-hydroxy-4-(prop-1-yn-1-yl)phenyl)-5-methylpyridazin-3-yl)amino)piperi din-1-yl) ethyl)urea

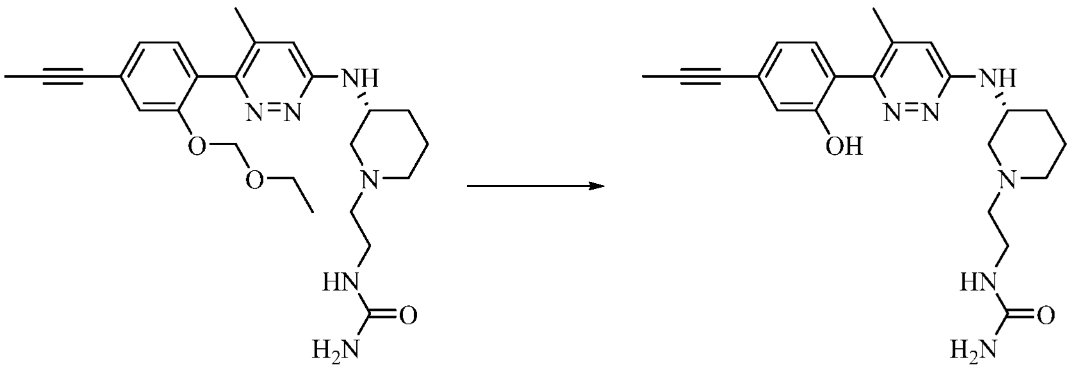

(*R*)-1-(2-(3-((6-(2-(Ethoxymethoxy)-4-(prop-1-yn-1-yl)phenyl)-5-methylpyrid azin-3-yl)amino)piperidin-1-yl) ethyl)urea (146 mg, 0.313 mmol, 1.0 eq) was dissolved in DCM (4 mL), and then a solution of hydrogen chloride in 1,4-dioxane (4 mol/L, 1 mL) was added dropwise. The mixture was stirred at room temperature for 1 h. The pH was adjusted to 8 with a saturated aqueous $NaHCO_3$ solution, and then extraction was performed with DCM (10 mL × 5). The organic phase was dried over anhydrous sodium sulfate and filtered. The filtrate was concentrated under reduced pressure, and the crude product was purified by preparative thin-layer chromatography (DCM:MeOH = 6:1) to give the product (62.0 mg, yield: 48.5%).

$^1$HNMR(400MHz, DMSO-*d*$_6$)δ(ppm): 10.03(s, 1H), 7.14 (d, *J*=7.8Hz, 1H), 6.90-6.88 (m, 2H), 6.67 (s, 1H), 6.58 (d, *J*=7.8Hz, 1H), 5.88-5.86 (m, 1H), 5.49 (s, 2H), 4.05-4.03 (m, 1H), 3.14-3.04 (m, 2H), 2.92 (d, *J* = 8.8 Hz, 1H), 2.66 (d, *J*=10.0Hz, 1H), 2.36-2.32 (m, 2H), 2.05 (s, 4H), 2.03 (s, 4H), 1.87-1.84 (m, 1H), 1.72-1.69 (m, 1H), 1.55-1.52 (m, 1H), 1.40-1.34 (m, 1H).

Molecular formula: $C_{22}H_{28}N_6O_2$ Exact molecular weight: 408.23 LC-MS (Pos, m/z) = 409.23 $[M+H]^+$.

**Example 10: Synthesis of (*R*)-3-((6-(2-hydroxy-4-(prop-1-yn-1-yl)phenyl)-5-methylpyridazin-3-yl)amino)-1,1-di methylpiperidinium chloride (Compound 49)**

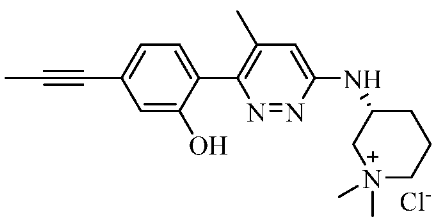

Step 1: Synthesis of (R)-3-((6-(2-(ethoxymethoxy)-4-(prop-1-yn-1-yl)phenyl)-5-methylpyridazin-3-yl)amino)-1, 1-dimethylpiperidinium iodide

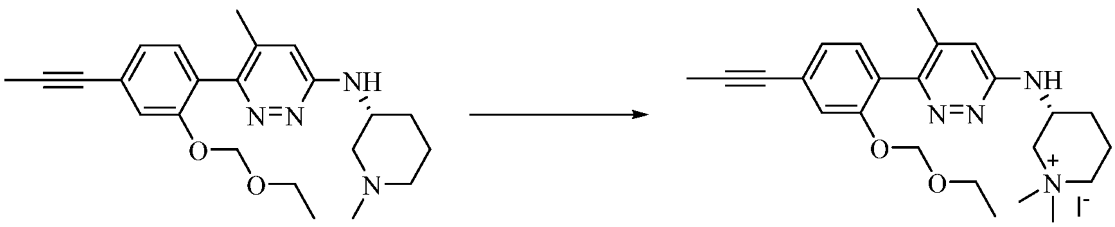

(*R*)-6-(2-(Ethoxymethoxy)-4-(prop-1-yn-1-yl)phenyl)-5-methyl-*N*-(1-methylpi peridin-3-yl)pyridazin-3-amine (120 mg, 0.304 mmol, 1.0 eq) was dissolved in DCM (3 mL), and iodomethane (216 mg, 1.52 mmol, 5.0 eq) was added. The mixture was allowed to react at room temperature for 5 days. The reaction mixture was concentrated, and the crude product was purified by preparative reversed phase chromatography (ACN:$H_2O$ = 3:7) to give the product (115 mg, yield: 70.5%).

Step 2: Synthesis of (*R*)-3-((6-(2-hydroxy-4-(prop-1-yn-1-yl)phenyl)-5-methylpyridazin-3-yl)amino)-1,1-dimeth ylpiperidinium chloride

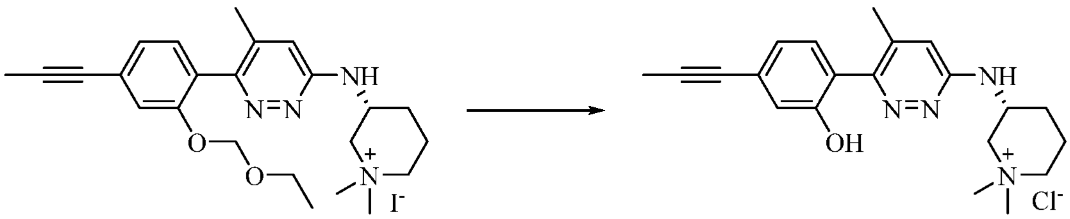

(*R*)-3-((6-(2-(Ethoxymethoxy)-4-(prop-1-yn-1-yl)phenyl)-5-methylpyridazin-3-yl)amino)-1,1-dimethylpiperidinium iodide (105 mg, 0.196 mmol, 1.0 eq) was added to DCM (2 mL), and then a solution of hydrogen chloride in 1,4-dioxane (4 mol/L, 0.15 mL, 3.0 eq) was added dropwise. The mixture was stirred at room temperature for 1 h. The reaction mixture was concentrated, and the crude product was purified by preparative thin-layer chromatography (DCM:MeOH = 5:1) to give the product (28.0 mg, yield: 37.0%).

$^1$HNMR (400 MHz, DMSO-$d_6$) δ(ppm): 11.10 (s, 1H), 8.71 (s, 1H), 7.61 (s, 1H), 7.36 (d, *J* = 7.9 Hz, 1H), 7.22 (d, *J* = 1.0 Hz, 1H), 7.07-7.05 (m, 1H), 4.48 (s, 2H), 4.12 (s, 1H), 3.99 (s, 3H), 3.39 (s, 4H), 2.08-2.06 (s, 3H), 2.06 (s, 3H), 1.88 (s, 2H), 1.73 (s, 1H), 1.52 (s, 1H).

Molecular formula: $C_{21}H_{27}ClN_4O$ Exact molecular weight: 386.19 Cationic molecular weight: 351.22
LC-MS (Pos, m/z) = 351.25 $[M+H]^+$.

**Example 11: Synthesis of (*R*)-5-ethynyl-3-methyl-2-(6-((1-methylpiperidin-3-yl)amino)pyridazin-3-yl)phenol (Compound 41)**

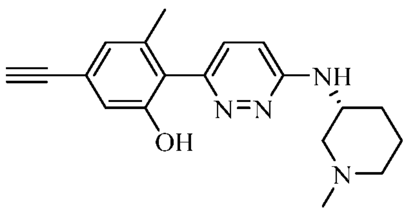

Step 1: Synthesis of intermediate methyl 4-amino-3-iodo-5-methylbenzoate

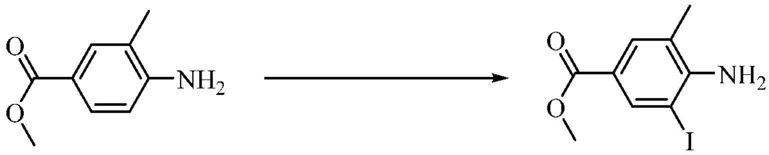

Methyl 4-amino-3-methylbenzoate (20.0 g, 121.07 mmol, 1.0 eq) was dissolved in *N,N*-dimethylformamide (200 mL), and N-iodosuccinimide (29.94 g, 133.18 mmol, 1.1 eq) was added in portions to the solution. After the addition, the mixture was allowed to react at 25 °C for 30 min, and TLC analysis showed the completion of the reaction. The reaction solution was poured into water (300 mL) and extracted with methyl *tert*-butyl ether (200 mL × 3). The organic phases were combined, dried over anhydrous sodium sulfate, and concentrated, and the crude product was purified by silica gel column chromatography (petroleum ether:ethyl acetate = 50:1 to 40:1) to give the product (21.3 g, yield: 60.4%).

Step 2: Synthesis of intermediate methyl 4-bromo-3-iodo-5-methylbenzoate

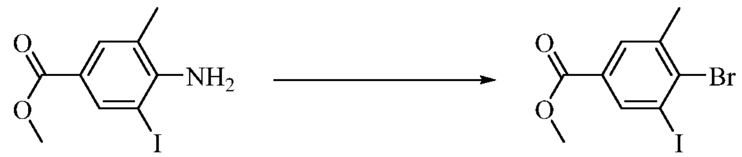

Methyl 4-amino-3-iodo-5-methylbenzoate (19.22 g, 66.03 mmol, 1.0 eq) was dissolved in hydrobromic acid (100 mL), and an aqueous solution (100 mL) of sodium nitrite (5.01 g, 72.63 mmol, 1.1 eq) was added dropwise and slowly to the solution at 0 °C. After the addition, the mixture was allowed to react at 0 °C for 30 min. A solution of cuprous bromide (11.37 g, 79.24 mmol, 1.2 eq) in hydrobromic acid (100 mL) was added dropwise to the reaction mixture. After the addition, the resulting mixture was allowed to react at 0 °C for 10 min, and TLC analysis showed the completion of the reaction. The reaction solution was added dropwise into ice water (400 mL) and extracted with ethyl acetate (100 mL × 3). The organic phases were combined, dried over anhydrous sodium sulfate, and concentrated, and the crude product was purified by silica gel column chromatography (petroleum ether:ethyl acetate = 50:1 to 20:1) to give the product (16.2 g, yield: 70.9%).

Step 3: Synthesis of intermediate 4-bromo-3-hydroxy-5-methylbenzoic acid

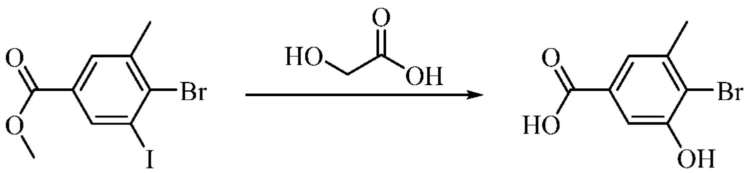

Methyl 4-bromo-3-iodo-5-methylbenzoate (12.0 g, 34.68 mmol, 1.0 eq) was dissolved in DMSO (60 mL), and 2-hydroxyacetic acid (791 mg, 10.40 mmol, 0.3 eq), copper hydroxide (338 mg, 3.468 mmol, 0.1 eq), and an aqueous solution (60 mL) of sodium hydroxide (8.32 g, 208.08 mmol, 6.0 eq) were sequentially added to the solution. The mixture was allowed to react at 120 °C for 6 h under a nitrogen atmosphere, and TLC analysis showed the completion of the reaction. The reaction solution was poured into ice water (120 mL). The pH was adjusted to 1 with 2 mol/L hydrochloric acid, and extraction was performed with methyl *tert*-butyl ether (100 mL × 3). The organic phases were combined, dried over anhydrous sodium sulfate, and concentrated to give a crude product, which was directly used in the next step.

Step 4: Synthesis of intermediate methyl 4-bromo-3-hydroxy-5-methylbenzoate

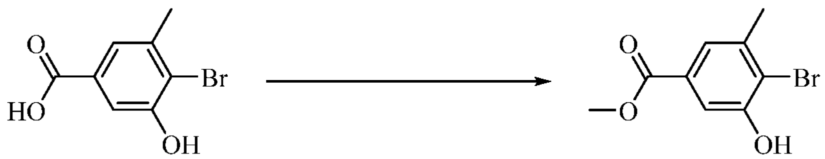

The crude product of 4-bromo-3-hydroxy-5-methylbenzoic acid (34.68 mmol) was dissolved in methanol (80 mL), and thionyl chloride (40 mL) was added dropwise and slowly to the solution. After the dropwise addition, TLC analysis showed the substantial completion of the reaction. The reaction solution was concentrated, and the crude product was purified by silica gel column chromatography (petroleum ether:ethyl acetate = 50:1 to 20:1) to give the product (4.18 g, two-step yield: 49.2%).

Step 5: Synthesis of intermediate methyl 4-bromo-3-ethoxymethoxy-5-methylbenzoate

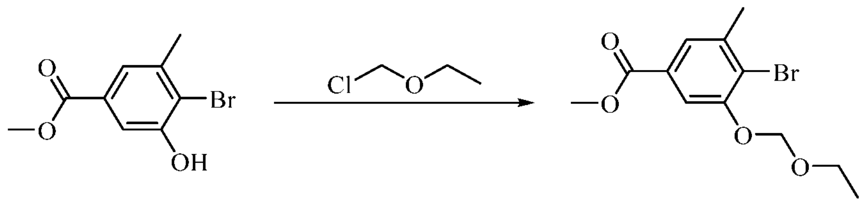

Methyl 4-bromo-3-hydroxy-5-methylbenzoate (4.18 g, 17.06 mmol, 1.0 eq) was dissolved in tetrahydrofuran (40 mL), and sodium hydride (1.03 g, 25.59 mmol, 1.5 eq) was added in portions to the solution at 0 °C. The mixture was allowed to react at 0 °C for 30 min, and chloromethyl ethyl ether (2.52 g, 25.59 mmol, 1.5 eq) was added dropwise and slowly to the reaction solution. After the addition, the mixture was allowed to react at 0 °C for 5 min, and TLC analysis showed the completion of the reaction. The reaction solution was poured into a saturated aqueous ammonium chloride solution (50 mL) and extracted with methyl *tert*-butyl ether (40 mL × 3). The organic phases were combined, dried, and concentrated to give the product (5.17 g, crude product, yield: 100%), which was directly used in the next step.

Step 6: Synthesis of intermediate (4-bromo-3-(ethoxymethoxy)-5-methylphenyl)methanol

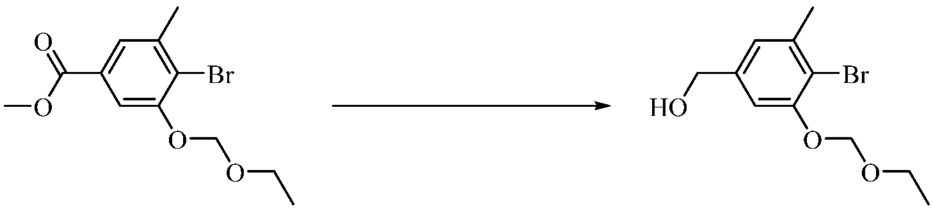

Methyl 4-bromo-3-ethoxymethoxy-5-methylbenzoate (3.67 g, 12.11 mmol, 1.0 eq) was dissolved in tetrahydrofuran (30 mL), and a 1.5 mol/L solution of diisobutyl aluminum hydride in toluene (25 mL, 36.33 mmol, 3.0 eq) was added dropwise and slowly to the solution at -78 °C. After the addition, the mixture was warmed to 25 °C and allowed to react for 30 min, and TLC analysis showed the completion of the reaction. The reaction solution was cooled to 0 °C and diluted with methyl *tert*-butyl ether (30 mL). Water (1.5 mL), a 15% aqueous sodium hydroxide solution (1.5 mL), and water (3.6 mL) were sequentially added dropwise to quench the reaction. The mixture was warmed to 25 °C, stirred for 15 min, dried over anhydrous magnesium sulfate, further stirred for 15 min, and filtered through celite. The filtrate was concentrated to give a crude product, which was directly used in the next step.

Step 7: Synthesis of intermediate 4-bromo-3-ethoxymethoxy-5-methylbenzaldehyde

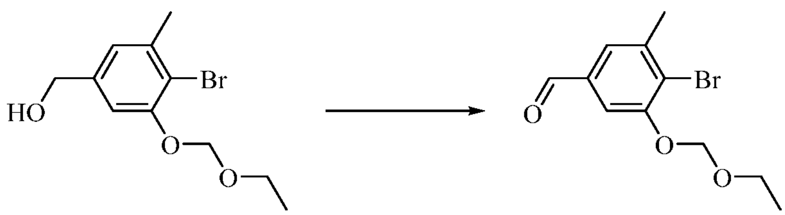

(4-Bromo-3-(ethoxymethoxy)-5-methylphenyl)methanol (crude, 12.11 mmol, 1.0 eq) was dissolved in dichloromethane (40 mL), and manganese dioxide (10.53 g, 121.1 mmol, 10.0 eq) was added to the solution. The mixture was allowed to react at room temperature of 25 °C for 5 h, and TLC analysis showed the completion of the reaction. The reaction solution was filtered through celite and concentrated to give a crude product, which was directly used in the next step.

Step 8: Synthesis of intermediate 3-ethoxymethoxy-5-methyl-4-(4,4,5,5-tetramethyl-1,3,2-dioxaborolan-2-yl)benzaldehyde

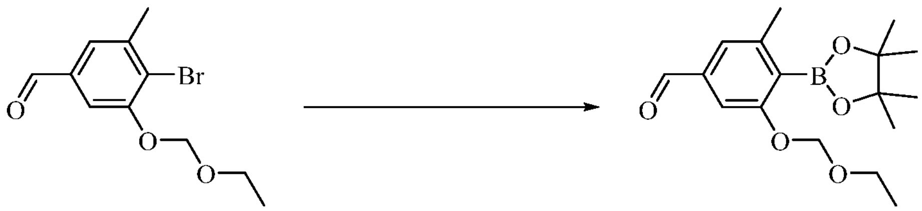

The crude product of 4-bromo-3-ethoxymethoxy-5-methylbenzaldehyde (12.11 mmol, 1.0 eq) was dissolved in 1,4-dioxane (35 mL), and bis(pinacolato)diboron (4.61 g, 18.17 mmol, 1.5 eq), potassium acetate (2.38 g, 24.22 mmol, 2.0 eq), and $PdCl_2$(dppf) (886 mg, 1.211 mmol, 0.1 eq) were sequentially added to the solution. The mixture was heated to 110 °C and allowed to react for 13 h under a nitrogen atmosphere, and TLC analysis showed the completion of the reaction. The reaction solution was filtered through celite and concentrated, and the crude product was purified by silica gel column chromatography (petroleum ether:ethyl acetate = 90:1 to 5:1) to give the product (1.36 g, three-step yield: 35.8%).

Step 9: Synthesis of intermediate (*R*)-3-ethoxymethoxy-5-methyl-4-(6-(1-methylpiperidin-3-amino)pyridazin-3-yl)benzaldeh yde

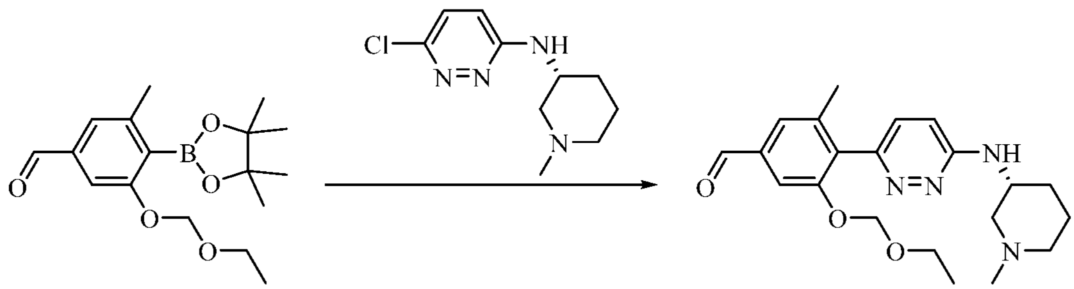

6-Chloro-*N*-(1-methylpiperidin-3-yl)pyridazin-3-amine (400 mg, 1.76 mmol, 1.0 eq) was dissolved in 1,4-dioxane (10 mL), and 3-ethoxymethoxy-5-methyl-4-(4,4,5,5-tetramethyl-1,3,2-dioxaborolan-2-yl)benzaldehyde (788 mg, 2.46 mmol, 1.4 eq), an aqueous solution (2 mL) of potassium carbonate (487 mg, 3.52 mmol, 2.0 eq), and $PdCl_2$(dppf) (322 mg, 0.44 mmol, 0.25 eq) were sequentially added to the solution. The mixture was heated to 95 °C and allowed to react for 13 h under a nitrogen atmosphere, and TLC analysis showed the completion of the reaction. The reaction solution was filtered through celite and concentrated, and the crude product was purified by silica gel column chromatography (dichloromethane:methanol = 100:1 to 10:1) to give the product (265 mg, yield: 39.1%).

Step 10: Synthesis of intermediate (*R*)-6-(2-(ethoxymethoxy)-4-ethynyl-6-methylphenyl)-*N*-(1-methylpiperidin-3-yl)pyridazin -3-amine

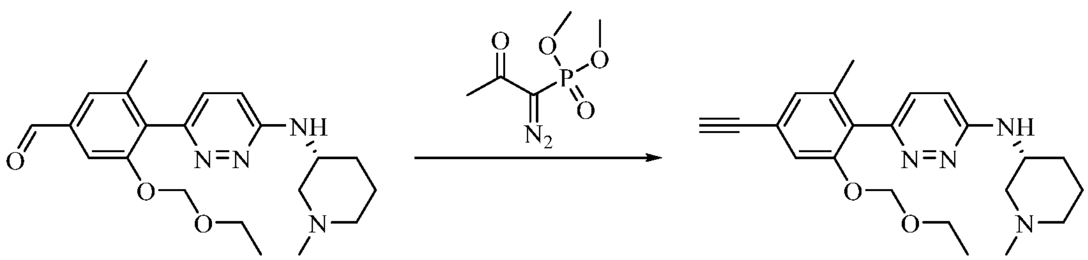

(*R*)-3-Ethoxymethoxy-5-methyl-4-(6-(1-methylpiperidin-3-amino)pyridazin-3 -yl)benzaldehyde (265 mg, 0.689 mmol, 1.0 eq) was dissolved in methanol (3 mL), and potassium carbonate (191 mg, 1.378 mmol, 2.0 eq) and dimethyl (1-diazo-2-oxopropyl)phosphonate (199 mg, 1.034 mmol, 1.5 eq) were sequentially added to the solution. The mixture was allowed to react at 25 °C for 0.5 h, and TLC analysis showed the completion of the reaction. The reaction solution was concentrated, and the crude product was purified by silica gel column chromatography (dichloromethane:methanol = 50:1 to 10:1) to give the product (230 mg, yield: 87.8%).

Step 11: Synthesis of compound (*R*)-5-ethynyl-3-methyl-2-(6-((1-methylpiperidin-3-yl)amino)pyridazin-3-yl)phenol

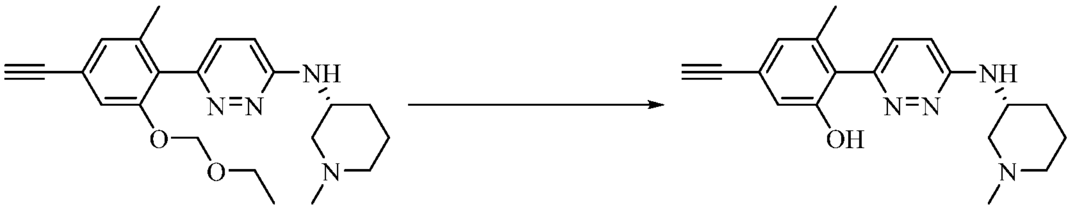

(*R*)-3-Ethoxymethoxy-5-methyl-4-(6-(1-methylpiperidin-3-amino)pyridazin-3 -yl)benzaldehyde (230 mg, 0.605 mmol, 1.0 eq) was dissolved in dichloromethane (3 mL), and the solution was added dropwise and slowly to a 4 mol/L solution of hydrogen chloride in 1,4-dioxane (3 mL). The mixture was allowed to react at 25 °C for 0.5 h, and TLC analysis showed the completion of the reaction. The reaction solution was concentrated. A saturated aqueous sodium bicarbonate solution (10 mL) was added to the concentrate, and extraction was performed with dichloromethane (10 mL × 6). The organic phases were combined, dried over anhydrous magnesium sulfate, and concentrated, and the crude product was purified by preparative thin-layer chromatography (dichloromethane:methanol = 10:1) to give the product (91 mg, yield: 46.7%).

$^1$H-NMR (400 MHz, *D*MSO-$d_6$) δ(ppm): 9.86 (s, 1H), 7.25-7.23 (d, 1H), 7.01 (s, 1H), 6.91-6.88 (m, 3H), 4.25-4.24 (s, 1H), 4.13 (s, 1H), 3.18-3.17 (m, 1H), 2.98 (m, 1H), 2.54 (s, 3H), 2.05 (s, 3H), 1.92-1.86 (m, 2H), 1.73-1.70 (m, 1H), 1.46 (m, 1H), 1.26-1.24 (m, 2H).

Molecular formula: $C_{19}H_{22}N_4O$ Exact molecular weight: 322.18 LC-MS(*m*/*z*): 323.15 $[M+H]^+$.

**Example 12: Synthesis of 2-(6-(((1*S*,2*R*)-2-hydroxycyclohexyl)amino)-4-methylpyridazin-3-yl)-5-(prop-1-yn-1-yl) phenol (Compound 43)**

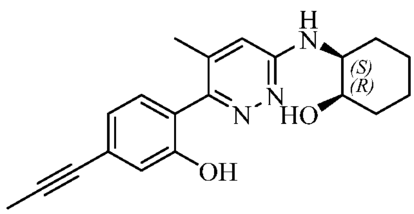

Step 1: Synthesis of (1*R*,2*S*)-2-((6-chloro-5-methylpyridazin-3-yl)amino)cyclohexan-1-ol

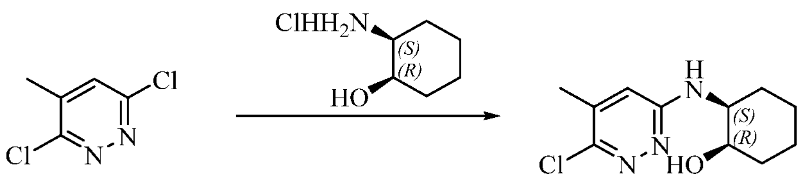

3,6-Dichloro-4-methylpyridazine (4.5 g, 27.61 mmol, 1.0 eq), (1*R*,2*S*)-2-aminocyclohexan-1-ol hydrochloride (5.0 g, 33.12 mmol, 1.2 eq), and *N,N*-diisopropylethylamine (7.13 g, 55.22 mmol, 2.0 eq) were added to *N,N*-dimethylacetamide (15.0 mL). The mixture was stirred at 120 °C for 48 h, and TLC monitoring showed that the reaction was not completed. The reaction mixture was cooled to room temperature. Water (100.0 mL) was added, and extraction was performed with EA (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane: methanol = 130:1 to 60:1) to give the product (1.2 g, yield: 18.0%).

Step 2: Synthesis of (1*R*,2*S*)-2-((6-(2-(ethoxymethoxy)-4-(prop-1-yn-1-yl)phenyl)-5-methylpyridazin-3-yl)amin o)cyclohexan-1-ol

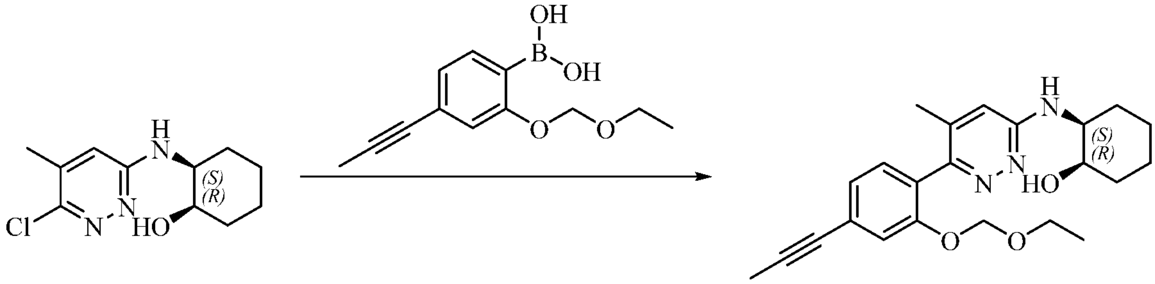

(1*R*,2*S*)-2-((6-Chloro-5-methylpyridazin-3-yl)amino)cyclohexan-1-ol (320.0 mg, 1.32 mmol, 1.0 eq), (2-(ethoxymethoxy)-4-(prop-1-yn-1-yl)phenyl)boronic acid (371.8 mg, 1.58 mmol, 1.2 eq), sodium bicarbonate (222.2 mg, 2.64 mmol, 2.0 eq), and Pd(dppf)$Cl_2$ (96.8 mg, 0.13 mmol, 0.1 eq) were added to a mixed solution of 1,4-dioxane (10.0 mL) and water (5.0 mL). The mixture was allowed to react at 110 °C for 3 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane:methanol = 100:1 to 60:1) to give the product (400.0 mg, yield: 76.4%).

Step 3: Synthesis of 2-(6-(((1*S*,2*R*)-2-hydroxycyclohexyl)amino)-4-methylpyridazin-3-yl)-5-(prop-1-yn-1-yl)ph enol

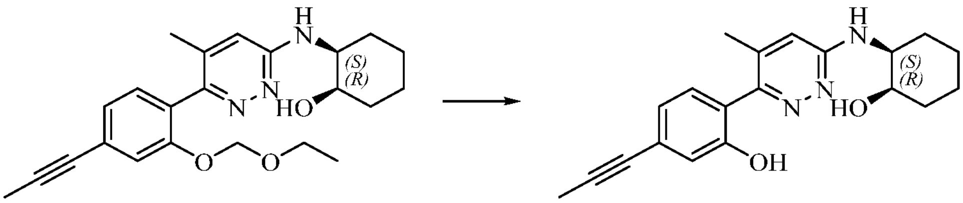

(1*R*,2*S*)-2-((6-(2-(Ethoxymethoxy)-4-(prop-1-yn-1-yl)phenyl)-5-methylpyrida zin-3-yl)amino)cyclohexan-1-ol (390.0 mg, 0.98 mmol, 1.0 eq) was added to dichloromethane (4.0 mL), and a solution of hydrogen chloride in 1,4-dioxane (4.0 mol/L, 4.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane: methanol = 100:1 to 60:1) to give the product (130.0 mg, yield: 39.1%).

$^1$HNMR (400 MHz, DMSO-$d_6$) δ(ppm): 10.06 (s, 1H), 7.13-7.11 (d, *J*=8 Hz, 1H), 6.90-6.88 (m, 2H), 6.77 (s, 1H), 6.32-6.30 (d, *J*=8 Hz, 1H), 4.67-4.66 (d, *J*=4 Hz, 1H), 3.98-3.90 (m, 2H), 2.05 (s, 3H), 2.02 (s, 3H), 1.75-1.47 (m, 6H), 1.33-1.24 (m, 2H).

Molecular formula: $C_{20}H_{23}N_3O_2$ Exact molecular weight: 337.18 LC-MS (Pos, m/z) = 338.15 $[M+H]^+$.

**Example 13: Synthesis of (*R*)-2-(4-cyclopropyl-6-((1-(2-hydroxyethyl)piperidin-3-yl)amino)pyridazin-3-yl)-5-(pr op-1-yn-1-yl)phenol (Compound 44)**

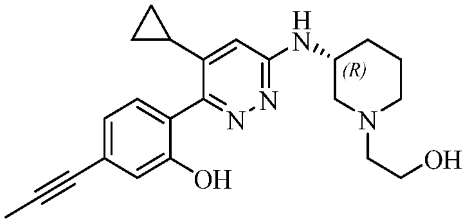

Step 1: Synthesis of (*R*)-2-(3-((6-chloro-5-cyclopropylpyridazin-3-yl)amino)piperidin-1-yl)ethan-1-ol

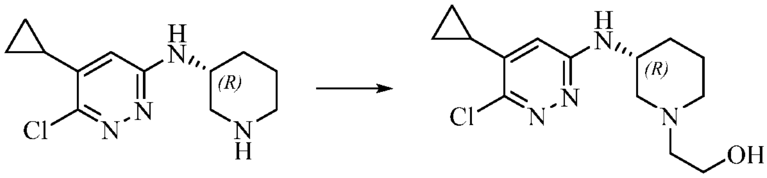

(*R*)-6-Chloro-5-cyclopropyl-*N*-(piperidin-3-yl)pyridazin-3-amine (310.0 mg, 1.22 mmol, 1.0 eq), bromoethanol (459.7 mg, 3.67 mmol, 3.0 eq), and triethylamine (372.2 mg, 3.67 mmol, 3.0 eq) were added to dichloromethane (10.0 mL). The mixture was stirred at room temperature for 24 h, and TLC monitoring showed the completion of the reaction. The reaction mixture was concentrated under reduced pressure, and the crude product was purified by silica gel column chromatography (specification of silica gel: 100-200 mesh, dichloromethane: methanol = 40:1 to 10:1) to give the product (300.0 mg, yield: 81.4%).

Step 2: Synthesis of (*R*)-2-(3-((5-cyclopropyl-6-(2-(ethoxymethoxy)-4-(prop-1-yn-1-yl)phenyl)pyridazin-3-yl)a mino)piperidin-1-yl)ethan-1-ol

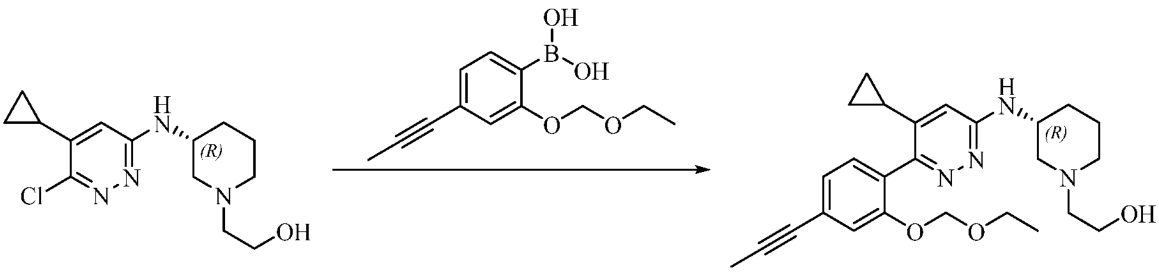

(*R*)-2-(3-((6-Chloro-5-cyclopropylpyridazin-3-yl)amino)piperidin-1-yl)ethan-1-ol (295.0 mg, 0.99 mmol, 1.0 eq), (2-(ethoxymethoxy)-4-(prop-1-yn-1-yl)phenyl)boronic acid (279.1 mg, 1.19 mmol, 1.2 eq), sodium bicarbonate (166.9 mg, 1.98 mmol, 2.0 eq), and Pd(dppf)$Cl_2$ (72.4 mg, 0.09 mmol, 0.1 eq) were added to a mixed solution of 1,4-dioxane (10.0 mL) and water (5.0 mL). The mixture was allowed to react at 110 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane:methanol = 50:1 to 10:1) to give the product (234.0 mg, yield: 52.3%).

Step 3: Synthesis of (*R*)-2-(4-cyclopropyl-6-((1-(2-hydroxyethyl)piperidin-3-yl)amino)pyridazin-3-yl)-5-(prop-1 -yn-1-yl) phenol

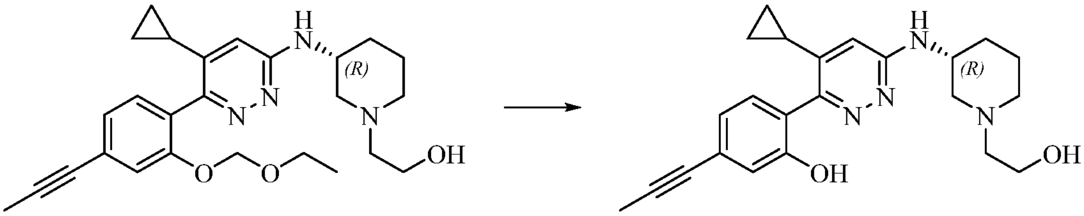

(*R*)-2-(3-((5-Cyclopropyl-6-(2-(ethoxymethoxy)-4-(prop-1-yn-1-yl)phenyl)pyr idazin-3-yl)amino)piperidin-1-yl) ethan-1-ol (234.0 mg, 0.51 mmol, 1.0 eq) was added to dichloromethane (4.0 mL), and a solution of hydrogen chloride in 1,4-dioxane (4.0 mol/L, 4.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by thin-layer chromatography (dichloromethane:methanol = 10:1) to give the product (80.0 mg, yield: 39.4%).

$^1$HNMR (400 MHz, DMSO-*d*$_6$) δ(ppm): 9.99 (s, 1H), 7.20-7.18 (d, *J*=8 Hz, 1H), 6.91-6.89 (m, 2H), 6.51 (s, 1H), 6.31 (s, 1H), 4.43-4.36 (m, 1H), 4.07 (s, 1H), 3.52-3.51 (m, 2H), 2.94 (s, 1H), 2.67-2.62 (m, 1H), 2.40-2.46 (m, 2H), 2.12-2.05 (m, 5H), 1.80-1.24 (m, 5H), 0.89-0.84 (m, 2H), 0.63-0.59 (m, 2H).

Molecular formula: $C_{23}H_{28}N_4O_2$ Exact molecular weight: 392.22 LC-MS (Pos, m/z) = 393.20 $[M+H]^+$.

**Example 14: Synthesis of 2-(6-(((1*R*,2*S*)-2-hydroxycyclohexyl)amino)-4-methylpyridazin-3-yl)-5-(prop-1-yn-1-yl) phenol (Compound 48)**

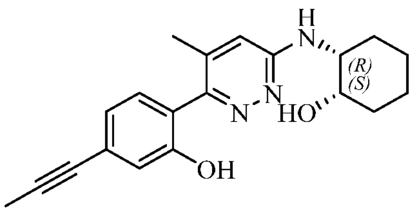

Step 1: Synthesis of (1*S*,2*R*)-2-((6-chloro-5-methylpyridazin-3-yl)amino)cyclohexan-1-ol

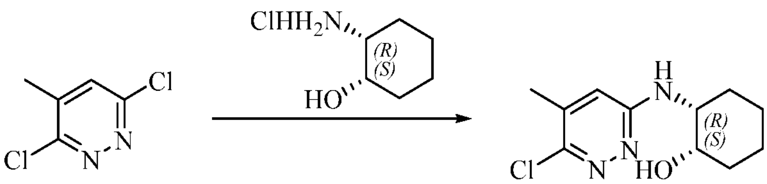

3,6-Dichloro-4-methylpyridazine (3.8 g, 23.31 mmol, 1.0 eq), (1*S*,2*R*)-2-aminocyclohexan-1-ol hydrochloride (4.59 g, 30.30 mmol, 1.3 eq), and *N,N*-diisopropylethylamine (6.0 g, 46.62 mmol, 2.0 eq) were added to *N,N*-dimethylacetamide (15.0 mL). The mixture was stirred at 120 °C for 48 h, and TLC monitoring showed that the reaction was not completed. The reaction mixture was cooled to room temperature. Water (100.0 mL) was added, and extraction was performed with EA (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane: methanol = 130:1 to 50:1) to give the product (864.0 mg, yield: 15.4%).

Step 2: Synthesis of (1*S*,2*R*)-2-((6-(2-(ethoxymethoxy)-4-(prop-1-yn-1-yl)phenyl)-5-methylpyridazin-3-yl)amin o)cyclohexan-1-ol

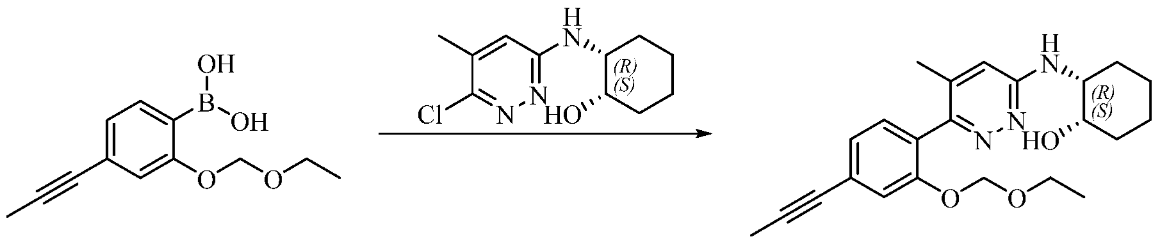

(1*S*,2*R*)-2-((6-Chloro-5-methylpyridazin-3-yl)amino)cyclohexan-1-ol (300.0 mg, 1.24 mmol, 1.0 eq), (2-(ethoxymethoxy)-4-(prop-1-yn-1-yl)phenyl)boronic acid (348.6 mg, 1.48 mmol, 1.2 eq), sodium bicarbonate (208.5 mg, 2.48 mmol, 2.0 eq), and Pd(dppf)$Cl_2$ (90.8 mg, 0.12 mmol, 0.1 eq) were added to a mixed solution of 1,4-dioxane (10.0 mL) and water (5.0 mL). The mixture was allowed to react at 110 °C for 3 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure to give the product (630.0 mg, crude product), which was directly used in the next step.

Step 3: Synthesis of 2-(6-(((1*R*,2*S*)-2-hydroxycyclohexyl)amino)-4-methylpyridazin-3-yl)-5-(prop-1-yn-1-yl)ph enol

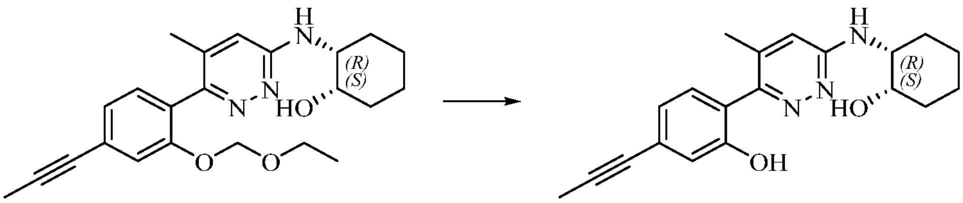

(1*S*,2*R*)-2-((6-(2-(Ethoxymethoxy)-4-(prop-1-yn-1-yl)phenyl)-5-methylpyrida zin-3-yl)amino)cyclohexan-1-ol (630.0 mg, crude product, 1.24 mmol, 1.0 eq) was added to dichloromethane (4.0 mL), and a solution of hydrogen chloride in 1,4-dioxane (4.0 mol/L, 4.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (100.0 mg, yield: 23.9%).

$^1$HNMR (400 MHz, DMSO-$d_6$) δ(ppm): 10.05 (s, 1H), 7.13-7.11 (d, *J*=8 Hz, 1H), 6.90-6.88 (m, 2H), 6.77 (s, 1H), 6.33-6.31 (d, *J*=8 Hz, 1H), 4.67-4.66 (d, *J*=4 Hz, 1H), 3.98-3.90 (m, 2H), 2.05 (s, 3H), 2.02 (s, 3H), 1.75-1.46 (m, 6H), 1.33-1.24 (m, 2H).

Molecular formula: $C_{20}H_{23}N_3O_2$ Exact molecular weight: 337.18 LC-MS (Pos, m/z) = 338.17 $[M+H]^+$.

**Example 15: Synthesis of (*R*)-5-(2,2-difluorovinyl)-2-(4-methyl-6-((1-methylpiperidin-3-yl)amino)pyridazin-3-yl) phenol (Compound 39)**

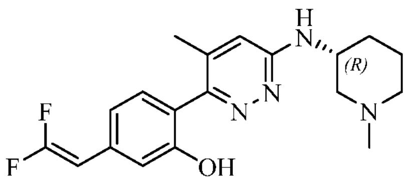

Step 1: Synthesis of 1-bromo-4-(2,2-difluorovinyl)-2-(ethoxymethoxy)benzene

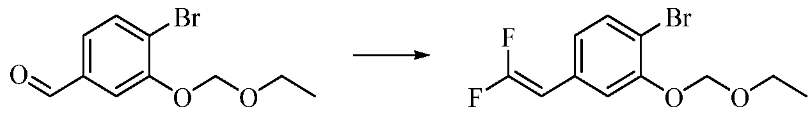

4-Bromo-3-(ethoxymethoxy)benzaldehyde (2.0 g, 7.71 mmol, 1.0 eq) and 2,2-difluoro-2-(triphenylphosphonio) acetate (3.3 g, 9.26 mmol, 1.2 eq) were added to *N,N*-dimethylformamide (10.0 mL), and the mixture was stirred at 40 °C for 12 h. TLC monitoring showed the completion of the reaction. Water (50.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (specification of silica gel: 100-200 mesh, petroleum ether:ethyl acetate = 100:1) to give the product (1.0 g, yield: 44.2%).

Step 2: Synthesis of 2-(4-(2,2-difluorovinyl)-2-(ethoxymethoxy)phenyl)-4,4,5,5-tetramethyl-1,3,2-dioxaborolan e

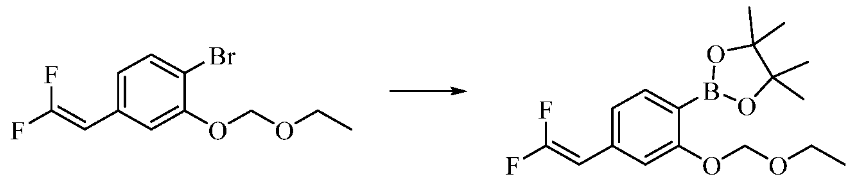

1-Bromo-4-(2,2-difluorovinyl)-2-(ethoxymethoxy)benzene (1.0 g, 3.41 mmol, 1.0 eq), bis(pinacolato)diboron (1.3 g, 5.11 mmol, 1.5 eq), potassium acetate (669.5 mg, 6.82 mmol, 2.0 eq), and Pd(dppf)$Cl_2$ (249.5 mg, 0.34 mmol, 0.1 eq) were added to 1,4-dioxane (15.0 mL). The mixture was allowed to react at 100 °C for 4 h under a nitrogen atmosphere, and TLC monitoring showed the completion of the reaction. Water (50.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (specification of silica gel: 100-200 mesh, petroleum ether:ethyl acetate = 20:1) to give the product (630.0 mg, yield: 54.3%).

Step 3: Synthesis of (*R*)-6-chloro-5-methyl-*N*-(1-methylpiperidin-3-yl)pyridazin-3-amine

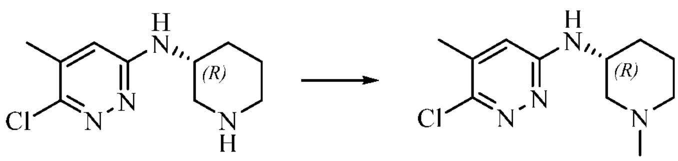

(*R*)-6-Chloro-5-methyl-*N*-(piperidin-3-yl)pyridazin-3-amine (980.0 mg, 4.32 mmol, 1.0 eq), an aqueous formaldehyde solution (37%, 526.2 mg, 6.48 mmol, 1.5 eq), and acetic acid (0.5 mL) were added to methanol (20.0 mL). The mixture was stirred at room temperature for 0.5 h, and then sodium cyanoborohydride (407.4 mg, 6.48 mmol, 1.5 eq) was added. The resulting mixture was allowed to react at room temperature for 2 h. TLC monitoring showed the completion of the reaction. The system was concentrated under reduced pressure, and a saturated aqueous sodium bicarbonate solution (100.0 mL) was added. The mixture was stirred for 0.5 h and extracted with dichloromethane (100.0 mL). The organic phase was dried over anhydrous sodium sulfate and filtered. The filtrate was concentrated under reduced pressure, and the crude product was purified by silica gel column chromatography (dichloromethane:methanol = 40:1 to 20:1) to give the product (750.0 mg, yield: 72.1%).

Step 4: Synthesis of (*R*)-6-(4-(2,2-difluorovinyl)-2-(ethoxymethoxy)phenyl)-5-methyl-*N*-(1-methylpiperidin-3-y l)pyridazin-3-amine

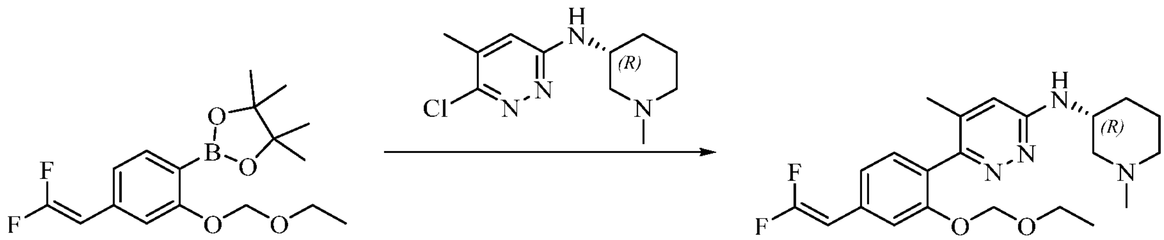

(*R*)-6-Chloro-5-methyl-*N*-(1-methylpiperidin-3-yl)pyridazin-3-amine (230.0 mg, 0.95 mmol, 1.0 eq), 2-(4-(2,2-difluorovinyl)-2-(ethoxymethoxy)phenyl)-4,4,5,5-tetramethyl-1,3,2-dioxaborolan e (389.9 mg, 1.14 mmol, 1.2 eq), sodium bicarbonate (160.4 mg, 1.91 mmol, 2.0 eq), and Pd(dppf)$Cl_2$ (34.9 mg, 0.04 mmol, 0.05 eq) were added to a mixed solution of 1,4-dioxane (10.0 mL) and water (5.0 mL). The mixture was allowed to react at 110 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (specification of silica gel: 100-200 mesh, dichloromethane: methanol = 40:1 to 10:1) to give the product (160.0 mg, yield: 40.0%).

Step 5: Synthesis of (*R*)-5-(2,2-difluorovinyl)-2-(4-methyl-6-((1-methylpiperidin-3-yl)amino)pyridazin-3-yl)phe nol

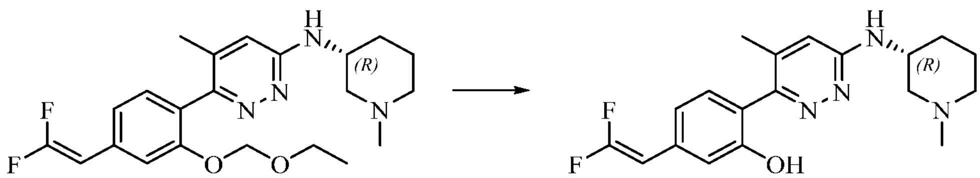

;

(*R*)-6-(4-(2,2-Difluorovinyl)-2-(ethoxymethoxy)phenyl)-5-methyl-N-(1-methy lpiperidin-3-yl)pyridazin-3-amine (140.0 mg, 0.33 mmol, 1.0 eq) was added to dichloromethane (4.0 mL), and a solution of hydrogen chloride in 1,4-dioxane (4.0 mol/L, 2.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (60.0 mg, yield: 49.8%).

$^1$HNMR (400 MHz, DMSO-$d_6$) δ(ppm): 10.00 (s, 1H), 7.19-7.17 (d, *J*=8 Hz, 1H), 6.97 (s, 1H), 76.91-6.89 (d, *J*=8 Hz, 1H), 6.68 (s, 1H), 6.64-6.62 (d, *J*=8 Hz, 1H), 5.83-5.75 (m, 1H), 4.06 (s, 1H), 2.95 (s, 1H), 2.66 (s, 1H), 2.25 (s, 3H), 2.12-1.95 (m, 5H), 1.87-1.34 (m, 4H).

Molecular formula: $C_{19}H_{22}F_2N_4O$ Exact molecular weight: 360.18 LC-MS (Pos, m/z) = 361.19 $[M+H]^+$.

**Example 16: Synthesis of (*R*)-2-(7-((1-methylpiperidin-3-yl)amino)pyrazolo[1,5-d][1,2,4]triazin-4-yl)-5-(prop-1-y n-1-yl)phenol (Compound 40)**

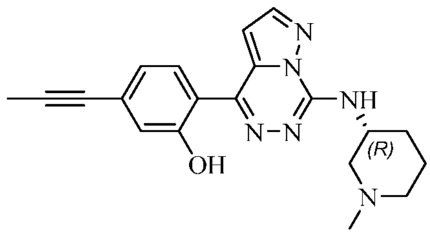

Step 1: Synthesis of 1-bromo-2-methoxy-4-(prop-1-yn-1-yl)benzene

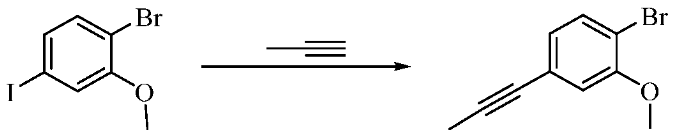

1-Bromo-4-iodo-2-methoxybenzene (15.65 g, 50 mmol, 1.0 eq), DIPEA (9.7 g, 75 mmol, 1.5 eq), bis(triphenylphosphine)palladium(II) chloride (3.5 g, 5 mmol, 0.1 eq), and cuprous iodide (1.9 g, 10 mmol, 0.2 eq) were added to anhydrous THF (150 mL). A 1 mol/L solution of propyne in THF (55 mL) was added under a nitrogen atmosphere, and the mixture was allowed to react at room temperature for 64 h. TLC monitoring showed that no starting material remained. Water (100 mL) and ethyl acetate (100 mL) were added, and the mixture was stirred for 5 min and filtered. The filter cake was rinsed with ethyl acetate, followed by liquid separation. The organic phase was retained, and the aqueous phase was extracted with ethyl acetate (100 mL × 2). The organic phases were combined, dried over anhydrous sodium sulfate, and filtered. The filtrate was concentrated under reduced pressure, and the crude product was purified by silica gel column chromatography (EA:PE = 1:100) to give the product (11.1 g, yield: 99.1%).

Step 2: Synthesis of (2-methoxy-4-(prop-1-yn-1-yl)phenyl)magnesium bromide

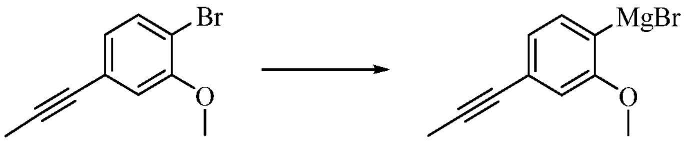

Magnesium strips (1.3 g, 52.19 mmol, 1.1 eq) were added to anhydrous THF (50 mL), and the mixture was heated to 70 °C under a nitrogen atmosphere. 1,2-Dibromoethane (1 mL) was added, and a solution of 1-bromo-2-methoxy-4-(prop-1-yn-1-yl)benzene (10.68 g, 47.45 mmol, 1.0 eq) in anhydrous THF (30 mL) was added dropwise. After the dropwise addition, the mixture was allowed to react at 70 °C for 1 h. The resulting product was used in the next step according to a theoretical amount.

Step 3: Synthesis of (2-methoxy-4-(prop-1-yn-1-yl)phenyl)(1-(4-methoxybenzyl)-1*H*-pyrazol-5-yl)methanone

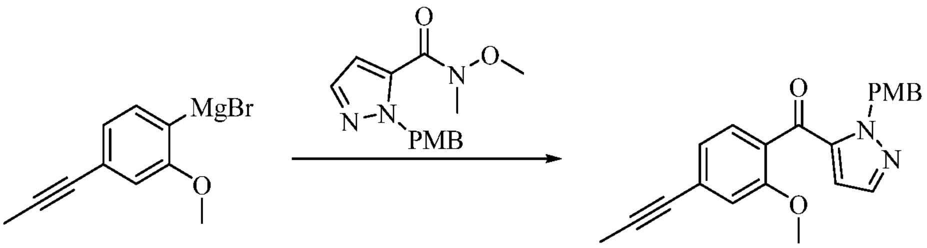

A solution of (2-methoxy-4-(prop-1-yn-1-yl)phenyl)magnesium bromide (47.45 mmol, 1.63 eq) in anhydrous THF was heated to 70 °C, and a solution of *N*-methoxy-1-(4-methoxybenzyl)-*N*-methyl-1*H*-pyrazole-5-carboxamide (8 g, 29.06 mmol, 1.0 eq) in anhydrous THF (20 mL) was added dropwise. The mixture was allowed to react at 70 °C for 20 h. TLC monitoring showed that a significant amount of starting material remained. The reaction mixture was cooled to room temperature. A saturated aqueous ammonium chloride solution (200 mL) was added, and extraction was performed with ethyl acetate (200 mL × 2). The organic phases were combined, dried over anhydrous sodium sulfate, and filtered. The filtrate was concentrated under reduced pressure, and the crude product was purified by silica gel column chromatography (EA:PE = 1: 100 to 1:5) to give the product (1 g, yield: 9.5%).

Step 4: Synthesis of (2-methoxy-4-(prop-1-yn-1-yl)phenyl)(1*H*-pyrazol-5-yl)methanone

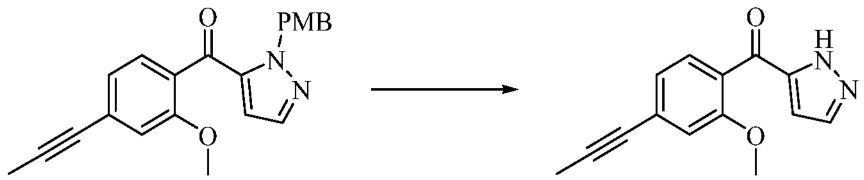

(2-Methoxy-4-(prop-1-yn-1-yl)phenyl)(1-(4-methoxybenzyl)-1*H*-pyrazol-5-yl )methanone (1.6 g, 4.44 mmol, 1.0 eq) was added to a mixed solution of 1,2-dichloroethane (10 mL) and TFA (10 mL), and the mixture was heated to reflux and allowed to react for 7 h. TLC monitoring showed that no starting material remained. The reaction mixture was concentrated under reduced pressure. A saturated aqueous sodium carbonate solution (50 mL) was added, and extraction was performed with DCM (50 mL × 3). The organic phases were combined, dried over anhydrous sodium sulfate, and filtered. The filtrate was concentrated under reduced pressure, and the crude product was purified by silica gel column chromatography (EA:PE = 1:10 to 1:3) to give the product (1.0 g, yield: 90.9%).

Step 5: Synthesis of 4-(2-methoxy-4-(prop-1-yn-1-yl)phenyl)pyrazolo[1,5-*d*][1,2,4]triazin-7-ol

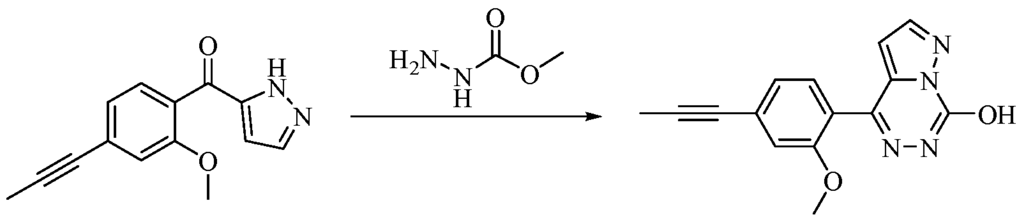

(2-Methoxy-4-(prop-1-yn-1-yl)phenyl)(1*H*-pyrazol-5-yl)methanone (1.0 g, 4.16 mmol, 1.0 eq), methyl hydrazinecarboxylate (1.1 g, 12.48 mmol, 3.0 eq), and acetic acid (249.6 mg, 4.16 mmol, 1.0 eq) were added to absolute methanol (50 mL), and the mixture was heated to reflux and allowed to react for 72 h. LC-MS analysis showed that no starting material remained. The reaction mixture was concentrated under reduced pressure, and absolute ethanol (50 mL) was added, followed by the addition of sodium hydride (mass fraction: 60%) (499.2 mg, 12.48 mmol, 3.0 eq). The resulting mixture was heated to reflux and allowed to react for 16 h. LC-MS monitoring showed that no starting material remained. The reaction mixture was concentrated under reduced pressure, and water (50 mL) was added. Hydrochloric acid (2 mol/L) was added dropwise to adjust the pH to about 3, and extraction was performed with DCM (50 mL × 3). The organic phases were combined, dried over anhydrous sodium sulfate, and filtered. The filtrate was concentrated under reduced pressure to give the product (1.2 g, yield: 100%).

Step 6: Synthesis of 4-(2-methoxy-4-(prop-1-yn-1-yl)phenyl)pyrazolo[1,5-*d*][1,2,4]triazine-7-thiol

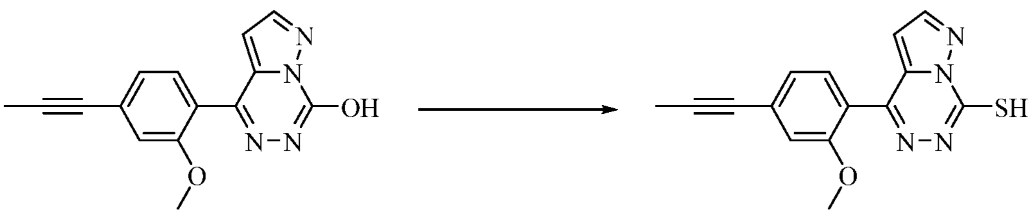

4-(2-Methoxy-4-(prop-1-yn-1-yl)phenyl)pyrazolo[1,5-*d*][1,2,4]triazin-7-ol (1.2 g, 14.16 mmol, 1.0 eq) and Lawesson's reagent (1.7 g, 4.16 mmol, 1.0 eq) were added to dimethylbenzene (150 mL), and the mixture was heated to 110 °C and allowed to react for 19 h under a nitrogen atmosphere. TLC monitoring showed that no starting material remained. The reaction mixture was cooled to room temperature and concentrated under reduced pressure, and the crude product was purified by silica gel column chromatography (MeOH:DCM = 1:100) to give the product (1.1 g, yield: 91.6%).

Step 7: Synthesis of 4-(2-methoxy-4-(prop-1-yn-1-yl)phenyl)-7-(methylthio)pyrazolo[1,5-*d*][1,2,4]triazine

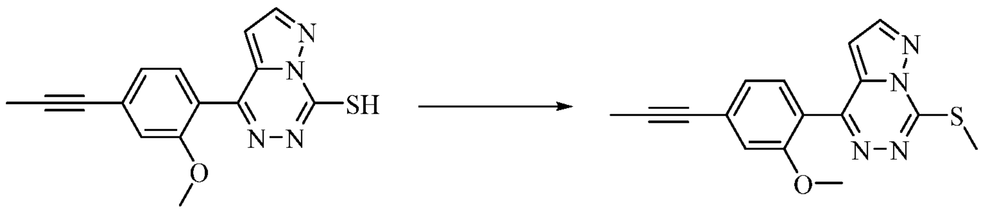

4-(2-Methoxy-4-(prop-1-yn-1-yl)phenyl)pyrazolo[1,5-*d*][1,2,4]triazine-7-thiol (1.1 g, 3.71 mmol, 1.0 eq) was added to DMA (5 mL), and anhydrous potassium carbonate (512 mg, 3.71 mmol, 1.0 eq) and iodomethane (1 g, 7.42 mmol, 2.0 eq) were added. The mixture was heated to 60 °C and allowed to react for 3 h. TLC monitoring showed that no starting material remained. The reaction solution was poured into ice water (50 mL) and extracted with ethyl acetate (50 mL × 3). The organic phases were combined, dried over anhydrous sodium sulfate, and filtered. The filtrate was concentrated under reduced pressure, and the crude product was purified by silica gel column chromatography (EA:PE = 1:10) to give the product (200 mg, yield: 18.2%).

Step 8: Synthesis of *tert-butyl* (*R*)-3-((4-(2-methoxy-4-(prop-1-yn-1-yl)phenyl)pyrazolo[1,5-*d*][1,2,4]triazin-7-yl)amino) pi peridine-1-carboxylate

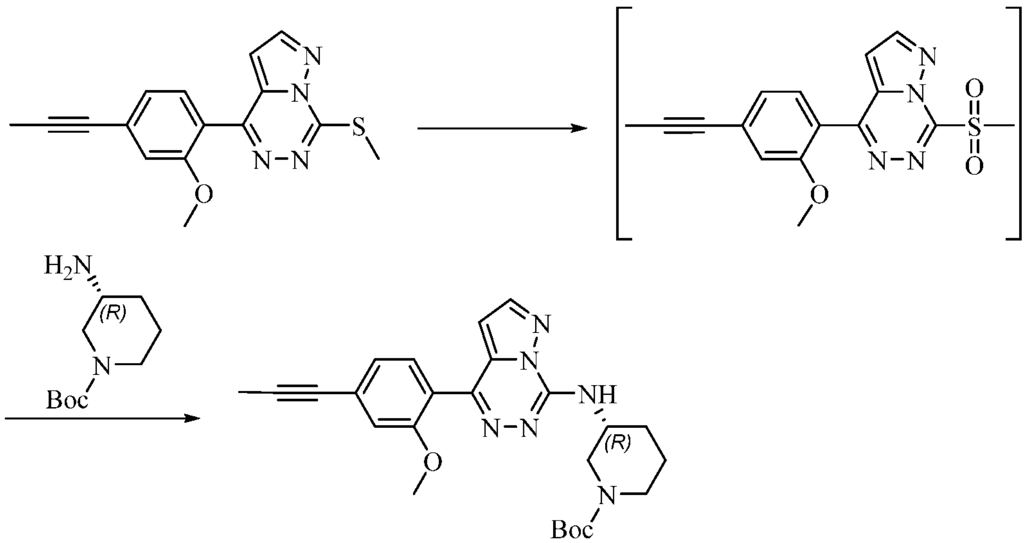

4-(2-Methoxy-4-(prop-1-yn-1-yl)phenyl)-7-(methylthio)pyrazolo[1,5-*d*][1,2,4] triazine (200 mg, 0.64 mmol, 1.0 eq) and 3A molecular sieve (500 mg) were added to anhydrous DCM (5 mL), and a solution of mCPBA (mass fraction: 85%, 324.8 mg, 1.6 mmol, 2.5 eq) in DCM (5 mL), previously dried over 3A molecular sieve, was added dropwise. The mixture was allowed to react at room temperature for 1 h. TLC monitoring showed that no starting material remained. A solution of *tert*-butyl (*R*)-3-aminopiperidine-1-carboxylate (897.2 mg, 4.48 mmol, 7.0 eq) in DCM (2 mL) was added, and the mixture was allowed to react at room temperature for 16 h. TLC analysis showed that no starting material remained. Water (30 mL) was added, and extraction was performed with DCM (30 mL × 3). The organic phases were combined, dried over anhydrous sodium sulfate, and filtered. The filtrate was concentrated under reduced pressure, and the crude product was purified by silica gel column chromatography (EA:PE = 1: 10 to 1:2) to give the product (146 mg, two-step yield: 49.3%).

Step 9: Synthesis of (*R*)-2-(7-(piperidin-3-ylamino)pyrazolo[1,5-*d*][1,2,4]triazin-4-yl)-5-(prop-1-yn-1-yl)phenol

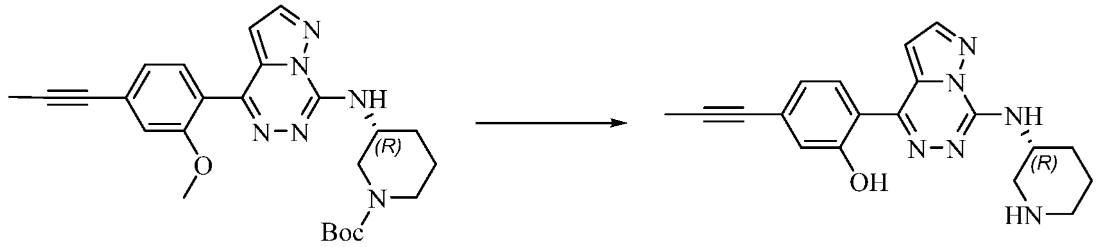

*tert*-Butyl (*R*)-3-((4-(2-methoxy-4-(prop-1-yn-1-yl)phenyl)pyrazolo[1,5-*d*][1,2,4]triazin-7-yl)amino)pi peridine-1-carboxylate (146 mg, 0.31 mmol, 1.0 eq) was added to anhydrous DCM (10 mL), and the mixture was cooled to -50 °C, followed by the dropwise addition of boron tribromide (776.6 mg, 3.1 mmol, 10 eq). The mixture was naturally warmed to room temperature and allowed to react for 23 h, and LC-MS monitoring showed that no starting material remained. Methanol (10 mL) was then added dropwise, and the resulting mixture was stirred for 10 min and concentrated under reduced pressure. A saturated aqueous sodium bicarbonate solution (30 mL) was added, and extraction was performed with DCM (30 mL × 3). The organic phases were combined, dried over anhydrous sodium sulfate, and filtered. The filtrate was concentrated under reduced pressure to give the product (108 mg, yield: 100%).

Step 10: Synthesis of (*R*)-2-(7-((1-methylpiperidin-3-yl)amino)pyrazolo[1,5-*d*][1,2,4]triazin-4-yl)-5-(prop-1-yn-1 -yl)phenol

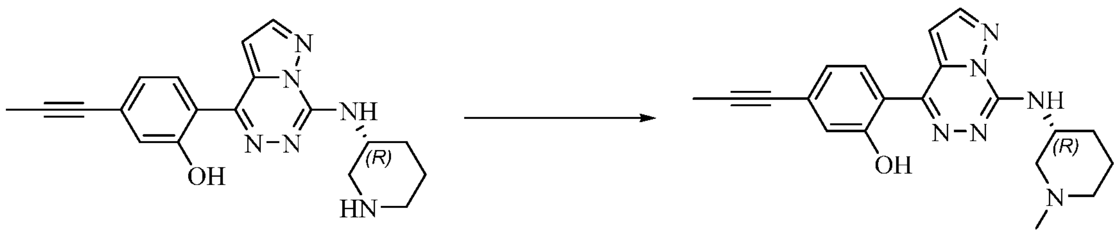

(*R*)-2-(7-(Piperidin-3-ylamino)pyrazolo[1,5-*d*][1,2,4]triazin-4-yl)-5-(prop-1-y n-1-yl)phenol (108 mg, 0.31 mmol, 1.0 eq) was added to methanol (5 mL), and an aqueous formaldehyde solution (mass fraction: 37%) (25.2 mg, 0.31 mmol, 1.0 eq) was added. The mixture was allowed to react at room temperature for 0.5 h, and sodium cyanoborohydride (19.5 mg, 0.31 mmol, 1.0 eq) was added. The resulting mixture was allowed to react at room temperature for 1 h. TLC monitoring showed that no starting material remained. The reaction mixture was concentrated under reduced pressure. A saturated aqueous sodium bicarbonate solution (20 mL) was added, and extraction was performed with DCM (20 mL × 4). The organic phases were combined, dried over anhydrous sodium sulfate, and filtered. The filtrate was concentrated under reduced pressure, and the crude product was purified by preparative thin-layer chromatography (MeOH:DCM = 1:10) to give the product (67 mg, yield: 59.7%).

$^1$HNMR (400 MHz, DMSO-$d_6$) δ(ppm): 12.65 (s, 1H), 8.41 (s, 1H), 7.87-7.85 (d, 2H), 7.31 (s, 1H), 6.99 (d, 2H), 4.35-4.33 (m, 1H), 2.83-2.81 (m, 1H), 2.51 (m, 1H), 2.23 (m, 4H), 2.08 (m, 4H), 1.82 (m, 1H), 1.73-1.68 (m, 2H), 1.59-1.56 (m, 1H).

Molecular formula: $C_{20}H_{22}N_6O$ Exact mass: 362.19 LC-MS (Pos, *m/z*) = 363.31 $[M+H]^+$.

**Example 17: (*R*)-2-(4-Cyclopropyl-6-((1-(2-hydroxyethyl)piperidin-3-yl)amino)pyridazin-3-yl)-5-eth ynylphenol (Compound 45)**

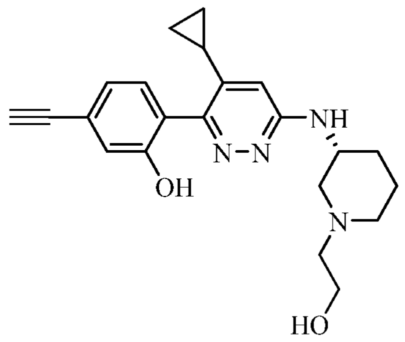

Step 1: Synthesis of (*R*)-4-(4-cyclopropyl-6-((1-(2-hydroxyethyl)piperidin-3-yl)amino)pyridazin-3-yl)-3-(ethoxy methoxy)benzaldehyde

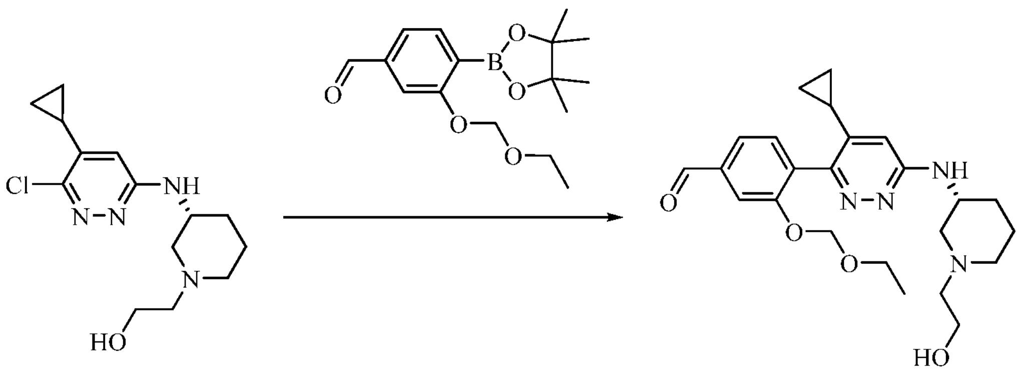

(*R*)-2-(3-((6-Chloro-5-cyclopropyl-pyridazin-3-yl)amino)piperidin-1-yl)ethan-1-ol (237 mg, 0.799 mmol, 1.0 eq) was dissolved in 1,4-dioxane (4 mL), and 3-(ethoxymethoxy)-4-(4,4,5,5-tetramethyl-1,3,2-dioxaborolan-2-yl)benzaldehyde (318 mg, 1.04 mmol, 1.3 eq), an aqueous solution (1 mL) of sodium bicarbonate (134 mg, 1.598 mmol, 2.0 eq), and $PdCl_2$(dppf) (58 mg, 0.0799 mmol, 0.1 eq) were added to the solution. The mixture was heated to 90 °C and allowed to react for 1 h under a nitrogen atmosphere, and TLC analysis showed the completion of the reaction. The reaction solution was concentrated, and dichloromethane (10 mL) and water (10 mL) were added to the concentrate, followed by liquid separation. The aqueous phase was extracted with dichloromethane (10 mL × 3). The organic phases were combined, dried over anhydrous magnesium sulfate, and concentrated, and the crude product was purified by silica gel column chromatography (dichloromethane:methanol = 100:1 to 20:1) to give the product (120 mg, yield: 34.1%).

Step 2: Synthesis of (*R*)-2-(3-((5-cyclopropyl-6-(2-ethoxymethoxy-4-ethynylphenyl)pyridazin-3-yl)amino)piperi din-1-yl)ethan-1-ol

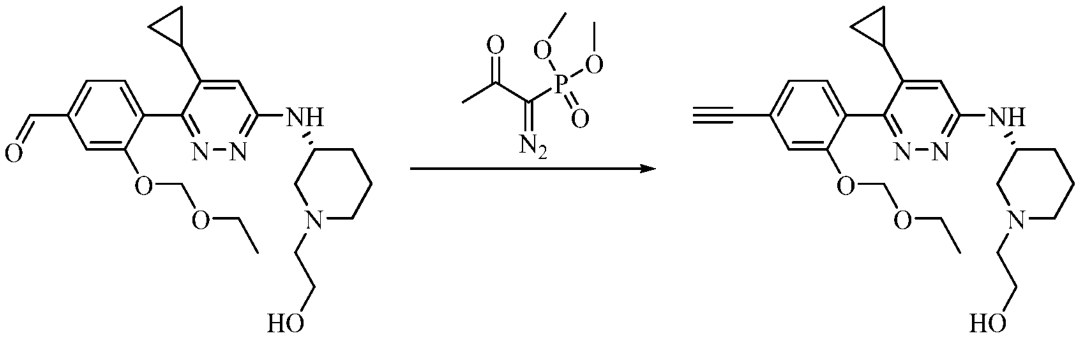

(*R*)-4-(4-Cyclopropyl-6-((1-(2-hydroxyethyl)piperidin-3-yl)amino)pyridazin-3 -yl)-3-(ethoxymethoxy)benzaldehyde (120 mg, 0.273 mmol, 1.0 eq) was dissolved in methanol (2 mL), and potassium carbonate (75 mg, 0.546 mmol, 2.0 eq) and dimethyl (1-diazo-2-oxopropyl)phosphonate (79 mg, 0.41 mmol, 1.5 eq) were sequentially added to the solution. The mixture was allowed to react at 25 °C for 30 min, and TLC analysis showed the completion of the reaction. The reaction solution was concentrated. A saturated aqueous sodium chloride solution (5 mL) was added to the concentrate, and extraction was performed with dichloromethane (10 mL × 3). The organic phases were combined, dried over anhydrous magnesium sulfate, filtered, and concentrated, and the crude product was directly used in the next step.

Step 3: Synthesis of (*R*)-2-(4-cyclopropyl-6-((1-(2-hydroxyethyl)piperidin-3-yl)amino)pyridazin-3-yl)-5-ethynyl phenol

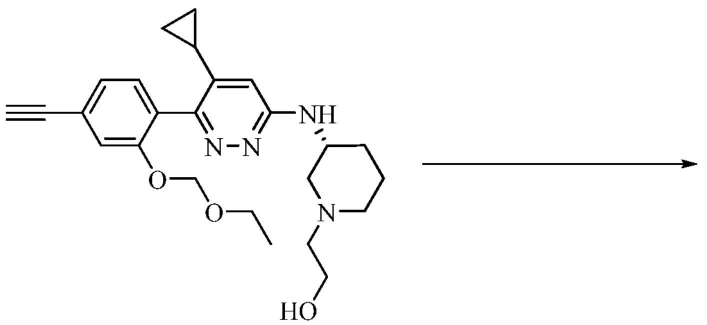

(*R*)-2-(3-((5-Cyclopropyl-6-(2-ethoxymethoxy-4-ethynylphenyl)pyridazin-3-y l)amino)piperidin-1-yl)ethan-1-ol (crude, 0.273 mmol) was dissolved in dichloromethane (1 mL), and the solution was added dropwise to trifluoroacetic acid (1 mL). The mixture was allowed to react at 25 °C for 15 min, and TLC analysis showed the completion of the reaction. The reaction solution was concentrated, and the crude product was purified by preparative thin-layer chromatography (dichloromethane:solution of ammonia in methanol = 15:1) to give the product (21 mg, two-step yield: 20.4%).

$^1$H-NMR (400 MHz, DMSO-*$d_6$*) δ(ppm): 10.08 (s, 1H), 7.24-7.22 (m, 1H), 7.01-7.00 (m, 2H), 6.63 (s, 1H), 6.32 (s, 1H), 4.19 (d, 2H), 3.59 (s, 2H), 3.11-2.77 (m, 2H), 2.69-2.57 (m, 1H), 2.39-2.18 (m, 2H), 1.91-1.71 (m, 2H), 1.67-1.65 (m, 1H), 1.59-1.53 (m, 1H), 1.40-1.39 (m, 1H), 1.34-1.17 (m, 2H), 0.90-0.86 (m, 2H), 0.63-0.60 (m, 2H).

Molecular formula: $C_{22}H_{26}N_4O_2$ Exact molecular weight: 378.21 LC-MS *(m/z):* 379.26 $[M+H]^+$.

**Example 18: Synthesis of (*R*)-2-(4-cyclopropyl-6-((1-ethylpiperidin-3-yl)amino)pyridazin-3-yl)-5-ethynylphenol (Compound 90)**

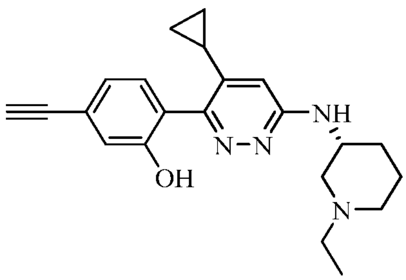

Step 1: Synthesis of (*R*)-5-cyclopropyl-6-(2-(ethoxymethoxy)-4-ethynylphenyl)-*N*-(piperidin-3-yl)pyridazin-3-a mine

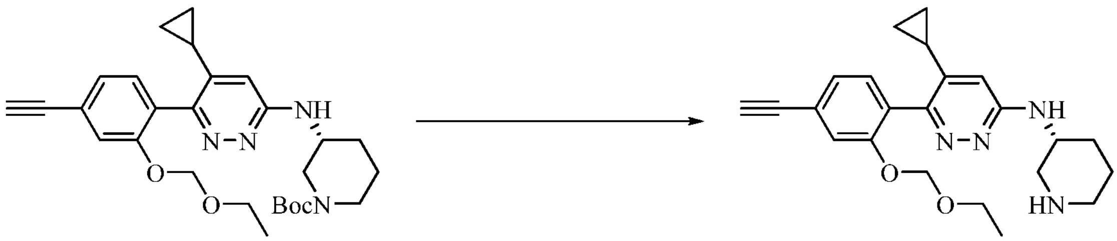

*tert*-Butyl (*R*)-3-((5-cyclopropyl-6-(2-(ethoxymethoxy)-4-ethynylphenyl)pyridazin-3-yl)amino)piperid ine-1-carboxylate (1.0 g, 2.03 mmol, 1.0 eq) was dissolved in dichloromethane (10 mL), and 2,6-dimethylpyridine (1.74 g, 16.24 mmol, 8.0 eq) and trimethylsilyl trifluoromethanesulfonate (1.8 g, 8.12 mmol, 4.0 eq) were added dropwise to the solution at 0 °C. TLC analysis showed the completion of the reaction. Water (20 mL) was added to the reaction solution, followed by liquid separation. The aqueous phase was extracted with dichloromethane (20 mL × 2). The organic phases were combined, dried over anhydrous magnesium sulfate, and concentrated, and the crude product was purified by silica gel column chromatography (dichloromethane:methanol = 50:1 to 10:1) to give the product (1.15 g, crude product).

Step 2: Synthesis of (*R*)-5-cyclopropyl-6-(2-(ethoxymethoxy)-4-ethynylphenyl)-N-(1-ethylpiperidin-3-yl)pyrida zin-3-amine

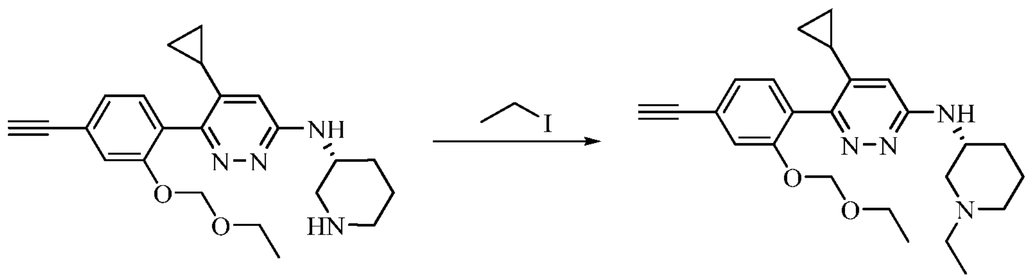

(*R*)-5-Cyclopropyl-6-(2-(ethoxymethoxy)-4-ethynylphenyl)-*N*-(piperidin-3-yl) pyridazin-3-amine (400 mg, crude product, 0.703 mmol, 1.0 eq) was dissolved in dichloromethane (3 mL), and triethylamine (356 mg, 3.515 mmol, 5.0 eq) and iodoethane (548 mg, 3.515 mmol, 5.0 eq) were added to the solution. The mixture was allowed to react at 25 °C for 16 h, and TLC analysis showed the completion of the reaction. Dichloromethane (7 mL) and water (10 mL) were added to the reaction solution, followed by liquid separation. The aqueous phase was extracted with dichloromethane (10 mL × 2). The organic phases were combined and dried, and the crude product was purified by preparative thin-layer chromatography (dichloromethane:solution of ammonia in methanol = 15:1) to give the product (150 mg, yield: 50.7%).

Step 3: Synthesis of (*R*)-2-(4-cyclopropyl-6-((1-ethylpiperidin-3-yl)amino)pyridazin-3-yl)-5-ethynylphenol

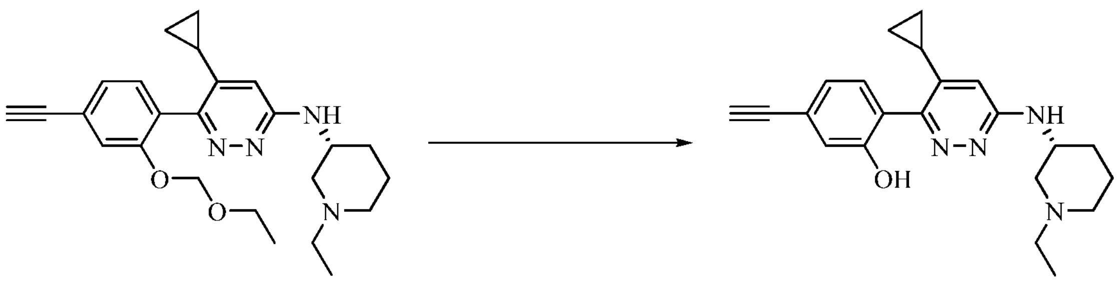

(*R*)-5-Cyclopropyl-6-(2-(ethoxymethoxy)-4-ethynylphenyl)-*N*-(1-ethylpiperidi n-3-yl)pyridazin-3-amine (150 mg, 0.357 mmol, 1.0 eq) was dissolved in dichloromethane (1 mL), and the solution was added dropwise to trifluoroacetic acid (1 mL). The mixture was allowed to react at 25 °C for 5 min, and TLC analysis showed the completion of the reaction. The reaction solution was concentrated, and the crude product was purified by preparative thin-layer chromatography

(dichloromethane:solution of ammonia in methanol = 10:1) to give the product (28 mg, yield: 21.7%).

$^1$H-NMR (400 MHz, DMSO-$d_6$) δ(ppm): 10.09 (s, 1H), 7.25-7.23 (d, 1H), 7.01-7.00 (m, 2H), 6.47-6.45 (d, 1H), 6.33 (s, 1H), 4.18 (s, 1H), 4.03-4.01 (m, 1H), 2.93-2.91 (m, 1H), 2.61 (m, 1H), 2.37-2.32 (m, 2H), 2.03-2.02 (m, 1H), 1.91-1.81 (m, 2H), 1.71-1.69 (m, 1H), 1.59-1.48 (m, 2H), 1.34-1.26 (m, 1H), 1.02-0.98 (t, 3H), 0.89-0.84 (m, 2H), 0.63-0.59 (m, 2H).

Molecular formula: $C_{22}H_{26}N_4O$ Exact molecular weight: 362.21 LC-MS *(m/z):* 363.29 [M+H]$^+$.

**Example 19: Synthesis of (*R*)-2-(4-cyclopropyl-6-((1-cyclopropylpiperidin-3-yl)amino)pyridazin-3-yl)-5-ethynylp henol (Compound 95)**

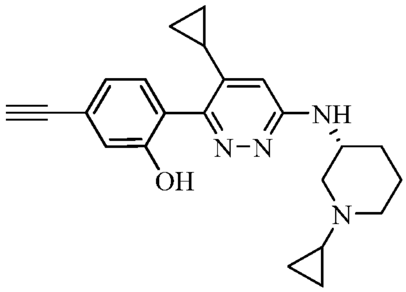

Step 1:

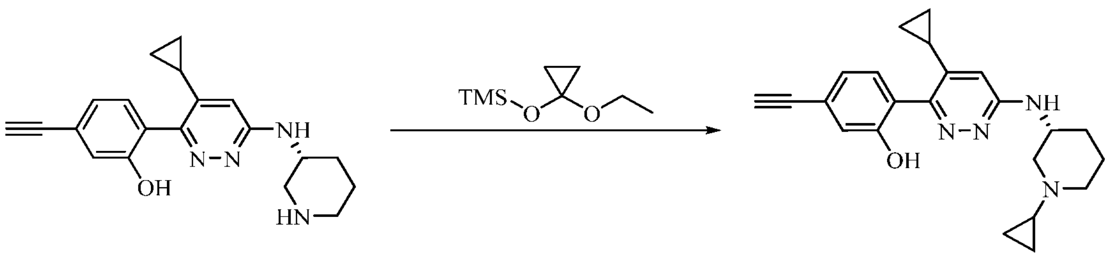


(*R*)-2-(4-Cyclopropyl-6-(piperidin-3-ylamino)pyridazin-3-yl)-5-ethynylphenol (crude product, 0.75 mmol, 1.0 eq) was dissolved in methanol (3 mL), and (1-ethoxycyclopropoxy)trimethylsilane (523 mg, 3.0 mmol, 4.0 eq) and cesium fluoride (228 mg, 1.5 mmol, 2.0 eq) were added to the solution. The mixture was heated to 50 °C and allowed to react for 1 h, and sodium cyanoborohydride (189 mg, 3.0 mmol, 4.0 eq) was added to the reaction mixture. The resulting mixture was allowed to react at 50 °C for 10 min, and TLC analysis showed the completion of the reaction. The reaction solution was concentrated. A saturated aqueous sodium bicarbonate solution (10 mL) was added to the reaction solution, and extraction was performed with dichloromethane (10 mL × 3). The organic phases were combined, dried, and concentrated to give a crude product, which was then purified by preparative thin-layer chromatography (dichloromethane:solution of ammonia in methanol = 10:1) to give the product (15 mg, yield: 5.3%).

$^1$H-NMR (400 MHz, DMSO-$d_6$) δ(ppm): 10.08 (s, 1H), 7.24-7.22 (m, 1H), 7.01-7.00 (m, 2H), 6.46 (s, 1H), 6.32 (s, 1H), 4.18 (s, 1H), 3.97 (s, 1H), 3.07 (s, 1H), 2.76 (s, 1H), 2.33 (s, 1H), 2.19 (s, 1H), 1.82 (s, 1H), 1.67 (s, 2H), 1.59-1.50 (m, 1H), 1.48 (m, 1H), 1.34-1.33 (m, 1H), 0.89-0.85 (m, 2H), 0.63-0.60 (m, 2H), 0.44-0.35 (m, 4H).

Molecular formula: $C_{23}H_{26}N_4O$ Exact molecular weight: 374.21 LC-MS *(m/z):* 375.29 [M+H]$^+$.

**Example 20: Synthesis of (*R*)-2-(4-cyclopropyl-6-((1-(methyl-$d_3$)piperidin-3-yl)amino)pyridazin-3-yl)-5-ethynylp henol (Compound 85)**

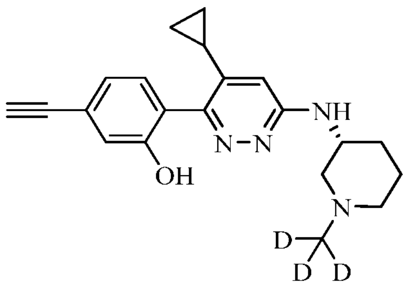

Step 1: Synthesis of compound (*R*)-5-cyclopropyl-6-(2-(ethoxymethoxy)-4-ethynylphenyl)-*N*-(1-(methyl-$d_3$)piperidin-3-yl) pyridazin-3-amine

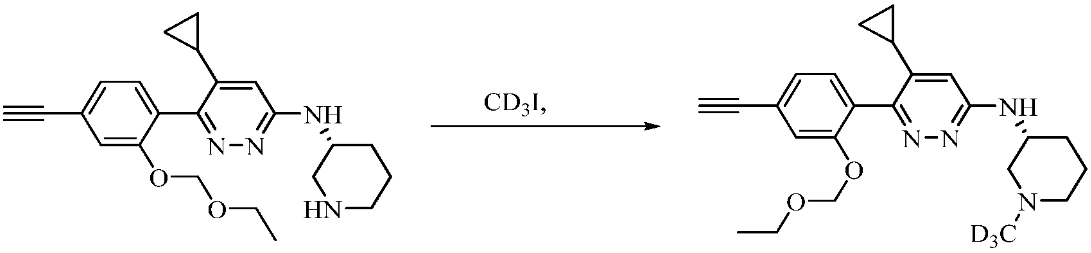


(*R*)-5-Cyclopropyl-6-(2-(ethoxymethoxy)-4-ethynylphenyl)-*N*-(piperidin-3-yl) pyridazin-3-amine (400 mg, crude product, 0.703 mmol, 1.0 eq) was dissolved in dichloromethane (3 mL), and triethylamine (356 mg, 3.515 mmol, 5.0 eq) and deuterated iodomethane (296 mg, 3.515 mmol, 5.0 eq) were added to the solution. The mixture was allowed to react at 25 °C for 2 h (LC-MS analysis showed 60% conversion of the starting material). Water (10 mL) was added to the reaction solution, and extraction was performed with dichloromethane (10 mL). The aqueous phase was further extracted with dichloromethane (10 mL × 2). The organic phases were combined, dried over anhydrous magnesium sulfate, and concentrated, and the crude product was purified by preparative thin-layer chromatography (dichloromethane:solution of ammonia in methanol = 15:1) to give the product (125 mg, yield: 43.4%).

Step 2: Synthesis of compound (*R*)-2-(4-cyclopropyl-6-((1-(methyl-$d_3$)piperidin-3-yl)amino)pyridazin-3-yl)-5-ethynyl-phen ol

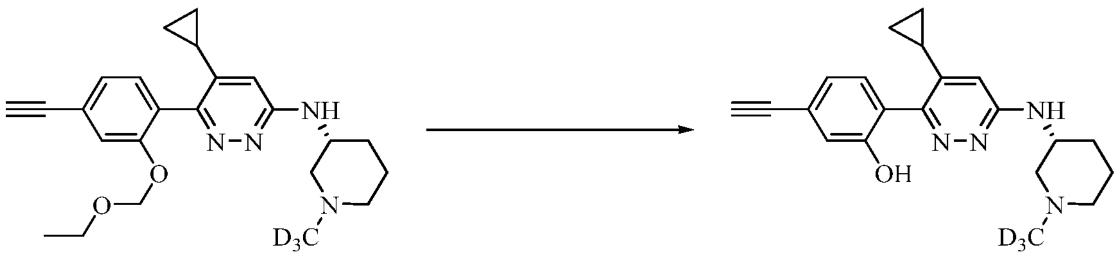

(*R*)-5-Cyclopropyl-6-(2-(ethoxymethoxy)-4-ethynylphenyl)-*N*-(1-(methyl-$d_3$)p iperidin-3-yl)pyridazin-3-amine (125 mg, 0.305 mmol, 1.0 eq) was dissolved in dichloromethane (1 mL), and the solution was added dropwise to trifluoroacetic acid (1 mL). The mixture was allowed to react at 25 °C for 15 min, and TLC analysis showed the completion of the reaction. The reaction solution was concentrated, and the crude product was purified by preparative thin-layer chromatography (dichloromethane:solution of ammonia in methanol = 10:1) to give the product (40 mg, yield: 37.4%).

$^1$H-NMR (400 MHz, DMSO-$d_6$) δ(ppm): 10.11 (s, 1H), 7.23-7.21 (d, 1H), 7.02-7.00 (m, 2H), 6.68-6.67 (d, 1H), 6.34 (s, 1H), 4.19 (s, 1H), 4.15 (m, 1H), 3.11 (m, 1H), 2.83 (m, 1H), 2.41-2.29 (m, 2H), 1.84-1.79 (m, 2H), 1.66-1.63 (m, 1H), 1.59-1.53 (m, 1H), 1.44-1.30 (m, 1H) , 0.91-0.83 (m, 2H), 0.65-0.58 (m, 2H).

Molecular formula: $C_{21}H_{21}D_3N_4O$ Exact molecular weight: 351.21 LC-MS (*m/z*): 352.22 [M+H]$^+$.

**Example 21: Synthesis of (*R*)-3-((6-(2-hydroxy-4-(prop-1-yn-1-yl)phenyl)-5-methylpyridazin-3-yl)amino)piperidine-1-carboximidamide trifluoroacetate (Compound 46)**

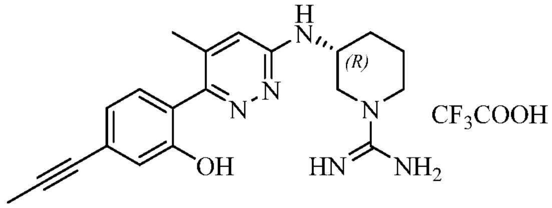

Step 1: Synthesis of *tert-butyl* (*R*)-(((*tert*-butoxycarbonyl)imino)(3-((6-(2-(ethoxymethoxy)-4-(prop-1-yn-1-yl)phenyl)-5-methylpyridazin-3-yl)amino)piperidin-1-yl)methyl)carbamate

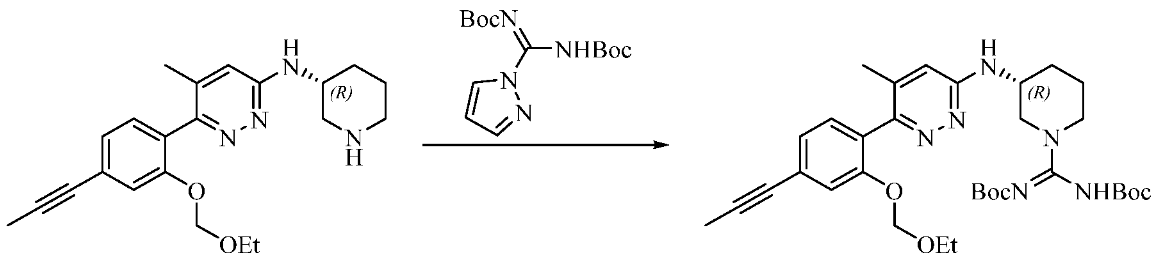

(*R*)-6-(2-(Ethoxymethoxy)-4-(prop-1-yn-1-yl)phenyl)-5-methyl-*N*-(piperidin-3-yl)pyridazin-3-amine (400.0 mg, 1.05 mmol, 1.0 eq) and *tert-butyl* (((*tert*-butoxycarbonyl)amino)(1*H*-pyrazol-1-yl)methylene)carbamate (391.5 mg, 1.26 mmol, 1.2 eq) were added to methanol (10.0 mL). The mixture was stirred at room temperature for 3 h, and TLC monitoring showed the completion of the reaction. The reaction mixture was concentrated under reduced pressure, and the crude product was purified by silica gel column chromatography (dichloromethane:methanol = 100:1 to 60:1) to give the product (400.0 mg, yield: 61.1%).

Step 2: Synthesis of (*R*)-3-((6-(2-hydroxy-4-(prop-1-yn-1-yl)phenyl)-5-methylpyridazin-3-yl)amino)piperidine-1-carboximidamide trifluoroacetate

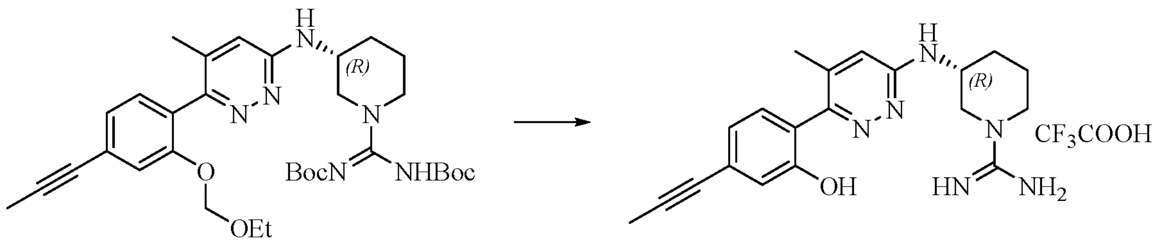

*tert*-Butyl (*R*)-(((*tert*-butoxycarbonyl)imino)(3-((6-(2-(ethoxymethoxy)-4-(prop-1-yn-1-yl)phenyl)-5-methylpyridazin-3-yl)amino)piperidin-1-yl)methyl)carbamate (340.0 mg, 0.54 mmol, 1.0 eq) was added to dichloromethane (5.0 mL), and trifluoroacetic acid (5.0 mL) was added dropwise. The mixture was allowed to react at room temperature for 4 h, and LC-MS monitoring showed the completion of the reaction. Water (30.0 mL) was added, and extraction was performed with dichloromethane (100.0 mL). The aqueous phase was purified by reversed phase column chromatography (water:acetonitrile = 4:6) to give the product (180.0 mg, yield: 69.2%).

$^1$HNMR(400MHz, DMSO-$d_6$) $\delta$(ppm): 10.55(s,1H), 8.72(s,1H), 7.57(s,4H), 7.37-7.36(m,1H), 7.20-7.18(d, *J*=8Hz,1H), 6.98-6.95(m,2H), 3.99-3.98(m,1H),3.90-3.87(m,1H), 3.69-3.66(m,1H), 3.24-3.13(m,2H), 2.17(s,3H), 2.06(s,4H), 1.85-1.83(m, 1H), 1.67-1.55(m,2H).

Molecular formula: $C_{22}H_{25}F_3N_6O_3$ Exact molecular weight of the free base: 364.20 LC-MS (Pos, *m*/*z*) = 365.21

[M+H]$^+$.

**Example 22: Synthesis of (*R*)-5-ethynyl-2-(4-isopropyl-6-((1-methylpiperidin-3-yl)amino)pyridazin-3-yl)phenol (Compound 81)**

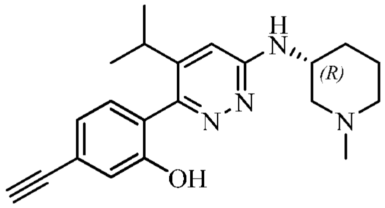

Step 1: Synthesis of 3,6-dichloro-4-isopropylpyridazine

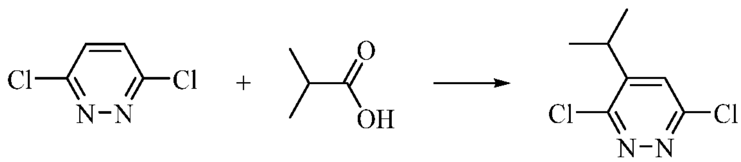

3,6-Dichloropyridazine (10.0 g, 67.12 mmol, 1.0 eq), isobutyric acid (5.9 g, 67.12 mmol, 1.0 eq), and silver nitrate (11.4 g, 67.12 mmol, 1.0 eq) were added to water (100 mL), and the mixture was heated to 50 °C. Concentrated sulfuric acid (11.0 mL) was added dropwise, followed by the dropwise addition of an aqueous solution (100.0 mL) of ammonium persulfate (45.9 g, 201.36 mmol, 3.0 eq). The resulting mixture was heated to 70 °C and allowed to react for 2 h, and TLC analysis showed the completion of the reaction. The reaction mixture was then cooed and filtered under vacuum. The pH of the filtrate was adjusted to 9 with a 2.0 mol/L aqueous sodium hydroxide solution, and extraction was performed with ethyl acetate (400 mL). The organic phase was dried and filtered, and the resulting filtrate was concentrated under reduced pressure to give the product (10.1 g, yield: 78.9%).

Step 2: Synthesis of *tert-butyl* (*R*)-3-((6-chloro-5-isopropylpyridazin-3-yl)amino)piperidine-1-carboxylate

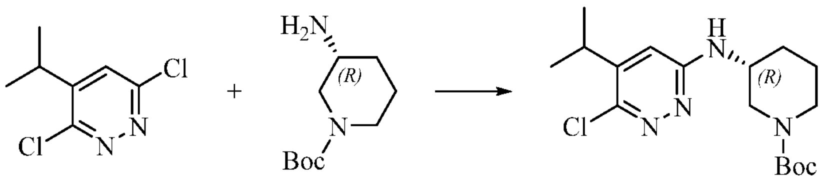

3,6-Dichloro-4-isopropylpyridazine (5.0 g, 26.17 mmol, 1.0 eq), (*R*)-1-*tert*-butoxycarbonyl-3-aminopiperidine (5.76 g, 28.79 mmol, 1.1 eq), and *N,N*-diisopropylethylamine (6.7 g, 52.34 mmol, 2.0 eq) were added to *N,N*-dimethylacetamide (50.0 mL). The mixture was allowed to react at 120 °C for 72 h, and TLC analysis showed the completion of the reaction. Ethyl acetate (200.0 mL) was added, and the mixture was washed with water (100 mL). The organic phase was dried and filtered. The filtrate was concentrated under reduced pressure, and the crude product was purified by silica gel column chromatography (dichloromethane:methanol = 200:1 to 20:1) to give the product (3.8 g, yield: 40.9%).

Step 3: Synthesis of (*R*)-6-chloro-5-isopropyl-*N*-(piperidin-3-yl)pyridazin-3-amine

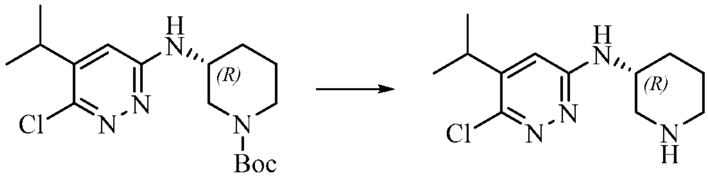

*tert-Butyl* (*R*)-3-((6-chloro-5-isopropylpyridazin-3-yl)amino)piperidine-1-carboxylate (3.7 g, 10.43 mmol, 1.0 eq) was added to dichloromethane (10.0 mL), and a solution of hydrogen chloride in 1,4-dioxane (4.0 mol/L, 10.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure to give the product (2.5 g, yield: 96.1%).

Step 4: Synthesis of (*R*)-6-chloro-5-isopropyl-*N*-(1-methylpiperidin-3-yl)pyridazin-3-amine

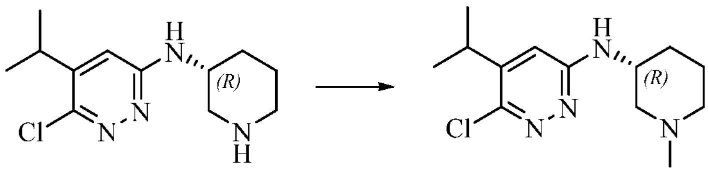

(*R*)-6-Chloro-5-isopropyl-*N*-(piperidin-3-yl)pyridazin-3-amine (860.0 mg, 3.37 mmol, 1.0 eq) and an aqueous formaldehyde solution (mass fraction: 37%, 356.1 mg, 4.38 mmol, 1.3 eq) were added to methanol (12.0 mL), and the mixture was stirred at room temperature for 1 h. Then, sodium cyanoborohydride (318.1 mg, 5.05 mmol, 1.5 eq) was added, and the resulting mixture was allowed to react at room temperature for 2 h. TLC monitoring showed the completion of the reaction. The reaction mixture was concentrated under reduced pressure, and a saturated aqueous sodium bicarbonate solution (100.0 mL) was added. The resulting mixture was stirred for 0.5 h and extracted with dichloromethane (100.0 mL). The organic phase was dried over anhydrous sodium sulfate and filtered. The filtrate was concentrated under reduced pressure, and the crude product was purified by silica gel column chromatography (dichloromethane:methanol = 50:1 to 10:1) to give the product (770.0 mg, yield: 84.9%).

Step 5: Synthesis of (*R*)-3-(ethoxymethoxy)-4-(4-isopropyl-6-((1-methylpiperidin-3-yl)amino)pyridazin-3-yl)be nzaldehyde

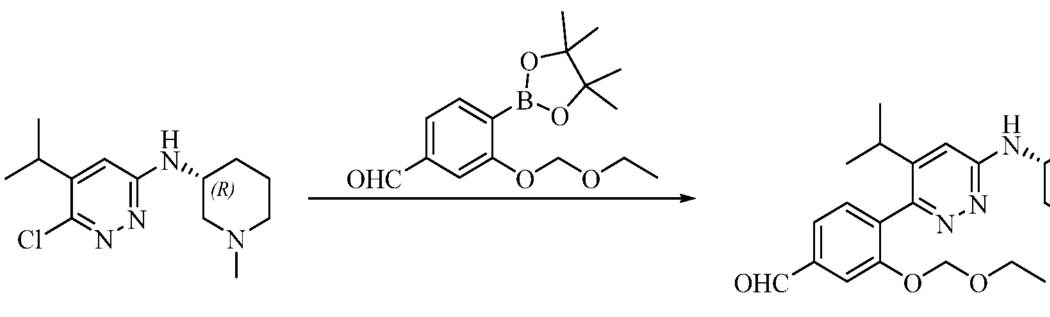

(*R*)-6-Chloro-5-isopropyl-*N*-(1-methylpiperidin-3-yl)pyridazin-3-amine (352.5 mg, 1.31 mmol, 1.0 eq), 3-(ethoxymethoxy)-4-(4,4,5,5-tetramethyl-1,3,2-dioxaborolan-2-yl)benzaldehyde (441.6 mg, 1.44 mmol, 1.1 eq), sodium bicarbonate (220.0 mg, 2.62 mmol, 2.0 eq), and Pd(dppf)$Cl_2$ (47.9 mg, 0.06 mmol, 0.05 eq) were added to a mixed solution of 1,4-dioxane (10.0 mL) and water (5.0 mL). The mixture was allowed to react at 110 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 50:1 to 10:1) to give the product (230.0 mg, yield: 42.6%).

Step 6: Synthesis of (*R*)-6-(2-(ethoxymethoxy)-4-ethynylphenyl)-5-isopropyl-*N*-(1-methylpiperidin-3-yl)pyridaz in-3-amine

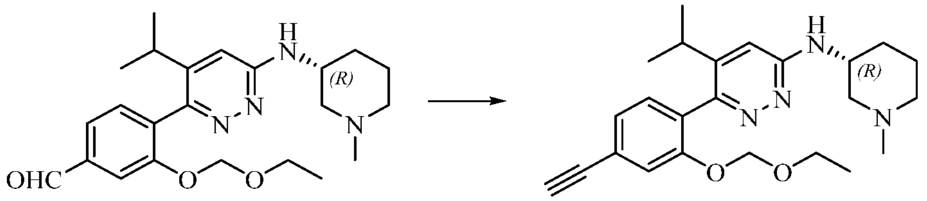

(*R*)-3-(Ethoxymethoxy)-4-(4-isopropyl-6-((1-methylpiperidin-3-yl)amino)pyri dazin-3-yl)benzaldehyde (223.0 mg, 0.54 mmol, 1.0 eq), dimethyl (1-diazo-2-oxopropyl)phosphonate (155.7 mg, 0.81 mmol, 1.5 eq), and anhydrous potassium carbonate (149.2 mg, 1.08 mmol, 2.0 eq) were added to methanol (10.0 mL), and the mixture was allowed to react at room temperature for 12 h. LC-MS monitoring showed the completion of the reaction. The reaction mixture was concentrated under reduced pressure, and the crude product was purified by silica gel column chromatography (dichloromethane:methanol = 50:1 to 10:1) to give the product (200.0 mg, yield: 90.9%).

Step 7: Synthesis of (*R*)-5-ethynyl-2-(4-isopropyl-6-((1-methylpiperidin-3-yl)amino)pyridazin-3-yl)phenol

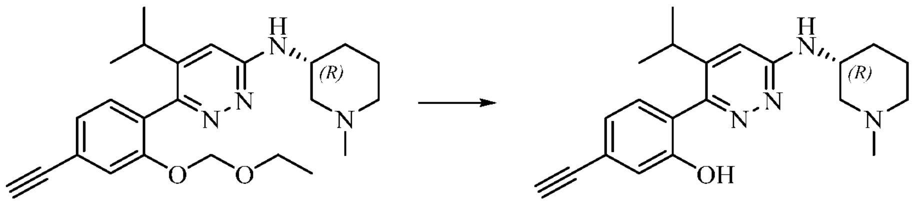

(*R*)-6-(2-(Ethoxymethoxy)-4-ethynylphenyl)-5-isopropyl-*N*-(1-methylpiperidi n-3-yl)pyridazin-3-amine (200.0 mg, 0.49 mmol, 1.0 eq) was added to dichloromethane (5.0 mL), and a solution of hydrogen chloride in 1,4-dioxane (4.0 mol/L, 5.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (110.0 mg, yield: 64.3%).

$^1$H-NMR (400 MHz, DMSO-*d*$_6$) δ(ppm): 9.90 (s, 1H), 7.15-7.13 (d, *J*=8Hz, 1H), 7.00-6.99 (m, 2H), 6.77-6.73 (m, 2H), 4.17 (s, 2H), 3.13 (s, 1H), 2.82(s, 1H), 2.65-2.59 (m, 1H), 2.50 (s, 3H), 2.41 (s, 3H), 1.89-1.80 (m, 2H), 1.66-1.64 (m, 1H), 1.03-1.01 (m, 6H).

Molecular formula: $C_{21}H_{26}N_4O$ Exact molecular weight: 350.21 LC-MS *(m/z):* 351.25 [M+H]$^+$.

**Example 23: Synthesis of (*R*)-5-ethynyl-2-(4-cyclobutyl-6-((1-methylpiperidin-3-yl)amino)pyridazin-3-yl)phenol (Compound 82)**

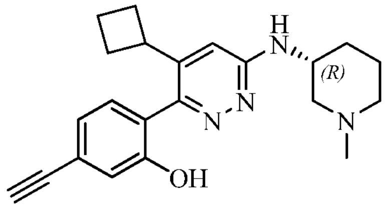

Step 1: Synthesis of 3,6-dichloro-4-cyclobutylpyridazine

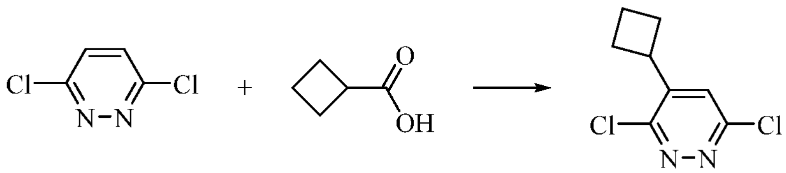

3,6-Dichloropyridazine (10.0 g, 67.12 mmol, 1.0 eq), cyclobutanecarboxylic acid (6.7 g, 67.12 mmol, 1.0 eq), and silver nitrate (11.4 g, 67.12 mmol, 1.0 eq) were added to water (100 mL), and the mixture was heated to 50 °C. Concentrated sulfuric acid (11.0 mL) was added dropwise, followed by the dropwise addition of an aqueous solution (100.0 mL) of ammonium persulfate (45.9 g, 201.36 mmol, 3.0 eq). The resulting mixture was heated to 70 °C and allowed to react for 2 h, and TLC analysis showed the completion of the reaction. The reaction mixture was then cooed and filtered under vacuum. The pH of the filtrate was adjusted to 9 with a 2.0 mol/L aqueous sodium hydroxide solution, and extraction was performed with ethyl acetate (400 mL). The organic phase was dried and filtered, and the resulting filtrate was concentrated under reduced pressure to give the product (9.8 g, yield: 72.0%).

Step 2: Synthesis of *tert-butyl* (*R*)-3-((6-chloro-5-cyclobutylpyridazin-3-yl)amino)piperidine-1-carboxylate

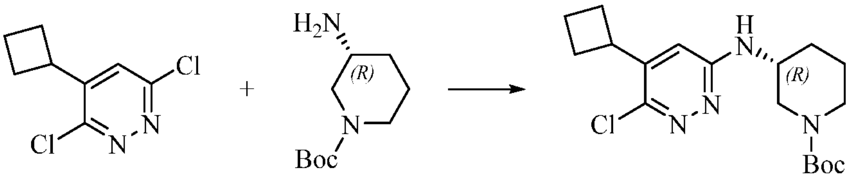

3,6-Dichloro-4-cyclobutylpyridazine (5.0 g, 24.62 mmol, 1.0 eq), (*R*)-1-*tert*-butoxycarbonyl-3-aminopiperidine (7.4 g, 36.93 mmol, 1.5 eq), and *N,N*-diisopropylethylamine (6.3 g, 49.24 mmol, 2.0 eq) were added to *N,N*-dimethylacetamide (50.0 mL). The mixture was allowed to react at 120 °C for 72 h, and TLC analysis showed the completion of the reaction. Ethyl acetate (200.0 mL) was added, and the mixture was washed with water (100 mL) and a saturated aqueous ammonium chloride solution (100 mL). The organic phase was dried and filtered. The filtrate was concentrated, and the crude product was purified by silica gel column chromatography (dichloromethane:methanol = 200:1 to 100:1) to give the product (1.8 g, yield: 20.0%).

Step 3: Synthesis of (*R*)-6-chloro-5-cyclobutyl-*N*-(piperidin-3-yl)pyridazin-3-amine

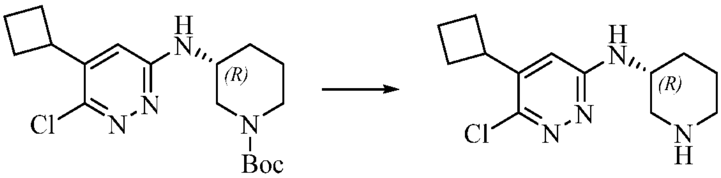

*tert*-Butyl (*R*)-3-((6-chloro-5-cyclobutylpyridazin-3-yl)amino)piperidine-1-carboxylate (1.8 g, 4.91 mmol, 1.0 eq) was added to dichloromethane (10.0 mL), and a solution of hydrogen chloride in 1,4-dioxane (4.0 mol/L, 10.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure to give the product (1.2 g, yield: 92.3%).

Step 4: Synthesis of (*R*)-6-chloro-5-cyclobutyl-*N*-(1-methylpiperidin-3-yl)pyridazin-3-amine

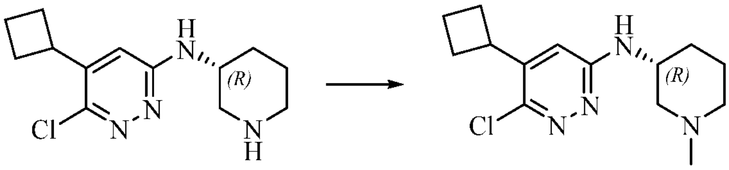

(*R*)-6-Chloro-5-cyclobutyl-*N*-(piperidin-3-yl)pyridazin-3-amine (1.17 g, 4.38 mmol, 1.0 eq) and an aqueous formaldehyde solution (mass fraction: 37%, 462.7 mg, 5.69 mmol, 1.3 eq) were added to methanol (15.0 mL), and the mixture was stirred at room temperature for 1 h. Sodium cyanoborohydride (413.3 mg, 6.57 mmol, 1.5 eq) was added, and the resulting mixture was allowed to react at room temperature for 2 h. TLC monitoring showed the completion of the reaction. The reaction mixture was concentrated under reduced pressure, and a saturated aqueous sodium bicarbonate solution (100.0 mL) was added. The resulting mixture was stirred for 0.5 h and extracted with dichloromethane (100.0 mL). The organic phase was dried over anhydrous sodium sulfate and filtered. The filtrate was concentrated under reduced pressure, and the crude product was purified by silica gel column chromatography (dichloromethane:methanol = 50:1 to 10:1) to give the product (1.1 mg, yield: 91.6%).

Step 5: Synthesis of (*R*)-3-(ethoxymethoxy)-4-(4-cyclobutyl-6-((1-methylpiperidin-3-yl)amino)pyridazin-3-yl)b enzaldehyde

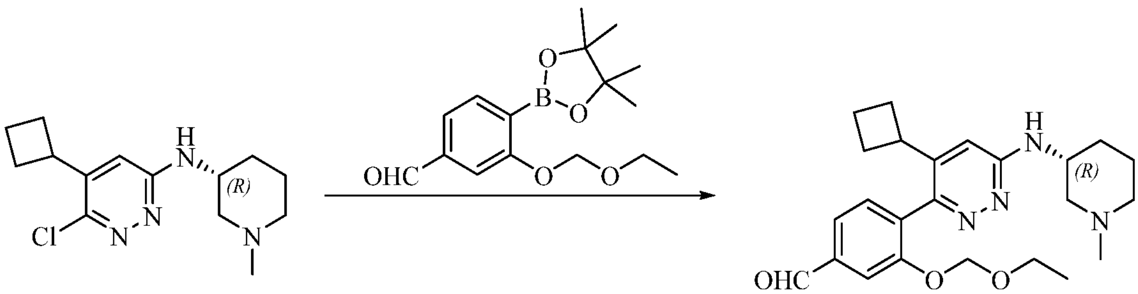

(*R*)-6-Chloro-5-cyclobutyl-*N*-(1-methylpiperidin-3-yl)pyridazin-3-amine (330.0 mg, 1.17 mmol, 1.0 eq), 3-(ethoxymethoxy)-4-(4,4,5,5-tetramethyl-1,3,2-dioxaborolan-2-yl)benzaldehyde (395.8 mg, 1.28 mmol, 1.1 eq), sodium bicarbonate (197.4 mg, 2.34 mmol, 2.0 eq), and Pd(dppf)$Cl_2$ (42.9 mg, 0.06 mmol, 0.05 eq) were added to a mixed solution of 1,4-dioxane (10.0 mL) and water (5.0 mL). The mixture was allowed to react at 110 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 50:1 to 10:1) to give the product (317.2 mg, yield: 63.5%).

Step 6: Synthesis of (*R*)-6-(2-(ethoxymethoxy)-4-ethynylphenyl)-5-cyclobutyl-*N*-(1-methylpiperidin-3-yl)pyrida zin-3-amine

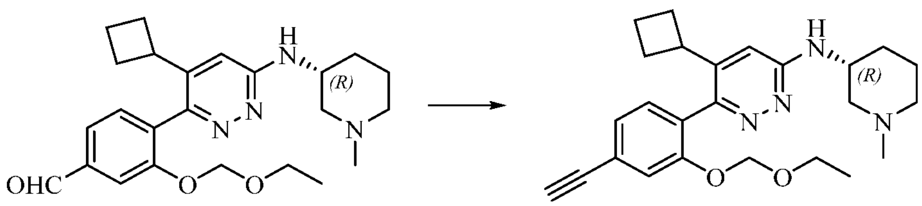

(*R*)-3-(Ethoxymethoxy)-4-(4-cyclobutyl-6-((1-methylpiperidin-3-yl)amino)py ridazin-3-yl)benzaldehyde (314.0 mg, 0.74 mmol, 1.0 eq), dimethyl (1-diazo-2-oxopropyl)phosphonate (213.1 mg, 1.11 mmol, 1.5 eq), and anhydrous potassium carbonate (204.2 mg, 1.48 mmol, 2.0 eq) were added to methanol (10.0 mL). The mixture was allowed to react at room temperature for 12 h, and LC-MS monitoring showed the completion of the reaction. The reaction mixture was concentrated under reduced pressure, and the crude product was purified by silica gel column chromatography (dichloromethane:methanol = 50:1 to 10:1) to give the product (280.0 mg, yield: 90.1%).

Step 7: Synthesis of (*R*)-5-ethynyl-2-(4-cyclobutyl-6-((1-methylpiperidin-3-yl)amino)pyridazin-3-yl)phenol

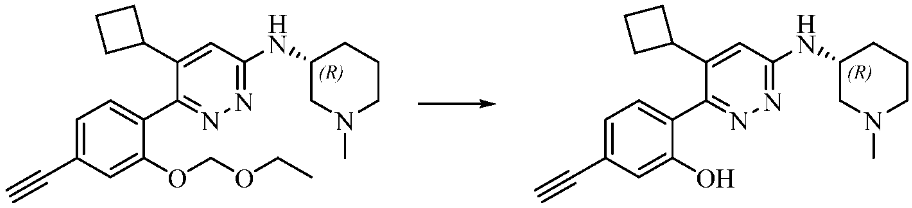

(*R*)-6-(2-(Ethoxymethoxy)-4-ethynylphenyl)-5-cyclobutyl-*N*-(1-methylpiperid in-3-yl)pyridazin-3-amine (280.0 mg, 0.66 mmol, 1.0 eq) was added to dichloromethane (10.0 mL), and a solution of hydrogen chloride in 1,4-dioxane (4.0 mol/L, 5.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (140.0 mg, yield: 58.0%).

$^1$H-NMR(400MHz, DMSO-$d_6$) δ(ppm): 9.89 (s, 1H), 7.10-7.08 (d, *J*=8Hz, 1H), 6.97-6.96 (m, 2H), 6.75 (s, 1H), 6.64-6.63 (m, 1H), 4.17 (s, 1H), 4.10-4.08 (m, 1H), 3.39-3.35 (m, 1H), 2.91 (s, 1H), 2.59(s, 1H), 2.23(s, 3H), 2.11(s, 1H), 1.99(s, 1H), 1.88-1.83(m, 6H), 1.81-1.52(m, 4H).

Molecular formula: $C_{22}H_{26}N_4O$ Exact molecular weight: 362.21 LC-MS (*m/z*): 363.26 $[M+H]^+$.

**Example 24: Synthesis of 5-ethynyl-2-(6-(((1*R*,2*R*)-2-hydroxycyclohexyl) amino)-4-methylpyridazin-3-yl)phenol (Compound 83)**

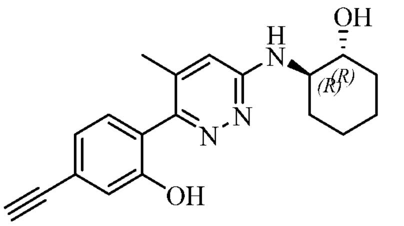

Step 1: Synthesis of (1*R*,2*R*)-2-((6-chloro-5-methylpyridazin-3-yl)amino)cyclohexan-1-ol

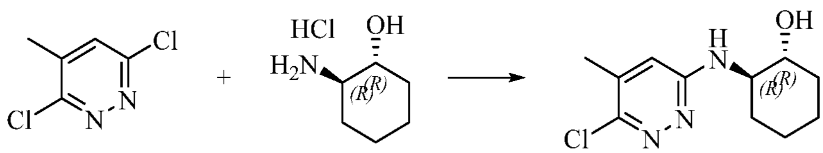

3,6-Dichloro-4-methylpyridazine (3.2 g, 19.63 mmol, 1.0 eq), (1*R*,2*R*)-2-aminocyclohexan-1-ol hydrochloride (3.8 g, 25.52 mmol, 1.3 eq), and *N*,*N*-diisopropylethylamine (5.0 g, 39.26 mmol, 2.0 eq) were added to *N*,*N*-dimethylacetamide (20.0 mL). The mixture was stirred at 120 °C for 72 h, and TLC monitoring showed the completion of the reaction. The reaction mixture was cooled to room temperature. Ethyl acetate (100.0 mL) was added, and the resulting

mixture was washed with water (100.0 mL). The organic phase was dried over anhydrous sodium sulfate and filtered. The filtrate was concentrated under reduced pressure, and the crude product was purified by silica gel column chromatography (dichloromethane:methanol = 200:1 to 50:1) to give the product (1.2 g, yield: 25.5%).

Step 2: Synthesis of 3-(ethoxymethoxy)-4-(6-(((1*R*,2*R*)-2-hydroxycyclohexyl) amino)-4-methylpyridazin-3-yl)benzaldehyde

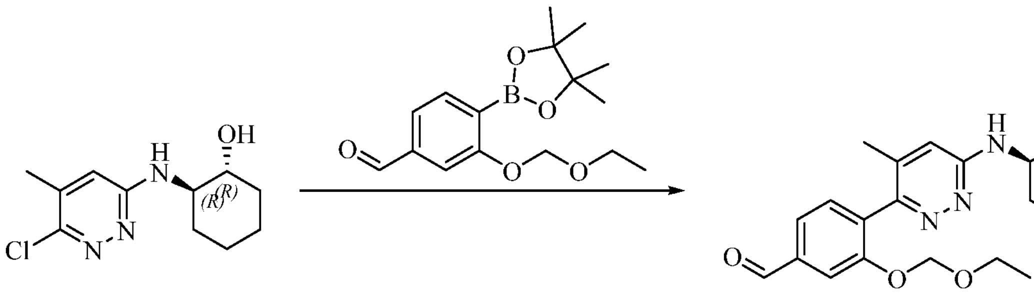

(1*R*,2*R*)-2-((6-Chloro-5-methylpyridazin-3-yl)amino)cyclohexan-1-ol (500.0 mg, 2.06 mmol, 1.0 eq), 3-(ethoxymethoxy)-4-(4,4,5,5-tetramethyl-1,3,2-dioxaborolan-2-yl)benzaldehyde (696.6 mg, 2.27 mmol, 1.1 eq), sodium bicarbonate (347.4 mg, 4.12 mmol, 2.0 eq), and Pd(dppf)$Cl_2$ (75.6 mg, 0.10 mmol, 0.05 eq) were added to a mixed solution of 1,4-dioxane (10.0 mL) and water (5.0 mL). The mixture was allowed to react at 110 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 80:1 to 30:1) to give the product (454.0 mg, yield: 56.9%).

Step 3: Synthesis of (1*R*,2*R*)-2-((6-(2-(ethoxymethoxy)-4-ethynylphenyl)-5-methylpyridazin-3-yl)amino)cycloh exan-1-ol

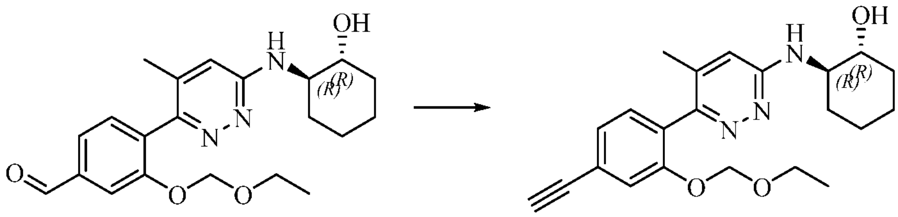

3-(Ethoxymethoxy)-4-(6-(((1*R*,2*R*)-2-hydroxycyclohexyl)amino)-4-methylpyr idazin-3-yl)benzaldehyde (454.0 mg, 1.18 mmol, 1.0 eq), dimethyl (1-diazo-2-oxopropyl)phosphonate (339.4 mg, 1.77 mmol, 1.5 eq), and anhydrous potassium carbonate (325.3 mg, 2.36 mmol, 2.0 eq) were added to methanol (15.0 mL). The mixture was allowed to react at room temperature for 12 h, and TLC monitoring showed the completion of the reaction. The reaction mixture was concentrated under reduced pressure, and the crude product was purified by silica gel column chromatography (dichloromethane:methanol = 60:1 to 20:1) to give the product (447.0 mg, yield: 99.5%).

Step 4: Synthesis of 5-ethynyl-2-(6-(((1*R*,2*R*)-2-hydroxycyclohexyl)amino)-4-methylpyridazin-3-yl)phenol

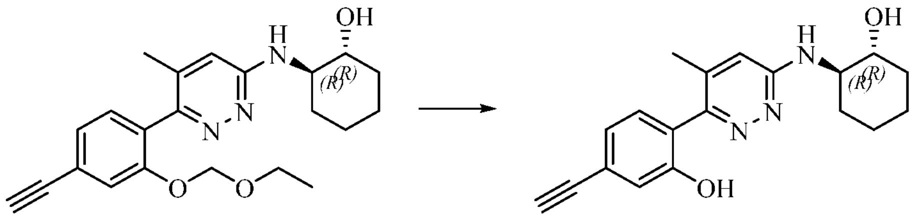

(1*R*,2*R*)-2-((6-(2-(Ethoxymethoxy)-4-ethynylphenyl)-5-methylpyridazin-3-yl) amino)cyclohexan-1-ol (447.0 mg, 1.17 mmol, 1.0 eq) was added to dichloromethane (5.0 mL), and a solution of hydrogen chloride in 1,4-dioxane (4.0 mol/L, 5.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by silica gel column chromatography (dichloromethane:methanol = 40:1 to 20:1) to give the product (260.0 mg, yield: 68.6%).

$^1$HNMR (400 MHz, DMSO-$d_6$) δ(ppm): 10.13 (s, 1H), 7.19-7.17 (d, *J*=8 Hz, 1H), 7.00-6.98 (m, 2H), 6.70 (s, 1H), 6.57-6.55 (d, *J*=8 Hz, 1H), 4.79-4.78 (d, *J*=4 Hz, 1H), 4.17 (s, 1H), 3.66-3.64 (m, 1H), 3.38-3.36 (m, 1H), 2.05 (s, 1H), 2.03 (s, 3H), 1.92-1.89 (m, 1H), 1.67-1.62 (m, 2H), 1.34-1.16 (m, 4H).

Molecular formula: $C_{19}H_{21}N_3O_2$ Exact molecular weight: 323.16 LC-MS (Pos, *m/z*) = 324.21 [M+H]$^+$.

**Example 25: Synthesis of 2-(6-(((1*S*,2*R*)-2-hydroxy-2-methylcyclohexyl) amino)-4-methylpyridazin-3-yl)-5-(prop-1-yn-1-yl)phenol (Compound 80)**

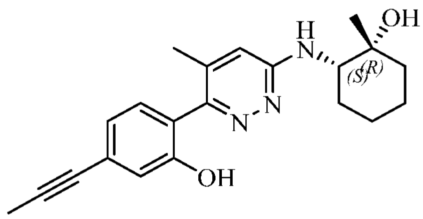

Step 1: Synthesis of (1*R*,2*S*)-2-((6-chloro-5-methylpyridazin-3-yl)amino)cyclohexan-1-ol

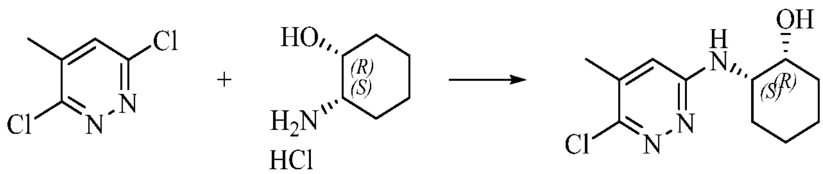

3,6-Dichloro-4-methylpyridazine (3.8 g, 23.33 mmol, 1.0 eq), (1*R*,2*S*)-2-aminocyclohexan-1-ol hydrochloride (4.6 g, 30.33 mmol, 1.3 eq), and *N,N*-diisopropylethylamine (9.0 g, 69.99 mmol, 3.0 eq) were added to *N,N*-dimethylacetamide (20.0 mL). The mixture was stirred at 120 °C for 72 h, and TLC monitoring showed the completion of the reaction. The reaction mixture was cooled to room temperature. Ethyl acetate (100.0 mL) was added, and the resulting mixture was washed with water (100.0 mL). The organic phase was dried over anhydrous sodium sulfate and filtered. The filtrate was concentrated under reduced pressure, and the crude product was purified by silica gel column chromatography (dichloromethane:methanol = 200:1 to 20:1) to give the product (1.4 g, yield: 25.0%).

Step 2: Synthesis of (*S*)-2-((5,6-dimethylpyridazin-3-yl)amino)cyclohexan-1-one

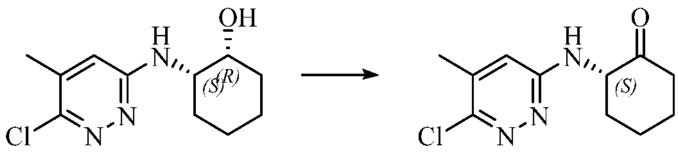

*N*-Chlorosuccinimide (2.25 g, 16.89 mmol, 3.0 eq) was added to dichloromethane (20.0 mL), and the mixture was cooled to 0 °C under a nitrogen atmosphere. Dimethyl sulfide (1.0 g, 16.89 mmol, 3.0 eq) was added dropwise, and the resulting mixture was allowed to react for 0.5 h and then cooled to -40 °C. A solution of (1*R*,2*S*)-2-((6-chloro-5-methylpyridazin-3-yl)amino)cyclohexan-1-ol (1.36 g, 5.63 mmol, 1.0 eq) in dichloromethane (20.0 mL) was added, and the resulting mixture was allowed to react at -40 °C for 1.5 h, followed by the dropwise addition of triethylamine (1.7 g, 16.89 mmol, 3.0 eq). The mixture was gradually warmed to room temperature and allowed to react for 14 h, and TLC monitoring showed the completion of the reaction. Dichloromethane (100.0 mL) was added, and the mixture was washed with water (100.0 mL). The organic phase was dried over anhydrous sodium sulfate and filtered. The filtrate was concentrated under reduced pressure, and the crude product was purified by silica gel column chromatography (petroleum ether:ethyl acetate = 10:1 to 2:1) to give the product (1.21 g, yield: 81.4%).

Step 3: Synthesis of (1*R*,2*S*)-2-((6-chloro-5-methylpyridazin-3-yl)amino)-1-methylcyclohexan-1-ol

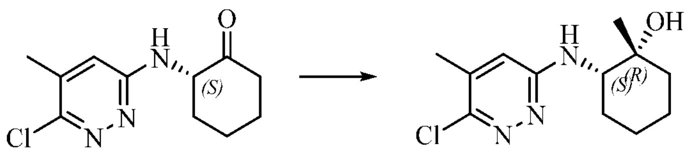

(*S*)-2-((5,6-Dimethylpyridazin-3-yl)amino)cyclohexan-1-one (995.0 mg, 4.15 mmol, 1.0 eq) was added to dry tetrahydrofuran (15.0 mL), and a solution of methylmagnesium chloride in tetrahydrofuran (3.0 mol/L, 5.5 mL, 16.60 mmol, 4.0 eq) was added dropwise under a nitrogen atmosphere. The mixture was allowed to react for 7 h. Then, ethyl acetate (100.0 mL) was added, and the resulting mixture was washed with a saturated aqueous ammonium chloride solution (100.0 mL). The organic phase was dried over anhydrous sodium sulfate and filtered. The filtrate was concentrated under reduced pressure, and the crude product was purified by silica gel column chromatography (dichloromethane:methanol = 130:1 to 100:1) to give the product (380.0 mg, yield: 35.8%).

Step 4: Synthesis of (1*R*,2*S*)-2-((6-(2-(ethoxymethoxy)-4-(prop-1-yn-1-yl)phenyl)-5-methylpyridazin-3-yl)amin o)-1-methylcyclohexan-1-ol

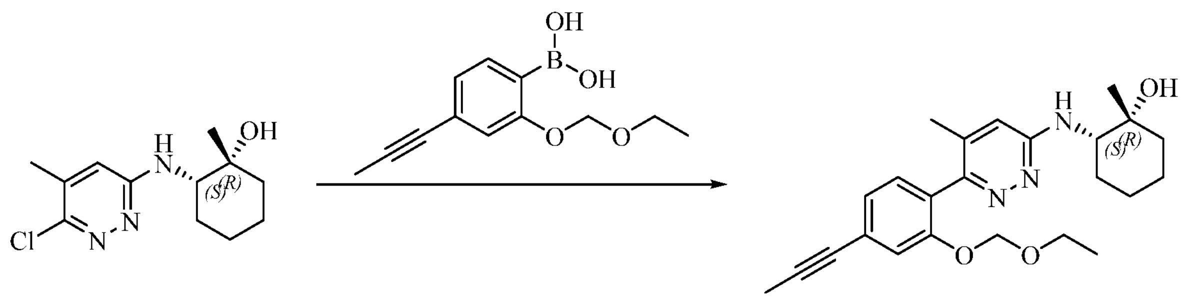

(1*R*,2*S*)-2-((6-Chloro-5-methylpyridazin-3-yl)amino)-1-methylcyclohexan-1-o l (380.0 mg, 1.48 mmol, 1.0 eq), (2-(ethoxymethoxy)-4-(prop-1-yn-1-yl)phenyl)boronic acid (417.3 mg, 1.77 mmol, 1.2 eq), sodium bicarbonate (249.6 mg, 2.96 mmol, 2.0 eq), and Pd(dppf)$Cl_2$ (54.3 mg, 0.07 mmol, 0.05 eq) were added to a mixed solution of 1,4-dioxane (10.0 mL) and water (5.0 mL). The mixture was allowed to react at 110 °C for 3 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 150:1 to 100:1) to give the product (210.0 mg, yield: 34.5%).

Step 5: Synthesis of 2-(6-(((1*S*,2*R*)-2-hydroxy-2-methylcyclohexyl)amino)-4-methylpyridazin-3-yl)-5-(prop-1-y n-1-yl) phenol

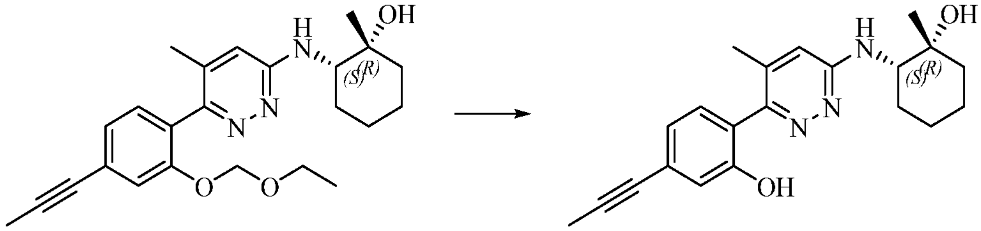

(1*R*,2*S*)-2-((6-(2-(Ethoxymethoxy)-4-(prop-1-yn-1-yl)phenyl)-5-methylpyrida zin-3-yl)amino)-1-methylcyclohexan-1-ol (200.0 mg, 0.49 mmol, 1.0 eq) was added to dichloromethane (4.0 mL), and a solution of hydrogen chloride in 1,4-dioxane (4.0 mol/L, 4.0 mL) was added dropwise. The mixture was allowed to react at room temperature for 12 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (53.0 mg, yield: 30.9%).

$^1$HNMR (400 MHz, DMSO-$d_6$) δ(ppm): 10.04 (s, 1H), 7.14-7.12 (d, *J*=8 Hz, 1H), 6.90-6.88 (m, 2H), 6.81 (s, 1H), 6.50-6.48 (d, *J*=8 Hz, 1H), 4.91 (s, 1H), 3.93-3.89 (m, 1H), 2.05 (s, 3H), 2.02 (s, 3H), 1.88-1.86 (m, 1H), 1.70-1.60 (m, 3H), 1.46-1.31 (m, 4H), 1.11 (s, 3H).

Molecular formula: $C_{21}H_{25}N_3O_2$ Exact molecular weight: 351.19 LC-MS (Pos, *m/z*) = 352.18 $[M+H]^+$.

**Example 26: Synthesis of (*R*)-2-(5-cyclopropyl-3-((1-cyclopropylpiperidin-3-yl)amino)-1,2,4-triazin-6-yl)-5-ethyn ylphenol (Compound 97)**

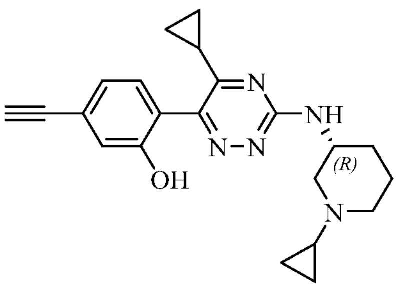

Step 1: Synthesis of *tert-butyl* (*R*)-3-((5-cyclopropyl-6-(2-(ethoxymethoxy)-4-formylphenyl)-1,2,4-triazin-3-yl)amino)pip eridine-1-carboxylate

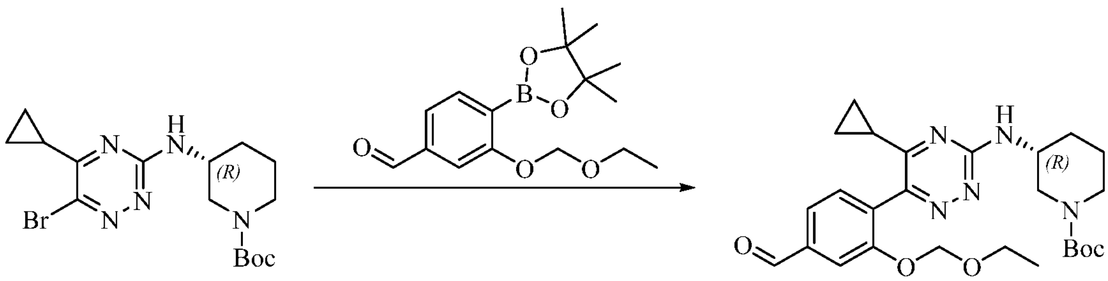

*tert*-Butyl (*R*)-3-((6-bromo-5-cyclopropyl-1,2,4-triazin-3-yl)amino)piperidine-1-carboxylate (4.68 g, 11.75 mmol, 1.0 eq), 3-(ethoxymethoxy)-4-(4,4,5,5-tetramethyl-1,3,2-dioxaborolan-2-yl)benzaldehyde (4.67 g, 15.27 mmol, 1.3 eq), sodium bicarbonate (1.97 g, 23.50 mmol, 2.0 eq), and Pd(dppf)$Cl_2$ (428.9 mg, 0.58 mmol, 0.05 eq) were added to a mixed solution of 1,4-dioxane (50.0 mL) and water (25.0 mL). The mixture was heated to 110 °C and allowed to react for 14 h under a nitrogen atmosphere, and TLC monitoring showed the completion of the reaction. Water (150 mL) was added, and extraction was performed with ethyl acetate (200.0 mL). The organic phase was dried over anhydrous sodium sulfate and filtered. The filtrate was concentrated under reduced pressure, and the crude product was purified by silica gel column chromatography (dichloromethane:methanol = 80:1 to 60:1) to give the product (2.1 g, yield: 36.2%).

Step 2: Synthesis of *tert*-butyl (*R*)-3-((5-cyclopropyl-6-(2-(ethoxymethoxy)-4-ethynylphenyl)-1,2,4-triazin-3-yl)amino) pip eridine-1-carboxylate

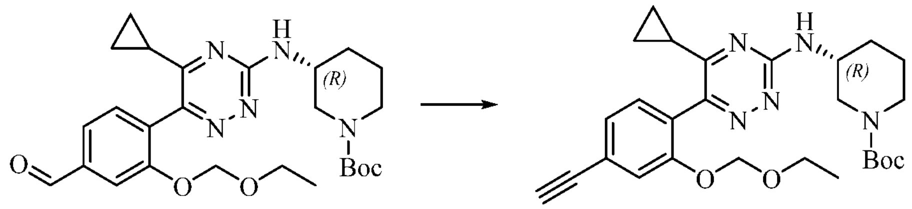

*tert*-Butyl (*R*)-3-((5-cyclopropyl-6-(2-(ethoxymethoxy)-4-formylphenyl)-1,2,4-triazin-3-yl)amino)pip eridine-1-carboxylate (2.0 g, 4.02 mmol, 1.0 eq), dimethyl (1-diazo-2-oxopropyl)phosphonate (1.1 g, 6.03 mmol, 1.5 eq), and anhydrous potassium carbonate (1.1 g, 8.04 mmol, 2.0 eq) were added to methanol (20.0 mL). The mixture was allowed to react at room temperature for 2 h, and TLC monitoring showed the completion of the reaction. The reaction mixture was concentrated under reduced pressure, and the crude product was purified by silica gel column chromatography (dichloromethane:methanol = 130:1 to 100:1) to give the product (1.6 g, yield: 80.8%).

Step 3: Synthesis of (*R*)-5-cyclopropyl-6-(2-(ethoxymethoxy)-4-ethynylphenyl)-*N*-(piperidin-3-yl)-1,2,4-triazin-3-amine

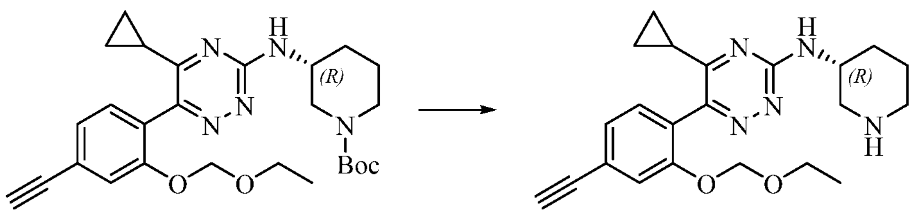

*tert*-Butyl (*R*)-3-((5-cyclopropyl-6-(2-(ethoxymethoxy)-4-ethynylphenyl)-1,2,4-triazin-3-yl)amino)pip eridine-1-carboxylate (1.6 g, 3.24 mmol, 1.0 eq) was added to dichloromethane (25.0 mL), and 2,6-dimethylpyridine (2.0 g, 19.44 mmol, 6.0 eq) was added dropwise. The mixture was cooled to 0 °C, and trimethylsilyl trifluoromethanesulfonate (2.1 g, 9.72 mmol, 3.0 eq) was added dropwise. The resulting mixture was gradually warmed to room temperature and allowed to react for 2 h, and TLC monitoring showed the completion of the reaction. Dichloromethane (100.0 mL) was added, and the mixture was washed with water (100.0 mL). The organic phase was dried over anhydrous sodium sulfate and filtered. The filtrate was concentrated under reduced pressure, and the crude product was purified by silica gel column chromatography (dichloromethane:methanol = 80:1 to 20:1) to give the product (603.0 mg, yield: 47.2%).

Step 4: Synthesis of (*R*)-5-cyclopropyl-*N*-(1-cyclopropylpiperidin-3-yl)-6-(2-(ethoxymethoxy)-4-ethynylphenyl) -1,2,4-triazin-3-amine

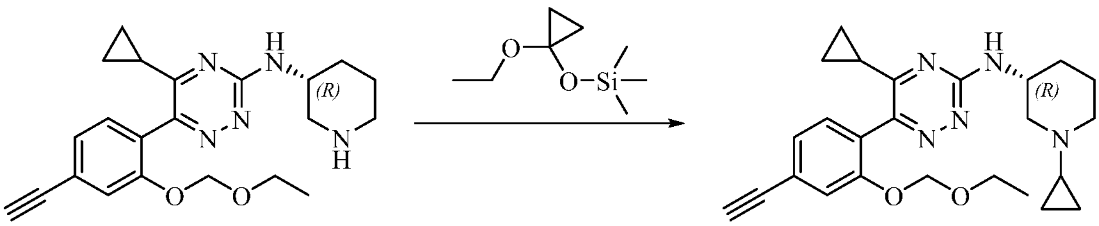

(*R*)-5-Cyclopropyl-6-(2-(ethoxymethoxy)-4-ethynylphenyl)-*N*-(piperidin-3-yl) -1,2,4-triazin-3-amine (603.0 mg, 1.53 mmol, 1.0 eq), (1-ethoxycyclopropoxy) trimethylsilane (1.0 g, 6.12 mmol, 4.0 eq), cesium fluoride (931.0 mg, 6.12 mol, 4.0 eq), and glacial acetic acid (0.5 mL) were added to methanol (20.0 mL). The mixture was stirred at 50 °C for 3 h, and then sodium cyanoborohydride (481.3 mg, 7.65 mmol, 5.0 eq) was added. The resulting mixture was allowed to react at 50 °C for 12 h. TLC monitoring showed the completion of the reaction. The reaction mixture was concentrated under reduced pressure. A saturated aqueous sodium bicarbonate solution (100.0 mL) was added, and the mixture was stirred for 0.5 h and extracted with dichloromethane (100.0 mL). The organic phase was dried over anhydrous sodium sulfate and filtered. The filtrate was concentrated under reduced pressure to give a crude product (712.0 mg, yield: based on 100%).

Step 5: Synthesis of (*R*)-2-(5-cyclopropyl-3-((1-cyclopropylpiperidin-3-yl)amino)-1,2,4-triazin-6-yl)-5-ethynylp henol

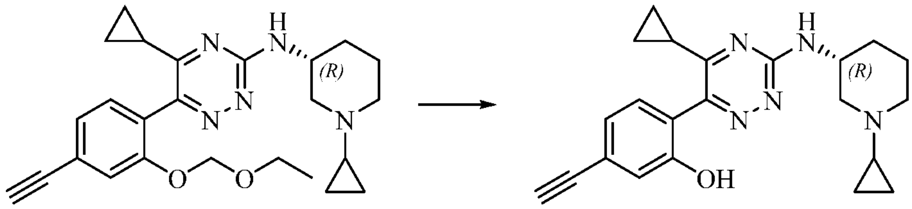

(*R*)-5-Cyclopropyl-*N*-(1-cyclopropylpiperidin-3-yl)-6-(2-(ethoxymethoxy)-4-e thynylphenyl)-1,2,4-triazin-3-amine (712.0 mg, crude product, 1.53 mmol, 1.0 eq) was added to dichloromethane (10.0 mL), and trifluoroacetic acid (10.0 mL) was added dropwise. The mixture was allowed to react at room temperature for 0.5 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (50.0 mg, yield: 8.7%).

$^1$H-NMR (400 MHz, DMSO-*d*$_6$) δ(ppm): 10.04 (s, 1H), 7.54 (s, 1H), 7.30-7.28 (d, *J*=8 Hz, 1H), 7.05-7.02 (m, 2H), 4.21 (s, 1H), 3.98-3.57 (m, 1H), 3.09-3.07 (d, *J*=8 Hz, 1H), 2.82-2.80 (d, *J*=8 Hz, 1H), 2.18-1.97 (m, 2H), 1.84-1.82 (m, 1H), 1.70-1.64 (m, 3H), 1.46-1.30 (m, 2H), 1.01-1.09 (m, 4H).

Molecular formula: $C_{22}H_{25}N_5O$ Exact molecular weight: 375.21 LC-MS (m/*z*): 376.25 [M+H]$^+$.

**Example 27: Synthesis of (*R*)-2-(5-cyclopropyl-3-((1-(2-hydroxyethyl) piperidin-3-yl)amino)-1,2,4-triazin-6-yl)-5-ethynylphenol (Compound 100)**

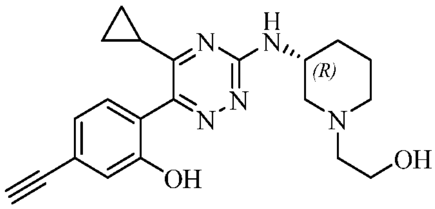

Step 1: Synthesis of (*R*)-6-bromo-5-cyclopropyl-*N*-(piperidin-3-yl)-1,2,4-triazin-3-amine

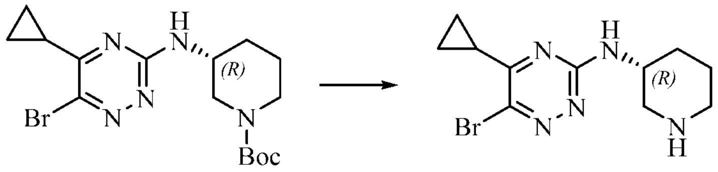

*tert*-Butyl (*R*)-3-((6-bromo-5-cyclopropyl-1,2,4-triazin-3-yl)amino)piperidine-1-carboxylate (2.4 g, 6.03 mmol, 1.0 eq) was added to dichloromethane (10.0 mL), and trifluoroacetic acid (5.0 mL) was added dropwise. The mixture was allowed to react at room temperature for 2 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure to give the product (1.75 g, yield: 97.7%).

Step 2: Synthesis of (*R*)-2-(3-((6-bromo-5-cyclopropyl-1,2,4-triazin-3-yl)amino)piperidin-1-yl)ethan-1-ol

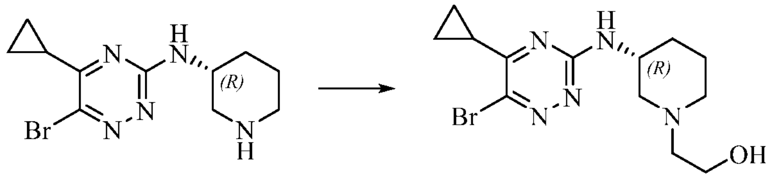

(*R*)-6-Bromo-5-cyclopropyl-*N*-(piperidin-3-yl)-1,2,4-triazin-3-amine (383.0 mg, 1.28 mmol, 1.0 eq), bromoethanol (481.5 mg, 3.84 mmol, 3.0 eq), and triethylamine (519.7 mg, 5.12 mmol, 4.0 eq) were added to dichloromethane (10.0 mL). The mixture was stirred at room temperature for 14 h, and TLC monitoring showed the completion of the reaction. Dichloromethane (100.0 mL) was added, and the resulting mixture was washed with a saturated aqueous ammonium chloride solution (50.0 mL). The organic phase was dried over anhydrous sodium sulfate and filtered. The filtrate was concentrated under reduced pressure, and the crude product was purified by silica gel column chromatography (dichloromethane:methanol = 50:1 to 10:1) to give the product (300.0 mg, yield: 68.5%).

Step 3: Synthesis of (*R*)-4-(5-cyclopropyl-3-((1-(2-hydroxyethyl)piperidin-3-yl)amino)-1,2,4-triazin-6-yl)-3-(eth oxymethoxy)benzaldehyde

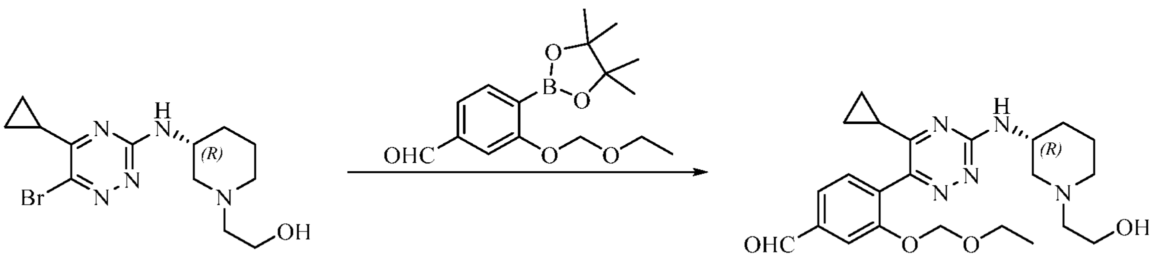

(*R*)-2-(3-((6-Bromo-5-cyclopropyl-1,2,4-triazin-3-yl)amino)piperidin-1-yl)eth an-1-ol (297.3 mg, 0.87 mmol, 1.0 eq), 3-(ethoxymethoxy)-4-(4,4,5,5-tetramethyl-1,3,2-dioxaborolan-2-yl)benzaldehyde (319.0 mg, 1.04 mmol, 1.2 eq), sodium bicarbonate (145.8 mg, 1.73 mmol, 2.0 eq), and Pd(dppf)$Cl_2$ (31.7 mg, 0.04 mmol, 0.05 eq) were added to a mixed solution of 1,4-dioxane (10.0 mL) and water (5.0 mL). The mixture was allowed to react at 110 °C for 5 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 40:1 to 10:1) to give the product (160.0 mg, yield: 41.7%).

Step 4: Synthesis of (*R*)-2-(3-((5-cyclopropyl-6-(2-(ethoxymethoxy)-4-ethynylphenyl)-1,2,4-triazin-3-yl)amino) piperidin-1-yl)ethan-1-ol

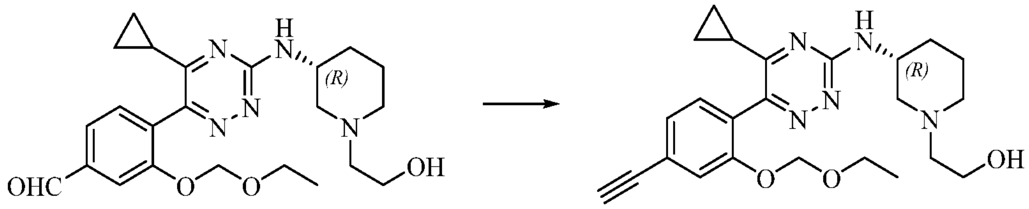

(*R*)-4-(5-Cyclopropyl-3-((1-(2-hydroxyethyl)piperidin-3-yl)amino)-1,2,4-triaz in-6-yl)-3-(ethoxymethoxy)benzaldehyde (160.0 mg, 0.36 mmol, 1.0 eq), dimethyl (1-diazo-2-oxopropyl)phosphonate (104.4 mg, 0.54 mmol, 1.5 eq), and anhydrous potassium carbonate (100.0 mg, 0.72 mmol, 2.0 eq) were added to methanol (10.0 mL). The mixture was allowed to react at room temperature for 12 h, and LC-MS monitoring showed the completion of the reaction. The reaction mixture was concentrated under reduced pressure, and the crude product was purified by silica gel column chromatography (dichloromethane:methanol = 50:1 to 10:1) to give the product (141.0 mg, yield: 89.0%).

Step 5: Synthesis of (*R*)-2-(5-cyclopropyl-3-((1-(2-hydroxyethyl)piperidin-3-yl)amino)-1,2,4-triazin-6-yl)-5-eth ynylphenol

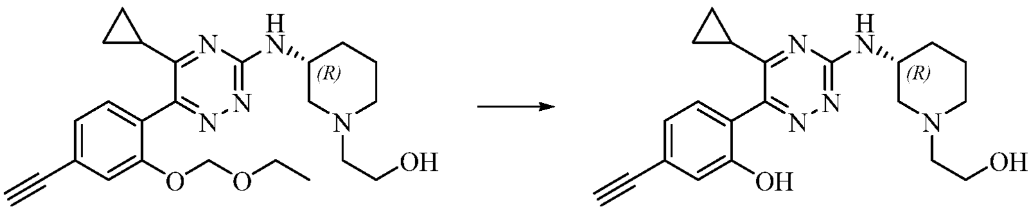

(*R*)-2-(3-((5-Cyclopropyl-6-(2-(ethoxymethoxy)-4-ethynylphenyl)-1,2,4-triazi n-3-yl)amino)piperidin-1-yl) ethan-1-ol (141.0 mg, 0.32 mmol, 1.0 eq) was added to dichloromethane (5.0 mL), and trifluoroacetic acid (1.0 mL) was added dropwise. The mixture was allowed to react at room temperature for 1 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (60.0 mg, yield: 49.5%).

$^1$HNMR (400 MHz, DMSO-$d_6$) δ(ppm): 10.03 (s, 1H), 7.61-7.53 (m, 1H), 7.30-7.28 (d, *J*=8 Hz, 1H), 7.05-7.02 (m, 2H), 4.42 (s, 1H), 4.21 (s, 1H), 4.10-3.72 (m, 1H), 3.51-3.48 (m, 2H), 2.91 (s, 1H), 2.67 (s, 1H), 2.40 (s, 2H), 1.78-1.64 (m, 3H), 1.52-1.36 (m, 2H), 1.02-0.96 (m, 4H).

Molecular formula: $C_{21}H_{25}N_5O_2$ Exact molecular weight: 379.20 LC-MS (Pos, *m*/*z*) = 380.24 $[M+H]^+$.

**Example 28: Synthesis of (*R*)-2-(5-cyclopropyl-3-((1-(methyl-*d*$_3$)piperidin-3-yl)amino)-1,2,4-triazin-6-yl)-5-ethyn ylphenol (Compound 87)**

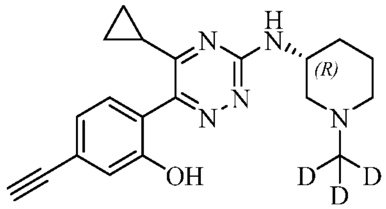

Step 1: Synthesis of (*R*)-6-bromo-5-cyclopropyl-N-(1-(methyl-$d_3$)piperidin-3-yl)-1,2,4-triazin-3-amine

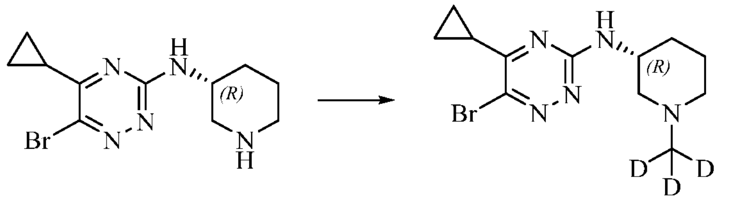

(*R*)-6-Bromo-5-cyclopropyl-*N*-(piperidin-3-yl)-1,2,4-triazin-3-amine (458.6 mg, 1.54 mmol, 1.0 eq), deuterated iodomethane (891.78 mg, 6.15 mmol, 4.0 eq), and triethylamine (778.1 mg, 7.69 mmol, 5.0 eq) were added to dichloromethane (10.0 mL), and the mixture was stirred at room temperature for 5 h. Dichloromethane (100.0 mL) was added, and the resulting mixture was washed with a saturated aqueous ammonium chloride solution (50.0 mL). The organic phase was dried over anhydrous sodium sulfate and filtered. The filtrate was concentrated under reduced pressure, and the crude product was purified by silica gel column chromatography (dichloromethane:methanol = 40:1 to 10:1) to give the product (225.0 mg, yield: 46.4%).

(*R*)-6-Bromo-5-cyclopropyl-*N*-(piperidin-3-yl)-1,2,4-triazin-3-amine (550.0 mg, 1.84 mmol, 1.0 eq), deuterated iodomethane (1.46 g, 10.12 mmol, 5.5 eq), and triethylamine (1.0 g, 10.12 mmol, 5.5 eq) were added to dichloromethane (10.0 mL), and the mixture was stirred at room temperature for 5 h. Dichloromethane (100.0 mL) was added, and the resulting mixture was washed with a saturated aqueous ammonium chloride solution (50.0 mL). The organic phase was dried over anhydrous sodium sulfate and filtered. The filtrate was concentrated under reduced pressure, and the crude product was purified by silica gel column chromatography (dichloromethane:methanol = 40:1 to 10:1) to give the product (235.5 mg, yield: 40.5%). The two products were combined to give the product (460.5 mg).

Step 2: Synthesis of (*R*)-4-(5-cyclopropyl-3-((1-(methyl-$d_3$)piperidin-3-yl)amino)-1,2,4-triazin-6-yl)-3-(ethoxym ethoxy) benzaldehyde

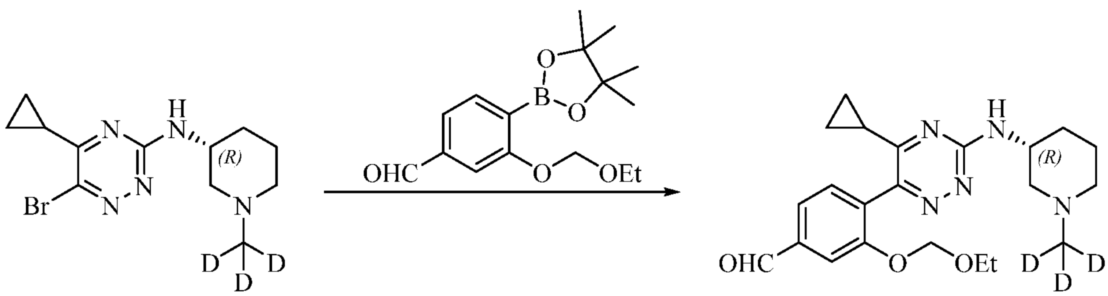

(*R*)-6-Bromo-5-cyclopropyl-*N*-(1-(methyl-$d_3$)piperidin-3-yl)-1,2,4-triazin-3-a mine (340.0 mg, 1.08 mmol, 1.0 eq), 3-(ethoxymethoxy)-4-(4,4,5,5-tetramethyl-1,3,2-dioxaborolan-2-yl)benzaldehyde (396.2 mg, 1.29 mmol, 1.2 eq), anhydrous potassium carbonate (297.9 mg, 2.16 mmol, 2.0 eq), and $Pd(PPh_3)_4$ (124.5 mg, 0.11 mmol, 0.1 eq) were added to a mixed solution of 1,4-dioxane (10.0 mL) and water (5.0 mL). The mixture was allowed to react at 100 °C for 12 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 40:1 to 10:1) to give the product (260.0 mg, yield: 58.2%).

Step 4: Synthesis of (*R*)-5-cyclopropyl-6-(2-(ethoxymethoxy)-4-ethynylphenyl)-*N*-(1-(methyl-$d_3$)piperidin-3-yl) -1,2,4-triazin-3-amine

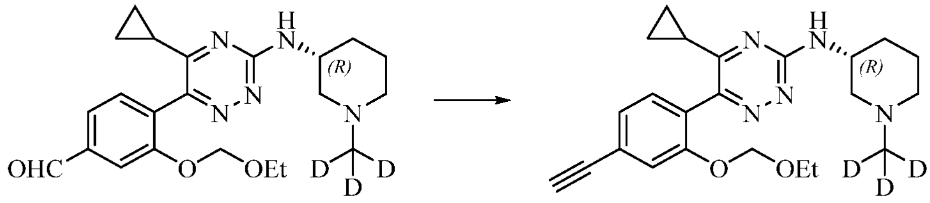

(*R*)-4-(5-Cyclopropyl-3-((1-(methyl-$d_3$)piperidin-3-yl)amino)-1,2,4-triazin-6-y l)-3-(ethoxymethoxy)benzaldehyde (260.0 mg, 0.63 mmol, 1.0 eq), dimethyl (1-diazo-2-oxopropyl)phosphonate (180.7 mg, 0.95 mmol, 1.5 eq), and anhydrous potassium carbonate (173.3 mg, 1.26 mmol, 2.0 eq) were added to methanol (15.0 mL). The mixture was allowed to react at room temperature for 4 h, and LC-MS monitoring showed the completion of the reaction. The reaction mixture was concentrated under reduced pressure, and the crude product was purified by silica gel column chromatography (dichloromethane:methanol = 40:1 to 10:1) to give the product (188.0 mg, yield: 72.7%).

Step 5: Synthesis of (*R*)-2-(5-cyclopropyl-3-((1-(methyl-$d_3$)piperidin-3-yl)amino)-1,2,4-triazin-6-yl)-5-ethynylp henol

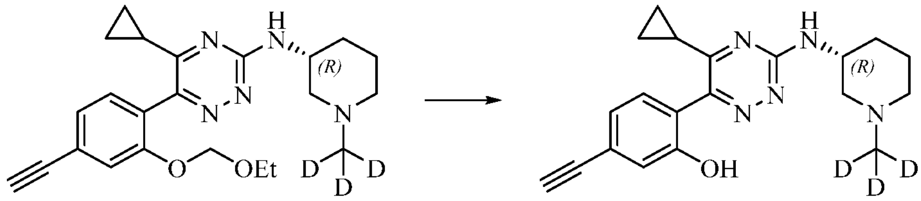

(*R*)-5-Cyclopropyl-6-(2-(ethoxymethoxy)-4-ethynylphenyl)-*N*-(1-(methyl-$d_3$)p iperidin-3-yl)-1,2,4-triazin-3-amine (188.0 mg, 0.46 mmol, 1.0 eq) was added to dichloromethane (5.0 mL), and trifluoroacetic acid (2.0 mL) was added dropwise. The mixture was allowed to react at room temperature for 1 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (110.0 mg, yield: 67.9%).

$^1$HNMR(400MHz, DMSO-$d_6$) δ(ppm): 10.04 (s, 1H), 7.55-7.43 (m, 1H), 7.30-7.28 (d, *J*=8 Hz, 1H), 7.05-7.01 (m, 2H), 4.21 (s, 1H), 3.92-3.85 (m, 1H), 2.85-2.84 (m, 1H), 2.62 (s, 1H), 1.92-1.80 (m, 3H), 1.70-1.64 (m, 2H), 1.53-1.50(m, 1H), 1.34-1.23(m, 1H), 1.03-0.95(m, 4H).

Molecular formula: $C_{20}H_{20}D_3N_5O$ Exact molecular weight: 352.21 LC-MS (Pos, *m/z)* = 353.23 [M+H]$^+$.

**Example 29: Synthesis of (*R*)-2-(5-cyclopropyl-3-((1-ethylpiperidin-3-yl)amino)-1,2,4-triazin-6-yl)-5-ethynylphen ol (Compound 92)**

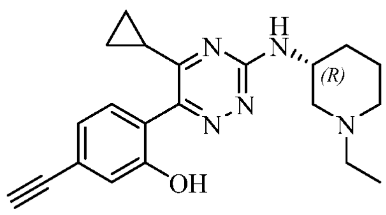

Step 1: Synthesis of (*R*)-6-bromo-5-cyclopropyl-*N*-(1-ethylpiperidin-3-yl)-1,2,4-triazin-3-amine

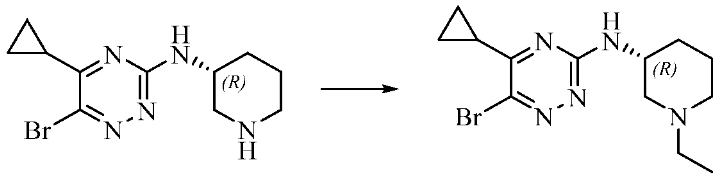

(*R*)-6-Bromo-5-cyclopropyl-N-(piperidin-3-yl)-1,2,4-triazin-3-amine (920.0 mg, 3.08 mmol, 1.0 eq), iodoethane (4.8 g, 30.8 mmol, 10.0 eq), and triethylamine (3.1 g, 30.8 mmol, 10.0 eq) were added to dichloromethane (20.0 mL), and the mixture was stirred at room temperature for 14 h. Dichloromethane (100.0 mL) was added, and the resulting mixture was washed with a saturated aqueous ammonium chloride solution (50.0 mL). The organic phase was dried over anhydrous sodium sulfate and filtered. The filtrate was concentrated under reduced pressure, and the crude product was purified by silica gel column chromatography (dichloromethane:methanol = 40:1 to 10: 1) to give the product (970.0 mg, yield: 96.4%).

Step 2: Synthesis of (*R*)-4-(5-cyclopropyl-3-((1-ethylpiperidin-3-yl)amino)-1,2,4-triazin-6-yl)-3-(ethoxymethox y)benzaldehyde

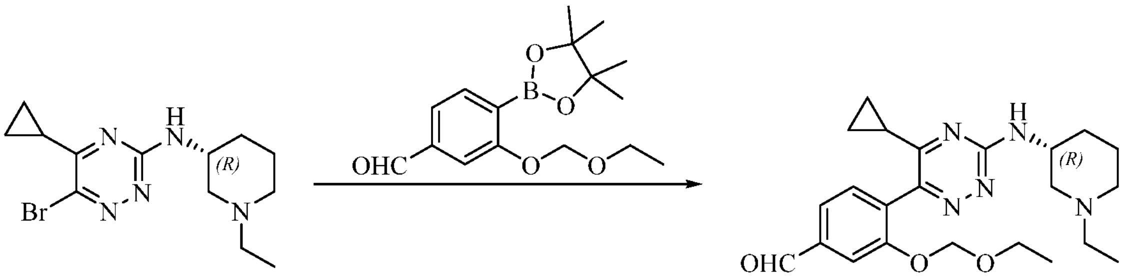

(*R*)-6-Bromo-5-cyclopropyl-*N*-(1-ethylpiperidin-3-yl)-1,2,4-triazin-3-amine (390.0 mg, 1.19 mmol, 1.0 eq), 3-(ethoxymethoxy)-4-(4,4,5,5-tetramethyl-1,3,2-dioxaborolan-2-yl)benzaldehyde (366.0 mg, 1.19 mmol, 1.0 eq), anhydrous potassium carbonate (330.3 mg, 2.38 mmol, 2.0 eq), and $Pd(PPh_3)_4$ (138.0 mg, 0.11 mmol, 0.1 eq) were added to a mixed solution of 1,4-dioxane (10.0 mL) and water (4.0 mL). The mixture was allowed to react at 100 °C for 4 h under a nitrogen atmosphere, and TLC monitoring showed the completion of the reaction. Water (100.0 mL) was added, and extraction was performed with ethyl acetate (100.0 mL). The organic phase was dried over anhydrous sodium sulfate and filtered. The filtrate was concentrated under reduced pressure, and the crude product was purified by silica gel column chromatography (dichloromethane:methanol = 40:1 to 10:1) to give the product (285.0 mg, yield: 56.1%).

Step 3: Synthesis of (R)-5-cyclopropyl-6-(2-(ethoxymethoxy)-4-ethynylphenyl)-*N*-(1-ethylpiperidin-3-yl)-1,2,4-triazin-3-amine

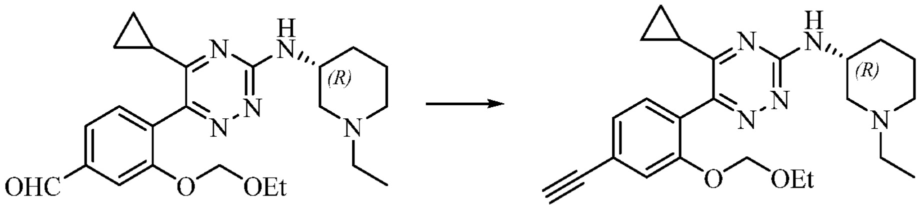

(*R*)-4-(5-Cyclopropyl-3-((1-ethylpiperidin-3-yl)amino)-1,2,4-triazin-6-yl)-3-(e thoxymethoxy)benzaldehyde (285.0 mg, 0.67 mmol, 1.0 eq), dimethyl (1-diazo-2-oxopropyl)phosphonate (192.9 mg, 1.00 mmol, 1.5 eq), and anhydrous potassium carbonate (184.9 mg, 1.34 mmol, 2.0 eq) were added to methanol (15.0 mL). The mixture was allowed to react at room temperature for 4 h, and LC-MS monitoring showed the completion of the reaction. The reaction mixture was concentrated under reduced pressure, and the crude product was purified by silica gel column chromatography (dichloromethane:methanol = 40:1 to 10:1) to give the product (170.0 mg, yield: 60.2%).

Step 4: Synthesis of (*R*)-2-(5-cyclopropyl-3-((1-ethylpiperidin-3-yl)amino)-1,2,4-triazin-6-yl)-5-ethynylphenol

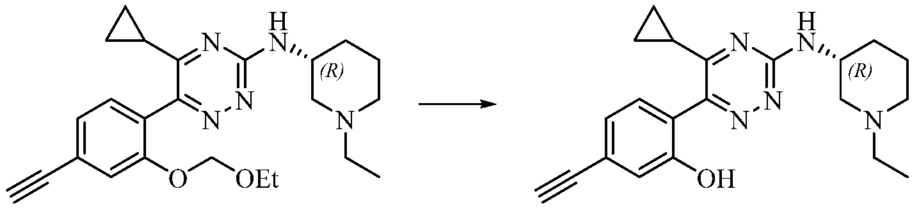

(*R*)-5-Cyclopropyl-6-(2-(ethoxymethoxy)-4-ethynylphenyl)-*N*-(1-ethylpiperidi n-3-yl)-1,2,4-triazin-3-amine (170.0 mg, 0.40 mmol, 1.0 eq) was added to dichloromethane (10.0 mL), and trifluoroacetic acid (2.0 mL) was added dropwise. The mixture was allowed to react at room temperature for 1 h, and TLC monitoring showed the completion of the reaction. The pH was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (80.0 mg, yield: 55.1%).

$^1$HNMR (400 MHz, DMSO-$d_6$) δ(ppm): 10.04 (s, 1H), 7.58-7.56 (m, 1H), 7.30-7.28 (d, *J*=8 Hz, 1H), 7.05-7.01 (m, 2H), 4.22 (s, 1H), 3.94-3.86 (m, 1H), 2.99-2.97 (m, 1H), 2.72 (s, 1H), 2.36 (s, 2H), 1.93-1.83 (m, 3H), 1.70-1.64 (m, 2H), 1.53-1.47 (m, 1H), 1.40-1.30 (m, 2H),1.02-1.98 (m, 5H), 0.86-0.81 (m, 1H).

Molecular formula: $C_{21}H_{25}N_5O$ Exact molecular weight: 363.21 LC-MS (Pos, *m/z)* = 364.22 $[M+H]^+$.

**Example 30: Synthesis of (*R*)-6-((1-ethylpiperidin-3-yl)amino)-3-(4-ethynyl-2-hydroxyphenyl)-4-methyl-1,2,4-tri azin-5(4*H*)-one (Compound 93)**

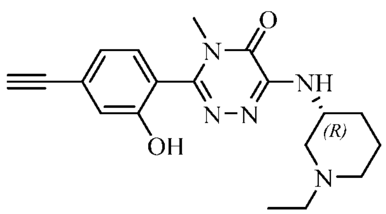

Step 1: Synthesis of (*R*)-6-((1-ethylpiperidin-3-yl)amino)-3-(4-ethynyl-2-hydroxyphenyl)-4-methyl-1,2,4-triazin -5(4*H*)-one

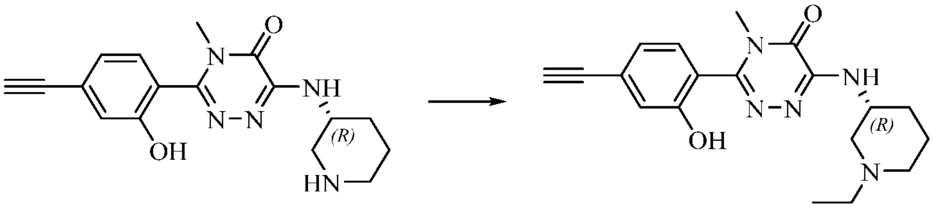

(*R*)-3-(4-Ethynyl-2-hydroxyphenyl)-4-methyl-6-(piperidin-3-ylamino)-1,2,4-tr iazin-5(4*H*)-one (200 mg, 0.61 mmol, 1.0 eq) and a solution of acetaldehyde in tetrahydrofuran (5 mol/L, 0.18 mL, 0.92 mmol, 1.5 eq) were dissolved in methanol (10 mL), and the solution was stirred at room temperature for 18 h. Sodium cyanoborohydride (38 mg, 0.61 mmol, 1.0 eq) was added, and the mixture was allowed to react at room temperature for 1 h. TLC monitoring showed the completion of the reaction. The reaction solution was concentrated under reduced pressure, and the crude product was dispersed in water (10 mL). The dispersion was extracted with DCM (10 mL × 4). The organic phase was dried and concentrated, and the residue was purified by preparative thin-layer chromatography (DCM:MeOH = 10:1) to give the product (50 mg, yield: 23.2%).

$^1$HNMR (400MHz, DMSO-$d_6$) δ(ppm): 10.47 (s, 1H), 7.32-7.30 (d, 1H), 7.05-7.03 (d, 2H), 6.86 (s, 1H), 4.29 (s, 1H), 4.04-4.03 (d, 1H), 3.18 (s, 3H), 2.82 (s, 1H), 2.59 (s, 1H), 2.42 (s, 2H), 2.19 (s, 2H), 1.71-1.67 (d, 2H), 1.60-1.56 (t, 2H), 1.06-1.00 (t, 3H).

Molecular formula: $C_{19}H_{23}N_5O_2$ Exact molecular weight: 353.19 LC-MS (Pos, m/z) = 354.22 $[M+H]^+$.

**Example 31: Synthesis of (*R*)-6-((1-cyclopropylpiperidin-3-yl)amino)-3-(4-ethynyl-2-hydroxyphenyl)-4-methyl-1, 2,4-triazin-5(4*H*)-one (Compound 98)**

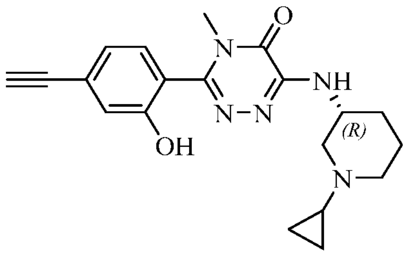

Step 1: Synthesis of (*R*)-6-((1-cyclopropylpiperidin-3-yl)amino)-3-(4-ethynyl-2-hydroxyphenyl)-4-methyl-1,2,4 -triazin-5(4*H*)-one

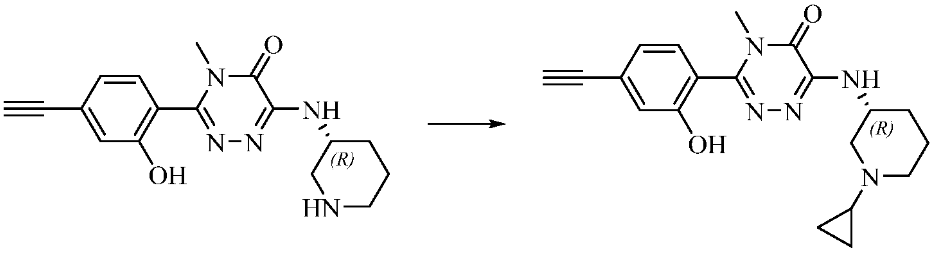

(*R*)-3-(4-Ethynyl-2-hydroxyphenyl)-4-methyl-6-(piperidin-3-ylamino)-1,2,4-tr iazin-5(4*H*)-one (200 mg, 0.61 mmol, 1.0 eq), (1-ethoxycyclopropoxy)trimethylsilane (532 mg, 3.05 mmol, 5.0 eq), and CsF (140 mg, 0.92 mmol, 1.5 eq) were dissolved in methanol (10 mL) and AcOH (0.5 mL). The solution was allowed to react at 50 °C for 2 h. Sodium cyanoborohydride (153 mg, 2.44 mmol, 4.0 eq) was added, and the mixture was allowed to react at 50 °C for 1 h. TLC monitoring showed the completion of the reaction. The reaction solution was concentrated under reduced pressure. The crude product was dispersed in water (20 mL), and the pH was adjusted to about 8 with sodium bicarbonate, followed by extraction with DCM (20 mL × 3). The organic phase was dried and concentrated, and the residue was purified by preparative thin-layer chromatography (DCM:MeOH = 10:1) to give the product (70 mg, yield: 31.4%).

$^1$HNMR (400MHz, DMSO-$d_6$) δ(ppm): 10.42 (s, 1H), 7.32-7.31 (d, 1H), 7.05-7.02 (t, 2H), 6.78-6.76 (d, 1H), 4.29 (s, 1H), 3.96-3.94 (t, 1H), 3.17 (s, 3H), 2.95-2.93 (d, 1H), 2.68 (s, 1H), 2.33-2.32 (d, 2H), 1.72-1.70 (d, 1H), 1.65-1.55 (m, 3H), 1.49-1.44 (m, 1H), 0.43-0.41 (t, 2H), 0.31 (s, 2H).

Molecular formula: $C_{20}H_{23}N_5O_2$ Exact molecular weight: 365.19 LC-MS (Pos, m/z) = 366.24 $[M+H]^+$.

**Example 32: Synthesis of (R)-4-cyclopropyl-6-((1-ethylpiperidin-3-yl)amino)-3-(4-ethynyl-2-hydroxyphenyl)-1,2, 4-triazin-5(4*H*)-one (Compound 109)**

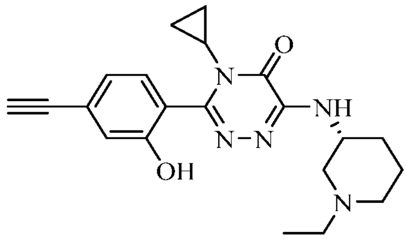

Step 1: Synthesis of*N*-cyclopropyl-4-iodo-2-methoxybenzamide

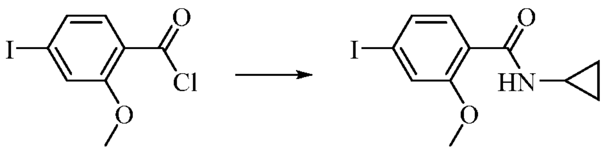

A solution of 4-iodo-2-methoxybenzoyl chloride (26.66 g, 89.91 mmol, 1.0 eq) in DCM (200 mL) was added dropwise to a solution of cyclopropylamine (15.3 g, 267.93 mmol, 3.0 eq) in DCM (100 mL), and the mixture was allowed to react at room temperature for 10 min. TLC analysis showed the completion of the reaction. The reaction solution was poured into water (200 mL) and extracted with DCM (100 mL × 2). The organic phase was dried and concentrated to give the product (28.51 g, yield: 100%).

Step 2: Synthesis of*N*-cyclopropyl-4-iodo-2-methoxybenzothioamide

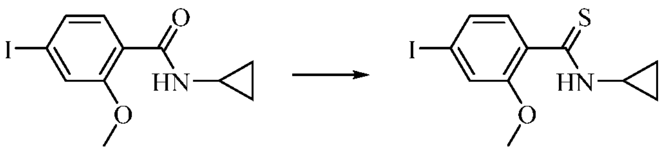

*N*-cyclopropyl-4-iodo-2-methoxybenzamide (28.51 g, 89.91 mmol, 1.0 eq) and Lawesson's reagent (20.0 g, 49.45 mmol, 0.55 eq) were dissolved in THF (300 mL), and the solution was allowed to react at 60 °C for 1 h. TLC analysis showed the completion of the reaction. The reaction solution was concentrated under reduced pressure, and the crude product was purified by silica gel column chromatography (EA:PE = 1:10) to give the product (25 g, yield: 83.4%).

Step 3: Synthesis of methyl *N*-cyclopropyl-4-iodo-2-methoxybenzimidothioate

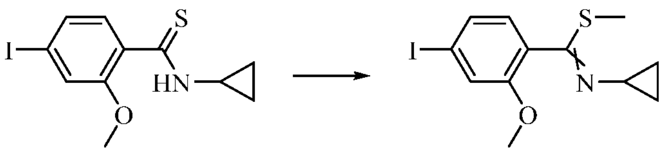

*N*-Cyclopropyl-4-iodo-2-methoxybenzothioamide (25 g, 75.03 mmol, 1.0 eq) and iodomethane (21.3 g, 150.06 mmol, 2.0 eq) were dissolved in THF (300 mL). The solution was allowed to react at room temperature for 17 h. TLC analysis showed the completion of the reaction. The reaction solution was poured into water (500 mL), and the pH was adjusted to about 9 with potassium carbonate, followed by extraction with EA (200 mL × 2). The organic phase was dried and concentrated to give the product (26.05 g, yield: 100%).

Step 4: Synthesis of *N*-amino-*N'*-cyclopropyl-4-iodo-2-methoxybenzamidine

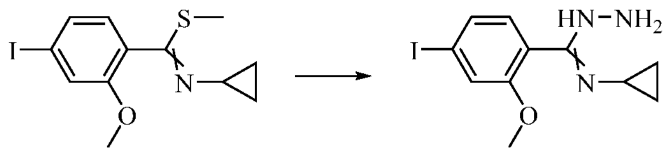

Methyl *N*-cyclopropyl-4-iodo-2-methoxybenzimidothioate (26.05 g, 122.90 mmol, 1.0 eq) and hydrazine hydrate (8.84 g, 150.06 mmol, 2.0 eq) were dissolved in EtOH (300 mL), and the solution was allowed to react at 80 °C for 1 h. LC-MS monitoring showed the completion of the reaction. The reaction solution was concentrated under reduced pressure to give the product (24.85 g, yield: 100%).

Step 5: Synthesis of 6-amino-4-cyclopropyl-3-(4-iodo-2-methoxyphenyl)-1,2,4-triazin-5(4*H*)-one

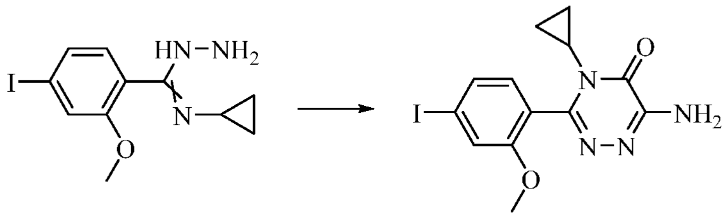

*N*-Amino-*N'*-cyclopropyl-4-iodo-2-methoxybenzamidine (15 g, 45.29 mmol, 1.0 eq), ethyl thiooxamate (9.05 g, 67.94 mmol, 1.5 eq), and TEA (13.75 g, 135.87 mmol, 3.0 eq) were dissolved in EtOH (150 mL), and the solution was allowed to react at 80 °C for 4 h. LC-MS monitoring showed the completion of the reaction. The reaction solution was concentrated under reduced pressure, and the residue was purified by silica gel column chromatography (EA:PE = 1:1) to give the product (9.0 g, yield: 51.7%).

Step 6: Synthesis of 6-bromo-4-cyclopropyl-3-(4-iodo-2-methoxyphenyl)-1,2,4-triazin-5(4*H*)-one

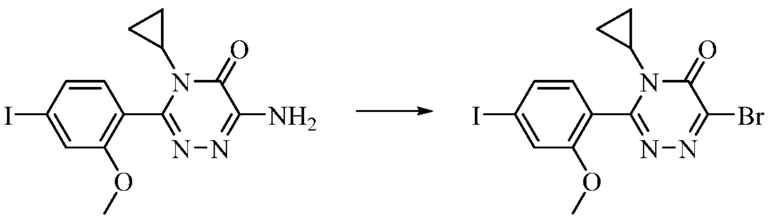

6-Amino-4-cyclopropyl-3-(4-iodo-2-methoxyphenyl)-1,2,4-triazin-5(4*H*)-one (9.0 g, 23.42 mmol, 1.0 eq) and CuBr (6.72 g, 46.84 mmol, 2.0 eq) were dispersed in ACN (90 mL), and *tert*-butyl nitrite (4.83 g, 46.84 mmol, 2.0 eq) was added dropwise at 70 °C under a nitrogen atmosphere. The mixture was allowed to react at 70 °C for 0.5 h. TLC monitoring showed the completion of the reaction. The reaction solution was concentrated under reduced pressure, and the residue was purified by silica gel column chromatography (EA:DCM = 1:10) to give the product (3.0 g, yield: 28.6%).

Step 7: Synthesis of *tert*-butyl (*R*)-3-((4-cyclopropyl-3-(4-iodo-2-methoxyphenyl)-5-oxo-4,5-dihydro-1,2,4-triazin-6-yl)amino)piperidine-1-carboxylate

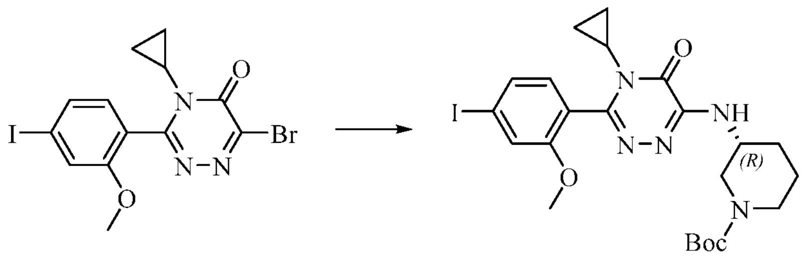

6-Bromo-4-cyclopropyl-3-(4-iodo-2-methoxyphenyl)-1,2,4-triazin-5(4*H*)-one (3.0 g, 6.69 mmol, 1.0 eq), *tert*-butyl (*R*)-3-aminopiperidine-1-carboxylate (2.0 g, 10.03 mmol, 1.5 eq), and DIPEA (1.30 g, 10.03 mmol, 1.5 eq) were dissolved in 1,4-dioxane (30 mL), and the solution was allowed to react at 100 °C for 18 h. TLC analysis showed the completion of the reaction. The reaction solution was concentrated under reduced pressure, and the crude product was dispersed in water (30 mL). The dispersion was extracted with EA (30 mL × 3). The organic phase was dried and concentrated to give the product (3.8 g, yield: 100%).

Step 8: Synthesis of *tert-butyl* (*R*)-3-((4-cyclopropyl-3-(2-methoxy-4-((trimethylsilyl)ethynyl)phenyl)-5-oxo-4, 5-dihydro-1,2,4-triazin-6-yl)amino)piperidine-1-carboxylate

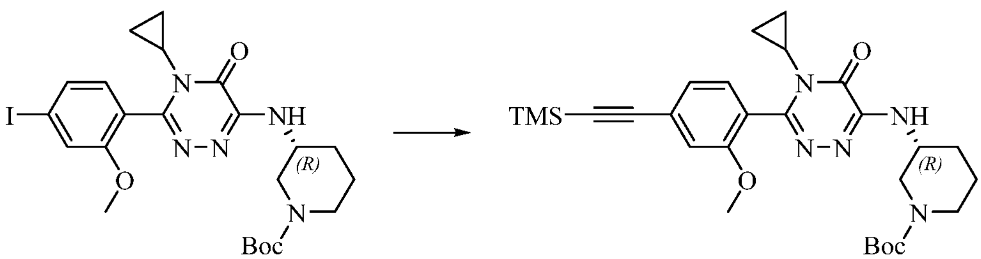

*tert*-Butyl (*R*)-3-((4-cyclopropyl-3-(4-iodo-2-methoxyphenyl)-5-oxo-4,5-dihydro-1,2,4-triazin-6-yl)a mino)piperidine-1-carboxylate (3.8 g, 6.69 mmol, 1.0 eq), trimethylsilylacetylene (3.28 g, 33.45 mmol, 5.0 eq), $PdCl_2(PPh_3)_2$ (470 mg, 0.67 mmol, 0.1 eq), and CuI (383 mg, 2.01 mmol, 0.3 eq) were dispersed in diisopropylamine (20 mL) and THF (20 mL). The dispersion was allowed to react at 40 °C for 1 h under a nitrogen atmosphere. TLC analysis showed the completion of the reaction. The reaction solution was concentrated under reduced pressure, and the crude product was purified by silica gel column chromatography (EA:PE = 1:2) to give the product (3.0 g, yield: 83.3%).

Step 9: Synthesis of (*R*)-4-cyclopropyl-3-(2-methoxy-4-((trimethylsilyl)ethynyl) phenyl)-6-(piperidin-3-ylamino)-1,2,4-triazin-5(4*H*)-one

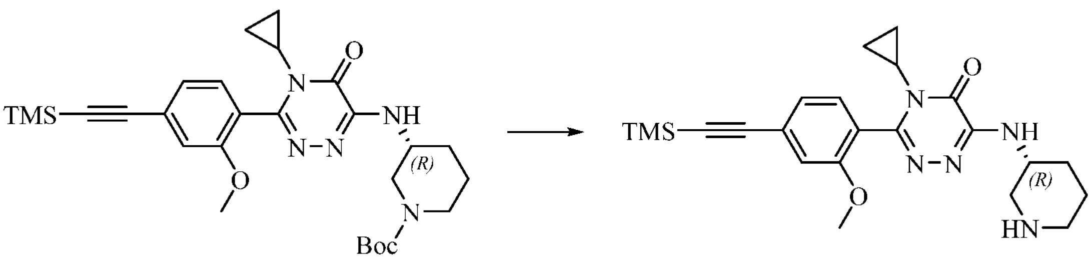

*tert*-Butyl (*R*)-3-((4-cyclopropyl-3-(2-methoxy-4-((trimethylsilyl)ethynyl) phenyl)-5-oxo-4,5-dihydro-1,2,4-triazin-6-yl)amino)piperidine-1-carboxylate (3.0 g, 5.57 mmol, 1.0 eq) was dissolved in EA (30 mL), and a solution of hydrogen chloride in 1,4-dioxane (4 mol/L, 10 mL) was added. The mixture was allowed to react at room temperature for 3 h. TLC analysis showed the completion of the reaction. The reaction solution was concentrated under reduced pressure, and the crude product was dispersed in water (20 mL). The dispersion was extracted with EA (20 mL × 2). The aqueous phase was retained, and the pH was adjusted to about 8 with sodium bicarbonate, followed by extraction with EA (20 mL ×

4). The organic phase was dried and concentrated to give the product (1.7 g, yield: 70%).

Step 10: Synthesis of (*R*)-4-cyclopropyl-6-((1-ethylpiperidin-3-yl)amino)-3-(2-methoxy-4-((trimethylsilyl)ethyny l)phenyl)-1,2,4-triazin-5(4*H*)-one

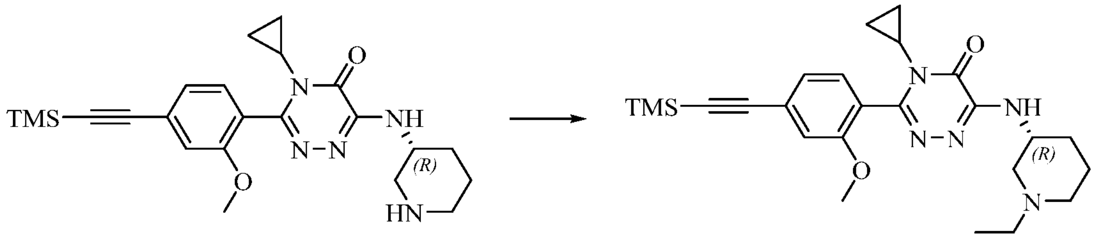

(*R*)-4-Cyclopropyl-3-(2-methoxy-4-((trimethylsilyl)ethynyl)phenyl)-6-(piperi din-3-ylamino)-1,2,4-triazin-5(4*H*)-one (0.5 g, 1.14 mmol, 1.0 eq), TEA (577 mg, 5.70 mmol, 5.0 eq), and iodoethane (889 mg, 5.70 mmol, 5.0 eq) were dissolved in DCM (10 mL), and the solution was allowed to react at room temperature for 19 h. LC-MS analysis showed the completion of the reaction. The reaction solution was poured into water (10 mL) and extracted with DCM (10 mL × 3). The organic phase was dried and concentrated to give the product (530 mg, yield: 100%).

Step 11: Synthesis of (*R*)-4-cyclopropyl-6-((1-ethylpiperidin-3-yl)amino)-3-(4-ethynyl-2-hydroxyphenyl)-1,2,4-tr iazin-5(4*H*)-one

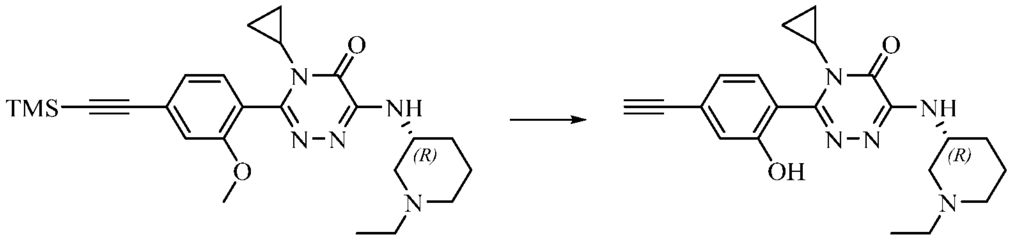

(*R*)-4-Cyclopropyl-6-((1-ethylpiperidin-3-yl)amino)-3-(2-methoxy-4-((trimeth ylsilyl)ethynyl)phenyl)-1,2,4-triazin-5(4*H*)-one (530 mg, 1.14 mmol, 1.0 eq) was dissolved in DCM (10 mL), and the solution was cooled to -60 °C. Boron tribromide (857 mg, 3.42 mmol, 3.0 eq) was added, and the mixture was naturally warmed to room temperature and allowed to react for 3 h. TLC analysis showed the completion of the reaction. The reaction solution was quenched by adding an appropriate amount of methanol and then concentrated under reduced pressure. The crude product was dispersed in water (10 mL), and the pH was adjusted to about 8 with sodium bicarbonate, followed by extraction with DCM (20 mL × 3). The organic phase was dried and concentrated, and the resulting crude product was purified first by silica gel column chromatography (DCM:MeOH = 20:1) and then by preparative thin-layer chromatography (DCM:MeOH = 10:1) to give the product (240 mg, yield: 55.6%).

$^1$HNMR (400MHz, DMSO-*d*$_6$) δ(ppm): 10.34 (s, 1H), 7.34-7.32 (d, 1H), 7.03-6.99 (d, 2H), 6.78-6.76 (d, 1H), 4.26 (s, 1H), 3.97 (s, 1H), 2.99 (s, 1H), 2.77 (s, 1H), 2.51 (s, 1H), 2.36-2.35 (d, 2H), 2.14 (s, 2H), 1.68-1.64 (d, 2H), 1.58-1.51 (m, 2H), 1.01-1.00 (d, 3H), 0.67-0.66 (d, 2H), 0.50 (s, 2H).

Molecular formula: $C_{21}H_{25}N_5O_2$ Exact molecular weight: 379.20 LC-MS (Pos, *m/z)* = 380.24 [M+H]$^+$.

**Example 33: Synthesis of (*R*)-3-(4-ethynyl-2-hydroxyphenyl)-4-methyl-6-((1-(methyl-*d*$_3$)piperidin-3-yl)amino)-1,2,4-triazin-5(4*H*)-one (Compound 88)**

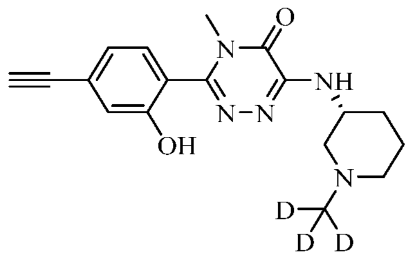

Step 1: Synthesis of (*R*)-3-(2-methoxy-4-((trimethylsilyl)ethynyl)phenyl)-4-methyl-6-(piperidin-3-ylamino)-1,2, 4-triazin-5(4H)-one

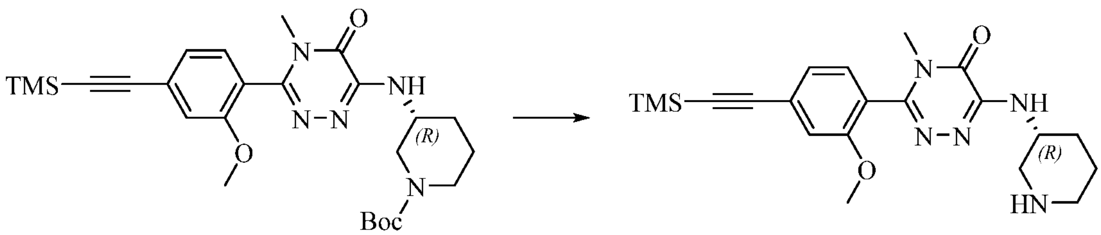

*tert*-Butyl (*R*)-3-((3-(2-methoxy-4-((trimethylsilyl)ethynyl)phenyl)-4-methyl-5-oxo-4,5-dihydro-1,2,4-triazin-6-yl) amino)piperidine-1-carboxylate (3.0 g, 5.86 mmol, 1.0 eq) was dissolved in EA (30 mL), and a solution of hydrogen chloride in 1,4-dioxane (4 mol/L, 10 mL) was added. The mixture was allowed to react at room temperature for 2.5 h. TLC analysis showed the completion of the reaction. The reaction solution was concentrated under reduced pressure, and the crude product was dissolved in water (20 mL). The solution was extracted with EA (20 mL × 2). The aqueous phase was retained, and the pH was adjusted to about 8 with sodium bicarbonate, followed by extraction with EA (30 mL × 3). The organic phase was dried and concentrated to give the product (2.25 g, yield: 93.3%).

Step 2: Synthesis of (*R*)-3-(2-methoxy-4-((trimethylsilyl)ethynyl)phenyl)-4-methyl-6-((1-(methyl-$d_3$)piperidin-3 -yl)amino)-1,2,4-triazin-5(4H)-one

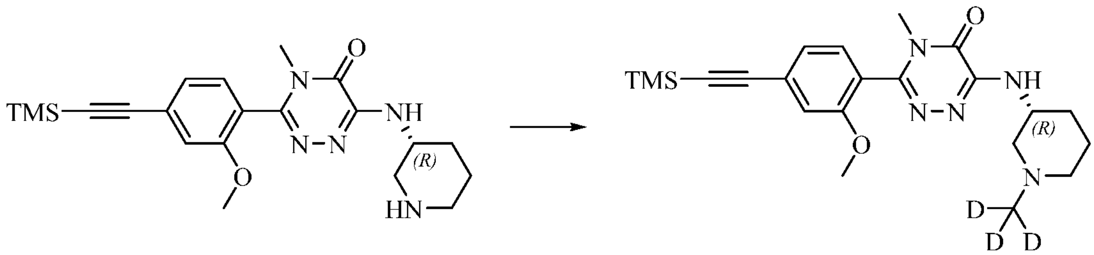

(*R*)-3-(2-Methoxy-4-((trimethylsilyl)ethynyl)phenyl)-4-methyl-6-(piperidin-3-ylamino)-1,2,4-triazin-5(4*H*)-one (1.0 g, 2.43 mmol, 1.0 eq) and TEA (1.76 g, 12.15 mmol, 5.0 eq) were dissolved in DCM (30 mL), and deuterated iodomethane (1.23 g, 12.15 mmol, 5.0 eq) was added. The mixture was allowed to react at room temperature for 10 min. TLC analysis showed the completion of the reaction. The reaction solution was poured into water (20 mL) and extracted with DCM (20 mL × 2). The organic phase was dried and concentrated to give the product (1.04 g, yield: 100%).

Step 3: Synthesis of (*R*)-3-(4-ethynyl-2-hydroxyphenyl)-4-methyl-6-((1-(methyl-$d_3$)piperidin-3-yl)amino)-1,2,4-triazin-5(4*H*)-one

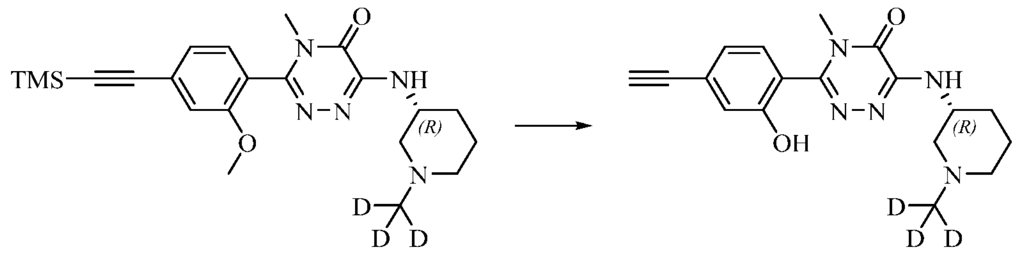

(*R*)-3-(2-Methoxy-4-((trimethylsilyl)ethynyl)phenyl)-4-methyl-6-((1-(methyl-$d_3$)piperidin-3-yl)amino)-1,2,4-triazin-5(4*H*)-one (1.04 g, 2.43 mmol, 1.0 eq) was dissolved in DCM (20 mL), and the solution was cooled to -60 °C. Boron tribromide (1.83 g, 7.29 mmol, 3.0 eq) was added, and the mixture was naturally warmed to room temperature and allowed to react for 5 h. LC-MS analysis showed the completion of the reaction. The reaction solution was quenched by adding an appropriate amount of water and then extracted with DCM (20 mL × 2). The aqueous phase was retained, and the pH was adjusted to about 8 with sodium bicarbonate, followed by extraction with EA (20 mL × 6). The organic phase was dried and concentrated, and the residue was purified by preparative thin-layer chromatography (DCM:MeOH = 10:1) to give the product (160 mg, yield: 19.2%).

$^1$HNMR (400MHz, DMSO-$d_6$) δ(ppm): 10.44 (s, 1H), 7.32-7.30 (d, 1H), 7.05-7.02 (d, 2H), 6.80-6.78 (d, 1H), 4.28 (s, 1H), 4.04-4.01 (s, 1H), 3.18 (s, 3H), 2.71-2.68 (d, 1H), 2.44 (s, 1H), 2.11 (s, 2H), 1.67 (s, 2H), 1.59-1.48 (m, 2H).

Molecular formula: $C_{18}H_{18}D_3N_5O_2$ Exact molecular weight: 342.19 LC-MS (Pos, *m/z)* = 343.25 $[M+H]^+$.

**Example 34: Synthesis of (*R*)-2-(6-((1-cyclopropylpiperidin-3-yl)amino)-4-methylpyridazin-3-yl)-5-ethynylpheno l (Compound 94):**

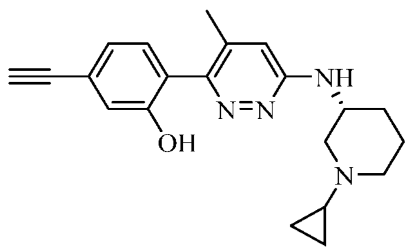

Step 1: Synthesis of *tert*-butyl (*R*)-3-((6-(2-(ethoxymethoxy)-4-formylphenyl)-5-methylpyridazin-3-yl)amino)piperidine-1 -carboxylate

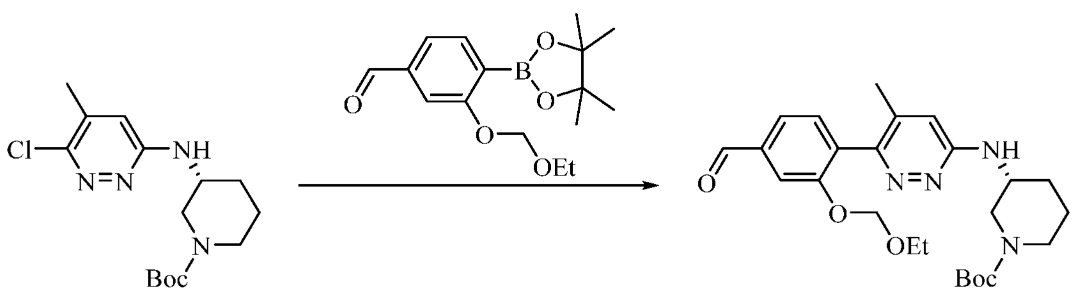

*tert*-Butyl (*R*)-3-((6-chloro-5-methylpyridazin-3-yl)amino)piperidine-1-carboxylate (2.20 g, 6.73 mmol, 1.0 eq), 3-(ethoxymethoxy)-4-(4,4,5,5-tetramethyl-1,3,2-dioxaborolan-2-yl)benzaldehyde (2.47 g, 8.08 mmol, 1.2 eq), $PdCl_2$ (dppf) (0.49 g, 0.67 mmol, 0.1 eq), and sodium bicarbonate (1.13 g, 13.46 mmol, 2.0 eq) were added to a mixed solvent of 1,4-dioxane (40 mL) and water (10 mL), and the mixture was heated to 110 °C and allowed to react for 2 h under a nitrogen atmosphere. TLC monitoring showed the completion of the reaction. The reaction mixture was cooled to room temperature. Water (50 mL) was added, and extraction was performed with EA (50 mL × 3). The organic phases were combined, dried over anhydrous sodium sulfate, and filtered. The filtrate was concentrated under reduced pressure, and

the crude product was purified by silica gel column chromatography (DCM:MeOH = 100:1 to 10:1) to give the product (2.68 g, yield: 84.6%).

Step 2: Synthesis of *tert-butyl* (*R*)-3-((6-(2-(ethoxymethoxy)-4-ethynylphenyl)-5-methylpyridazin-3-yl)amino)piperidine-1-carboxylate

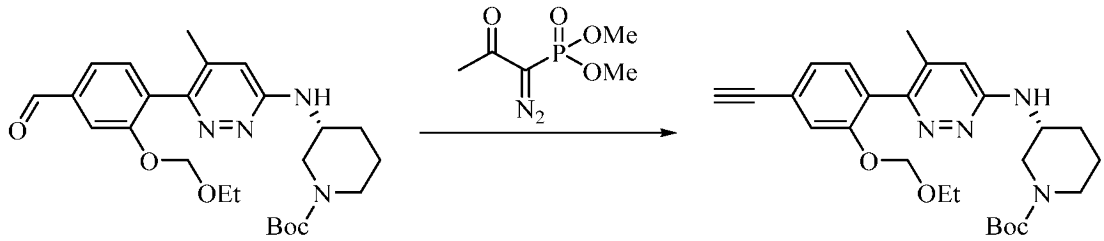

*tert-Butyl* (*R*)-3-((6-(2-(ethoxymethoxy)-4-formylphenyl)-5-methylpyridazin-3-yl)amino)piperidine-1 -carboxylate (2.68 g, 5.70 mmol, 1.0 eq) and $K_2CO_3$ (1.57 g, 11.39 mmol, 2.0 eq) were added to methanol (30 mL). The mixture was stirred, and dimethyl (1-diazo-2-oxopropyl)phosphonate (1.64 g, 8.54 mmol, 1.5 eq) was added. The resulting mixture was allowed to react at room temperature for 2 h. TLC monitoring showed the completion of the reaction. The reaction mixture was concentrated. EA (50 mL) was added, and the resulting mixture was washed with water (25 mL × 2), followed by liquid separation. The organic phase was dried over anhydrous sodium sulfate and filtered. The filtrate was concentrated under reduced pressure, and the crude product was purified by silica gel column chromatography (DCM:MeOH = 100:1 to 10:1) to give the product (2.32 g, yield: 87.3%).

Step 3: Synthesis of (*R*)-5-ethynyl-2-(4-methyl-6-(piperidin-3-ylamino)pyridazin-3-yl)phenol

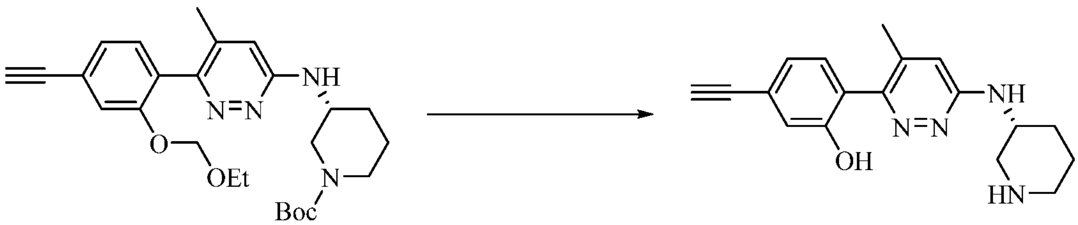

*tert*-Butyl (*R*)-3-((6-(2-(ethoxymethoxy)-4-ethynylphenyl)-5-methylpyridazin-3-yl)amino)piperidine-1-carboxylate (400.0 mg, 0.86 mmol, 1.0 eq) was dissolved in DCM (4 mL), and a 4 mol/L solution of hydrogen chloride in 1,4-dioxane (2.0 mL, 8.14 mmol, 9.5 eq) was added dropwise. The mixture was allowed to react at room temperature for 2 h. TLC monitoring showed the completion of the reaction. The reaction mixture was quenched by adding water (2 mL), and the pH was adjusted to 8 with a saturated $NaHCO_3$ solution, followed by extraction with DCM (5 mL × 5). The organic phases were combined, dried over anhydrous sodium sulfate, and filtered. The filtrate was concentrated under reduced pressure, and the residue was purified by preparative thin-layer chromatography (DCM:MeOH = 10:1) to give the product (169.8 mg, yield: 64.2%).

Step 4: Synthesis of (*R*)-2-(6-((1-cyclopropylpiperidin-3-yl)amino)-4-methylpyridazin-3-yl)-5-ethynylphenol

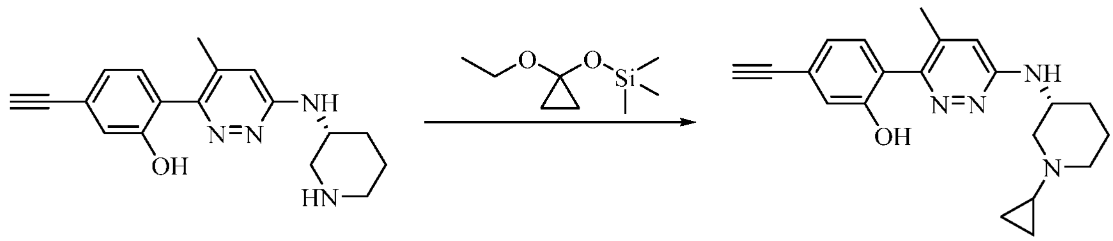

(*R*)-5-Ethynyl-2-(4-methyl-6-(piperidin-3-ylamino)pyridazin-3-yl)phenol (169.8 mg, 0.55 mmol, 1.0 eq) was dissolved in MeOH (10 mL), and (1-ethoxycyclopropoxy)trimethylsilane (383.9 mg, 2.20 mmol, 4.0 eq) and CsF (167.3 mg, 1.10 mmol, 2.0 eq) were added. The mixture was stirred at 50 °C for 1 h, and then $NaBH_3CN$ (167.3 mg, 1.10 mmol, 2.0 eq) was added. The resulting mixture was stirred for 0.5 h. TLC monitoring showed the completion of the reaction. The reaction mixture was cooled to room temperature and concentrated under reduced pressure. A saturated aqueous $NaHCO_3$ solution (20 mL) was added to the crude product, and extraction was performed with DCM (20 mL × 5). The organic phases were combined, dried over anhydrous sodium sulfate, and filtered. The filtrate was concentrated under reduced pressure, and the residue was purified by preparative thin-layer chromatography (DCM:MeOH = 10:1) to give the product (67.3 mg, yield: 35.1%).

$^1$HNMR (400 MHz, DMSO-$d_6$) δ(ppm): 10.10 (s, 1H), 7.19-7.17 (m, 1H), 7.00-6.98 (m, 2H), 6.68 (s, 1H), 6.58 (d, *J* = 6.8 Hz, 1H), 4.19 (s, 1H), 3.96 (s, 1H), 3.10 (s, 1H), 2.79 (s, 1H), 2.27 (s, 1H), 2.17-2.15 (m, 1H), 2.02 (s, 3H), 1.88-1.85 (m, 1H), 1.67 (s, 2H), 0.86-0.82 (m, 2H), 0.43-0.35 (m, 4H).

Molecular formula: $C_{21}H_{24}N_4O$ Exact molecular weight: 348.20 LC-MS (Pos, *m/z)* = 349.36 $[M+H]^+$.

**Example 35: Synthesis of (*R*)-5-ethynyl-2-(4-methyl-6-((1-(methyl-*d*$_3$)piperidin-3-yl)amino)pyridazin-3-yl)phenol (Compound 84):**

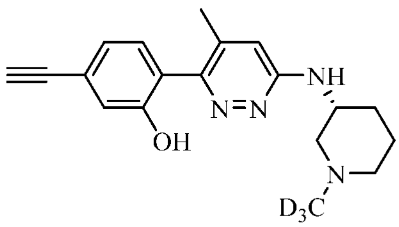

Step 1: Synthesis of (*R*)-6-(2-(ethoxymethoxy)-4-ethynylphenyl)-5-methyl-*N*-(piperidin-3-yl)pyridazin-3-amine

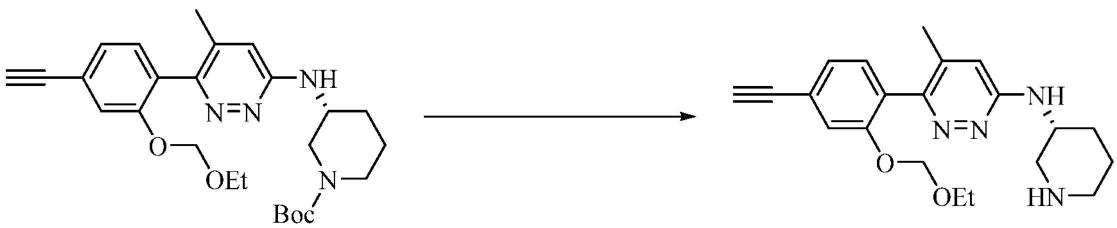

*tert*-Butyl (*R*)-3-((6-(2-(ethoxymethoxy)-4-ethynylphenyl)-5-methylpyridazin-3-yl)amino)piperidine-1-carboxylate (0.80 g, 1.71 mmol, 1.0 eq) was dissolved in DCM (8 mL), and the solution was cooled to 0 °C. Then, 2,6-dimethylpyridine (1.47 g, 13.72 mmol, 8.0 eq) and TMSOTf (1.52g, 6.86 mmol, 4.0 eq) were sequentially added. The mixture was stirred and allowed to react for 5 min, and TLC showed the completion of the reaction. The reaction mixture was quenched by adding water (5 mL) and extracted with DCM (10 mL × 3). The organic phases were combined, dried over anhydrous sodium sulfate, and filtered. The filtrate was concentrated under reduced pressure, and the crude product was purified by silica gel column chromatography (DCM:MeOH = 100:1 to 10:1) to give the product (912.2 mg, crude product).

Step 2: Synthesis of (*R*)-6-(2-(ethoxymethoxy)-4-ethynylphenyl)-5-methyl-*N*-(1-(methyl-$d_3$)piperidin-3-yl)pyrid azin-3-amine

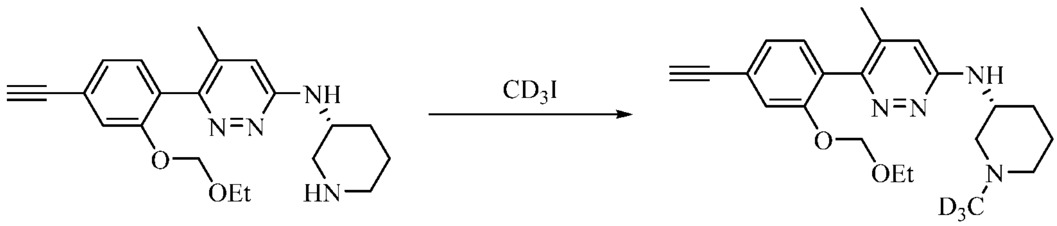


(*R*)-6-(2-(Ethoxymethoxy)-4-ethynylphenyl)-5-methyl-*N*-(piperidin-3-yl)pyrid azin-3-amine (400.0 mg, crude product, 0.75 mmol, 1.0 eq) was dissolved in DCM (3 mL), and deuterated iodomethane (317.0 mg, 3.77 mmol, 5.0 eq) was added. The mixture was allowed to react at room temperature for 18 h. TLC monitoring showed the completion of the reaction. Water (5 mL) was added, and extraction was performed with DCM (5 mL × 3). The organic phases were combined, dried over anhydrous sodium sulfate, and filtered. The filtrate was concentrated under reduced pressure, and the residue was purified by preparative thin-layer chromatography (DCM:MeOH = 10:1) to give the product (135.9 mg, yield: 47.0%).

Step 3: Synthesis of (*R*)-5-ethynyl-2-(4-methyl-6-((1-(methyl-$d_3$)piperidin-3-yl)amino)pyridazin-3-yl)phenol

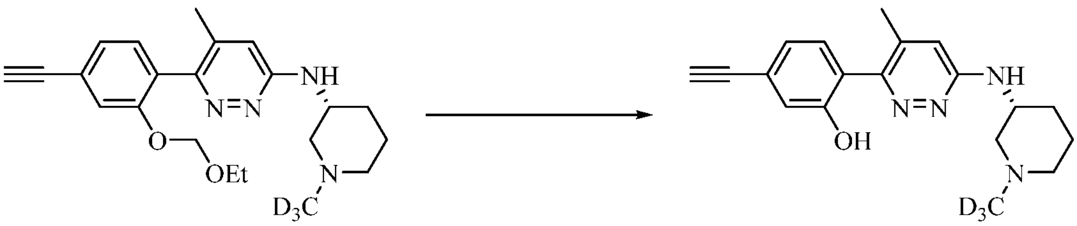

(*R*)-6-(2-(Ethoxymethoxy)-4-ethynylphenyl)-5-methyl-*N*-(1-(methyl-$d_3$)piperi din-3-yl)pyridazin-3-amine (135.9 mg, 0.35 mmol) was dissolved in DCM (1 mL), and a 4 mol/L solution of hydrogen chloride in 1,4-dioxane (1 mL) was added dropwise. The mixture was allowed to react at room temperature for 5 min. TLC monitoring showed the completion of the reaction. The reaction mixture was quenched by adding water (1 mL), and the pH was adjusted to 8 with a saturated $NaHCO_3$ solution, followed by extraction with DCM (2 mL × 5). The organic phases were combined, dried over anhydrous sodium sulfate, and filtered. The filtrate was concentrated under reduced pressure, and the residue was purified by preparative thin-layer chromatography (DCM:MeOH = 10:1) to give the product (67.1 mg, yield: 58.2%).

$^1$HNMR (400 MHz, DMSO-$d_6$) δ(ppm): 10.14 (s, 1H), 7.18-7.16 (m, 1H), 7.02-6.98 (m, 2H), 6.75 (d, *J*=7.2 Hz, 1H), 6.70 (s, 1H), 4.19 (s, 1H), 4.12 (s, 1H), 3.05 (s, 1H), 2.75 (s, 1H), 2.28-2.18(m, 2H), 2.02(s, 3H), 1.86(s, 1H), 1.78-1.77(m, 1H), 1.63-1.60(m, 1H), 1.38(m, 1H).

Molecular formula: $C_{19}H_{19}D_3N_4O$ Exact molecular weight: 325.20 LC-MS (Pos, *m/z)* = 326.33 $[M+H]^+$.

**Example 36: Synthesis of (*R*)-2-(6-((1-ethylpiperidin-3-yl)amino)-4-methylpyridazin-3-yl)-5-ethynylphenol (Compound 89)**

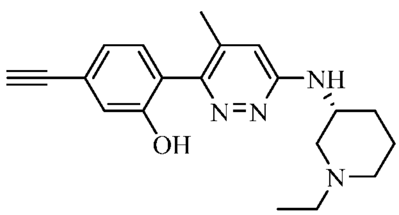

Step 1: Synthesis of (*R*)-6-(2-(ethoxymethoxy)-4-ethynylphenyl)-N-(1-ethylpiperidin-3-yl)-5-methylpyridazin-3 -amine

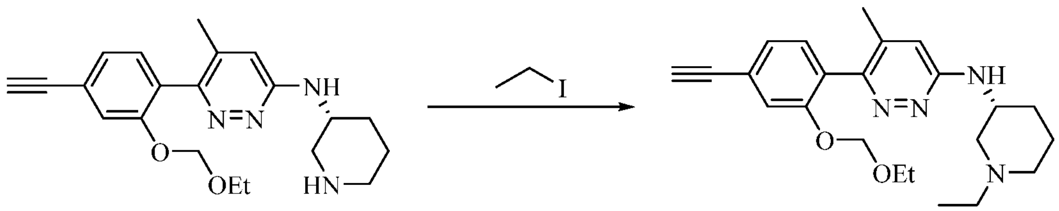

(*R*)-6-(2-(Ethoxymethoxy)-4-ethynylphenyl)-5-methyl-*N*-(piperidin-3-yl)pyrid azin-3-amine (400.0 mg, crude product, 0.75 mmol, 1.0 eq) was dissolved in DCM (3 mL), and iodoethane (587.3 mg, 3.77 mmol, 5.0 eq) was added. The mixture was allowed to react at room temperature for 18 h. TLC monitoring showed the completion of the reaction. Water (5 mL) was added, and extraction was performed with DCM (5 mL × 3). The organic phases were combined, dried over anhydrous sodium sulfate, and filtered. The filtrate was concentrated under reduced pressure, and the residue was purified by preparative thin-layer chromatography (DCM:MeOH = 10:1) to give the product (76.7 mg, yield: 25.8%).

Step 2: Synthesis of (*R*)-2-(6-((1-ethylpiperidin-3-yl)amino)-4-methylpyridazin-3-yl)-5-ethynylphenol

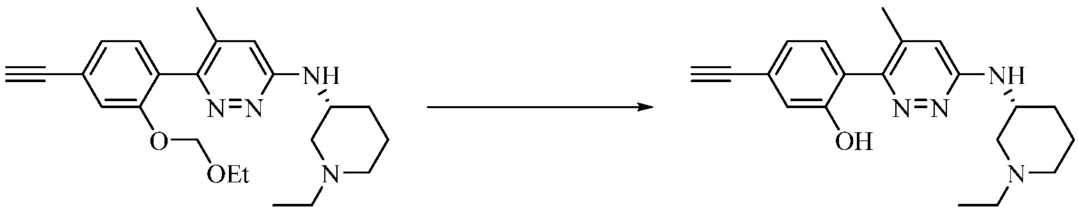

(*R*)-6-(2-(Ethoxymethoxy)-4-ethynylphenyl)-*N*-(1-ethylpiperidin-3-yl)-5-meth ylpyridazin-3-amine (76.7 mg, 0.19 mmol) was dissolved in DCM (0.5 mL), and a 4 mol/L solution of hydrogen chloride in 1,4-dioxane (0.5 mL) was added dropwise. The mixture was allowed to react at room temperature for 5 min. TLC monitoring showed the completion of the reaction. The reaction mixture was quenched by adding water (0.5 mL), and the pH was adjusted to 8 with a saturated $NaHCO_3$ solution, followed by extraction with DCM (1 mL × 5). The organic phases were combined, dried over anhydrous sodium sulfate, and filtered. The filtrate was concentrated under reduced pressure, and the residue was purified by preparative thin-layer chromatography (DCM:MeOH = 10:1) to give the product (29.7 mg, yield: 45.4%).

$^1$HNMR (400 MHz, DMSO-$d_6$) δ(ppm): 10.12 (s, 1H), 7.18-7.16 (m, 1H), 7.02-6.99 (m, 2H), 6.70 (m, 2H), 4.19 (m, 2H), 2.90-2.70 (m, 6H), 2.03 (s, 3H), 1.96-1.92 (m, 1H), 1.87-1.82 (m, 1H), 1.66 (s, 1H), 1.41 (s, 1H), 1.10-1.09 (m, 3H).

Molecular formula: $C_{20}H_{24}N_4O$ Exact molecular weight: 336.20 LC-MS (Pos, *m/z)* = 337.32 $[M+H]^+$.

**Example 37: Synthesis of (*R*)-3-(3-((6-(2-hydroxy-4-(prop-1-yn-1-yl)phenyl)-5-methylpyridazin-3-yl)amino)pipe ridin-1-yl)propanenitrile (Compound 78)**

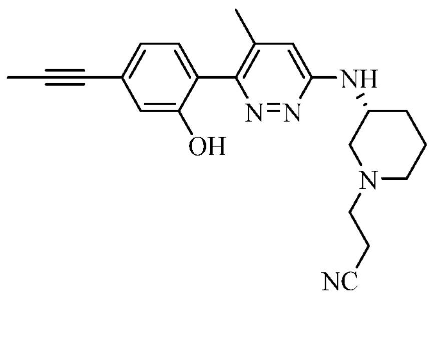

Step 1: Synthesis of *(R)-3-(3-((6-(2-(ethoxymethoxy)-4-(prop-1-yn-1-yl)phenyl)-5-methylpyridazin-3-yl)amino)* piperidin-1-yl)propanenitrile

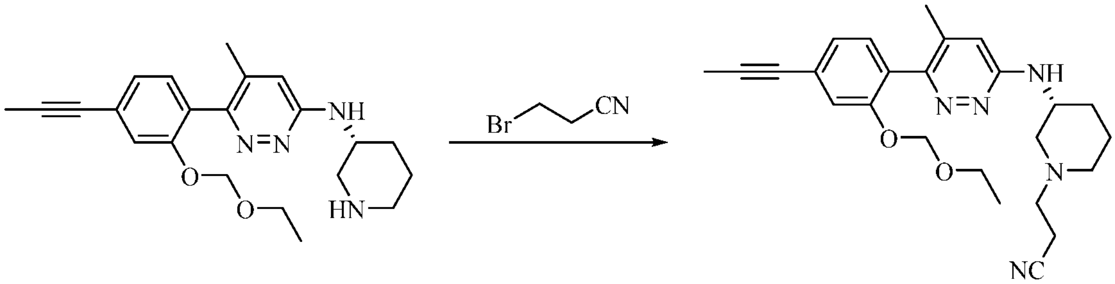


(*R*)-6-(2-(Ethoxymethoxy)-4-(prop-1-yn-1-yl)phenyl)-5-methyl-*N*-(piperidin-3-yl)pyridazin-3-amine (250 mg, 0.657 mmol, 1.0 eq) was dissolved in DCM (5 mL), and triethylamine (333 mg, 3.29 mmol, 5.0 eq) and 3-bromopropanenitrile (441 mg, 3.29 mmol, 5.0 eq) were added. The mixture was stirred at room temperature for 20 h. The reaction mixture was concentrated, and the residue was purified by silica gel column chromatography (DCM:MeOH = 100:1 to 20:1) to give the product (263 mg, yield: 92.3%).

Step 3: Synthesis of *(R)-3-(3-((6-(2-hydroxy-4-(prop-1-yn-1-yl)phenyl)-5-methylpyridazin-3-yl)amino)piperidin* -1-yl) propanenitrile

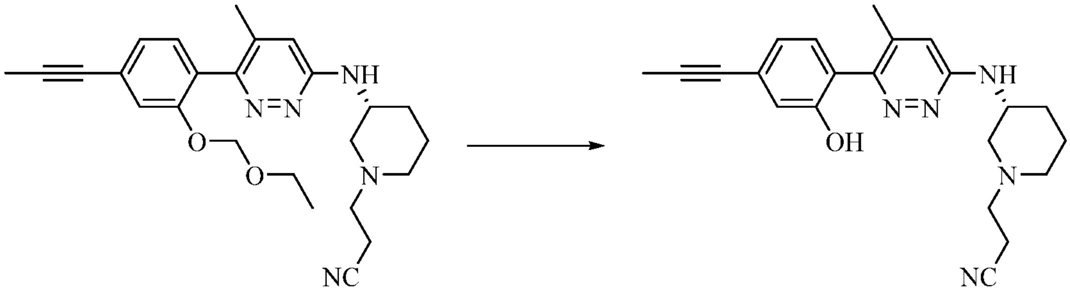

(*R*)-3-(3-((6-(2-(Ethoxymethoxy)-4-(prop-1-yn-1-yl)phenyl)-5-methylpyridazi n-3-yl)amino)piperidin-1-yl)propanenitrile (260 mg, 0.600 mmol, 1.0 eq) was dissolved in DCM (4 mL), and then a solution of hydrogen chloride in 1,4-dioxane (4 mol/L, 0.45 mL, 1.80 mmol, 3.0 eq) was added dropwise. The mixture was stirred at room temperature for 2 h. The pH was adjusted to 8 with a saturated aqueous $NaHCO_3$ solution, and extraction was performed with DCM (10 mL × 3). The organic phase was dried over anhydrous sodium sulfate and filtered. The filtrate was concentrated under reduced pressure, and the residue was purified by silica gel column chromatography (DCM:MeOH = 100:1 to 20:1) to give the product (166 mg, yield: 73.7%).

$^1$HNMR(400 MHz, DMSO-$d_6$) δ(ppm): 10.01 (s, 1H), 7.13 (d, *J* = 8.3 Hz, 1H), 6.96-6.89 (m, 2H), 6.66 (s, 1H), 6.57 (d, *J* = 8.0 Hz, 1H), 4.03-4.01 (m, 1H), 3.02 (d, *J* = 8.2 Hz, 1H), 2.73 (d, *J* = 10.8 Hz, 1H), 2.69-2.66 (m, 2H), 2.64-2.60 (m, 2H), 2.11-1.96 (m, 8H), 1.89-1.86 (m, 1H), 1.73-1.70 (m, 1H), 1.58-1.49 (m, 1H), 1.34-1.24 (m, 1H).

Molecular formula: $C_{22}H_{25}N_5O$ Exact molecular weight: 375.21 LC-MS (Pos, *m/z)* = 376.19 $[M+H]^+$.

**Example 38: Synthesis of (*R*)-2-(4-methyl-6-((1-(2,2,2-trifluoroethyl)piperidin-3-yl)amino)pyridazin-3-yl)-5-(prop-1-yn-1-yl)phenol (Compound 79)**

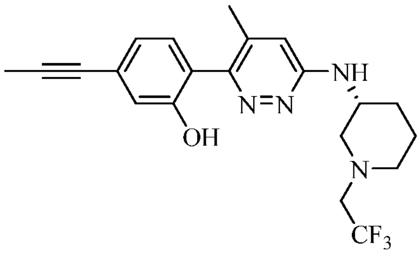

Step 1: Synthesis of 2,2,2-trifluoroethyl-4-methylbenzenesulfonate

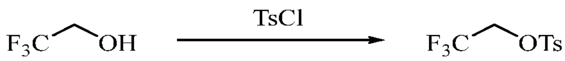


2,2,2-Trifluoroethanol (5.00 g, 50.0 mmol, 1.0 eq) was dissolved in DCM (100 mL), and triethylamine (7.59 g, 75.0 mmol, 1.5 eq) and 4-methylbenzenesulfonyl chloride (9.53 g, 50.0 mmol, 1.0 eq) were added. The mixture was stirred at room temperature for 20 h. The reaction solution was washed with water (50 mL × 3). The organic phase was dried over anhydrous sodium sulfate and filtered. The filtrate was concentrated under reduced pressure, and the residue was purified by silica gel column chromatography (PE:EA = 10:1) to give the product (11.9 g, yield: 93.7%).

Step 2: Synthesis of (*R*)-6-(2-(ethoxymethoxy)-4-(prop-1-yn-1-yl)phenyl)-5-methyl-*N*-(1-(2,2,2-trifluoroethyl)pi peridin-3-yl)pyridazin-3-amine

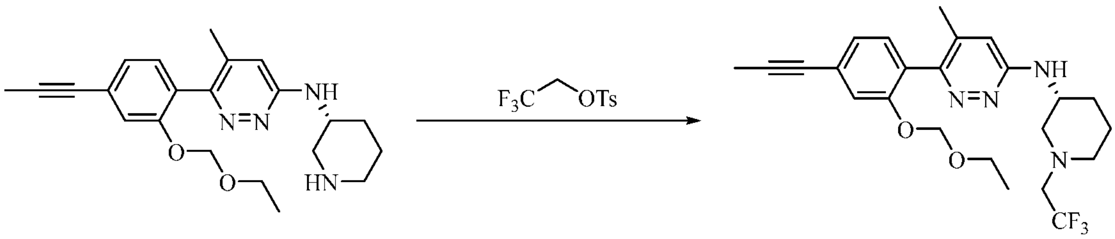

(*R*)-6-(2-(Ethoxymethoxy)-4-(prop-1-yn-1-yl)phenyl)-5-methyl-*N*-(piperidin-3-yl)pyridazin-3-amine (250 mg, 0.657 mmol, 1.0 eq) was dissolved in DMF (5 mL), and 2,2,2-trifluoroethyl-4-methylbenzenesulfonate (334 mg, 1.31 mmol, 2.0 eq) and $K_2CO_3$ (181 mg, 1.31 mmol, 2.0 eq) were added. The mixture was heated to 100 °C and allowed to react for 48 h. The reaction mixture was quenched by adding water (30 mL) and extracted with EA (30 mL). The organic phase was washed with saturated brine (20 mL × 3), dried over anhydrous sodium sulfate, and filtered. The filtrate was concentrated under reduced pressure, and the residue was purified by silica gel column chromatography (DCM:MeOH = 100:1 to 50:1) to give the product (148 mg, yield: 48.7%).

Step 3: Synthesis of (*R*)-2-(4-methyl-6-((1-(2,2,2-trifluoroethyl)piperidin-3-yl)amino)pyridazin-3-yl)-5-(prop-1-yn-1-yl) phenol

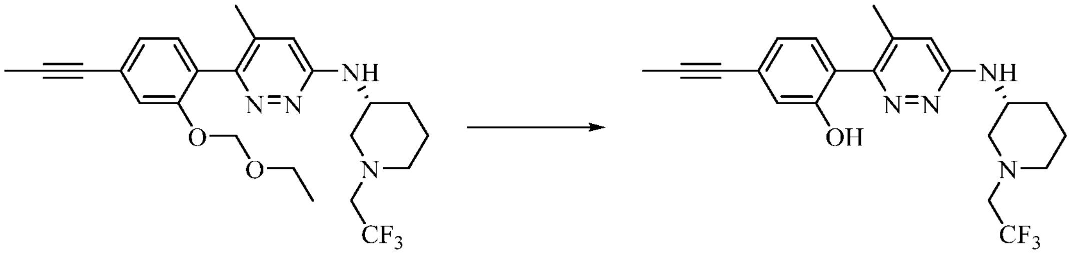

(*R*)-6-(2-(Ethoxymethoxy)-4-(prop-1-yn-1-yl)phenyl)-5-methyl-*N*-(1-(2,2,2-tri fluoroethyl)piperidin-3-yl)pyridazin-3-amine (148 mg, 0.320 mmol, 1.0 eq) was dissolved in DCM (2 mL), and then a solution of hydrogen chloride in 1,4-dioxane (4 mol/L, 0.24 mL, 0.960 mmol, 3.0 eq) was added dropwise. The mixture was stirred at room temperature for 2 h. The pH was adjusted to 8 with a saturated aqueous $NaHCO_3$ solution, and extraction was performed with DCM (10 mL × 2). The organic phase was dried over anhydrous sodium sulfate and filtered. The filtrate was concentrated under reduced pressure, and the residue was purified by silica gel column chromatography (DCM:MeOH = 100:1 to 50:1) to give the product (67.0 mg, yield: 51.8%).

$^1$HNMR(400 MHz, DMSO-$d_6$) δ(ppm): 10.01 (s, 1H), 7.13 (d, *J* = 7.6 Hz, 1H), 6.90 (d, *J* = 7.9 Hz, 2H), 6.66 (s, 1H), 6.61 (d, *J* = 7.8 Hz, 1H), 4.04-4.01 (m, 1H), 3.25-3.15 (m, 3H), 2.83 (d, *J*= 10.8 Hz, 1H), 2.40-2.35 (m, 1H), 2.26-2.21 (m, 1H), 2.05 (s, 3H), 2.03 (s, 3H), 1.90 (d, *J* = 8.8 Hz, 1H), 1.71 (d, *J* = 13.2 Hz, 1H), 1.60-1.55 (m, 1H), 1.31-1.24 (m, 1H).

Molecular formula: $C_{21}H_{23}F_3N_4O$ Exact molecular weight: 404.18 LC-MS (Pos, *m/z)* = 405.22 $[M+H]^+$.

**Example 39: Synthesis of (*R*)-2-(3-((1-cyclopropylpiperidin-3-yl)amino)-5-methyl-1,2,4-triazin-6-yl)-5-ethynylphenol (Compound 96)**

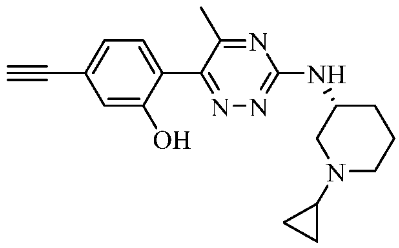

Step 1: Synthesis of 5-methyl-3-(methylsulfinyl)-1,2,4-triazine

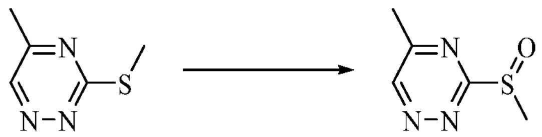

5-Methyl-3-methylthio-1,2,4-triazine (70.0 g, 0.496 mol, 1.0 eq) was dissolved in DCM (700 mL), and the solution was stirred at room temperature. *meta*-Chloroperoxybenzoic acid (101 g, 0.496 mol, 1.0 eq) was added in portions, and the mixture was stirred at room temperature for 1 h. The reaction mixture was filtered under vacuum. The filtrate was concentrated under reduced pressure, and the residue was directly used in the next step.

Step 2: Synthesis of *tert-butyl (R)-3-((5-methyl-1,2,4-triazin-3-yl)amino)piperidine-1-carboxylate*

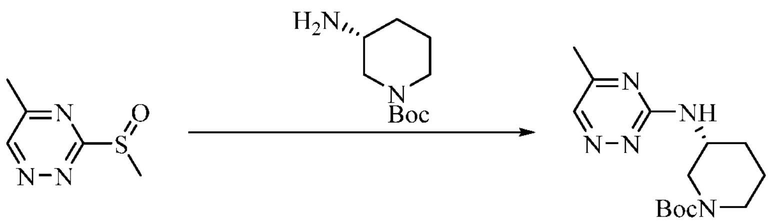

The crude product of 5-methyl-3-(methylsulfinyl)-1,2,4-triazine obtained in the previous step was dissolved in 1,4-dioxane (200 mL), and triethylamine (100 g, 0.992 mol, 2.0 eq) and *tert*-butyl (*R*)-3-aminopiperidine-1-carboxylate (99.3 g, 0.496 mol, 1.0 eq) were added. The mixture was heated to 100 °C and allowed to react for 1 h. The reaction mixture was cooled to room temperature, quenched by adding water (600 mL), and extracted with EA (300 mL × 2). The organic phase was dried over anhydrous sodium sulfate and filtered. The filtrate was concentrated under reduced pressure, and

the residue was purified by silica gel column chromatography (PE:EA = 10:1 to 1:1) to give the product (82.5 g, two-step yield: 56.7%).

Step 3: Synthesis of *tert-butyl* (*R*)-3-((6-bromo-5-methyl-1,2,4-triazin-3-yl)amino)piperidine-1-carboxylate

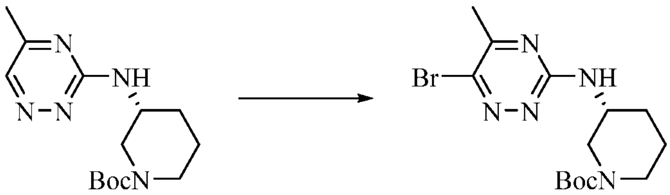

*tert-Butyl (R)-3-((5-methyl-1,2,4-triazin-3-yl)amino)piperidine-1-carboxylate* (12.0 g, 40.9 mmol, 1.0 eq) was dissolved in DMF (100 mL), and the solution was stirred at room temperature. Then, NBS (7.28 g, 40.9 mmol, 1.0 eq) was added in portions, and the mixture was allowed to react at room temperature for 4 h. The reaction mixture was quenched by adding water (300 mL) and extracted with EA (200 mL). The organic phase was washed with water (100 mL × 3), dried over anhydrous sodium sulfate, and filtered. The filtrate was concentrated under reduced pressure, and the residue was purified by silica gel column chromatography (PE:EA = 20:1 to 2:1) to give the product (6.30 g, yield: 41.4%).

Step 4: Synthesis of (*R*)-6-bromo-5-methyl-*N*-(piperidin-3-yl)-1,2,4-triazin-3-amine

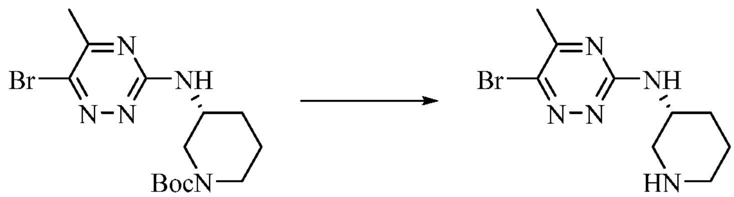

*tert*-Butyl (*R*)-3-((6-bromo-5-methyl-1,2,4-triazin-3-yl)amino)piperidine-1-carboxylate (6.30 g, 16.9 mmol, 1.0 eq) was dissolved in DCM (15 mL), and TFA (15 mL) was added dropwise. The mixture was allowed to react at room temperature for 2 h. The pH was adjusted to 8 with a saturated aqueous $NaHCO_3$ solution, and extraction was performed with a mixed solvent (DCM:MeOH = 10:1) (40 mL × 5). The organic phase was dried over anhydrous sodium sulfate and filtered. The filtrate was concentrated under reduced pressure, and the residue was purified by silica gel column chromatography (DCM:MeOH = 50:1 to 10:1) to give the product (4.10 g, yield: 89.0%).

Step 5: Synthesis of (*R*)-6-bromo-*N*-(1-cyclopropylpiperidin-3-yl)-5-methyl-1,2,4-triazin-3-amine

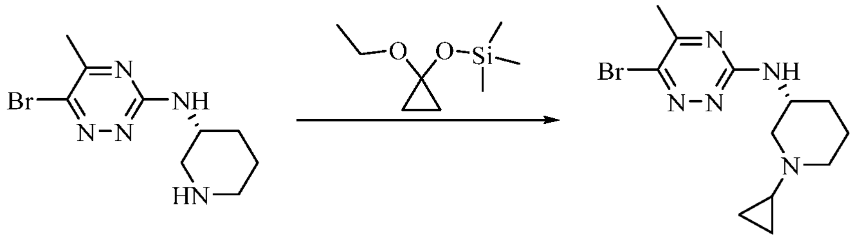

(*R*)-6-Bromo-5-methyl-*N*-(piperidin-3-yl)-1,2,4-triazin-3-amine (300 mg, 1.10 mmol, 1.0 eq) was dissolved in MeOH (20 mL), and (1-ethoxycyclopropoxy)trimethylsilane (767 mg, 4.40 mmol, 4.0 eq) and CsF (334 mg, 2.20 mmol, 2.0 eq) were added. The mixture was heated to 50 °C and stirred for 1 h. Then, $NaBH_3CN$ (276 mg, 4.40 mmol, 4.0 eq) was added, and the resulting mixture was allowed to react for 0.5 h. The reaction mixture was cooled to room temperature and concentrated. A saturated aqueous $NaHCO_3$ solution was added, and extraction was performed with EA (20 mL × 2). The organic phase was dried over anhydrous sodium sulfate and filtered. The filtrate was concentrated under reduced

pressure, and the residue was purified by silica gel column chromatography (DCM:MeOH = 100:1 to 20:1) to give the product (308 mg, yield: 89.5%).

Step 6: Synthesis of (*R*)-4-(3-((1-cyclopropylpiperidin-3-yl)amino)-5-methyl-1,2,4-triazin-6-yl)-3-(ethoxymetho xy)benzaldehyde

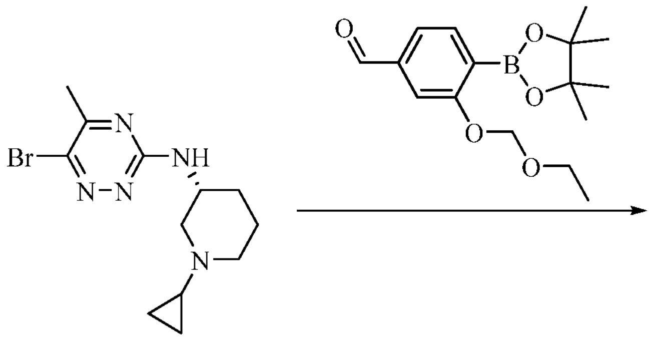

3-(Ethoxymethoxy)-4-(4,4,5,5-tetramethyl-1,3,2-dioxaborolan-2-yl)benzaldeh yde (230 mg, 0.751 mmol, 1.3 eq), (*R*)-6-bromo-*N*-(1-cyclopropylpiperidin-3-yl)-5-methyl-1,2,4-triazin-3-amine (180 mg, 0.577 mmol, 1.0 eq), $Pd(PPh_3)_4$ (42.2 mg, 0.0577 mmol, 0.1 eq), and $NaHCO_3$ (96.9 mg, 1.15 mmol, 2.0 eq) were sequentially added to 1,4-dioxane (6 mL), and $H_2O$ (3 mL) was added. The mixture was heated to 80 °C and allowed to react for 24 h under a nitrogen atmosphere. The reaction mixture was cooled to room temperature. Water (10 mL) was added, and extraction was performed with EA (20 mL × 2). The organic phase was dried over anhydrous sodium sulfate and filtered. The filtrate was concentrated under reduced pressure, and the residue was purified by silica gel column chromatography (DCM:MeOH = 100:1 to 20:1) to give the product (112 mg, yield: 49.7%).

Step 7: Synthesis of (*R*)-*N*-(1-cyclopropylpiperidin-3-yl)-6-(2-(ethoxymethoxy)-4-ethynylphenyl)-5-methyl-1,2,4-triazin-3-amine

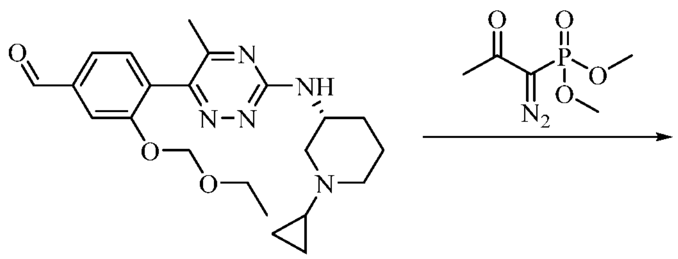

(*R*)-4-(3-((1-Cyclopropylpiperidin-3-yl)amino)-5-methyl-1,2,4-triazin-6-yl)-3-(ethoxymethoxy)benzaldehyde (112 mg, 0.272 mmol, 1.0 eq) was dissolved in MeOH (4 mL), and $K_2CO_3$ (75.2 mg, 0.544 mmol, 2.0 eq) and dimethyl (1-diazo-2-oxopropyl)phosphonate (78.4 mg, 0.408 mmol, 1.5 eq) were added. The mixture was stirred at room temperature for 1 h. Water (20 mL) was added, and extraction was performed with EA (15 mL × 2). The organic phase was dried over anhydrous sodium sulfate and filtered. The filtrate was concentrated under reduced pressure, and the residue was purified by silica gel column chromatography (DCM:MeOH = 100:1 to 20:1) to give the product (102 mg, yield: 92.0%).

Step 8: Synthesis of (*R*)-2-(3-((1-cyclopropylpiperidin-3-yl)amino)-5-methyl-1,2,4-triazin-6-yl)-5-ethynylpheno l

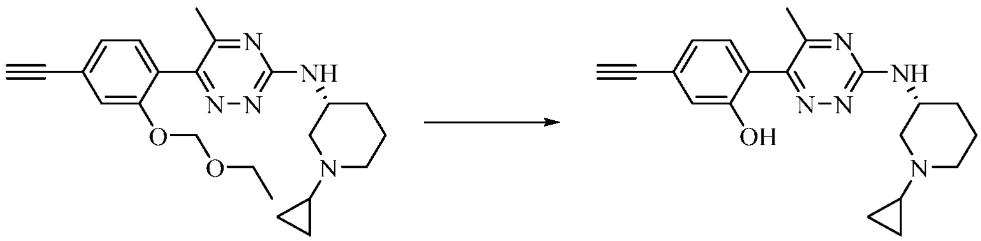

(*R*)-*N*-(1-Cyclopropylpiperidin-3-yl)-6-(2-(ethoxymethoxy)-4-ethynylphenyl)-5-methyl-1,2,4-triazin-3-amine (102 mg, 0.250 mmol, 1.0 eq) was dissolved in DCM (3 mL), and then a solution of hydrogen chloride in 1,4-dioxane (4 mol/L, 0.25 mL, 1.00 mmol, 4.0 eq) was added dropwise. The mixture was stirred at room temperature for 1 h. The pH was adjusted to 8 with a saturated aqueous $NaHCO_3$ solution, and extraction was performed with DCM (10 mL × 3). The organic phase was dried over anhydrous sodium sulfate and filtered. The filtrate was concentrated under reduced pressure, and the residue was purified by silica gel column chromatography (DCM:MeOH = 100:1 to 20:1) to give the product (68.5 mg, yield: 78.3%).

$^1$HNMR(400 MHz, DMSO-$d_6$) δ(ppm): 10.09 (s, 1H), 7.40 (s, 1H), 7.26 (d, *J*= 7.6 Hz, 1H), 7.03 (d, *J* = 8.0 Hz, 2H), 4.22 (s, 1H), 3.91 (s, 1H), 3.07 (d, *J* = 7.0 Hz, 1H), 2.81 (d, *J* = 10.5 Hz, 1H), 2.18-2.10 (m, 5H), 1.86 (d, *J* = 8.4 Hz, 1H), 1.69-1.63 (m, 2H), 1.50-1.34 (m, 2H), 0.41 (d, *J* = 6.1 Hz, 2H), 0.31 (s, 2H).

Molecular formula: $C_{20}H_{23}N_5O$ Exact molecular weight: 349.19 LC-MS (Pos, *m/z)* = 350.22 [M+H]$^+$.

**Example 40: Synthesis of (R)-2-(3-((1-ethylpiperidin-3-yl)amino)-5-methyl-1,2,4-triazin-6-yl)-5-ethynylphenol (Compound 91)**

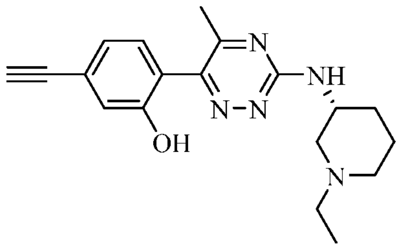

Step 1: Synthesis of *tert-butyl* (*R*)-3-((6-(2-(ethoxymethoxy)-4-formylphenyl)-5-methyl-1,2,4-triazin-3-yl)amino)piperidin e-1-carboxylate

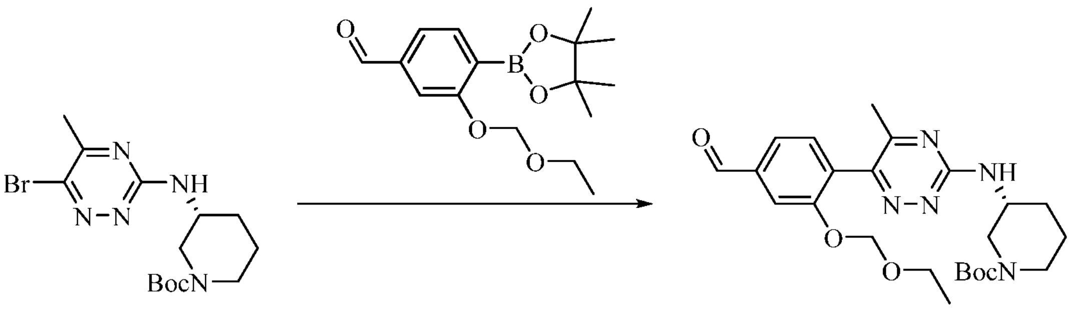

3-(Ethoxymethoxy)-4-(4,4,5,5-tetramethyl-1,3,2-dioxaborolan-2-yl)benzaldeh yde (2.68 g, 8.75 mmol, 1.3 eq), *tert-butyl* (*R*)-3-((6-bromo-5-methyl-1,2,4-triazin-3-yl)amino)piperidine-1-carboxylate (2.50 g, 6.72 mmol, 1.0 eq), Pd(dppf)$Cl_2$ (492 mg, 0.672 mmol, 0.1 eq), and $K_2CO_3$ (1.86 g, 13.4 mmol, 2.0 eq) were sequentially added to 1,4-dioxane (40 mL), and $H_2O$ (20 mL) was added. The mixture was heated to 110 °C and allowed to react for 2 h under a nitrogen atmosphere. The reaction mixture was cooled to room temperature. Water (50 mL) was added, and extraction was performed with EA (50 mL × 3). The organic phase was dried over anhydrous sodium sulfate and filtered. The filtrate was concentrated under reduced pressure, and the residue was purified by silica gel column chromatography (PE:EA =

10:1 to 2:1) to give the product (2.62 g, yield: 82.7%).

Step 2: Synthesis of *tert-butyl* (*R*)-3-((6-(2-(ethoxymethoxy)-4-ethynylphenyl)-5-methyl-1,2,4-triazin-3-yl)amino)piperidi ne-1-carboxylate

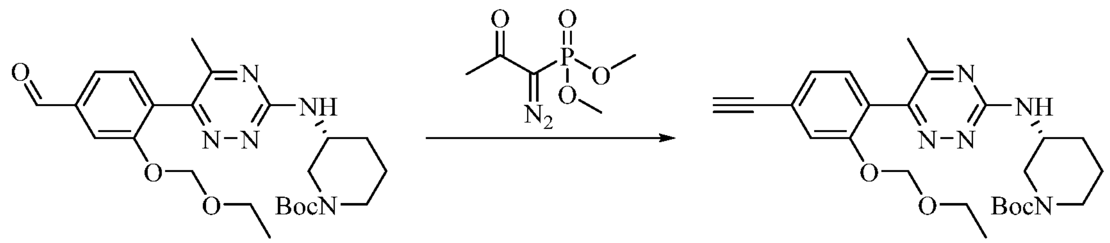

*tert*-Butyl (*R*)-3-((6-(2-(ethoxymethoxy)-4-formylphenyl)-5-methyl-1,2,4-triazin-3-yl)amino)piperidin e-1-carboxylate (2.62 g, 5.56 mmol, 1.0 eq) was dissolved in MeOH (30 mL), and $K_2CO_3$ (1.54 g, 11.1 mmol, 2.0 eq) and dimethyl (1-diazo-2-oxopropyl)phosphonate (1.60 g, 8.34 mmol, 1.5 eq) were added. The mixture was stirred at room temperature for 1 h. The reaction mixture was concentrated. Water (50 mL) was added, and extraction was performed with EA (50 mL × 2). The organic phase was dried over anhydrous sodium sulfate and filtered. The filtrate was concentrated under reduced pressure, and the residue was purified by silica gel column chromatography (PE:EA = 10:1 to 2:1) to give the product (2.34 g, yield: 90.1%).

Step 3: Synthesis of (*R*)-6-(2-(ethoxymethoxy)-4-ethynylphenyl)-5-methyl-*N*-(piperidin-3-yl)-1,2,4-triazin-3-am ine

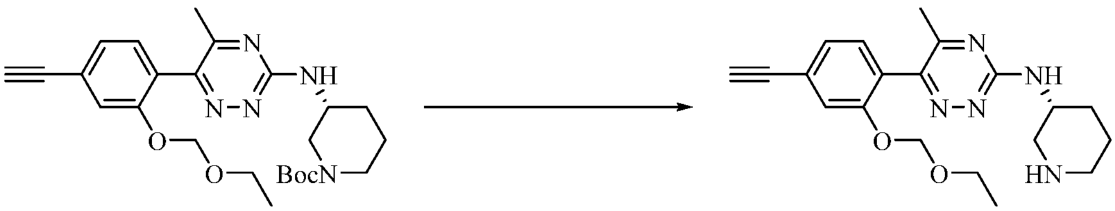

*tert*-Butyl (*R*)-3-((6-(2-(ethoxymethoxy)-4-ethynylphenyl)-5-methyl-1,2,4-triazin-3-yl)amino)piperidi ne-1-carboxylate (2.34 g, 5.00 mmol, 1.0 eq) was dissolved in DCM (40 mL), and then 2,6-dimethylpyridine (4.29 g, 40.0 mmol, 8.0 eq) was added. The mixture was cooled to 0 °C under an ice-water bath, followed by the dropwise addition of TMSOTf (4.45 g, 20.0 mmol, 4.0 eq). After the dropwise addition, the mixture was immediately quenched with water (50 mL), followed by liquid separation. The aqueous phase was extracted with DCM (50 mL). The organic phases were combined, dried over anhydrous sodium sulfate, and filtered. The filtrate was concentrated under reduced pressure, and the residue was purified by silica gel column chromatography (DCM:MeOH = 50:1 to 10:1) to give the product (1.30 g, yield: 70.7%).

Step 4: Synthesis of (*R*)-6-(2-(ethoxymethoxy)-4-ethynylphenyl)-*N*-(1-ethylpiperidin-3-yl)-5-methyl-1,2,4-triazi n-3-amine

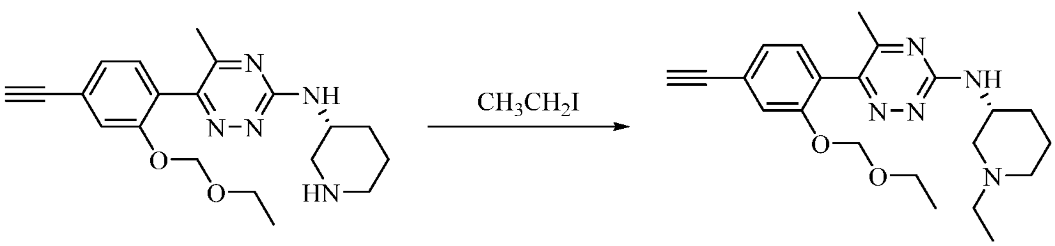


(*R*)-6-(2-(Ethoxymethoxy)-4-ethynylphenyl)-5-methyl-*N*-(piperidin-3-yl)-1,2, 4-triazin-3-amine (250 mg, 0.680

mmol, 1.0 eq) was dissolved in DCM (5 mL), and triethylamine (344 mg, 3.40 mmol, 5.0 eq) and iodoethane (530 mg, 3.40 mmol, 5.0 eq) were added. The mixture was stirred at room temperature for 16 h. Water was added, and extraction was performed with DCM (30 mL × 2). The organic phase was dried over anhydrous sodium sulfate and filtered. The filtrate was concentrated under reduced pressure, and the residue was purified by silica gel column chromatography (DCM:MeOH = 50:1 to 10:1) to give the product (212 mg, yield: 78.8%).

Step 5: Synthesis of (*R*)-2-(3-((1-ethylpiperidin-3-yl)amino)-5-methyl-1,2,4-triazin-6-yl)-5-ethynylphenol

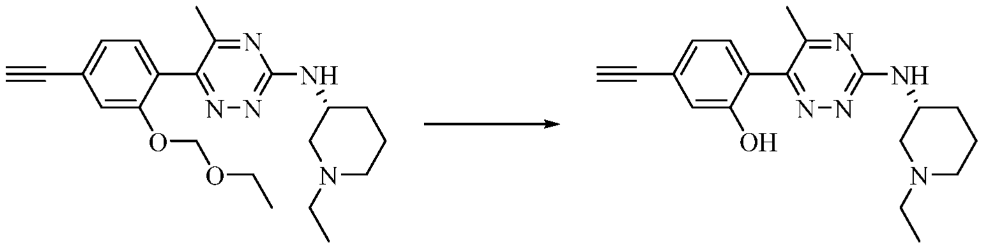

(*R*)-6-(2-(Ethoxymethoxy)-4-ethynylphenyl)-*N*-(1-ethylpiperidin-3-yl)-5-meth yl-1,2,4-triazin-3-amine (200 mg, 0.506 mmol, 1.0 eq) was dissolved in DCM (3 mL), and then a solution of hydrogen chloride in 1,4-dioxane (4 mol/L, 0.51 mL, 2.02 mmol, 4.0 eq) was added dropwise. The mixture was stirred at room temperature for 1 h. The pH was adjusted to 8 with a saturated aqueous $NaHCO_3$ solution, and extraction was performed with DCM (20 mL × 3). The organic phase was dried over anhydrous sodium sulfate and filtered. The filtrate was concentrated under reduced pressure, and the residue was purified by preparative thin-layer chromatography (DCM:MeOH = 10:1) to give the product (102 mg, yield: 59.8%).

$^1$HNMR(400 MHz, DMSO-$d_6$) δ(ppm): 10.17 (s, 1H), 7.60 (s, 1H), 7.26 (d, J= 8.1 Hz, 1H), 7.04 (d, *J* = 6.8 Hz, 2H), 4.23 (s, 1H), 4.10 (m, 1H), 3.18 (s, 1H), 2.93 (s, 1H), 2.67-2.60 (m, 2H), 2.19 (s, 4H), 1.91 (d, *J*= 4.9 Hz, 1H), 1.78 (s, 1H), 1.63 (s, 1H), 1.44 (s, 1H), 1.08 (s, 3H).

Molecular formula: $C_{19}H_{23}N_5O$ Exact molecular weight: 337.19 LC-MS (Pos, m/z) = 338.22 $[M+H]^+$.

**Example 41: Synthesis of (*R*)-5-ethynyl-2-(3-((1-(2-hydroxyethyl) piperidin-3-yl)amino)-5-methyl-1,2,4-triazin-6-yl)phenol (Compound 99)**

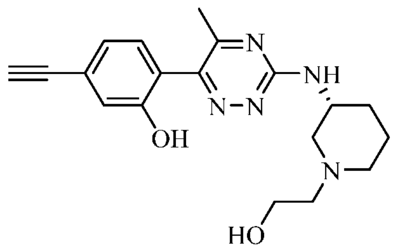

Step 1: Synthesis of (*R*)-2-(3-((6-(2-(ethoxymethoxy)-4-ethynylphenyl)-5-methyl-1,2,4-triazin-3-yl)amino)piper idin-1-yl)ethan-1-ol

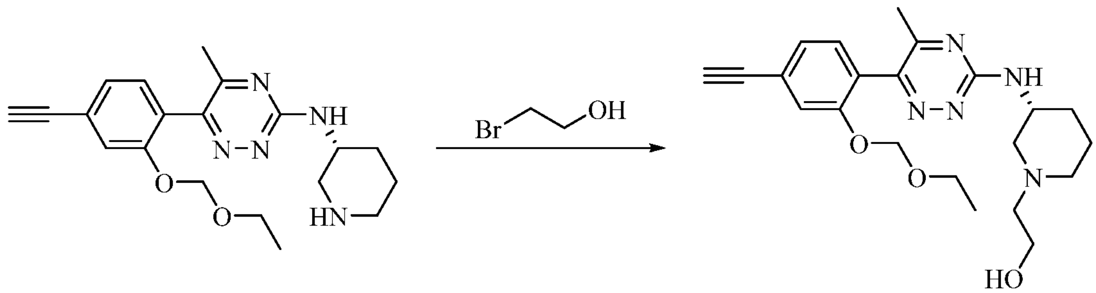


(*R*)-6-(2-(Ethoxymethoxy)-4-ethynylphenyl)-5-methyl-*N*-(piperidin-3-yl)-1,2, 4-triazin-3-amine (250 mg, 0.680 mmol, 1.0 eq) was dissolved in DCM (5 mL), and triethylamine (344 mg, 3.40 mmol, 5.0 eq) and 2-bromoethanol (425 mg, 3.40 mmol, 5.0 eq) were added. The mixture was stirred at room temperature for 30 h. Water was added, and extraction was performed with DCM (30 mL × 2). The organic phase was dried over anhydrous sodium sulfate and filtered. The filtrate was concentrated under reduced pressure, and the residue was purified by silica gel column chromatography (DCM:MeOH = 50:1 to 10:1) to give the product (178 mg, yield: 63.6%).

Step 2: Synthesis of (*R*)-5-ethynyl-2-(3-((1-(2-hydroxyethyl)piperidin-3-yl)amino)-5-methyl-1,2,4-triazin-6-yl)p henol

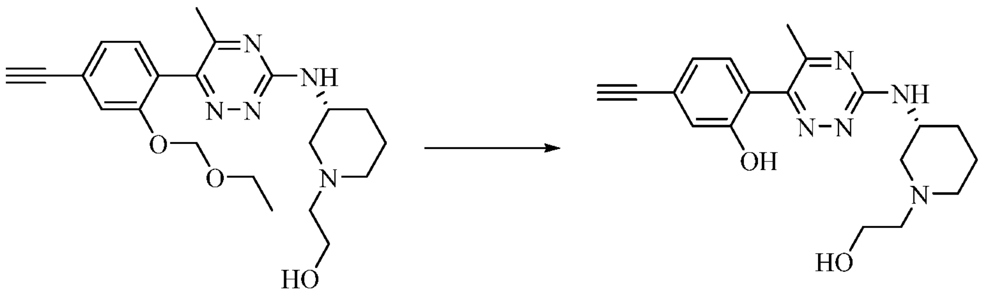

(*R*)-2-(3-((6-(2-(Ethoxymethoxy)-4-ethynylphenyl)-5-methyl-1,2,4-triazin-3-y l)amino)piperidin-1-yl)ethan-1-ol (178 mg, 0.433 mmol, 1.0 eq) was dissolved in DCM (3 mL), and then a solution of hydrogen chloride in 1,4-dioxane (4 mol/L, 0.43 mL, 1.73 mmol, 4.0 eq) was added dropwise. The mixture was stirred at room temperature for 1 h. The pH was adjusted to 8 with a saturated aqueous $NaHCO_3$ solution, and extraction was performed with DCM (20 mL × 3). The organic phase was dried over anhydrous sodium sulfate and filtered. The filtrate was concentrated under reduced pressure, and the residue was purified by silica gel column chromatography (DCM:MeOH = 50:1 to 10:1) to give the product (102 mg, yield: 66.7%).

$^1$HNMR(400 MHz, DMSO-$d_6$) δ(ppm): 10.22 (s, 1H), 7.72 (s, 1H), 7.26 (d, *J*= 7.7 Hz, 1H), 7.07-7.03 (m, 2H), 4.25 (s, 1H), 4.91 (s, 1H), 4.23 (s, 2H), 3.64 (s, 2H), 2.85 (s, 3H), 2.20 (s, 3H), 1.89-1.71 (m, 3H), 1.50 (s, 1H), 0.96-0.82 (m, 1H).

Molecular formula: $C_{19}H_{23}N_5O_2$ Exact molecular weight: 353.19 LC-MS (Pos, m/z) = 354.28 $[M+H]^+$.

**Example 42: Synthesis of (*R*)-5-ethynyl-2-(5-methyl-3-((1-(methyl-$d_3$)piperidin-3-yl)amino)-1,2,4-triazin-6-yl)ph enol (Compound 86)**

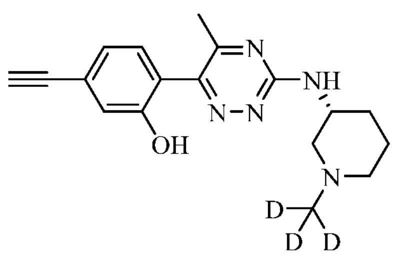

Step 1: Synthesis of (*R*)-6-(2-(ethoxymethoxy)-4-ethynylphenyl)-5-methyl-*N*-(1-(methyl-$d_3$)piperidin-3-yl)-1,2, 4-triazin-3-amine

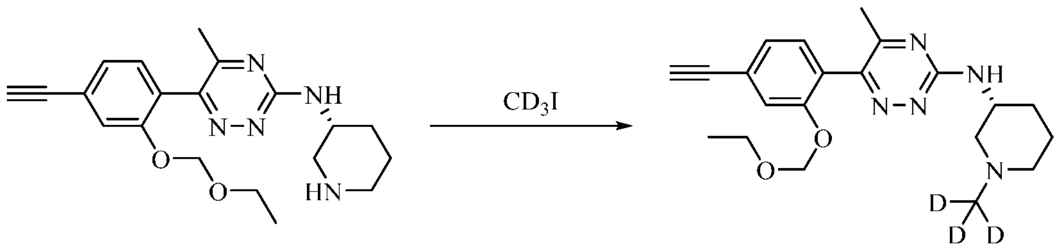


(*R*)-6-(2-(Ethoxymethoxy)-4-ethynylphenyl)-5-methyl-*N*-(piperidin-3-yl)-1,2, 4-triazin-3-amine (450 mg, 1.22 mmol, 1.0 eq) was dissolved in DCM (20 mL), and triethylamine (370 mg, 3.66 mmol, 3.0 eq) and deuterated iodomethane (530 mg, 3.66 mmol, 3.0 eq) were added. The mixture was stirred at room temperature for 2 h. Water was added, followed by liquid separation. The aqueous phase was extracted with DCM (20 mL). The organic phases were combined, dried over anhydrous sodium sulfate, and filtered. The filtrate was concentrated under reduced pressure, and the residue was purified by silica gel column chromatography (DCM:MeOH = 50:1 to 10:1) to give the product (192 mg, yield: 40.8%).

Step 2: Synthesis of (*R*)-5-ethynyl-2-(5-methyl-3-((1-(methyl-$d_3$)piperidin-3-yl)amino)-1,2,4-triazin-6-yl)phenol

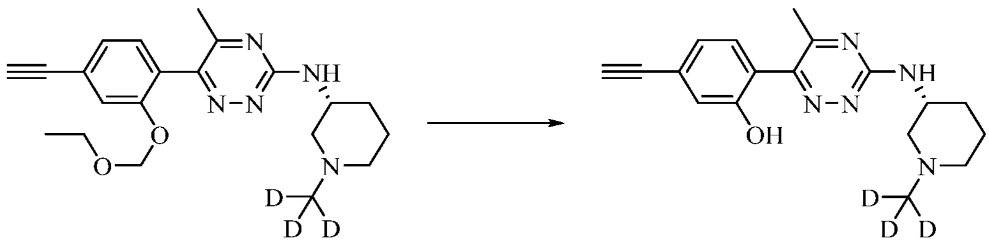

(*R*)-6-(2-(Ethoxymethoxy)-4-ethynylphenyl)-5-methyl-*N*-(1-(methyl-$d_3$)piperi din-3-yl)-1,2,4-triazin-3-amine (192 mg, 0.499 mmol, 1.0 eq) was dissolved in DCM (3 mL), and then a solution of hydrogen chloride in 1,4-dioxane (4 mol/L, 0.50 mL, 2.00 mmol, 4.0 eq) was added dropwise. The mixture was stirred at room temperature for 1 h. The pH was adjusted to 8 with a saturated aqueous $NaHCO_3$ solution, and extraction was performed with DCM (20 mL × 3). The organic phase was dried over anhydrous sodium sulfate and filtered. The filtrate was concentrated under reduced pressure, and the residue was purified by silica gel column chromatography (DCM:MeOH = 50:1 to 10:1) to give the product (108 mg, yield: 66.3%).

$^1$HNMR (400 MHz, DMSO-$d_6$) δ(ppm): 10.10 (s, 1H), 7.45 (s, 1H), 7.26 (d, *J* = 7.6 Hz, 1H), 7.04-7.02 (m, 2H), 4.22 (s, 1H), 3.99 (s, 1H), 2.87 (s, 1H), 2.63 (d, *J* = 10.8 Hz, 1H), 2.18 (s, 3H), 1.90-1.84 (m, 3H), 1.72-1.68 (m, 1H), 1.58-1.49 (m, 1H), 1.35-1.29 (m, 1H).

Molecular formula: $C_{18}H_{18}D_3N_5O$ Exact molecular weight: 326.19 LC-MS (Pos, *m/z*) = 327.24 $[M+H]^+$.

**Example 43: Synthesis of 2-(4-difluoromethyl-6-(((cis)-3-hydroxy-3-methylcyclobutyl)amino)pyridazin-3-yl)-5-et hynylphenol (Compound 102)**

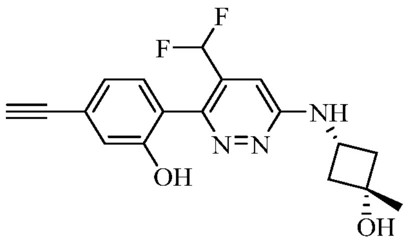

Step 1: Synthesis of intermediate 3,6-dichloro-4-difluoromethylpyridazine

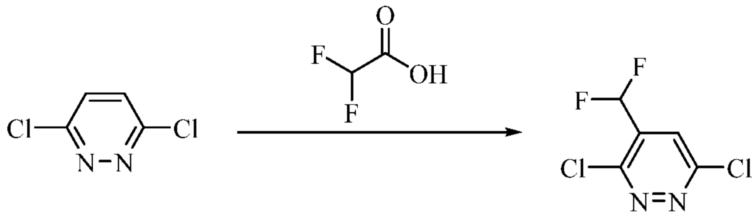

3,6-Dichloropyridazine (30.0 g, 148.98 mmol, 1.0 eq) was dissolved in water (800 mL), and difluoroacetic acid (38.6 g, 402 mmol, 2.0 eq) and silver nitrate (34.2 g, 201 mmol, 1.0 eq) were added to the solution. Concentrated sulfuric acid (33 mL, 603 mmol, 3.0 eq) was added dropwise to the reaction solution at 50 °C. After the dropwise addition, the mixture was heated to 60 °C. An aqueous solution (400 mL) of ammonium persulfate (137.6 g, 603 mmol, 3.0 eq) was then added dropwise to the reaction solution. After the dropwise addition, the mixture was heated to 70 °C and allowed to react for 5 min. The pH of the reaction solution was adjusted to 8 with a 15% aqueous sodium hydroxide solution, and extraction was performed with ethyl acetate (500 mL × 2). The organic phases were combined, dried over anhydrous sodium sulfate, and concentrated, and the crude product was purified by silica gel column chromatography (petroleum ether:ethyl acetate = 100:1) to give the product (8.98 g, yield: 22.5%).

Step 2: Synthesis of intermediate (*cis*)-3-((6-chloro-5-difluoromethylpyridazin-3-yl)amino)-1-methylcyclobutan-1-ol

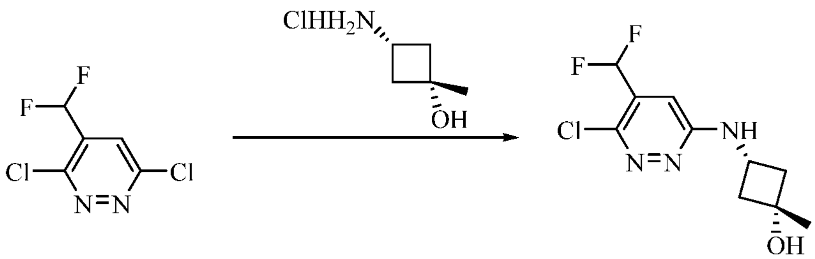

3,6-Dichloro-4-difluoromethylpyridazine (3.3 g, 16.58 mmol, 1.0 eq) was dissolved in *n*-butanol (33 mL), and (*cis*)-3-amino-1-methylcyclobutan-1-ol hydrochloride (4.56 g, 33.16 mmol, 2.0 eq) and N,N-diisopropylethylamine (8.57 g, 66.32 mmol, 4.0 eq) were added to the solution. The mixture was allowed to react at 120 °C for 1 h, and TLC analysis showed the completion of the reaction. The reaction solution was concentrated. Water (50 mL) was added, and extraction was performed with dichloromethane (50 mL × 10). The organic phases were combined, dried over anhydrous magnesium sulfate, and concentrated, and the crude product was purified by silica gel column chromatography (petroleum ether:ethyl acetate = 3:1 to 1:1) to give the product (1.35 g, yield: 30.9%).

Step 3: Synthesis of intermediate 4-(4-difluoromethyl-6-(((*cis*)-3-hydroxy-3-methylcyclobutyl)amino)pyiidazin-3-yl)-3-(etho xymethoxy)benzaldehyde

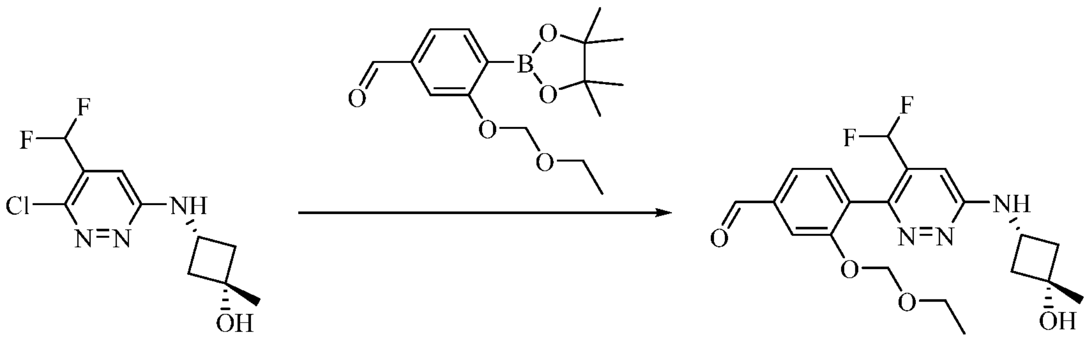

(*cis*)-3-((6-Chloro-5-difluoromethylpyridazin-3-yl)amino)-1-methylcyclobuta n-1-ol (200 mg, 0.759 mmol, 1.0 eq) was dissolved in 1,4-dioxane (2 mL), and 3-(ethoxymethoxy)-4-(4,4,5,5-tetramethyl-1,3,2-dioxaborolan-2-yl)benzaldehyde (349 mg, 1.139 mmol, 1.5 eq), potassium carbonate (210 mg, 1.518 mmol, 2.0 eq), tetrakis(triphenylphosphine) palladium (88 mg, 0.0759 mmol, 0.1 eq), and water (0.5 mL) were added to the solution. The mixture was heated to 90 °C and allowed to react for 6 h under a nitrogen atmosphere, and TLC analysis showed the completion of the reaction. The reaction solution was filtered through celite. The filter cake was washed with dichloromethane (20 mL), and the filtrate was dried over anhydrous magnesium sulfate, filtered, and concentrated. The crude product was purified by silica gel column chromatography (petroleum ether:ethyl acetate = 2:1 to 1:2) to give the product (178 mg, yield: 57.6%).

Step 4: Synthesis of intermediate (*cis*)-3-((5-difluoromethyl-6-(2-ethoxymethoxy-4-ethynylphenyl)pyridazin-3-yl)amino)-1-methylcyclobutan-1-ol

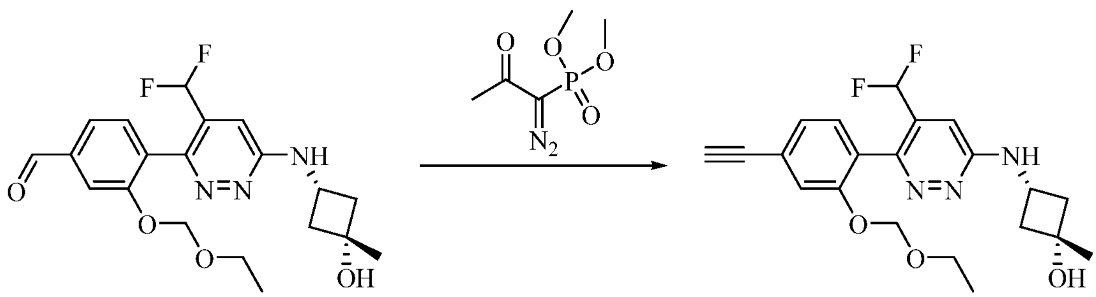

4-(4-Difluoromethyl-6-(((*cis*)-3-hydroxy-3-methylcyclobutyl)amino)pyridazin -3-yl)-3-(ethoxymethoxy)benzaldehyde (178 mg, 0.437 mmol, 1.0 eq) was dissolved in methanol (3 mL), and potassium carbonate (121 mg, 0.874 mmol, 2.0 eq) and dimethyl (1-diazo-2-oxopropyl)phosphonate (126 mg, 0.656 mmol, 1.5 eq) were sequentially added to the solution. The mixture was allowed to react at 25 °C for 2 h, and TLC analysis showed the completion of the reaction. The reaction solution was concentrated. Water (20 mL) was added, and extraction was performed with dichloromethane (10 mL × 2). The organic phases were combined, dried over anhydrous magnesium sulfate, and concentrated to give the product (117 mg, yield: 66.5%).

Step 5: Synthesis of compound 2-(4-difluoromethyl-6-(((*cis*)-3-hydroxy-3-methylcyclobutyl)amino)pyridazin-3-yl)-5-ethyn ylphenol

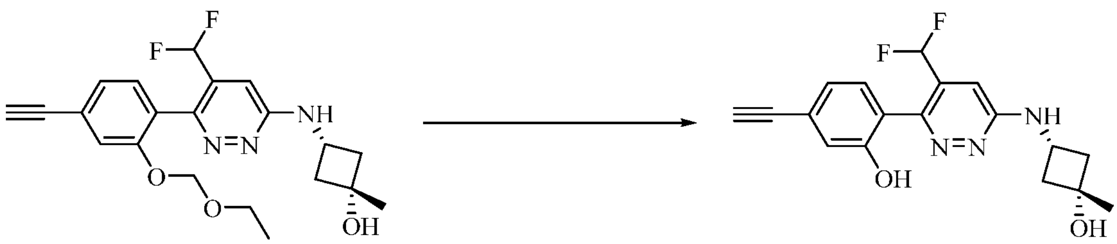

(*cis*)-3-((5-Difluoromethyl-6-(2-ethoxymethoxy-4-ethynylphenyl)pyridazin-3-yl)amino)-1-methylcyclobutan-1-ol (117 mg, 0.29 mmol, 1.0 eq) was dissolved in dichloromethane (1 mL), and the solution was added dropwise to trifluoroacetic acid (1 mL). The mixture was allowed to react at 25 °C for 10 min, and TLC analysis showed the completion of the reaction. The reaction solution was concentrated, and the crude product was purified by preparative thin-layer chromatography (petroleum ether:ethyl acetate = 1:2) to give the product (37 mg, yield: 37.0%).

$^1$H-NMR (400 MHz, DMSO-$d_6$) $\delta$(ppm): 10.24 (s, 1H), 7.49-7.48 (d, 1H), 7.27-7.25 (d, 1H), 7.04-7.00 (m, 3H), 6.77 (t, J=54.6 Hz, 1H), 5.06 (s, 1H), 4.32 (s, 1H), 4.06-3.98 (m, 1H), 2.47-2.42 (m, 2H), 1.99-1.95 (m, 2H), 1.30 (s, 3H).

Molecular formula: $C_{18}H_{17}F_2N_3O_2$ Exact molecular weight: 345.13 LC-MS (*m/z*): 346.15 [M+H]$^+$.

**Example 44: Synthesis of 2-(4,5-bis(difluoromethyl)-6-(((*cis*)-3-hydroxy-3-methylcyclobutyl)amino)pyridazin-3-yl)-5-ethynylphenol (Compound 110)**

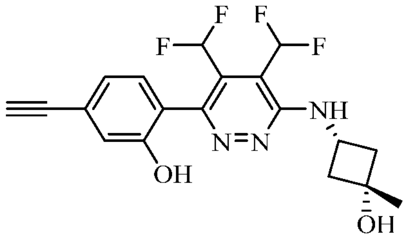

Step 1: Synthesis of intermediate 3,6-dichloro-4,5-bis(difluoromethyl)pyridazine

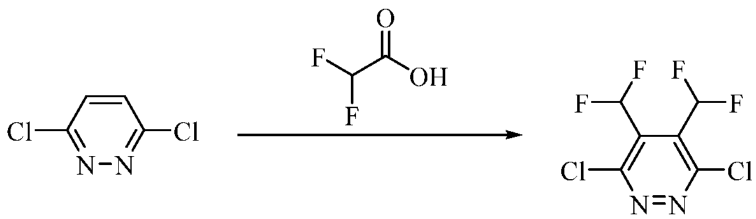

3,6-Dichloropyridazine (30.0 g, 148.98 mmol, 1.0 eq) was dissolved in water (800 mL), and difluoroacetic acid (38.6 g, 402 mmol, 2.0 eq) and silver nitrate (34.2 g, 201 mmol, 1.0 eq) were added to the solution. Concentrated sulfuric acid (33 mL, 603 mmol, 3.0 eq) was added dropwise to the reaction solution at 50 °C. After the dropwise addition, the mixture was heated to 60 °C. An aqueous solution (400 mL) of ammonium persulfate (137.6 g, 603 mmol, 3.0 eq) was then added dropwise to the reaction solution. After the dropwise addition, the mixture was heated to 70 °C and allowed to react for 5 min. The pH was adjusted to 8 with a 15% aqueous sodium hydroxide solution, and extraction was performed with ethyl acetate (500 mL × 2). The organic phases were combined, dried over anhydrous sodium sulfate, and concentrated, and the crude product was purified by silica gel column chromatography (petroleum ether:ethyl acetate = 100:1) to give the product (9.15 g, yield: 18.3%).

Step 2: Synthesis of intermediate (*cis*)-3-((6-chloro-4,5-bis(difluoromethyl) pyridazin-3-yl)amino)-1-methylcyclobutan-1-ol

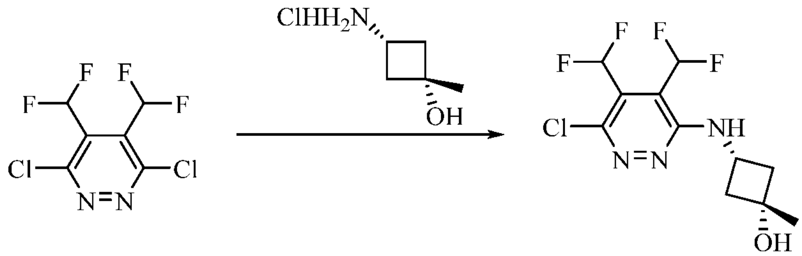


3,6-Dichloro-4,5-bis(difluoromethyl)pyridazine (2.5 g, 10.04 mmol, 1.0 eq) was dissolved in *n*-butanol (20 mL), and (*cis*)-3-amino-1-methylcyclobutan-1-ol hydrochloride (2.76 g, 20.08 mmol, 2.0 eq) and *N,N*-diisopropylethylamine (5.19 g, 40.16 mmol, 4.0 eq) were added to the solution. The mixture was allowed to react at 120 °C for 0.5 h, and TLC analysis showed the completion of the reaction. The reaction solution was concentrated, and the crude product was purified by silica gel column chromatography (petroleum ether:ethyl acetate = 3:1 to 2:1) to give the product (232 mg, yield: 7.3%).

Step 3: Synthesis of intermediate 4-(4,5-bis(difluoromethyl)-6-(((*cis*)-3-hydroxy-3-methylcyclobutyl)amino)pyridazin-3-yl)-3 -(ethoxymethoxy)benzaldehyde

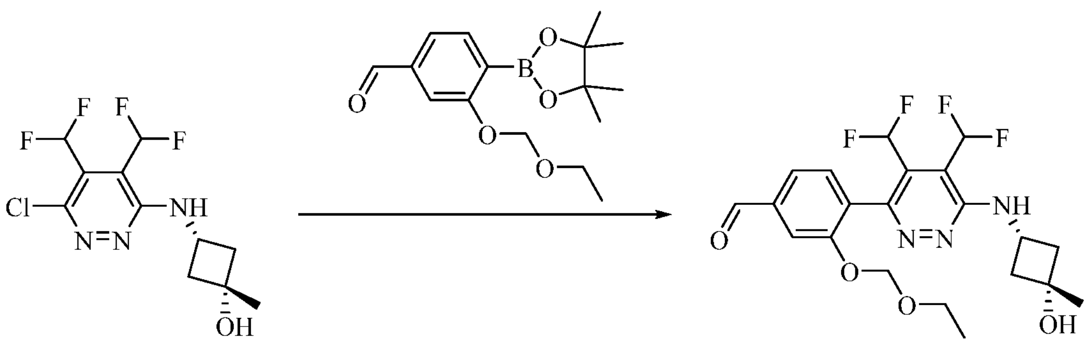

(*cis*)-3-((6-Chloro-4,5-bis(difluoromethyl)pyridazin-3-yl)amino)-1-methylcycl obutan-1-ol (212 mg, 0.676 mmol, 1.0 eq) was dissolved in 1,4-dioxane (2 mL), and 3-(ethoxymethoxy)-4-(4,4,5,5-tetramethyl-1,3,2-dioxaborolan-2-yl) benzaldehyde (310 mg, 1.014 mmol, 1.5 eq), potassium carbonate (187 mg, 1.352 mmol, 2.0 eq), tetrakis(triphenylphosphine)palladium (78 mg, 0.0676 mmol, 0.1 eq), and water (0.5 mL) were added to the solution. The mixture was heated to 90 °C and allowed to react for 1 h under a nitrogen atmosphere, and TLC analysis showed the completion of the reaction. The reaction solution was filtered through celite. The filter cake was washed with dichloromethane (10 mL), and the filtrate was dried over anhydrous magnesium sulfate, filtered, and concentrated. The crude product was purified by silica gel column chromatography (petroleum ether:ethyl acetate = 3:1) to give the product (188 mg, yield: 60.8%).

Step 4: Synthesis of intermediate *(cis)-3-((4,5-bis(difluoromethyl)-6-(2-ethoxymethoxy-4-ethynylphenyl)pyridazin-3-yl) amin* o)-1-methylcyclobutan-1-ol

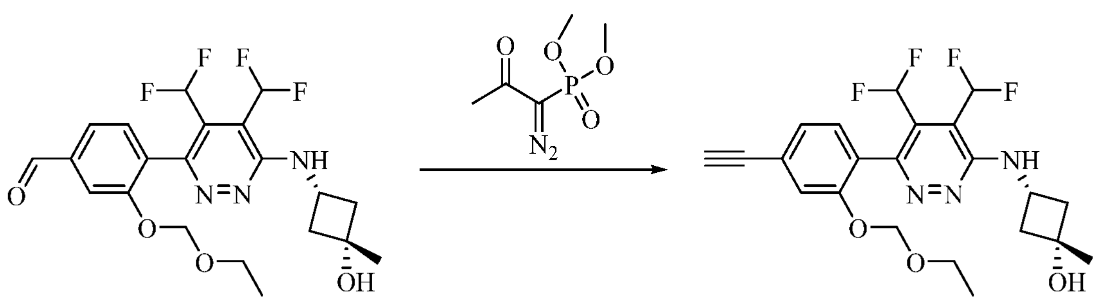

4-(4,5-Bis(difluoromethyl)-6-(((*cis*)-3-hydroxy-3-methylcyclobutyl)amino)pyr idazin-3-yl)-3-(ethoxymethoxy)benzaldehyde (188 mg, 0.41 mmol, 1.0 eq) was dissolved in methanol (2 mL), and potassium carbonate (113 mg, 0.82 mmol, 2.0 eq) and dimethyl (1-diazo-2-oxopropyl)phosphonate (119 mg, 0.62 mmol, 1.5 eq) were sequentially added to the solution. The mixture was allowed to react at 25 °C for 3 h, and TLC analysis showed the completion of the reaction. The reaction solution was concentrated. Water (5 mL) was added to the concentrate, and extraction was performed with dichloromethane (5 mL × 3). The organic phases were combined, dried over anhydrous magnesium sulfate, and concentrated, and the crude product was purified by silica gel column chromatography (petroleum ether:ethyl acetate = 1:1) to give the product (100 mg, yield: 53.8%).

Step 5: Synthesis of compound 2-(4,5-bis(difluoromethyl)-6-(((*cis*)-3-hydroxy-3-methylcyclobutyl)amino)pyridazin-3-yl)-5 -ethynylphenol

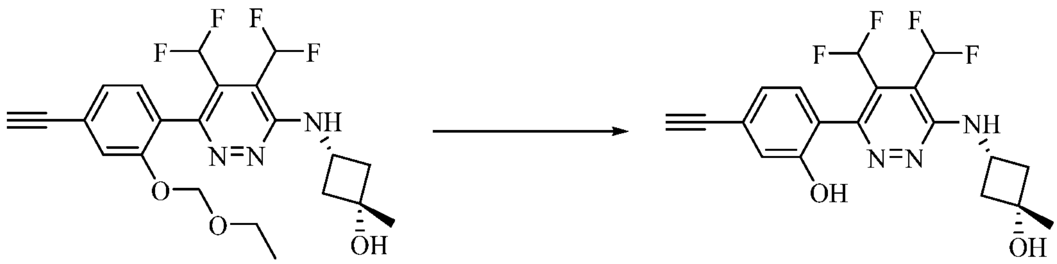

(*cis*)-3-((4,5-Bis(difluoromethyl)-6-(2-ethoxymethoxy-4-ethynylphenyl)pyrida zin-3-yl)amino)-1-methylcyclobutan-1-ol (100 mg, 0.22 mmol, 1.0 eq) was dissolved in dichloromethane (1 mL), and the solution was added dropwise to trifluoroacetic acid (1 mL). The mixture was allowed to react at 25 °C for 5 min, and TLC analysis showed the completion of the reaction. The reaction solution was concentrated, and the crude product was purified by preparative thin-layer chromatography (petroleum ether:ethyl acetate = 1:1) to give the product (24 mg, yield: 27.6%).

$^1$H-NMR (400 MHz, DMSO-$d_6$) δ(ppm): 10.32 (s, 1H), 7.56-7.30 (m, 2H), 7.07-7.02 (t, 2H), 6.86-6.60 (m, 2H), 5.01 (s, 1H), 4.26-4.21 (m, 2H), 2.47-2.45 (m, 2H), 2.16-2.11 (m, 2H), 1.30 (s, 3H).

Molecular formula: $C_{19}H_{17}F_4N_3O_2$ Exact molecular weight: 395.13 LC-MS (*m*/*z*): 396.13 [M+H]$^+$.

**Example 45: Synthesis of (*R*)-6-(4-ethynyl-2-hydroxyphenyl)-5-methyl-3-((1-methylpiperidin-3-yl)amino)pyrida zine-4-carbonitrile (Compound 103)**

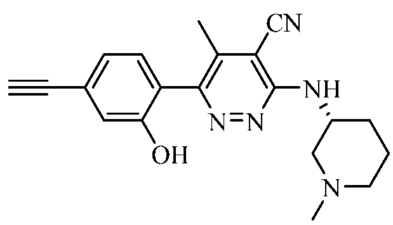

Step 1: Synthesis of intermediate 3,6-dichloropyridazine-4-carbonyl chloride

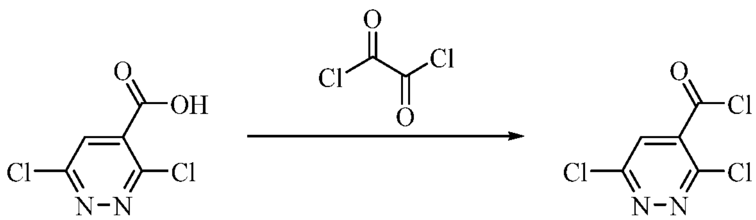

3,6-Dichloropyridazine-4-carboxylic acid (24.07 g, 124.7 mmol, 1.0 eq) was dissolved in dichloromethane (240

mL), and N,N-dimethylformamide (0.05 mL) was added dropwise to the solution. Oxalyl chloride (47.48 g, 374.1 mmol, 3.0 eq) was added dropwise and slowly to the mixture at 0 °C. After the addition, the resulting mixture was allowed to react at 25 °C for 2 h, and TLC analysis showed the completion of the reaction. The reaction solution was concentrated, and the crude product was directly used in the next step.

Step 2: Synthesis of intermediate 3,6-dichloropyridazine-4-carboxamide

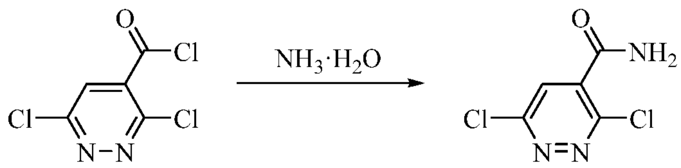


3,6-Dichloropyridazine-4-carbonyl chloride (crude product, 124.7 mmol, 1.0 eq) was dissolved in acetonitrile (250 mL), and the solution was added dropwise to aqueous ammonia (350 g, 2494 mmol, 10.0 eq) at 0 °C. After the dropwise addition, TLC analysis showed the completion of the reaction. Extraction was performed with ethyl acetate (300 mL × 4). The organic phases were combined, dried over anhydrous sodium sulfate, and concentrated to give the product (22.94 g, yield: 95.8%).

Step 3: Synthesis of intermediate 3,6-dichloropyridazine-4-carbonitrile

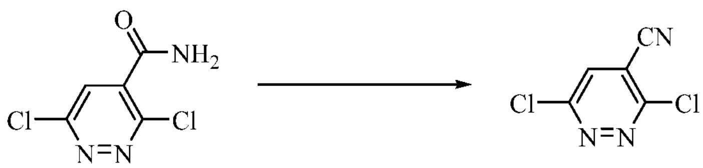

3,6-Dichloropyridazine-4-carboxamide (22.94 g, 119.5 mmol, 1.0 eq) was dissolved in dichloromethane (230 mL), and triethylamine (48.37 g, 478.0 mmol, 4.0 eq) and trifluoroacetic anhydride (50.2 g, 239.0 mmol, 2.0 eq) were sequentially added to the solution at 0 °C. After the dropwise addition, the mixture was allowed to react at 0 °C for 1 h. The reaction mixture was then warmed to 25 °C and allowed to react for 1 h, and TLC analysis showed the completion of the reaction. The reaction solution was poured into a saturated aqueous sodium bicarbonate solution (200 mL) and extracted with dichloromethane (200 mL × 3). The organic phases were combined, dried over anhydrous magnesium sulfate, and concentrated, and the crude product was purified by silica gel column chromatography (petroleum ether:ethyl acetate = 10:1) to give the product (15.86 g, yield: 76.3%).

Step 4: Synthesis of intermediate 3,6-dichloro-5-methylpyridazine-4-carbonitrile

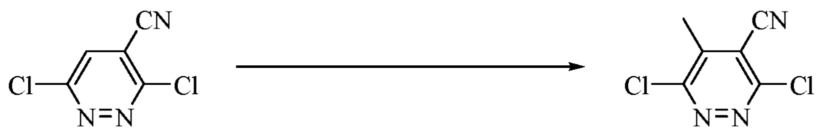

3,6-Dichloropyridazine-4-carbonitrile (4.69 g, 26.96 mmol, 1.0 eq) was dissolved in water (150 mL), and acetic acid (4.86 g, 80.88 mmol, 3.0 eq) and silver nitrate (4.58 g, 26.96 mmol, 1.0 eq) were added to the solution. Concentrated sulfuric acid (4.3 mL, 80.88 mmol, 3.0 eq) was added dropwise to the reaction solution at 50 °C. After the dropwise addition, the mixture was heated to 60 °C. An aqueous solution (50 mL) of ammonium persulfate (18.46 g, 80.88 mmol, 3.0 eq) was then added dropwise to the reaction solution. After the dropwise addition, the mixture was allowed to react at 70 °C for 10 min, and TLC analysis showed the completion of the reaction. The pH was adjusted to 8 with a 15% aqueous sodium hydroxide solution, and extraction was performed with ethyl acetate (300 mL × 3). The organic phases were combined, dried, and concentrated, and the residue was purified by silica gel column chromatography (petroleum ether:ethyl acetate

= 20:1) to give the product (2.8 g, yield: 55.3%).

Step 5: Synthesis of *tert-butyl* (*R*)-3-((6-chloro-4-cyano-5-methylpyridazin-3-yl)amino)piperidine-1-carboxylate

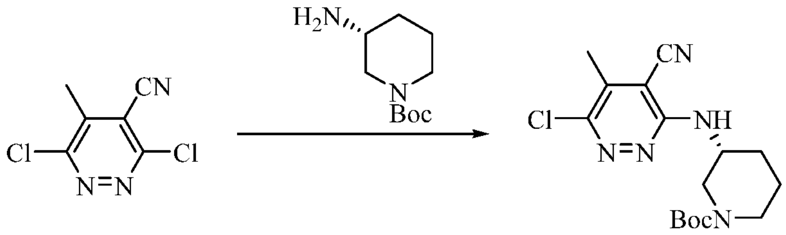

3,6-Dichloro-5-methylpyridazine-4-carbonitrile (1.4 g, 7.45 mmol, 1.0 eq) was dissolved in *N,N*-dimethylacetamide (10 mL), and (*R*)-1-*tert*-butoxycarbonyl-3-aminopiperidine (2.98 g, 14.9 mmol, 2.0 eq) and *N,N*-diisopropylethylamine (1.93 g, 14.9 mmol, 2.0 eq) were added to the solution. The mixture was allowed to react at 120 °C for 0.5 h, and TLC analysis showed the substantial completion of the reaction. The reaction solution was poured into water (30 mL) and extracted with ethyl acetate (20 mL × 2). The organic phases were combined, dried over anhydrous sodium sulfate, and concentrated, and the crude product was purified by silica gel column chromatography (PE:EA = 10:1 to 4:1) to give the product (714 mg, yield: 27.3%).

Step 6: Synthesis of intermediate (*R*)-6-chloro-5-methyl-3-(piperidin-3-ylamino)pyridazine-4-carbonitrile

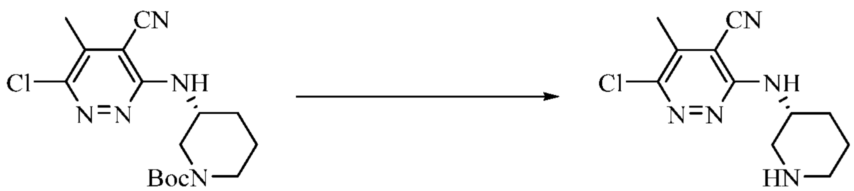

*tert*-Butyl (*R*)-3-((6-chloro-4-cyano-5-methylpyridazin-3-yl)amino)piperidine-1-carboxylate (714 mg, 2.03 mmol) was dissolved in dichloromethane (10 mL), and the solution was added dropwise to trifluoroacetic acid (10 mL). The mixture was allowed to react at 25 °C for 5 min, and TLC analysis showed the completion of the reaction. The reaction solution was concentrated, and the residue was dissolved in dichloromethane (10 mL). The resulting solution was poured into a saturated aqueous sodium bicarbonate solution (20 mL), followed by liquid separation. The aqueous phase was extracted with a mixed solvent (dichloromethane:methanol = 5:1) (10 mL × 10). The organic phases were combined, dried over anhydrous magnesium sulfate, and concentrated to give a crude product, which was directly used in the next step.

Step 7: Synthesis of intermediate (*R*)-6-chloro-5-methyl-3-((1-methylpiperidin-3-yl)amino)pyridazine-4-carbonitrile

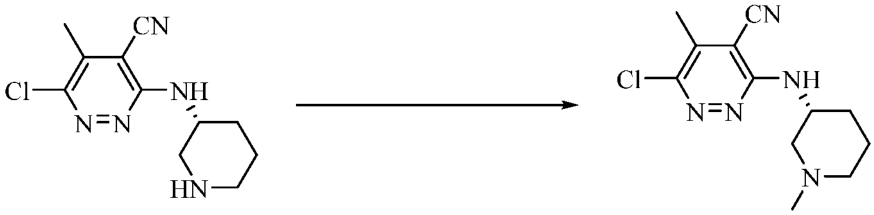

(*R*)-6-Chloro-5-methyl-3-(piperidin-3-ylamino)pyridazine-4-carbonitrile (crude product, 2.03 mmol, 1.0 eq) was dissolved in methanol (5 mL), and an aqueous formaldehyde solution (mass fraction: 37%) (165 mg, 2.03 mmol, 1.0 eq) was added to the solution. The mixture was allowed to react at 25 °C for 2 h, and sodium cyanoborohydride (140 mg, 2.23

mmol, 1.1 eq) was added. The resulting mixture was allowed to react at 25 °C for 5 min, and TLC analysis showed the completion of the reaction. The reaction solution was concentrated. A saturated aqueous sodium bicarbonate solution (5 mL) was added to the concentrate, and extraction was performed with dichloromethane (10 mL × 3). The organic phases were combined, dried over anhydrous magnesium sulfate, and concentrated, and the crude product was purified by silica gel column chromatography (dichloromethane:methanol = 100:1 to 10:1) to give the product (402 mg, two-step yield: 74.6%).

Step 8: Synthesis of intermediate (*R*)-6-(2-ethoxymethoxy-4-formylphenyl)-5-methyl-3-((1-methylpiperidin-3-yl)amino) pyri dazine-4-carbonitrile

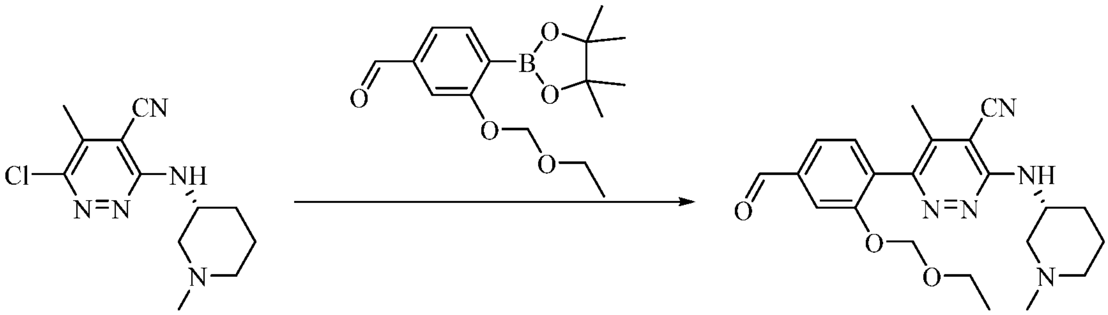

(*R*)-6-Chloro-5-methyl-3-((1-methylpiperidin-3-yl)amino)pyridazine-4-carbon itrile (172 mg, 0.645 mmol, 1.0 eq) was dissolved in 1,4-dioxane (2 mL), and 3-ethoxymethoxy-5-methyl-4-(4,4,5,5-tetramethyl-1,3,2-dioxaborolan-2-yl) benzaldehyde (238 mg, 0.774 mmol, 1.2 eq), an aqueous solution (0.5 mL) of potassium carbonate (178 mg, 1.29 mmol, 2.0 eq), and $Pd(PPh_3)_4$ (75 mg, 0.0645 mmol, 0.1 eq) were sequentially added to the solution. The mixture was heated to 90 °C and allowed to react for 3 h under a nitrogen atmosphere, and TLC analysis showed the completion of the reaction. The reaction solution was filtered through celite. The filtrate was concentrated, and the crude product was purified by silica gel column chromatography (dichloromethane:methanol = 100:1 to 50:1) to give the product (121 mg, yield: 45.8%).

Step 9: Synthesis of intermediate (*R*)-6-(2-ethoxymethoxy-4-ethynylphenyl)-5-methyl-3-((1-methylpiperidin-3-yl)amino)pyri dazine-4-carbonitrile

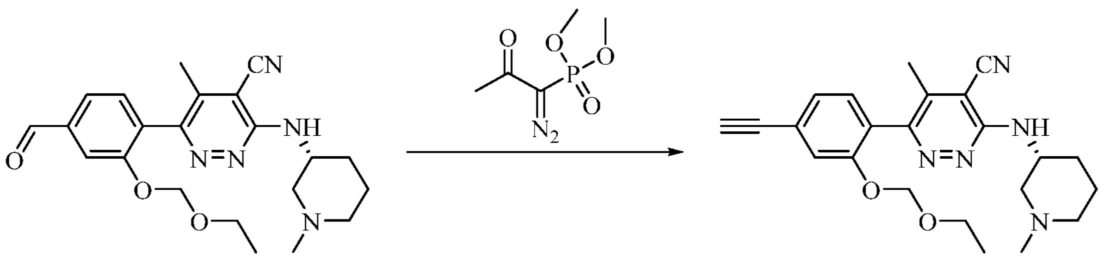

(*R*)-6-(2-Ethoxymethoxy-4-formylphenyl)-5-methyl-3-((1-methylpiperidin-3-yl)amino)pyridazine-4-carbonitrile (121 mg, 0.295 mmol, 1.0 eq) was dissolved in methanol (2 mL), and potassium carbonate (82 mg, 0.59 mmol, 2.0 eq) and dimethyl (1-diazo-2-oxopropyl)phosphonate (85 mg, 0.443 mmol, 1.5 eq) were sequentially added to the solution. The mixture was allowed to react at 25 °C for 3 h, and TLC analysis showed the completion of the reaction. The reaction solution was concentrated. Water (5 mL) was added, and extraction was performed with dichloromethane (5 mL × 3). The organic phases were combined, dried over anhydrous magnesium sulfate, and concentrated, and the crude product was purified by preparative thin-layer chromatography (dichloromethane:methanol = 7:1) to give the product (40 mg, yield: 33.3%).

Step 10: Synthesis of compound (*R*)-6-(4-ethynyl-2-hydroxyphenyl)-5-methyl-3-((1-methylpiperidin-3-yl)amino)pyridazine-4-carbonitrile

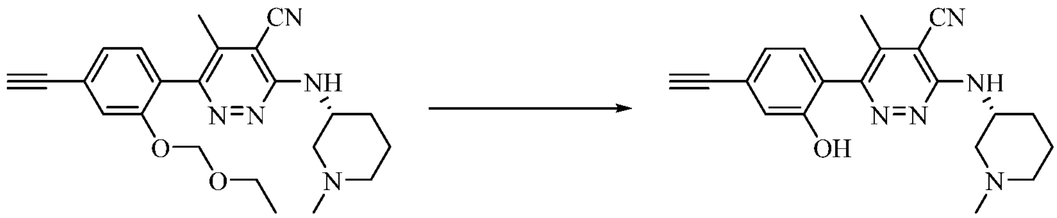

(*R*)-6-(2-Ethoxymethoxy-4-ethynylphenyl)-5-methyl-3-((1-methylpiperidin-3-yl)amino)pyridazine-4-carbonitrile (40 mg, 0.0986 mmol) was dissolved in dichloromethane (1 mL), and the solution was added dropwise and slowly to trifluoroacetic acid (1 mL). The mixture was allowed to react at 25 °C for 5 min, and TLC analysis showed the completion of the reaction. The reaction solution was concentrated, and dichloromethane (5 mL) was added to the concentrate. The pH was adjusted to alkalinity with a saturated aqueous sodium bicarbonate solution, and extraction was performed with a mixed solvent (dichloromethane:methanol = 10:1) (10 mL × 3). The organic phases were combined, dried over anhydrous magnesium sulfate, and concentrated, and the crude product was purified by preparative thin-layer chromatography (dichloromethane:methanol = 7:1) to give the product (9 mg, yield: 26.5%).

$^1$H-NMR (400 MHz, DMSO-$d_6$) δ(ppm): 10.16 (s, 1H), 7.23-7.21 (t, 1H), 7.05-6.95 (m, 2H), 6.90 (s, 1H), 4.42 (s, 1H), 4.24 (s, 1H), 3.35 (s, 3H), 3.12-3.05 (m, 1H), 2.78-2.74 (m, 1H), 2.40-2.34 (m, 2H), 2.24 (s, 3H), 1.91-1.85 (m, 2H), 1.62-1.54 (m, 2H).

Molecular formula: $C_{20}H_{21}N_5O$ Exact molecular weight: 347.17 LC-MS (*m/z*): 348.21 $[M+H]^+$.

**Example 46: Synthesis of (*R*)-2-(4-methyl-6-((1-methylpiperidin-3-yl)amino)pyridazin-3-yl)-5-(3,3,3-trifluoropro p-1-yn-1-yl)phenol (Compound 111)**

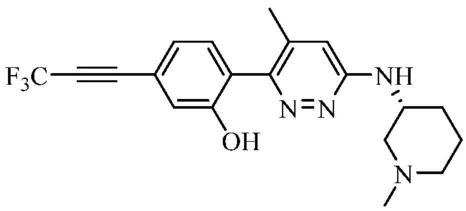

Step 1: Synthesis of *tert*-butyl (*R*)-3-((6-(2-(ethoxymethoxy)-4-(3,3,3-trifluoroprop-1-yn-1-yl)phenyl)-5-methylpyridazin-3-yl)amino)piperidine-1-carboxylate

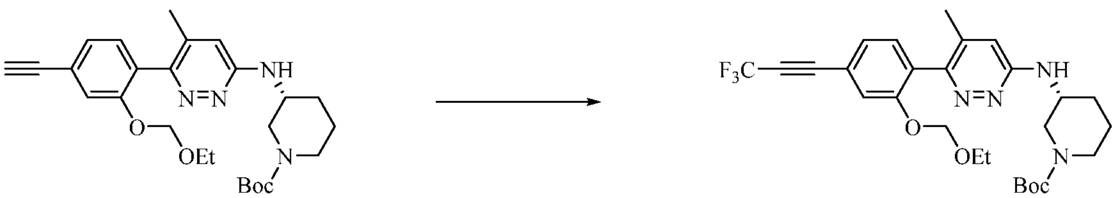

Togni's reagent (749.9 mg, 2.27 mmol, 2.0 eq), CuI (129.8 mg, 0.68 mmol, 0.6 eq), 1,10-phenanthroline (122.8 mg, 0.68 mmol, 0.6 eq), and $KHCO_3$ (227.4 mg, 2.27 mmol, 2.0 eq) were added to DCM (10 mL). *tert*-Butyl (*R*)-3-((6-(2-(ethoxymethoxy)-4-ethynylphenyl)-5-methylpyridazin-3-yl)amino)piperidine-1-carboxylate (530.0 mg, 1.14 mmol, 1.0 eq) was dissolved in DCM (10 mL) under a nitrogen atmosphere, and the solution was added into a syringe, and then injected evenly and slowly into the above reaction system over 6 h. After the injection, the mixture was stirred at room temperature overnight. TLC showed the completion of the reaction. Water (15 mL) was added, followed by liquid separation. The aqueous phase was then extracted with DCM (10 mL × 2). The organic phases were combined, dried over anhydrous sodium sulfate, and filtered. The filtrate was concentrated under reduced pressure, and the residue was purified by preparative thin-layer chromatography (DCM:MeOH = 10:1) to give the product (374.6 mg, yield: 61.7%).

Step 2: Synthesis of (*R*)-2-(4-methyl-6-(piperidin-3-ylamino)pyridazin-3-yl)-5-(3,3,3-trifluoroprop-1-yn-1-yl)ph enol

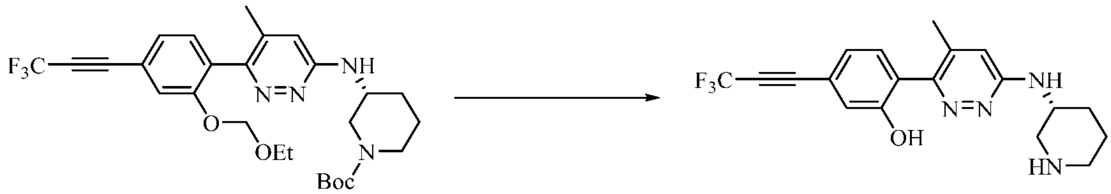

*tert*-Butyl (*R*)-3-((6-(2-(ethoxymethoxy)-4-(3,3,3-trifluoroprop-1-yn-1-yl)phenyl)-5-methylpyridazin-3-yl)amino) piperidine-1-carboxylate (374.6 mg, 0.70 mmol) was dissolved in DCM (2 mL), and a 4 mol/L solution of hydrogen chloride in 1,4-dioxane (2 mL) was added dropwise. The mixture was allowed to react at room temperature for 1 h. TLC monitoring showed the completion of the reaction. Water (2 mL) was added. The pH was adjusted to 8 with a saturated $NaHCO_3$ solution, and extraction was performed with a mixed solvent (DCM:MeOH = 10:1) (2 mL × 5). The organic phases were combined, dried over anhydrous sodium sulfate, and filtered. The filtrate was concentrated under reduced pressure, and the residue was purified by preparative thin-layer chromatography (DCM: 7 mol/L solution of ammonia in methanol = 10:1) to give the product (137.5 mg, yield: 52.1%).

Step 3: Synthesis of (*R*)-2-(4-methyl-6-((1-methylpiperidin-3-yl)amino) pyridazin-3-yl)-5-(3,3,3-trifluoroprop-1-yn-1-yl) phenol

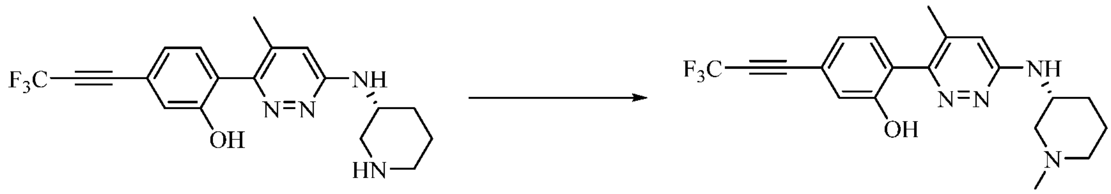

(*R*)-2-(4-Methyl-6-(piperidin-3-ylamino)pyridazin-3-yl)-5-(3,3,3-trifluoroprop -1-yn-1-yl)phenol (137.5 mg, 0.37 mmol, 1.0 eq) was dissolved in MeOH (2 mL), and an aqueous formaldehyde solution (37%, 29.7 mg, 0.37 mmol, 1.0 eq) was added. The mixture was stirred for 5 min, and then sodium cyanoborohydride (23.0 mg, 0.37 mmol, 1.0 eq) was added. The resulting mixture was stirred and allowed to react at room temperature for 1 h. TLC monitoring showed the completion of the reaction. The reaction mixture was concentrated under reduced pressure. A saturated sodium bicarbonate solution (5 mL) was added, and extraction was performed with a mixed solvent (DCM:MeOH = 10:1) (2 × 2 mL). The organic phases were combined, dried over anhydrous sodium sulfate, and filtered. The filtrate was concentrated under reduced pressure, and the residue was purified by preparative thin-layer chromatography (DCM:7 mol/L solution of ammonia in methanol = 10:1) to give the product (67.8 mg, yield: 47.5%).

$^1$HNMR (400 MHz, DMSO-$d_6$) δ(ppm): 10.48 (s, 1H), 7.32-7.30 (m, 1H), 7.25-7.23 (m, 1H), 7.19 (s, 1H), 6.78-6.76 (m, 1H), 6.71 (s, 1H), 4.10-4.08 (m, 1H), 3.00-2.99 (m, 1H), 2.69 (s, 1H), 2.30 (s, 3H), 2.20 (s, 1H), 2.02 (m, 4H), 1.87-1.85 (m, 1H), 1.78-1.74 (m, 1H), 1.63-1.55 (m, 1H), 1.36-1.34 (m, 1H).

Molecular formula: $C_{20}H_{21}N_4F_3O$ Exact mass: 390.17 LC-MS (Pos, *m/z)* = 391.37 $[M+H]^+$.

**Example 47: Synthesis of (*R*)-2-(4-cyclopropyl-6-(piperidin-3-ylamino) pyridazin-3-yl)-5-ethynylphenol (Compound 149)**

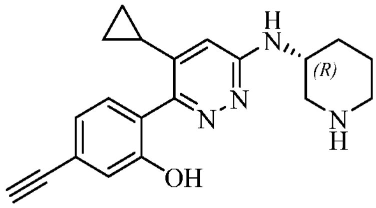

Step 1: Synthesis of *tert*-butyl (*R*)-3-((5-cyclopropyl-6-(2-(ethoxymethoxy)-4-formylphenyl)pyridazin-3-yl)amino)piperidi ne-1-carboxylate

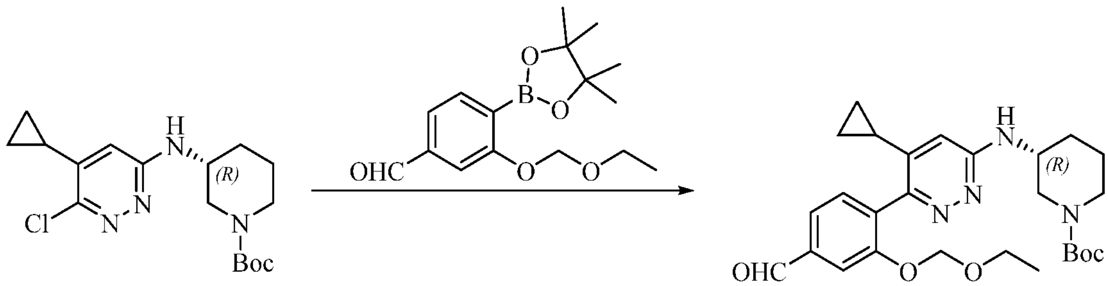

*tert*-Butyl (*R*)-3-((6-chloro-5-cyclopropylpyridazin-3-yl)amino)piperidine-1-carboxylate (1.5 g, 4.25 mmol, 1.0 eq), 3-(ethoxymethoxy)-4-(4,4,5,5-tetramethyl-1,3,2-dioxaborolan-2-yl)benzaldehyde (1.69 g, 5.52 mmol, 1.3 eq), anhydrous potassium carbonate (1.17 g, 8.50 mmol, 2.0 eq), and $Pd(PPh_3)_4$ (491.0 mg, 0.42 mmol, 0.1 eq) were added to a mixed solution of 1,4-dioxane (10.0 mL) and water (5.0 mL). The mixture was allowed to react at 100 °C for 4 h under a nitrogen atmosphere, and TLC monitoring showed the completion of the reaction. Ethyl acetate (100.0 mL) was added, and the mixture was washed with water (100.0 mL). The organic phase was dried over anhydrous sodium sulfate and filtered. The filtrate was concentrated under reduced pressure, and the crude product was purified by silica gel column chromatography (dichloromethane:methanol = 100:1 to 80:1) to give the product (2.0 g, yield: 94.7%).

Step 2: Synthesis of *tert*-butyl (*R*)-3-((5-cyclopropyl-6-(2-(ethoxymethoxy)-4-ethynylphenyl)pyridazin-3-yl)amino)piperid ine-1-carboxylate

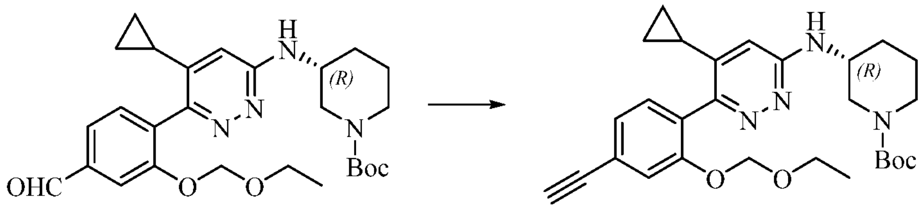

*tert*-Butyl (*R*)-3-((5-cyclopropyl-6-(2-(ethoxymethoxy)-4-formylphenyl) pyridazin-3-yl)amino)piperidine-1-carboxylate (2.0 g, 4.02 mmol, 1.0 eq), dimethyl (1-diazo-2-oxopropyl)phosphonate (1.16 g, 6.04 mmol, 1.5 eq), and anhydrous potassium carbonate (1.11 g, 8.04 mmol, 2.0 eq) were added to methanol (20.0 mL). The mixture was allowed to react at room temperature for 12 h, and TLC monitoring showed the completion of the reaction. The system was concentrated under reduced pressure, and the crude product was purified by silica gel column chromatography (dichloromethane:methanol = 100:1 to 80:1) to give the product (1.5 g, yield: 75.7%).

Step 3: Synthesis of (*R*)-2-(4-cyclopropyl-6-(piperidin-3-ylamino)pyridazin-3-yl)-5-ethynylphenol

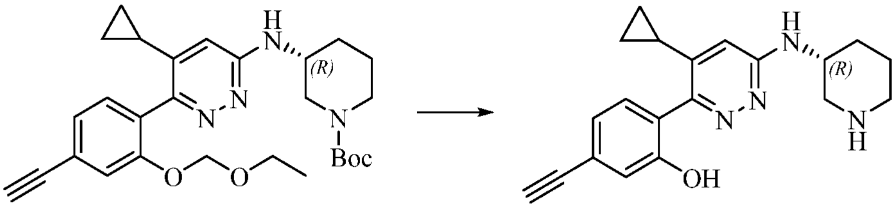

*tert*-Butyl (*R*)-3-((5-cyclopropyl-6-(2-(ethoxymethoxy)-4-ethynylphenyl) pyridazin-3-yl)amino)piperidine-1-carboxylate (1.5 g, 3.04 mmol, 1.0 eq) was added to dichloromethane (10.0 mL), and trifluoroacetic acid (5.0 mL) was added dropwise. The mixture was allowed to react at room temperature for 1 h, and TLC monitoring showed the completion of the reaction. The pH of the system was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by silica gel column chromatography (dichloromethane:methanol = 10:1 to 6:1) to give the product (550.0 mg, yield: 55.0%).

$^1$HNMR (400 MHz, DMSO-$d_6$) δ(ppm): 7.24-7.22 (d, *J*=8 Hz, 1H), 7.01-7.00 (m, 2H), 6.59-6.58 (d, *J*=4 Hz, 1H), 6.30 (s, 1H), 4.17 (s, 1H), 4.00-3.98 (m, 1H), 3.25-3.21 (m, 1H), 2.95-2.92 (m, 1H), 2.66-2.61 (m, 1H), 2.48 (s, 1H), 1.96-1.93 (m, 1H), 1.76-1.73 (m, 1H), 1.58-1.52 (m, 3H), 0.89-0.83 (m, 2H), 0.63-0.59 (m, 2H).

Molecular formula: $C_{20}H_{22}N_4O$ Exact molecular weight: 334.18 LC-MS (*m*/*z*) = 335.20 $[M+H]^+$.

**Example 48: Synthesis of (*R*)-2-(4-cyclopropyl-6-((1-(2-methoxyethyl) piperidin-3-yl)amino)pyridazin-3-yl)-5-ethynylphenol (Compound 240)**

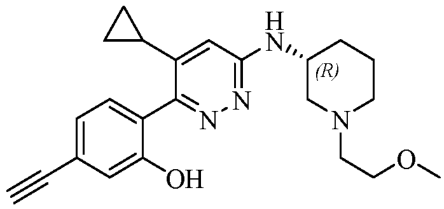

Step 1: Synthesis of (*R*)-6-chloro-5-cyclopropyl-*N*-(1-(2-methoxyethyl) piperidin-3-yl)pyridazin-3-amine

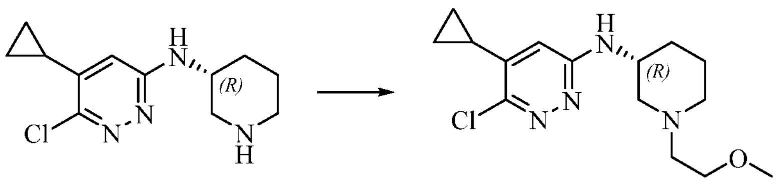

(*R*)-6-Chloro-5-cyclopropyl-*N*-(piperidin-3-yl)pyridazin-3-amine (400.0 mg, 1.58 mmol, 1.0 eq), 1-bromo-2-methoxyethane (1.1 g, 7.90 mmol, 5.0 eq), and triethylamine (1.6 g, 15.8 mmol, 10.0 eq) were added to dichloromethane (15.0 mL). The mixture was stirred at room temperature for 14 h, and TLC monitoring showed the completion of the reaction. Dichloromethane (100.0 mL) was added, and the resulting mixture was washed with a saturated aqueous ammonium chloride solution (50.0 mL). The organic phase was dried over anhydrous sodium sulfate and filtered. The filtrate was concentrated under reduced pressure, and the crude product was purified by silica gel column chromatography (dichloromethane:methanol = 80:1 to 30:1) to give the product (300.0 mg, yield: 61.0%).

Step 2: Synthesis of (*R*)-4-(4-cyclopropyl-6-((1-(2-methoxyethyl)piperidin-3-yl)amino)pyridazin-3-yl)-3-(ethox ymethoxy)benzaldehyde

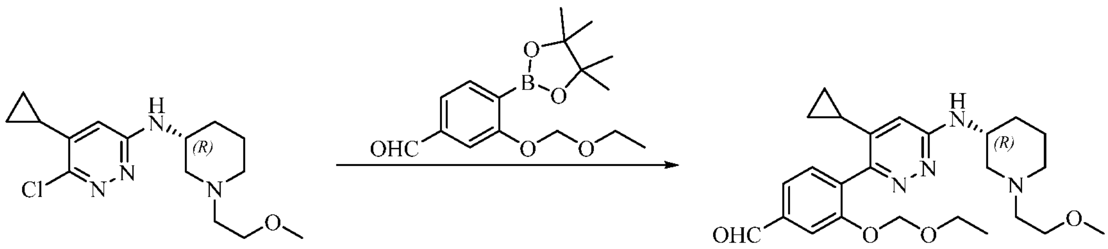

(*R*)-6-Chloro-5-cyclopropyl-*N*-(1-(2-methoxyethyl)piperidin-3-yl)pyridazin-3-amine (300.0 mg, 0.96 mmol, 1.0 eq), 3-(ethoxymethoxy)-4-(4,4,5,5-tetramethyl-1,3,2-dioxaborolan-2-yl)benzaldehyde (354.5 mg, 1.15 mmol, 1.2 eq), anhydrous potassium carbonate (200.0 mg, 1.44 mmol, 1.5 eq), and $Pd(PPh_3)_4$ (111.5 mg, 0.096 mmol, 0.1 eq) were added to a mixed solution of 1,4-dioxane (10.0 mL) and water (5.0 mL). The mixture was allowed to react at 110 °C for 5 h under a nitrogen atmosphere, and TLC monitoring showed the completion of the reaction. Ethyl acetate (100.0 mL) was added, and the mixture was washed with water (100.0 mL). The organic phase was dried over anhydrous sodium sulfate and filtered. The filtrate was concentrated under reduced pressure, and the crude product was purified by silica gel column chromatography (dichloromethane:methanol = 80:1 to 30:1) to give the product (300.0 mg, yield: 68.3%).

Step 3: Synthesis of (R)-5-cyclopropyl-6-(2-(ethoxymethoxy)-4-ethynylphenyl)-N-(1-(2-methoxyethyl)piperidin -3-yl) pyridazin-3-amine

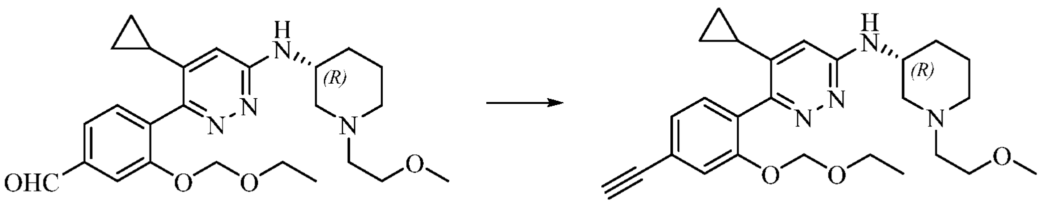

(R)-4-(4-cyclopropyl-6-((1-(2-methoxyethyl)piperidin-3-yl)amino)pyridazin-3 -yl)-3-(ethoxymethoxy)benzaldehyde (280.0 mg, 0.61 mmol, 1.0 eq), dimethyl (1-diazo-2-oxopropyl)phosphonate (177.5 mg, 0.92 mmol, 1.5 eq), and anhydrous potassium carbonate (170.2 mg, 1.23 mmol, 2.0 eq) were added to methanol (10.0 mL). The mixture was allowed to react at room temperature for 14 h, and LC-MS monitoring showed the completion of the reaction. The system was concentrated under reduced pressure, and the crude product was purified by silica gel column chromatography (dichloromethane:methanol = 80:1 to 30:1) to give the product (170.0 mg, yield: 61.2%).

Step 4: Synthesis of (*R*)-2-(4-cyclopropyl-6-((1-(2-methoxyethyl)piperidin-3-yl)amino)pyridazin-3-yl)-5-ethyny lphenol

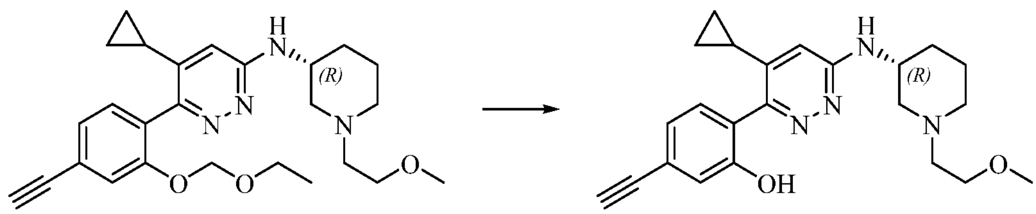

(*R*)-5-Cyclopropyl-6-(2-(ethoxymethoxy)-4-ethynylphenyl)-*N*-(1-(2-methoxye thyl)piperidin-3-yl)pyridazin-3-amine (140.0 mg, 0.31 mmol, 1.0 eq) was added to dichloromethane (3.0 mL), and trifluoroacetic acid (1.0 mL) was added dropwise. The mixture was allowed to react at room temperature for 1 h, and TLC monitoring showed the completion of the reaction. The pH of the system was adjusted to 8-9 with a saturated aqueous sodium carbonate solution, and extraction was performed with dichloromethane (100.0 mL). The organic phase was dried over anhydrous sodium sulfate and filtered. The filtrate was concentrated under reduced pressure, and the crude product was purified by preparative thin-layer chromatography (dichloromethane:methanol = 10:1) to give the product (40.0 mg, yield: 32.7%).

$^1$HNMR (400 MHz, DMSO-$d_6$) δ(ppm): 10.01 (s, 1H), 7.24-7.22 (d, J=8 Hz, 1H), 7.01-6.99 (m, 2H), 6.46 (s, 1H), 6.30 (s, 1H), 4.18 (s, 1H), 4.01 (s, 1H), 3.44 (s, 2H), 3.23 (s, 3H), 2.97 (s, 1H), 2.63 (s, 1H), 2.11-1.95 (m, 2H), 1.85-1.68 (m,

2H), 1.59-1.52 (m, 2H), 0.89-0.84 (m, 2H), 0.63-0.59 (m, 2H).

Molecular formula: $C_{23}H_{28}N_4O_2$ Exact molecular weight: 392.22 LC-MS *(m/z)* = 393.22 $[M+H]^+$.

Example 49: Synthesis of 2-(6-(((cis)-3-hydroxy-3-methylcyclobutyl) amino)-4-methylpyridazin-3-yl)-5-(3,3,3-trifluoroprop-1-yn-1-yl)phenol (Compound 101)

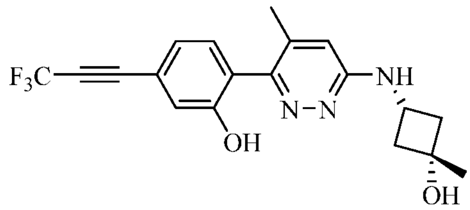

Step 1: Synthesis of 4-bromo-3-hydroxybenzaldehyde

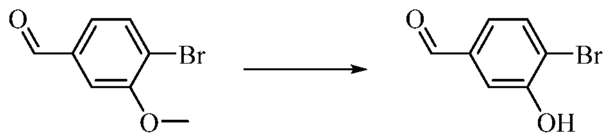

4-Bromo-3-methoxybenzaldehyde (10.00 g, 46.50 mmol, 1.0 eq) was added to an aqueous HBr solution (48%, 100 mL) in three portions. The mixture was allowed to react in a sealed tube at 100 °C for 20 h, and TLC monitoring showed the completion of the reaction. The reaction mixture was cooled to room temperature. Ethyl acetate (100 mL) and water (20 mL) were added, followed by liquid separation. The aqueous phase was extracted with ethyl acetate (50 mL × 2). The organic phases were combined, dried over anhydrous sodium sulfate, and filtered. The filtrate was concentrated under reduced pressure, and the crude product was purified by silica gel column chromatography (petroleum ether:ethyl acetate = 20:1 to 5:1) to give the product (6.10 g, yield: 65.3%).

Step 2: Synthesis of 4-bromo-3-(ethoxymethoxy)benzaldehyde

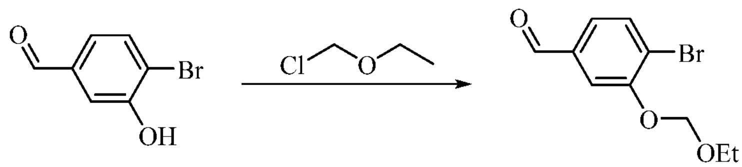

4-Bromo-3-hydroxybenzaldehyde (6.10 g, 30.35 mmol, 1.0 eq) was dissolved in THF (60 mL), and the solution was cooled to 0 °C. Then, NaH (60%, 2.43 g, 60.70 mmol, 2.0 eq) was added in portions, and the mixture was stirred for 30 min. Chloromethoxyethane (4.30 mg, 45.52 mmol, 1.5 eq) was then added dropwise, and the resulting mixture was allowed to react at room temperature for 2 h. TLC monitoring showed the completion of the reaction. The reaction mixture was quenched by adding a saturated aqueous $NH_4Cl$ solution (10 mL) and extracted with ethyl acetate (30 mL × 2). The organic phases were combined, dried over anhydrous sodium sulfate, and filtered. The filtrate was concentrated under reduced pressure, and the crude product was purified by silica gel column chromatography (petroleum ether:ethyl acetate =100:1 to 10:1) to give the product (7.61 g, yield: 96.8%).

Step 3: Synthesis of 3-(ethoxymethoxy)-4-(4,4,5,5-tetramethyl-1,3,2-dioxaborolan-2-yl)benzaldehyde

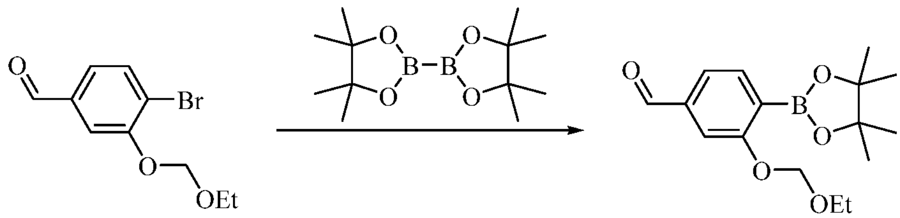

4-Bromo-3-(ethoxymethoxy)benzaldehyde (3.80 g, 14.67 mmol, 1.0 eq), bis(pinacolato)diboron (5.59 g, 22.00 mmol, 1.5 eq), Pd(dppf)$Cl_2$ (0.54 g, 0.73 mmol, 0.05 eq), and potassium acetate (2.88 g, 29.33 mmol, 2.0 eq) were added to 1,4-dioxane (60 mL), and the mixture was heated to 100 °C and allowed to react for 12 h under a nitrogen atmosphere. TLC monitoring showed the completion of the reaction. The reaction mixture was cooled to room temperature and filtered through celite under vacuum. Ethyl acetate (40 mL) was added, and the mixture was washed with a saturated aqueous NaCl solution (20 mL × 3), dried over anhydrous sodium sulfate, and filtered. The filtrate was concentrated under reduced pressure, and the crude product was purified by silica gel column chromatography (petroleum ether:ethyl acetate = 20:1 to 5:1) to give the product (3.53 g, yield: 78.6%).

Step 4: Synthesis of (*cis*)-3-((6-chloro-5-methylpyridazin-3-yl)amino)-1-methylcyclobutan-1-ol

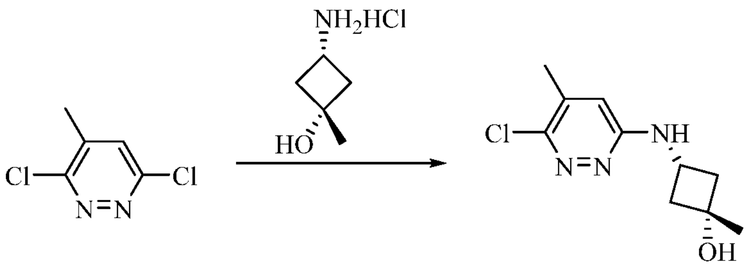

3,6-Dichloro-4-methylpyridazine (3.00 g, 18.40 mmol, 1.0 eq) was dissolved in n-butanol (30 mL), and (*cis*)-3-amino-1-methylcyclobutan-1-ol hydrochloride (5.06 g, 36.81 mmol, 2.0 eq) and DIPEA (9.51 g, 73.62 mmol, 4.0 eq) were added. The mixture was allowed to react at 120 °C for 2 h. TLC monitoring showed the completion of the reaction. The reaction mixture was concentrated under reduced pressure, and the crude product was purified by silica gel column chromatography (petroleum ether:ethyl acetate = 3:1 to 2:1) to give the product (0.99 g, yield: 23.6%).

Step 5: Synthesis of 3-(ethoxymethoxy)-4-(6-(((cis)-3-hydroxy-3-methylcyclobutyl) amino)-4-methylpyridazin-3-yl)benzaldehyde

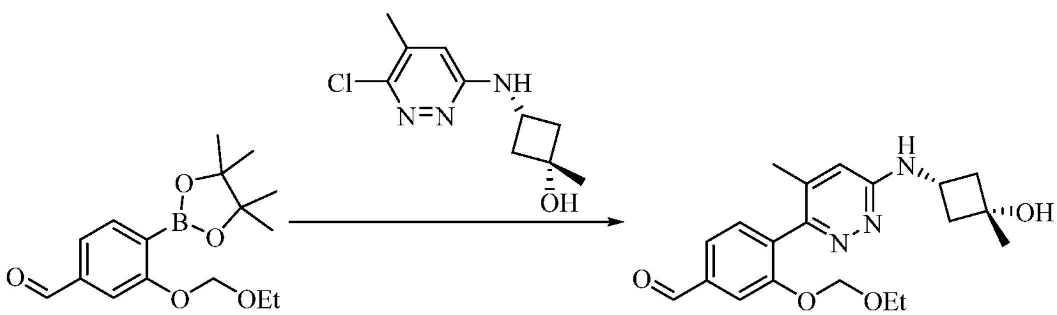

3-(Ethoxymethoxy)-4-(4,4,5,5-tetramethyl-1,3,2-dioxaborolan-2-yl)benzaldeh yde (1.58 g, 5.16 mmol, 1.2 eq), (*cis*)-3-((6-chloro-5-methylpyridazin-3-yl)amino)-1-methylcyclobutan-1-ol (0.98 g, 4.30 mmol, 1.0 eq), Pd(dppf)$Cl_2$ (0.31 g, 0.43 mmol, 0.1 eq), and sodium bicarbonate (0.72 g, 8.60 mmol, 2.0 eq) were added to a mixed solvent of 1,4-dioxane (22 mL) and water (5.5 mL). The mixture was heated to 110 °C and allowed to react for 2 h under a nitrogen atmosphere. TLC monitoring showed the completion of the reaction. The reaction mixture was cooled to room temperature. Water (25 mL) was added, and extraction was performed with ethyl acetate (25 mL × 3). The organic phases were combined, dried

over anhydrous sodium sulfate, and filtered. The filtrate was concentrated under reduced pressure, and the crude product was purified by silica gel column chromatography (dichloromethane:methanol = 100:1 to 10:1) to give the product (1.13 g, yield: 70.7%).

Step 6: Synthesis of (cis)-3-((6-(2-(ethoxymethoxy)-4-ethynylphenyl)-5-methylpyridazin-3-yl)amino)-1-methyl cyclobutan-1-ol

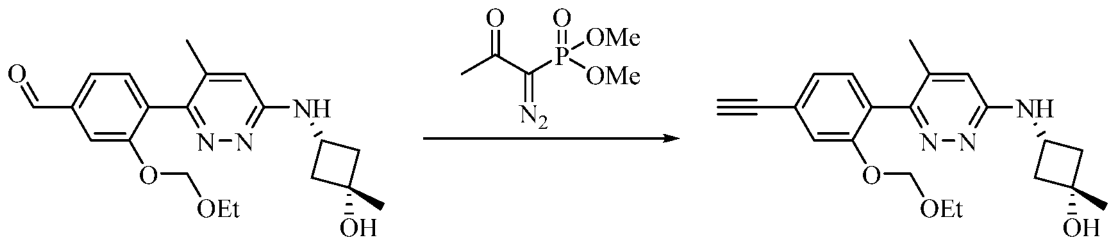

3-(Ethoxymethoxy)-4-(6-(((cis)-3-hydroxy-3-methylcyclobutyl)amino)-4-met hylpyridazin-3-yl)benzaldehyde (1.13 g, 3.04 mmol, 1.0 eq) and $K_2CO_3$ (0.84 g, 6.08 mmol, 2.0 eq) were added to methanol (15 mL), and dimethyl (1-diazo-2-oxopropyl)phosphonate (0.88 g, 4.56 mmol, 1.5 eq) was added with stirring. The mixture was allowed to react at room temperature for 2 h. TLC monitoring showed the completion of the reaction. The reaction solution was concentrated. Ethyl acetate (25 mL) was added, and the mixture was washed with water (15 mL × 2), followed by liquid separation. The organic phase was dried over anhydrous sodium sulfate and filtered. The filtrate was concentrated under reduced pressure, and the crude product was purified by silica gel column chromatography (dichloromethane:methanol = 100:1 to 10:1) to give the product (1.07 g, yield: 95.7%).

Step 7: Synthesis of (cis)-3-((6-(2-(ethoxymethoxy)-4-(3,3,3-trifluoroprop-1-yn-1-yl)phenyl)-5-methylpyridazin -3-yl) amino)-1-methylcyclobutan-1-ol

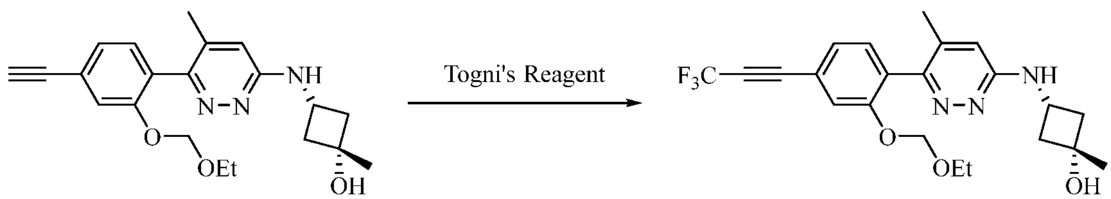


Togni's reagent (377.3 mg, 1.14 mmol, 2.0 eq), CuI (65.3 mg, 0.34 mmol, 0.6 eq), 1,10-phenanthroline (61.8 mg, 0.34 mmol, 0.6 eq), and $KHCO_3$ (114.4 mg, 1.14 mmol, 2.0 eq) were added to dichloromethane (5 mL). Then, (cis)-3-((6-(2-(ethoxymethoxy)-4-ethynylphenyl)-5-methylpyridazin-3-yl)amino)-1-methyl cyclobutan-1-ol (210.0 mg, 0.57 mmol, 1.0 eq) was dissolved in dichloromethane (5 mL) under a nitrogen atmosphere, and the solution was injected evenly and slowly into the above reaction system over 6 h. After the injection, the mixture was stirred at room temperature overnight. TLC showed the completion of the reaction. Water (10 mL) was added, followed by liquid separation. The aqueous phase was then extracted with dichloromethane (5 mL × 2). The organic phases were combined, dried over anhydrous sodium sulfate, and filtered. The filtrate was concentrated under reduced pressure, and the residue was purified by preparative thin-layer chromatography (dichloromethane:methanol = 10:1) to give the product (131.5 mg, yield: 52.8%).

Step 8: Synthesis of 2-(6-(((*cis*)-3-hydroxy-3-methylcyclobutyl)amino)-4-methylpyridazin-3-yl)-5-(3,3,3-trifluo roprop-1-yn-1-yl)phenol

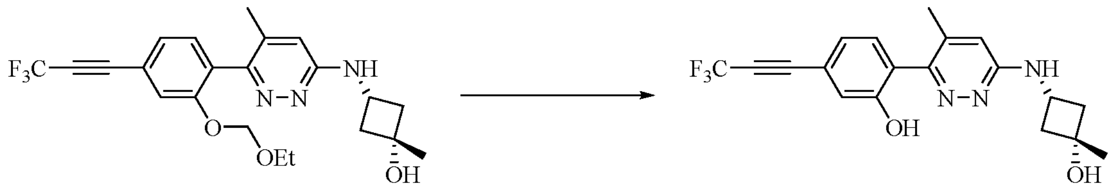

(*cis*)-3-((6-(2-(Ethoxymethoxy)-4-(3,3,3-trifluoroprop-1-yn-1-yl)phenyl)-5-m ethylpyridazin-3-yl)amino)-1-methylcyclobutan-1-ol (131.5 mg, 0.30 mmol) was dissolved in dichloromethane (1 mL), and a 4 mol/L solution of hydrogen chloride in 1,4-dioxane (1 mL) was added dropwise. The mixture was allowed to react at room temperature for 5 min. TLC monitoring showed the completion of the reaction. The reaction mixture was quenched by adding water (1 mL), and the pH was adjusted to 8 with a saturated aqueous $NaHCO_3$ solution, followed by extraction with dichloromethane (2 mL × 5). The organic phases were combined, dried over anhydrous sodium sulfate, and filtered. The filtrate was concentrated under reduced pressure, and the residue was purified by preparative thin-layer chromatography (dichloromethane:methanol = 10:1) to give the product (68.7 mg, yield: 60.3%).

$^1$HNMR (400 MHz, DMSO-$d_6$) δ(ppm): 10.45 (s, 1H), 7.31 (d, *J* = 8 Hz, 1H), 7.25-7.23 (m, 1H), 7.17 (d, *J* = 0.8 Hz, 1H), 7.06 (d, *J* = 6.8 Hz, 1H), 6.64 (s, 1H), 5.01 (s, 1H), 3.94-3.88 (m, 1H), 2.44-2.39 (m, 2H), 2.03 (s, 3H), 1.98-1.93 (m, 2H), 1.34-1.24 (m, 3H).

Molecular formula: $C_{19}H_{18}F_3N_3O_2$ Exact molecular weight: 377.14 LC-MS *(m/z)* = 378.30 $[M+H]^+$.

**Example 50: Synthesis of (R)-5-ethynyl-2-(4-methyl-6-(piperidin-3-ylamino)pyridazin-3-yl)phenol (Compound 139)**

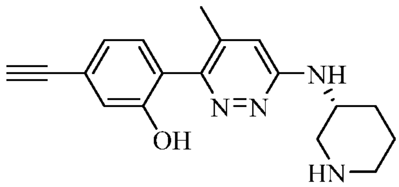

Step 1: Synthesis of *tert-butyl* (*R*)-3-((6-(2-(ethoxymethoxy)-4-formylphenyl)-5-methylpyridazin-3-yl)amino)piperidine-1 -carboxylate

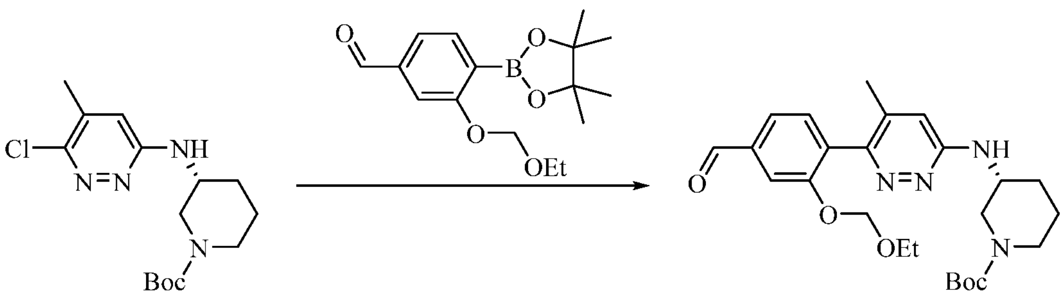

*tert-Butyl* (*R*)-3-((6-chloro-5-methylpyridazin-3-yl)amino)piperidine-1-carboxylate (2.20 g, 6.73 mmol, 1.0 eq), 3-(ethoxymethoxy)-4-(4,4,5,5-tetramethyl-1,3,2-dioxaborolan-2-yl)benzaldehyde (2.47 g, 8.08 mmol, 1.2 eq), Pd(dppf)$Cl_2$ (0.49 g, 0.67 mmol, 0.1 eq), and sodium bicarbonate (1.13 g, 13.46 mmol, 2.0 eq) were added to a mixed solvent of 1,4-dioxane (40 mL) and water (10 mL), and the mixture was heated to 110 °C and allowed to react for 2 h under a nitrogen atmosphere. TLC monitoring showed the completion of the reaction. The reaction mixture was cooled to room temperature. Water (50 mL) was added, and extraction was performed with ethyl acetate (50 mL × 3). The organic phases were combined, dried over anhydrous sodium sulfate, and filtered. The filtrate was concentrated under reduced pressure, and the crude product was purified by silica gel column chromatography (dichloromethane:methanol = 100:1 to 10:1) to give the product (2.68 g, yield: 84.6%).

Step 2: Synthesis of *tert-butyl* (R)-3-((6-(2-(ethoxymethoxy)-4-ethynylphenyl)-5-methylpyridazin-3-yl)amino)piperidine-1-carboxylate

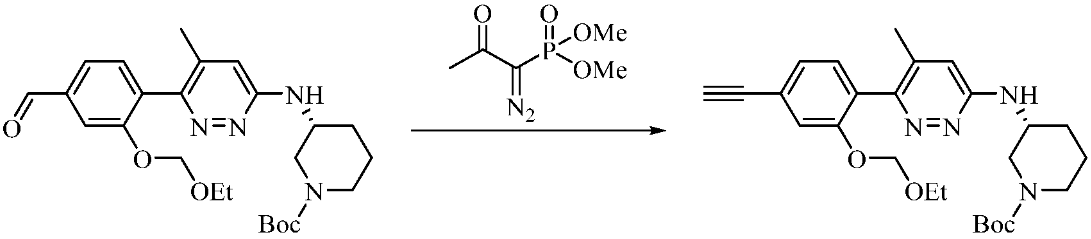

*tert-Butyl* (*R*)-3-((6-(2-(ethoxymethoxy)-4-formylphenyl)-5-methylpyridazin-3-yl)amino)piperidine-1 -carboxylate (2.68 g, 5.70 mmol, 1.0 eq) and $K_2CO_3$ (1.57 g, 11.39 mmol, 2.0 eq) were added to methanol (30 mL). The mixture was stirred, and dimethyl (1-diazo-2-oxopropyl)phosphonate (1.64 g, 8.54 mmol, 1.5 eq) was added. The resulting mixture was allowed to react at room temperature for 2 h. TLC monitoring showed the completion of the reaction. The reaction solution was concentrated. Ethyl acetate (50 mL) was added, and the mixture was washed with water (25 mL × 2), followed by liquid separation. The organic phase was dried over anhydrous sodium sulfate and filtered. The filtrate was concentrated under reduced pressure, and the crude product was purified by silica gel column chromatography (dichloromethane:methanol = 100:1 to 10:1) to give the product (2.32 g, yield: 87.3%).

Step 3: Synthesis of (R)-5-ethynyl-2-(4-methyl-6-(piperidin-3-ylamino) pyridazin-3-yl)phenol

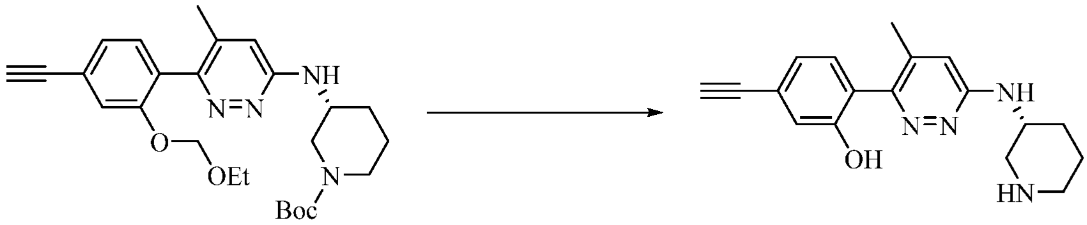

*tert-Butyl* (R)-3-((6-(2-(ethoxymethoxy)-4-ethynylphenyl)-5-methylpyridazin-3-yl)amino)piperidine-1-carboxylate (400.0 mg, 0.86 mmol, 1.0 eq) was dissolved in dichloromethane (4 mL), and a 4 mol/L solution of hydrogen chloride in 1,4-dioxane (2.0 mL, 8.14 mmol, 9.5 eq) was added dropwise. The mixture was allowed to react at room temperature for 2 h. TLC monitoring showed the completion of the reaction. The reaction mixture was quenched by adding water (2 mL), and the pH was adjusted to 8 with a saturated aqueous $NaHCO_3$ solution, followed by extraction with dichloromethane (5 mL × 5). The organic phases were combined, dried over anhydrous sodium sulfate, and filtered. The filtrate was concentrated under reduced pressure, and the residue was purified by preparative thin-layer chromatography (dichloromethane:methanol = 10:1) to give the product (169.8 mg, yield: 64.2%).

$^1$HNMR (400 MHz, DMSO-$d_6$) δ(ppm): 10.15 (s, 1H), 7.18-7.16 (m, 1H), 7.05-6.99 (m, 3H), 6.72 (s, 1H), 4.26-4.24 (m, 1H), 4.19 (s, 1H), 3.45-3.41 (m, 1H), 3.17-3.14 (m, 2H), 2.88 (t, *J* = 10 Hz, 1H), 2.78 (t, *J* = 10.6 Hz, 1H), 2.04 (s, 4H), 1.91-1.90 (m, 1H), 1.75-1.73 (m, 1H), 1.72-1.60 (m, 1H).

Molecular formula: $C_{18}H_{20}N_4O$ Exact molecular weight: 308.16 LC-MS *(m/z)* = 309.28 $[M+H]^+$.

**Example 51: (*R*)-2-(4-Difluoromethyl-6-((1-methylpiperidin-3-yl)amino)pyridazin-3-yl)-5-ethynylph enol (Compound 115)**

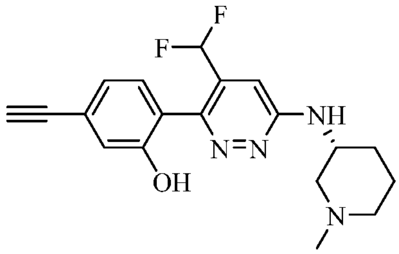

Step 1: Synthesis of *tert-butyl* (*R*)-3-((6-chloro-5-difluoromethylpyridazin-3-yl)amino)piperidine-1-carboxylate

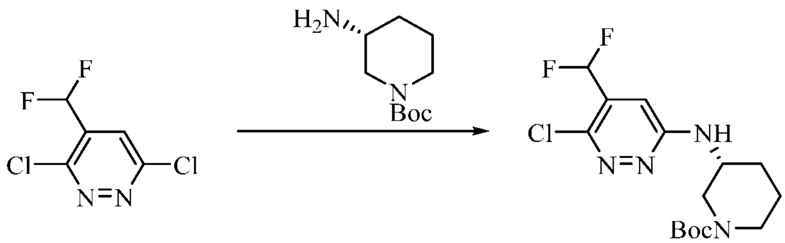

3,6-Dichloro-4-difluoromethylpyridazine (1.92 g, 9.65 mmol, 1.0 eq) was dissolved in *N,N*-dimethylacetamide (20 mL), and (*R*)-1-*tert*-butoxycarbonyl-3-aminopiperidine (3.87 g, 19.3 mmol, 2.0 eq) and *N*,*N*-diisopropylethylamine (2.49 g, 19.3 mmol, 2.0 eq) were added to the solution. The mixture was allowed to react at 120 °C for 1.5 h, and TLC analysis showed the substantial completion of the reaction. The reaction solution was poured into water (30 mL) and extracted with ethyl acetate (30 mL × 3). The organic phases were combined, dried over anhydrous sodium sulfate, and concentrated, and the crude product was purified by silica gel column chromatography (petroleum ether:ethyl acetate = 100:1 to 15:1) to give the product (1.38 g, yield: 39.4%).

Step 2: Synthesis of intermediate (R)-6-chloro-5-difluoromethyl-N-(piperidin-3-yl)pyridazin-3-amine

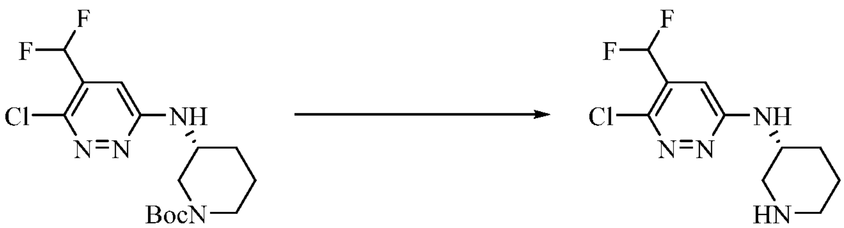

*tert-Butyl* (*R*)-3-((6-chloro-5-difluoromethylpyridazin-3-yl)amino)piperidine-1-carboxylate (1.38 g, 3.8 mmol, 1.0 eq) was dissolved in dichloromethane (10 mL), and the solution was added dropwise to trifluoroacetic acid (10 mL). The mixture was allowed to react at 25 °C for 5 min, and TLC analysis showed the completion of the reaction. The reaction solution was concentrated, and the concentrate was adjusted to weakly alkaline with a saturated aqueous sodium bicarbonate solution. The aqueous phase was extracted with a 5:1 mixture of dichloromethane and methanol (10 mL × 15). The organic phases were combined, dried over anhydrous magnesium sulfate, and concentrated to give a crude product, which was directly used in the next step.

Step 3: Synthesis of intermediate (*R*)-6-chloro-5-difluoromethyl-*N*-(1-methylpiperidin-3-yl)pyridazin-3-amine

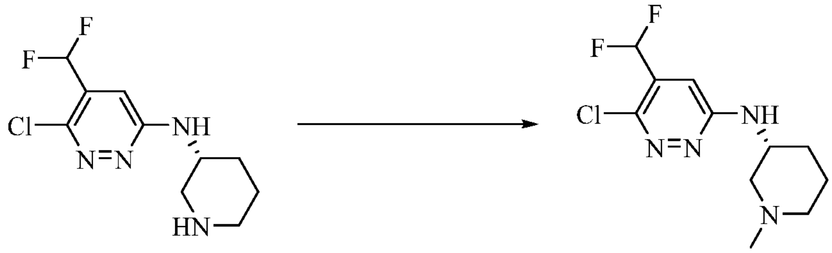

(*R*)-6-Chloro-5-difluoromethyl-*N*-(piperidin-3-yl)pyridazin-3-amine (crude product, 3.8 mmol, 1.0 eq) was dissolved in methanol (10 mL), and an aqueous formaldehyde solution (mass fraction: 37%) (308 mg, 3.8 mmol, 1.0 eq) was added to the solution. The mixture was allowed to react at 25 °C for 2 h, and sodium cyanoborohydride (263 mg, 4.18 mmol, 1.1 eq) was added. The resulting mixture was allowed to react at 25 °C for 5 min, and TLC analysis showed the completion of the reaction. The reaction solution was concentrated. A saturated aqueous sodium bicarbonate solution (20 mL) was added to the concentrate, and extraction was performed with dichloromethane (20 mL × 5). The organic phases were combined, dried over anhydrous magnesium sulfate, and concentrated, and the crude product was purified by silica gel column chromatography (dichloromethane:methanol = 100:1 to 10:1) to give the product (641 mg, two-step yield: 61.0%).

Step 4: Synthesis of intermediate (*R*)-4-(4-difluoromethyl-6-((1-methylpiperidin-3-yl)amino)pyridazin-3-yl)-3-ethoxymethox ybenzaldehyde

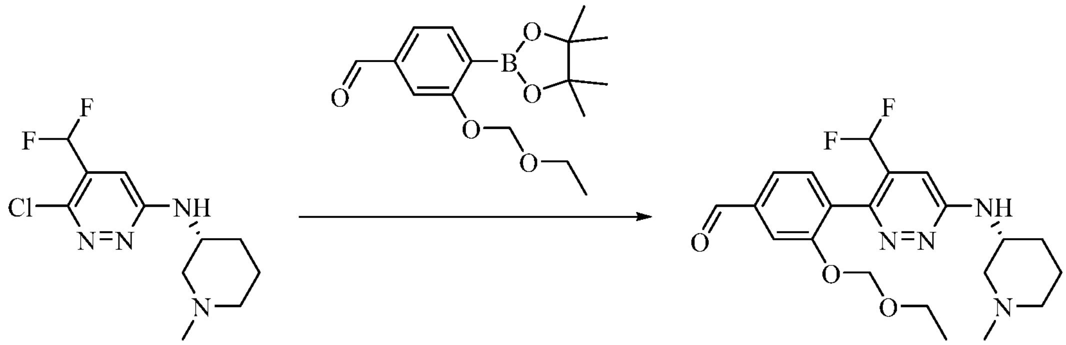

(*R*)-6-Chloro-5-difluoromethyl-*N*-(1-methylpiperidin-3-yl)pyridazin-3-amine (641 mg, 2.32 mmol, 1.0 eq) was dissolved in 1,4-dioxane (8 mL), and 3-ethoxymethoxy-5-methyl-4-(4,4,5,5-tetramethyl-1,3,2-dioxaborolan-2-yl)benzaldehyde (1.42 g, 4.64 mmol, 2.0 eq), an aqueous solution (2 mL) of potassium carbonate (641 mg, 4.64 mmol, 2.0 eq), and tetrakis(triphenylphosphine)palladium (268 mg, 0.232 mmol, 0.1 eq) were added to the solution. The mixture was heated to 90 °C and allowed to react for 2 h under a nitrogen atmosphere, and TLC analysis showed the completion of the reaction. Dichloromethane (10 mL) was added to the reaction solution, and the mixture was dried over anhydrous magnesium sulfate and filtered through celite. The filtrate was concentrated, and the crude product was purified by silica gel column chromatography (dichloromethane:methanol = 100:1 to 20:1) to give the product (707 mg, yield: 72.5%).

Step 5: Synthesis of intermediate (R)-5-difluoromethyl-6-(2-ethoxymethoxy-4-ethynylphenyl)-N-(1-methylpiperidin-3-yl)pyr idazin-3-amine

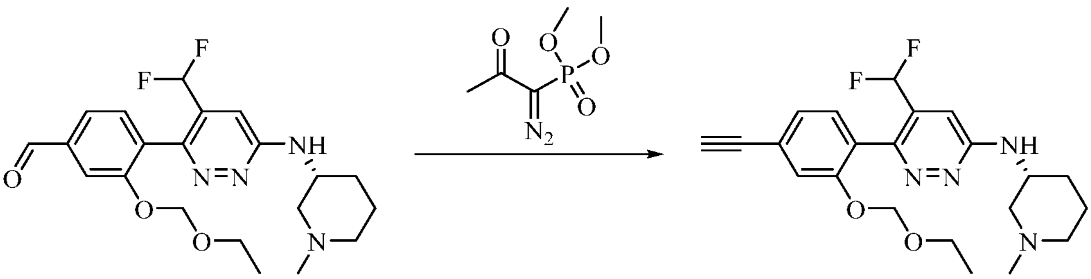

(*R*)-4-(4-Difluoromethyl-6-((1-methylpiperidin-3-yl)amino)pyridazin-3-yl)-3-ethoxymethoxybenzaldehyde (707 mg, 1.68 mmol, 1.0 eq) was dissolved in methanol (10 mL), and potassium carbonate (465 mg, 3.36 mmol, 2.0 eq) and dimethyl (1-diazo-2-oxopropyl)phosphonate (484 mg, 2.52 mmol, 1.5 eq) were added to the solution. The mixture was allowed to react at 25 °C for 1 h, and TLC analysis showed the completion of the reaction. The reaction solution was concentrated. Water (20 mL) was added to the concentrate, and extraction was performed with dichloromethane (20 mL × 3). The organic phases were combined, dried over anhydrous magnesium sulfate, and concentrated, and the crude product was purified by silica gel column chromatography (dichloromethane:methanol = 100:1 to 20:1) to give the product (403 mg, yield: 57.6%).

Step 6: Synthesis of compound (*R*)-2-(4-difluoromethyl-6-((1-methylpiperidin-3-yl)amino)pyridazin-3-yl)-5-ethynylphenol

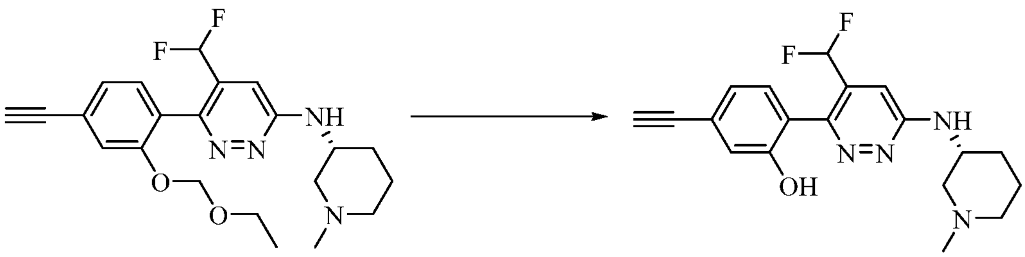

(*R*)-5-Difluoromethyl-6-(2-ethoxymethoxy-4-ethynylphenyl)-N-(1-methylpipe ridin-3-yl)pyridazin-3-amine (403 mg, 0.968 mmol) was dissolved in dichloromethane (4 mL), and the solution was added dropwise and slowly to trifluoroacetic acid (4 mL). The mixture was allowed to react at 25 °C for 5 min, and TLC analysis showed the completion of the reaction. The reaction solution was concentrated, and the crude product was purified by silica gel column chromatography (dichloromethane:methanol = 100:1 to 10:1) to give the product (150 mg, yield: 43.2%).

$^{1}$H-NMR (400 MHz, DMSO-$d_6$) δ(ppm): 10.26 (s, 1H), 7.27-7.25 (d, 1H), 7.21-7.19 (d, 1H), 7.11 (s, 1H), 7.04-7.03 (m, 2H), 6.90-6.03 (t, 1H), 4.22 (s, 1H), 4.18 (m, 1H), 2.97 (m, 1H), 2.68-2.65 (m, 1H), 2.30 (s, 3H), 2.15-2.12 (m, 2H), 1.87-1.76 (m, 2H), 1.62-1.34 (m, 2H).

Molecular formula: $C_{19}H_{20}F_2N_4O$ Exact molecular weight: 358.16 LC-MS (m/z): 359.18 $[M+H]^+$.

**Example 52: 5-Ethynyl-2-(6-(((*cis*)-3-hydroxy-3-methylcyclobutyl)amino)-4-trifluoromethylpyridazi n-3-yl)phenol (Compound 239)**

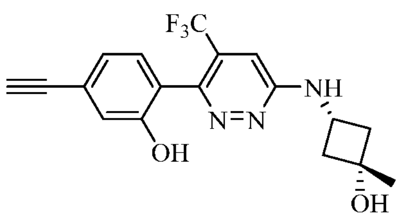

Step 1: Synthesis of intermediate (*cis*)-3-((6-chloro-5-trifluoromethylpyridazin-3-yl)amino)-1-methylcyclobutan-1-ol

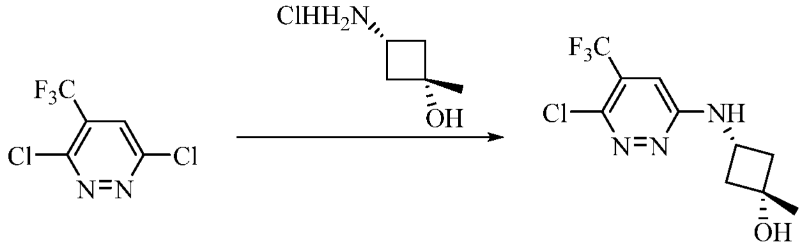

3,6-Dichloro-4-trifluoromethylpyridazine (1.5 g, 6.91 mmol, 1.0 eq) was dissolved in *n*-butanol (15 mL), and (*cis*)-3-amino-1-methylcyclobutan-1-ol hydrochloride (1.9 g, 13.82 mmol, 2.0 eq) and *N*,*N*-diisopropylethylamine (3.6 g, 27.64 mmol, 4.0 eq) were added to the solution. The mixture was allowed to react at 120 °C for 0.5 h, and TLC analysis showed the completion of the reaction. The reaction solution was concentrated, and the crude product was purified by silica gel column chromatography (petroleum ether:ethyl acetate = 10:1 to 1:1) to give the product (1.16 g, yield: 59.5%).

Step 2: Synthesis of intermediate 4-(4-trifluoromethyl-6-(((*cis*)-3-hydroxy-3-methylcyclobutyl)amino)pyridazin-3-yl)-3-(etho xymethoxy)benzaldehyde

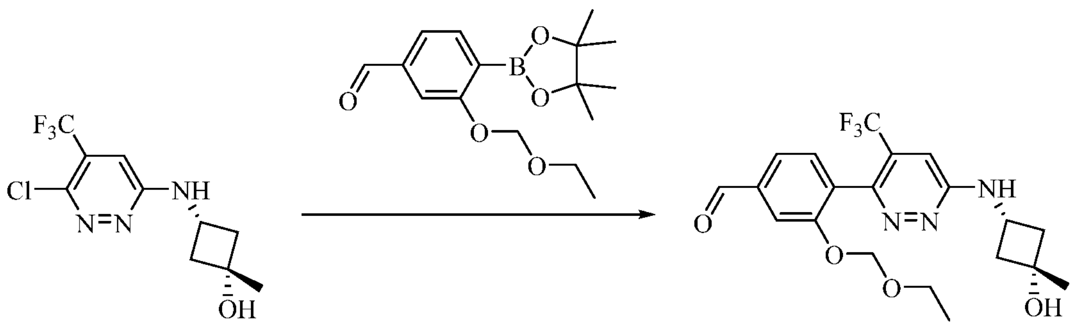

(*cis*)-3-((6-Chloro-5-trifluoromethylpyridazin-3-yl)amino)-1-methylcyclobuta n-1-ol (600 mg, 2.13 mmol, 1.0 eq) was dissolved in 1,4-dioxane (6 mL), and 3-(ethoxymethoxy)-4-(4,4,5,5-tetramethyl-1,3,2-dioxaborolan-2-yl)benzaldehyde (1.3 g, 4.26 mmol, 2.0 eq), potassium carbonate (589 mg, 4.26 mmol, 2.0 eq), tetrakis(triphenylphosphine)palladium (246 mg, 0.213 mmol, 0.1 eq), and water (1.5 mL) were added to the solution. The mixture was heated to 90 °C and allowed to react for 2 h under a nitrogen atmosphere, and TLC analysis showed the completion of the reaction. The reaction solution was filtered through celite, and the filter cake was washed with dichloromethane (10 mL). The filtrates were combined, dried over anhydrous magnesium sulfate, filtered, and concentrated, and the crude product was purified by silica gel column chromatography (petroleum ether:ethyl acetate = 10: 1 to 3:1) to give the product (700 mg, yield: 77.3%).

Step 3: Synthesis of intermediate (*cis*)-3-((5-trifluoromethyl-6-(2-ethoxymethoxy-4-ethynylphenyl)pyridazin-3-yl)amino)-1-methylcyclobutan-1-ol

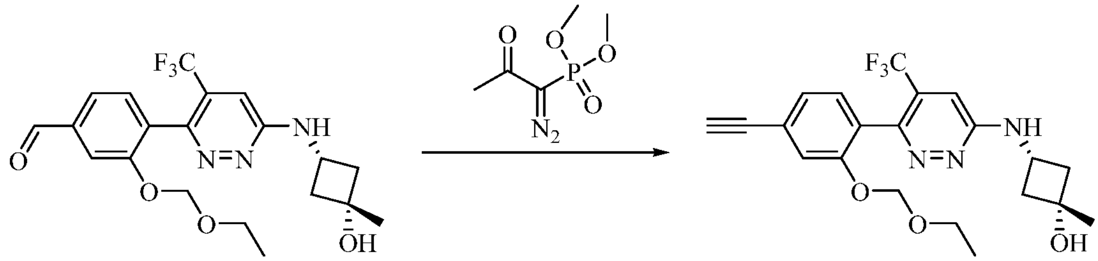

4-(4-Trifluoromethyl-6-(((*cis*)-3-hydroxy-3-methylcyclobutyl)amino) pyridazin-3-yl)-3-(ethoxymethoxy)benzaldehyde (700 mg, 1.645 mmol, 1.0 eq) was dissolved in methanol (10 mL), and potassium carbonate (455 mg, 3.290 mmol, 2.0 eq) and dimethyl (1-diazo-2-oxopropyl)phosphonate (474 mg, 2.468 mmol, 1.5 eq) were added to the solution. The mixture was allowed to react at 25 °C for 1 h, and TLC analysis showed the completion of the reaction. The reaction solution was concentrated, and the crude product was purified by silica gel column chromatography (dichloromethane:methanol = 100:1 to 50:1) to give the product (100 mg, yield: 14.4%).

Step 4: Synthesis of compound 5-ethynyl-2-(6-(((*cis*)-3-hydroxy-3-methylcyclobutyl)amino)-4-trifluoromethylpyridazin-3-yl)phenol

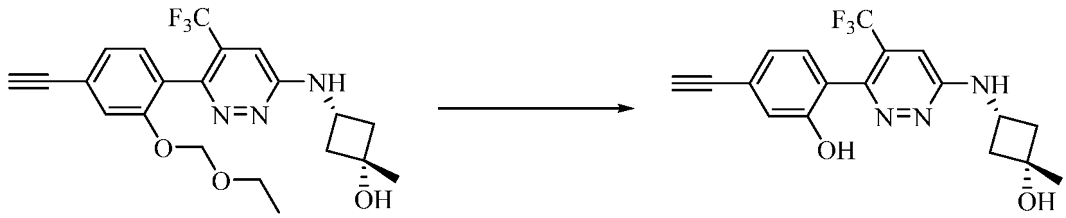

*(cis)-3-((5-Trifluoromethyl-6-(2-ethoxymethoxy-4-ethynylphenyl)pyridazin-3* -yl)amino)-1-methylcyclobutan-1-ol (100 mg, 0.237 mmol, 1.0 eq) was dissolved in dichloromethane (1 mL), and the solution was added dropwise to trifluoroacetic acid (1 mL). The mixture was allowed to react at 25 °C for 5 min, and TLC analysis showed the completion of the reaction. The reaction solution was concentrated, and the crude product was purified by preparative thin-layer chromatography (dichloromethane:methanol = 10:1) to give the product (38 mg, yield: 44.2%).

$^1$H-NMR (400 MHz, DMSO-$d_6$) δ(ppm): 9.83 (s, 1H), 7.64-7.62 (d, 1H), 7.14 (s, 1H), 7.13 (s, 1H), 6.99-6.97 (m, 2H), 5.06 (s, 1H), 4.20 (s, 1H), 4.06-3.98 (m, 1H), 2.48-2.43 (m, 2H), 2.01-1.96 (m, 2H), 1.30 (s, 3H).

Molecular formula: $C_{18}H_{16}F_3N_3O_2$ Exact molecular weight: 363.12 LC-MS (*m/z*): 364.15 $[M+H]^+$.

**Example 53: (*R*)-5-Ethynyl-2-(6-(piperidin-3-ylamino)-4-trifluoromethylpyridazin-3-yl)phenol (Compound 161)**

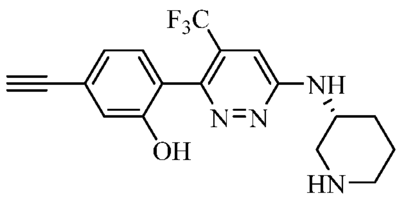

Step 1: Synthesis of compound *tert*-butyl (*R*)-3-((6-chloro-5-trifluoromethylpyridazin-3-yl)amino)piperidine-1-carboxylate

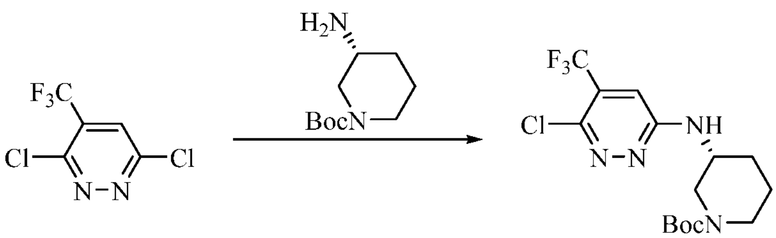

3,6-Dichloro-4-trifluoromethylpyridazine (3.0 g, 13.83 mmol, 1.0 eq) was dissolved in DMAc (30 mL), and (*R*)-1-*tert*-butoxycarbonyl-3-aminopiperidine (5.5 g, 27.66 mmol, 2.0 eq) and *N*,*N*-diisopropylethylamine (3.6 g, 27.66 mmol, 2.0 eq) were added to the solution. The mixture was allowed to react at 120 °C for 0.5 h, and TLC analysis showed the completion of the reaction. The reaction solution was poured into water (50 mL) and extracted with ethyl acetate (40 mL ×

4). The organic phases were combined, dried over anhydrous magnesium sulfate, and concentrated, and the crude product was purified by silica gel column chromatography (petroleum ether:ethyl acetate = 40:1 to 10:1) to give the product (3.53 g, yield: 67.0%).

Step 2: Synthesis of intermediate *tert-butyl* (*R*)-3-((5-trifluoromethyl-6-(2-ethoxymethoxy-4-formylphenyl)pyridazin-3-yl)amino)piperi dine-1-carboxylate

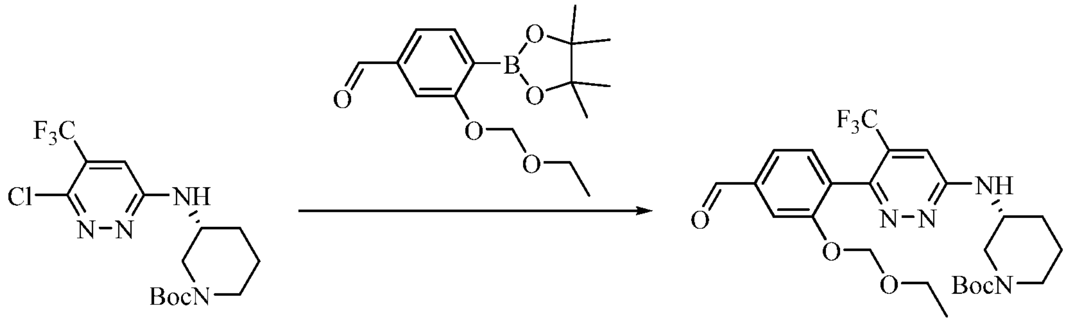

*tert*-Butyl (*R*)-3-((6-chloro-5-trifluoromethylpyridazin-3-yl)amino)piperidine-1-carboxylate (2.0 g, 5.25 mmol, 1.0 eq) was dissolved in 1,4-dioxane (20 mL), and 3-(ethoxymethoxy)-4-(4,4,5,5-tetramethyl-1,3,2-dioxaborolan-2-yl)benzaldehyde (3.21 g, 10.5 mmol, 2.0 eq), potassium carbonate (1.45 g, 10.5 mmol, 2.0 eq), tetrakis(triphenylphosphine) palladium (607 mg, 0.525 mmol, 0.1 eq), and water (5 mL) were added to the solution. The mixture was heated to 90 °C and allowed to react for 2 h under a nitrogen atmosphere, and TLC analysis showed the completion of the reaction. The reaction solution was filtered through celite. Water (20 mL) was added to the filtrate, and extraction was performed with dichloromethane (20 mL × 3). The organic phases were combined, dried over anhydrous magnesium sulfate, and concentrated, and the crude product was purified by silica gel column chromatography (petroleum ether:ethyl acetate = 10: 1 to 1:1) to give the product (2.25 g, yield: 81.8%).

Step 3: Synthesis of intermediate *tert-butyl* (R)-3-((5-trifluoromethyl-6-(2-ethoxymethoxy-4-ethynylphenyl)pyridazin-3-yl)amino)piper idine-1-carboxylate

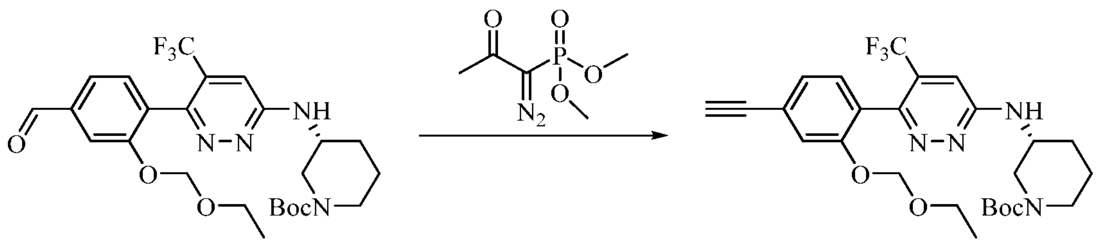

*tert*-Butyl (*R*)-3-((5-trifluoromethyl-6-(2-ethoxymethoxy-4-formylphenyl) pyridazin-3-yl)amino)piperidine-1-carboxylate (2.25 g, 4.29 mmol, 1.0 eq) was dissolved in methanol (25 mL), and potassium carbonate (1.19 g, 8.58 mmol, 2.0 eq) and dimethyl (1-diazo-2-oxopropyl)phosphonate (1.24 g, 6.44 mmol, 1.5 eq) were added to the solution. The mixture was allowed to react at 25 °C for 1 h, and TLC analysis showed the completion of the reaction. The reaction solution was concentrated, and the crude product was purified by silica gel column chromatography (petroleum ether:ethyl acetate = 8:1 to 2:1) to give the product (400 mg, yield: 17.9%).

Step 4: Synthesis of compound (*R*)-5-ethynyl-2-(6-(piperidin-3-ylamino)-4-trifluoromethylpyridazin-3-yl)phenol

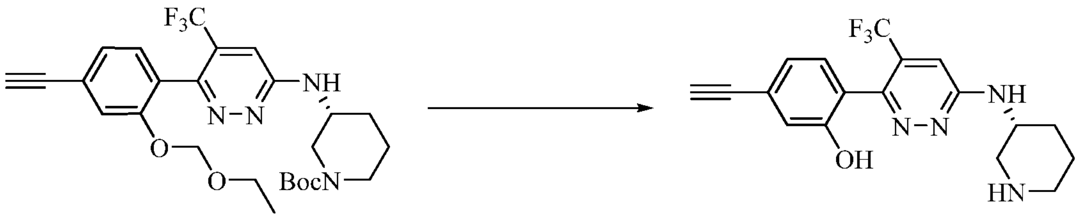

*tert*-Butyl (*R*)-3-((5-trifluoromethyl-6-(2-ethoxymethoxy-4-ethynylphenyl) pyridazin-3-yl)amino)piperidine-1-carboxylate (400 mg, 0.768 mmol, 1.0 eq) was dissolved in dichloromethane (4 mL), and the solution was added dropwise to trifluoroacetic acid (4 mL). The mixture was allowed to react at 25 °C for 5 min, and TLC analysis showed the completion of the reaction. The reaction solution was concentrated, and dichloromethane (10 mL) and water (10 mL) were added to the concentrate, followed by liquid separation. The pH of the aqueous phase was adjusted to weakly alkaline with sodium bicarbonate, and extraction was performed with a 5:1 mixture of dichloromethane and methanol (10 mL × 20). The organic phases were combined, dried over anhydrous magnesium sulfate, and concentrated to give a crude product. Half of the crude product was taken and purified by preparative thin-layer chromatography (dichloromethane:methanol = 7:1) to give the product (70 mg, yield: 50.4%).

$^1$H-NMR (400 MHz, DMSO-*d*$_6$) δ(ppm): 7.37-7.35 (d, 1H), 7.22 (s, 1H), 7.14-7.12 (m, 1H), 6.99-6.97 (m, 2H), 4.17 (s, 1H), 4.07 (s, 1H), 3.24-3.21 (m, 1H), 2.93-2.90 (m, 1H), 2.66-2.53 (m, 2H), 2.00-1.98 (m, 1H), 1.77-1.74 (m, 1H), 1.57-1.50 (m, 2H) .

Molecular formula: $C_{18}H_{17}F_3N_4O$ Exact molecular weight: 362.14 LC-MS (*m*/*z*): 363.15 [M+H]$^+$.

**Example 54: (*R*)-5-Ethynyl-2-(4-trifluoromethyl-6-((1-methylpiperidin-3-yl)amino)pyridazin-3-yl)p henol (Compound 114)**

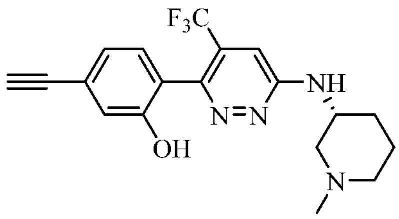

Step 1: Synthesis of (*R*)-5-ethynyl-2-(4-trifluoromethyl-6-((1-methylpiperidin-3-yl)amino)pyridazin-3-yl)phenol

(*R*)-5-Ethynyl-2-(6-(piperidin-3-yl)amino)-4-trifluoromethylpyridazin-3-yl)ph enol (crude product, 0.384 mmol, 1.0 eq) was dissolved in methanol (2 mL), and an aqueous formaldehyde solution (mass fraction: 37%) (32 mg, 0.384 mmol, 1.0 eq) and sodium cyanoborohydride (27 mg, 0.422 mmol, 1.1 eq) were added to the solution. The mixture was allowed to react at 25 °C for 5 min, and TLC analysis showed the completion of the reaction. The reaction solution was concentrated. Saturated brine (5 mL) was added to the reaction solution, and extraction was performed with a mixed solvent (dichloromethane:methanol = 10:1) (10 mL × 6). The organic phases were combined, dried over anhydrous magnesium sulfate, and filtered. The filtrate was concentrated to give a crude product, which was then purified by preparative thin-layer chromatography (dichloromethane:methanol = 10:1) to give the product (60 mg, yield: 41.7%).

$^1$H-NMR (400 MHz, DMSO-*d*$_6$) δ(ppm): 9.93 (s, 1H), 7.32-7.30 (d, 1H), 7.25 (s, 1H), 7.14-7.12 (d, 1H), 6.99-6.67 (s, 2H), 4.17 (s, 1H), 4.15-4.10 (m, 1H), 2.88-2.86 (m, 1H), 2.55 (m, 1H), 2.23 (s, 3H), 2.17 (m, 1H), 2.09 (m, 1H), 1.83 (m, 1H) , 1.76-1.73 (m, 1H), 1.61-1.53 (m, 1H), 1.39-1.37 (m, 1H).

Molecular formula: $C_{19}H_{19}F_3N_4O$ Exact molecular weight: 376.15 LC-MS (*m*/*z*): 377.17 [M+H]$^+$.

**Example** 55: **(*R*)-5-Ethynyl-2-(6-((1-(2-hydroxyethylpiperidin)-3-yl)amino)-4-trifluoromethylpyrida zin-3-yl)phenol (Compound 163)**

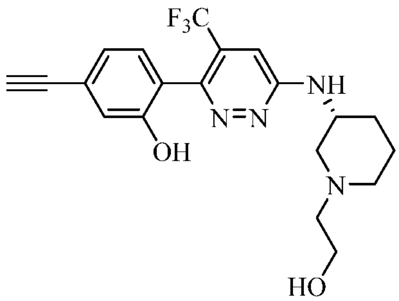

Step 1: Synthesis of (*R*)-6-chloro-*N*-(piperidin-3-yl)-5-trifluoromethylpyridazin-3-amine

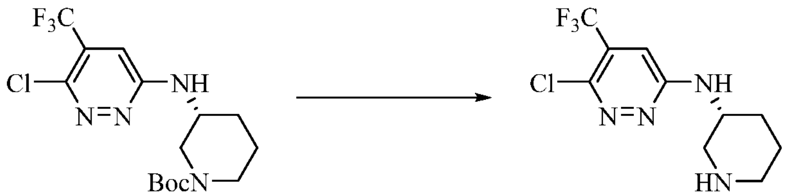

*tert*-Butyl (*R*)-3-((6-chloro-5-trifluoromethylpyridazin-3-yl)amino)piperidine-1-carboxylate (860 mg, 2.26 mmol, 1.0 eq) was dissolved in dichloromethane (8 mL), and the solution was added dropwise to trifluoroacetic acid (8 mL). The mixture was allowed to react at 25 °C for 5 min, and TLC analysis showed the completion of the reaction. The reaction solution was concentrated, and the concentrate was adjusted to weakly alkaline with a saturated aqueous sodium bicarbonate solution. The aqueous phase was extracted with a 5:1 mixture of dichloromethane and methanol (10 mL × 15). The organic phases were combined, dried over anhydrous magnesium sulfate, and concentrated to give a crude product, which was directly used in the next step.

Step 2: Synthesis of intermediate (*R*)-2-(3-((6-chloro-5-trifluoromethylpyridazin-3-yl)amino)piperidin-1-yl)ethan-1-ol

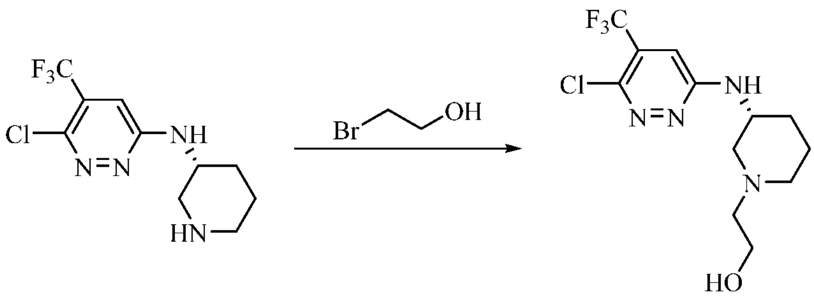

(*R*)-6-Chloro-*N*-(piperidin-3-yl)-5-trifluoromethylpyridazin-3-amine (crude product, 2.26 mmol, 1.0 eq) was dissolved in dichloromethane (10 mL), and triethylamine (2.29 g, 22.6 mmol, 10.0 eq) and bromoethanol (1.41 g, 11.3 mmol, 5.0 eq) were added to the solution. The mixture was allowed to react at 25 °C for 24 h, and TLC analysis showed the completion of the reaction. The reaction solution was poured into a saturated aqueous ammonium chloride solution (10 mL) and extracted with dichloromethane (10 mL × 4). The organic phases were combined, dried over anhydrous magnesium sulfate, and concentrated, and the crude product was purified by silica gel column chromatography (dichloromethane:methanol = 100:1 to 10:1) to give the product (730 mg, two-step yield: 99.5%).

Step 3: Synthesis of intermediate (*R*)-3-ethoxymethoxy-4-(6-((1-(2-hydroxyethyl)piperidin-3-yl)amino)-4-trifluoromethylpyr idazin-3-yl)benzaldehyde

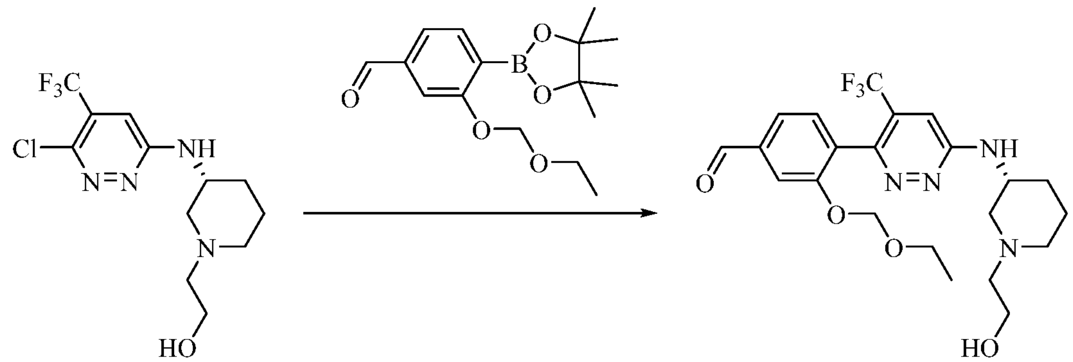

(*R*)-2-(3-((6-Chloro-5-trifluoromethylpyridazin-3-yl)amino)piperidin-1-yl)eth an-1-ol (500 mg, 1.54 mmol, 1.0 eq) was dissolved in 1,4-dioxane (5 mL), and 3-ethoxymethoxy-5-methyl-4-(4,4,5,5-tetramethyl-1,3,2-dioxaborolan-2-yl) benzaldehyde (566 mg, 1.85 mmol, 1.2 eq), an aqueous solution (1 mL) of potassium carbonate (426 mg, 3.08 mmol, 2.0 eq), and tetrakis(triphenylphosphine)palladium (178 mg, 0.154 mmol, 0.1 eq) were added to the solution. The mixture was heated to 90 °C and allowed to react for 5 h under a nitrogen atmosphere, and TLC analysis showed the completion of the reaction. The reaction solution was filtered through celite and concentrated, and the crude product was purified by silica gel column chromatography (dichloromethane:methanol = 100:1 to 50:1) to give the product (280 mg, yield: 38.8%).

Step 4: Synthesis of intermediate (*R*)-2-(3-((6-(2-ethoxymethoxy-4-ethynylphenyl)-5-trifluoromethylpyridazin-3-yl)amino)pi peridin-1-yl)ethan-1-ol

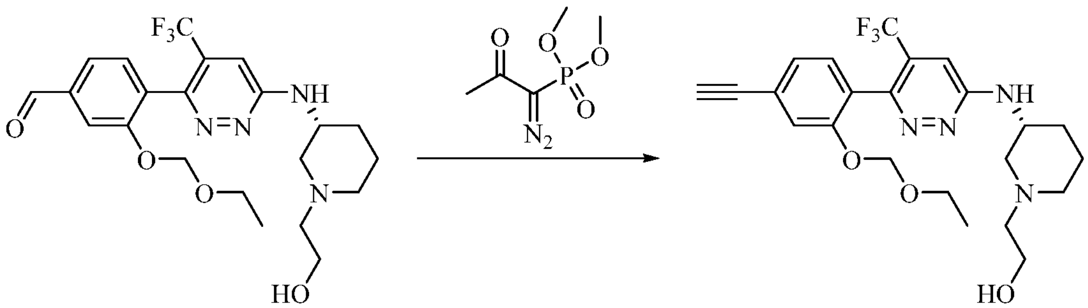

(*R*)-3-Ethoxymethoxy-4-(6-((1-(2-hydroxyethyl)piperidin-3-yl)amino)-4-triflu oromethylpyridazin-3-yl)benzaldehyde (140 mg, 0.3 mmol, 1.0 eq) was dissolved in methanol (10 mL), and potassium carbonate (83 mg, 0.6 mmol, 2.0 eq) and dimethyl (1-diazo-2-oxopropyl)phosphonate (87 mg, 0.45 mmol, 1.5 eq) were added to the solution. The mixture was allowed to react at 25 °C for 1.5 h, and TLC analysis showed the completion of the reaction. The reaction solution was concentrated. Water (10 mL) was added, and extraction was performed with dichloromethane (10 mL × 3). The organic phases were combined, dried over anhydrous magnesium sulfate, and concentrated, and the crude product was purified by silica gel column chromatography (dichloromethane:methanol = 10:1) to give the product (43 mg, yield: 30.9%).

Step 5: Synthesis of compound (*R*)-5-ethynyl-2-(6-((1-(2-hydroxyethyl) piperidin-3-yl)amino)-4-trifluoromethylpyridazin-3-yl)phenol

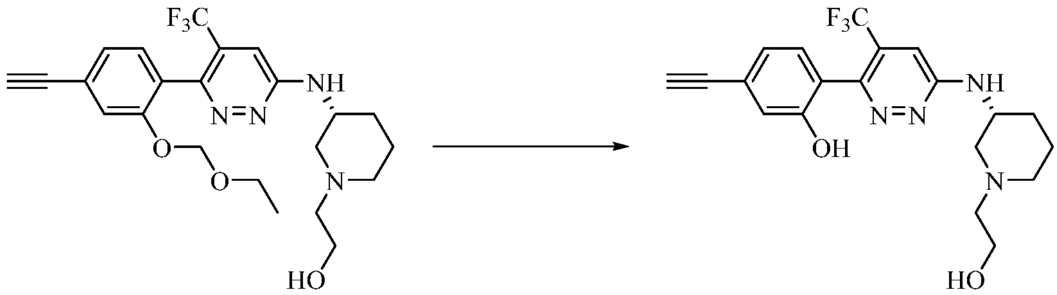

($R$)-2-(3-((6-(2-Ethoxymethoxy-4-ethynylphenyl)-5-trifluoromethylpyridazin-3-yl)amino)piperidin-1-yl)ethan-1-ol (43 mg, 0.918 mmol) was dissolved in dichloromethane (1 mL), and the solution was added dropwise and slowly to trifluoroacetic acid (1 mL). The mixture was allowed to react at 25 °C for 1 h, and TLC analysis showed the completion of the reaction. The reaction solution was concentrated, and the crude product was purified by preparative thin-layer chromatography (dichloromethane:methanol = 10:1) to give the product (20 mg, yield: 54.1%).

$^1$H-NMR (400 MHz, DMSO-$d_6$) δ(ppm): 9.86 (s, 1H), 7.32 (s, 1H), 7.25 (s, 1H), 7.15-7.13 (m, 1H), 6.98-6.97 (m, 2H), 4.40 (s, 1H), 4.21 (s, 2H), 3.55 (m, 2H), 3.12-2.93 (m, 1H), 2.81-2.67 (m, 1H), 2.61-2.53 (m, 2H), 2.48-2.17 (m, 2H), 1.92-1.76 (m, 2H), 1.59-1.46 (m, 2H).

Molecular formula: $C_{20}H_{21}F_3N_4O_2$ Exact molecular weight: 406.16 LC-MS (m/z): 407.17 $[M+H]^+$.

**Example 56: (*R*)-3-(4-Ethynyl-2-hydroxyphenyl)-4-isopropyl-6-((1-methylpiperidin-3-yl)amino)-1,2, 4-triazin-5(4*H*)-one (Compound 112)**

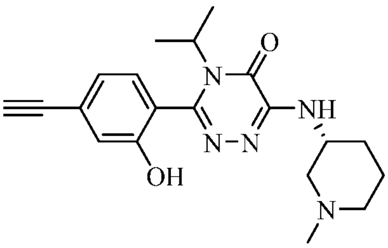

Step 1: Synthesis of 4-bromo-*N*-isopropyl-2-methoxybenzamide

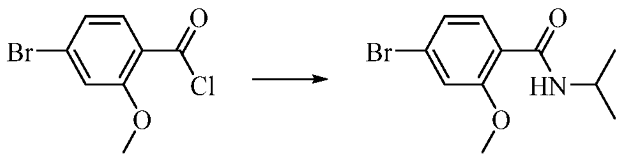

Isopropylamine (23 g, 389.52 mmol, 3.0 eq) was added dropwise to a solution of 4-bromo-2-methoxybenzoyl chloride (32.39 g, 129.84 mmol, 1.0 eq) in dichloromethane (300 mL) under an ice bath, and the mixture was allowed to react for 10 min. TLC analysis showed the completion of the reaction. The reaction solution was poured into water (200 mL) and extracted with dichloromethane (200 mL × 2). The organic phases were combined, dried, and concentrated to give the product (35.33 g, yield: 100%).

Step 2: Synthesis of 4-bromo-*N*-isopropyl-2-methoxybenzothioamide

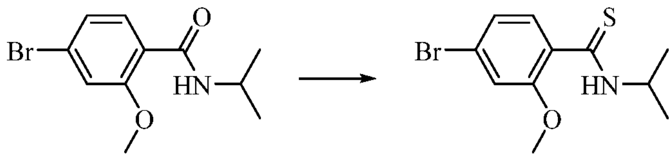

4-Bromo-*N*-isopropyl-2-methoxybenzamide (35.33 g, 129.84 mmol, 1.0 eq) and Lawesson's reagent (28.88 g, 71.41 mmol, 0.55 eq) were dissolved in THF (300 mL), and the mixture was allowed to react at 60 °C for 2 h. TLC analysis showed the completion of the reaction. The reaction solution was concentrated under reduced pressure, and the crude product was purified by silica gel column chromatography (ethyl acetate:petroleum ether = 1:15) to give the product (30 g, yield: 80.1%).

Step 3: Synthesis of methyl 4-bromo-*N*-isopropyl-2-methoxybenzimidothioate

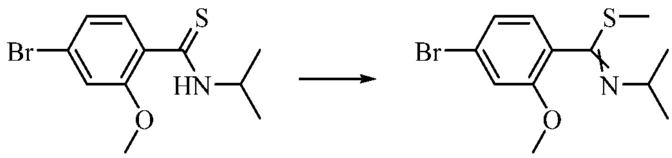

4-Bromo-*N*-isopropyl-2-methoxybenzothioamide (30 g, 104.09 mmol, 1.0 eq) and iodomethane (29.55 g, 208.18 mmol, 2.0 eq) were dissolved in THF (300 mL), and the solution was allowed to react at room temperature for 17 h. TLC analysis showed the completion of the reaction. The reaction solution was poured into water (300 mL), and the pH was adjusted to about 9 with potassium carbonate, followed by extraction with ethyl acetate (200 mL × 2). The organic phases were combined, dried, and concentrated to give the product (31.45 g, yield: 100%).

Step 4: Synthesis of *N*-amino-4-bromo-2-methoxy-N-isopropyl-benzamidine

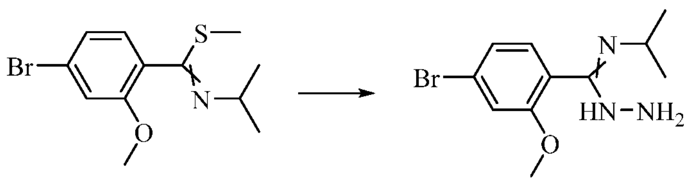

Methyl 4-bromo-*N*-isopropyl-2-methoxybenzimidothioate (31.45 g, 108.09 mmol, 1.0 eq) and hydrazine hydrate (6.13 g, 104.09 mmol, 1.0 eq) were dissolved in EtOH (300 mL), and the solution was allowed to react at 80 °C for 5 h. LC-MS monitoring showed the completion of the reaction. The reaction solution was concentrated under reduced pressure, and the crude product was slurried with methyl *tert*-butyl ether (200 mL). The slurry was filtered under vacuum to give the product (20 g, yield: 67.2%).

Step 5: Synthesis of 6-amino-3-(4-bromo-2-methoxyphenyl)-4-isopropyl-1,2,4-triazin-5(4*H*)-one

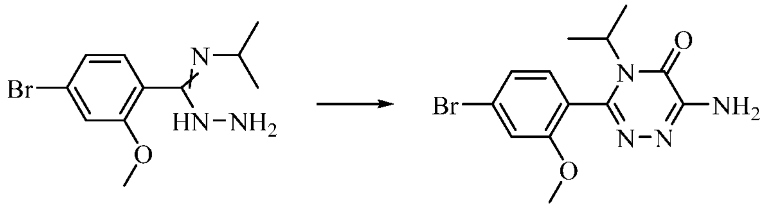

*N*-Amino-4-bromo-2-methoxy-*N*-isopropyl-benzamidine (20 g, 69.9 mmol, 1.0 eq), ethyl thiooxamate (9.31 g,

69.9 mmol, 1.0 eq), and ethyl acetate (14.15 g, 139.8 mmol, 2.0 eq) were dissolved in EtOH (200 mL), and the solution was allowed to react at 70 °C for 25 h. TLC monitoring showed the completion of the reaction. The reaction solution was concentrated under reduced pressure, and the residue was purified by silica gel column chromatography (ethyl acetate:petroleum ether = 1:2) to give the product (6.5 g, yield: 27.4%).

Step 6: Synthesis of 6-bromo-3-(4-bromo-2-methoxyphenyl)-4-isopropyl-1,2,4-triazin-5(4*H*)-one

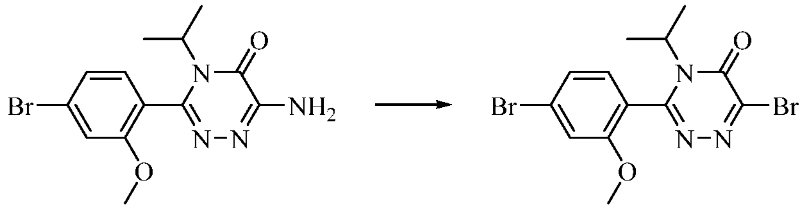

6-Amino-3-(4-bromo-2-methoxyphenyl)-4-isopropyl-1,2,4-triazin-5(4*H*)-one (6.5 g, 19.16 mmol, 1.0 eq) and CuBr (5.50 g, 38.32 mmol, 2.0 eq) were dispersed in ACN (65 mL), and *tert*-butyl nitrite (3.95 g, 38.32 mmol, 2.0 eq) was added dropwise and slowly at 70 °C under a nitrogen atmosphere. The mixture was allowed to react for 0.5 h. LC-MS monitoring showed the completion of the reaction. The reaction solution was concentrated under reduced pressure, and the crude product was purified by silica gel column chromatography (ethyl acetate:petroleum ether = 1:5) to give the product (2.4 g, yield: 31.1%).

Step 7: Synthesis of *tert-butyl* (*R*)-3-((3-(4-bromo-2-methoxyphenyl)-4-isopropyl-5-oxo-4,5-dihydro-1,2,4-triazin-6-yl) am ino)piperidine-1-carboxylate

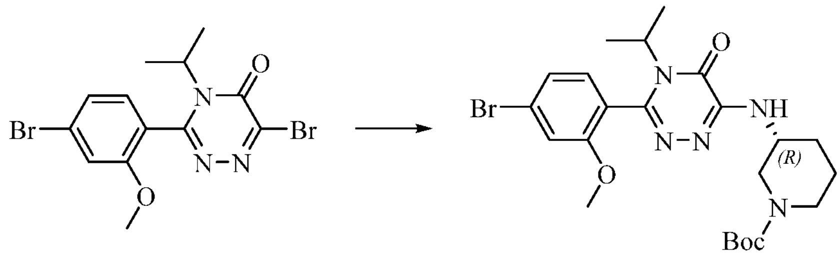

6-Bromo-3-(4-bromo-2-methoxyphenyl)-4-isopropyl-1,2,4-triazin-5(4*H*)-one (2.4 g, 5.95 mmol, 1.0 eq), *tert*-butyl (*R*)-3-aminopiperidine-1-carboxylate (1.43 g, 7.14 mmol, 1.2 eq), and DIPEA (1.54 g, 11.9 mmol, 2.0 eq) were dissolved in 1,4-dioxane (25 mL), and the solution was allowed to react at 100 °C for 20 h. TLC monitoring showed the completion of the reaction. The reaction solution was poured into water (30 mL) and extracted with ethyl acetate (30 mL × 2). The organic phases were combined, dried, and concentrated to give the product (2.4 g, yield: 77.4%).

Step 8: Synthesis of *tert*-butyl (*R*)-3-((4-isopropyl-3-(2-methoxy-4-((trimethylsilyl)ethynyl)phenyl)-5-oxo-4,5-dihydro-1,2 ,4-triazin-6-yl)amino)piperidine-1-carboxylate

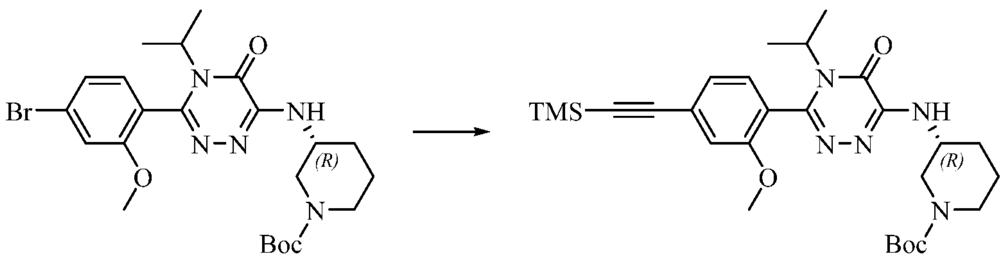

*tert-Butyl* (*R*)-3-((3-(4-bromo-2-methoxyphenyl)-4-isopropyl-5-oxo-4,5-dihydro-1,2,4-triazin-6-yl)am ino)piperidine-1-carboxylate (2.4 g, 4.59 mmol, 1.0 eq), trimethylsilylacetylene (2.25 g, 22.95 mmol, 5.0 eq), $PdCl_2(PPh_3)_2$ (323 mg, 0.46 mmol, 0.1 eq), and CuI (263 mg, 1.38 mmol, 0.3 eq) were dispersed in THF (20 mL) and diisopropylamine (20 mL), and the dispersion was allowed to react at 60 °C for 24 h under a nitrogen atmosphere. TLC analysis showed the completion of the reaction. The reaction solution was concentrated under reduced pressure, and the crude product was purified by silica gel column chromatography (ethyl acetate: petroleum ether = 1:3) to give the product (2.0 g, yield: 88.7%).

Step 9: Synthesis of (*R*)-4-isopropyl-3-(2-methoxy-4-((trimethylsilyl)ethynyl) phenyl)-6-(piperidin-3-ylamino)-1,2,4-triazin-5(4*H*)-one

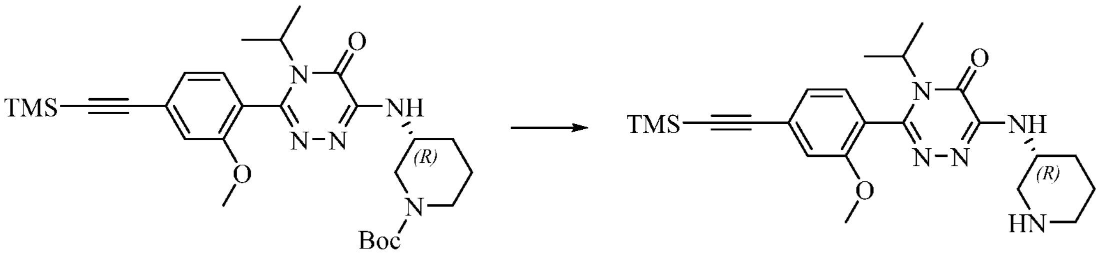

*tert*-Butyl (*R*)-3-((4-isopropyl-3-(2-methoxy-4-((trimethylsilyl)ethynyl) phenyl)-5-oxo-4,5-dihydro-1,2,4-triazin-6-yl)amino)piperidine-1-carboxylate (2.2 g, 4.08 mmol, 1.0 eq) was dissolved in ethyl acetate (20 mL), and a solution of hydrogen chloride in 1,4-dioxane (4 mol/L, 10 mL) was added. The mixture was allowed to react at room temperature for 2 h. TLC analysis showed the completion of the reaction. The reaction solution was poured into water (30 mL), and the pH was adjusted to about 9 with sodium carbonate, followed by extraction with ethyl acetate (30 mL × 2). The organic phases were combined, dried, and concentrated to give the product (1.7 g, yield: 100%).

Step 10: Synthesis of (*R*)-4-isopropyl-6-((1-ethylpiperidin-3-yl)amino)-3-(2-methoxy-4-((trimethylsilyl)ethynyl)p henyl)-1,2,4-triazin-5(4*H*)-one

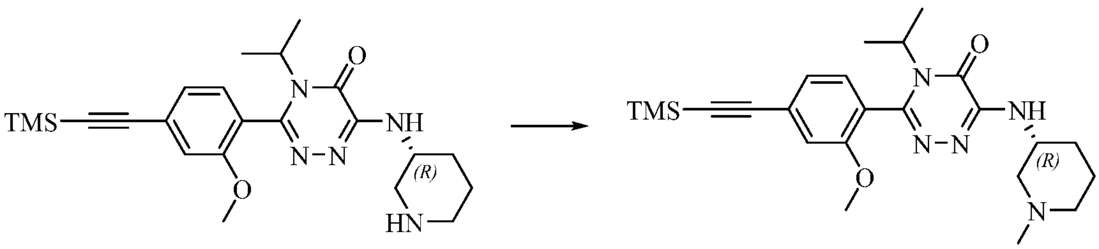

(*R*)-4-Isopropyl-3-(2-methoxy-4-((trimethylsilyl)ethynyl)phenyl)-6-(piperidin-3-ylamino)-1,2,4-triazin-5(4H)-one (0.5 g, 1.13 mmol, 1.0 eq) and an aqueous formaldehyde solution (37%, 92 mg, 1.13 mmol, 1.0 eq) were dissolved in methanol (20 mL), and the solution was stirred at room temperature for 1 h. Sodium cyanoborohydride (71 mg, 1.13 mmol, 1.0 eq) was added, and the mixture was allowed to react at room temperature for 10 min. TLC monitoring showed the completion of the reaction. The reaction solution was concentrated under reduced pressure, and the crude product was dispersed in water (10 mL). The dispersion was extracted with dichloromethane (20 mL × 2). The organic phases were combined, dried, and concentrated to give the product (513 mg, yield: 100%).

Step 11: Synthesis of (*R*)-3-(4-ethynyl-2-hydroxyphenyl)-4-isopropyl-6-((1-methylpiperidin-3-yl)amino)-1,2,4-tri azin-5(4H)-one

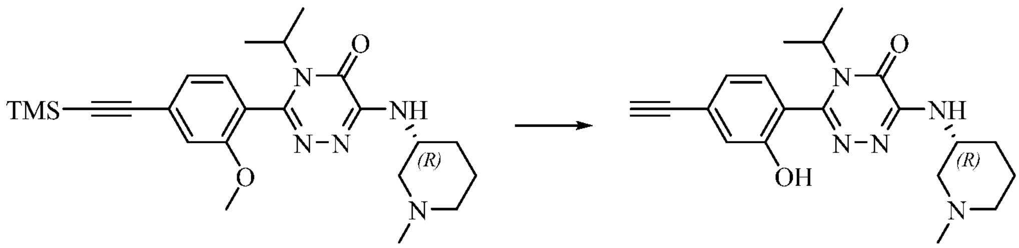

(*R*)-4-Isopropyl-6-((1-ethylpiperidin-3-yl)amino)-3-(2-methoxy-4-((trimethyls ilyl)ethynyl)phenyl)-1,2,4-triazin-5(4*H*)-one (513 mg, 1.13 mmol, 1.0 eq) was dissolved in dichloromethane (20 mL), and the solution was cooled to -60 °C. Boron tribromide (1.42 g, 5.65 mmol, 5.0 eq) was added, and the mixture was naturally warmed to room temperature and allowed to react for 17 h. TLC analysis showed the completion of the reaction. An appropriate amount of methanol was added to the reaction solution, and the mixture was concentrated under reduced pressure. The crude product was dispersed in water (20 mL), and the pH was adjusted to about 8 with sodium bicarbonate, followed by extraction with ethyl acetate (20 mL × 3). The organic phases were combined, dried, and concentrated, and the crude product was purified by preparative thin-layer chromatography (dichloromethane:methanol = 10:1) to give the product (80 mg, yield: 19.3%).

$^1$HNMR (400 MHz, DMSO-$d_6$) δ(ppm): 10.43 (s, 1H), 7.32-7.30 (d, 1H), 7.04-7.02 (d, 2H), 6.91 (s, 1H), 4.28 (s, 1H), 4.06-4.03 (t, 1H), 3.91-3.85 (m, 1H), 2.90 (s, 1H), 2.68 (s, 1H), 2.35 (s, 5H), 1.73 (s, 2H), 1.58 (s, 2H), 1.43-1.41 (d, 6H).

Molecular formula: $C_{20}H_{25}N_5O_2$ Exact molecular weight: 367.20 LC-MS *(m/z)* = 368.24 [M+H]$^+$.

**Example 57: (*R*)-3-(4-Ethynyl-2-hydroxyphenyl)-4-methyl-6-(piperidin-3-ylamino)-1,2,4-triazin-5(4 *H*)-one (Compound 201)**

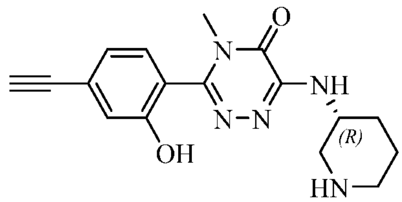

Step 1: Synthesis of (*R*)-3-(4-ethynyl-2-hydroxyphenyl)-4-methyl-6-(piperidin-3-ylamino)-1,2,4-triazin-5(4*H*)-o ne

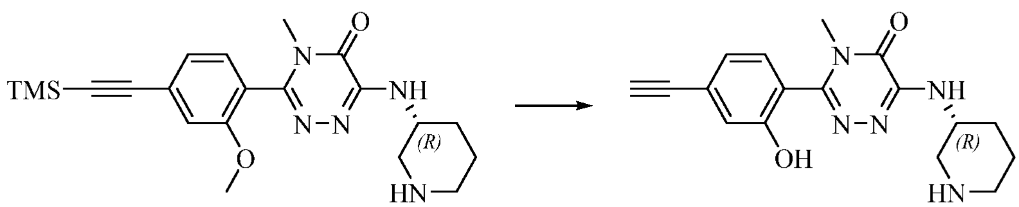

(*R*)-3-(2-Methoxy-4-((trimethylsilyl)ethynyl)phenyl)-4-methyl-6-(piperidin-3-ylamino)-1,2,4-triazin-5(4*H*)-one (0.3 g, 0.72 mmol, 1.0 eq) was dissolved in dichloromethane (5 mL), and the solution was cooled to -70 °C. Boron tribromide (541 mg, 2.16 mmol, 3.0 eq) was added, and the mixture was naturally warmed to room temperature and allowed to react for 16 h. LC-MS analysis showed the completion of the reaction. An appropriate amount of methanol was added to the reaction solution, and the mixture was concentrated under reduced pressure. The crude product was dissolved in methanol (20 mL), and the pH was adjusted to about 9 with an aqueous sodium bicarbonate solution. The crude product solution was subjected to silica gel column chromatography (dichloromethane:methanol = 10:1) to give a crude product (100 mg), which was then purified by silica gel column chromatography (dichloromethane:methanol = 12:1) to give the product (12 mg, yield: 5.1%).

$^1$HNMR (400 MHz, DMSO-$d_6$) δ(ppm): 10.46 (s, 1H),8.63 (s, 1H), 7.38-7.36 (d, 1H), 7.32-7.30 (d, 1H) 7.06-7.04 (d, 2H), 4.30 (s, 1H), 4.26-4.25 (d, 1H), 3.39 (s, 1H), 3.32 (s, 1H), 3.19 (s, 3H), 2.96-2.90 (t, 1H), 2.87-2.85 (t, 1H), 1.95-1.90 (d, 2H), 1.74-1.71 (d, 2H).

Molecular formula: $C_{17}H_{19}N_5O_2$ Molecular weight: 325.15 LC-MS (*m*/*z*) = 326.18 $[M+H]^+$.

**Example** 58: **(*R*)-6-((1-(2,2-Difluoroethyl)piperidin-3-yl)amino)-3-(4-ethynyl-2-hydroxyphenyl)-4-m ethyl-1,2,4-triazin-5(4H)-one (Compound 204)**

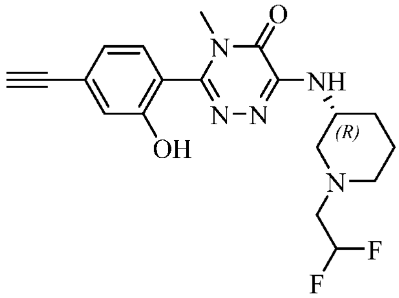

Step 1: Synthesis of (*R*)-6-((1-(2,2-difluoroethyl)piperidin-3-yl)amino)-3-(2-methoxy-4-((trimethylsilyl)ethynyl) phenyl)-4-methyl-1,2,4-triazin-5(4*H*)-one

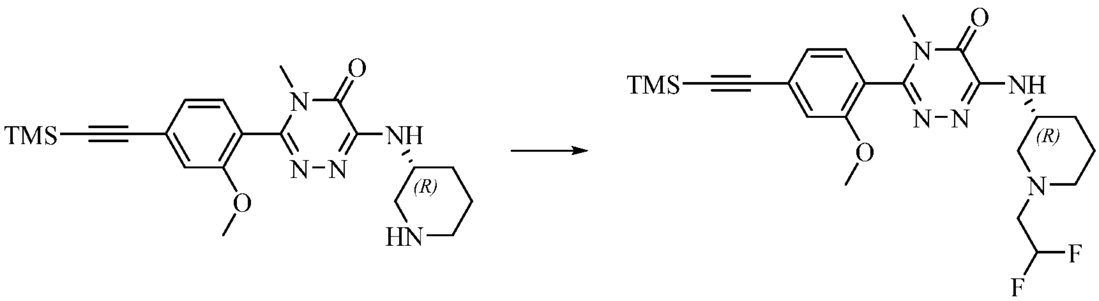

(*R*)-3-(2-Methoxy-4-((trimethylsilyl)ethynyl)phenyl)-4-methyl-6-(piperidin-3-ylamino)-1,2,4-triazin-5(4*H*)-one (0.5 g, 1.21 mmol, 1.0 eq), potassium carbonate (334 mg, 2.42 mmol, 2.0 eq), and 1,1-difluoro-2-iodoethane (464 mg, 2.42 mmol, 2.0 eq) were added to DMF (10 mL), and the mixture was allowed to react at 80 °C for 16 h. LC-MS analysis showed the completion of the reaction. The reaction solution was poured into water (20 mL) and extracted with ethyl acetate (20 mL × 2). The organic phases were combined, dried, and concentrated, and the crude product was purified by silica gel column chromatography (dichloromethane:methanol = 40:1) to give the product (200 mg, yield: 41.0%).

Step 2: Synthesis of (*R*)-6-((1-(2,2-difluoroethyl)piperidin-3-yl)amino)-3-(4-ethynyl-2-hydroxyphenyl)-4-methy l-1,2,4-triazin-5(4*H*)-one

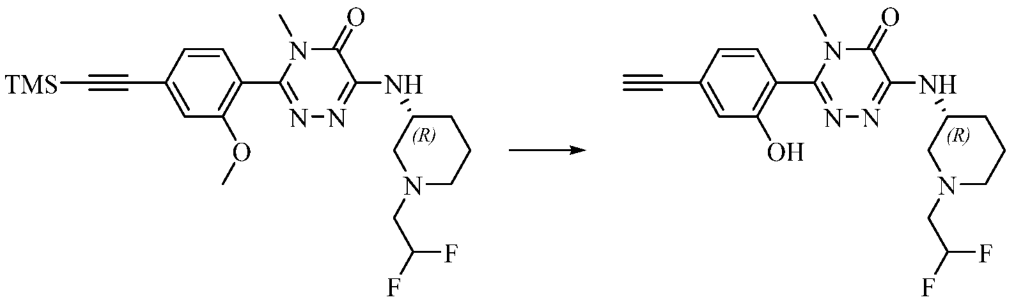

(*R*)-6-((1-(2,2-Difluoroethyl)piperidin-3-yl)amino)-3-(2-methoxy-4-((trimethy lsilyl)ethynyl)phenyl)-4-methyl-1,2,4-triazin-5(4*H*)-one (200 mg, 0.49 mmol, 1.0 eq) was dissolved in dichloromethane (5 mL), and the solution

was cooled to -60 °C. Boron tribromide (614 mg, 2.45 mmol, 5.0 eq) was added, and the mixture was naturally warmed to room temperature and allowed to react for 4 h. LC-MS analysis showed the completion of the reaction. An appropriate amount of methanol was added to the reaction solution, and the reaction solution was concentrated under reduced pressure. The crude product was dissolved in water (20 mL), and the pH was adjusted to about 8 with sodium bicarbonate, followed by extraction with ethyl acetate (20 mL × 3). The organic phases were combined, dried, and concentrated, and the crude product was purified by preparative thin-layer chromatography (ethyl acetate:methanol = 10:1) to give the product (40 mg, yield: 21%).

$^1$HNMR (400 MHz, DMSO-$d_6$) δ(ppm): 10.42 (s, 1H), 7.32-7.30 (d, 1H), 7.05-703 (t, 2H), 6.88-6.86 (d, 1H), 6.29-6.01 (t, 1H), 4.28 (s, 1H), 4.05-4.01 (t, 1H), 3.18 (s, 3H), 2.94-2.92 (d, 1H), 2.80-2.69 (m, 3H), 2.33 (s, 2H), 1.72-1.66 (t, 2H), 1.58-1.51 (m, 2H).

Molecular formula: $C_{19}H_{21}F_2N_5O_2$ Exact molecular weight: 389.17 LC-MS *(m/z)* = 390.18 $[M+H]^+$.

**Example 59: Synthesis of (*R*)-3,5-dimethyl-2-(6-((1-methylpiperidin-3-yl)amino)pyridazin-3-yl)phenol (reference substance)**

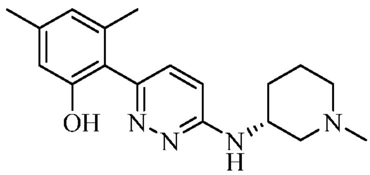

Step 1: Synthesis of 2-iodo-3,5-dimethylphenol

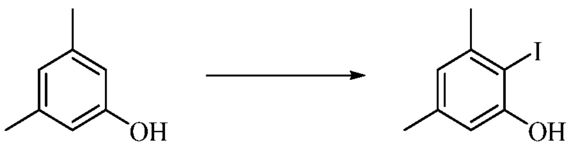

3,5-Dimethylphenol (10.0 g, 81.9 mmol, 1.0 eq) was dissolved in dimethylbenzene (500 mL), and the solution was cooled to 0 °C under a nitrogen atmosphere. NaH (6.55 g, 164 mmol, 2.0 eq) was added in portions. After the addition, the mixture was allowed to react at 0 °C for 30 min, and then warmed to room temperature and allowed to react for 1 h. The reaction mixture was cooled to 0 °C, and a solution of iodine (20.7 g, 81.9 mmol, 1.0 eq) in dimethylbenzene (250 mL) was added dropwise. After the addition, the resulting mixture was allowed to react for 30 min. After completion of the reaction, 0.5 mol/L dilute hydrochloric acid was added to quench the reaction, and extraction was performed with EA (200 mL × 3). The organic phases were combined, dried over anhydrous sodium sulfate, filtered, and concentrated, and the crude product was purified by silica gel column chromatography (PE:EA = 100:1) to give the product (5.2 g, yield: 25.6%).

Step 2: Synthesis of 2-iodo-1-methoxy-3,5-dimethylbenzene

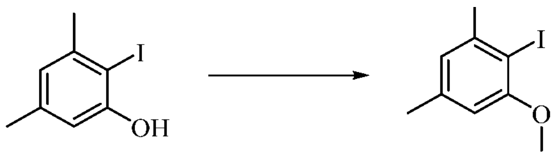

2-Iodo-3,5-dimethylphenol (2.0 g, 8.06 mmol, 1.0 eq) and potassium carbonate (2.23 g, 16.1 mmol, 2.0 eq) were added to DMF (20 mL), and iodomethane (1.72 g, 12.1 mmol, 1.5 eq) was added with stirring. The mixture was allowed to react at room temperature for 2 h. After completion of the reaction, water was added, and extraction was performed with EA

(50 mL × 3). The organic phase was washed with saturated brine, dried over anhydrous sodium sulfate, filtered, and concentrated, and the crude product was purified by silica gel column chromatography (PE:EA = 100:1) to give the product (1.9 g, yield: 89.9%).

Step 3: Synthesis of (2-methoxy-4,6-dimethylphenyl)boronic acid

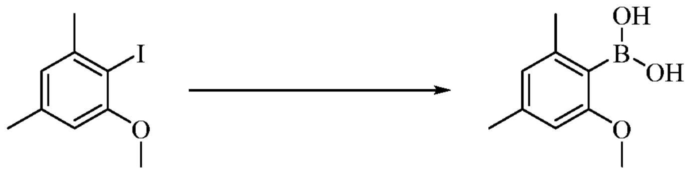

2-Iodo-1-methoxy-3,5-dimethylbenzene (1.8 g, 6.87 mmol, 1.0 eq) and triisopropyl borate (2.57 g, 13.7 mmol, 2.0 eq) were dissolved in tetrahydrofuran (20 mL), and the solution was cooled to -78 °C under a nitrogen atmosphere. *n*-Butyllithium (2.5 mol/L solution in *n*-hexane, 5.48 mL, 13.7 mmol, 2.0 eq) was added dropwise and slowly. After the dropwise addition, the mixture was allowed to react for 30 min with the temperature maintained at -78 °C. Then, the reaction mixture was slowly warmed to room temperature and allowed to react for 2 h. After completion of the reaction, 2 mol/L dilute hydrochloric acid was added to quench the reaction, and the reaction mixture was stirred and allowed to react for 0.5 h. Extraction was performed with EA (20 mL × 3). The organic phase was dried, filtered, and concentrated. The resulting crude product was slurried with PE, and the slurry was filtered under vacuum to give the product (850 mg, yield: 68.8%).

Step 4: Synthesis of *tert*-butyl (*R*)-3-((6-chloropyridazin-3-yl)amino)piperidine-1-carboxylate

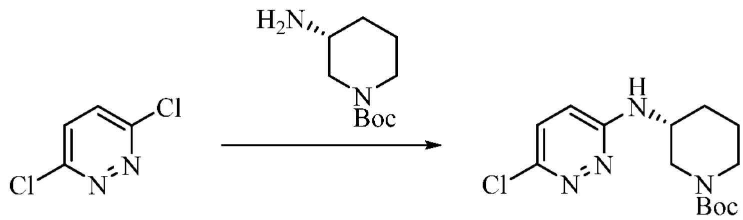

3,6-Dichloropyridazine (10.0 g, 67.1 mmol, 1.0 eq) and *tert*-butyl (*R*)-3-aminopiperidine-1-carboxylate (16.1 g, 80.5 mmol, 1.2 eq) were dissolved in n-butanol (100 mL), and the solution was heated to 115 °C and allowed to react for 20 h under a nitrogen atmosphere. After completion of the reaction, the reaction mixture was concentrated under reduced pressure, and the residue was slurried with EA. The slurry was filtered under vacuum to give the product (16.5 g, yield: 78.6%).

Step 5 : Synthesis of *tert-butyl* (*R*)-3-((6-(2-methoxy-4,6-dimethylphenyl)pyridazin-3-yl)amino)piperidine-1-carboxylate

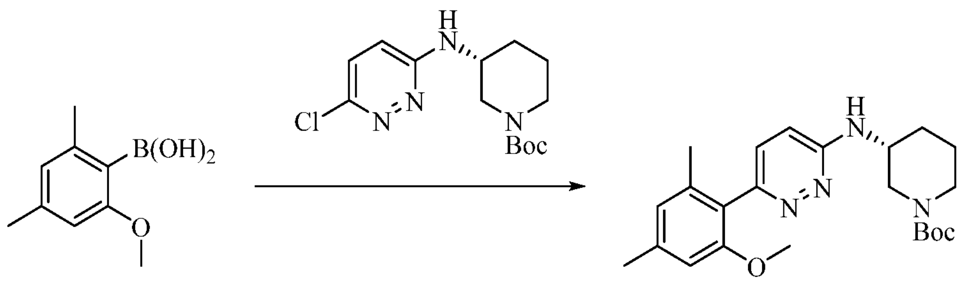

(2-Methoxy-4,6-dimethylphenyl)boronic acid (800 mg, 4.44 mmol, 1.0 eq), *tert*-butyl (*R*)-3-((6-chloropyridazin-3-yl)amino)piperidine-1-carboxylate (1.39 g, 4.44 mmol, 1.0 eq), Pd(dppf)$Cl_2$ (325 mg, 0.444 mmol, 0.1 eq), and sodium bicarbonate (746 mg, 8.88 mmol, 2.0 eq) were added to 1,4-dioxane (10 mL) and water (5 mL), and the mixture was allowed to react at 110 °C for 2 h under a nitrogen atmosphere. After completion of the reaction, the reaction mixture was cooled to room temperature. Water was added, and extraction was performed with EA (50 mL × 2). The organic phase was dried, filtered, and concentrated, and the crude product was purified by silica gel column chromatography (DCM:MeOH = 100:1 to 50:1) to give the product (540 mg, yield: 29.5%).

Step 6 Synthesis of (*R*)-3,5-dimethyl-2-(6-(piperidin-3-ylamino)pyridazin-3-yl)phenol

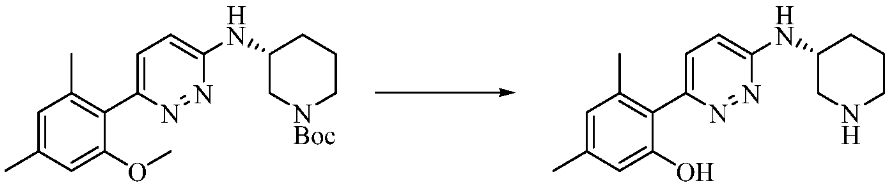

*tert*-Butyl (*R*)-3-((6-(2-methoxy-4,6-dimethylphenyl)pyridazin-3-yl)amino)piperidine-1-carboxylate (540 mg, 1.31 mmol, 1.0 eq) was added to DCM (5 mL), and the mixture was cooled to -10 °C (ice-salt bath). Boron tribromide (1.64 g, 6.55 mmol) was added dropwise and slowly. After the dropwise addition, the resulting mixture was naturally warmed to room temperature and allowed to react. After completion of the reaction, methanol was added to quench the reaction, and the mixture was concentrated and diluted with water. The pH was adjusted to 8, and extraction was performed with DCM (30 mL × 3). The organic phase was dried over anhydrous sodium sulfate, filtered, and concentrated to give a crude product (290 mg, yield: 74.2%).

Step 7: Synthesis of (*R*)-3,5-dimethyl-2-(6-((1-methylpiperidin-3-yl)amino)pyridazin-3-yl)phenol

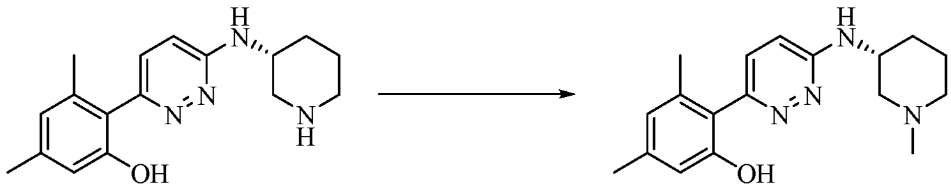

(*R*)-3,5-Dimethyl-2-(6-(piperidin-3-ylamino)pyridazin-3-yl)phenol (280 mg, 0.938 mmol, 1.0 eq) was dissolved in methanol (5 mL), and an aqueous formaldehyde solution (37%, 230 mg, 2.81 mmol, 3.0 eq) was added. The mixture was stirred at room temperature for 30 min, and sodium cyanoborohydride (177 mg, 2.81 mmol, 3.0 eq) was added. The resulting mixture was allowed to react at room temperature for 2 h. After completion of the reaction, a saturated aqueous ammonium chloride solution was added to quench the reaction, and extraction was performed with a mixed solution (DCM/MeOH = 10:1) (30 mL × 3). The organic phase was dried over anhydrous sodium sulfate and concentrated, and the residue was separated by preparative reversed phase chromatography (acetonitrile/water = 30%) to give the product (202 mg, yield: 68.9%).

$^1$H NMR (300 MHz, DMSO-$d_6$) $\delta$: 9.49 (s, 1H), 7.16 (d, $J$ = 9.2 Hz, 1H), 6.84 (d, $J$ = 9.2 Hz, 1H), 6.67 (d, $J$ = 7.8 Hz, 1H), 6.59-6.51 (m, 2H), 4.15-3.95 (m, 1H), 2.95-2.76 (m, 1H), 2.21 (s, 3H), 2.18 (s, 3H), 2.03 (s, 3H), 1.98-1.78 (m, 2H), 1.77-1.64 (m, 1H), 1.63-1.45 (m, 1H), 1.38-1.18 (m, 2H).

Molecular formula: $C_{18}H_{24}N_4O$ Exact molecular weight: 312.19 LC-MS (*m/z*) = 313.26 [M+H]$^+$.

**Experimental Example 1: Assay for Inhibitory Activity of Compounds Disclosed Herein Against Cell NLRP3 Inflammasomes**

Test substances: The compounds of the present disclosure, prepared according to the methods described in the examples.

THP-1 is an immortalized human macrophage cell line.

Instrument: microplate reader (made by PE)

Assay method:

1. THP-1 cells were cultured in 1640 complete medium (500 mL of 1640 + 56 mL of FBS + 560 μL of 1000× P/S + 2 μL of mercaptoethanol), and cells at passages 3-20 were used.

2. Culture plate coating: 100 μL of polylysine solution was added to a 96-well cell culture plate. After 30 min of incubation at 37 °C, the solution was discarded, and the plate was washed with PBS twice for later use.

3. Induction of THP-1 differentiation: THP-1 cells were resuspended in an appropriate amount of complete medium containing 10 ng/mL PMA to form a cell suspension with a density of $5 \times 10^5$ cells/mL. The suspension was added to the 96-well plate at 100 μL/well, and the plate was incubated overnight in a 37 °C carbon dioxide cell incubator for 16 h.

4. Stimulation of THP-1:

a. Serum-free THP-1 culture medium containing LPS was added, with the final concentration being 500 ng/mL. The plate was incubated in a 37 °C carbon dioxide cell incubator for 3 h.

b. Mother liquors of the compounds to be tested with gradient concentrations were prepared with DMSO, added to the cells, and well mixed. Dilution was finally performed in a 1:1000 ratio. The plate was incubated in a 37 °C carbon dioxide cell incubator for 1 h.

c. Nigericin was added to each well, with the final concentration being 10 g/mL. The plate was incubated in a carbon dioxide cell incubator at 37 °C for 30 min.

d. The medium in the wells was transferred to a new culture plate, and after 5 min of centrifugation at 3000 rpm, the supernatant was transferred to a new 96-well plate.

e. The content of human IL-1β in the collected cell culture supernatant samples was determined with a commercial ELISA kit by following the instructions.

The test results are shown in Table 2 below:

Table 2. Inhibitory activity of compounds disclosed herein against THP-1 cell NLRP3

| Test substance | $IC_{50}$ (nM) |
|---|---|
| Compound 23 | 2.4 |
| Compound 25 | 2.2 |
| Compound 27 | 5.2 |
| Compound 33 | 11.5 |
| Compound 35 | 3.3 |
| Compound 36 | 12.7 |
| Compound 37 | 0.8 |
| Compound 39 | 0.3 |
| Compound 40 | 12.8 |
| Compound 41 | 0.3 |
| Compound 42 | 4.6 |
| Compound 44 | 3.6 |
| Compound 45 | 0.7 |
| Compound 61 | 1.4 |
| Compound 81 | 1.1 |

(continued)

| Test substance | $IC_{50}$ (nM) |
|---|---|
| Compound 82 | 1.1 |
| Compound 83 | 6.1 |
| Compound 84 | 0.2 |
| Compound 85 | 0.6 |
| Compound 86 | 0.3 |
| Compound 87 | 0.7 |
| Compound 88 | 1.2 |
| Compound 89 | 0.2 |
| Compound 90 | 0.4 |
| Compound 91 | 0.2 |
| Compound 92 | 0.5 |
| Compound 93 | 1.4 |
| Compound 94 | 0.3 |
| Compound 95 | 0.4 |
| Compound 96 | 0.5 |
| Compound 97 | 1.0 |
| Compound 98 | 5.0 |
| Compound 99 | 0.7 |
| Compound 100 | 2.4 |
| Compound 103 | 0.6 |
| Compound 109 | 16.0 |
| Compound 111 | 1.1 |
| Compound 112 | 12.0 |
| Compound 114 | 1.3 |
| Compound 115 | 0.3 |
| Compound 139 | 1.1 |
| Compound 149 | 0.5 |
| Compound 161 | 9.9 |
| Compound 163 | 1.1 |
| Compound 240 | 1.3 |

It can be seen from the experimental results in Table 2 that the compounds of the present disclosure exhibited good inhibitory activity against NLRP3 inflammasomes. Therefore, the compounds of the present disclosure can be used for preventing and/or treating NLRP3 inflammasome-associated diseases.

**Experimental Example 2: Evaluation of Stability of Compounds Disclosed Herein in Liver Microsomes**

Test substances: The compounds of the present disclosure and the reference substance, prepared according to the methods described in the examples, where the reference substance is Example Ex 047 from Novartis' patent WO2020234715A1, with the structure being

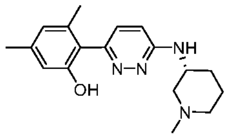.

Composition of experimental incubation system:

| Substances to be added | | Initial concentration | Proportion | Final concentration |
|---|---|---|---|---|
| Phosphate buffer (containing $MgCl_2$) | $K_2HPO4$ | 50 mM | 67.5% | 50 mM |
| | $KH_2PO4$ | | | |
| | $MgCl_2$ | 3 mM | | 3 mM |
| Liver microsome | | 20 mg protein/mL | 2.50% | 0.5 mg protein/mL |
| Test substance | | 10 μM | 10.0% | 1 μM |
| β-NADPH | | 5 mM | 20.0% | 1 mM |

Preparation of test substance:

An appropriate amount of the compound was precisely weighed out and dissolved in DMSO to prepare a 10.0 mM stock solution. The 10.0 mM stock solution was diluted with DMSO to 1.0 mM, and then diluted with phosphate buffer (containing 3 mM $MgCl_2$) to prepare a 10 μM test substance working solution for later use (DMSO content in the reaction system: 0.1%).

Preparation of β-NADPH:

An appropriate amount of β-NADPH was precisely weighed out and then prepared into a 5 mM β-NADPH working solution using phosphate buffer (containing 3 mM $MgCl_2$) for later use.

Experimental procedures:

(1) The liver microsomes (20 mg protein/mL) were taken out from a -80 °C freezer and pre-incubated on a 37 °C water bath thermostatic oscillator for 3 min to thaw for later use.

(2) A mixed solution of the incubation system (withoutβ-NADPH) was prepared according to "composition of experimental incubation system" described above, and pre-incubated on a 37 °C water bath thermostatic oscillator for 2 min.

(3) Control group (without β-NADPH): 10 μL of phosphate buffer (containing 3 mM $MgCl_2$) and 40 μL of the mixed solution of the incubation system in step (2) were taken and mixed, and the mixture was vortexed for 30 s and mixed well, resulting in a total reaction volume of 50 μL; the sample was duplicated. The mixture was placed on a 37 °C water bath thermostatic oscillator for incubation, and timing was started, with termination time points set at 0 min and 60 min.

(4) Sample group: 10 μL of the β-NADPH solution (5 mM) and 40 μL of the mixed solution in step (2) were taken and mixed, resulting in a total reaction volume of 50 μL; the mixture was vortexed for 30 s and mixed well; the sample was duplicated. The mixture was placed on a 37 °C water bath thermostatic oscillator for incubation, and timing was started, with termination time points set at 0 min, 5 min, 10 min, 20 min, 30 min, and 60 min.

(5) The mixture was vortexed for 5 min and then centrifuged at 4000 rpm for 10 min.

(6) 50 μL of the supernatant was taken and added to 150 μL of water, and the resulting mixture was vortexed and mixed well before LC/MS/MS analysis.

Data analysis:

The half-life ($t_{1/2}$) and clearance rate (CL) were calculated using the following first-order kinetic formula:

$$C_t = C_0 \times e^{-kt}$$

$$t_{1/2} = \ln 2/k = 0.693/k$$

$$Clint = Vd \times k$$

Vd = 1/protein content in liver microsomes

Notes: k denotes the slope of the logarithm of the remaining amount of a compound vs. time, and Vd denotes apparent volume of distribution.

The experimental results are shown in Table 3 below:

Table 3. Experiment on stability of the compounds disclosed herein in canine and rat liver microsomes

| Compound | Canine | | Rat | |
|---|---|---|---|---|
| | Clint (mL/min/mg) | $t_{1/2}$ (min) | Clint (mL/min/mg) | $t_{1/2}$ (min) |
| Reference substance | 0.0296 | 46.8 | 0.0420 | 33.0 |
| 23 | →0 | →∞ | →0 | →∞ |
| 27 | →0 | →∞ | 0.0062 | 224 |
| 42 | 0.0012 | 1155 | 0.0042 | 330 |
| 81 | 0.0050 | 277 | 0.0030 | 462 |
| 82 | 0.0080 | 173 | 0.0104 | 133 |
| 83 | 0.0038 | 365 | 0.0100 | 139 |
| 93 | 0.0050 | 277 | 0.0056 | 248 |
| 100 | 0.0028 | 495 | 0.0084 | 165 |
| 45 | 0.0014 | 990 | 0.0068 | 204 |
| 85 | 0.0052 | 267 | 0.0046 | 301 |
| 99 | 0.0066 | 210 | 0.0032 | 433 |
| 88 | 0.0016 | 866 | 0.0020 | 693 |
| 86 | 0.0066 | 210 | 0.0078 | 178 |
| 84 | 0.0040 | 347 | 0.0040 | 347 |
| 89 | 0.0062 | 224 | 0.0080 | 173 |
| 103 | 0.0084 | 165 | 0.0082 | 169 |
| 149 | →0 | →∞ | →0 | →∞ |
| 115 | 0.0036 | 385 | 0.0202 | 68.6 |
| 114 | 0.0010 | 1386 | 0.0004 | 3465 |

It can be seen from the experimental results in Table 3 that the compounds of the present disclosure exhibited relatively low clearance rates and relatively long half-lives, and especially had superior metabolic stability compared to the reference substance.

**Experimental Example 3: Pharmacokinetic Study of Compounds in Mice**

Test substances: The compounds of the present disclosure and the reference substance, prepared according to the methods described in the examples.

**Animal administration and sample collection:**

The compounds were prepared into solutions using a mixed vehicle composed of 5% dimethyl sulfoxide, 20% (30% solutol), and 75% normal saline. The compound solutions were intragastrically administered to female C57BL/6J mice at a dose of 10 mg/kg, and the blood collection time points were: 15 min, 30 min, 1 h, 2 h, 4 h, 6 h, 8 h, and 24 h.
The compounds were prepared into solutions using a mixed vehicle composed of 5% dimethyl sulfoxide, 20% (30% solutol), and 75% normal saline. The compound solutions were intravenously injected into female C57BL/6J mice at a dose of 5 mg/kg, and the blood collection time points were: 5 min, 15 min, 30 min, 1 h, 2 h, 4 h, 6 h, 8 h, and 24 h.

**Sample analysis method:**

The samples to be tested were taken out from a -80 °C freezer, naturally thawed at room temperature, and then vortexed for 5 min.
Plasma samples of 10 μL from different individuals were accurately pipetted into 1.5 mL centrifuge tubes.
900 μL of a 100 ng/mL internal standard working solution (a solution of tolbutamide in acetonitrile) was added, and the mixture was well mixed.
After 5 min of vortexing, centrifugation was performed at 12,000 rpm for 5 min.
50 μL of the supernatant was accurately pipetted into a 96-well plate to which water was added in advance at 150 μL/well.
After 5 min of vortexing, LC-MS/MS analysis was performed.

**Data processing method:**

The results of the test substance concentrations were output by Analyst 1.6.3 of AB Sciex. Parameters such as mean, standard deviation, and coefficient of variation (excluding those output directly by Analyst 1.6.3) were calculated using Microsoft Excel. PK parameters were calculated using WinNonlin Phenoix 8.2 software NCA ($T_{max}$ was a median).

**Results**

Table 4. Results of pharmacokinetic testing of compounds in mice

| Compound | Dose iv/po (mg/kg) | $t_{z1/2}$ iv/po (h) | $V_{ss}$ iv (L/kg) | $Cl_{obs}$ iv (L/h/kg) | $T_{max}$ po (h) | $C_{max}$ po (ng/mL) | $AUC_{last}$ iv/po (h*ng/mL) | F% |
|---|---|---|---|---|---|---|---|---|
| Reference substance | 5/10 | 1.18/1.08 | 5.03 | 3.30 | 0.500 | 1480 | 1505/3003 | 99.8 |
| 23 | 5/10 | 0.920/0.983 | 1.65 | 1.54 | 0.500 | 3030 | 3233/5532 | 85.7 |
| 27 | 5/10 | 9.55/3.99 | 2.39 | 0.708 | 0.250 | 5520 | 6827/10949 | 77.7 |
| 44 | 5/10 | 3.95/4.05 | 2.76 | 0.551 | 0.500 | 1710 | 8963/12358 | 69.1 |
| 100 | 5/10 | 1.65/3.63 | 2.41 | 1.08 | 0.250 | 1720 | 4459/6278 | 68.4 |
| 45 | 5/10 | 3.57/3.18 | 1.47 | 0.436 | 1.00 | 4130 | 11398/19087 | 83.7 |
| 90 | 5/10 | 3.23/3.33 | 6.23 | 1.24 | 2.00 | 900 | 4018/7579 | 94.5 |
| 85 | 5/10 | 5.55/4.26 | 1.85 | 0.319 | 1.00 | 3540 | 15132/29105 | 94.4 |
| 84 | 5/10 | 3.81/3.10 | 3.02 | 0.889 | 0.500 | 1910 | 5589/10398 | 92.7 |

**Notes:** $t_{z1/2}$: terminal half-life; $Cl_{obs}$: clearance rate; $V_{ss}$: distribution volume; $T_{max}$: time to peak plasma concentration; $C_{max}$: peak concentration; $AUC_{last}$: area under plasma concentration-time curve (0-24 h); F%: absolute bioavailability

It can be seen from the experimental results in Table 4 that the compounds of the present disclosure exhibited relatively good pharmacokinetic properties in mice, including relatively high exposure and oral bioavailability, and

especially demonstrated superior PK properties in mice compared to the reference substance.

**Experimental Example 4: Pharmacokinetic Study of Compounds in Male SD Rats**

Test substances: The compounds of the present disclosure and the reference substance, prepared according to the methods described in the examples.

**Animal administration and sample collection:**

The compounds were prepared into solutions using a mixed vehicle composed of 5% dimethyl sulfoxide, 20% (30% solutol), and 75% normal saline. The compound solutions were intragastrically administered to male SD rats at a dose of 5.0 mg/kg, and the blood collection time points were: 15 min, 30 min, 1 h, 2 h, 4 h, 6 h, 8 h, 10 h, and 24 h.

The compounds were prepared into solutions using a mixed vehicle composed of 5% dimethyl sulfoxide, 20% (30% solutol), and 75% normal saline. The compound solutions were intravenously injected into male SD rats at a dose of 1.0 mg/kg, and the blood collection time points were: 5 min, 15 min, 30 min, 1 h, 2 h, 4 h, 6 h, 8 h, 10 h, and 24 h.

Intravenous catheterization was performed on the animals the day before the administration. After the administration, blood samples of about 300 μL were collected from the jugular vein and placed into anticoagulation tubes containing EDTA-$K_2$. The blood samples were centrifuged at 5000 rpm at 4 °C for 10 min to give plasma samples. The plasma samples were prepared within 30 min after blood collection. The plasma samples were stored in a -80 °C freezer before testing.

**Sample analysis and data processing methods:**

Using the same sample analysis and data processing methods as described in Experimental Example 3, the following results were obtained:

Table 5. Results of pharmacokinetic testing of compounds in SD rats

| Compound | Dose iv/po (mg/kg) | $t_{z1/2}$ iv/po (h) | $V_{ss}$ iv (L/kg) | $Cl_{_obs}$ iv (L/h/kg) | $T_{max}$ po (h) | $C_{max}$ po (ng/mL) | $AUC_{last}$ iv/po (h*ng/mL) | F% |
|---|---|---|---|---|---|---|---|---|
| Reference substance | 1/NA | 0.707/NA | 12.6 | 13.1 | NA | NA | 66.4/NA | NA |
| 23 | 1/5 | 3.24/3.54 | 2.15 | 0.477 | 0.500 | 1263 | 1863/8476 | 81.0 |
| 42 | 1/5 | 1.55/3.99 | 0.694 | 0.399 | 1.00 | 2490 | 2490/9724 | 77.8 |
| 45 | 1/5 | 5.63/5.19 | 3.28 | 0.503 | 4.00 | 629 | 1920/6844 | 71.5 |

**Notes:** $t_{z1/2}$: terminal half-life; $Cl_{_obs}$: clearance rate; $V_{ss}$: distribution volume; $T_{max}$: time to peak plasma concentration; $C_{max}$: peak concentration; $AUC_{last}$: area under plasma concentration-time curve (0-24 h); F%: absolute bioavailability; NA: not tested or not calculated

It can be seen from the experimental results in Table 5 that the compounds of the present disclosure exhibited relatively good exposure and oral bioavailability in rats. Compared to the reference substance, the compounds of the present disclosure demonstrated superior PK properties in rats.

**Experimental Example 5: Inhibition of hERG Potassium Ion Channel by Compounds**

Experimental method

1. Cell culture

1.1. An HEK-293 cell line stably expressing the hERG potassium channel was cultured at 37 °C with 5% carbon dioxide in DMEM medium containing 10% fetal bovine serum and 0.8 mg/mL G418. The cell density must not exceed 80%.

1.2. Before patch-clamp testing, cells were dissociated with TrypLE™ Express, and $4 \times 10^3$ cells were applied to a coverslip. After 18 h, testing was performed.

2. Method for preparing administration formulation stock solutions

2.1. Blank control: DMSO stock solution.

2.2. Positive control: An appropriate amount of cisapride was weighed out and prepared into a 10 mM stock solution using dimethyl sulfoxide (DMSO). The stock solution was aliquoted, and the aliquots were stored at -20 °C.

2.3. Compound: An appropriate amount of the compound was weighed out, and the required volume of DMSO was calculated using the formula: DMSO volume = actual amount × purity (content)/(molecular weight × theoretical concentration), and measured out using a pipette. The compound that had been weighed out was dissolved in the DMSO that had been measured out using a pipette. Meanwhile, the mass of the DMSO was measured. The actual stock solution concentration was calculated based on the amount of DMSO finally used.

3. Method for preparing administration formulation working solutions

Before hERG channel current testing, the blank control stock solution was diluted with an appropriate amount of extracellular liquid to prepare a working solution. The positive control stock solution and the compound stock solution were diluted with an appropriate amount of extracellular liquid to prepare working solutions.

The highest test concentration of the compound was obtained by directly diluting the stock solution with extracellular liquid or further diluting the stock solution with DMSO. For other concentrations, the stock solution was first diluted with DMSO from high to low concentrations, and the dilutions were further diluted with extracellular liquid to the working solution concentrations. The DMSO concentration in each working solution was 0.3%. Before patch-clamp testing, the compound working solutions were sonicated for 20 min.

4. Basis for concentration selection

The default test concentrations for the compound were 30 μM, 10 μM, 3 μM, 1 μM, and 0.3 μM. The blank control was 0.3% DMSO.

5. Electrophysiological recording

5.1 Liquids used for recording

5.1.1. Extracellular liquid: K-007-1

140 mM NaCl, 3.5 mM KCl, 1 mM $MgCl_2$•$6H_2O$, 2 mM $CaCl_2$•$2H_2O$, 10 mM D-Glucose, 10 mM HEPES, 1.25 mM $NaH_2PO_4$•$2H_2O$; the pH was adjusted to 7.4 with NaOH.

5.1.2. Intracellular liquid: K-002-2

20 mM KCl, 115 mM K-Aspartic, 1 mM $MgCl_2$•$6H_2O$, 5 mM EGTA, 10 mM HEPES, 2 mM Na2-ATP; the pH was adjusted to 7.2 with KOH.

5.2. Patch-clamp testing

The voltage stimulation protocol for whole-cell patch-clamp recording of hERG currents was as follows: Once a whole-cell seal was formed, the cell membrane voltage was clamped at -80 mV. The clamping voltage was depolarized from -80 mV to -50 mV for 0.5 s (as leak current detection), then stepped to 30 mV for 2.5 s, and rapidly returned to -50 mV for 4 s to elicit a tail current of the hERG channel. Data acquisition was repeated every 10 s to observe the effect of the compound on hERG tail currents. The 0.5 s, -50 mV stimulation was used as leak current detection.

Recording electrodes were prepared from capillary glass tubes using a microelectrode puller. An electrode filled with intracellular liquid was mounted on the electrode holder. Under an inverted microscope, a microelectrode manipulator was used to immerse the electrode in extracellular liquid, and the electrode resistance (Rpip) was recorded. The electrode was brought into contact with the cell surface, and negative pressure was applied to form a high-resistance seal (GΩ). At this time, fast capacitance compensation was performed, and the application of negative pressure was continued to break the cell membrane, forming a whole-cell recording mode. Then, slow capacitance compensation was performed, and experimental parameters such as membrane capacitance (Cm) and series resistance (Rs) were recorded. No current leakage compensation was provided.

Once the whole-cell recorded hERG current stabilized, drug administration was started. Each compound concentration was applied for 5 min (or until the current stabilized) before the next concentration was tested. Multiple concentrations were tested for each compound. The coverslip with cells applied thereon was placed in the recording chamber under the inverted microscope. Gravity perfusion was used to flow the blank control external liquid and the working solutions of the compound to be tested sequentially from low to high concentrations over the cells in the recording

chamber. A peristaltic pump was used for liquid exchange during recording. For each cell, the current detected in the compound-free external liquid was used as its control group. The test of each concentration was independently repeated three times using at least three cells. All electrophysiological tests were performed at room temperature.

6. Data analysis

$IC_{50}$ calculation and curve fitting were performed using GraphPad Prism software.

7. Test results

Table 6. $IC_{50}$ values of compounds disclosed herein for hERG potassium ion channel

| Compound | hERG $IC_{50}$($\mu$M) |
|---|---|
| Compound 23 | >10 |
| Compound 81 | >10 |
| Compound 103 | >10 |

It can be seen from the experimental results in Table 6 that the compounds of the present disclosure had no significant inhibitory effect on the hERG potassium ion channel, indicating good safety.

The above description is only for the purpose of illustrating preferred examples of the present disclosure, and is not intended to limit the scope of the present disclosure. Any modifications, equivalents, improvements, and the like made without departing from the spirit and principle of the present disclosure should be included in the protection scope of the present disclosure.

## Claims

**1.** A compound represented by general formula (A') or a pharmaceutically acceptable salt, a stereoisomer or a deuteride thereof:

$$Y\text{-}W\text{-}R_3 \qquad (A')$$

wherein W is selected from

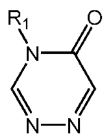 , 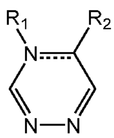 , 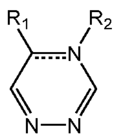

, and

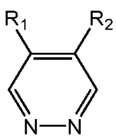 ;

- - - - - is selected from a single bond and a double bond;

$R_1$ is independently selected from hydrogen, hydroxy, amino, carboxyl, cyano, nitro, halogen, $C_{1-6}$ alkyl, halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, $-N(C_{1-6}\ alkyl)_2$, and $-S-C_{1-6}$ alkyl, or is absent;

$R_2$ is independently selected from hydrogen, hydroxy, amino, carboxyl, cyano, nitro, halogen, $C_{1-6}$ alkyl,

halogenated $C_{1-6}$ alkyl, $C_{1-6}$ alkoxy, halogenated $C_{1-6}$ alkoxy, $C_{2-6}$ alkenyl, $C_{2-6}$ alkynyl, 3-7 membered heterocyclyl, 3-7 membered cycloalkyl, aryl, 5-7 membered heteroaryl, -N($C_{1-6}$ alkyl)$_2$, and -S-$C_{1-6}$ alkyl, or is absent;

$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl;

or

$R_1$ and $R_2$, together with the C or N atom to which they are attached, form a 5-12 membered ring A, wherein the 5-12 membered ring A is optionally substituted with 1-4 substituents selected from hydroxy, amino, carboxyl, cyano, nitro, halogen, carbonyl, oxo, $C_{1-6}$ alkyl, -NH-$C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, 5-7 membered heteroaryl, $C_{1-6}$ alkylsulfonyl, and -N($C_{1-6}$ alkyl)$_2$; the 5-12 membered ring is selected from 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocycle, aryl, 5-12 membered heteroaryl, 8-12 membered fused heteroaryl, and 5-7 membered heteroaryl;

$R_3$ is selected from -($C_{1-6}$ alkylene)$_{0-2}$-$NR_4R_5$, -($C_{1-6}$ alkylene)$_{0-2}$-$NR_4$-$COR_5$, -($C_{1-6}$ alkylene)$_{0-2}$-CO-$NR_4$-$R_5$, and -($C_{1-6}$ alkylene)$_{0-2}$-$NR_4$-$C_{1-6}$ alkylene-$R_5$;

$R_4$ is selected from hydrogen and $C_{1-6}$ alkyl;

$R_5$ is selected from 3-7 membered heterocyclyl, 3-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, $C_{1-6}$ alkylcarbonyl, ureido $C_{1-6}$ alkyl, hydroxy $C_{1-6}$ alkyl, hydrazino, and $C_{1-6}$ alkylsulfonyl $C_{1-6}$ alkyl;

the substituent on $R_5$, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, 3-6 membered cycloalkyl, and $C_{1-6}$ alkylsulfonyl;

Y is selected from aryl, 5-14 membered heteroaryl, 3-14 membered heterocyclyl, and 3-12 membered cycloalkyl; Y is substituted with 1-2 substituents selected from $C_{2-6}$ alkenyl and $C_{2-6}$ alkynyl, and can be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, -N($C_{1-6}$ alkyl)$_2$, and -S-$C_{1-6}$ alkyl;

the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;

the substituent on Y, which is selected from $C_{2-6}$ alkenyl and $C_{2-6}$ alkynyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

when W is selected from

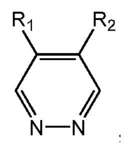

, $R_1$ and $R_2$, together with the C atom to which they are attached, do not form a ring.

**2.** The compound or the pharmaceutically acceptable salt, the stereoisomer or the deuteride thereof according to claim 1,
wherein

W is selected from

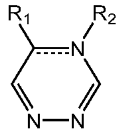

;

$R_1$ is selected from hydrogen, hydroxy, amino, carboxyl, cyano, nitro, halogen, carbonyl, halogenated $C_{1-6}$ alkyl, halogenated $C_{1-6}$ alkoxy, $C_{2-6}$ alkenyl, $C_{2-6}$ alkynyl, 3-7 membered heterocyclyl, 4-7 membered cycloalkyl, aryl, and 5-7 membered heteroaryl.

**3.** The compound or the pharmaceutically acceptable salt, the stereoisomer or the deuteride thereof according to claim 1,
wherein

W is selected from

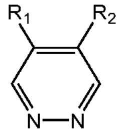

;

Y is selected from aryl, 5-14 membered heteroaryl, 3-14 membered heterocyclyl, and 3-12 membered cycloalkyl;
Y is substituted with $C_{2-6}$ alkynyl, and can be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, $-N(C_{1-6}$ alkyl$)_2$, and $-S-C_{1-6}$ alkyl;
the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;
the substituent on Y, which is selected from $C_{2-6}$ alkynyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;
$R_5$ is optionally substituted with a substituent selected from hydroxy $C_{1-6}$ alkyl, $C_{1-6}$ alkoxy $C_{1-6}$ alkyl, hydrazino, and ureido $C_{1-6}$ alkyl.

**4.** The compound or the pharmaceutically acceptable salt, the stereoisomer or the deuteride thereof according to claim 1,
wherein

W is selected from

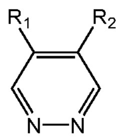

;

Y is selected from aryl, 5-14 membered heteroaryl, 3-14 membered heterocyclyl, and 3-12 membered cycloalkyl;
Y is substituted with $C_{2-6}$ alkenyl, and can be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, $-N(C_{1-6}$ alkyl$)_2$, and $-S-C_{1-6}$ alkyl;
the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;
the substituent on Y, which is selected from $C_{2-6}$ alkenyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl;

$R_5$ is selected from 3-7 membered heterocyclyl, 5-7 membered cycloalkyl, aryl, and 5-7 membered heteroaryl; $R_5$ is optionally substituted with 1-4 substituents selected from halogen, cyano, amino, hydroxy, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{2-6}$ alkenylcarbonyl, sulfonyl, $C_{1-6}$ alkylcarbonyl, ureido $C_{1-6}$ alkyl, hydroxy $C_{1-6}$ alkyl, hydrazino, and $C_{1-6}$ alkylsulfonyl $C_{1-6}$ alkyl.

**5.** The compound or the pharmaceutically acceptable salt, the stereoisomer or the deuteride thereof according to claim 1,
wherein

W is selected from

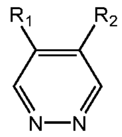 ;

at least one of $R_1$ and $R_2$ is selected from $C_{2-6}$ alkenyl, $C_{2-6}$ alkynyl, and cyano;
$R_1$ and $R_2$ are each optionally substituted with 1-3 substituents selected from hydroxy, amino, carboxyl, cyano, nitro, halogen, carbonyl, $C_{1-6}$ alkyl, halogenated $C_{1-6}$ alkyl, $C_{1-6}$ alkoxy, halogenated $C_{1-6}$ alkoxy, 3-7 membered heterocyclyl, 3-7 membered cycloalkyl, aryl, and 5-7 membered heteroaryl.

**6.** The compound or the pharmaceutically acceptable salt, the stereoisomer or the deuteride thereof according to claim 1,
wherein

W is selected from

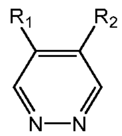 ;

$R_1$ is selected from hydrogen;
$R_2$ is selected from hydrogen.

**7.** The compound or the pharmaceutically acceptable salt, the stereoisomer or the deuteride thereof according to claim 1,
wherein

W is selected from

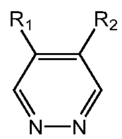 ;

Y is selected from aryl, 5-14 membered heteroaryl, 3-14 membered heterocyclyl, and 3-12 membered cycloalkyl;
Y is substituted with propynyl, and can be further optionally substituted with 1-2 substituents selected from halogen, cyano, amino, hydroxy, carbonyl, $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, $C_{1-6}$ alkylamino, $C_{1-6}$ alkylcarbonylamino, $C_{1-6}$ alkylsulfonyl, aminocarbonyl, $C_{1-6}$ alkylaminocarbonyl, sulfonyl, $-N(C_{1-6}\ alkyl)_2$, and $-S-C_{1-6}$ alkyl;
the substituent on Y, which is selected from $C_{1-6}$ alkyl, halogenated $C_{1-6}$ alkyl, $C_{1-6}$ alkoxy, 3-7 membered heterocyclyl, 3-7 membered cycloalkyl, aryl, 5-7 membered heteroaryl, and sulfonyl, is optionally substituted with 1-3 substituents selected from halogen, cyano, amino, hydroxy, carbonyl, $C_{1-6}$ alkyl, and 3-6 membered cycloalkyl;

the substituent on Y, which is selected from propynyl, is optionally substituted with 1-3 substituents selected from halogen, cyano, amino, hydroxy, carbonyl, $C_{1-6}$ alkyl, 3-6 membered cycloalkyl, and halogenated $C_{1-6}$ alkyl; $R_5$ is selected from 3-7 membered cycloalkyl.

**8.** The compound or the pharmaceutically acceptable salt, the stereoisomer or the deuteride thereof according to claim 1,
wherein
$R_5$ is selected from 3-7 membered heterocyclyl, 3-7 membered cycloalkyl, aryl, and 5-7 membered heteroaryl; $R_5$ is substituted with 1-2 substituents selected from deuterium, deuterated $C_{1-6}$ alkyl, ethyl, cyclopropyl, halogen, and halogenated $C_{1-6}$ alkyl.

**9.** The compound or the pharmaceutically acceptable salt, the stereoisomer or the deuteride thereof according to claim 1,
wherein

W is selected from

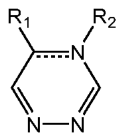;

$R_5$ is selected from 3-7 membered heterocyclyl, 3-7 membered cycloalkyl, aryl, and 5-7 membered heteroaryl; $R_5$ is substituted with 1-2 substituents selected from deuterium and hydroxy $C_{1-6}$ alkyl.

**10.** The compound or the pharmaceutically acceptable salt, the stereoisomer or the deuteride thereof according to claim 1,
wherein

Y is selected from aryl;
Y is substituted with 1-2 substituents selected from $C_{2-6}$ alkenyl and $C_{2-6}$ alkynyl, and can be further substituted with 1-2 substituents selected from hydroxy, halogen, and halogenated $C_{1-6}$ alkyl.

**11.** The compound or the pharmaceutically acceptable salt, the stereoisomer or the deuteride thereof according to claim 1,
wherein

W is selected from

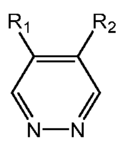;

when $R_2$ is hydrogen, $R_1$ is not selected from methyl and cyclopropyl.

**12.** The compound or the pharmaceutically acceptable salt, the stereoisomer or the deuteride thereof according to claim 3,
wherein $R_5$ is optionally substituted with a substituent selected from hydroxy $C_{1-6}$ alkyl and $C_{1-6}$ alkoxy $C_{1-6}$ alkyl, but the following compounds are excluded:

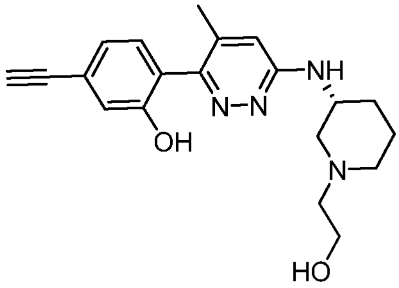 and 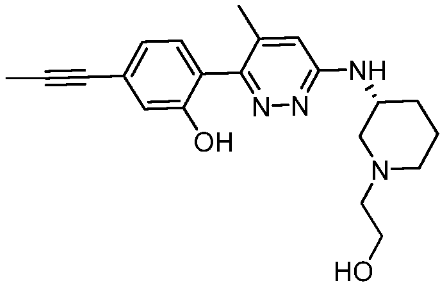 .

**13.** The compound or the pharmaceutically acceptable salt, the stereoisomer or the deuteride thereof according to claim 1,
wherein

W is selected from

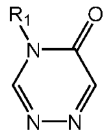 ;

$R_1$ is independently selected from hydrogen, halogen, $C_{1-6}$ alkyl, halogenated $C_{1-6}$ alkyl, and 3-7 membered cycloalkyl;
$R_3$ is selected from $-(C_{1-6}$ alkylene$)_{0-2}-NR_4R_5$;
$R_4$ is selected from hydrogen and $C_{1-6}$ alkyl;
$R_5$ is selected from 3-7 membered heterocyclyl and 3-7 membered cycloalkyl; $R_5$ is optionally substituted with 1-4 substituents selected from deuterated $C_{1-6}$ alkyl, $C_{1-6}$ alkyl, halogenated $C_{1-6}$ alkyl, 3-7 membered cycloalkyl, hydroxy, and hydroxy $C_{1-6}$ alkyl;
Y is selected from aryl;
Y is substituted with $C_{2-6}$ alkynyl, and can be further optionally substituted with 1-2 substituents selected from hydroxy, $C_{1-6}$ alkyl, halogenated $C_{1-6}$ alkyl, and 3-7 membered cycloalkyl;
the $C_{2-6}$ alkynyl substituent is optionally substituted with a substituent selected from halogen, $C_{1-6}$ alkyl, 3-6 membered cycloalkyl, and halogenated $C_{1-6}$ alkyl.

**14.** The compound or the pharmaceutically acceptable salt, the stereoisomer or the deuteride thereof according to claim 12 or 13, wherein

$R_1$ and $R_2$ are selected from hydrogen, halogen, $C_{1-6}$ alkyl, trifluoromethyl, difluoromethyl, cyclopropyl, and cyclobutyl;
$R_3$ is selected from $-NR_4R_5$;
$R_4$ is selected from hydrogen and methyl;
$R_5$ is selected from piperidine, cyclohexyl, cyclopentyl, and cyclobutyl;
Y is selected from a phenyl;
Y is substituted with a substituent selected from ethynyl and propynyl, and can be further optionally substituted with 1-2 substituents selected from hydroxy, $C_{1-6}$ alkyl, trifluoromethyl, difluoromethyl, and cyclopropyl;
the ethynyl and propynyl substituents are optionally substituted with a substituent selected from halogen, $C_{1-6}$ alkyl, cyclopropyl, trifluoromethyl, and difluoromethyl.

**15.** The compound or the pharmaceutically acceptable salt, the stereoisomer or the deuteride thereof according to claim 14, wherein

$R_1$ and $R_2$ are selected from methyl, trifluoromethyl, difluoromethyl, and cyclopropyl;
$R_3$ is selected from $-NR_4R_5$;
$R_4$ is selected from hydrogen;
$R_5$ is selected from piperidinyl;
Y is selected from a phenyl;
Y is substituted with a substituent selected from ethynyl and propynyl, and can be further optionally substituted

with 1-2 substituents selected from hydroxy, methyl, trifluoromethyl, difluoromethyl, and cyclopropyl;
the ethynyl and propynyl substituents are optionally substituted with a substituent selected from fluorine, methyl, cyclopropyl, trifluoromethyl, and difluoromethyl;
the substituent on $R_5$ is selected from methyl, ethyl, cyclopropyl, fluorine, chlorine, bromine, difluoroethane, and hydroxyethyl.

**16.** A compound of a formula shown below or a pharmaceutically acceptable salt, a stereoisomer or a deuteride thereof:

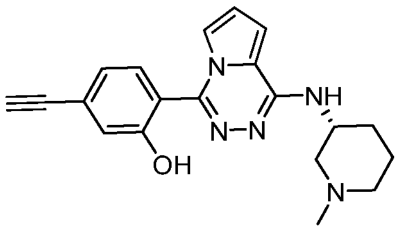
,
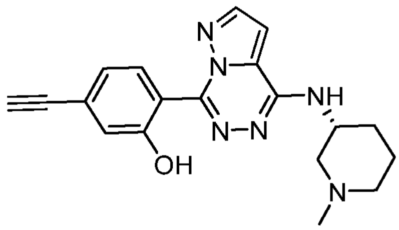
,
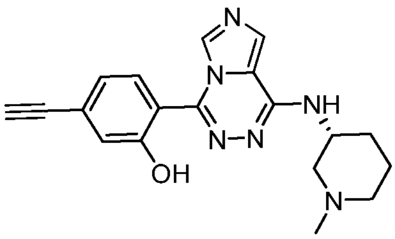
,
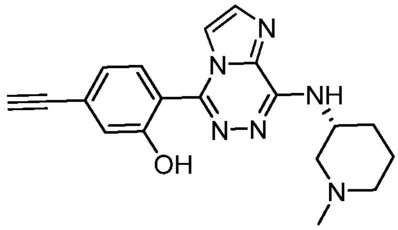
,
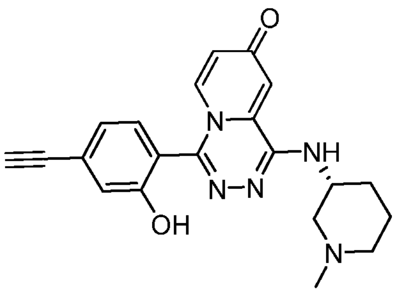
,
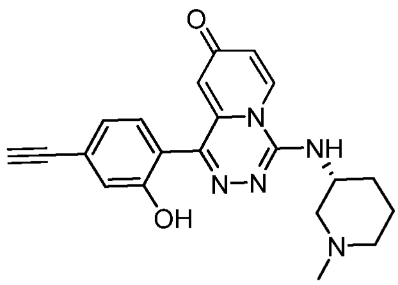
,
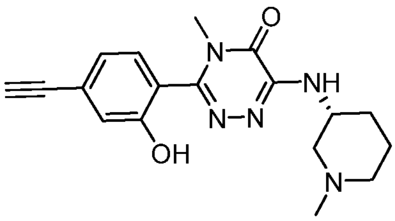
,
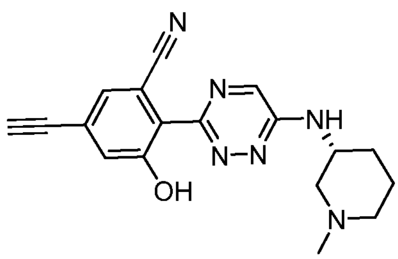
,
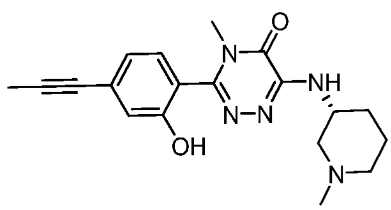
,

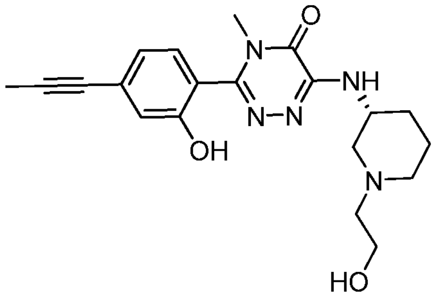
,
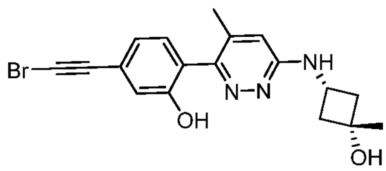
,

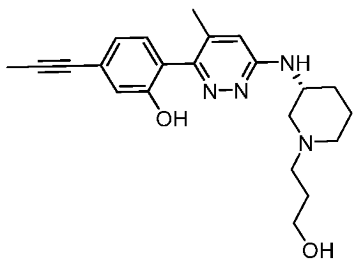
,
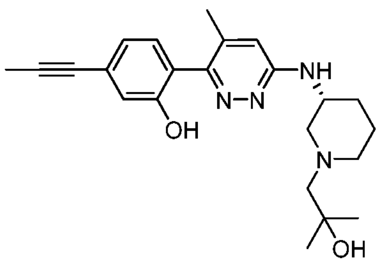
,
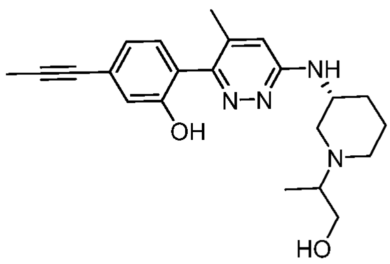
,

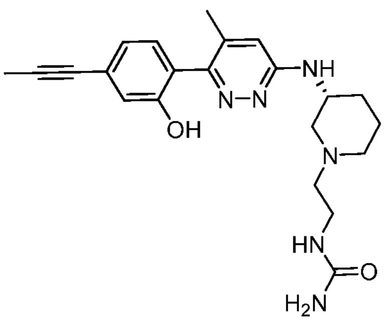
,
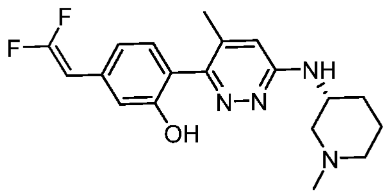
,

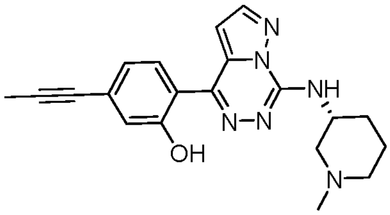
,
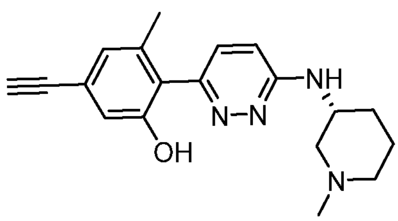
,

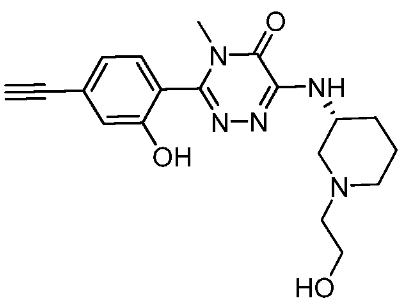
,
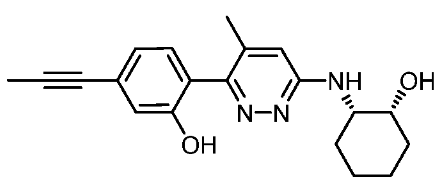
,

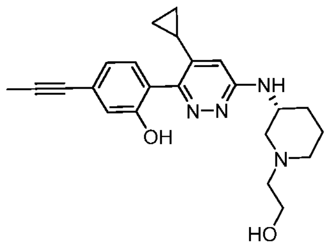,
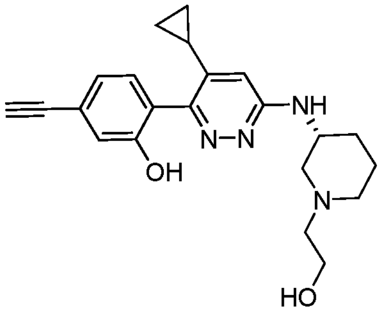,
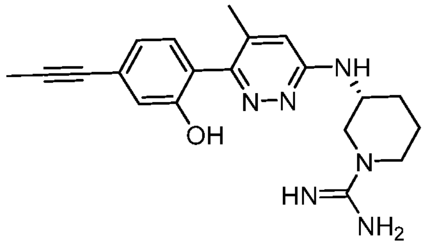,

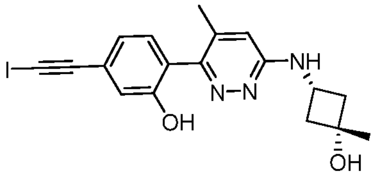,
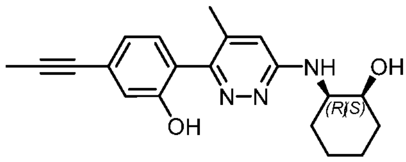,

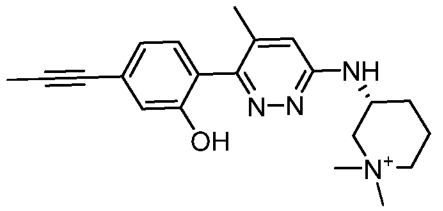,
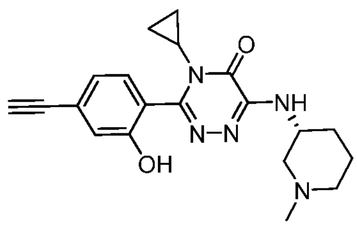,

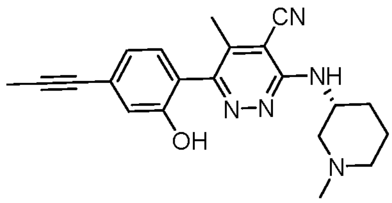,
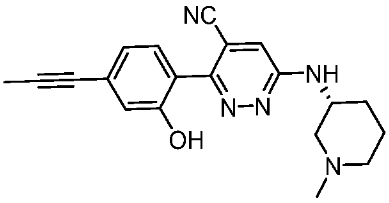,

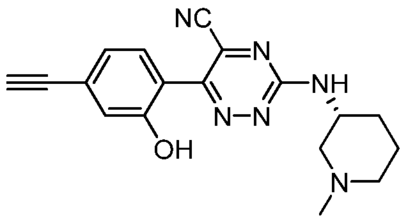,
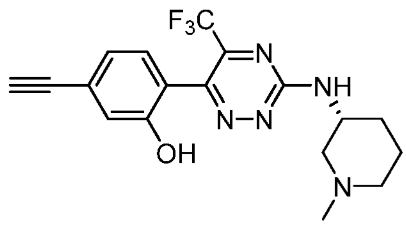,

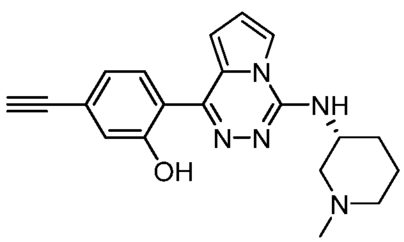,
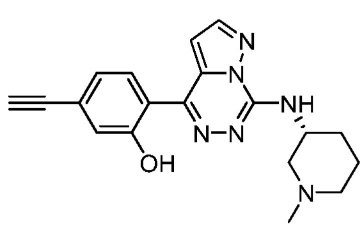,

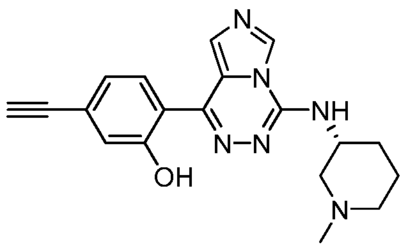

,

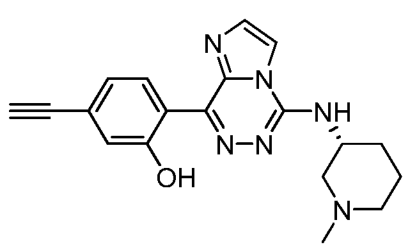

,

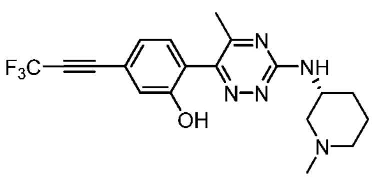

,

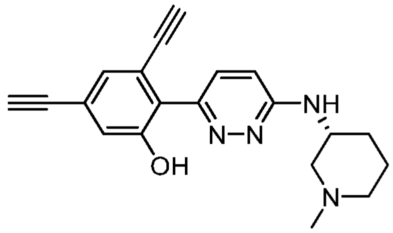

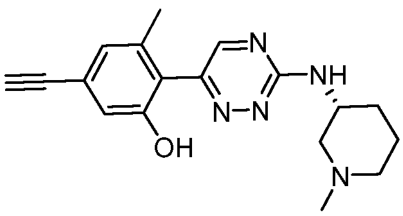

,

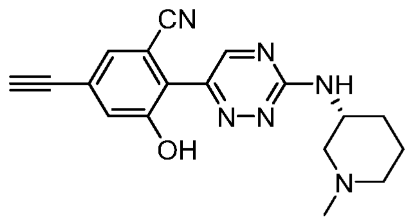

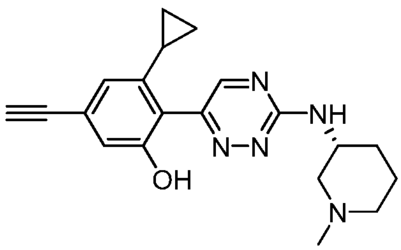

,

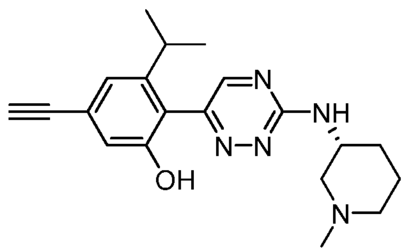

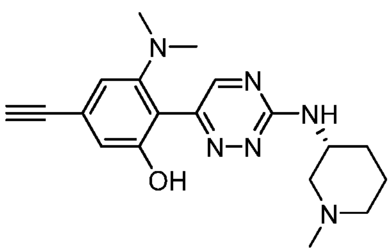

,

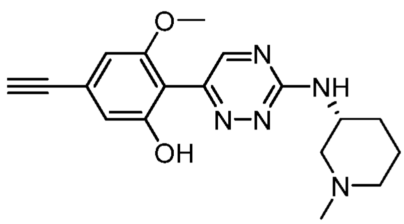

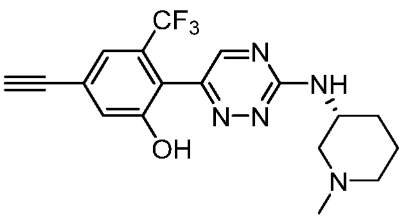

,

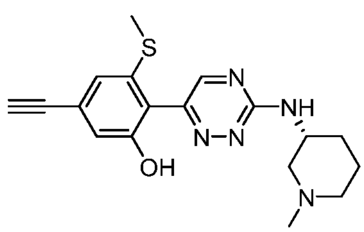

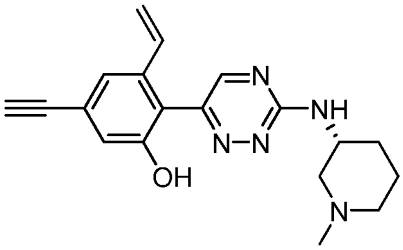,
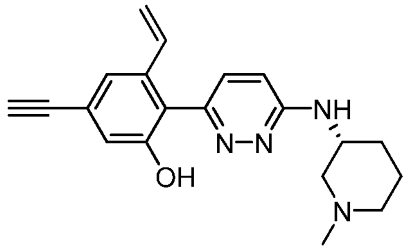

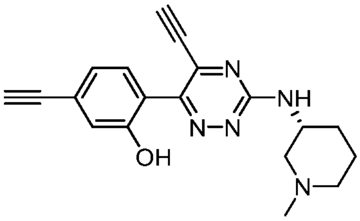,
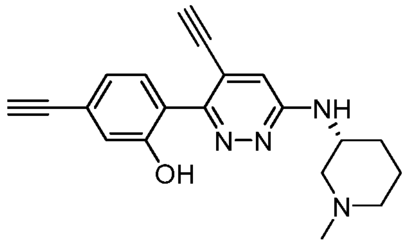

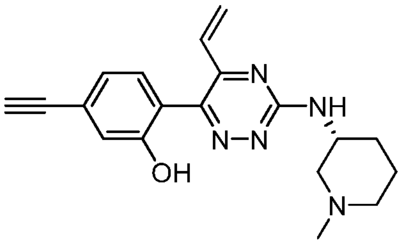,
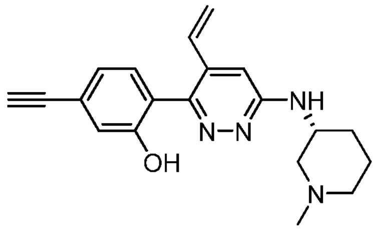,

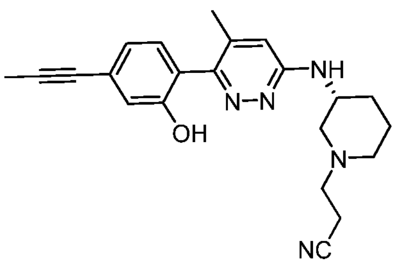,
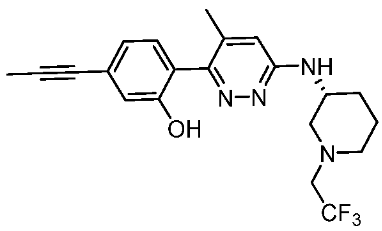,

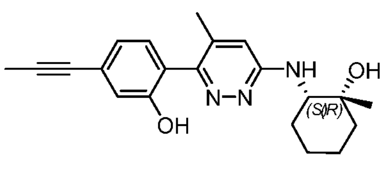,
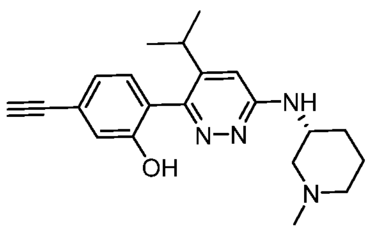,
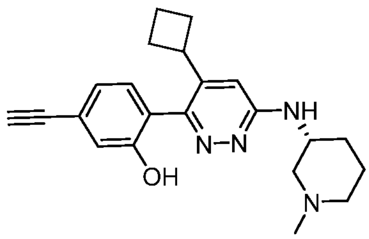,

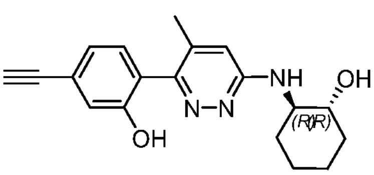,
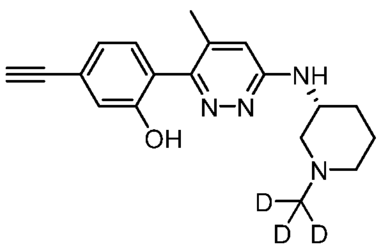,
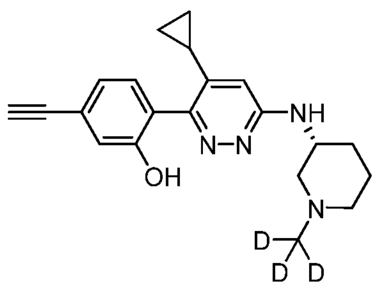,

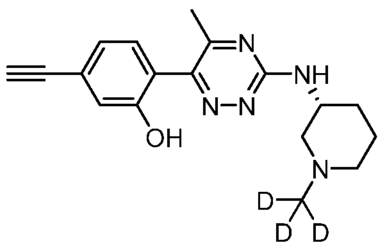 ,

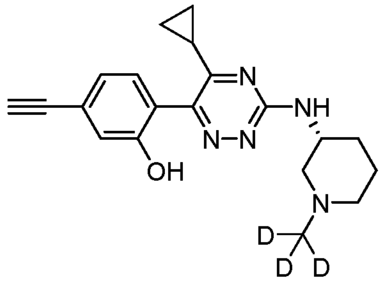 ,

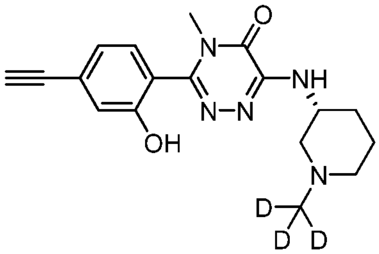 , 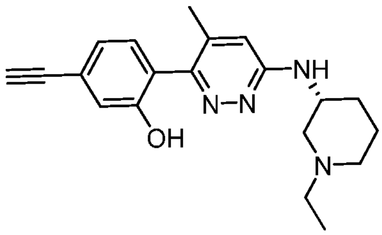 , 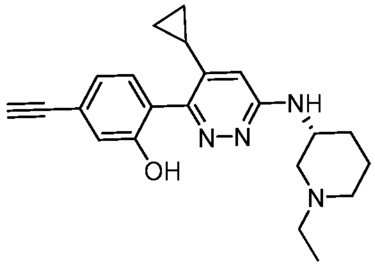 ,

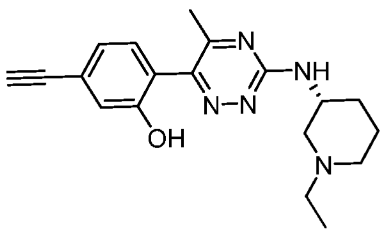 ,

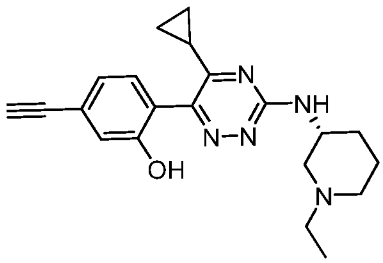 ,

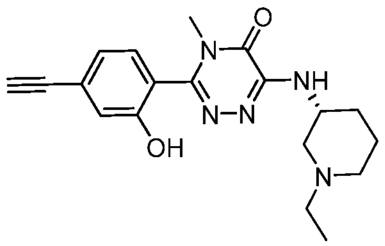 ,

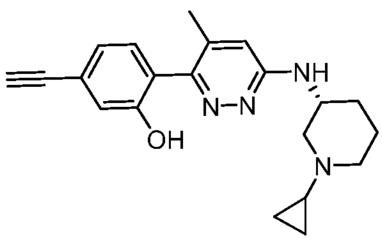 ,

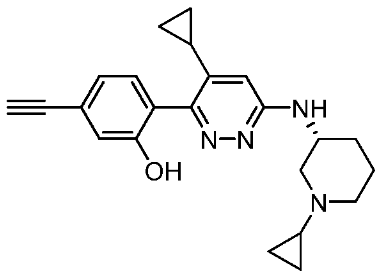 ,

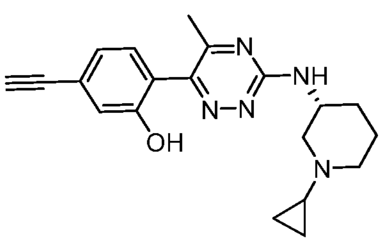 ,

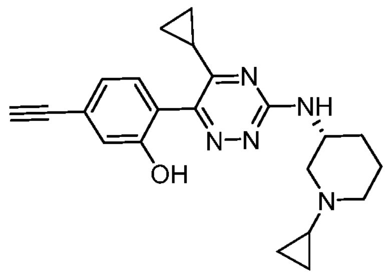
,

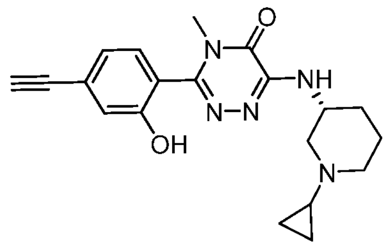
,

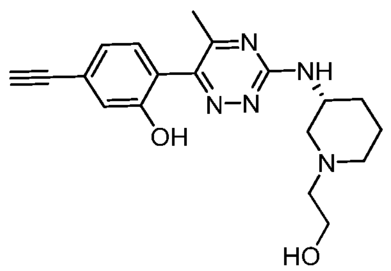
,

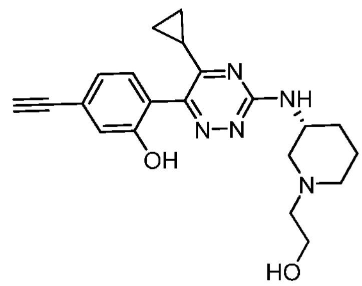
,

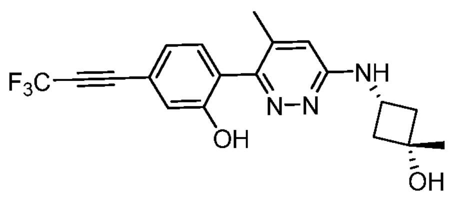
,

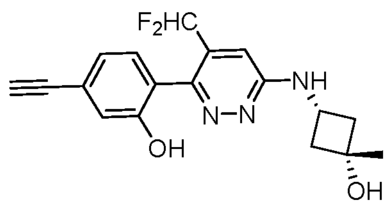
,

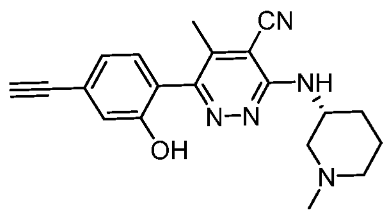
,

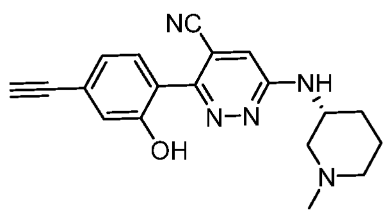
,

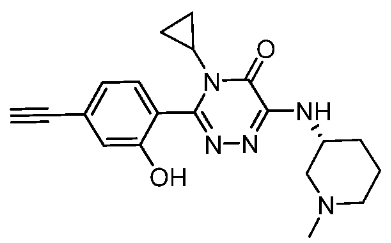
,

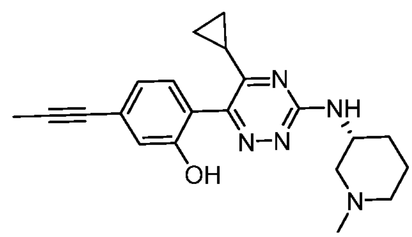
,

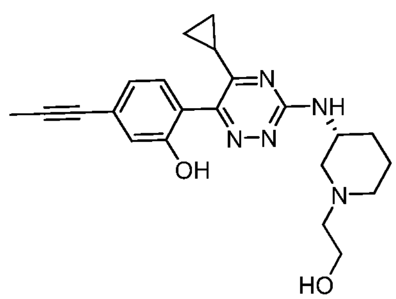
,

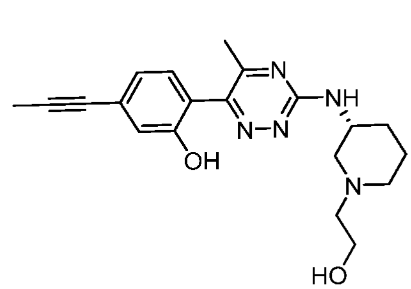
,

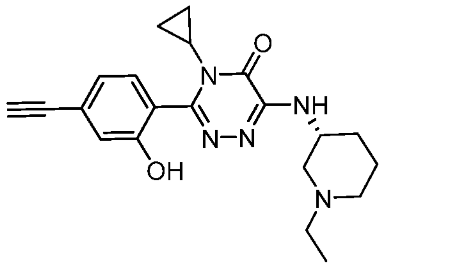
,
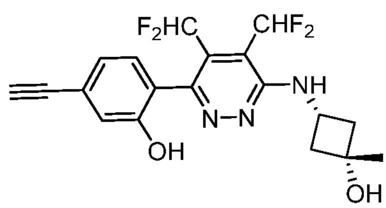
,

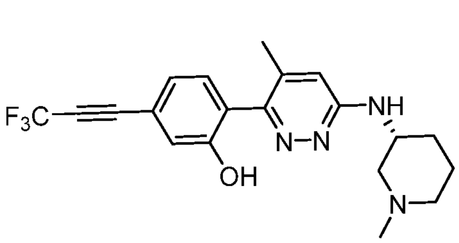
,
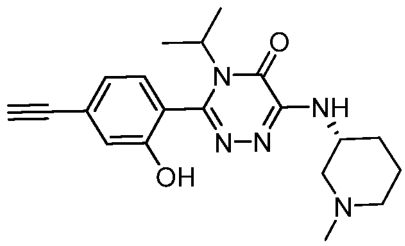
,

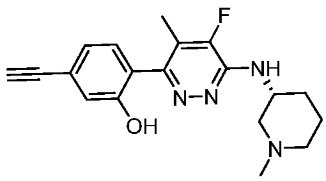
,
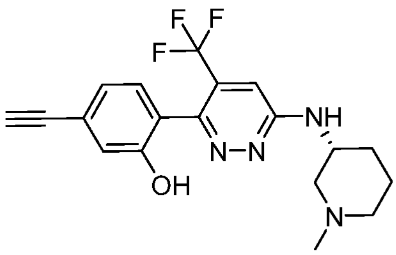
,
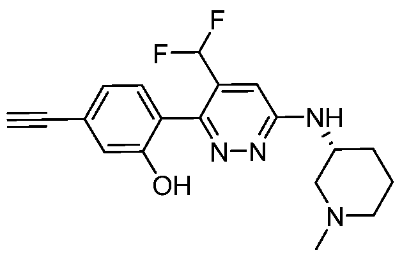
,

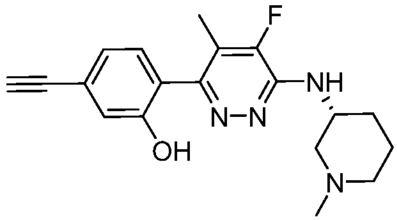
,
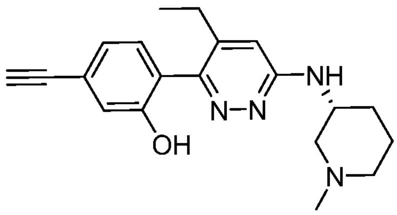
,

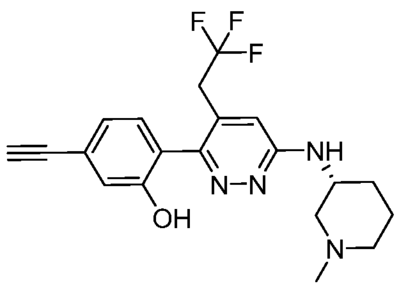
,
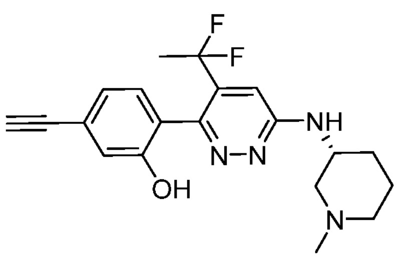
,

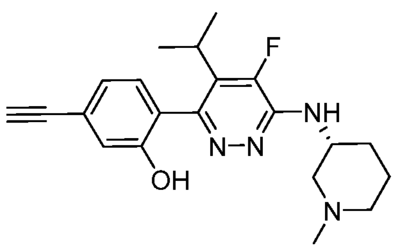
,
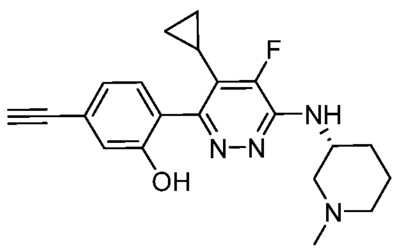
,

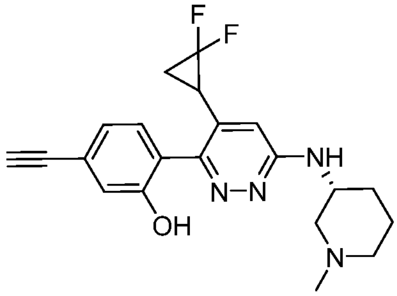

,

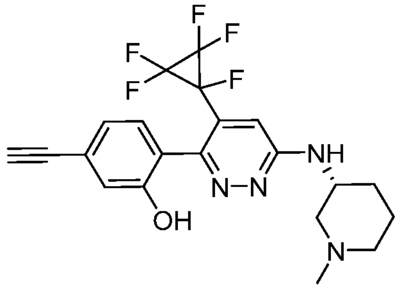

,

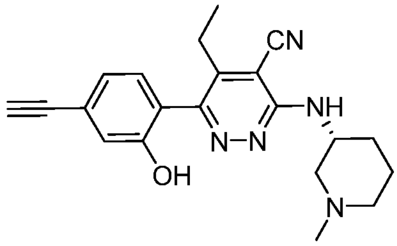

,

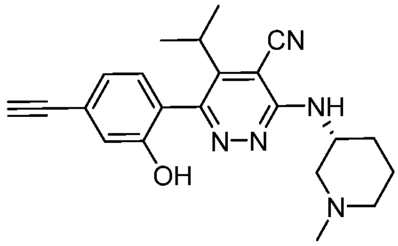

,

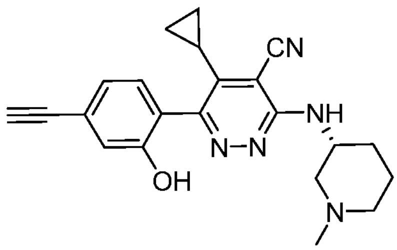

,

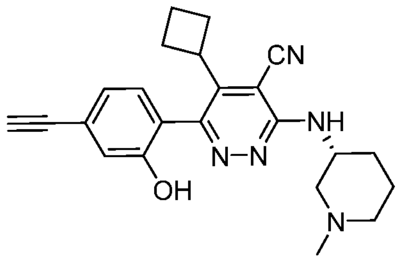

,

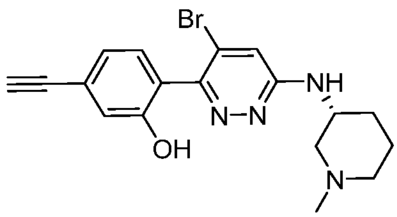

,

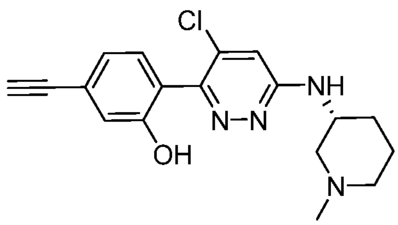

,

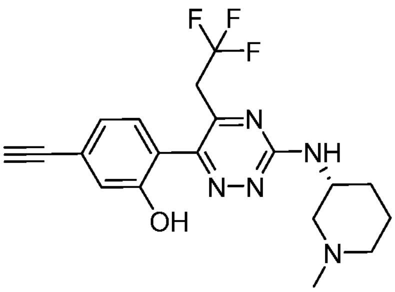

,

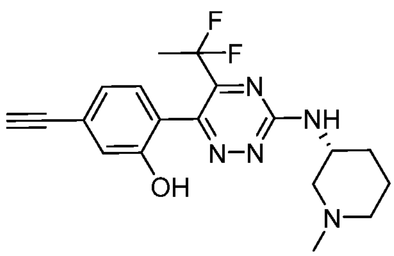

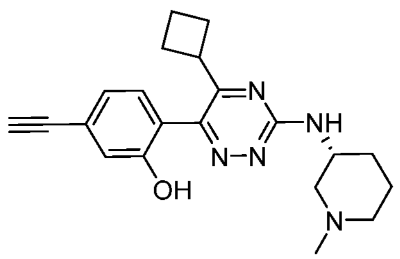

,

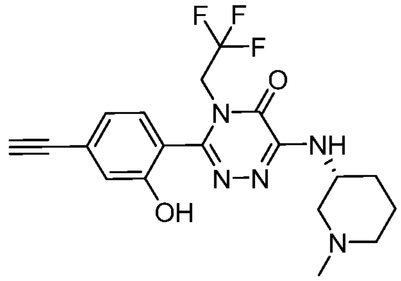

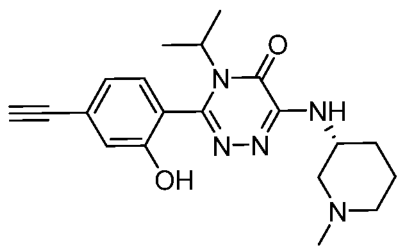

,

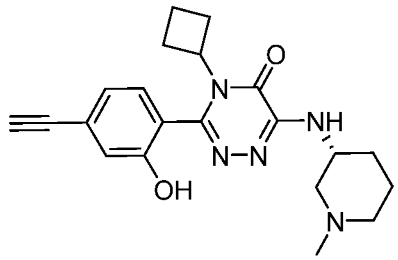

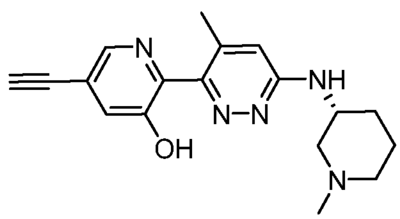

,

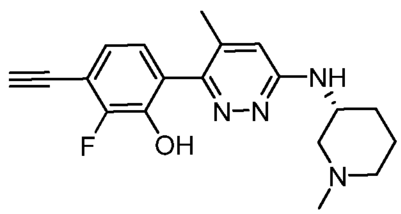

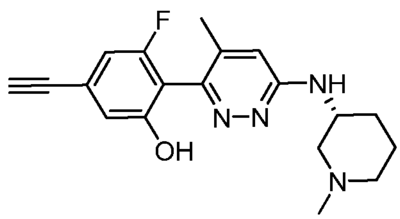

,

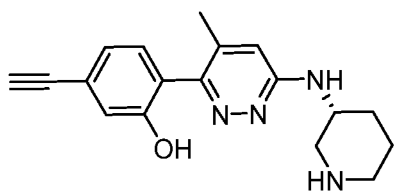

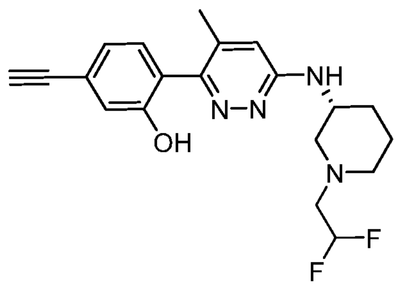

,

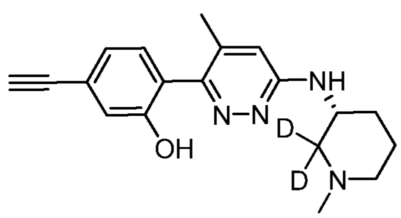

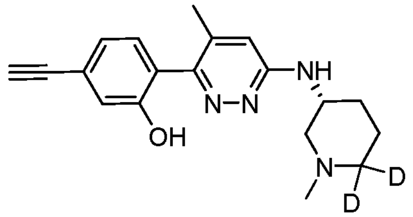

,

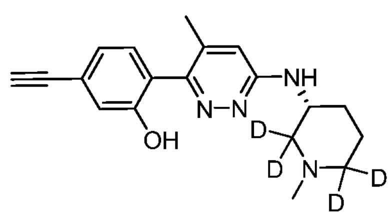

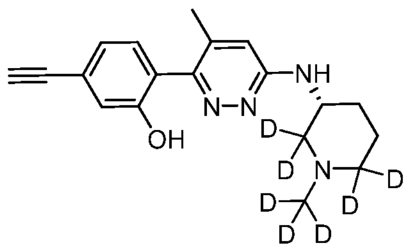

,

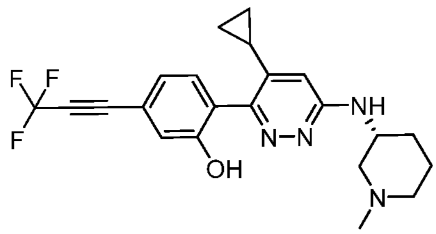

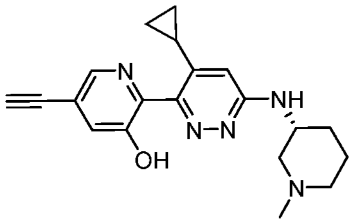

,

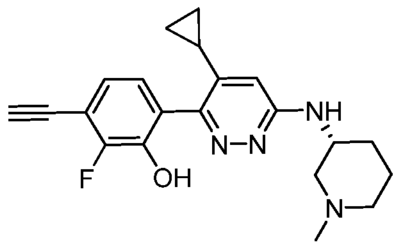

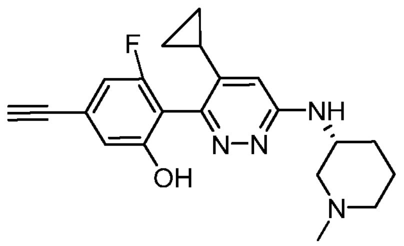

,

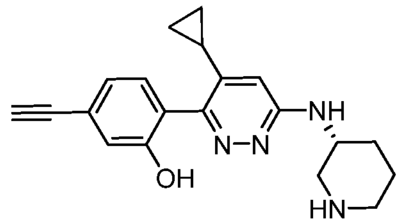

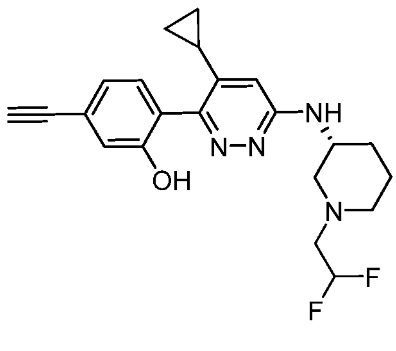

,

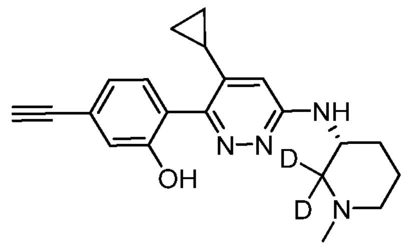

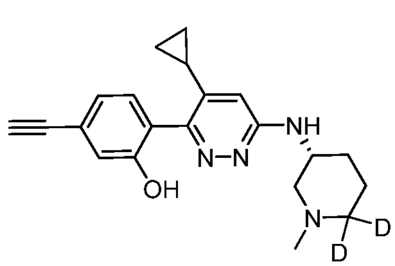

,

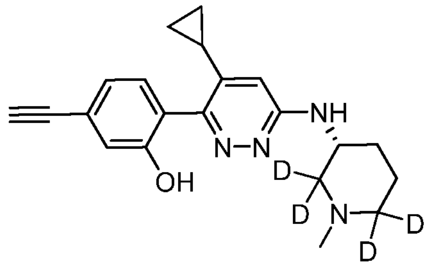

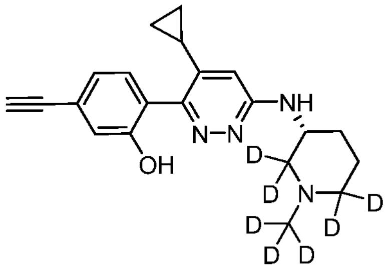

,

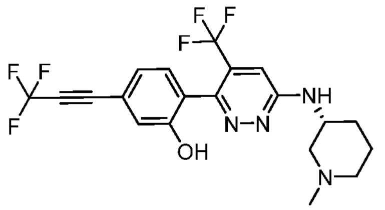

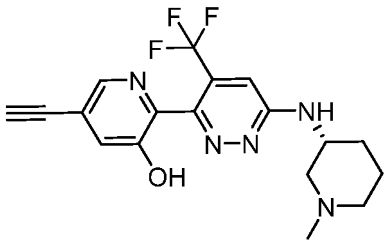

,

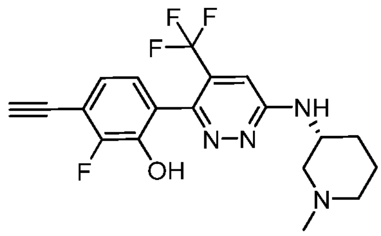

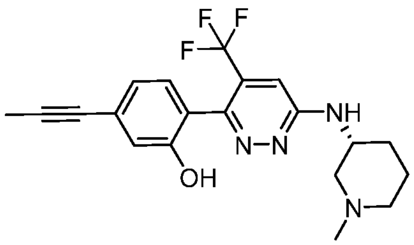

,

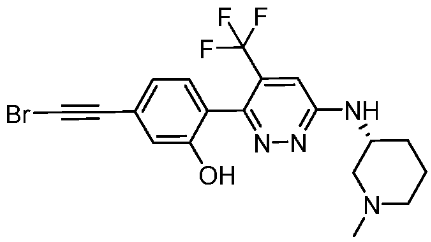

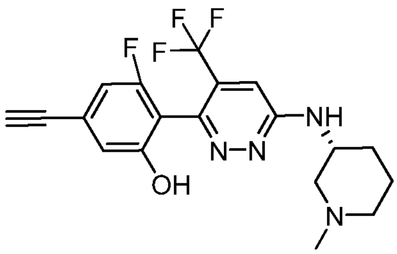

,

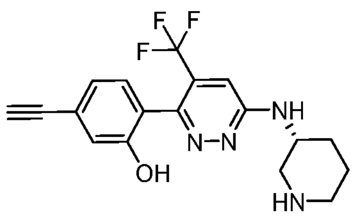

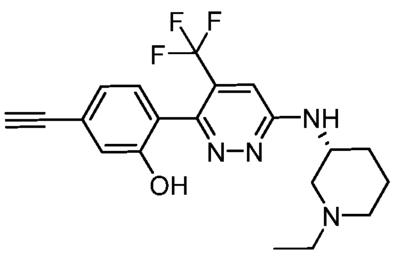

,

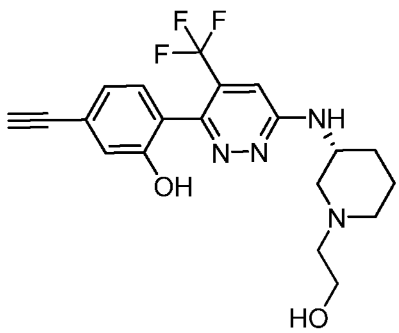

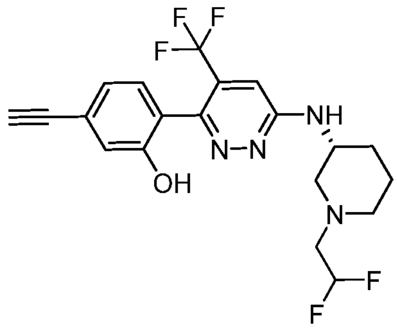

,

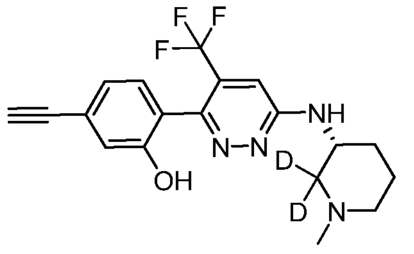

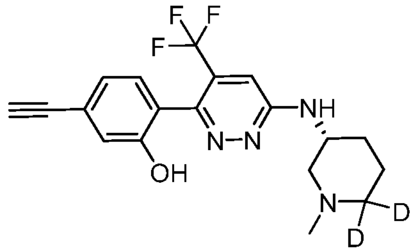

,

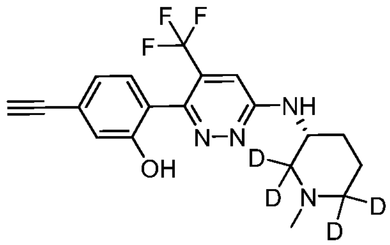

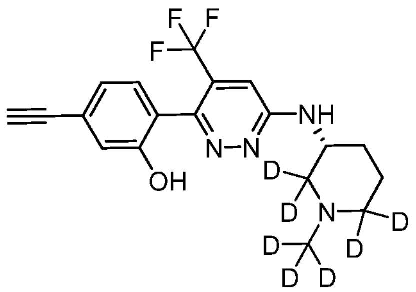

,

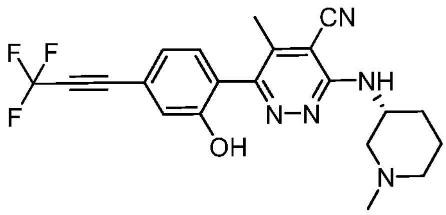

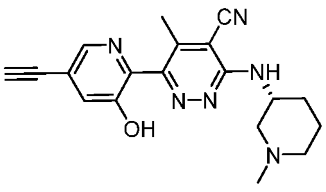

,

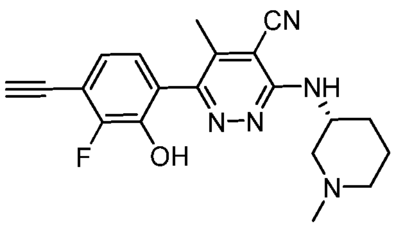

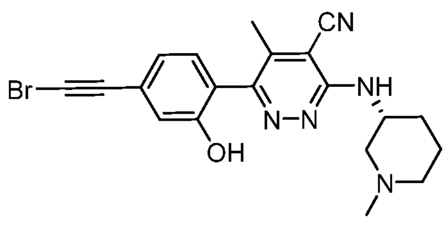

,

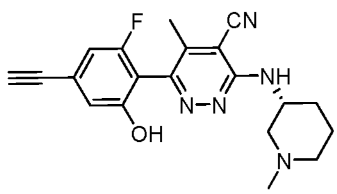

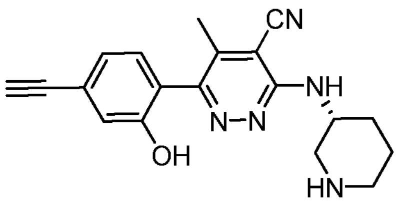

,

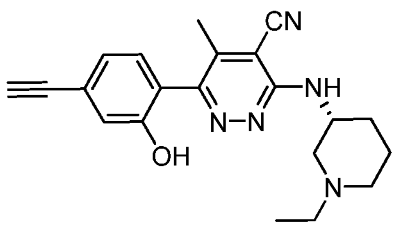

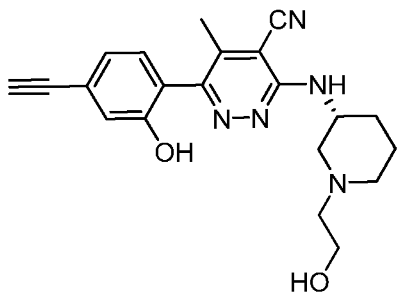

,

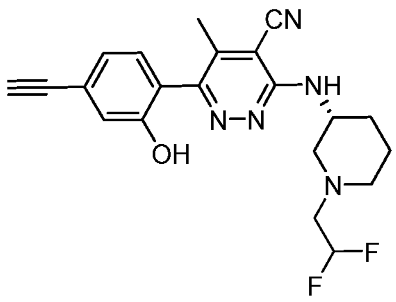

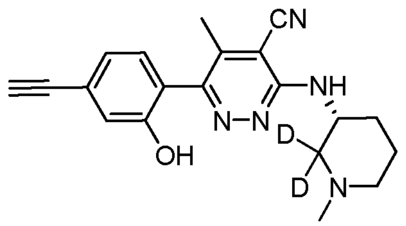

,

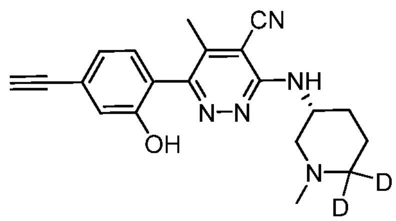

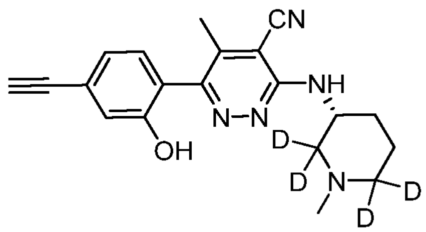

,

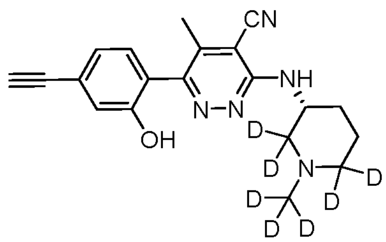

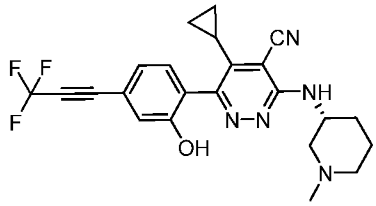

,

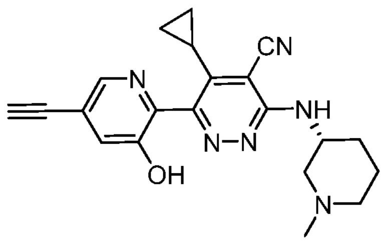

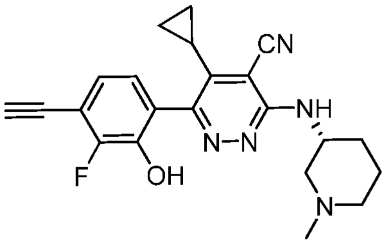

,

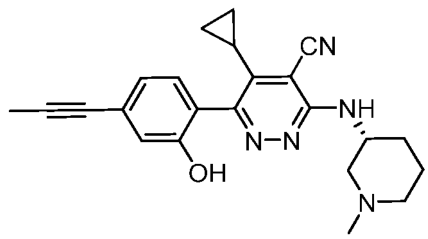

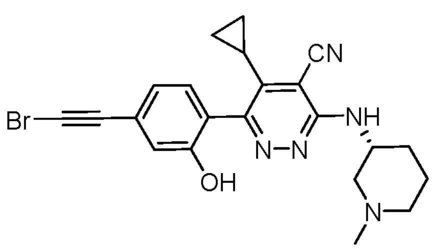

,

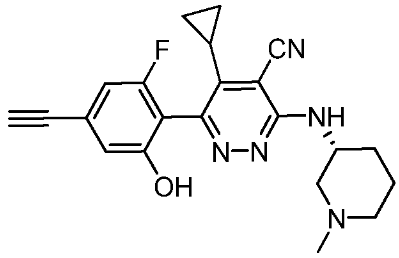

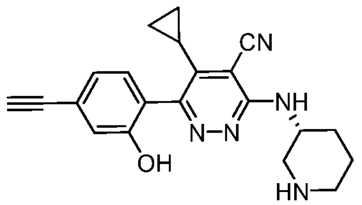

,

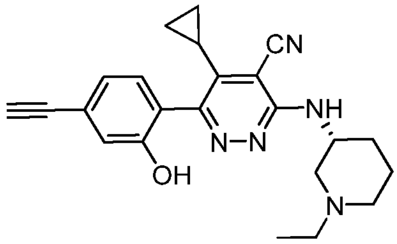

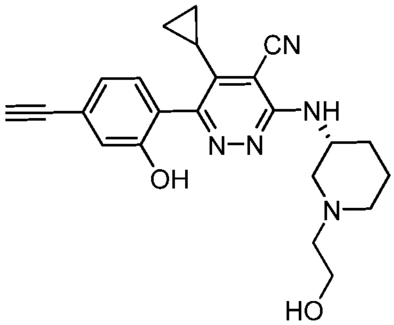

,

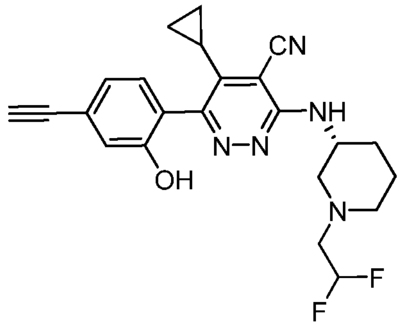

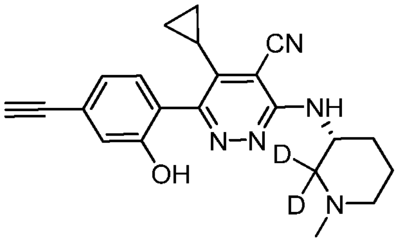

,

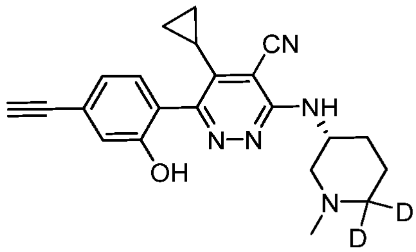

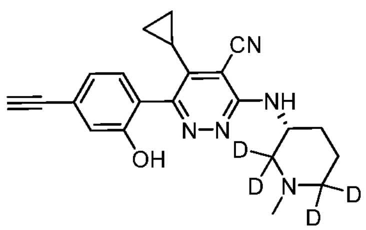

,

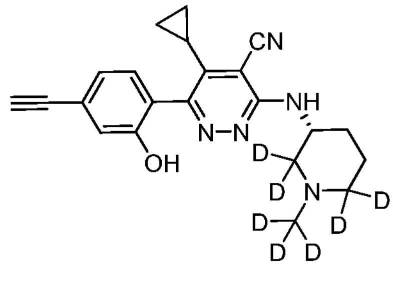

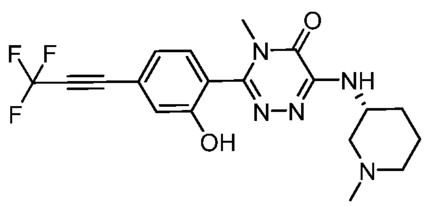

,

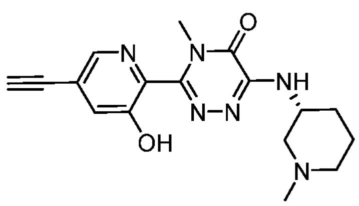

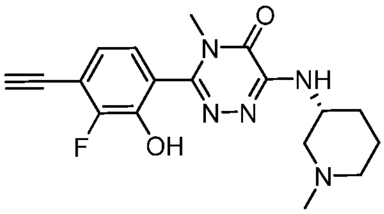

,

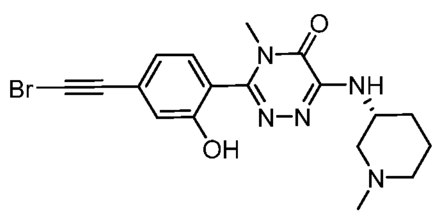

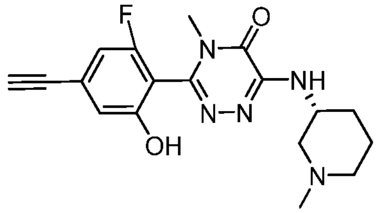

,

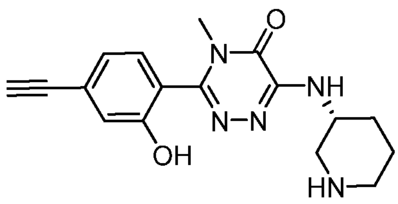

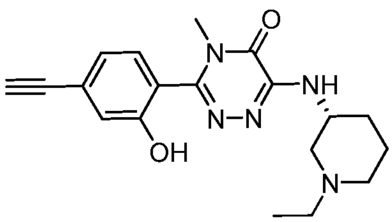

,

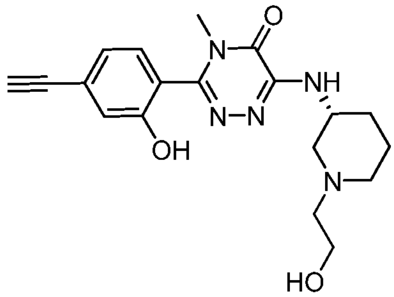

,

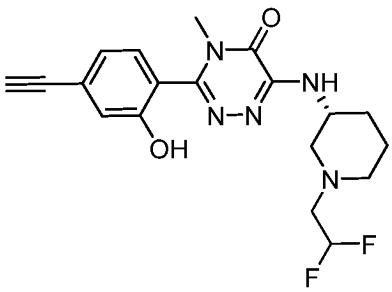

,

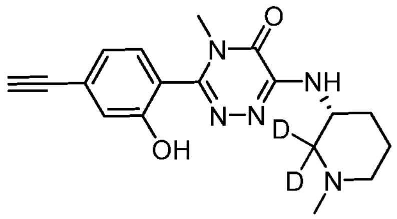

,

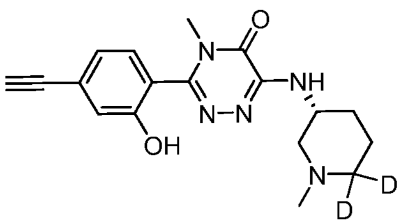

,

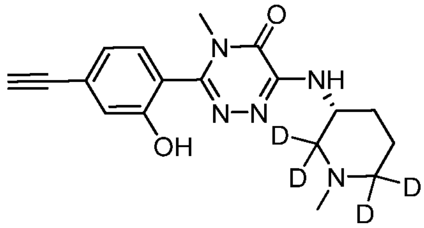

,

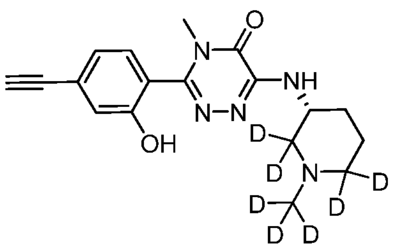

,

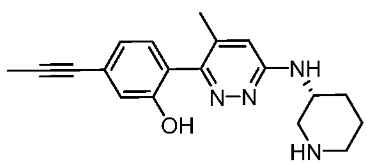

,

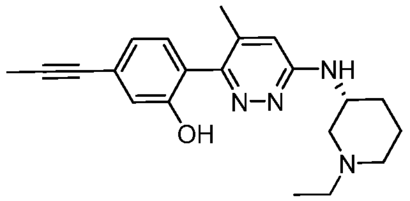,

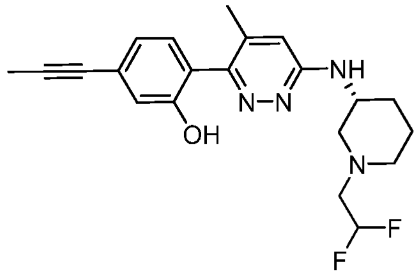,

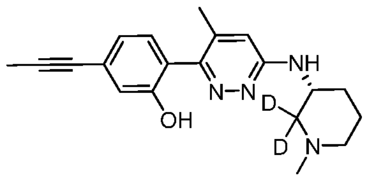,

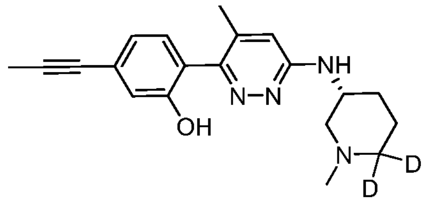,

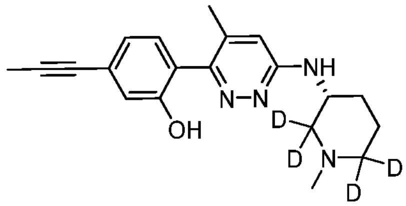,

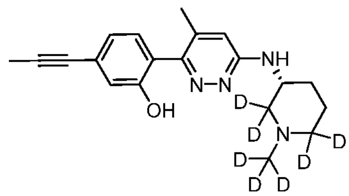

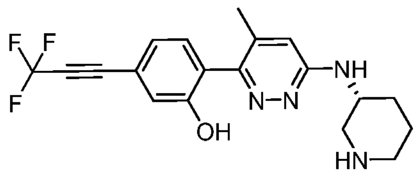,

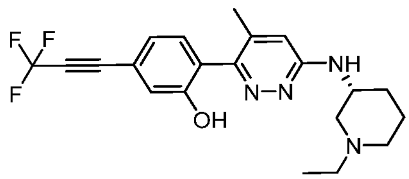

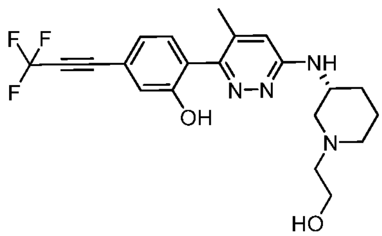,

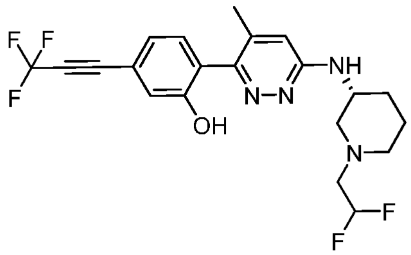

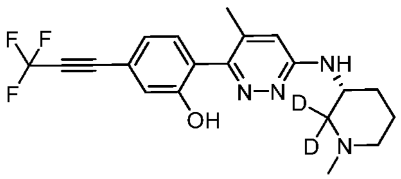,

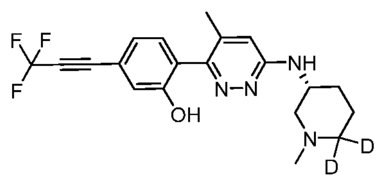

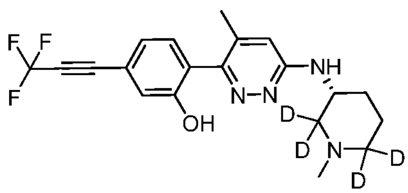

,

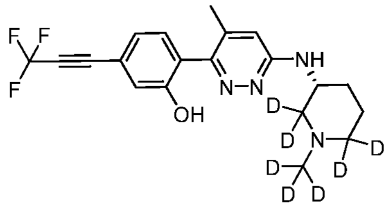

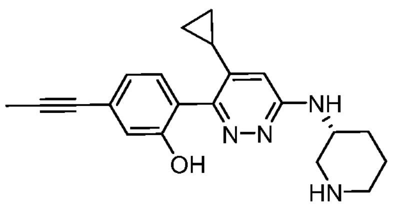

,

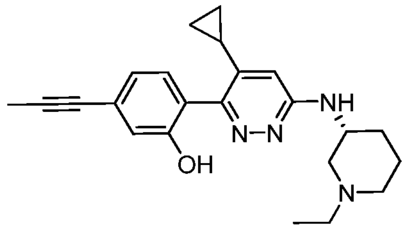

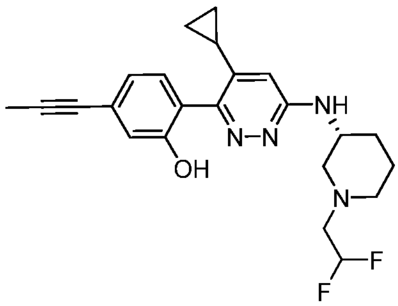

,

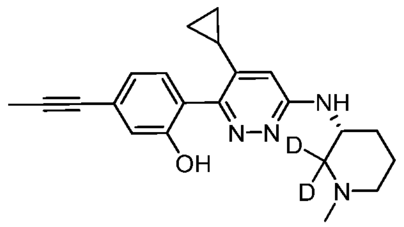

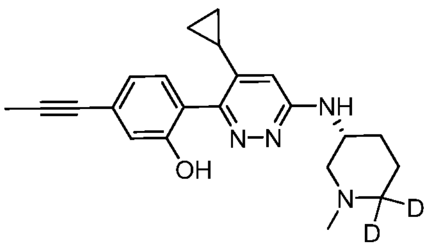

,

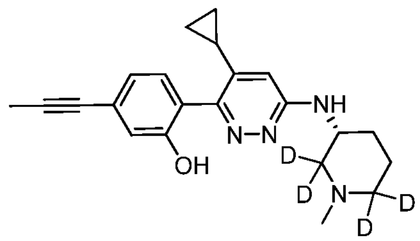

,

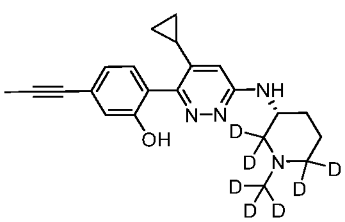

,

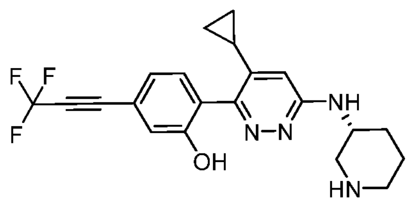

,

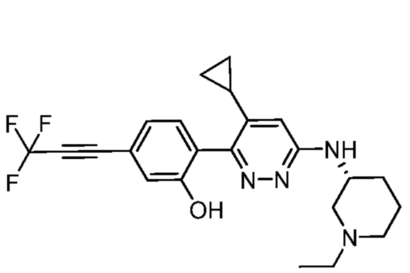

,

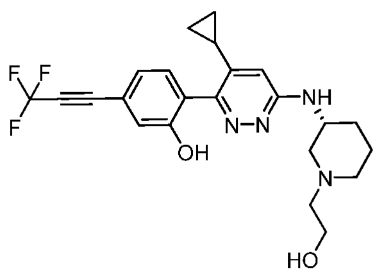

,

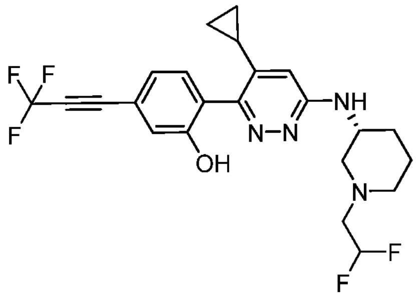,

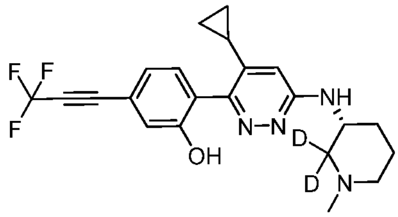,

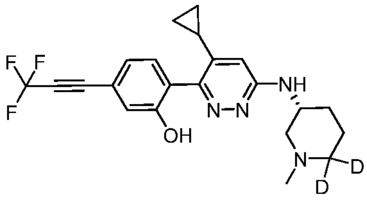,

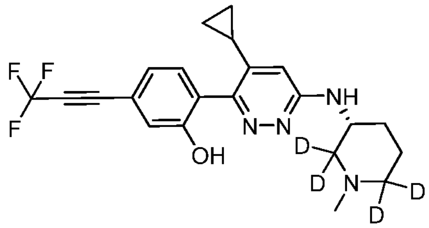,

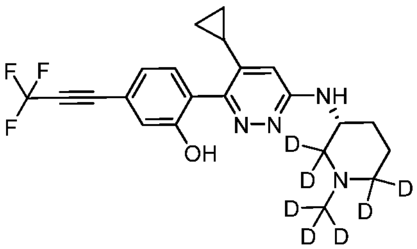,

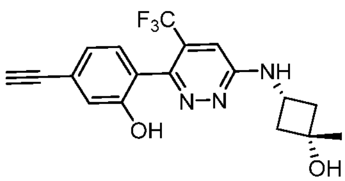,

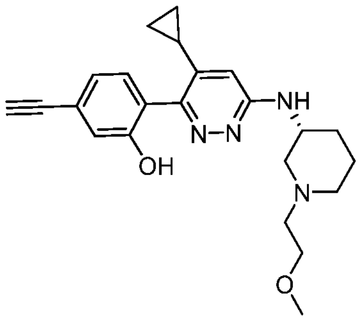.

17. A pharmaceutical composition, comprising the compound or the pharmaceutically acceptable salt, the stereoisomer or the deuteride thereof according to any one of claims 1-16 and a pharmaceutically acceptable carrier.

18. Use of the compound or the pharmaceutically acceptable salt, the stereoisomer or the deuteride thereof according to any one of claims 1-16 or the pharmaceutical composition according to claim 17 in the manufacture of a medicament for preventing and/or treating NLRP3 inflammasome-associated diseases.

19. Use of the compound or the pharmaceutically acceptable salt, the stereoisomer or the deuteride thereof according to any one of claims 1-16 or the pharmaceutical composition according to claim 17 in the manufacture of a medicament for preventing and/or treating inflammasome-associated diseases, immune diseases, inflammatory diseases, autoimmune diseases, or autoinflammatory diseases.

## INTERNATIONAL SEARCH REPORT

International application No.
**PCT/CN2023/129677**

### A. CLASSIFICATION OF SUBJECT MATTER

C07D487/04(2006.01)i; C07D237/34(2006.01)i; C07D401/12(2006.01)i; C07D405/12(2006.01)i; C07D471/04(2006.01)i; C07D237/26(2006.01)i; C07D495/04(2006.01)i; C07D409/12(2006.01)i; A61K31/502(2006.01)i; A61K31/504(2006.01)i; A61K31/53(2006.01)i; A61P37/02(2006.01)i

According to International Patent Classification (IPC) or to both national classification and IPC

### B. FIELDS SEARCHED

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, ENTXTC, CAPLUS(STN), REGISTRY(STN), CASREACT(STN), CNKI: 药捷安康, 李琳, 吴永谦, 哒嗪, 三嗪, 酞嗪, 炎症, NLPR?, +phthalazin+, +triazin+, +pyridazin+, 结构式检索, structural formula search.

### C. DOCUMENTS CONSIDERED TO BE RELEVANT

| Category* | Citation of document, with indication, where appropriate, of the relevant passages | Relevant to claim No. |
|---|---|---|
| PX | WO 2023028536 A1 (PTC THERAPEUTICS, INC.) 02 March 2023 (2023-03-02)<br>claims 1-11, and description, page 65 | 1-2, 8-10, 16-19 |
| X | CN 107266419 A (CYTOKINETICS INC.) 20 October 2017 (2017-10-20)<br>description, page 108, first compound | 1, 4, 6, 8, 11 |
| X | WO 2022216971 A1 (VENTUS THERAPEUTICS U.S., INC. et al.) 13 October 2022 (2022-10-13)<br>claims 1-42, and description, page 57 | 1, 3-8, 10-12, 14-19 |
| PX | WO 2023028534 A1 (PTC THERAPEUTICS, INC.) 02 March 2023 (2023-03-02)<br>claims 1-22, and description, page 429 | 1, 8-10, 17-19 |
| PX | WO 2022238347 A1 (F. HOFFMANN-LA ROCHE AG et al.) 17 November 2022 (2022-11-17)<br>claims 1-33, and description, page 67 | 1, 8, 17-19 |
| X | CN 113784957 A (NOVARTIS AG) 10 December 2021 (2021-12-10)<br>claims 1-19 | 1, 3-8, 11-12, 17-19 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 December 2023** | **05 January 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2023/129677

C. DOCUMENTS CONSIDERED TO BE RELEVANT

| Category* | Citation of document, with indication, where appropriate, of the relevant passages | Relevant to claim No. |
|---|---|---|
| X | CN 115279739 A (ASTELLAS PHARMA INC.) 01 November 2022 (2022-11-01) claims 1-17 | 1, 3-8, 11-12, 17-19 |
| PX | CN 115417856 A (CHENGDU AORUI PHARMACEUTICAL CO., LTD.) 02 December 2022 (2022-12-02) claims 1-4, 6-7, and 9-10 | 1, 8, 17-19 |
| PX | CN 116789674 A (HANGZHOU GAOGUANG PHARMACEUTICAL CO., LTD.) 22 September 2023 (2023-09-22) claims 1-3 and 5-28 | 1, 8, 17-19 |
| PX | WO 2022253936 A1 (F. HOFFMANN-LA ROCHE AG et al.) 08 December 2022 (2022-12-08) claims 1-22 and 25-34 | 1-2, 8-9, 17-19 |
| X | WO 2022166890 A1 (MEDSHINE DISCOVERY INC.) 11 August 2022 (2022-08-11) claims 1-25 | 1, 3-8, 11-12, 17-19 |
| PX | WO 2022230912 A1 (ASTELLAS PHARMA INC. et al.) 03 November 2022 (2022-11-03) claims 1-13 | 1-2, 8-9, 17-19 |
| PX | WO 2023066377 A1 (ZHEJIANG AIXPLORER BIOTECH CO., LTD.) 27 April 2023 (2023-04-27) claims 1-11 and 13-15 | 1-9, 11-12, 17-19 |
| PX | WO 2023088856 A1 (F. HOFFMANN-LA ROCHE AG et al.) 25 May 2023 (2023-05-25) claims 1-35 | 1-2, 8-9, 17-19 |
| PX | WO 2023088987 A1 (F. HOFFMANN-LA ROCHE AG et al.) 25 May 2023 (2023-05-25) claims 1-115 | 1, 3-8, 11-12, 17-19 |
| PX | WO 2023131277 A1 (TRANSTHERA SCIENCES (NANJING) , INC.) 13 July 2023 (2023-07-13) claims 1-18, and description, page 13, compounds 54-60, and pages 21-25, compounds 173-182, 189, 191, 193-198, 206, 221-222, 247-251, 253-254, and 259-264 et al. | 1-19 |
| PX | WO 2023186020 A1 (HANGZHOU HIGHLIGHTLL PHARMACEUTICAL CO., LTD.) 05 October 2023 (2023-10-05) claims 1-3 and 5-28 | 1, 3, 4, 7-9, 12, 17-19 |
| A | CN 113784957 A (NOVARTIS AG) 10 December 2021 (2021-12-10) claims 1-19 | 2, 9-10, 13-16 |

Form PCT/ISA/210 (second sheet) (July 2022)

**INTERNATIONAL SEARCH REPORT**
**Information on patent family members**

International application No.
**PCT/CN2023/129677**

| Patent document cited in search report | Publication date (day/month/year) | Patent family member(s) | Publication date (day/month/year) |
|---|---|---|---|
| WO 2023028536 A1 | 02 March 2023 | None | |
| CN 107266419 A | 20 October 2017 | US 2013143862 A1 | 06 June 2013 |
| | | US 8969346 B2 | 03 March 2015 |
| | | AR 081626 A1 | 10 October 2012 |
| | | KR 20130092403 A | 20 August 2013 |
| | | KR 101825735 B1 | 05 February 2018 |
| | | NZ 603593 A | 31 October 2014 |
| | | SG 10201700219 XA | 27 February 2017 |
| | | KR 20180014870 A | 09 February 2018 |
| | | KR 101960642 B1 | 20 March 2019 |
| | | CA 2796390 A1 | 27 October 2011 |
| | | CA 2796390 C | 01 October 2019 |
| | | JP 2016166217 A | 15 September 2016 |
| | | JP 6470224 B2 | 13 February 2019 |
| | | PH 12017501192 B1 | 11 December 2017 |
| | | BR 112012026950 A2 | 12 July 2016 |
| | | BR 112012026950 B1 | 08 October 2019 |
| | | WO 2011133882 A1 | 27 October 2011 |
| | | AU 2011242569 A1 | 06 December 2012 |
| | | AU 2011242569 B2 | 04 February 2016 |
| | | CO 6630154 A2 | 01 March 2013 |
| | | MY 191829 A | 18 July 2022 |
| | | TW 201204712 A | 01 February 2012 |
| | | TWI 501963 B | 01 October 2015 |
| | | US 2017281621 A1 | 05 October 2017 |
| | | US 10076519 B2 | 18 September 2018 |
| | | JP 2013525376 A | 20 June 2013 |
| | | JP 5918217 B2 | 18 May 2016 |
| | | IL 250824 A0 | 30 April 2017 |
| | | IL 250824 B | 31 March 2019 |
| | | CL 2012002945 A1 | 25 January 2013 |
| | | EP 3127541 A1 | 08 February 2017 |
| | | EP 3127541 B1 | 17 July 2019 |
| | | AU 2018201953 A1 | 12 April 2018 |
| | | AU 2018201953 B2 | 20 February 2020 |
| | | NZ 627973 A | 26 February 2016 |
| | | HK 1181662 A1 | 15 November 2013 |
| | | US 2015322018 A1 | 12 November 2015 |
| | | US 9604965 B2 | 28 March 2017 |
| | | EA 201201377 A1 | 30 April 2013 |
| | | EA 024881 B1 | 31 October 2016 |
| | | ECSP 12012292 A | 28 March 2013 |
| | | IL 222465 A0 | 31 December 2012 |
| | | IL 222465 A | 30 March 2017 |
| | | AU 2016202617 A1 | 19 May 2016 |
| | | AU 2016202617 B2 | 04 January 2018 |
| | | SG 184954 A1 | 29 November 2012 |
| | | MX 2012012259 A | 23 November 2012 |
| | | MX 343264 B | 28 October 2016 |
| | | EP 2563365 A1 | 06 March 2013 |

Form PCT/ISA/210 (patent family annex) (July 2022)

**INTERNATIONAL SEARCH REPORT**
**Information on patent family members**

International application No.
**PCT/CN2023/129677**

| Patent document cited in search report | | | Publication date (day/month/year) | Patent family member(s) | | | Publication date (day/month/year) |
|---|---|---|---|---|---|---|---|
| | | | | EP | 2563365 | A4 | 15 January 2014 |
| | | | | EP | 2563365 | B1 | 20 April 2016 |
| WO | 2022216971 | A1 | 13 October 2022 | WO | 2022216971 | A9 | 08 December 2022 |
| WO | 2023028534 | A1 | 02 March 2023 | | None | | |
| WO | 2022238347 | A1 | 17 November 2022 | AU | 2022275300 | A1 | 21 September 2023 |
| | | | | CA | 3214626 | A1 | 17 November 2022 |
| | | | | IL | 307188 | A | 01 November 2023 |
| | | | | AR | 125818 | A1 | 16 August 2023 |
| | | | | TW | 202311245 | A | 16 March 2023 |
| CN | 113784957 | A | 10 December 2021 | US | 2020361899 | A1 | 19 November 2020 |
| | | | | US | 11254653 | B2 | 22 February 2022 |
| | | | | DOP | 2021000228 | A | 15 December 2021 |
| | | | | JOP | 20210307 | A1 | 30 January 2023 |
| | | | | ECSP | 21080740 | A | 30 December 2021 |
| | | | | BR | 112021022796 | A2 | 12 April 2022 |
| | | | | US | 2022251067 | A1 | 11 August 2022 |
| | | | | PE | 20220133 | A1 | 27 January 2022 |
| | | | | IL | 287042 | A | 01 December 2021 |
| | | | | JP | 2022532354 | A | 14 July 2022 |
| | | | | CA | 3138226 | A1 | 26 November 2020 |
| | | | | WO | 2020234715 | A1 | 26 November 2020 |
| | | | | AU | 2020277738 | A1 | 18 November 2021 |
| | | | | AU | 2020277738 | B2 | 02 March 2023 |
| | | | | KR | 20220008887 | A | 21 January 2022 |
| | | | | US | 2020361898 | A1 | 19 November 2020 |
| | | | | US | 11208399 | B2 | 28 December 2021 |
| | | | | SG | 11202111029 | PA | 30 December 2021 |
| | | | | MX | 2021013941 | A | 11 March 2022 |
| | | | | CO | 2021015030 | A2 | 30 November 2021 |
| | | | | AR | 119731 | A1 | 05 January 2022 |
| | | | | EP | 3969441 | A1 | 23 March 2022 |
| | | | | CR | 20210552 | A | 26 November 2021 |
| | | | | TW | 202110809 | A | 16 March 2021 |
| | | | | CU | 20210094 | A7 | 06 June 2022 |
| | | | | CL | 2021003012 | A1 | 09 September 2022 |
| CN | 115279739 | A | 01 November 2022 | TW | 202204323 | A | 01 February 2022 |
| | | | | WO | 2021193897 | A1 | 30 September 2021 |
| | | | | EP | 4129986 | A1 | 08 February 2023 |
| | | | | AR | 121669 | A1 | 29 June 2022 |
| | | | | US | 2023107277 | A1 | 06 April 2023 |
| | | | | JPWO | 2021193897 | A1 | 30 September 2021 |
| CN | 115417856 | A | 02 December 2022 | WO | 2023051761 | A1 | 06 April 2023 |
| CN | 116789674 | A | 22 September 2023 | | None | | |
| WO | 2022253936 | A1 | 08 December 2022 | TW | 202313577 | A | 01 April 2023 |
| | | | | IL | 307201 | A | 01 November 2023 |
| | | | | CA | 3216026 | A1 | 08 December 2022 |
| | | | | AR | 126053 | A1 | 06 September 2023 |
| | | | | AU | 2022287224 | A1 | 21 September 2023 |
| WO | 2022166890 | A1 | 11 August 2022 | | None | | |

Form PCT/ISA/210 (patent family annex) (July 2022)

**INTERNATIONAL SEARCH REPORT**
**Information on patent family members**

International application No.
**PCT/CN2023/129677**

| Patent document cited in search report | Publication date (day/month/year) | Patent family member(s) | Publication date (day/month/year) |
|---|---|---|---|
| WO 2022230912 A1 | 03 November 2022 | TW 202309000 A | 01 March 2023 |
| | | AU 2022267160 A1 | 23 November 2023 |
| | | CA 3218212 A1 | 03 November 2022 |
| WO 2023066377 A1 | 27 April 2023 | None | |
| WO 2023088856 A1 | 25 May 2023 | None | |
| WO 2023088987 A1 | 25 May 2023 | TW 202327603 A | 16 July 2023 |
| WO 2023131277 A1 | 13 July 2023 | TW 202327604 A | 16 July 2023 |
| WO 2023186020 A1 | 05 October 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020234715 A1